(19) Europäisches Patentamt
European Patent Office
Office européen des brevets

(11) EP 4 389 738 A1

(12) **EUROPEAN PATENT APPLICATION**
published in accordance with Art. 153(4) EPC

(43) Date of publication:
**26.06.2024 Bulletin 2024/26**

(21) Application number: **22858734.1**

(22) Date of filing: **17.08.2022**

(51) International Patent Classification (IPC):
*C07D 237/14* (2006.01) *A61K 31/50* (2006.01)
*A61P 35/00* (2006.01) *C07D 405/04* (2006.01)
*C07D 401/04* (2006.01) *C07D 409/04* (2006.01)

(86) International application number:
**PCT/KR2022/012254**

(87) International publication number:
**WO 2023/022497 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.08.2021 KR 20210108316**

(71) Applicants:
- **Kanaph Therapeutics Inc.**
  **Seoul 04348 (KR)**
- **Cyrus Therapeutics Inc.**
  **Seoul 05836 (KR)**

(72) Inventors:
- **YU, Ha Na**
  **Gwacheon-si, Gyeonggi-do 13826 (KR)**
- **SHIN, Young Sook**
  **(Deceased) (KR)**
- **PARK, Dohyun**
  **Seoul 03736 (KR)**
- **YOON, Kyeong Jin**
  **Seoul 06065 (KR)**
- **LIM, Sang Kyun**
  **Seoul 04211 (KR)**
- **KIM, Donggeon**
  **Seoul 05629 (KR)**
- **KI, Dong Hyuk**
  **Gwacheon-si, Gyeonggi-do 13839 (KR)**
- **KIM, Eun-Jung**
  **Seoul 06788 (KR)**
- **NAM, Joonwoo**
  **Seongnam-si, Gyeonggi-do 13527 (KR)**
- **HAN, Wooseok**
  **Hanam-si, Gyeonggi-do 13015 (KR)**
- **YU, Jihyun**
  **Yongin-si, Gyeonggi-do 16830 (KR)**
- **KIM, Ji Eun**
  **Suwon-si, Gyeonggi-do 16700 (KR)**

(74) Representative: **HGF**
**HGF Limited**
**1 City Walk**
**Leeds LS11 9DX (GB)**

(54) **SOS1 INHIBITOR AND USE THEREOF**

(57) There is provided a novel compound of Formula I and a use thereof for the prevention or treatment of a disease associated with SOS1. In one aspect of the present invention, the novel compound is useful for the prevention or treatment of a SOS1 mediated disease such as cancer and RASopathy by inhibiting the interaction between SOS1 and RAS family proteins or between SOS1 and RAC1.

EP 4 389 738 A1

## Description

### Technical Field

The present invention relates to a novel compound having SOS1 inhibitory activity, a solvate, stereoisomer or pharmaceutically acceptable salt thereof, a pharmaceutical composition for preventing or treating a disease, comprising the same as an active ingredient, and a medicinal use thereof.

### Background Art

Mutations in the RAS gene are a major oncogene with a high incidence in human cancers, and are observed in 20 to 30% of human cancers, particularly in lung cancer, colon cancer, rectal cancer, and pancreatic cancer at high rates. RAS-family proteins include KRAS, NRAS or HRAS.

RAS proteins are small GTPases that exist in cells in either a GTP-bound or GDP-bound state, and are molecular switches that cycle between an active GTP-bound state and an inactive GDP-bound state. Mutations in the RAS gene reduce the ability of the RAS, a GTPase to hydrolyze GTP, leaving this molecular switch to maintain a constitutively active GTP-bound conformation, thereby inducing oncogenic downstream signaling (for example, Raf-MEK-ERK pathway or PI3K-PDK1-Akt pathway).

Meanwhile, binding of GTPase activating protein (GAP) such as NF1 accelerates the weak intrinsic GTPase activity of RAS proteins, thereby downregulating active RAS and returning it to an inactive form. On the other hand, binding of guanine nucleotide exchange factors (GEF) such as SOS1 promotes the release of GDP from RAS proteins and increases the GTP-bound active state.

Various studies on methods for directly or indirectly inhibiting RAS have been conducted in the prior art. However, it has been found that direct inhibition of RAS is extremely difficult due to the picomolar level of affinity of GTP for the binding site, lack of other well-defined pockets, and the fact that RAS interacts with GEFs, GAPs and effectors through the wide and flat protein-protein interaction surface, which makes difficult to apply small molecule drugs, and the like. In addition, a method of indirectly inhibiting RAS by targeting farnesyl transferase has also been attempted, but an approved drug has not yet been prepared. In view of this failure to directly or indirectly inhibit RAS, it has been generally considered to be difficult to target RAS for drug development.

Under these circumstances, a method of inhibiting RAS by inhibiting the interaction between RAS and GEF to prevent the reloading of GTP has emerged.

SOS1 (Son of Sevenless 1) is a type of guanine nucleotide exchange factor (GEF), which promotes the release of GDP from RAS family proteins to allow GTP binding, thereby regulating RAS family protein signaling. Son of Sevenless (SOS) protein exists in two isoforms, SOS1 and SOS2, and only SOS1 is phosphorylated by ERK. Growth factor-induced phosphorylation of SOS1 is mostly mediated by ERK, which phosphorylates at least four serine residues in the C-terminal region of SOS1. This suggests that SOS1 plays an important role in the regulation of negative feedback of the KRAS pathway. The SOS1 protein consists of 1333 amino acids (150 kDa). SOS1 is a multi-domain protein having two tandem N-terminal histone domains (HD) followed by a Dbl homology domain (DH), a plextrin homology domain (PH), a helical linker (HL), a RAS exchange motif (REM), a CDC25 homology domain and a C-terminal proline rich domain (PR). SOS1 has two binding sites for RAS family proteins (i.e., a catalytic site that binds GDP-binding RAS family proteins and promotes exchange of guanine nucleotides, and an allosteric site that binds GTP-binding RAS family proteins and up-regulates a catalytic site activity of SOS1) (J. Med. Chem. 2021, 64, 10, 6569-6580). Selective pharmacological inhibition of catalytic site binding of SOS1 to RAS family proteins is expected to prevent SOS1-mediated activation of RAS-family proteins in a GTP-bound form.

Therefore, SOS1 inhibitor compounds are expected to inhibit signaling (for example, ERK phosphorylation) in cells downstream of RAS-family proteins, and thus novel SOS1 inhibitor compounds that bind to the SOS1 catalytic site and prevent binding and activation of RAS family proteins are being developed.

It has been reported that SOS1 is critically involved in mutant KRAS activation and oncogenic signaling in cancer (Current Opinion in Chemical Biology, 2021, 62: 109-118). Depletion of SOS1 levels reduced the survival of tumor cells with KRAS mutations, but no such effect was observed in KRAS wild-type cell lines. The SOS1 depletion effect cannot be rescued by the $SOS1^{F929A}$ mutation in which the catalytic site is damaged or the SOS1 mutation ($SOS1^{L687E/R688A}$) in which the GTP-KRAS binding is defective at the allosteric site, which suggests that targeting the catalytic site or the allosteric site of SOS1 may be an effective option for the treatment of KRAS mutation cancers.

In addition, SOS1 is critically involved in the activation of RAS family protein signaling in cancer through mechanisms other than mutation of RAS family proteins. SOS1 interacts with the adapter protein Grb2 to form the SOS1/Grb2 complex. The complex binds to an activated/phosphorylated receptor tyrosine kinase (for example, EGFR, ErbB2, ErbB3, ErbB4, PDGFR-A/B, FGFR1/2/3, IGF1R, INSR, ALK, ROS, TrkA, TrkB, TrkC, RET, c-MET, VEGFR1/2/3, AXL). In addition, it has been reported that SOS1 is localized to other phosphorylated cell surface receptors such as T cell receptor

(TCR), B cell receptor (BCR) and monocyte colony stimulating factor receptor, resulting in activating RAS family proteins.

Furthermore, SOS1 is a GEF for activation of the GTPase RAC1 (Ras-associated C3 botulinum toxin substrate 1). RAC1, like the RAS-family protein, is known to be involved in the pathogenesis of various cancers and other diseases.

Currently, BI-3406, BI-1701963, MRTX0902, and the like are being developed as inhibitors of SOS1 activity, but they are still in the early stages of development. Therefore, there is still a need in the art for the development of a novel compound for treating cancer by inhibiting SOS1 and a pharmaceutical composition comprising the same.

## Detailed Description of Invention

### Technical Problem

An object of the present invention is to provide a compound of Formula I, a solvate, stereoisomer or pharmaceutically acceptable salt thereof.

Another object of the present invention is to provide a pharmaceutical composition comprising a compound of Formula I, a solvate, stereoisomer or pharmaceutically acceptable salt thereof.

Another object of the present invention is to provide a method for preventing or treating a SOS1 mediated disease by administering a compound of Formula I, a solvate, stereoisomer or pharmaceutically acceptable salt thereof.

### Solution to Problem

Each description and embodiment disclosed herein may also apply to each other description and embodiment. That is, all combinations of the various elements disclosed herein fall within the scope of the present application. In addition, it should not be construed that the scope of the present application is limited by the specific description set forth below.

In one aspect of the present invention, there is provided a compound of Formula 1 below, a solvate, stereoisomer or pharmaceutically acceptable salt thereof:

[Formula 1]

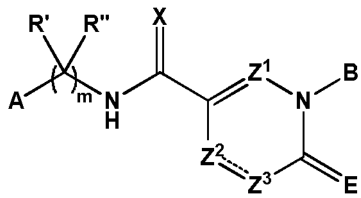

in Formula 1,

- \- - - - - - is a single bond or a double bond;
- E is O or S; and X is O or S;
- $Z^1$ is N or CH, $Z^2$ is N, NH, $CR^1$ or $CHR^1$, and $Z^3$ is $CR^1$ or $CHR^1$, provided that at most one of $Z^1$, $Z^2$ and $Z^3$ is N or NH, each $R^1$ is independently selected from the group consisting of H, halogen, OH, CN, $NR^bR^c$, $C_1$-$C_6$ alkyl optionally interrupted by 1 to 3 oxygen atoms or nitrogen atoms and/or optionally substituted, optionally substituted $C_2$-$C_6$ alkenyl, optionally substituted $C_2$-$C_6$ alkynyl, optionally substituted $C_1$-$C_6$ alkoxy, optionally substituted $C_1$-$C_6$ acylamino, optionally substituted ($C_1$-$C_6$ alkyl)sulfonylamino, optionally substituted $C_3$-$C_6$ cycloalkyl, optionally substituted 4- to 7-membered heterocycloalkyl, optionally substituted $C_6$-$C_{10}$ aryl, optionally substituted $C_6$-$C_{10}$ aryloxy, optionally substituted ($C_6$-$C_{10}$ aryl)-($C_1$-$C_6$ alkyl)oxy-, optionally substituted ($C_6$-$C_{10}$ aryl)amino and optionally substituted 5- to 10-membered heteroaryl; or
- when $Z^1$ is N, - - - - - - is a double bond, and $Z^2$ and $Z^3$ are both $CR^1$, then two $R^1$ are optionally linked to each other together with the carbon atom to which they are attached to form 5-membered heteroaryl containing one N, O or S;
- R' and R" are each independently H or $C_1$-$C_3$ alkyl, or R' and R" bonded to the same carbon or adjacent carbons may be taken together with the carbon atom to which they are attached to form $C_3$-$C_4$ cycloalkyl, and said $C_1$-$C_3$ alkyl and $C_3$-$C_4$ cycloalkyl may be optionally substituted with at least one halogen, OH, CN, $C_1$-$C_3$ alkoxy or $NR^bR^c$;
- m is an integer of 1 to 3;
- A is $Cy_1$ or $Cy_1$-Y-$Cy_2$;
- Y is $NR^d$, $CR^dR^e$, O, S, or a direct bond;
- $Cy_1$ and $Cy_2$ are each independently $C_6$-$C_{10}$ aryl optionally fused with $C_3$-$C_8$ cycloalkyl, or 5- to 10-membered heteroaryl;

said $Cy_1$ and $Cy_2$ may be each optionally substituted with 1 to 3 $R^2$;
$R^2$ is selected from the group consisting of H, halogen, OH, CN, oxo, amino, $-NR^bR^C$, $-N=S(O)R^b$, $-N=S(O)NR^bR^c$, $-SF_5$, $-Si(C_1-C_3\ alkyl)_3$, $-SO_2R^b$, $-C(O)R^b$, $C_1-C_6$ alkyl optionally interrupted by 1 to 3 oxygen atoms or nitrogen atoms and/or optionally substituted, optionally substituted $C_1-C_6$ alkoxy and optionally substituted $C_3-C_6$ cycloalkyl;
B is H, optionally substituted $C_1-C_6$ alkyl, $-(CH_2)_o-Cy_3$ or $-(CH_2)_o-Cy_3-W-Cy_4$;
o is an integer of 0 to 3;
W is $NR^d$, $CR^dR^e$, C(O), O, S, or a direct bond;
$Cy_3$ and $Cy_4$ are each independently selected from the group consisting of $C_3-C_6$ monocyclic cycloalkyl or $C_3-C_6$ monocyclic cycloalkenyl, optionally fused with 5- to 10-membered heterocycloalkyl or 5- to 10-membered heteroaryl; bicyclic, tricyclic or tetracyclic bridged, fused or spiro $C_5-C_{20}$ cycloalkyl or $C_5-C_{20}$ cycloalkenyl; $C_6-C_{10}$ aryl optionally fused with 5- to 10-membered heterocycloalkyl; 5- to 10-membered monocyclic heteroaryl optionally fused with $C_3-C_6$ cycloalkyl; 5- to 10-membered bicyclic heteroaryl; 4- to 10-membered saturated or partially unsaturated monocyclic heterocycloalkyl optionally fused with $C_3-C_6$ cycloalkyl; and 5-to 10-membered bicyclic bridged, fused or spiro heterocycloalkyl;
said $Cy_3$ and $Cy_4$ may be each independently optionally substituted with 1 to 3 $R^3$;
$R^3$ is selected from the group consisting of H, deuterium, halogen, OH, CN, oxo, $-NR^bR^c$, $-N=S(O)R^b$, $-N=S(O)NR^bR^c$, $-SO_2R^b$, $-C(O)R^b$, $-C(O)OR^b$, $-CONR^bR^c$, $-NR^bCOR^c$, $-NR^bC(O)OR^c$, $-NR^bSO_2R^c$, $-NHCO-(C_3-C_6\ cycloalkyl)$, optionally substituted $C_1-C_6$ alkyl, optionally substituted $C_1-C_6$ alkoxy and optionally substituted $C_3-C_6$ cycloalkyl;
$R^b$ and $R^c$ are each independently H or optionally substituted $C_1-C_6$ alkyl; and
$R^d$ and $R^e$ are each independently H or optionally substituted $C_1-C_6$ alkyl.

In the present disclosure, "optionally substituted" as used in the definition of substituents may mean that the structure is unsubstituted or substituted with at least one substituent selected from the group consisting of:

(i) halogen, OH, CN, oxo, $NH_2$, $NH(C_1-C_6\ alkyl)$, or $N(C_1-C_6\ alkyl)_2$;
(ii) $C_1-C_3$ alkyl optionally substituted with at least one substituent selected from the group consisting of halogen, OH, CN, oxo, $NH_2$, $NH(C_1-C_6\ alkyl)$ and $N(C_1-C_6\ alkyl)_2$;
(iii) $C_1-C_3$ alkoxy optionally substituted with at least one substituent selected from the group consisting of halogen, OH, CN, oxo, $NH_2$, $NH(C_1-C_6\ alkyl)$ and $N(C_1-C_6\ alkyl)_2$; and
(iv) $C_3-C_6$ cycloalkyl optionally substituted with at least one substituent selected from the group consisting of halogen, OH, CN, oxo, $NH_2$, $NH(C_1-C_6\ alkyl)$ and $N(C_1-C_6\ alkyl)_2$.

In one embodiment, the optionally substituted moiety may be substituted with one or more identical or different substituents selected from the group consisting of halogen, OH, CN, $NH_2$, $NH(C_1-C_6\ alkyl)$, $N(C_1-C_6\ alkyl)_2$ and $C_1-C_3$ alkoxy.

In one embodiment, an "optionally substituted" group may be unsubstituted or substituted with at least one substituent selected from the group consisting of deuterium, halogen, OH, CN, oxo, amino, $C_1-C_6$ alkylamino, di($C_1-C_6$ alkyl)amino, $C_1-C_6$ haloalkyl, $C_1-C_6$ hydroxyalkyl, $C_1-C_6$ cyanoalkyl, $C_1-C_6$ aminoalkyl and $C_1-C_6$ alkoxy. In this case, two or more substituents may be substituted on the same atom or different atoms. For example, 1-fluoro-2-oxopropyl is an alkyl group substituted with oxo and fluoro, respectively, on different carbon atoms of the propyl group, and is encompassed by "optionally substituted alkyl" in the present disclosure. In the present specification, when two or more substituents are substituted on the same moiety, they may be substituted on the same atom or different atoms of the moiety.

In Formula 1 above, E may be O or S. For example, E may be O.

In Formula 1, X may be O or S. For example, X may be O.

In Formula 1, $Z^1$ may be N or CH, $Z^2$ may be N, NH, $CR^1$ or $CHR^1$, and $Z^3$ may be $CR^1$ or $CHR^1$. ------ may be a single bond or a double bond. However, at most one of $Z^1$, $Z^2$ and $Z^3$ is N or NH.

Alternatively, when $Z^1$ is N, ------ is a double bond, and $Z^2$ and $Z^3$ are both $CR^1$, then two $R^1$ may be optionally linked to each other together with the carbon atom to which they are attached to form a 5-membered heteroaryl ring containing one N, O or S.

In Formula 1 above, m may be an integer of 1 to 3. For example, m may be 1 or 2. In one embodiment, m may be 1. When m is 2 or 3, R' bonded to each carbon of the alkylene chain may be the same or different from each other. When m is 2 or 3, R" bonded to each carbon of the alkylene chain may be the same or different from each other.

In one embodiment, R' and R" may be each independently H or $C_{1-3}$ alkyl, for example, $-CH_3$, $-CH_2CH_3$, $-CH_2CH_2CH_3$, $-CH(CH_3)_2$. Said $C_1-C_3$ alkyl may be optionally substituted with at least one halogen, OH, CN, $C_1-C_3$ alkoxy or $NR^bR^c$. In this case, $R^b$ and $R^c$ may be each independently H or optionally substituted $C_1-C_3$ alkyl. In one embodiment, R' and R" may be both $C_{1-3}$ alkyl. In one embodiment, R' and R" may be both H. In one embodiment, one of R' and R" may be H, and the other may be $C_{1-3}$ alkyl. For example, one of R' and R" may be H, and the other may

be methyl, ethyl, difluoromethyl, fluoromethyl, hydroxymethyl, aminomethyl, and the like, but is not limited thereto.

In some embodiments, R' and R" bonded to the same carbon or adjacent carbons may be taken together with the carbon atom to which they are attached to form a cyclopropyl or cyclobutyl ring. The cyclopropyl or cyclobutyl ring may be optionally substituted with at least one halogen, OH, CN, $C_1$-$C_3$ alkoxy or $NR^bR^c$. In this case, $R^b$ and $R^c$ may be each independently H or optionally substituted $C_1$-$C_3$ alkyl. For example, R' and R" may be taken together with the alkylene chain to which they are attached to form the following structures, but not limited to:

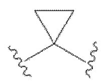, 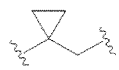, 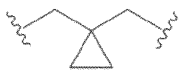, 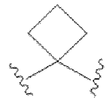, 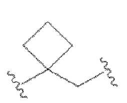, 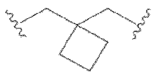, 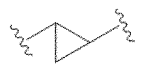,

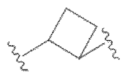, 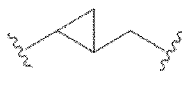, or 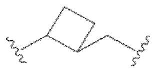.

In Formula 1 above, $R^1$ may be H, halogen, OH, CN, $NR^bR^c$, $C_1$-$C_6$ alkyl optionally interrupted by 1 to 3 oxygen atoms or nitrogen atoms, optionally substituted $C_1$-$C_6$ alkyl, optionally substituted $C_2$-$C_6$ alkenyl, optionally substituted $C_2$-$C_6$ alkynyl, optionally substituted $C_1$-$C_6$ alkoxy, optionally substituted $C_1$-$C_6$ acylamino, optionally substituted ($C_1$-$C_6$ alkyl)sulfonylamino, or $C_3$-$C_6$ cycloalkyl. In one embodiment, $R^1$ may be H, OH, $CH_3$, $-CH=CH_2$, $-C=CH$, CN, or optionally substituted cyclopropyl.

In one embodiment, $R^1$ may be optionally substituted $C_1$-$C_6$ alkyl, optionally substituted $C_2$-$C_6$ alkenyl, or optionally substituted $C_2$-$C_6$ alkynyl, preferably optionally substituted $C_1$-$C_3$ alkyl. In this case, optionally substituted $C_1$-$C_6$ alkyl or $C_1$-$C_3$ alkyl, optionally substituted $C_2$-$C_6$ alkenyl, or optionally substituted $C_2$-$C_6$ alkynyl may be substituted with at least one substituent selected from the group consisting of substituents (i) to (iv) described above. In this case, at least one substituent may include a combination of two or more substituents selected from any one of (i), (ii), (iii) and (iv), or a combination of two or more substituents each selected from two or more of (i), (ii), (iii) and (iv), or a combination thereof. When there are two or more substituents, they may be the same or different from each other. For example, optionally substituted $C_1$-$C_6$ alkyl or $C_1$-$C_3$ alkyl may be substituted with 1 to 5, 1 to 4, 1 to 3, 1, 2 or 3 substituents. For example, they may include $-CF_2CH_2OH$ substituted with two F and one OH, $-CF_3$ substituted with three F, and the like.

Said $C_1$-$C_6$ alkyl may be optionally interrupted by 1 to 3 oxygen atoms or nitrogen atoms, and it may include, for example, methoxymethyl, methoxymethoxymethyl, ethoxymethyl, ethoxyethoxymethyl, methylaminomethyl, methylaminoethyl, dimethylaminomethyl, dimethylaminoethyl, and the like, but is not limited thereto.

In some embodiments, $R^1$ may be optionally substituted 4- to 7-membered heterocycloalkyl, optionally substituted $C_6$-$C_{10}$ aryl, optionally substituted $C_6$-$C_{10}$ aryloxy, optionally substituted ($C_6$-$C_{10}$ aryl)-($C_1$-$C_6$ alkyl)oxy-, optionally substituted ($C_6$-$C_{10}$ aryl)amino or optionally substituted 5- to 10-membered heteroaryl. In this case, the substituents that may be optionally substituted are as described above. In one embodiment, $R^1$ may be 4- to 7-membered heterocycloalkyl containing 1 or 2 heteroatoms selected from N, O and S, and may be, for example, azetidinyl, oxetanyl, pyrrolidinyl, tetrahydrofuranyl, piperidinyl or piperazinyl, but is not limited thereto. In one embodiment, $R^1$ may include 5- to 10-membered heteroaryl containing 1 or 2 heteroatoms selected from N, O and S, for example, but not limited to, indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl. In one embodiment, $R^1$ may be phenyl or naphthyl.

In Formula 1 of the present invention, A may be $Cy_1$ or $Cy_1$-Y-$Cy_2$. In this case, Y may be $NR^d$, $CR^dR^e$, O, S, or a direct bond. $R^d$ and $R^e$ may be each H or optionally substituted $C_1$-$C_6$ alkyl, preferably H or optionally substituted $C_1$-$C_3$ alkyl. In this case, the substituents that may be optionally substituted are as described above.

In A in Formula 1 above, $Cy_1$ and $Cy_2$ may be each independently $C_6$-$C_{10}$ aryl, $C_6$-$C_{10}$ aryl fused with $C_3$-$C_8$ cycloalkyl, or 5- to 10-membered heteroaryl.

In one embodiment, said $Cy_1$ may be $C_6$-$C_{10}$ aryl, or 5- to 6-membered heteroaryl containing 1 to 3 heteroatoms selected from N, O or S. In one embodiment, $Cy_1$ may be $C_6$-$C_{10}$ aryl. In another embodiment, $Cy_1$ may be 5- to 6-membered heteroaryl containing one or two N or S. For example, $Cy_1$ may include phenyl, naphthalenyl, thiazolyl, thiophenyl or pyrazolyl.

In one embodiment, said $Cy_2$ may be $C_6$-$C_{10}$ aryl fused with $C_3$-$C_6$ cycloalkyl, $C_6$-$C_{10}$ aryl, or 5- to 6-membered heteroaryl containing 1 to 3 heteroatoms selected from N, O or S. In one embodiment, $Cy_2$ may be $C_6$-$C_{10}$ aryl fused with $C_3$-$C_5$ cycloalkyl, or $C_6$-$C_{10}$ aryl. In another embodiment, $Cy_2$ may be 5- to 6-membered heteroaryl containing one or two N or S. For example, $Cy_2$ may include phenyl, 2,3-dihydroindenyl or bicyclo[4.2.0]octa-1,3,5-trienyl, pyrazolyl, thiophenyl, pyridinyl, 2-oxo-1,2-dihydropyridinyl or pyrrolyl.

In some embodiments, A may be $Cy_1$, wherein $Cy_1$ may be $C_6$-$C_{10}$ aryl, such as phenyl or naphthyl. In some embodiments, A may be $Cy_1$, wherein $Cy_1$ may be 5- to 10-membered heteroaryl. In some embodiments, A may be

$Cy_1$-Y-$Cy_2$, wherein $Cy_1$ and $Cy_2$ may be each $C_6$-$C_{10}$ aryl, and Y may be O. For example, A may be phenyl-O-phenyl. In some embodiments, A may be $Cy_1$-Y-$Cy_2$, wherein $Cy_1$ may be $C_6$-$C_{10}$ aryl, and $Cy_2$ may be 5- to 10-membered heteroaryl. In some embodiments, A may be $Cy_1$-Y-$Cy_2$, wherein $Cy_1$ may be 5- to 10-membered heteroaryl, and $Cy_2$ is $C_6$-$C_{10}$ aryl.

In some specific embodiments, said 5- to 10-membered heteroaryl of $Cy_1$ or $Cy_2$ may be indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl, but is not limited thereto.

In addition, said $Cy_1$ and $Cy_2$ may be each optionally substituted with 1 to 3 $R^2$. $R^2$ is halogen, OH, CN, oxo, amino, $-NR^bR^c$, $-N=S(O)R^b$, $-N=S(O)NR^bR^c$, $-SF_5$, -Si($C_1$-$C_3$ alkyl)$_3$, - $SO_2R^b$, $-C(O)R^b$, $C_1$-$C_6$ alkyl optionally interrupted by 1 to 3 oxygen atoms or nitrogen atoms, optionally substituted $C_1$-$C_6$ alkyl, optionally substituted $C_1$-$C_6$ alkoxy and optionally substituted $C_3$-$C_6$ cycloalkyl. In this case, the substituents that may be optionally substituted are as described above. In addition, specific substituents of $R^2$ are as described in Formula I below.

In Formula 1 of the present invention, when A is $Cy_1$-Y-$Cy_2$, then $Cy^1$ may be optionally substituted with 1 to 3 $R^{2a}$, and $Cy_2$ may be optionally substituted with 1 to 3 $R^{2b}$. $R^{2a}$ and $R^{2b}$ are as described in Formula I below.

In Formula 1 of the present invention, B may be H, optionally substituted $C_1$-$C_6$ alkyl, - $(CH_2)_o$-$Cy_3$ or $-(CH_2)_o$-$Cy_3$-W-$Cy_4$. In this case, o may be an integer of 0 to 3. In addition, o may be 0 or 1.

In some embodiments, B may be $-(CH_2)_o$-$Cy_3$. In some embodiments, B may be $-(CH_2)_o$-$Cy_3$-W-$Cy_4$. In this case, W may be $NR^d$, $CR^dR^e$, O, S, or a direct bond, and $R^d$ and $R^e$ may be each H or optionally substituted $C_1$-$C_6$ alkyl, preferably H or optionally substituted $C_1$-$C_3$ alkyl. In this case, the substituents that may be optionally substituted are as described above.

In Formula 1 of the present invention, $Cy_3$ and $Cy_4$ are each independently $C_3$-$C_6$ monocyclic cycloalkyl or $C_3$-$C_6$ monocyclic cycloalkenyl, wherein the cycloalkyl or cycloalkenyl may be optionally fused with 5- to 10-membered heterocycloalkyl or 5- to 10-membered heteroaryl. In one embodiment, $Cy_3$ and $Cy_4$ may be each independently cyclopropyl; cyclobutyl, cyclopentyl, cyclohexyl; cyclobutenyl; cyclopentenyl, cyclohexenyl; cyclohexyl or cyclopentyl fused with pyrazole, piperazine or tetrahydropyran. In one embodiment, said 5- to 10-membered heteroaryl fused with cycloalkyl or cycloalkenyl may include indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl, but is not limited thereto. In one embodiment, said 5- to 10-membered heterocycloalkyl fused with cycloalkyl or cycloalkenyl may include tetrahydropyranyl, piperidinyl, tetrahydrofuranyl, or tetrahydro 2H-thiopyranyl, but is not limited thereto.

In some embodiments, $Cy_3$ and $Cy_4$ may be each independently bicyclic, tricyclic or tetracyclic bridged, fused or spiro $C_5$-$C_{20}$ cycloalkyl or $C_5$-$C_{20}$ cycloalkenyl. In one embodiment, $Cy_3$ and $Cy_4$ may be each independently bicyclic or tricyclic bridged or fused $C_5$-$C_{15}$ cycloalkyl or $C_5$-$C_{15}$ cycloalkenyl. In one embodiment, $Cy_3$ may be bicyclic or tricyclic bridged $C_5$-$C_{10}$ cycloalkyl or $C_5$-$C_{10}$ cycloalkenyl. In one embodiment, said $Cy_3$ may be bicyclo[2.2.2]octanyl, adamantyl, bicyclo[2.2.1]heptanyl, or bicyclo[2.2.1]hept-2-enyl, bicyclo[1.1.1]pentanyl, but is not limited thereto.

In some embodiments, $Cy_3$ and $Cy_4$ may be each independently $C_6$-$C_{10}$ aryl. In one embodiment, $Cy_3$ may be phenyl or naphthyl.

In some embodiments, $Cy_3$ and $Cy_4$ may be each independently 5- to 10-membered monocyclic or bicyclic heteroaryl, 5- to 10-membered monocyclic heterocycloalkyl, or 5- to 10-membered bicyclic bridged, fused or spiro heterocycloalkyl, wherein said 5- to 10-membered monocyclic heteroaryl and 5- to 10-membered monocyclic heterocycloalkyl may be optionally fused with $C_3$-$C_6$ cycloalkyl. In one embodiment, said 5- to 10-membered heteroaryl may contain 1 or 2 heteroatoms selected from N, O or S, and said 5- to 10-membered heterocycloalkyl may be 5- or 6-membered heterocycloalkyl containing 1 heteroatom selected from N, O or S. In one embodiment, said 5- to 10-membered heteroaryl may include indolyl, pyridinyl, pyrimidinyl, pyrazinyl, pyridazinyl, thiophenyl, pyrazolyl, oxazolyl, isoxazolyl, oxadiazolyl, thiadiazolyl, thiazolyl, isothiazolyl, imidazolyl, or triazolyl, but is not limited thereto. In one embodiment, said 5- or 6-membered heterocycloalkyl may include tetrahydropyranyl, piperidinyl, tetrahydrofuranyl, or tetrahydro 2H-thiopyranyl, but is not limited thereto. In one embodiment, said 5- to 10-membered heteroaryl or 5- to 10-membered heterocycloalkyl may be optionally fused with $C_3$-$C_6$ cycloalkyl or $C_3$-$C_6$ cycloalkenyl, and may form, for example, pyrazolyl, piperazine or tetrahydropyran fused with cyclohexyl. In one embodiment, said 5- to 10-membered heterocycloalkyl may be bicyclic bridged, fused or spiro heterocycloalkyl, which may be, for example, 3-oxabicyclo[2.1.1]hexanyl or 2-oxabicyclo[2.1.1]hexanyl, but is not limited thereto. In one embodiment, $Cy_3$ may be phenyl, naphthyl, pyridinyl, thiophenyl, tetrahydropyranyl or piperidinyl.

In some embodiments, B may be $-(CH_2)_o$-$Cy_3$-W-$Cy_4$, wherein $Cy_3$ and W may be as described above, and $Cy_4$ may be phenyl or naphthyl. In one embodiment, $Cy_3$ and $Cy_4$ may be each phenyl, and W may be a direct bond.

In one embodiment, B may be $-(CH_2)_o$-$Cy_3$-W-$Cy_4$, wherein $Cy_3$ may be selected from the group consisting of $C_3$-$C_6$ cycloalkyl, $C_3$-$C_6$ cycloalkenyl, 5- or 6-membered saturated or partially unsaturated heterocycloalkyl containing 1 or 2 heteroatoms selected from N, O or S, bridged bicyclic $C_{5-10}$ cycloalkyl, $C_6$-$C_{10}$ aryl optionally fused with 5- or 6-membered heterocycloalkyl containing 1 or 2 heteroatoms selected from N, O or S, and 5- or 6-membered monocyclic heteroaryl or 5- to 10-membered bicyclic heteroaryl containing 1 or 2 heteroatoms selected from N, O or S. In this case,

W may be NH, C(O) or a direct bond. In addition, $Cy_4$ may be selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered monocyclic heteroaryl containing 1 to 4 heteroatoms selected from N, O or S.

In B in Formula 1 of the present invention, said $Cy_3$ and $Cy_4$ may be each independently optionally substituted with 1 to 3 $R^3$. $R^3$ may be halogen, OH, CN, oxo, amino, $-NR^bR^c$, $-N=S(O)R^b$, $-N=S(O)NR^bR^c$, $-SO_2R^b$, $-C(O)R^b$, $-CONR^bR^c$, $-NR^bCOR^c$, $NR^bSO_2R^c$, optionally substituted $C_1$-$C_6$ alkyl, optionally substituted $C_1$-$C_6$ alkoxy and optionally substituted $C_3$-$C_6$ cycloalkyl, wherein $R^b$ and $R^c$ may be H or optionally substituted $C_1$-$C_6$ alkyl, preferably optionally substituted $C_1$-$C_3$ alkyl. In this case, the substituents that may be optionally substituted are as described above.

In Formula 1 of the present invention, when B is $-(CH_2)o-Cy_3-W-Cy_2$, then $Cy^3$ may be optionally substituted with 1 to 3 $R^{3a}$, and $Cy^4$ may be optionally substituted with 1 to 3 $R^{3b}$. $R^{3a}$ and $R^{3b}$ are as described in Formula I below.

Limitations on each structure and substituent of Formula 1 above may be equally applied to Formula I below, if applicable. Likewise, limitations on each structure and substituent of Formula I below may be equally applied to Formula 1 above, if applicable.

In one aspect of the present invention, there is provided a compound of Formula I below, a solvate, stereoisomer or pharmaceutically acceptable salt thereof:

[Formula I]

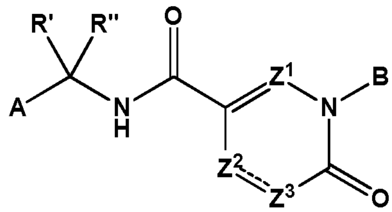

in Formula I,

------ is a single bond or a double bond;
$Z^1$ is N or CH;
when $Z^1$ is N, then $Z^2$ and $Z^3$ are both $CHR^1$ and ------ is a single bond, or $Z^2$ and $Z^3$ are both $CR^1$ and ------ is a double bond;
when $Z^1$ is CH, then $Z^2$ is N or $CR^1$, $Z^3$ is $CR^1$, and ------ is a double bond; or
when $Z^1$ is N, $Z^2$ and $Z^3$ are both $CR^1$, and ------ is a double bond, then two $R^1$ may be optionally linked to each other together with the carbon atom to which they are attached to form a thiophene or pyrrole ring;
each $R^1$ is independently selected from the group consisting of H, halogen, CN, OH, $NR^bR^c$, $C_1$-$C_6$ alkoxy, $C_1$-$C_6$ acylamino, $C_1$-$C_6$ alkylsulfonylamino, $C_1$-$C_6$ alkyl, $C_2$-$C_6$ alkenyl, $C_2$-$C_6$ alkynyl, $C_3$-$C_6$ cycloalkyl, $C_{6-}C_{10}$ aryl, $C_{6-}C_{10}$ aryloxy, ($C_{6-}C_{10}$ aryl)-($C_1$-$C_6$ alkyl)oxy and $C_{6-}C_{10}$ arylamino;
R' and R" are each independently H or $C_1$-$C_3$ alkyl, or R' and R" may be taken together with the carbon atom to which they are attached to form $C_3$-$C_4$ cycloalkyl, and said $C_1$-$C_3$ alkyl and $C_3$-$C_4$ cycloalkyl may be optionally substituted with at least one halogen, OH, CN, $C_1$-$C_3$ alkoxy or $NR^bR^c$;
A is $Cy_1$ or $Cy_1$-Y-$Cy_2$;
Y is O, S, or a direct bond;
$Cy_1$ is $C_6$-$C_{10}$ aryl or 5- or 6-membered heteroaryl containing 1 or 2 heteroatoms selected from N, O and S;
$Cy_1$ may be optionally substituted with 1 to 3 $R^{2a}$;
$R^{2a}$ is selected from the group consisting of H, halogen, OH, CN, oxo, $SF_5$, $NR^bR^c$, $-Si(C_{1-3}\ alkyl)_3$, $-SO_2R^b$, $-C(O)R^b$, $C_{1-}C_6$ alkyl, $C_{1-}C_6$ haloalkyl, $C_{1-}C_6$ alkoxy, $C_{1-}C_6$ haloalkoxy, $C_{3-}C_6$ cycloalkyl and

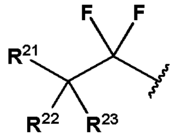

,

$R^{21}$ is H, halogen, OH, $NR^bR^c$, $C_{1-}C_6$ alkoxy or $C_{1-}C_6$ acyloxy, and $R^{22}$ and $R^{23}$ are each independently H, halogen or $C_{1-}C_2$ alkyl;
$Cy_2$ is $C_6$-$C_{10}$ aryl, phenyl fused with $C_3$-$C_6$ cycloalkyl, or 5- or 6-membered heteroaryl containing 1 or 2 heteroatoms

selected from N, O and S;

$Cy_2$ may be optionally substituted with 1 to 3 $R^{2b}$;

$R^{2b}$ is selected from the group consisting of H, halogen, OH, CN, oxo, $NR^bR^c$; $C_1$-$C_6$ alkyl; $C_1$-$C_6$ alkyl substituted with halogen, CN, OH, $NR^bR^c$ or $C_1$-$C_6$ alkoxy; $C_1$-$C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-;

B is H, $C_1$-$C_6$ alkyl, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkyl substituted with $NR^bR^c$, $-(CH_2)_o$-$Cy_3$ or $-(CH_2)_o$-$Cy_3$-W-$Cy_4$;

W is NH, C(O) or a direct bond;

o is an integer of 0 or 1;

$Cy_3$ is selected from the group consisting of $C_3$-$C_8$ cycloalkyl, $C_3$-$C_8$ cycloalkenyl, 5- or 6-membered saturated or partially unsaturated heterocycloalkyl containing 1 or 2 heteroatoms selected from N, O and S, bridged bicyclic $C_5$-$C_{10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S;

$Cy_3$ may be optionally substituted with 1 to 3 $R^{3a}$,

$R^{3a}$ is selected from the group consisting of H, halogen, OH, CN, oxo, $C_1$-$C_6$ alkyl; $C_1$-$C_6$ alkyl substituted with halogen, OH, CN or $C_1$-$C_6$ alkoxy; $C_3$-$C_6$ cycloalkyl, $C_1$-$C_6$ alkoxy, $C_1$-$C_6$ haloalkoxy, $C_1$-$C_6$ haloalkylamino, $C_1$-$C_6$ hydroxyalkylamino, ($C_3$-$C_6$ cycloalkyl)carbonylamino, - $NR^bR^c$, $-NR^bCOR^c$, $-NR^bC(O)OR^c$, $-SO_2R^b$, $-C(O)R^b$, $-C(O)OR^b$, $-NR^bSO_2R^c$ and $-CONR^{b1}R^{c1}$;

$Cy_4$ is selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S;

$Cy_4$ may be optionally substituted with 1 to 3 $R^{3b}$,

$R^{3b}$ is H, deuterium, halogen, OH, CN, oxo, $NR^bR^c$, $C_1$-$C_6$ alkyl, $C_1$-$C_6$ alkyl substituted with deuterium, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkoxy or $C_1$-$C_6$ haloalkoxy;

$R^b$ and $R^c$ are each independently H or $C_1$-$C_6$ alkyl; and

one of $R^{b1}$ and $R^{c1}$ is H or $C_1$-$C_6$ alkyl, and the other of $R^{b1}$ and $R^{c1}$ is H, $C_1$-$C_6$ alkyl, $C_1$-$C_6$ alkyl substituted with $NR^bR^c$, or $C_1$-$C_6$ alkyl substituted with $C_1$-$C_6$ alkoxy.

In Formula I above of the present invention, when $Z^1$ is N, then $Z^2$ and $Z^3$ may be both $CR^1$, and ------ may be a double bond. In addition, $Z^2$ and $Z^3$ may be both $CHR^1$, and ------ may be a single bond.

Alternatively, when $Z^1$ is N, $Z^2$ and $Z^3$ are both $CR^1$, and ------ is a double bond, then two $R^1$ may be optionally linked to each other together with the carbon atom to which they are attached to form a thiophene or pyrrole ring.

In Formula I above, when $Z^1$ is CH, then $Z^2$ may be N or $CR^1$, $Z^3$ may be $CR^1$, and ------ may be a double bond.

In Formula I above of the present invention,

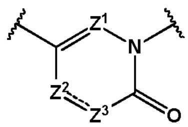

may be selected from the following structures:

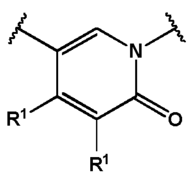
,
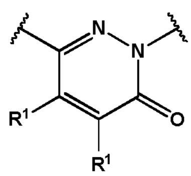
,
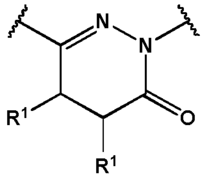
,
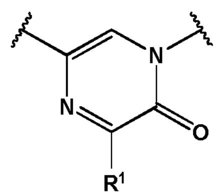
,
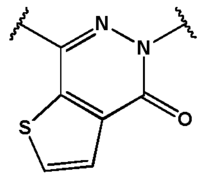
,

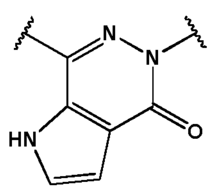
, and
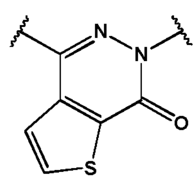
.

(In the above structures, two $R^1$ substituted on the same ring are the same or different from each other.)

In one embodiment, in Formula I,

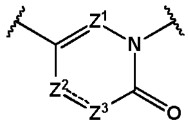

may be

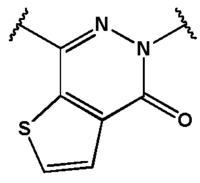

,

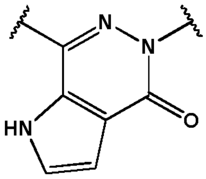

, or

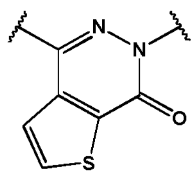

.

In one embodiment, in Formula I,

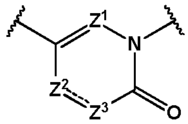

may be

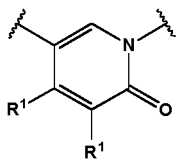

or

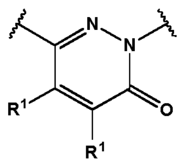

.

In Formula I above, each $R^1$ may be independently selected from the group consisting of H, halogen, CN, OH, $NR^bR^c$, $C_1$-$C_6$ alkoxy, $C_1$-$C_6$ acylamino, $C_1$-$C_6$ alkylsulfonylamino, $C_1$-$C_6$ alkyl, $C_2$-$C_6$ alkenyl, $C_2$-$C_6$ alkynyl, $C_3$-$C_6$ cycloalkyl, $C_6$-$C_{10}$ aryl, $C_6$-$C_{10}$ aryloxy, ($C_6$-$C_{10}$ aryl)-($C_1$-$C_6$ alkyl)oxy and $C_6$-$C_{10}$ arylamino. In this case, $R^b$ and $R^c$ are each independently H or $C_1$-$C_6$ alkyl. For example, each $R^1$ may be independently H, halogen, CN, OH or $C_1$-$C_6$ alkoxy. For example, $R^1$ may be an unsubstituted or substituted amino group such as $NR^bR^c$, $C_1$-$C_6$ acylamino, $C_1$-$C_6$ alkylsulfonylamino or $C_6$-$C_{10}$ arylamino. For example, $R^1$ may be a hydrocarbon group such as $C_1$-$C_6$ alkyl, $C_2$-$C_6$ alkenyl or $C_2$-$C_6$ alkynyl. For example, $R^1$ may be a ring substituent such as $C_3$-$C_6$ cycloalkyl, $C_6$-$C_{10}$ aryl, $C_6$-$C_{10}$ aryloxy or ($C_6$-$C_{10}$ aryl)-($C_1$-$C_6$ alkyl)oxy.

In one embodiment, when two $R^1$ substituted on the same ring are present, then one may be H, and the other may be not H. In another embodiment, two $R^1$ substituted on the same ring may be both H.

For example, $R^1$ may include H, F, Br, Cl, I, CN, OH, $OCH_3$, amino, methylamino, dimethylamino, ethylamino, acetylamino, methylsulfonylamino, ethylsulfonylamino, methyl, ethyl, ethenyl, ethynyl, cyclopropyl, cyclobutyl, cyclopentyl, phenyl, phenoxy, benzyloxy or phenylamino, but is not limited thereto.

In Formula I above of the present invention, R' and R" may be each independently H or $C_1$-$C_3$ alkyl, or R' and R" may be taken together with the carbon atom to which they are attached to form $C_3$-$C_4$ cycloalkyl. Optionally, said $C_1$-$C_3$ alkyl and $C_3$-$C_4$ cycloalkyl may be substituted with at least one halogen, OH, CN, $C_1$-$C_3$ alkoxy or $NR^bR^c$. In this case, $R^b$ and $R^c$ are each independently H or $C_1$-$C_6$ alkyl.

For example, R' and R" may be each independently H or $C_1$-$C_3$ alkyl. For example, R' and R" may be optionally taken together with the carbon atom to which they are attached to form a cyclopropane ring, wherein in Formula A,

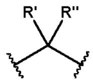

may be

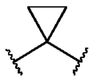

In one embodiment, R' and R" may be the same or different from each other. When R' and R" are different, then the carbon atom to which they are attached is a chiral center, and a compound of Formula I has stereoisomers, and any such stereoisomers are also included within the scope of the present invention.
For example, when any one of R' and R" is H,

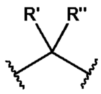

in Formula I has the steric structure of

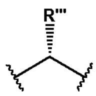
or
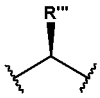

(R‴ is $C_1$-$C_3$ alkyl, such as methyl or ethyl).
In one embodiment, Formula I of the present invention may be a compound represented by Formula IA below, or a solvate, stereoisomer or pharmaceutically acceptable salt thereof:

[Formula IA]

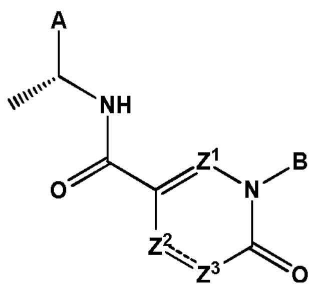

(in Formula IA, A, $Z^1$, $Z^2$, $Z^3$ and B are as defined in Formula I.)
In Formula I above of the present invention, A may be $Cy_1$. In this case, $Cy_1$ may be $C_6$-$C_{10}$ aryl, or 5- or 6-membered heteroaryl containing 1 or 2 heteroatoms selected from N, O and S.
In one embodiment, $Cy_1$ may be $C_6$-$C_{10}$ aryl, or 5- or 6-membered heteroaryl containing 1 or 2 heteroatoms selected from N and S. In one embodiment, $Cy_1$ may be phenyl, naphthalenyl, thiophenyl or pyridinyl.
For example, $Cy_1$ may have any one of the following ring structures optionally substituted with 1 to 3 $R^{2a}$:

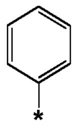 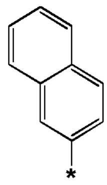 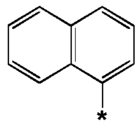 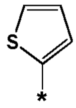 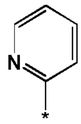 .

In Formula I above of the present invention, when A is $Cy_1$, then $Cy_1$ may be optionally substituted with 1 to 3 $R^{2a}$. For example, $Cy_1$ may be substituted with 1, 2 or 3 $R^{2a}$.

$R^{2a}$ may be selected from the group consisting of H, halogen, OH, CN, oxo, $SF_5$, $NR^bR^c$, $-Si(C_{1-3}\ alkyl)_3$, $-SO_2R^b$, $-C(O)R^b$, $C_1$-$C_6$ alkyl, $C_1$-$C_6$ haloalkyl, $C_1$-$C_6$ alkoxy, $C_1$-$C_6$ haloalkoxy, $C_3$-$C_6$ cycloalkyl and

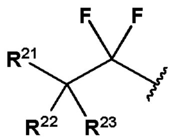 ,

wherein $R^{21}$ may be H, halogen, OH, $NR^bR^c$, $C_1$-$C_6$ alkoxy or $C_1$-$C_6$ acyloxy, and $R^{22}$ and $R^{23}$ may be each independently H, halogen or $C_1$-$C_2$ alkyl. In this case, $R^b$ and $R^c$ may be each independently H or $C_1$-$C_6$ alkyl.

In one embodiment, each $R^{2a}$ may be independently selected from the group consisting of H, F, Cl, Br, I, OH, CN, $SF_5$, $-Si(CH_3)_3$, $CH_3SO_2$-, methyl, ethyl, propyl, isopropyl, $CF_3$, $CHF_2$, $CH_2F$, $NH_2$, $CH_3NH_2$-, $(CH_3)_2N$-, methoxy, ethoxy, $OCF_3$, $OCHF_2$, $OCH_2F$, cyclopropyl, cyclobutyl, cyclopentyl,

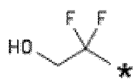 , 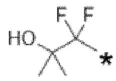 , 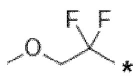 , 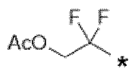 , 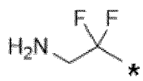 , 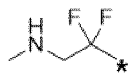

and

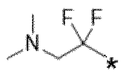 ,

but is not limited thereto.

When A is $Cy_1$, in Formula I of the present invention, A may be selected from the following structures:

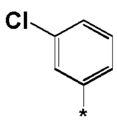 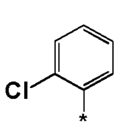 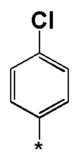 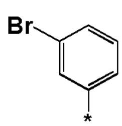 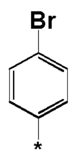 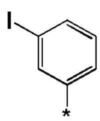 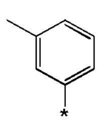 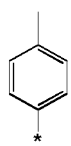 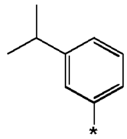

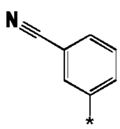 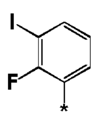 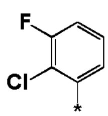 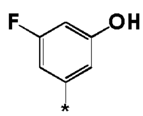 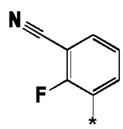 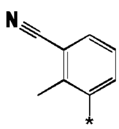 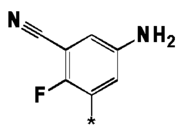 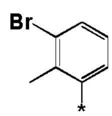

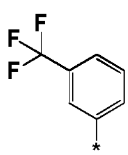 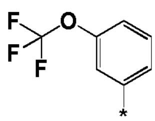 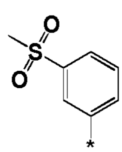 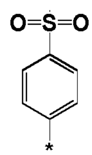 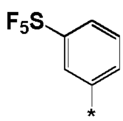 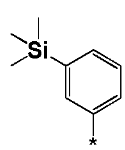

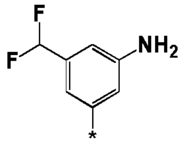 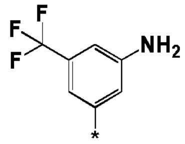 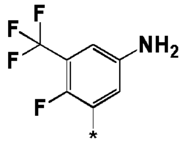 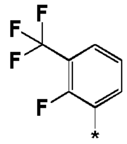 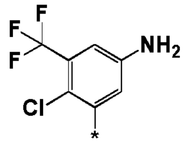 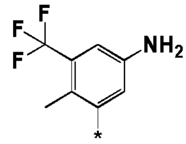

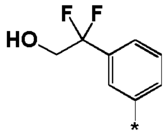 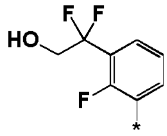 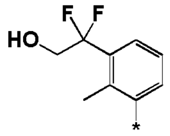 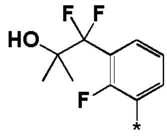 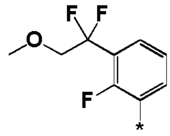 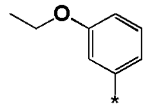

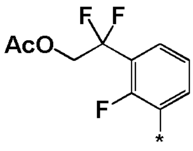 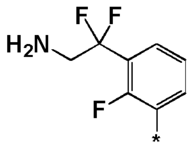 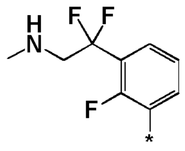 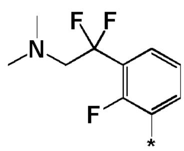 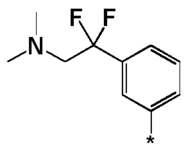 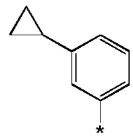

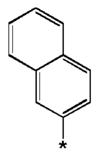 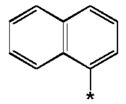 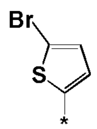 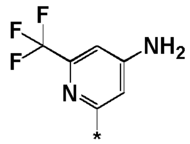

For example, in Formula I, A may be selected from the following structures:

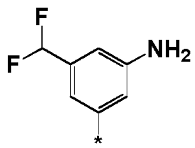 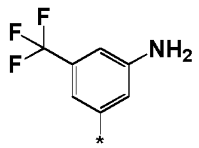 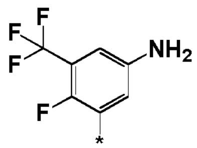 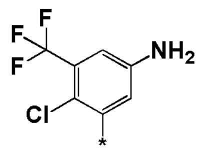 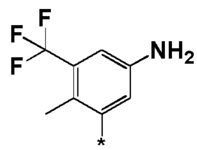

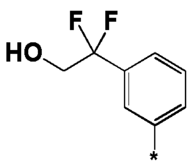 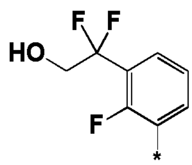 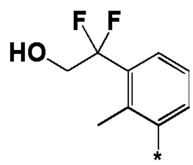 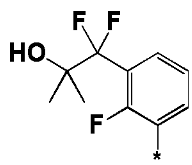 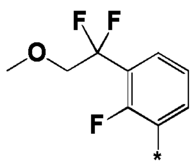 .

For example, in Formula I, A may be

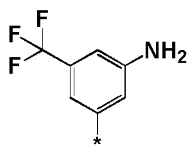 or 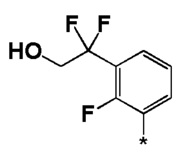 .

In Formula I above of the present invention, A may be $Cy_1$-Y-$Cy_2$. In this case, Y may be O, S, or a direct bond. For example, Y may be O or a direct bond. For example, Y may be a direct bond.

In Formula I, when A is $Cy_1$-Y-$Cy_2$, then $Cy_1$ may be $C_6$-$C_{10}$ aryl, or 5- to 6-membered heteroaryl containing 1 to 3 heteroatoms selected from N, O or S. In one embodiment, $Cy_1$ may be $C_6$-$C_{10}$ aryl, or 5- or 6-membered heteroaryl containing 1 or 2 heteroatoms selected from N, O and S. In one embodiment, it may be $C_6$-$C_{10}$ aryl, or 5- or 6-membered heteroaryl containing 1 or 2 heteroatoms selected from N and S. For example, $Cy_1$ may include phenyl, naphthalenyl, thiazolyl, thiophenyl or pyrazolyl.

In Formula I, when A is $Cy_1$-Y-$Cy_2$, then $Cy_2$ may be $C_6$-$C_{10}$ aryl fused with $C_3$-$C_6$ cycloalkyl, $C_6$-$C_{10}$ aryl, or 5- to 6-membered heteroaryl containing 1 to 3 heteroatoms selected from N, O or S. In one embodiment, $Cy_2$ may be $C_6$-$C_{10}$ aryl, phenyl fused with $C_3$-$C_6$ cycloalkyl, or 5- or 6-membered heteroaryl containing 1 or 2 heteroatoms selected

from N, O and S. In another embodiment, $Cy_2$ may be $C_6$-$C_{10}$ aryl, phenyl fused with $C_3$-$C_5$ cycloalkyl, or 5- or 6-membered heteroaryl containing 1 or 2 heteroatoms selected from N and S. For example, $Cy_2$ may include phenyl, 2,3-dihydroindenyl or bicyclo[4.2.0]octa-1,3,5-trienyl, pyrazolyl, thiophenyl, pyridinyl, 2-oxo-1,2-dihydropyridinyl or pyrrolyl.

In one embodiment, $Cy_1$ may be $C_6$-$C_{10}$ aryl, or 5- or 6-membered heteroaryl containing 1 or 2 heteroatoms selected from N and S; Y may be O or a direct bond; and $Cy_2$ may be $C_6$-$C_{10}$ aryl, phenyl fused with $C_3$-$C_5$ cycloalkyl, or 5- or 6-membered heteroaryl containing 1 or 2 heteroatoms selected from N and S.

For example, in Formula I, when A is $Cy_1$-Y-$Cy_2$, then $Cy_1$ may be phenyl, and $Cy_2$ may be phenyl, pyrrolyl, pyrazolyl, thiophenyl, pyridinyl, or 2-oxo-1,2-dihydropyridinyl. In this case, Y may be O or a direct bond. In one embodiment, Y may be a direct bond. In one embodiment, $Cy_1$ may be phenyl, Y may be O, and $Cy_2$ may be phenyl or pyridinyl.

In another embodiment, $Cy_1$ may be thiazolyl, thiophenyl or pyrazolyl, and $Cy_2$ may be phenyl, 2,3-dihydroindenyl or bicyclo[4.2.0]octa-1,3,5-trienyl. For example, $Cy_1$ may be thiophenyl, and $Cy_2$ may be phenyl.

In one embodiment, $Cy_1$-Y-$Cy_2$ may have any one of the following ring structures optionally substituted with $R^{2a}$ and $R^{2b}$:

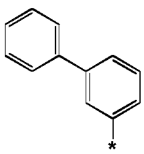
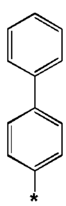
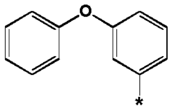
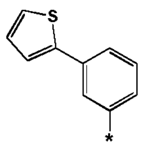
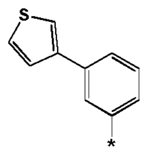
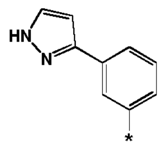
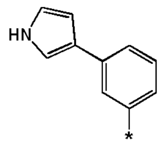

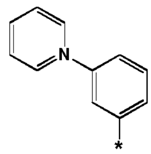
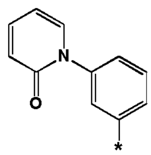
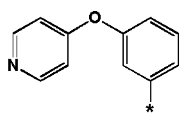
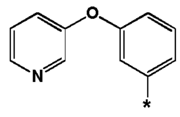
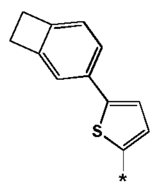
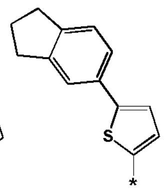

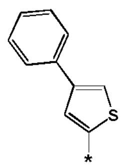
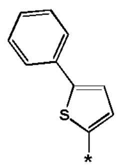
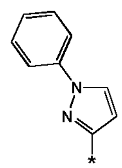
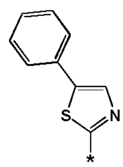
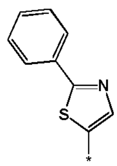
.

In Formula I, when A is $Cy_1$-Y-$Cy_2$, then said $Cy_1$ and $Cy_2$ may be each optionally substituted with 1 to 3 $R^2$. In this case, each $R^2$ may be independently selected from the group consisting of H, halogen, OH, CN, oxo, $SF_5$, -Si($C_1$-$C_3$ alkyl)$_3$, $C_1$-$C_6$ alkylsulfonyl, $C_1$-$C_6$ alkylcarbonyl, amino, $C_1$-$C_6$ alkylamino, di($C_1$-$C_6$ alkyl)amino; $C_1$-$C_6$ alkyl optionally substituted with halogen, CN, OH, $C_1$-$C_6$ alkoxy, amino, $C_1$-$C_6$ alkylamino, di($C_1$-$C_6$ alkyl)amino or hydroxy-($C_1$-$C_6$ alkyl)amino-; $C_1$-$C_6$ alkoxy, $C_1$-$C_6$ haloalkoxy, $C_3$-$C_6$ cycloalkyl and

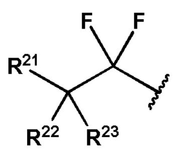
.

In this case, $R^{21}$ may be H, halogen, OH, $C_1$-$C_6$ alkoxy, $C_1$-$C_6$ acyloxy, amino, $C_1$-$C_6$ alkylamino or di($C_1$-$C_6$ alkyl)amino, and $R^{22}$ and $R^{23}$ may be each independently H, halogen or $C_1$-$C_2$ alkyl.

In Formula I, when A is $Cy_1$-Y-$Cy_2$, then said $Cy_1$ may be optionally substituted with 1 to 3 $R^{2a}$. In this case, $R^{2a}$ may be selected from the group consisting of H, halogen, OH, CN, oxo, $SF_5$, $NR^bR^c$, -Si($C_{1-3}$ alkyl)$_3$, -$SO_2R^b$, -C(O)$R^b$, $C_1$-$C_6$ alkyl, $C_1$-$C_6$ haloalkyl, $C_1$-$C_6$ alkoxy, $C_1$-$C_6$ haloalkoxy, $C_3$-$C_6$ cycloalkyl and

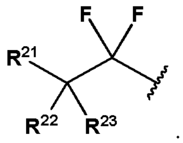

.

Said $R^{21}$ may be H, halogen, OH, $NR^bR^c$, $C_1$-$C_6$ alkoxy or $C_1$-$C_6$ acyloxy, and $R^{22}$ and $R^{23}$ may be each independently H, halogen or $C_1$-$C_2$ alkyl. In one embodiment, $Cy_1$ may be optionally substituted with one $R^{2a}$, and $R^{2a}$ may be H, halogen, OH, CN, amino, $C_1$-$C_6$ alkyl, $C_1$-$C_6$ haloalkyl, $C_1$-$C_6$ alkoxy or $C_1$-$C_6$ haloalkoxy. For example, $R^{2a}$ may include H, halogen, OH or CN. For example, $R^{2a}$ may be H or halogen. For example, $R^{2a}$ may be H.

In Formula I, when A is $Cy_1$-Y-$Cy_2$, then said $Cy_2$ may be optionally substituted with 1 to 3 $R^{2b}$. For example, $Cy_2$ may be optionally substituted with 1 to 3 $R^{2b}$.

In this case, $R^{2b}$ may be selected from the group consisting of H, halogen, OH, CN, oxo, $NR^bR^c$; $C_1$-$C_6$ alkyl; $C_1$-$C_6$ alkyl substituted with halogen, CN, OH, $NR^bR^c$ or $C_1$-$C_6$ alkoxy; $C_1$-$C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-.

For example, each $R^{2b}$ may be independently H, F, Cl, Br, I, OH, CN, oxo, amino, $CH_3NH$-, $(CH_3)_2N$-, $(CH_3)_2NCH_2$- methyl, ethyl, cyanomethyl, hydroxymethyl, aminomethyl, $CH_3NHCH_2$-, $C_2H_5NHCH_2$- or $HOC_2H_4NHCH_2$-, but is not limited thereto. For example, $R^{2b}$ may be H, halogen, $C_1$-$C_6$ alkyl; or $C_1$-$C_6$ alkyl substituted with amino, $C_1$-$C_6$ alkylamino or di($C_1$-$C_6$ alkyl)amino.

When A is $Cy_1$-Y-$Cy_2$, in Formula I of the present invention, A may be selected from the following structures:

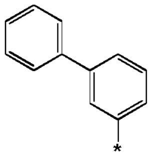 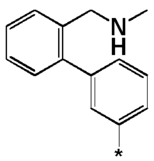 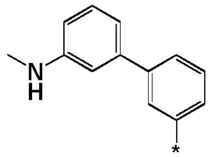 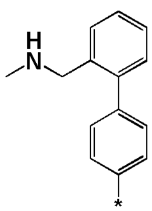 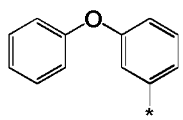 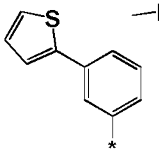 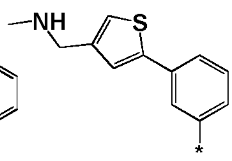

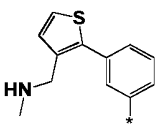 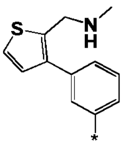 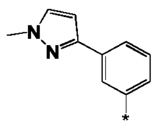 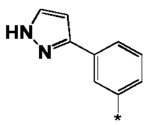 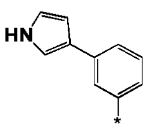 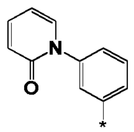 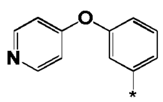 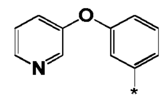

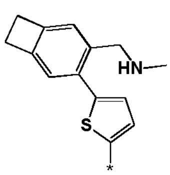 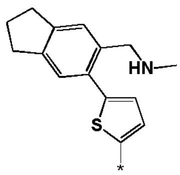 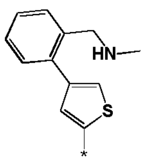 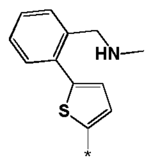 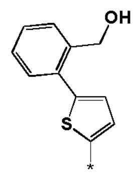 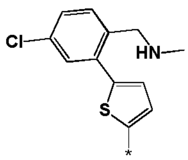 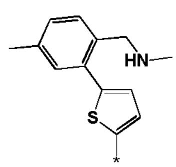

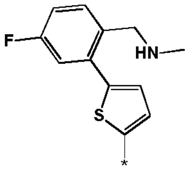 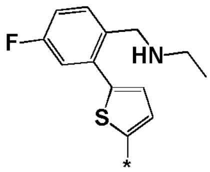 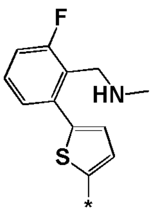 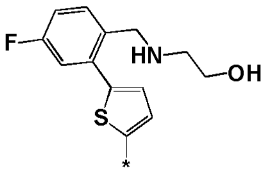 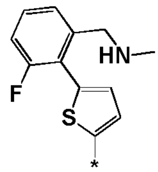 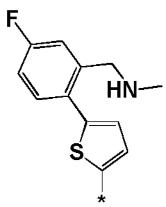

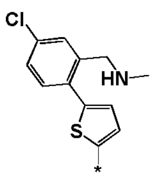 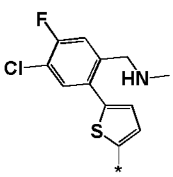 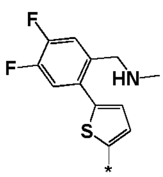 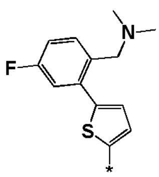 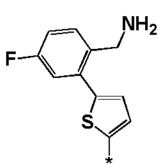 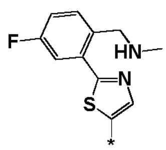 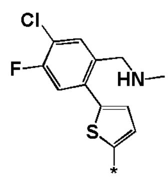

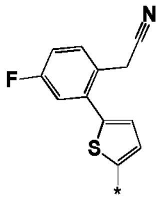 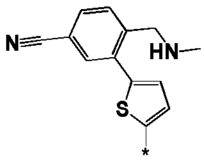 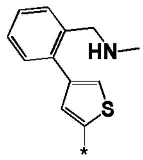 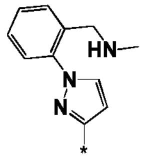 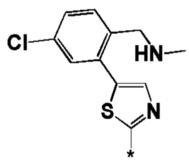 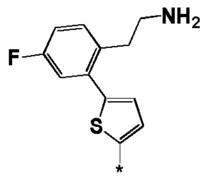

For example, in Formula I, A may be selected from the following structures:

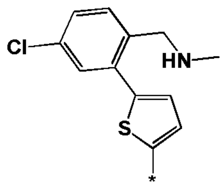 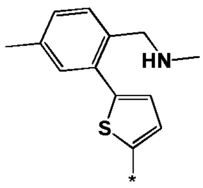 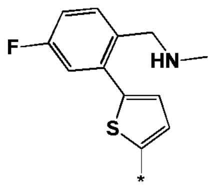 .

In Formula I above of the present invention, B may be H, $C_1$-$C_6$ alkyl, $C_1$-$C_6$ haloalkyl, $C_1$-$C_6$ hydroxyalkyl, $C_1$-$C_6$ alkoxy-$C_1$-$C_6$ alkyl, or $C_1$-$C_6$ alkyl substituted with $NR^bR^c$. In this case, $R^b$ and $R^c$ are each H or $C_1$-$C_6$ alkyl. For example, B may be H, $CH_3$,

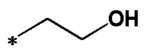 , 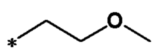 , 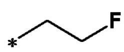 , or 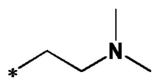 .

In Formula I above of the present invention, B may be $-(CH_2)_o-Cy_3$. In this case, o may be 0 or 1.

In Formula I above of the present invention, when B is $-(CH_2)_o-Cy_3$, then $Cy_3$ may be selected from the group consisting of $C_3$-$C_8$ cycloalkyl, $C_3$-$C_8$ cycloalkenyl, 5- or 6-membered saturated or partially unsaturated heterocycloalkyl containing 1 or 2 heteroatoms selected from N, O and S, bridged bicyclic $C_{5-10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S.

In one embodiment, $Cy_3$ may be selected from the group consisting of $C_3$-$C_8$ cycloalkyl, $C_3$-$C_8$ cycloalkenyl, 6-membered saturated or partially unsaturated heterocycloalkyl containing one N, O or S, bridged bicyclic $C_{5-8}$ cycloalkyl, $C_6$-$C_{10}$ aryl, phenyl fused with 5-membered heterocycloalkyl containing one N, O or S, 5- or 6-membered heteroaryl containing 1 or 2 heteroatoms selected from N or S, and 9- or 10-membered bicyclic heteroaryl containing 1 to 3 N.

For example, $Cy_3$ may be $C_3$-$C_6$ cycloalkyl, $C_3$-$C_6$ cycloalkenyl, tetrahydropyranyl, dihydropyranyl, thianyl, 1,1-dioxothianyl, piperidinyl, dihydropyridinyl, tetrahydropyridinyl, bicyclo[1.1.1]pentanyl, bicyclo[2.2.1]heptanyl, $C_{6-10}$ aryl, thiophenyl, thiazolyl, pyrazolyl, pyridinyl, pyrimidinyl, dihydroisobenzofuranyl, indolyl, indazolyl or benzotriazolyl, but is not limited thereto.

Said $Cy_3$ may include any one of the following ring structures, which may be optionally substituted with $R^{3a}$:

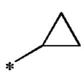 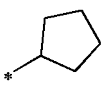 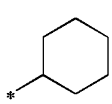 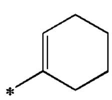 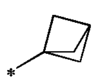 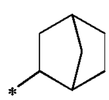 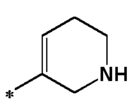 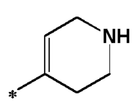

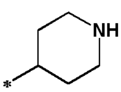 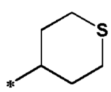 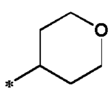 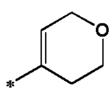 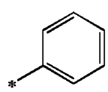 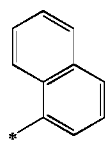 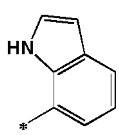 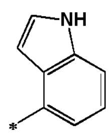

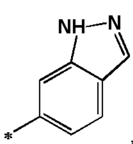 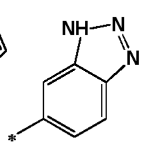 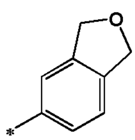 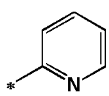 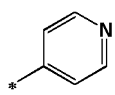 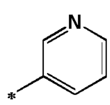 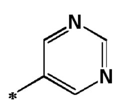 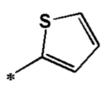

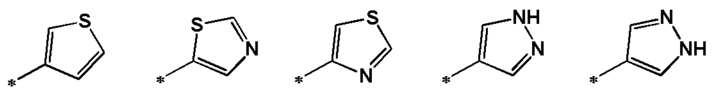

Said $Cy_3$ may be optionally substituted with 1 to 3 $R^{3a}$, wherein $R^{3a}$ may be selected from the group consisting of H, halogen, OH, CN, oxo, $C_{1-}C_6$ alkyl; $C_{1-}C_6$ alkyl substituted with halogen, OH, CN or $C_{1-}C_6$ alkoxy; $C_3$-$C_6$ cycloalkyl, $C_{1-}C_6$ alkoxy, $C_{1-}C_6$ haloalkoxy, $C_{1-}C_6$ haloalkylamino, $C_{1-}C_6$ hydroxyalkylamino, ($C_3$-$C_6$ cycloalkyl)carbonylamino, $-NR^bR^c$, $-NR^bCOR^c$, $-NR^bC(O)OR^c$, $-SO_2R^b$, $-C(O)R^b$, $-C(O)OR^b$, $-NR^bSO_2R^c$ and $-CONR^{b1}R^{c1}$. In this case, $R^b$ and $R^c$ may be each independently H or $C_1$-$C_6$ alkyl. In addition, one of $R^{b1}$ and $R^{c1}$ may be H or $C_{1-}C_6$ alkyl, and the other of $R^{b1}$ and $R^{c1}$ may be H, $C_{1-}C_6$ alkyl, $C_{1-}C_6$ alkyl substituted with $NR^bR^c$, or $C_{1-}C_6$ alkyl substituted with $C_{1-}C_6$ alkoxy.

For example, $R^{3a}$ may include H, F, Cl, Br, I, OH, CN, oxo, methyl, ethyl, amino, $CH_3NH$-, $(CH_3)_2NH$-, 1,1,1-trifluoropropan-2-ylamino, $CH_3CONH$-, $(CH_3CO)(CH_3)N$-, $CH_3OCONH$-, cyclopropylcarbonylamino, hydroxymethyl, 1-hydroxyethyl, 2-hydroxypropan-2-yl, methoxy, ethoxy, isopropoxy, methoxymethyl, 2-methoxyethyl, $OCHF_2$, $OCF_3$, $CH_3SO_2$-, $CH_3CO$-, $CH_3SO_2NH$-, -COOH, $-COOC(CH_3)_3$, $-CONH_2$, $-CONHCH_3$, $-CONHC_2H_5$, $-CON(CH_3)_2$, $-CONHC_2H_4OCH_3$ or $-CONHC_2H_4N(CH_3)_2$, but is not limited thereto.

When B is $-(CH_2)_o$-$Cy_3$, in Formula I of the present invention, B may be selected from the following structures:

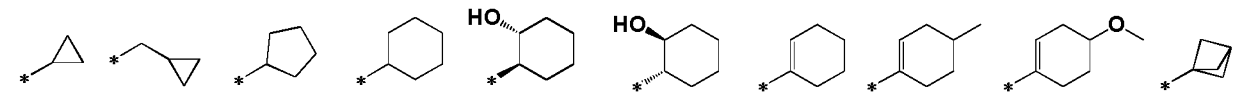

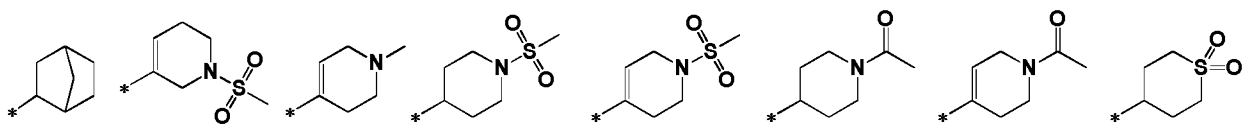

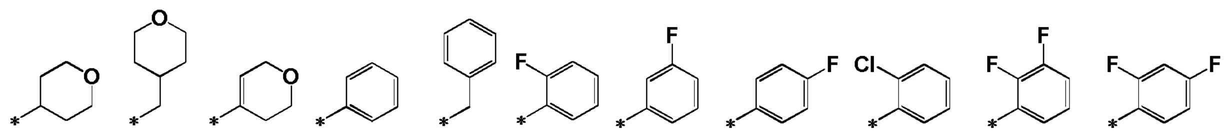

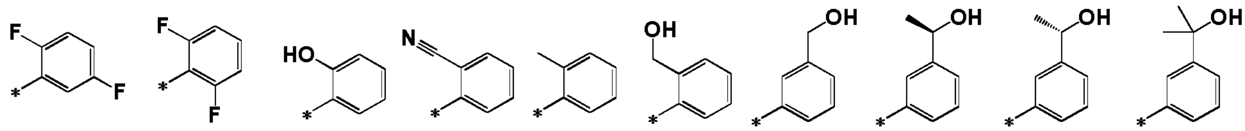

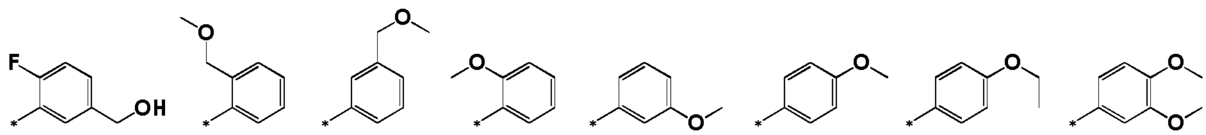

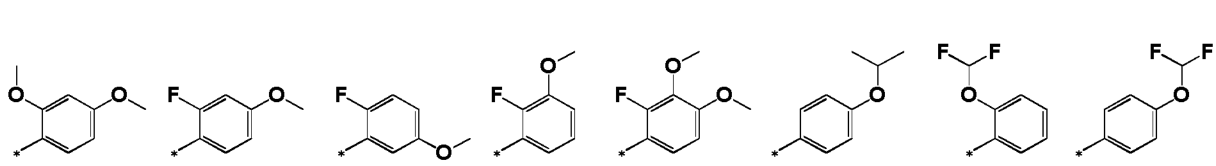

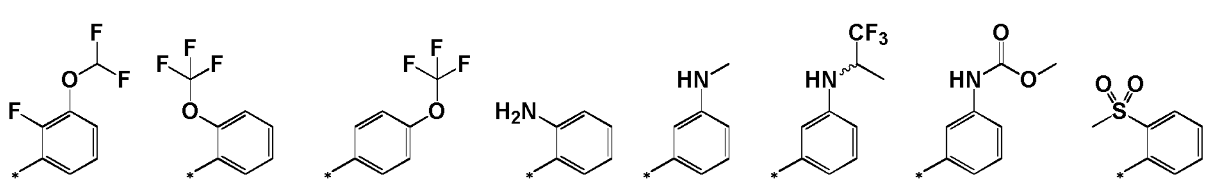

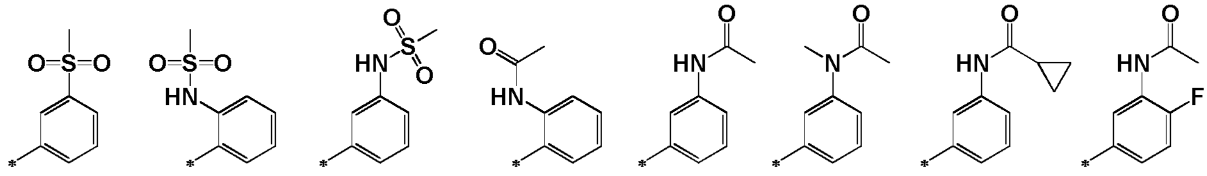

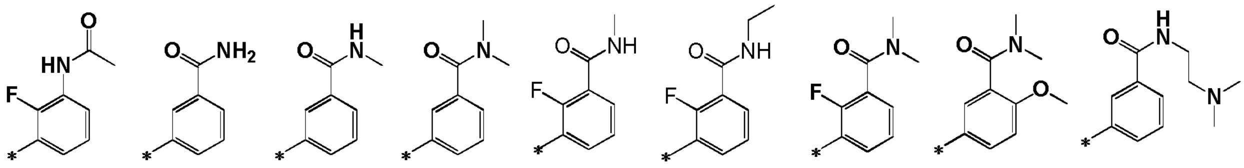

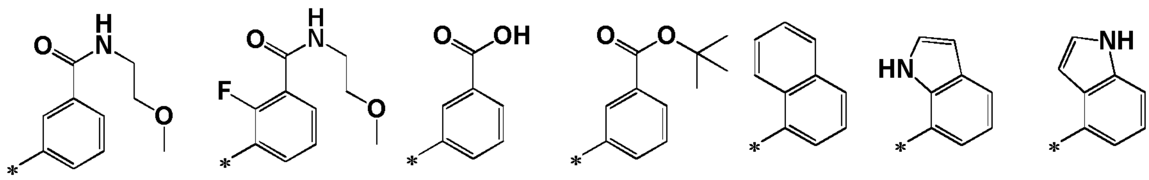

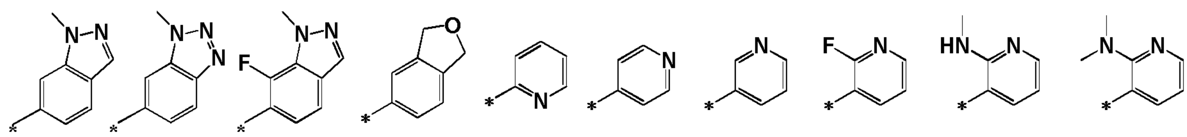

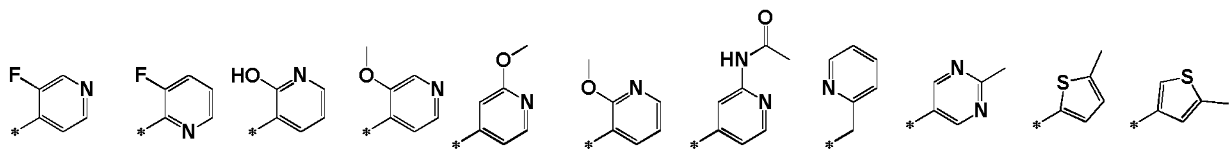

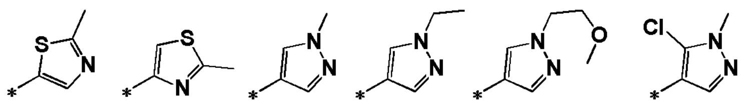

For example, B may be selected from

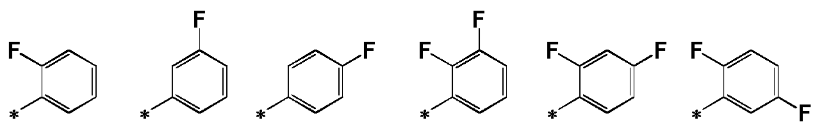,

but is not limited thereto. For example, B may be

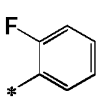 or 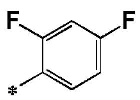,

but is not limited thereto.

In Formula I above of the present invention, B may be $-(CH_2)_o-Cy_3-W-Cy_4$. In this case, o may be 0 or 1. For example, o may be 0. In addition, W may be NH, C(O) or a direct bond.

When B is $-(CH_2)_o-Cy_3-W-Cy_4$, then $Cy_3$ may be selected from the group consisting of $C_3$-$C_8$ cycloalkyl, $C_3$-$C_8$ cycloalkenyl, 5- or 6-membered saturated or partially unsaturated heterocycloalkyl containing 1 or 2 heteroatoms selected

from N, O and S, bridged bicyclic $C_{5-10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S.

In one embodiment, $Cy_3$ may be $C_6$-$C_{10}$ aryl, or 5- or 6-membered heteroaryl containing 1 or 2 heteroatoms selected from N or S.

When B is $-(CH_2)_o$-$Cy_3$-W-$Cy_4$, then $Cy_4$ may be selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S.

In one embodiment, $Cy_4$ may be selected from the group consisting of saturated or partially unsaturated 4- to 7-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S.

In one embodiment, B may be $-(CH_2)_o$-$Cy_3$-W-$Cy_4$, and $Cy_3$ may be phenyl or pyridinyl. In addition, $Cy_4$ may be oxetanyl, tetrahydrofuranyl, pyrrolidinyl, 2-oxo-pyrrolidinyl, piperidinyl, morpholinyl, imidazolidinyl, 2-oxo-imidazolidinyl, piperazinyl, 2-oxo-piperazinyl, hexahydropyrimidinyl, 2-oxo-hexahydropyrimidinyl, phenyl, oxazolyl, isoxazolyl, thiazolyl, pyrazolyl, imidazolyl, oxadiazolyl, triazolyl, tetrazolyl, pyridinyl or 2-oxo-pyridinyl. For example, $Cy_3$ may be phenyl, and $Cy_4$ may be pyrazolyl, imidazolyl, triazolyl or tetrazolyl. For example, $Cy_3$ may be phenyl, and $Cy_4$ may be triazolyl. For example, $Cy_3$ may be pyridinyl, and $Cy_4$ may be triazolyl.

In one embodiment, W may be NH, C(O) or a direct bond. For example, W may be a direct bond.

In one embodiment, $Cy_3$ may be $C_6$-$C_{10}$ aryl, $Cy_4$ may be saturated or partially unsaturated 4- to 7-membered heterocycloalkyl containing 1 or 2 heteroatoms selected from N, O or S, and W may be NH or C(O).

Said $Cy_3$-W-$Cy_4$ may include any one of the following ring structures, and the rings corresponding to $Cy_3$ and $Cy_4$ may be optionally substituted with $R^{3a}$ and $R^{3b}$, respectively:

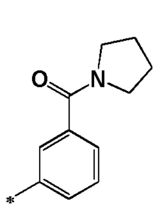 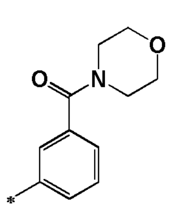 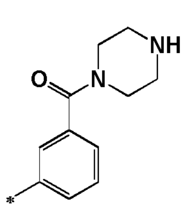 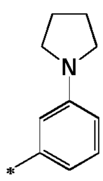 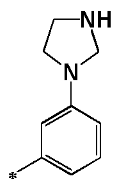 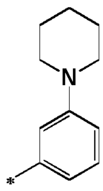 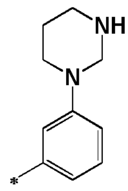 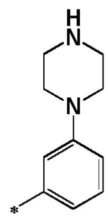

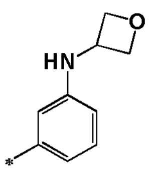 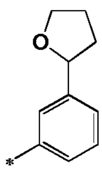 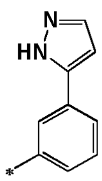 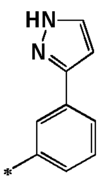 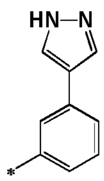 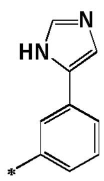 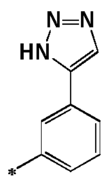 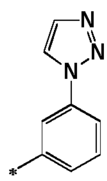 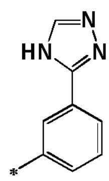

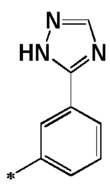 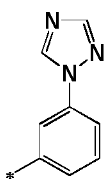 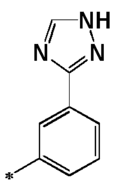 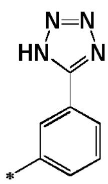 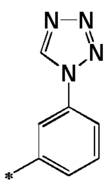 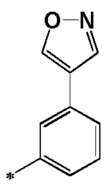 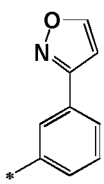 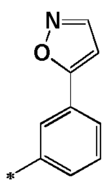 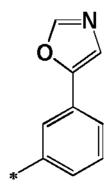

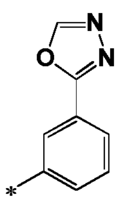 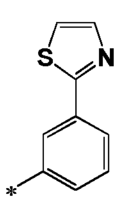 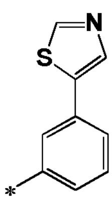 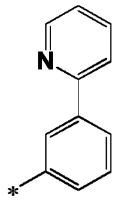 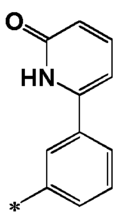 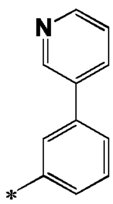 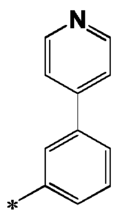 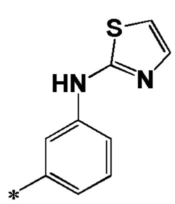

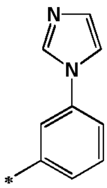 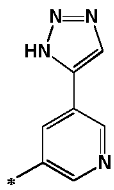 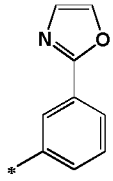 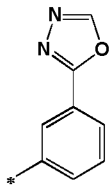 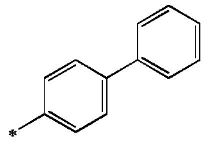 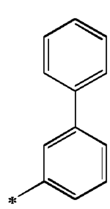 .

In one embodiment, said $Cy_3$ and $Cy_4$ may be each independently optionally substituted with 1 to 3 $R^3$.

When B is $-(CH_2)_o-Cy_3-W-Cy_4$, then said $R^3$, which is each independently substituted on $Cy_3$ and $Cy_4$, may be H, deuterium, halogen, OH, CN, oxo, $C_1$-$C_6$ alkyl, $C_1$-$C_6$ alkyl substituted with deuterium, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkoxy, $C_1$-$C_6$ haloalkoxy, $C_1$-$C_6$ haloalkylamino, ($C_3$-$C_6$ cycloalkyl)carbonylamino, $-NR^bR^c$, $-NR^bCOR^c$, $-NR^bC(O)OR^c$, $-SO_2R^b$, $-C(O)R^b$, $-C(O)OR^b$, $-NR^bSO_2R^c$ or $-CONR^{b1}R^{c1}$. In this case, $R^b$ and $R^c$ may be each independently H or $C_1$-$C_6$ alkyl. In addition, one of $R^{b1}$ and $R^{c1}$ may be H or $C_1$-$C_6$ alkyl, and the other may be H, $C_1$-$C_6$ alkyl; or $C_1$-$C_6$ alkyl substituted with amino, $C_1$-$C_6$ alkylamino, di($C_1$-$C_6$ alkyl)amino or $C_1$-$C_6$ alkoxy.

In one embodiment, $Cy_3$ may be optionally substituted with 1 to 3 $R^{3a}$. $R^{3a}$ may be selected from the group consisting of H, halogen, OH, CN, oxo, $C_1$-$C_6$ alkyl; $C_1$-$C_6$ alkyl substituted with halogen, OH, CN or $C_1$-$C_6$ alkoxy; $C_3$-$C_6$ cycloalkyl, $C_1$-$C_6$ alkoxy, $C_1$-$C_6$ haloalkoxy, $C_1$-$C_6$ haloalkylamino, $C_1$-$C_6$ hydroxyalkylamino, ($C_3$-$C_6$ cycloalkyl)carbonylamino, $-NR^bR^c$, $-NR^bCOR^c$, $-NR^bC(O)OR^c$, $-SO_2R^b$, $-C(O)R^b$, $-C(O)OR^b$, $-NR^bSO_2R^c$ and $-CONR^{b1}R^{c1}$. In this case, $R^b$ and $R^c$ may be each independently H or $C_1$-$C_6$ alkyl; and one of $R^{b1}$ and $R^{c1}$ may be H or $C_1$-$C_6$ alkyl, and the other of $R^{b1}$ and $R^{c1}$ may be H, $C_1$-$C_6$ alkyl, $C_1$-$C_6$ alkyl substituted with $NR^bR^c$, or $C_1$-$C_6$ alkyl substituted with $C_1$-$C_6$ alkoxy.

In one embodiment, said $Cy_3$ may be optionally substituted with one or two $R^{3a}$. In this case, $R^{3a}$ may be H, halogen, OH, CN, oxo, amino, $C_1$-$C_6$ alkyl, $C_1$-$C_6$ haloalkyl or $C_1$-$C_6$ haloalkoxy. For example, $R^{3a}$ may include H, halogen, OH or CN, but is not limited thereto. For example, $R^{3a}$ may be H or F, but is not limited thereto. For example, $R^{3a}$ may be H.

Said $Cy_4$ may be optionally substituted with 1 to 3 $R^{3b}$. In this case, $R^{3b}$ may be H, deuterium, halogen, OH, CN, oxo, $NR^bR^c$, $C_1$-$C_6$ alkyl, $C_1$-$C_6$ alkyl substituted with deuterium, $C_1$-$C_6$ haloalkyl, $C_1$-$C_6$ hydroxyalkyl, $C_1$-$C_6$ alkoxy-$C_1$-$C_6$ alkyl, $C_1$-$C_6$ alkoxy or $C_1$-$C_6$ haloalkoxy. In this case, $R^b$ and $R^c$ are H or $C_1$-$C_6$ alkyl. For example, $R^{3b}$ may include H, deuterium, halogen, OH, CN, oxo, $C_1$-$C_6$ alkyl, $C_1$-$C_6$ alkyl substituted with deuterium or $C_1$-$C_6$ haloalkyl, but is not limited thereto. For example, $R^{3b}$ may be H or $C_1$-$C_6$ alkyl. For example, $R^{3b}$ may include H, F, oxo, methyl, ethyl, $CHF_2$ and $CD_3$, but is not limited thereto. For example, $R^{3b}$ may be H or methyl.

In one embodiment, $Cy_3$ may be optionally substituted with one or two $R^{3a}$, wherein $R^{3a}$ may be H, halogen, OH or CN; and $Cy_4$ may be optionally substituted with 1 to 3 $R^{3b}$, wherein $R^{3b}$ may be H, deuterium, halogen, OH, CN, oxo, $C_1$-$C_6$ alkyl, $C_1$-$C_6$ alkyl substituted with deuterium or $C_1$-$C_6$ haloalkyl.

When B is $-(CH_2)_o-Cy_3-W-Cy_4$, then in Formula I, B may be selected from the following structures:

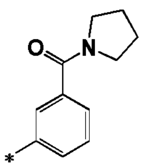 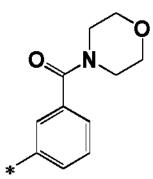 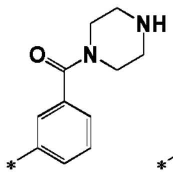 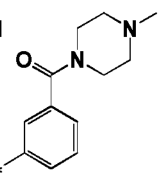 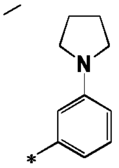 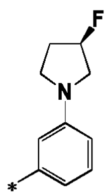 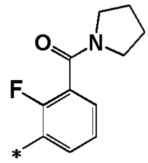 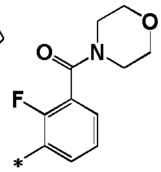

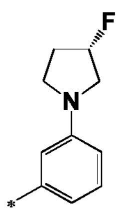 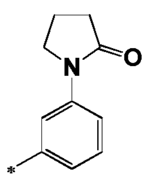 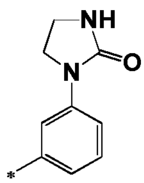 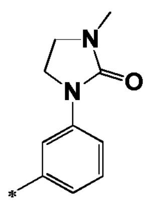 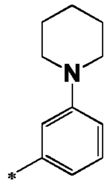 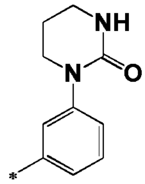 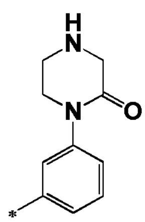 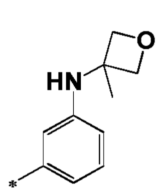

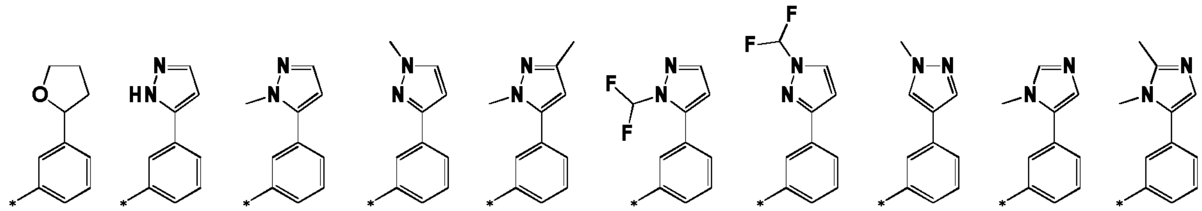

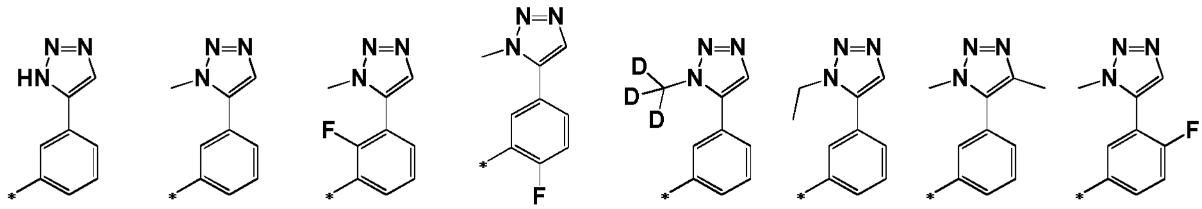

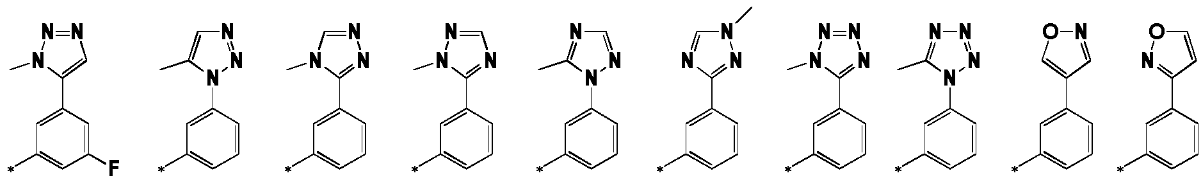

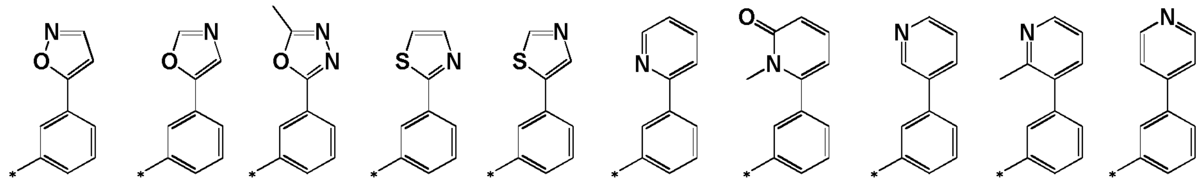

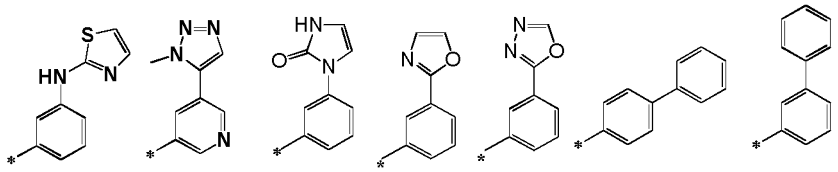

For example, B may be selected from the following structures:

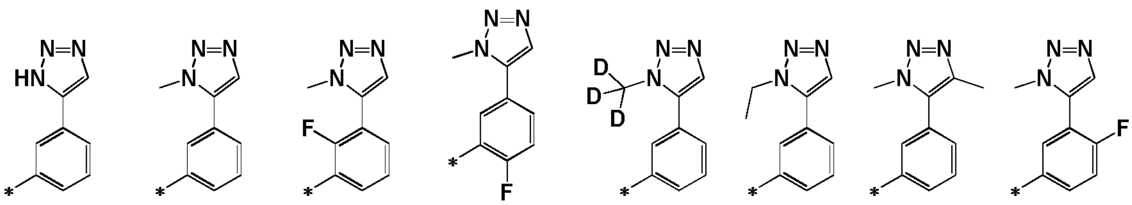

For example, B may be

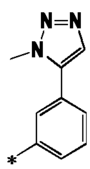

,

but is not limited thereto.

In one embodiment, Formula I of the present invention may be represented by any one of Formulas I-1, I-2, I-3, I-4, I-5, I-6, and I-7:

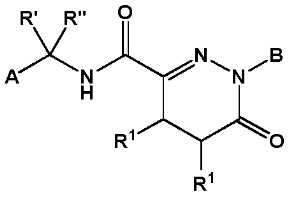

Formula I-1

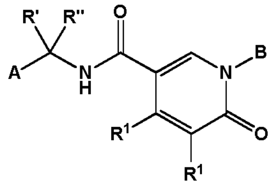

Formula I-2

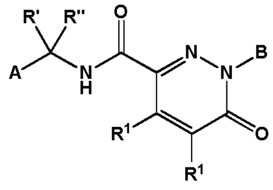

Formula I-3

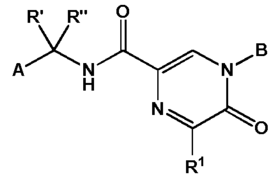

Formula I-4

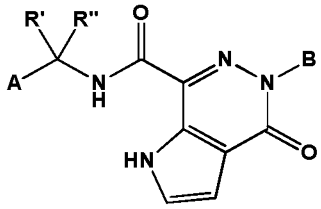

Formula I-5

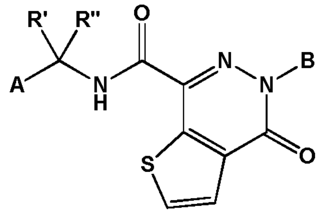

Formula I-6

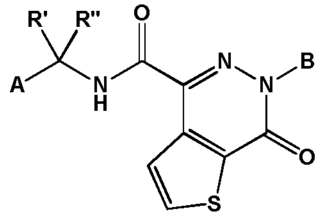

Formula I-7

(In Formulas I-1, I-2, I-3, I-4, I-5, I-6, and I-7, A, R', R", $R^1$ and B are as defined in Formula I, and each $R^1$ may be the same or different from each other.)

In some embodiments, the compound of Formula I may be a compound selected from the group consisting of the following compounds:

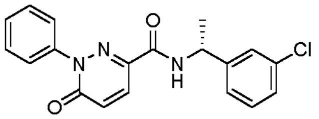 ,

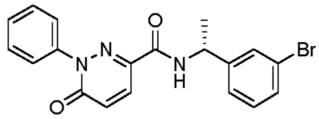 ,

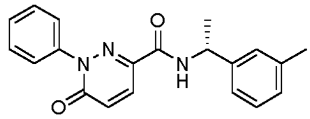 ,

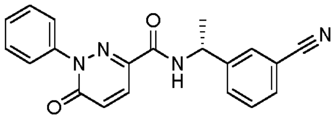 ,

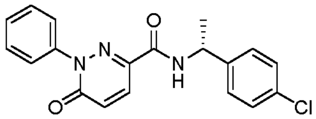 ,

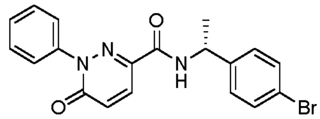 ,

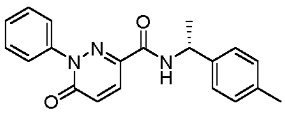 ,

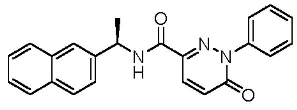 ,

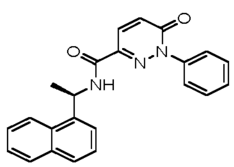 ,

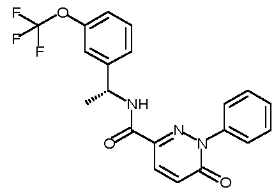 ,

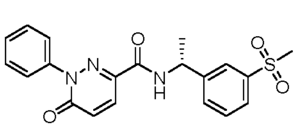 ,

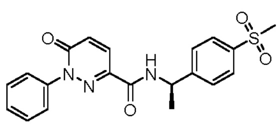 ,

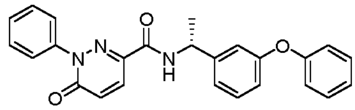 ,

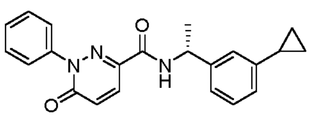 ,

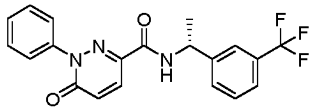 ,

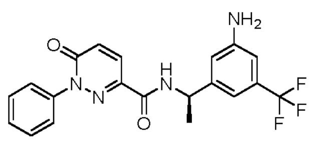 ,

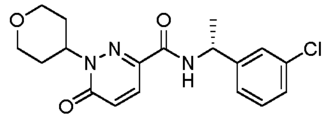 , 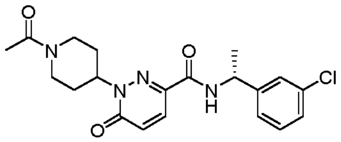 , 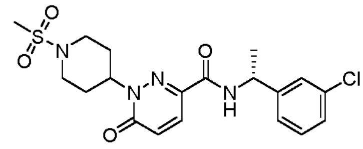 ,

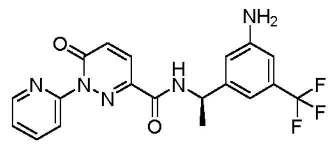 , 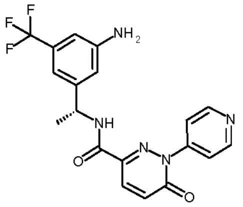 , 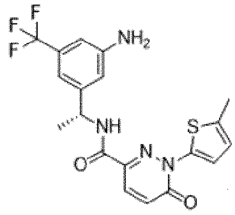 , 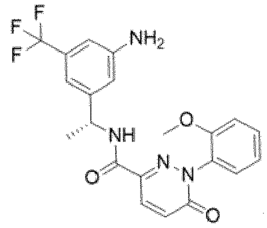 ,

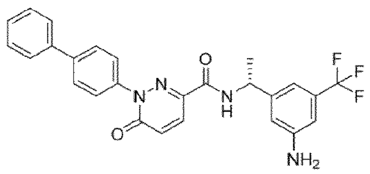 , 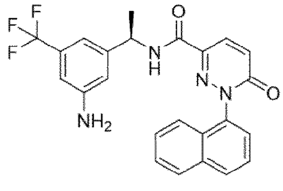 , 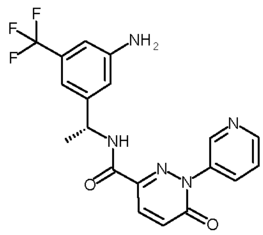 ,

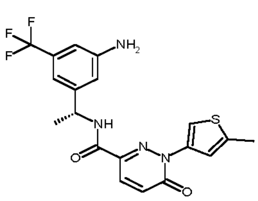 , 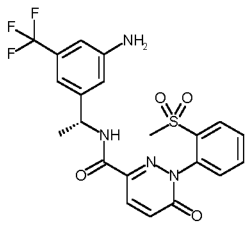 , 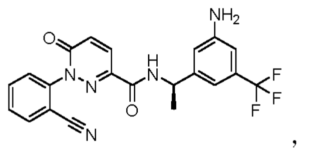 , 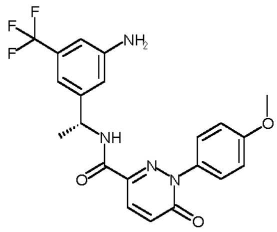 ,

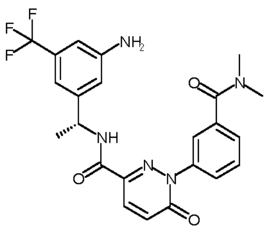 , 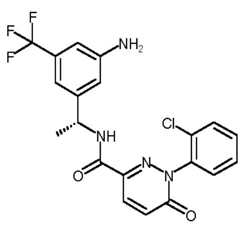 , 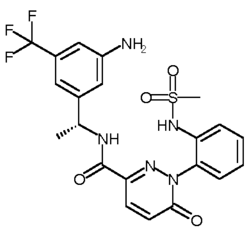 , 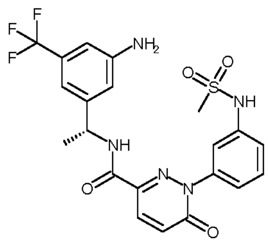 ,

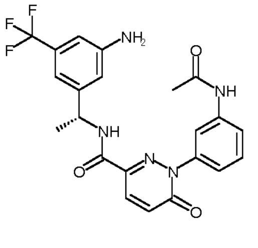 , 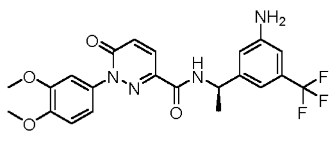 , 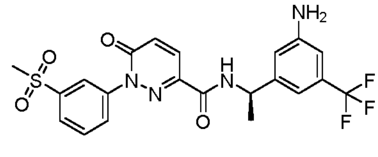 ,

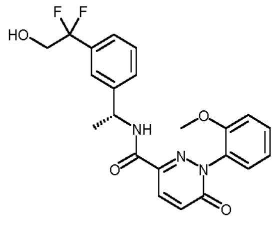 ,
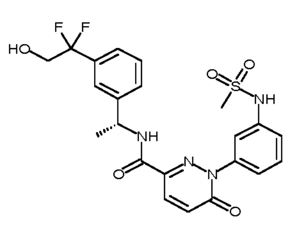 ,
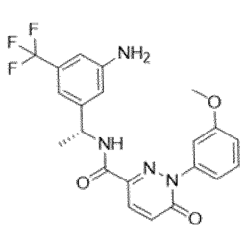 ,
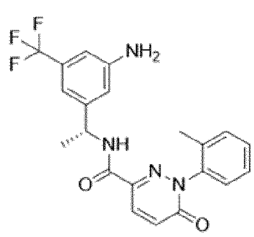 ,
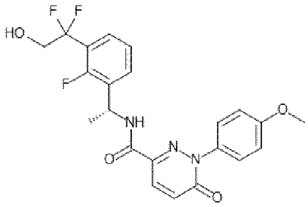 ,
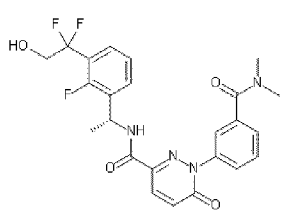 ,
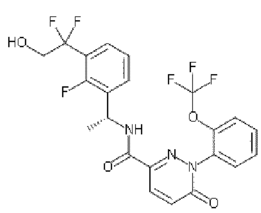 ,
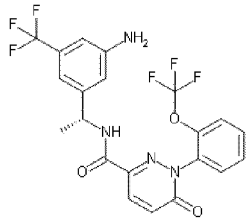 ,
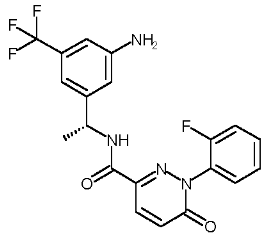 ,
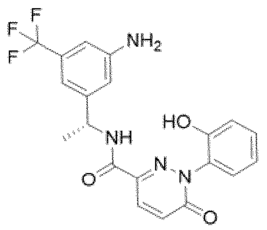 ,
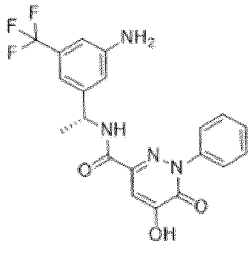 ,
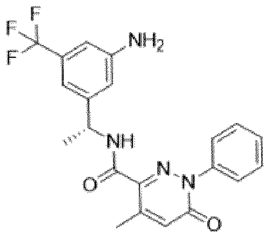 ,
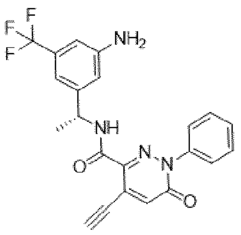 ,
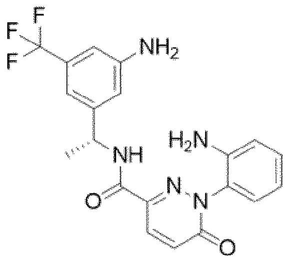 ,
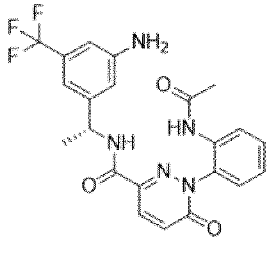 ,
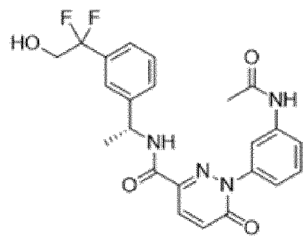 ,
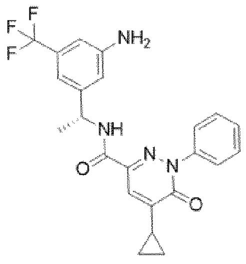 ,
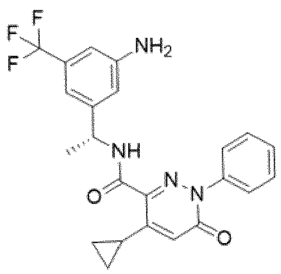 ,
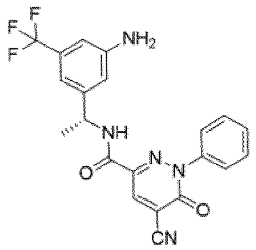 ,
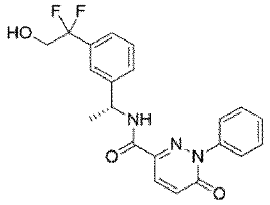 ,
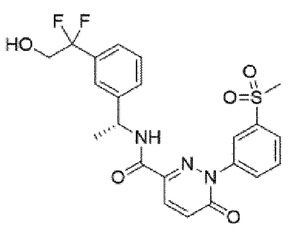 ,
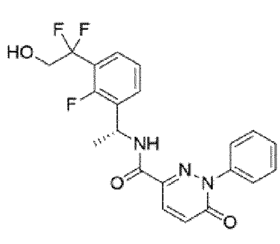 ,
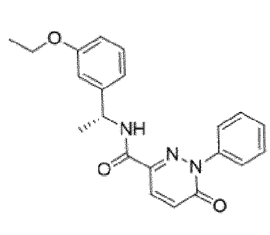 ,
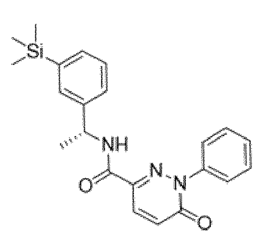 ,

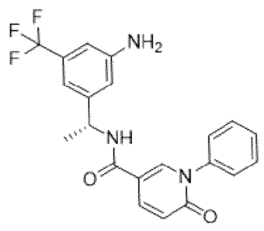

,

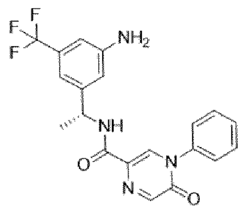

,

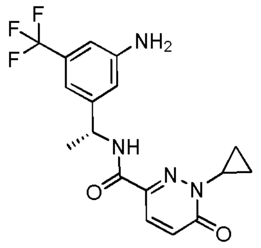

,

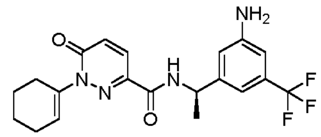

,

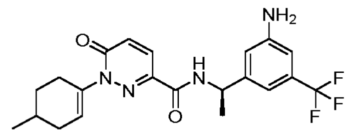

,

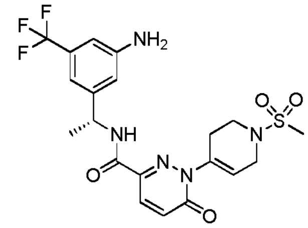

,

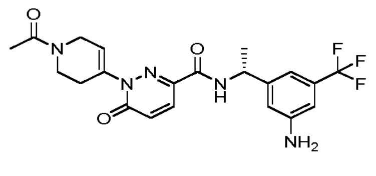

,

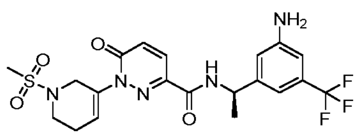

,

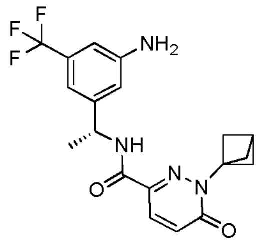

,

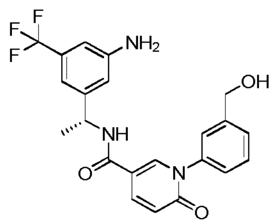

,

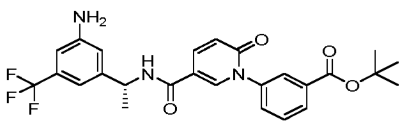

,

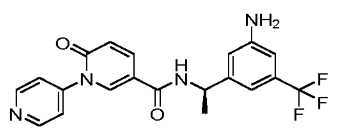

,

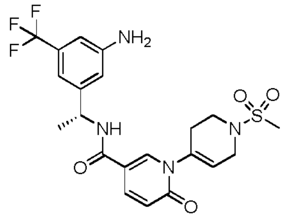

,

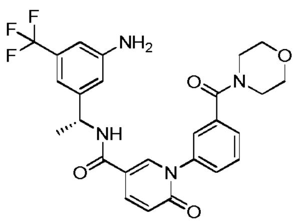

,

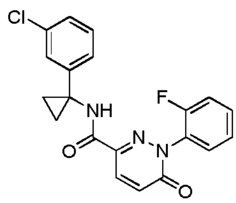

,

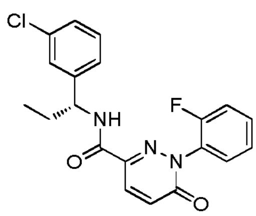

,

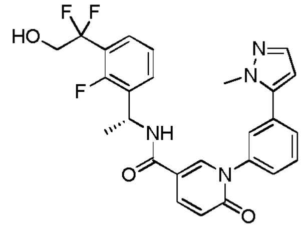

,

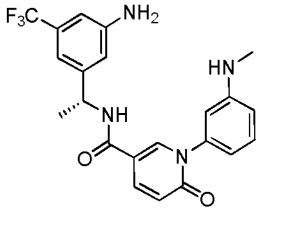

,

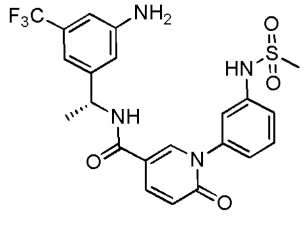

,

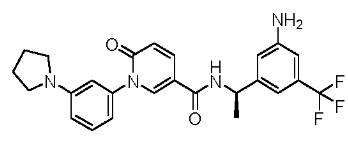

,

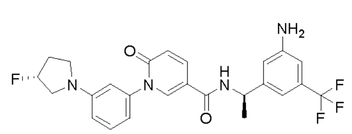, 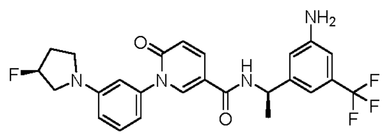, 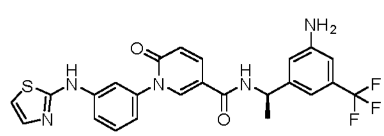,

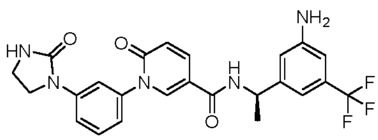, 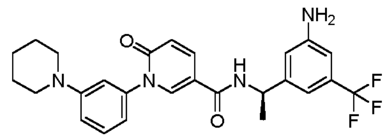, 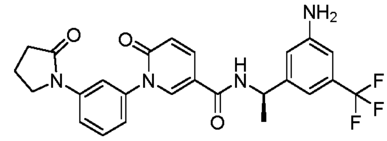,

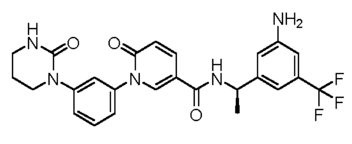 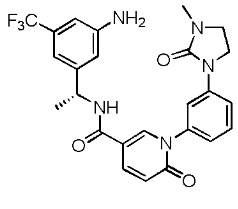, 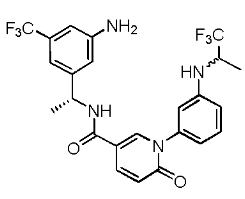, 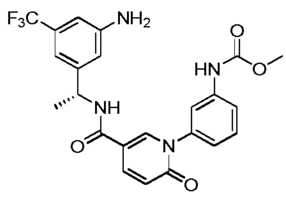,

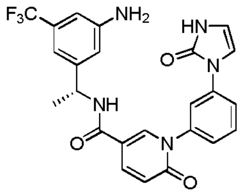, 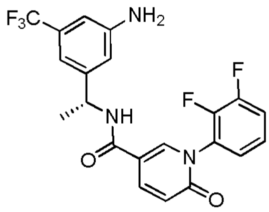, 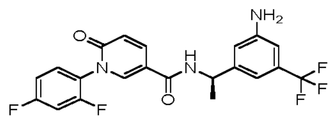,

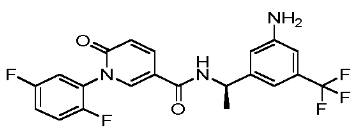, 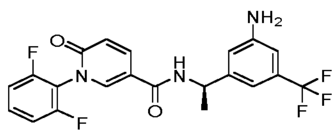, 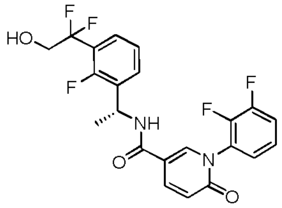,

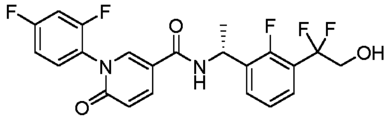, 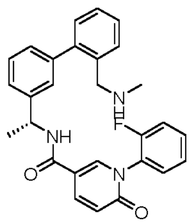, 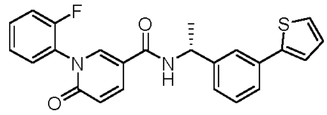,

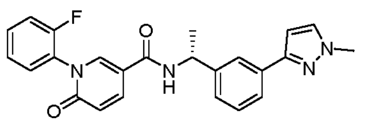, 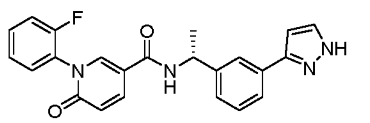, 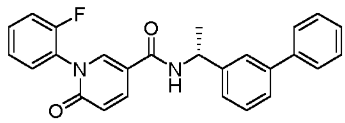,

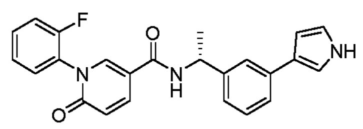 , 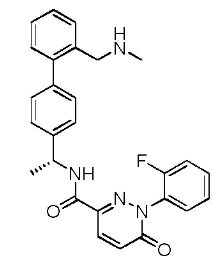 , 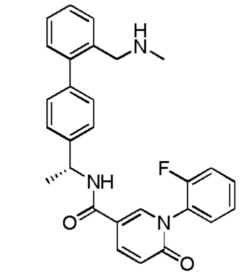 , 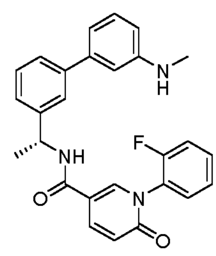 ,

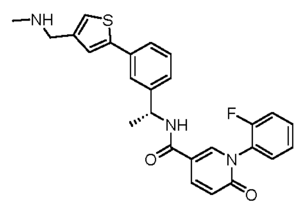 , 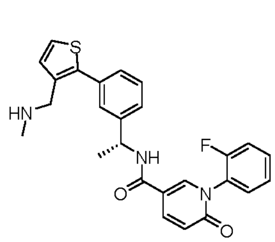 , 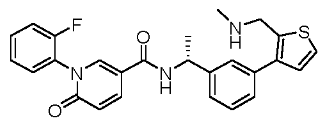 , 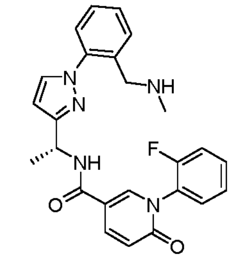 ,

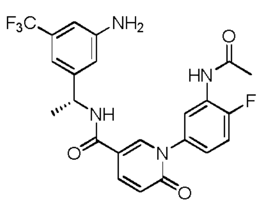 , 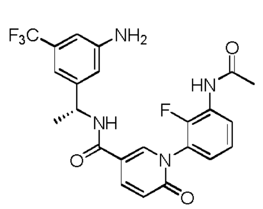 , 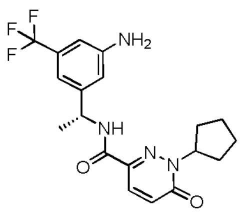 , 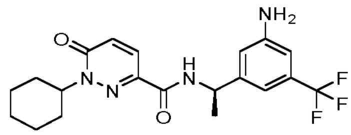 ,

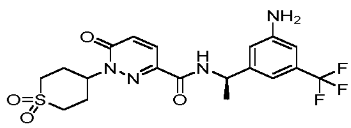 , 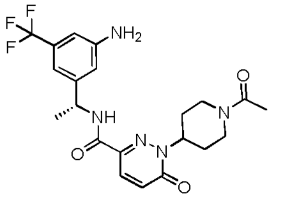 , 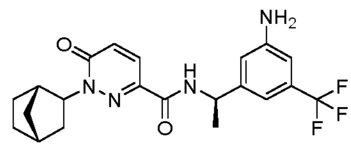 ,

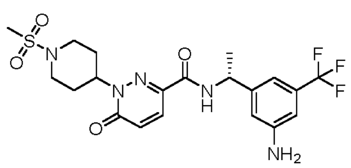 , 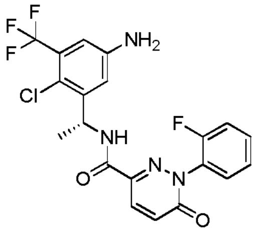 , 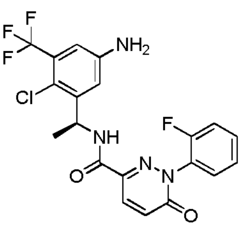 , 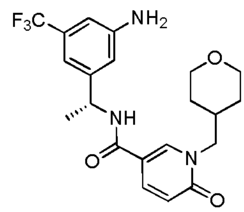 ,

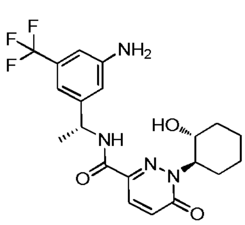 , 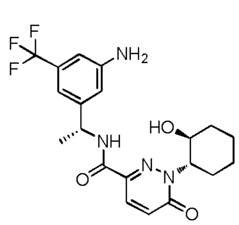 , 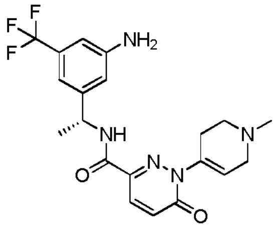 , 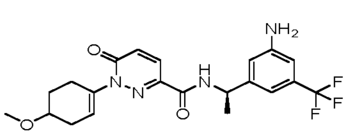 ,

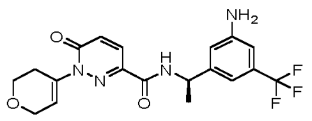,
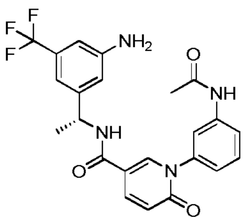,
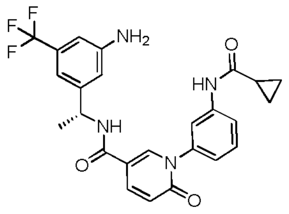,
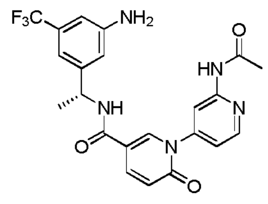,

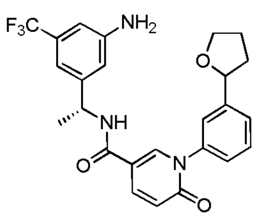,
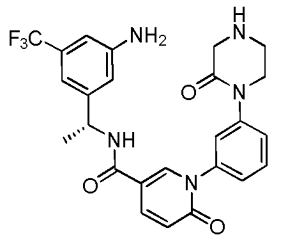,
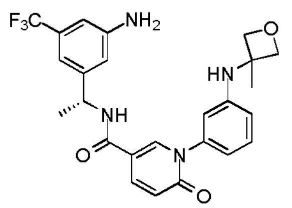,
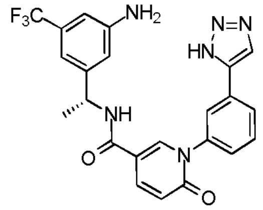,

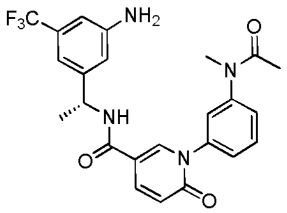,
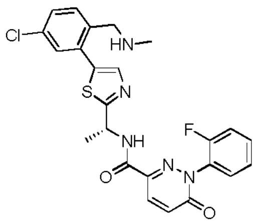,
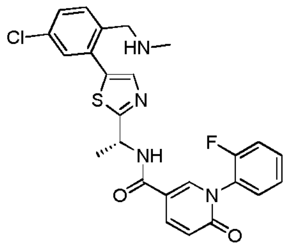,
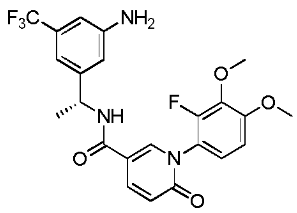,

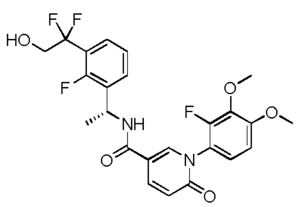,
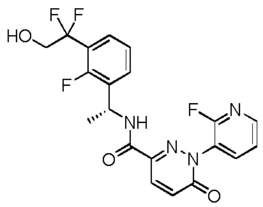,
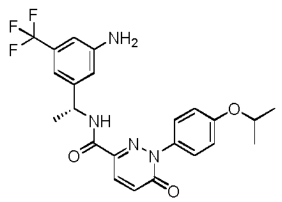,
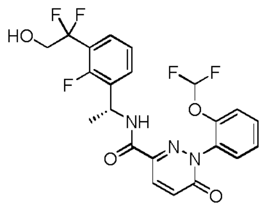,

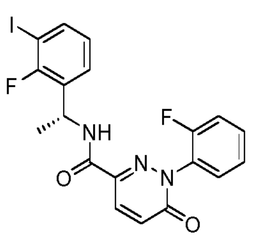,
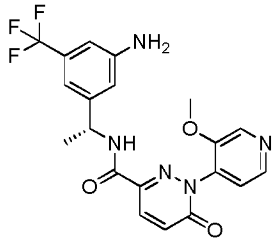,
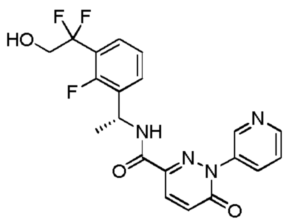,
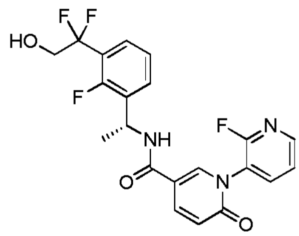,

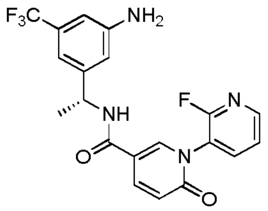,
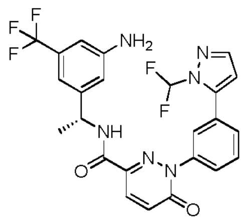,
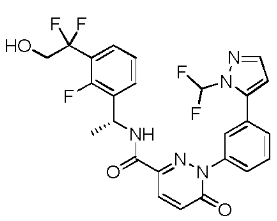,
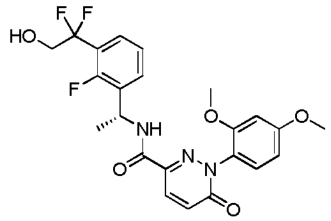,

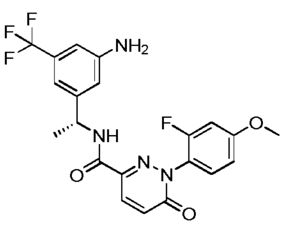 , 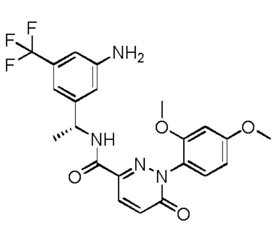 , 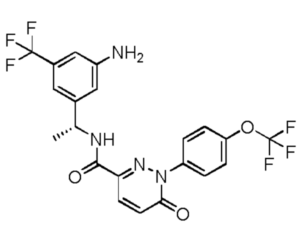 , 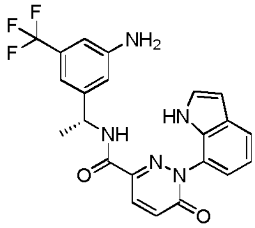 ,

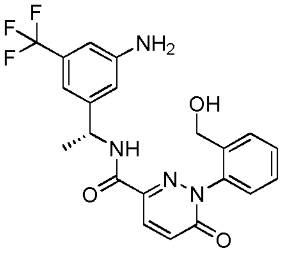 , 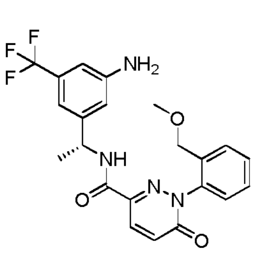 , 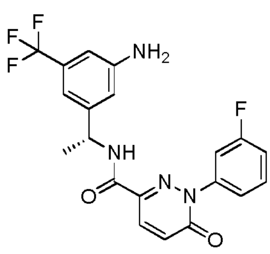 , 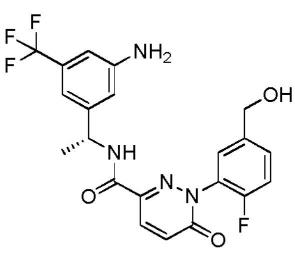 ,

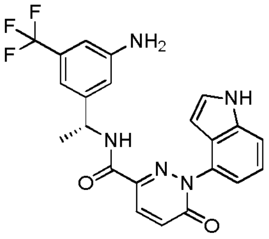 , 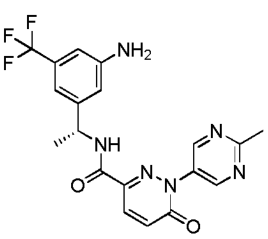 , 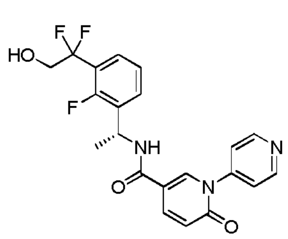 , 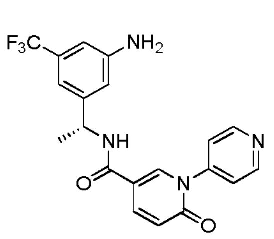 ,

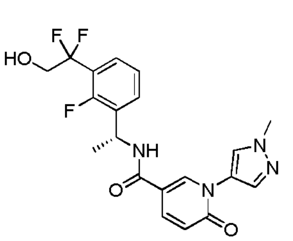 , 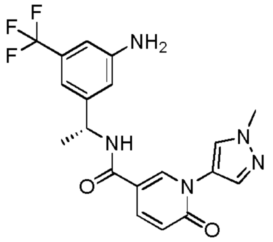 , 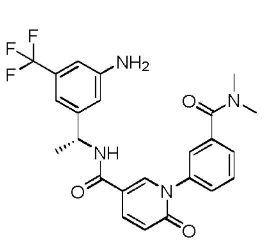 , 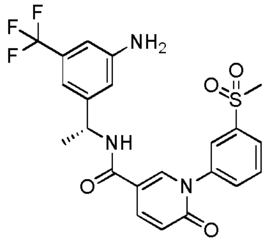 ,

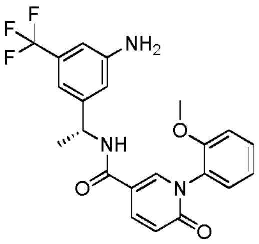 , 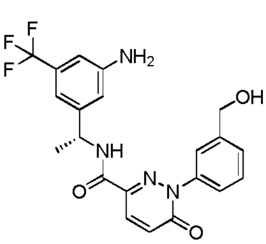 , 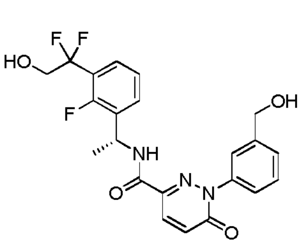 , 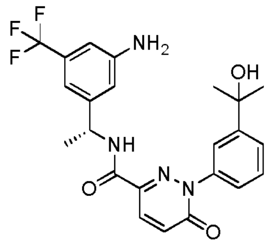 ,

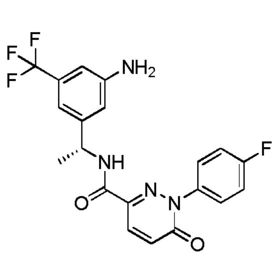 , 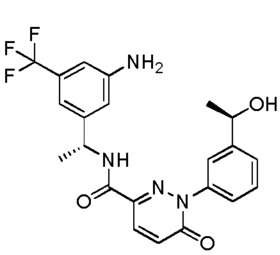 , 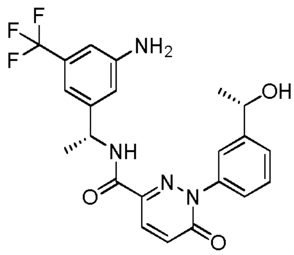 , 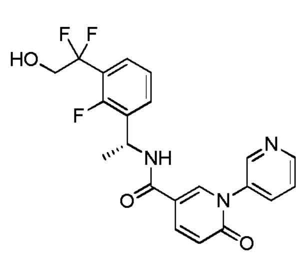 ,

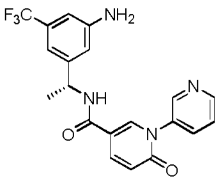 , 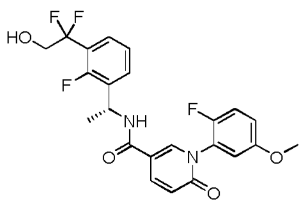 , 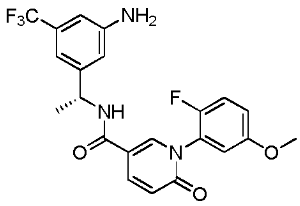 , 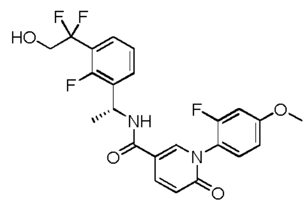 ,

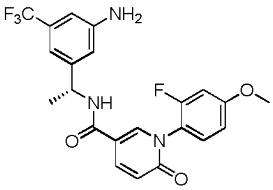 , 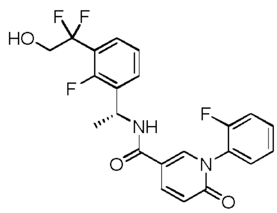 , 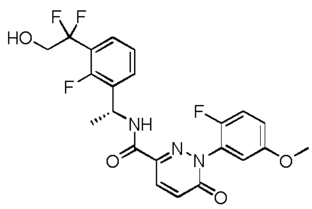 , 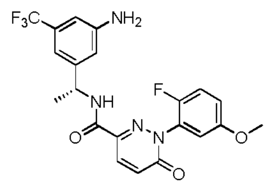 ,

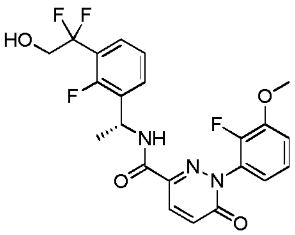 , 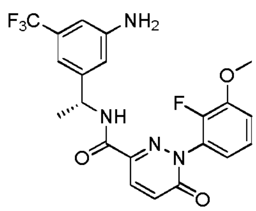 , 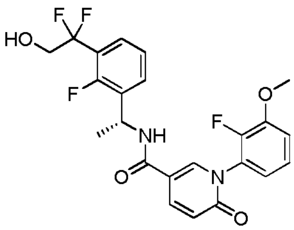 , 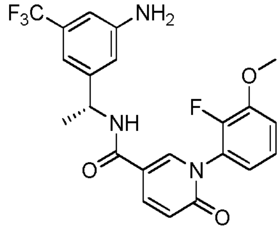 ,

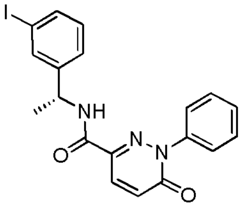 , 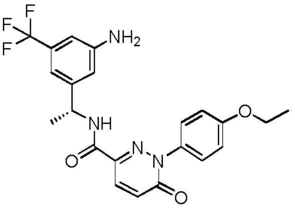 , 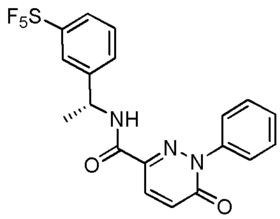 , 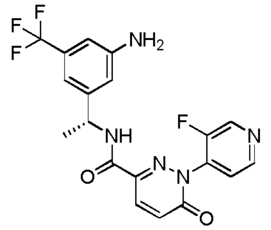 ,

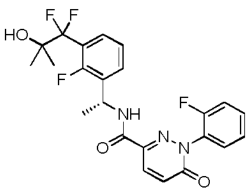 , 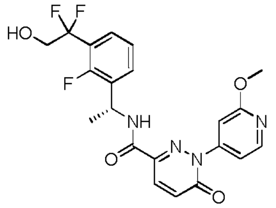 , 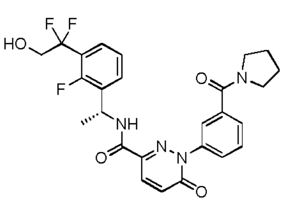 , 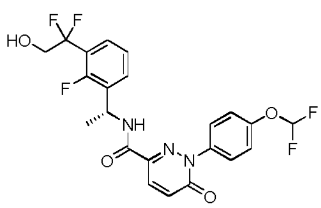 ,

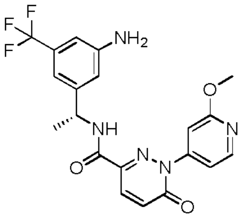 , 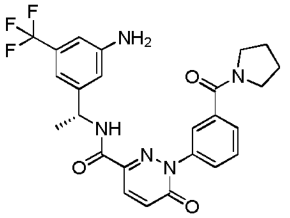 , 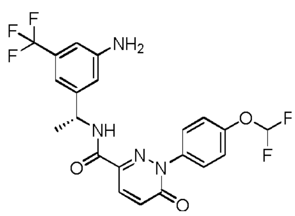 , 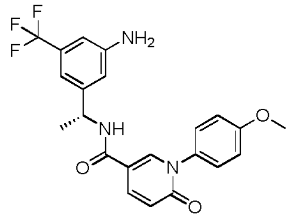 ,

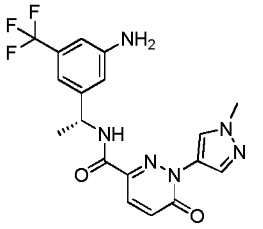 , 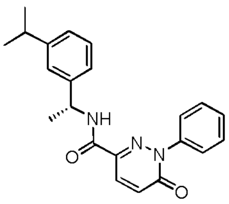 , 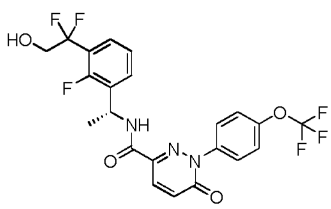 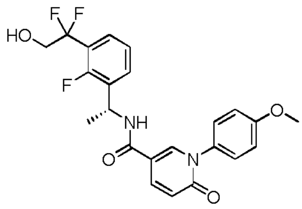 ,

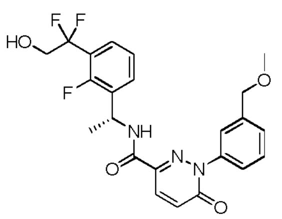 , 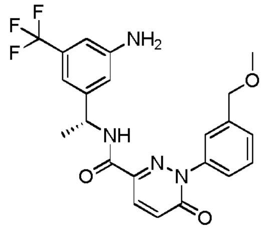 , 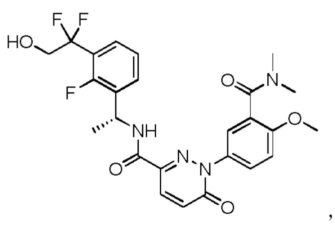 , 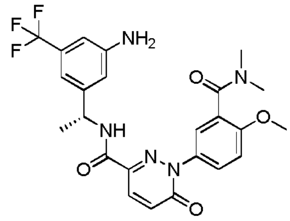 ,

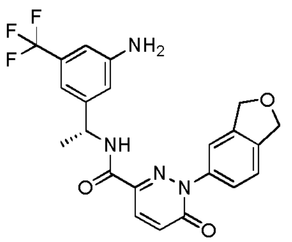 , 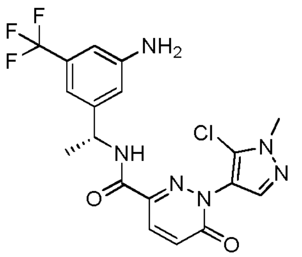 , 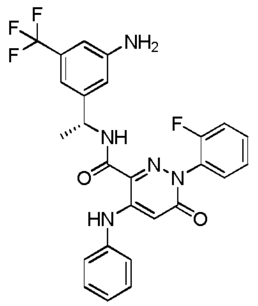 , 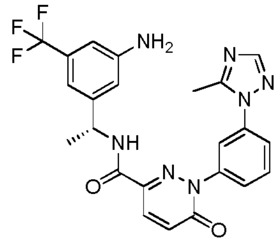 ,

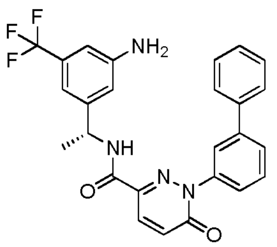 , 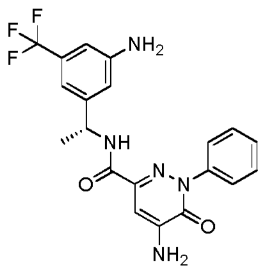 , 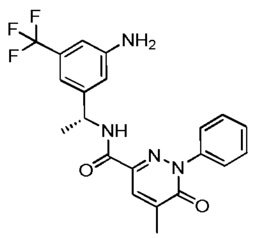 , 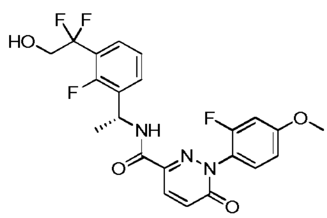 ,

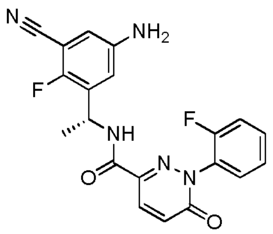 , 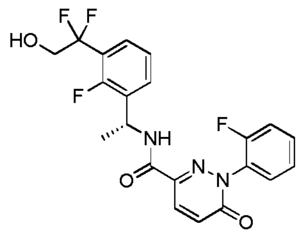 , 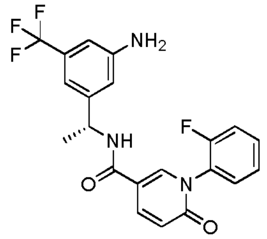 , 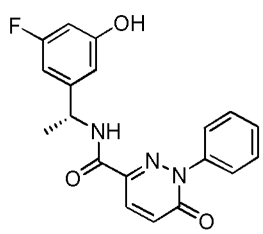 ,

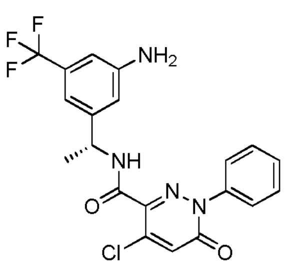 , 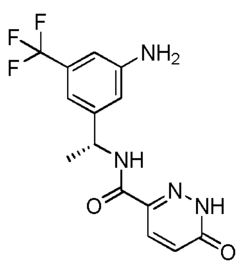 , 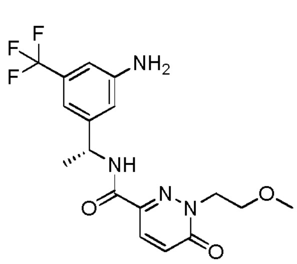 , 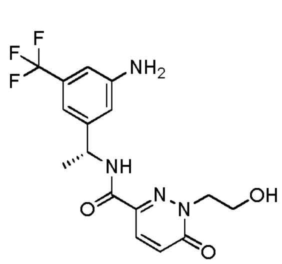 ,

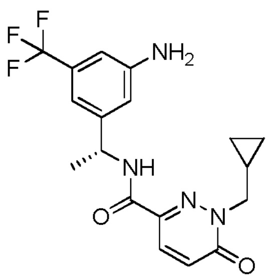 , 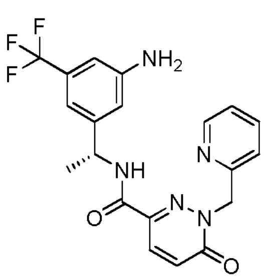 , 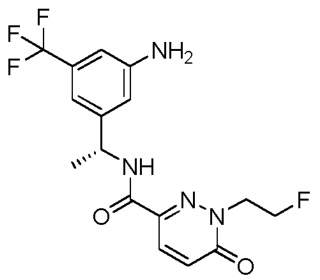 , 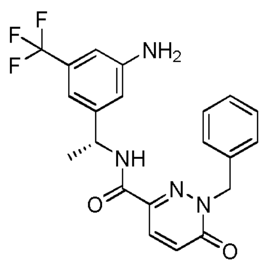 ,

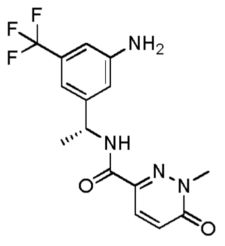 , 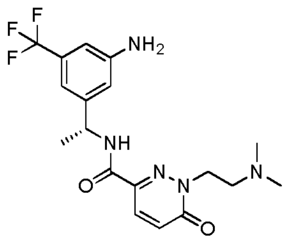 , 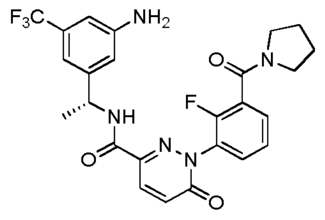 , 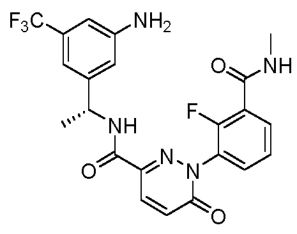 ,

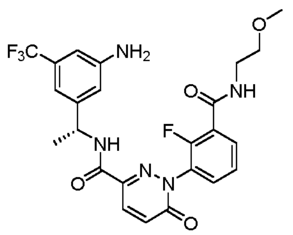 , 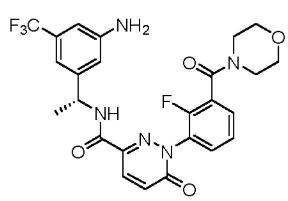 , 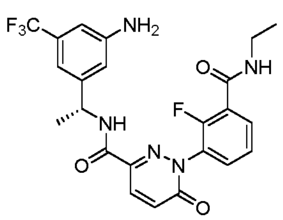 , 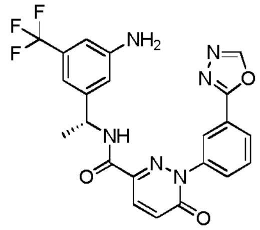 ,

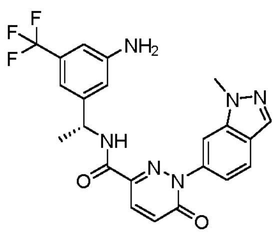 , 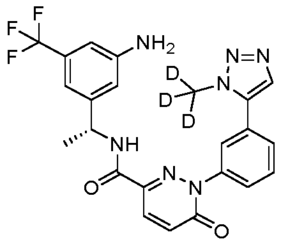 , 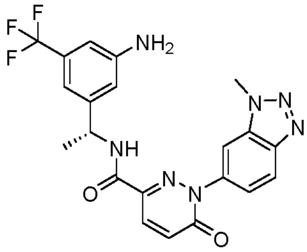 , 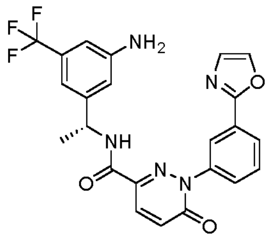 ,

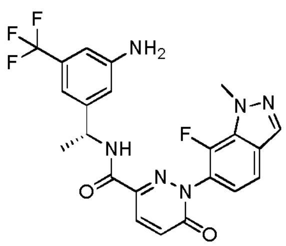 , 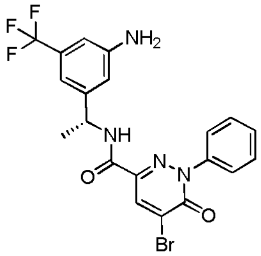 , 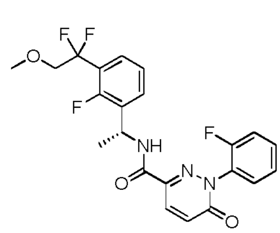 , 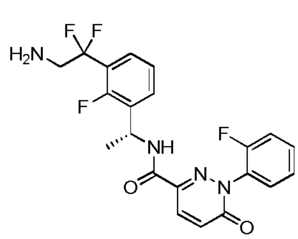 ,

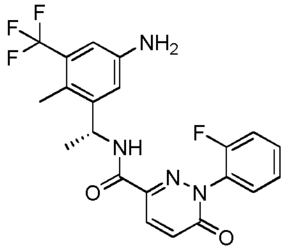 , 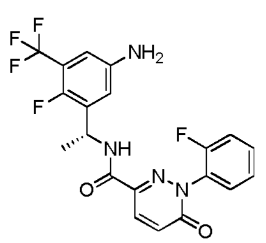 , 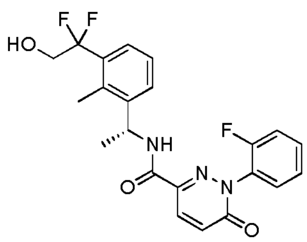 , 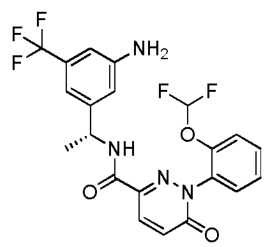 ,

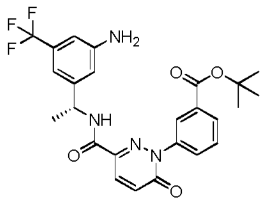 , 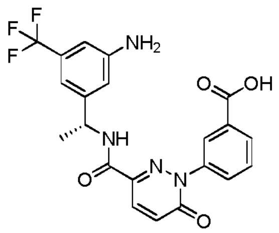 , 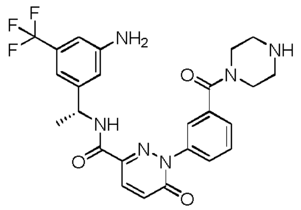 , 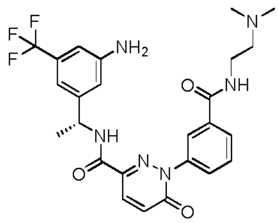 ,

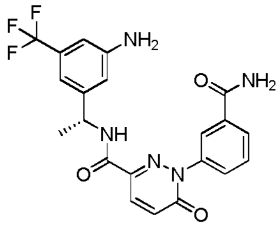 , 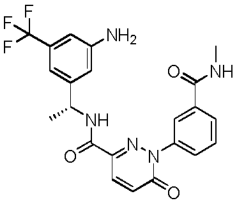 , 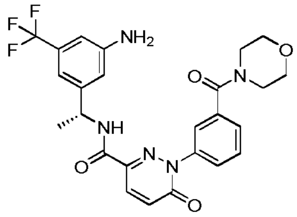 , 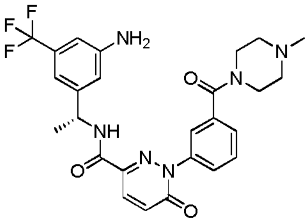 ,

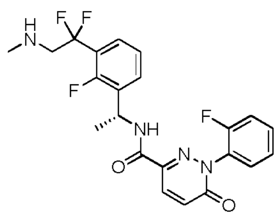 , 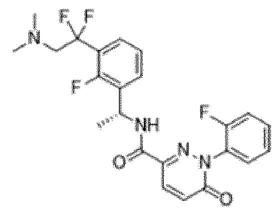 , 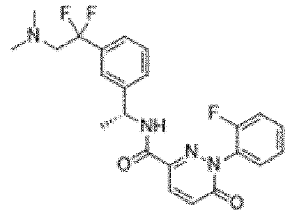 , 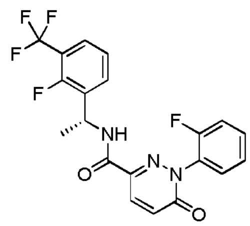 ,

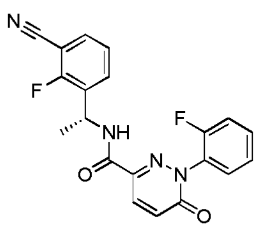 , 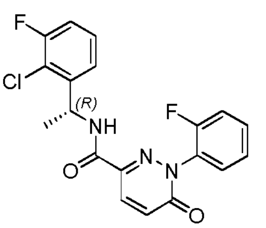 , 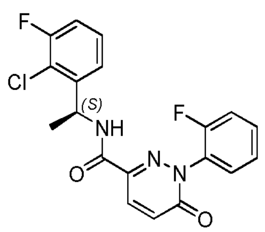 , 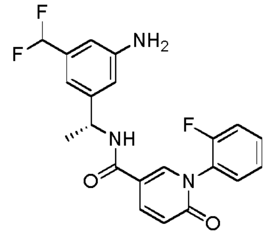 ,

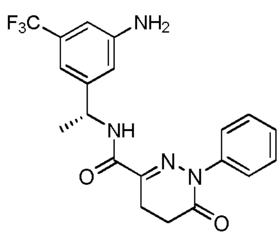 , 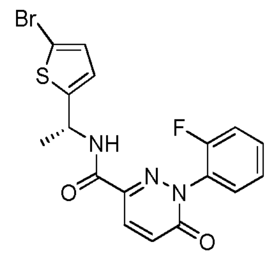 , 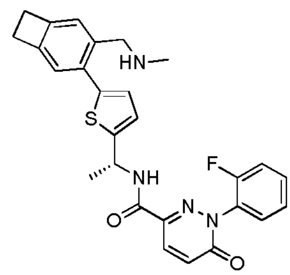 , 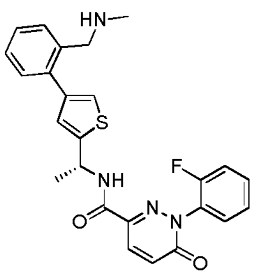 ,

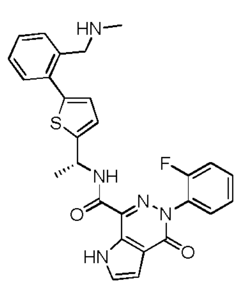 , 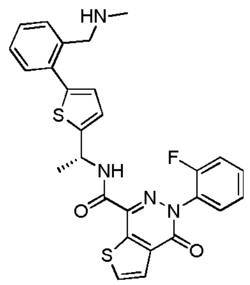 , 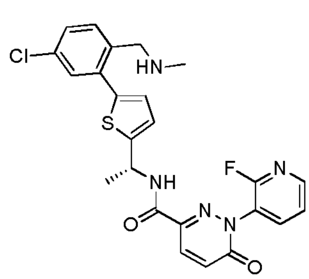 , 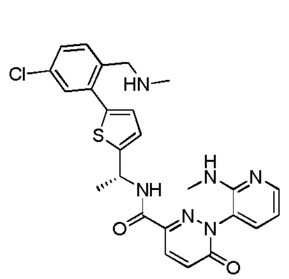 ,

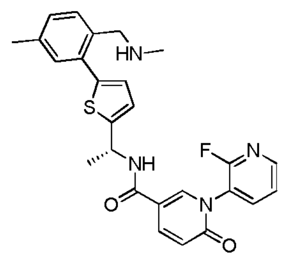
,
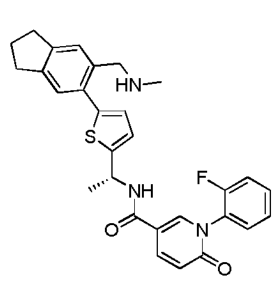
,
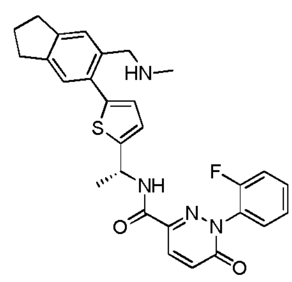
,
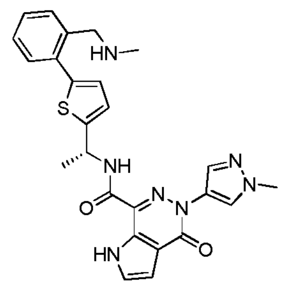
,

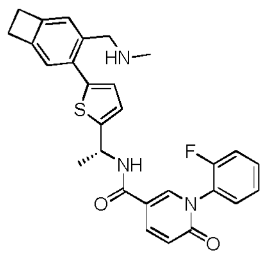
,
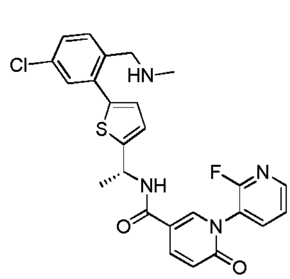
,
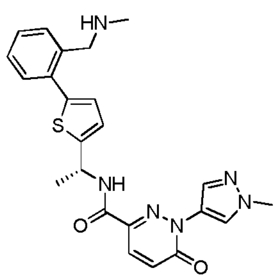
,
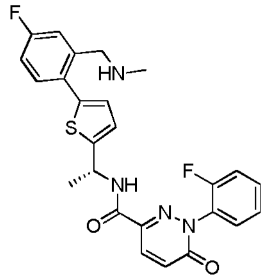
,

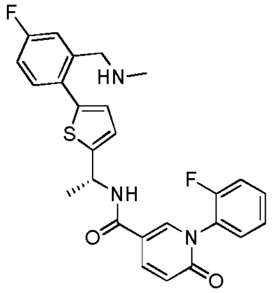
,
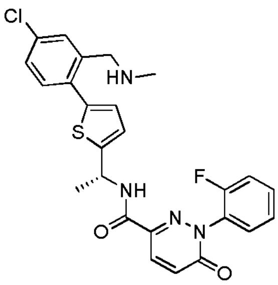
,
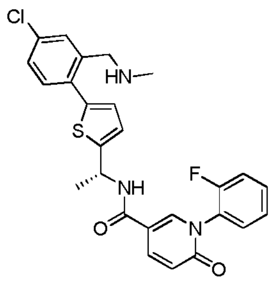
,
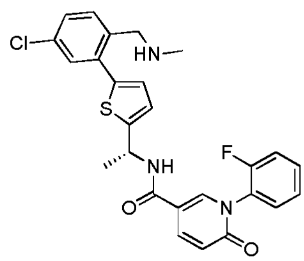
,

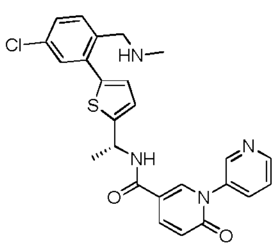
,
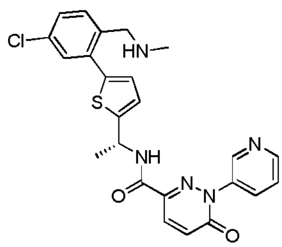
,
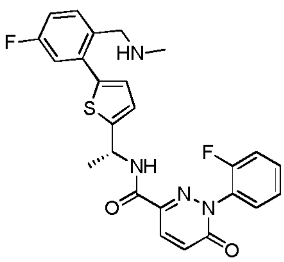
,
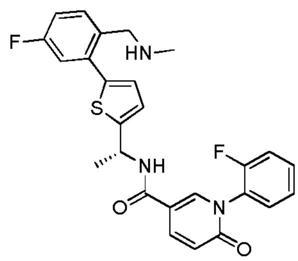
,

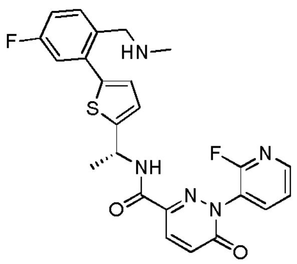
,
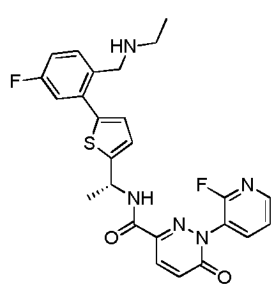
,
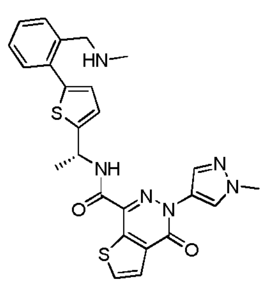
,
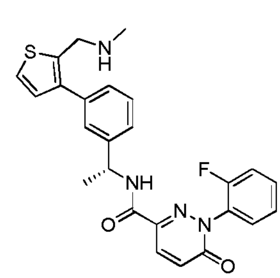
,

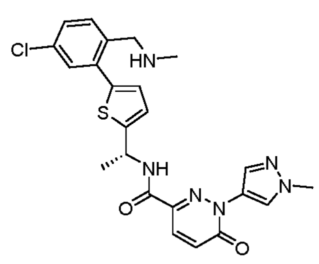 ,
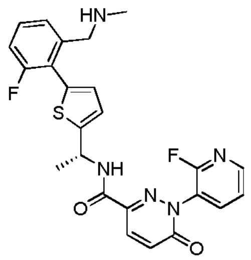 ,
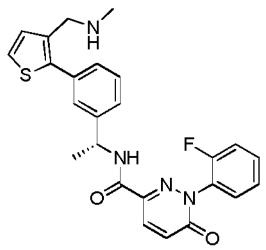 ,
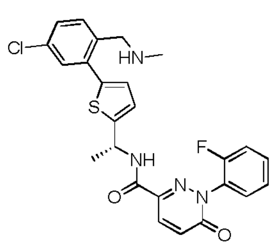 ,

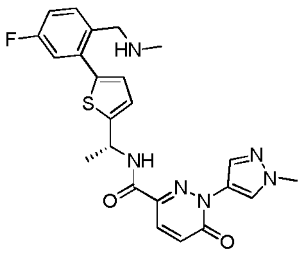 ,
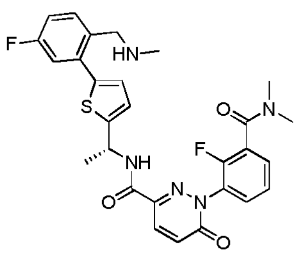 ,
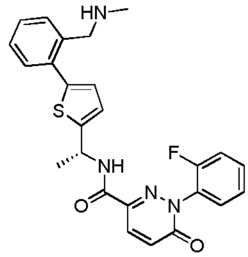 ,
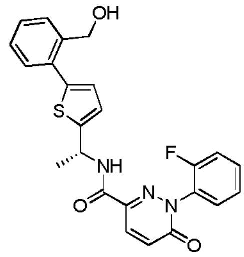 ,

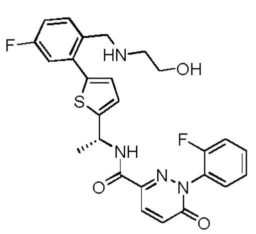 ,
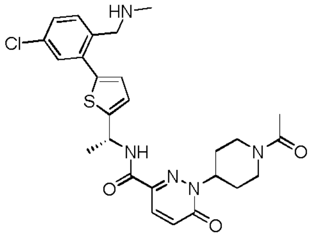 ,
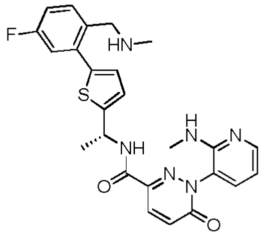 ,
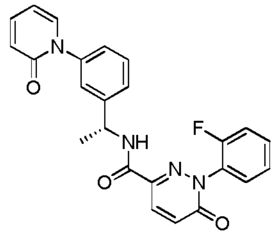 ,

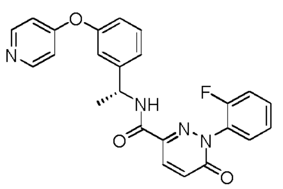 ,
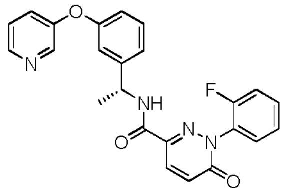 ,
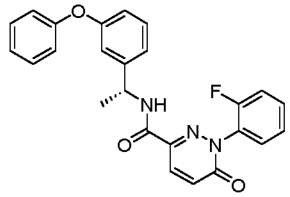 ,
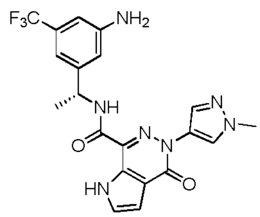 ,

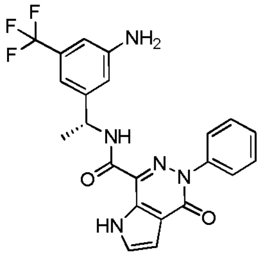 ,
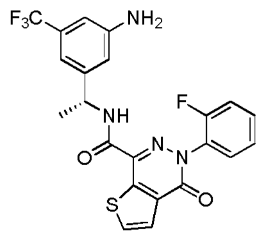 ,
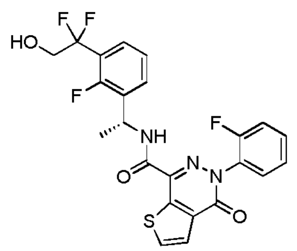 ,
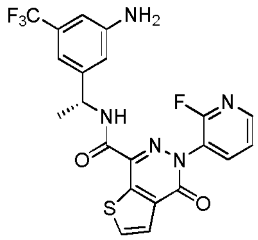 ,

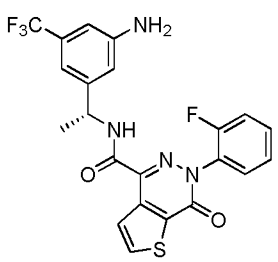 ,
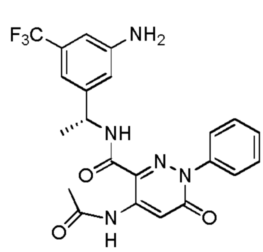 ,
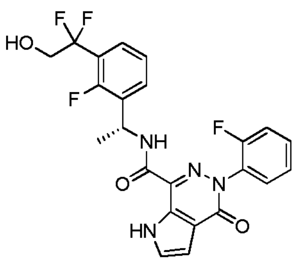 ,
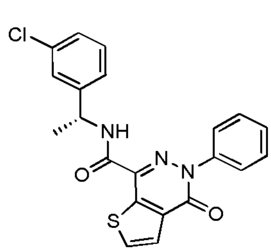 ,

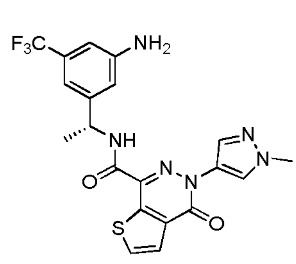 , 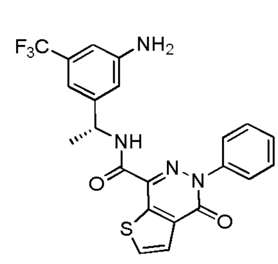 , 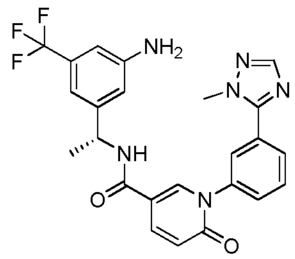 , 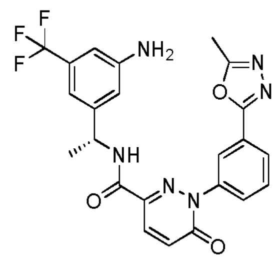 ,

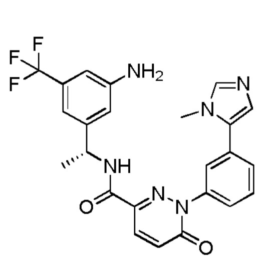 , 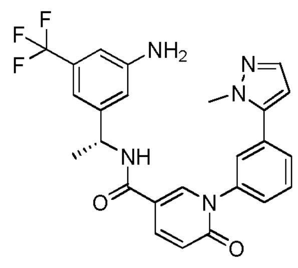 , 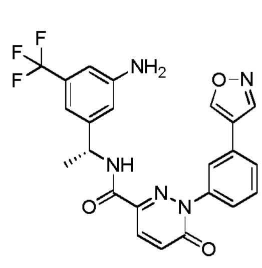 , 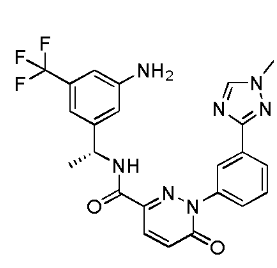 ,

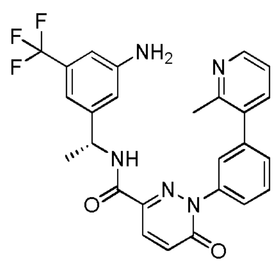 , 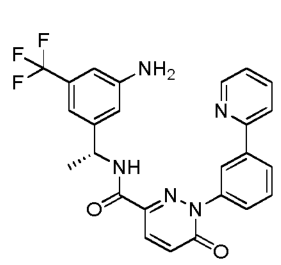 , 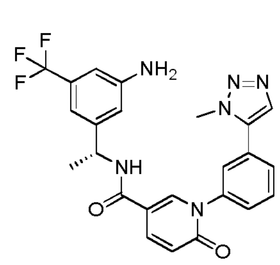 , 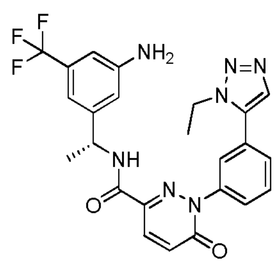 ,

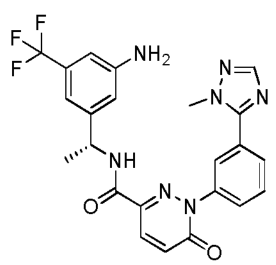 , 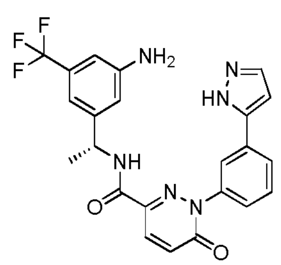 , 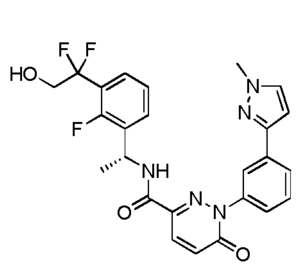 , 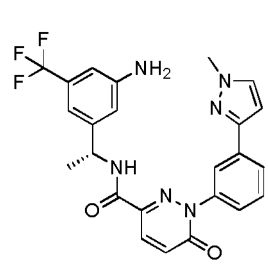 ,

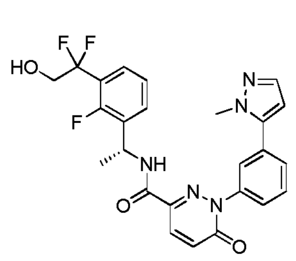 , 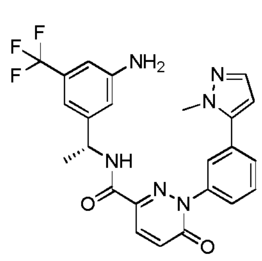 , 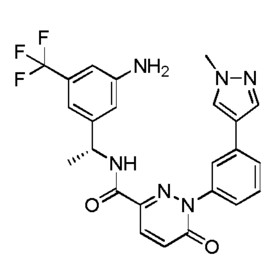 , 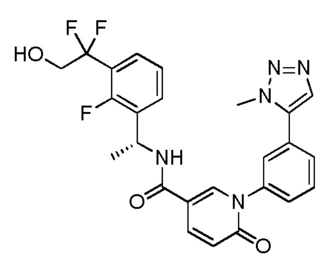 ,

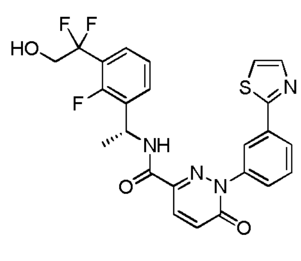 , 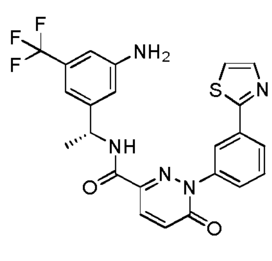 , 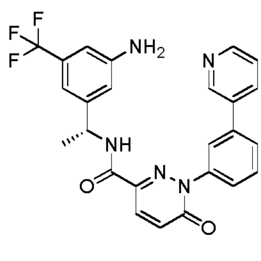 , 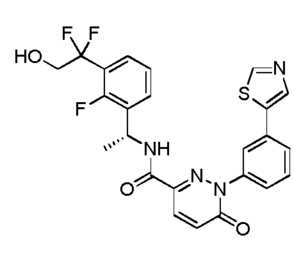 ,

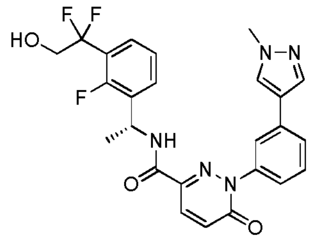, 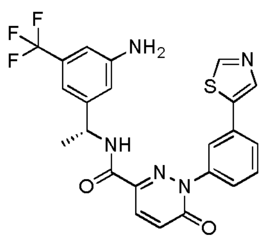, 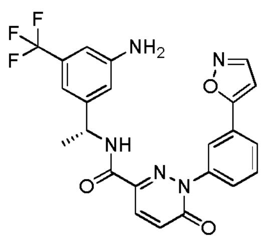, 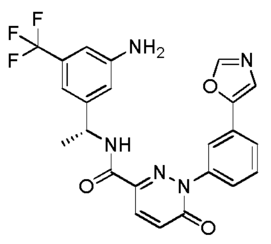,

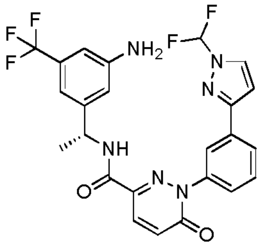, 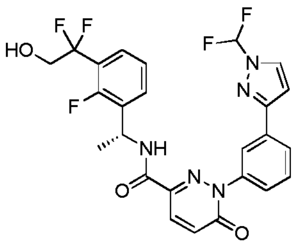, 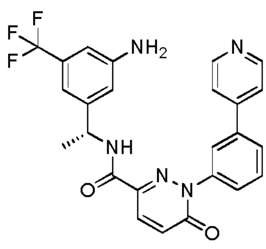, 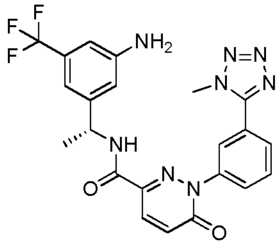,

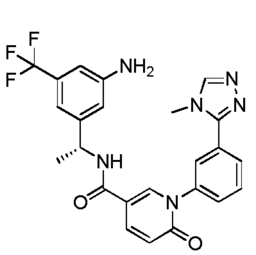, 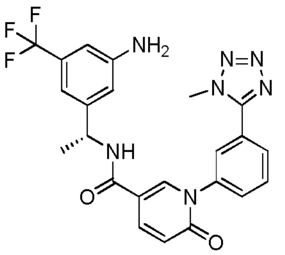, 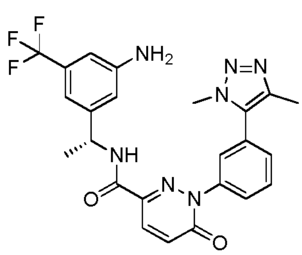, 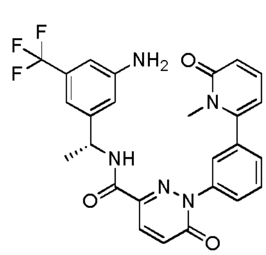,

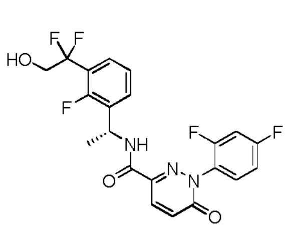, 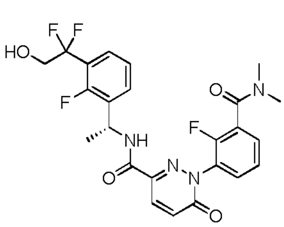, 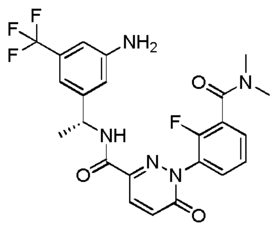, 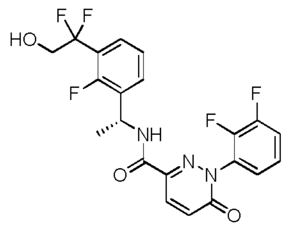,

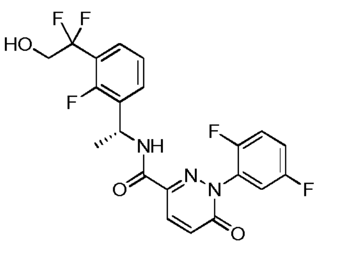, 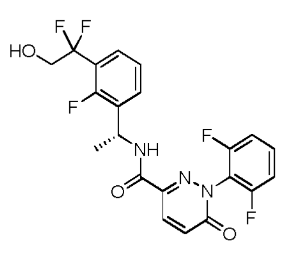, 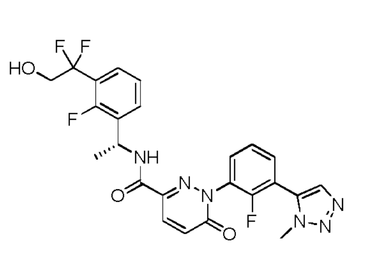,

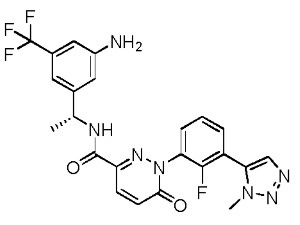, 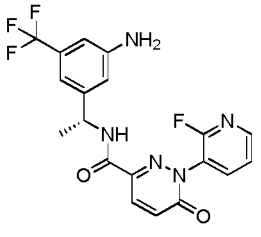, 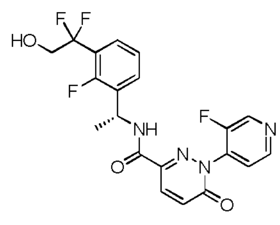, 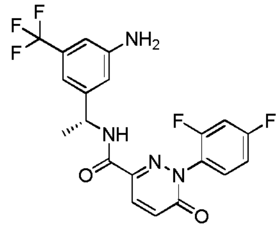,

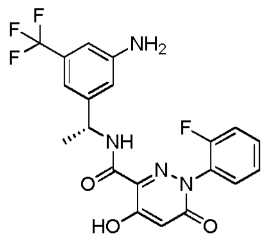 , 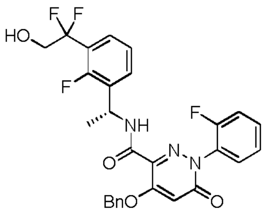 , 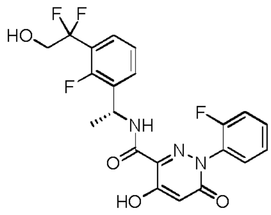 , 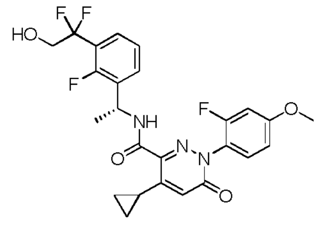 ,

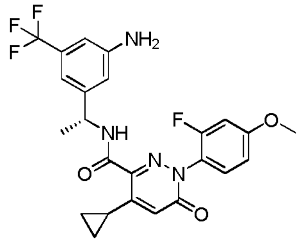 , 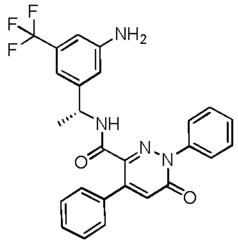 , 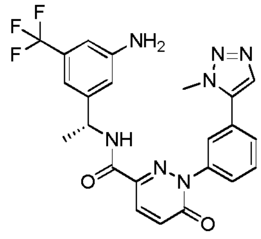 , 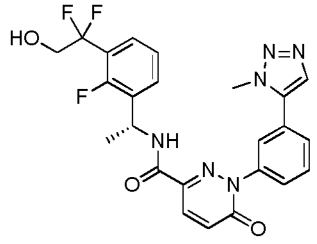 ,

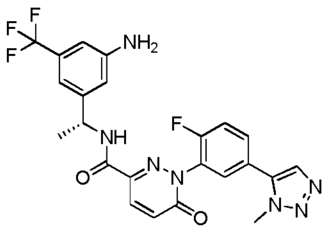 , 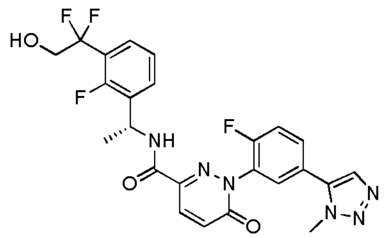 , 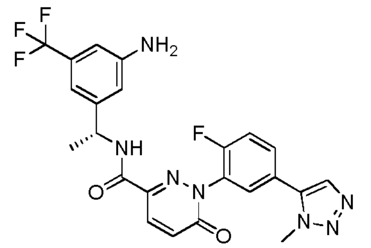 ,

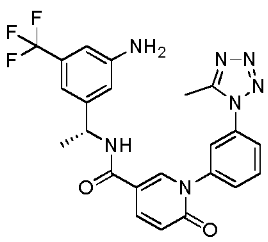 , 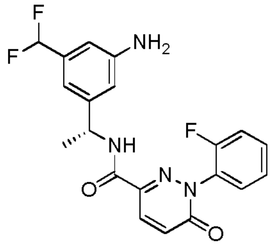 , 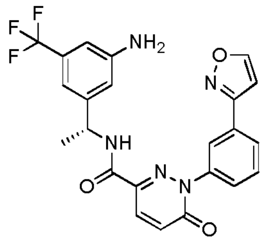 , 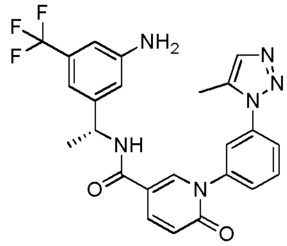 ,

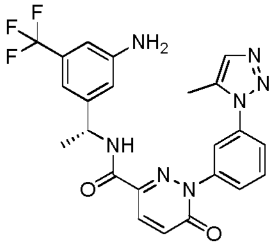 , 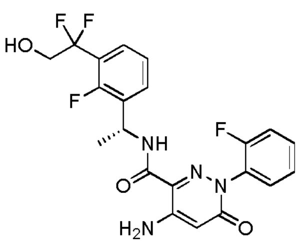 , 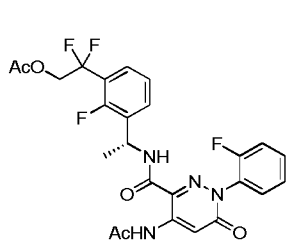 , 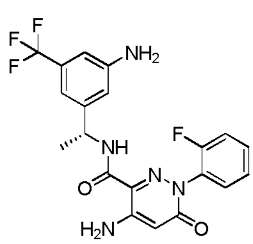 ,

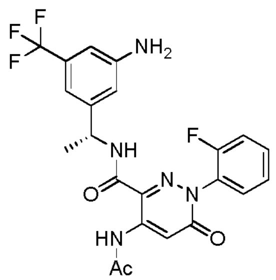 , 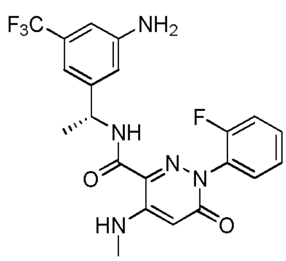 , 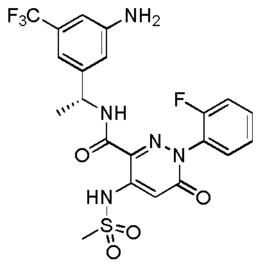 , 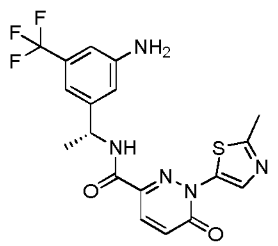 ,

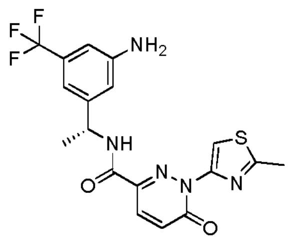 , 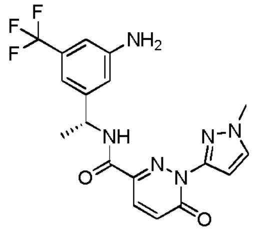 , 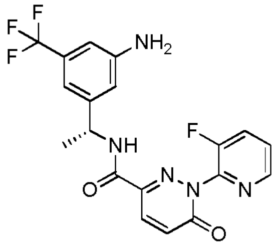 , 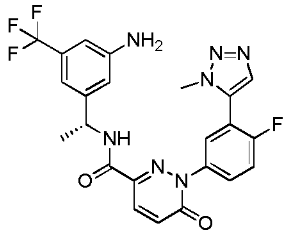 ,

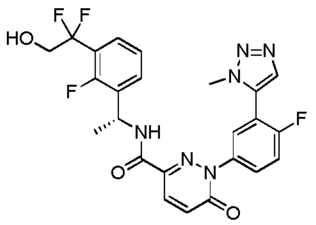 , 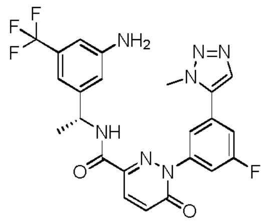 , 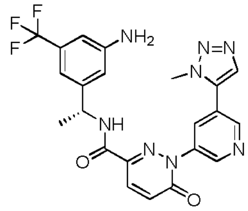 , 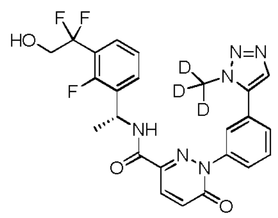 ,

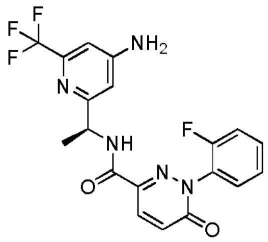 , 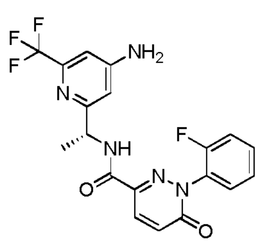 , 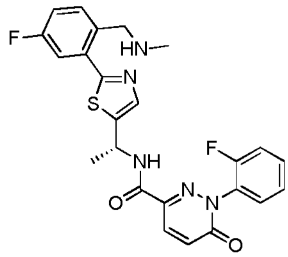 , 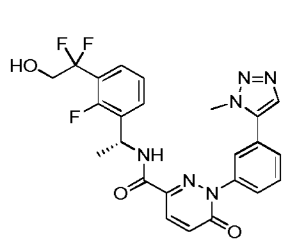 ,

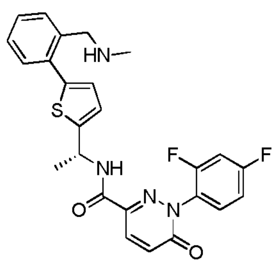 , 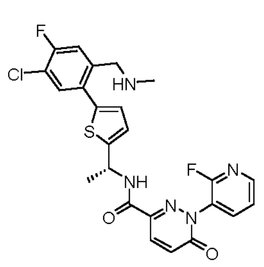 , 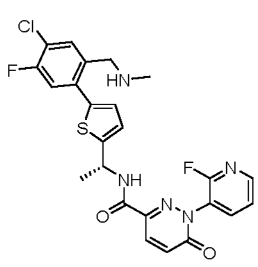 , 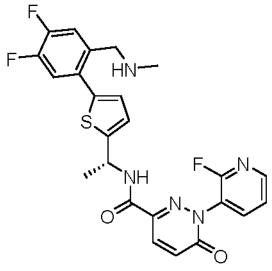 ,

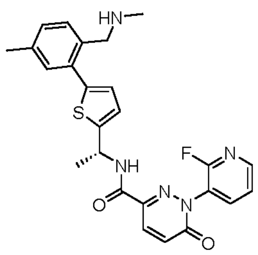, 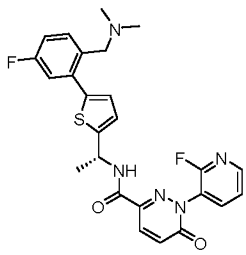, 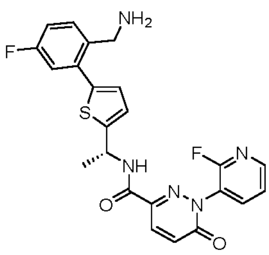, 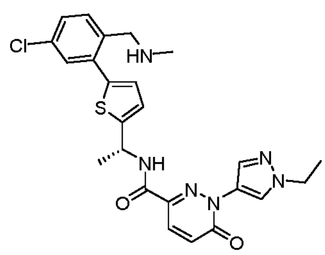,

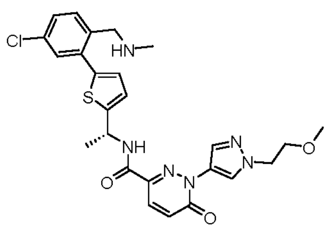, 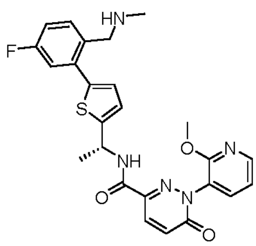, 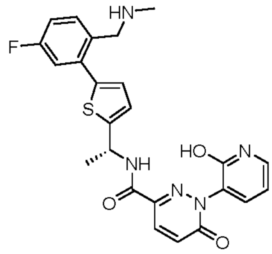, 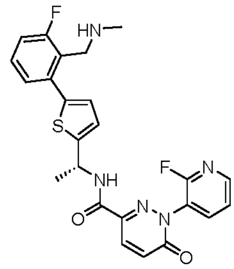,

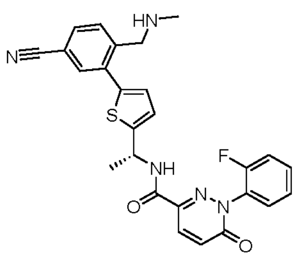, 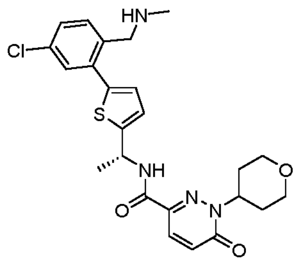, 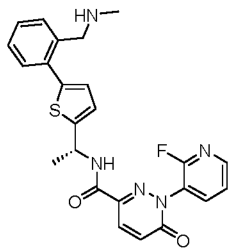, 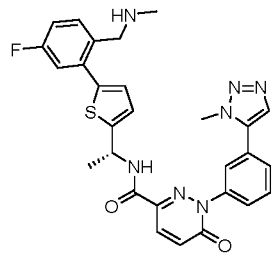,

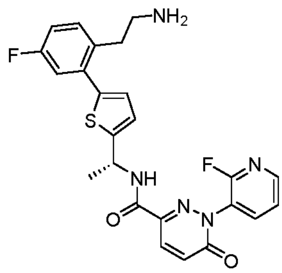, and 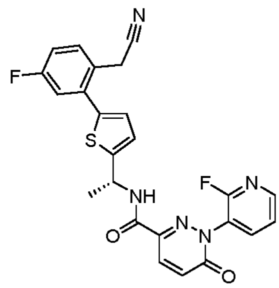.

**Definition**

All technical and scientific terms used herein have the meanings commonly understood by one of ordinary skill in the art, and unless otherwise stated, conventional methods of measurement, methods of manufacture, conventional ingredients or substances are used based on conventional techniques such as pharmacology, pharmaceutical manufacturing chemistry, mass spectrometry, NMR, HPLC, biochemistry, and the like.

Individual features and components of each embodiment described and illustrated herein may be combined with features and components of any other embodiment without departing from the scope or spirit of the present disclosure.

Unless otherwise specified, in the present specification and the appended claims, "or" and "and" mean "and/or". The terms "include" and "included" are open-ended, and mean that a compound, composition, or method may include additional features or ingredients in addition to the listed features or ingredients.

In the present specification, the numerical range indicated using the term "to" refers to a range including the numerical values described before and after the term "to" as the lower limit and the upper limit, respectively.

As used herein, the term "optional" or "optionally" is intended to include that the subsequently described event or circumstance may or may not occur, and that the description includes instances where the event or circumstance occurs and instances where it does not. For example, the term "optionally substituted" is intended to include both unsubstituted or substituted with the specified substituent.

## Compound

As used herein, the term "alkyl" refers to a fully saturated branched or unbranched (or straight chain or linear) hydrocarbon. The alkyl may be a substituted or unsubstituted alkyl group. The alkyl may be optionally interrupted by at least one oxygen atom or nitrogen atom, and the alkyl group interrupted by an oxygen atom or nitrogen atom refers to an alkyl group in which an oxygen atom or a nitrogen atom is inserted between carbon atoms of the alkyl chain. For example, the alkyl interrupted by an oxygen atom or nitrogen atom includes alkoxyalkyl, alkylaminoalkyl, and the like, and includes one in which an oxygen atom or nitrogen atom is located at the end of a substituent, such as hydroxyalkyl or aminoalkyl. The $C_{1-}C_6$ alkyl may be a $C_1$ to $C_6$, $C_1$ to $C_5$, $C_1$ to $C_4$, $C_1$ to $C_3$, or $C_1$ to $C_2$ alkyl group. Non-limiting examples of the alkyl may be methyl, ethyl, n-propyl, isopropyl, n-butyl, isobutyl, sec-butyl, n-pentyl, isopentyl, neopentyl, iso-amyl, or n-hexyl.

As used herein, the term "alkenyl" refers to a straight chain or branched chain hydrocarbon group having 2 to 6 carbon atoms, 2 to 5 carbon atoms, or 2 to 4 carbon atoms having one or more double bonds at any position. For example, it may include vinyl, propenyl, isopropenyl, butenyl, isobutenyl, prenyl, butadienyl, pentenyl, isopentenyl, pentadienyl, hexenyl, isohexenyl, hexadienyl, and the like.

As used herein, the term "alkynyl" refers to a hydrocarbon group containing at least one triple bond, and includes a straight chain or branched chain alkynyl having 2 to 6 carbon atoms, 2 to 5 carbon atoms, or 2 to 4 carbon atoms. For example, it may include ethynyl, propynyl, butynyl, pentynyl, hexynyl, and the like.

As used herein, unless otherwise stated, the term "alkoxy" refers to a substituent in which a substituted or unsubstituted straight chain or branched chain alkyl moiety is linked to another chemical structure by oxygen. The alkoxy may include all possible isomers thereof such as, for example, methoxy, ethoxy, propoxy, and butoxy, or isopropoxy, isobutoxy, and t-butoxy, but is not limited thereto.

As used herein, the term "cycloalkyl" refers to a saturated hydrocarbon ring having the specified number of carbon atoms as ring elements (that is, $C_3$-$C_8$ cycloalkyl refers to a cycloalkyl group having 3, 4, 5, 6, 7 or 8 carbon atoms as ring elements). The cycloalkyl may be $C_3$-$C_6$ monocyclic or $C_5$-$C_{20}$ polycyclic (for example, bicyclic, tricyclic or tetracyclic). For example, monocyclic cycloalkyl may be $C_3$-$C_6$, $C_3$-$C_5$, or $C_3$-$C_4$ cycloalkyl. Monocyclic cycloalkyl may be, for example, cyclopropyl, cyclobutyl, cyclopentyl, cyclohexyl, and the like. Bicyclic, tricyclic or tetracyclic cycloalkyl may be $C_5$-$C_{18}$ cycloalkyl, $C_5$-$C_{15}$ cycloalkyl, $C_5$-$C_{11}$ cycloalkyl, $C_5$-$C_{10}$ cycloalkyl. Polycyclic cycloalkyl may be one in which two or more cycloalkyls are bridged, fused, or spiro bonded, and in tricyclic or tetracyclic cycloalkyl, each cycloalkyl ring may be bonded in the form of two or more of bridged, fused and spiro bonded forms. For example, polycyclic bridged, fused or spiro cycloalkyl may include bicyclo[1.1.1]pentanyl, bicyclo[2.2.2]octanyl, adamantyl, bicyclo[2.2.1]heptanyl, bicyclo[3.1.0]hexanyl, bicyclo[3.2.0]heptanyl, bicyclo[3.2.1]octanyl, bicyclo[3.3.1]octanyl, bicyclo[3.3.0]octanyl, bicyclo[4.2.0]octanyl, spiro[2.3]hexanyl, spiro[2.4]heptanyl, spiro[3.3]heptanyl, spiro[2.5]octanyl, spiro[3.4]octanyl, octahydro-1H-indenyl, decahydronaphthalenyl, and the like. As used herein, cycloalkyl may optionally include one fused with heteroaryl or heterocycloalkyl (for example, cyclohexyl fused with pyrazole, piperazine or tetrahydropyran), in which case heteroaryl or heterocycloalkyl is as defined below.

As used herein, the term "cycloalkenyl" refers to a non-aromatic unsaturated monocyclic or polycyclic hydrocarbon ring having at least one carbon-carbon double bond and containing the specified number of carbon atoms. For example, monocyclic cycloalkenyl may include cyclopent-1-en-1-yl, cyclohex-1-en-1-yl, cyclohex-1,3-dien-1-yl, and the like, but is not limited thereto. The above matters regarding the carbon number and bond form of bicyclic, tricyclic or tetracyclic cycloalkyl apply equally to bicyclic, tricyclic or tetracyclic cycloalkenyl. For example, bicyclic, tricyclic or tetracyclic cycloalkenyl includes those in which a carbon-carbon double bond is introduced at any position in the bicyclic, tricyclic or tetracyclic cycloalkyl exemplified above. In the present specification, cycloalkenyl may optionally include one fused with heteroaryl or heterocycloalkyl (for example, cyclohexenyl fused with pyrazole, piperazine or tetrahydropyran), in which case heteroaryl or heterocycloalkyl is as defined below.

As used herein, the term "aryl" refers to a monocyclic or polycyclic aromatic hydrocarbon group. The aryl has alternating (resonance) double bonds between adjacent carbon atoms or suitable heteroatoms, and may also include a form in which two or more rings are simply attached to each other (pendant) or condensed. The aryl may be, for example, $C_6$-$C_{10}$ aryl, or $C_6$-$C_9$ aryl, and may include, for example, phenyl, naphthalenyl (naphthyl), toluyl, or all possible isomers thereof, but is not limited thereto. In the present specification, aryl may be fused with cycloalkyl. For example, $C_{6-10}$ aryl may be fused with 3- to 8-membered cycloalkyl. In this case, phenyl and cyclobutyl may be fused to form bicyclo[4.2.0]octa-1,3,5-trienyl, or phenyl and cyclopentyl may be fused to form 2,3-dihydroindenyl. In addition, in the present specification, aryl may be optionally fused with heterocycloalkyl. For example, $C_{6-10}$ aryl may be fused with 5- to 10-membered heterocycloalkyl. For example, phenyl and tetrahydrofuranyl may be fused to form dihydrobenzofuranyl or dihydroisobenzofuranyl.

As used herein, the term "heteroaryl" refers to a heterocyclic aromatic group containing at least one heteroatom selected from B, N, O, S, P(=O), Si and P as a ring-forming atom. The heteroaryl may also include a form in which two or more rings are simply attached to each other (pendant) or condensed. The heteroaryl may contain 1 to 4 heteroatoms,

1 to 3 heteroatoms, 1 or 2 heteroatoms, or 1 heteroatom selected from N, O and S. The heteroaryl may contain 5 to 10, or 5 to 6 ring atoms. Examples of monocyclic heteroaryl may include thiophenyl, furanyl, pyrrolyl, pyrazolyl, thiazolyl, isothiazolyl, oxazolyl, isooxazolyl, imidazolyl, triazolyl, oxadiazolyl, thiadiazolyl, tetrazolyl, pyridinyl, pyridazinyl, pyrimidinyl, pyrazinyl, triazinyl and similar groups thereto, but are not limited thereto. Examples of bicyclic heteroaryl may include indolyl, isoindolyl, indazolyl, indolizinyl, benzothiophenyl, benzofuranyl, benzimidazolyl, benzopyrazolyl, benzoxazolyl, benzisoxazolyl, benzthiazolyl, benzisothiazolyl, benzthiadiazolyl, benztriazolyl, quinolinyl, isoquinolinyl, quinoxalinyl, quinazolinyl, purinyl, phthalazinyl, pteridinyl, furopyridinyl, oxochromenyl, dioxoisoindolinyl, imidazopyridinyl, pyrrolopyridinyl, pyrrolopyrimidinyl, pyrazolopyridinyl and similar groups thereto, but are not limited thereto. In the present specification, heteroaryl optionally includes one fused with a cycloalkyl group (for example, pyrazolyl fused with cyclohexyl). In addition, heteroaryl may be a functional group in which the aromaticity of the ring is maintained by replacing the carbon of the ring with oxo, sulfanyldiene (=S), imino (=NH or =N($C_{1-6}$ alkyl)), and the like. For example, it may include pyridinonyl (pyridonyl), pyridazinonyl, pyrimidinonyl (pyrimidonyl), pyrazinonyl, and the like. When the heteroaryl contains N, B or P in the ring, N, B or P of the heteroaryl may be linked to another moiety.

As used herein, unless otherwise stated, the term "heterocycloalkyl" refers to a monocyclic or polycyclic, saturated or partially unsaturated ring system containing at least one heteroatom selected from B, N, O, S, P(=O), Si and P and having the specified number of ring elements (that is, 3- to 7-membered heterocycloalkyl refers to a heterocycloalkyl group having 3, 4, 5, 6 or 7 ring elements, including heteroatoms). The polycyclic heterocycloalkyl may also include a form in which two or more heterocycloalkyl rings are simply attached to each other (pendant) or bridged or condensed or spiro bonded. The heterocycloalkyl may contain 1 to 4 heteroatoms, 1 to 3 heteroatoms, 1 or 2 heteroatoms, or 1 heteroatom selected from N, O and S. In addition, the heterocycloalkyl may contain 5 to 10, 4 to 7, 5 or 6 ring atoms. For example, the heterocycloalkyl group includes azetidinyl, oxetanyl, thietanyl, pyrrolidinyl, pyrrolinyl, dihydrofuranyl, tetrahydrofuranyl (oxanyl), dihydrothiophenyl, tetrahydrothiophenyl, sulfolanyl, thianyl, dioxolanyl, imidazolinyl, imidazolidinyl, pyrazolinyl, pyrazolidinyl, thiazolinyl, thiazolidinyl, isothiazolinyl, isothiazolidinyl, oxazolinyl, oxazolidinyl, isoxazolinyl, isoxazolidinyl, triazolinyl, triazolidinyl, tetrazolinyl, tetrazolidinyl, pyranyl, dihydropyranyl, tetrahydropyranyl, thiopyranyl, tetrahydro 2H-thiopyranyl, dihydrothiopyranyl, dioxanyl, tetrahydrotriazinyl, hexahydrotriazinyl, morpholinyl, thiomorpholinyl, piperidinyl, dihydropyridinyl, tetrahydropyridinyl, piperazinyl, hexahydropyrimidinyl, tetrahydropyrimidinyl, dihydropyrimidinyl, dihydropyridazinyl, tetrahydropyridazinyl, tetrahydrooxazinyl, hexahydroazepinyl, perhydroazepinyl, perhydrooxepinyl, indolinyl, isoindolinyl, dihydrobenzimidazolyl, dihydrobenzofuranyl, dihydrobenzoxazolyl, dihydrobenzothiazolyl, chromanyl, isochromanyl, 3-oxabicyclo[2.1.1]hexanyl, 2-oxabicyclo[2.1.1]hexanyl, 2-azabicyclo[2.1.1]hexanyl, 3-azabicyclo[2.1.1]hexanyl, azabicyclo[2.2.1]heptanyl, 3-azabicyclo[3.2.1]heptanyl, 7-azabicyclo[4.1.0]-heptanyl, 2,5-diazabicyclo[2.2.1]heptanyl, 2-oxa-5-azabicyclo[2.2.1]heptanyl, tropanyl, 2-oxa-6-azaspiro[3.3]heptanyl, and N-oxide, sulfone or sulfoxide thereof, but is not limited thereto. In the present specification, heterocycloalkyl optionally includes one fused with a cycloalkyl group (for example, piperidinyl fused with cyclohexyl). When the heterocycloalkyl contains N, B or P in the ring, N, B or P of the heterocycloalkyl may be linked to another moiety.

As used herein to indicate a chemical bond between ring atoms, "- - - - - -" indicates that two atoms are bonded by a single bond or a double bond, and each atom may have as many H or substituents as its valence allows. For example, when - - - - - - is used to link these two ring carbon atoms, it represents -CH=CH- or $-CH_2-CH_2-$, and each H may be substituted with an appropriate substituent.

As used herein, the term "halogen" refers to an atom belonging to group 17 of the periodic table. The halogen atom includes fluorine, chlorine, bromine, iodine, and the like, and may be used interchangeably with the term "halo," which means a monovalent functional group composed of halogen.

As used herein, the term "cyano" refers to -CN, which is a functional group having a triple bond between a carbon atom and a nitrogen atom.

As used herein, the term "hydroxy" refers to a -OH functional group (hydroxyl group).

As used herein, the term "oxy" refers to a divalent functional group of -O-.

As used herein, the term "oxo" refers to a substituent having the structure =O, in which a double bond is present between the atom to which the substituent is attached and the oxygen atom.

As used herein, the term "carbonyl" refers to a divalent functional group of -C(=O)-.

As used herein, the term "acyl" refers to a functional group in which the carbon atom at the $1^{st}$ position of alkyl is substituted with oxo, and includes "formyl" and "alkylcarbonyl." For example, $C_{1-6}$ acyl is one in which the carbon atom at the 1st position of $C_{1-6}$ alkyl is substituted with oxo, and may include formyl (HC(O)-), acetyl ($CH_3C(O)$-), propionyl ($CH_3CH_2C(O)$-), butanoyl ($CH_3CH_2CH_2C(O)$-), pentanoyl ($CH_3CH_2CH_2CH_2CO$-), hexanoyl ($CH_3CH_2CH_2CH_2CH_2C(O)$-), and the like.

As used herein, the term "acyloxy" refers to a functional group in which acyl is bonded to one end of oxy, and includes "formyloxy" and "alkylcarbonyloxy." For example, $C_{1-3}$ acyloxy may include formyloxy, acetyloxy (acetoxy), propionyloxy, and the like.

As used herein, the term "carboxy" refers to -COOH.

As used herein, the term "sulfonyl" refers to a divalent functional group of $-S(O)_2-$. For example, $C_{1-6}$ alkylsulfonyl

may include methylsulfonyl, ethylsulfonyl, propylsulfonyl, butylsulfonyl, pentylsulfonyl, hexylsulfonyl, and the like.

As used herein, the term "amino" refers to $-NH_2$.

As used herein, the term "alkylamino" refers to a functional group in which one hydrogen of amino is substituted with alkyl. For example, $C_{1-6}$ alkylamino is -NH($C_1$-$C_6$ alkyl), and may include methylamino, ethylamino, propylamino, butylamino, and the like, but is not limited thereto.

As used herein, the term "dialkylamino" refers to a functional group in which two hydrogens of amino are each substituted with alkyl. In this case, the substituted alkyl may be the same or different from each other. For example, di($C_{1-6}$ alkyl)amino is -N($C_1$-$C_6$ alkyl)$_2$, and may include dimethylamino, diethylamino, dipropylamino, dibutylamino, ethylmethylamino, methylpropylamino, ethylpropylamino, and the like, but is not limited thereto.

As used herein, the term "acylamino" refers to a functional group in which the carbon atom at the 1$^{st}$ position of alkyl in alkylamino is substituted with oxo, and includes "formylamino" and "alkylcarbonylamino."

As used herein, the term "carbamoyl" refers to $-CONH_2$.

As used herein, the term "alkylcarbamoyl" refers to a functional group in which one hydrogen of carbamoyl is substituted with alkyl. For example, $C_{1-6}$ alkylcarbamoyl is -CONH($C_{1-6}$ alkyl), and may include $-CONHCH_3$, $-CONHCH_2CH_3$, $-CONHCH_2CH_2CH_3$, - $CONHCH_2CH_2CH_2CH_3$, and the like, but is not limited thereto.

As used herein, the term "dialkylcarbamoyl" refers to a functional group in which two hydrogens of carbamoyl are each substituted with alkyl. For example, $C_{1-6}$ alkylcarbamoyl is - CON($C_{1-6}$ alkyl)$_2$, and may include $-CON(CH_3)_2$, $-CON(CH_2CH_3)_2$, $-CON(CH_3)(CH_2CH_3)$, and the like, but is not limited thereto.

As used herein, the term "substituted" group refers to one in which one or more hydrogen atoms are replaced with one or more non-hydrogen atom groups, provided that valence requirements should be met and a chemically stable compound should occur from the substitution. In the present specification, unless explicitly stated as "unsubstituted," all substituents should be construed as being capable of being unsubstituted or substituted.

In the present specification, the "optionally substituted" moiety mentioned herein without limitation of a particular substituent may encompass a moiety unsubstituted or substituted with any substituent. For example, the "optionally substituted" moiety may refer to a moiety substituted with the following substituents:

(i) halogen, OH, CN, oxo, $NH_2$, NH($C_1$-$C_6$ alkyl), or N($C_1$-$C_6$ alkyl)$_2$;
(ii) $C_1$-$C_3$ alkyl optionally substituted with at least one substituent selected from the group consisting of halogen, OH, CN, oxo, $NH_2$, NH($C_1$-$C_6$ alkyl) and N($C_1$-$C_6$ alkyl)$_2$;
(iii) $C_1$-$C_3$ alkoxy optionally substituted with at least one substituent selected from the group consisting of halogen, OH, CN, oxo, $NH_2$, NH($C_1$-$C_6$ alkyl) and N($C_1$-$C_6$ alkyl)$_2$; or
(iv) $C_3$-$C_6$ cycloalkyl optionally substituted with at least one substituent selected from the group consisting of halogen, OH, CN, oxo, $NH_2$, NH($C_1$-$C_6$ alkyl) and N($C_1$-$C_6$ alkyl)$_2$.

In the present specification, when a combination of substituents is mentioned as one group, for example, arylalkyl, cycloalkylalkyl, or the like, the last-mentioned group generally contains the atom attached to the end of the molecule.

In the present specification,

"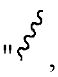,"

" "*," or "-" is used to indicate a position at which a substituent is bonded to the remaining moiety of the compound. For example, if - is indicated at the end of a substituent, it means that the end is attached to the remaining moiety of the compound. In addition, when two or more substituents are linked by "-," it means that the substituent immediately before "-" is bonded to a substitutable atom of the substituent immediately after "-".

As used herein, the term "solvate" may refer to a compound of the present invention or a salt thereof comprising a stoichiometric or non-stoichiometric amount of a solvent bound by noncovalent intermolecular forces. Preferred solvents therefor may be solvents that are volatile, nontoxic, and/or suitable for administration to humans.

As used herein, the term "stereoisomer" may refer to a compound of the present invention or a salt thereof that has the same chemical formula or molecular formula but is optically or sterically different, and specifically, may be a diastereomer, an enantiomer or a geometric isomer.

In some embodiments, the compound of the present invention may be in the form of a racemate, a single enantiomer, a mixture of enantiomers, a single diastereomer, a mixture of diastereomers, and the like, containing one or more asymmetric centers. In one embodiment, due to the limited rotation or nature of the asymmetric center, the compound of the present invention may be in the form of an enantiomer or a diastereomer.

When two or more asymmetric centers are present in the compound of the present invention, several diastereomers and enantiomers of the chemical structures disclosed herein may exist, and pure isomers, separated isomers, partially pure isomers, racemic mixtures or the like are all intended to fall within the scope of the present invention.

Purification of the isomers and separation of a mixture of the isomers may be achieved by standard techniques known in the art. For example, a diastereomeric mixture may be separated into its respective diastereomers by a chromatographic process or crystallization, and a racemate may be separated into its respective enantiomers by resolution or a chromatographic process on a chiral phase.

The compound of the present invention may be used in the form of a pharmaceutically acceptable salt derived from an inorganic acid or organic acid, and for example, the salt may be a salt derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, toluenesulfonic acid, or the like.

A pharmaceutically acceptable salt of the compound may be prepared by dissolving the compound of formula I in a water-miscible organic solvent, such as acetone, methanol, ethanol, acetonitrile, or the like, and adding an excess of an organic acid or adding an aqueous acid solution of an inorganic acid, and then precipitating or crystallizing. Subsequently, after evaporating the solvent or an excess of acid from this mixture, it may be prepared by drying to obtain an addition salt or by suction filtration of the precipitated salt.

## General preparation method of compound

The compound according to the present invention can be prepared through chemical modifications well known to one of ordinary skill in the art of organic/pharmaceutical chemistry according to the method representatively shown below.

The following general reaction scheme is a general illustration of a representative preparation method of the compound of formula I. One of ordinary skill in the art will be able to easily prepare the compound of formula I by appropriately selecting a starting material, a reaction temperature, a reaction condition, a catalyst, a solvent, a treatment method, and the like suitable for the desired compound, based on the preparation methods specifically disclosed in the examples herein. Hereinafter, in Reaction Schemes 1 to 9, the representation of each substituent of Formula I is the same as that of the substituent at the corresponding position in Formula I unless otherwise limited. In addition, in Reaction Schemes 1 to 9, the same variables are defined the same, and the descriptions of repeated definitions may be omitted.

In one aspect, a compound of Formula I can be prepared by reacting Intermediate a and Intermediate b according to the method of Reaction Scheme 1 below.

[Reaction Scheme 1]

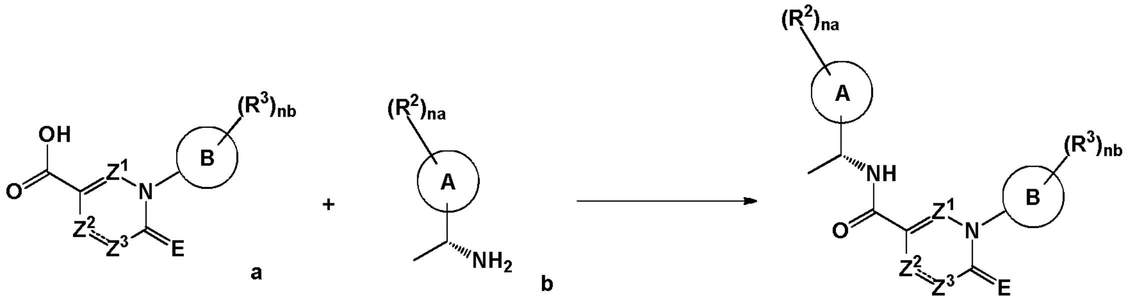

Compound of Formula 1

(In Reaction Scheme 1, na and nb are each independently appropriate integers satisfying the number of $R^2$ and $R^3$ defined in Formula I above.)

For example, a compound of Formula I can be prepared by coupling Intermediate Compound a with Intermediate Compound b through an amide coupling reaction using HATU. In one embodiment, when $R^2$ is an $NO_2$ group, the compound of Formula I having an $NH_2$ substituent on ring B can be prepared by reduction to an $NH_2$ group under reducing reaction conditions.

One of ordinary skill in the art can replace the reaction reagent used in Reaction Scheme 1 with various reagents for performing the amide coupling reaction based on common knowledge in the related field, and accordingly, will be able to select reaction conditions such as appropriate reaction time and reaction temperature. In one embodiment, Intermediates a and b may be reacted in HATU, TEA and DMF at about 20 °C to about room temperature for about 2 hours to about 3 hours. Alternatively, Intermediates a and b may be reacted in HATU, DIEA and DMF at about 10 °C to

about 30 °C for about 2 hours to about 15 hours. Alternatively, Intermediates a and b may be reacted in EDCI, HOBT, DMAP and DCM at about 10 °C to about 20 °C for about 10 hours to about 15 hours. Alternatively, Intermediates a and b may be reacted in TEA, HOBT, EDCI and DCM at about 20 °C to about 30 °C for about 2 hours to about 5 hours. Alternatively, Intermediates a and b may be reacted in DIEA, HOBT, EDCI and DMF at about 15°C to about 25°C for about 2 hours to about 15 hours.

In one embodiment, a compound of Formula I can be prepared according to the reaction of Reaction Scheme 1A below.

[Reaction Scheme 1A]

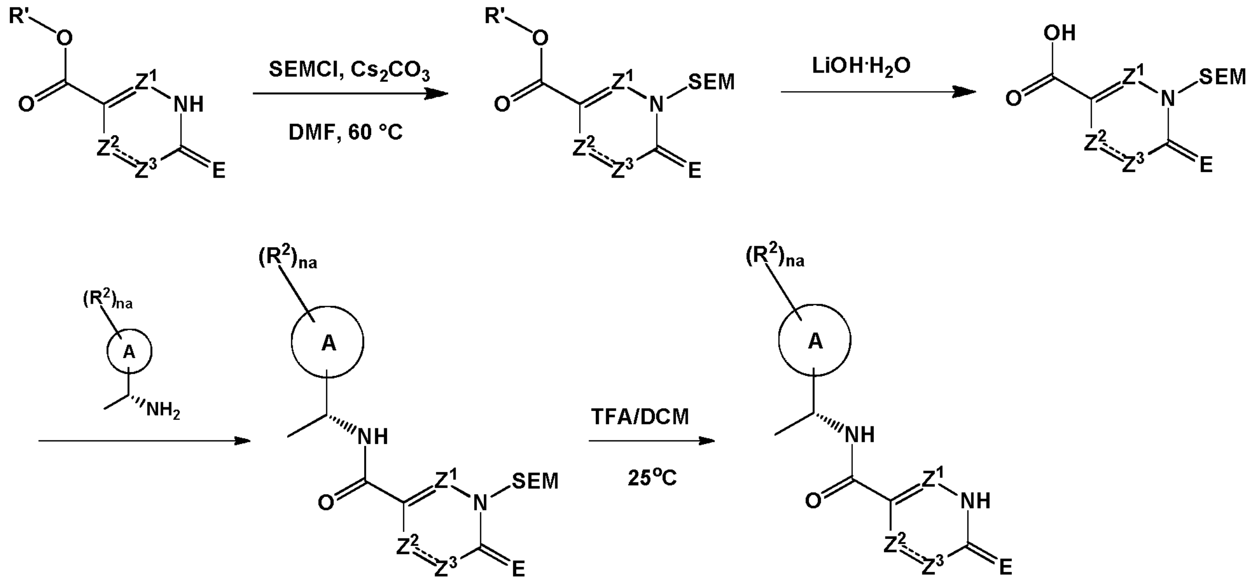


(In Reaction Scheme 1A, R' is alkyl.)

For example, according to Reaction Scheme 1A, a compound of Formula I in which the nitrogen atom of the ring is unsubstituted can be prepared by protecting the nitrogen atom in the ring of Intermediate a with SEM, and reacting with Intermediate b, and then removing the SEM.

In one embodiment, a compound of Formula I can be prepared according to the reaction of Reaction Scheme 1B below.

[Reaction Scheme 1B]

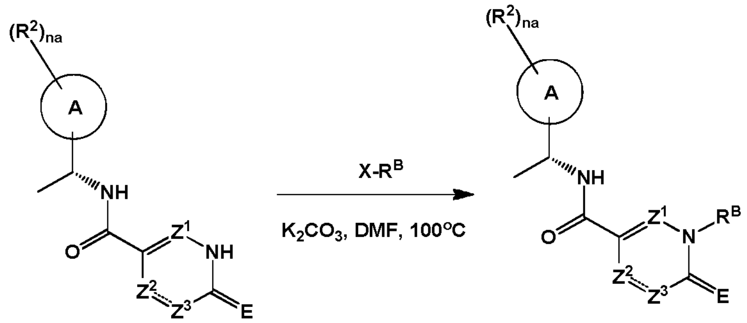


(In Reaction Scheme 1B, $R^B$ is alkyl optionally substituted with, for example, halogen, hydroxy, alkoxy, amino, alkylamino, dialkylamino, aryl or cycloalkyl.)

For example, $R^B$ may be introduced into the nitrogen atom by reacting a compound prepared according to Reaction Scheme 1A with a halide of $R^B$.

In one embodiment, a compound of Formula I can be prepared according to the method of Reaction Scheme 1C below.

[Reaction Scheme 1C]

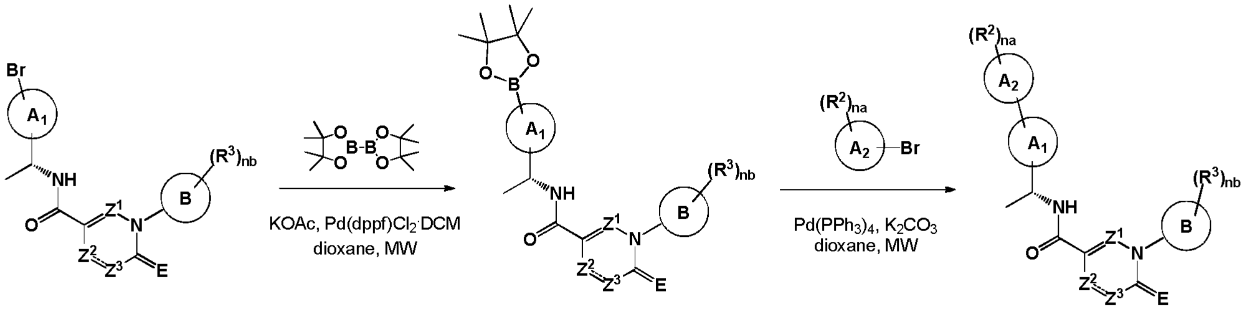


(In Reaction Scheme 1C, $A^1$ and $A^2$ are structures corresponding to $Cy^1$ and $Cy^2$ of Formula I, respectively.)

For example, according to Reaction Scheme 1C, a compound of Formula I can be prepared by coupling a starting material in which ring $A_1$ is halogenated with bis(pinacolato)diborane under an appropriate catalyst (for example, $Pd(dppf)Cl_2$) to synthesize a pinacolborane compound, and then coupling with a halide of ring $A_2$.

In one embodiment, a compound of Formula I can be prepared according to the method of Reaction Scheme 1D below.

[Reaction Scheme 1D]

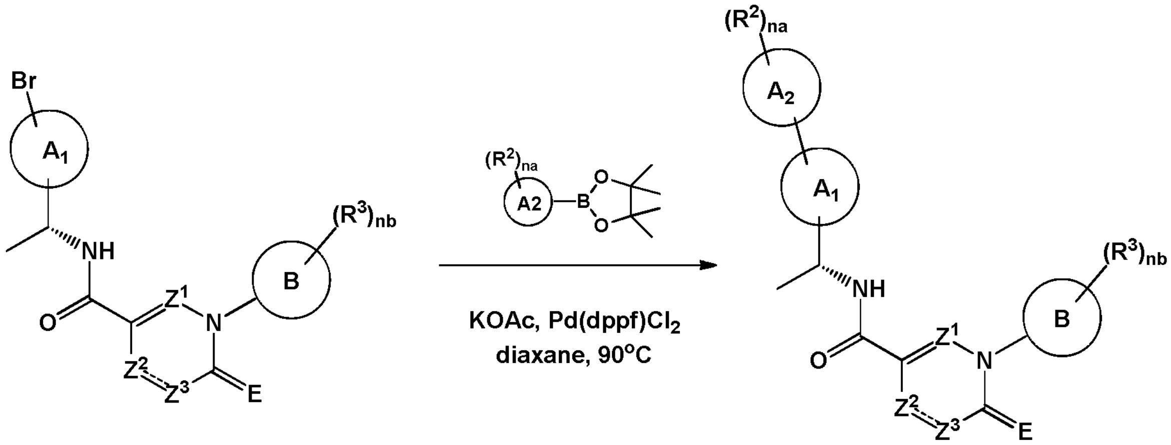


For example, according to Reaction Scheme 1D, a compound of Formula I can be prepared by coupling a starting material in which ring $A_1$ is halogenated with a pinacolborane or boronic acid derivative of ring $A_2$ under an appropriate catalyst (for example, $Pd(dppf)Cl_2$).

In one embodiment, a compound of Formula I can be prepared according to the method of Reaction Scheme 1E below.

[Reaction Scheme 1E]

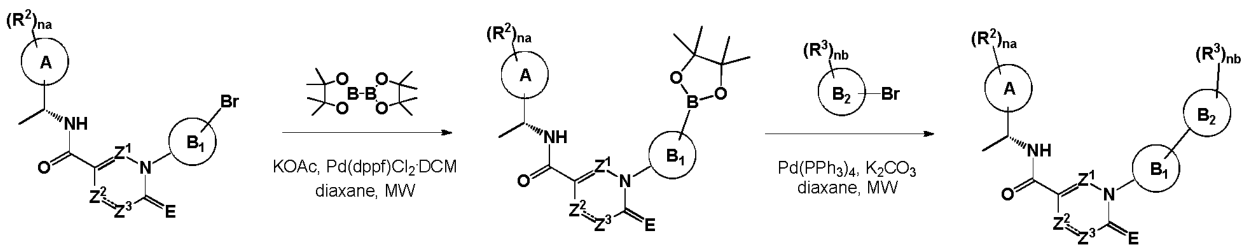


(In Reaction Scheme 1E, $B_1$ and $B_2$ are structures corresponding to $Cy^3$ and $Cy^4$ of Formula I, respectively.)

For example, according to Reaction Scheme 1E, a compound of Formula I can be prepared by coupling a

starting material in which ring $B_1$ is halogenated with bis(pinacolato)diborane under an appropriate catalyst (for example, Pd(dppf)$Cl_2$) to synthesize a pinacolborane compound, and then coupling with a halogenated derivative of ring $B_2$.

In one embodiment, a compound of Formula I can be prepared according to the method of Reaction Scheme 1F below.

[Reaction Scheme 1F]

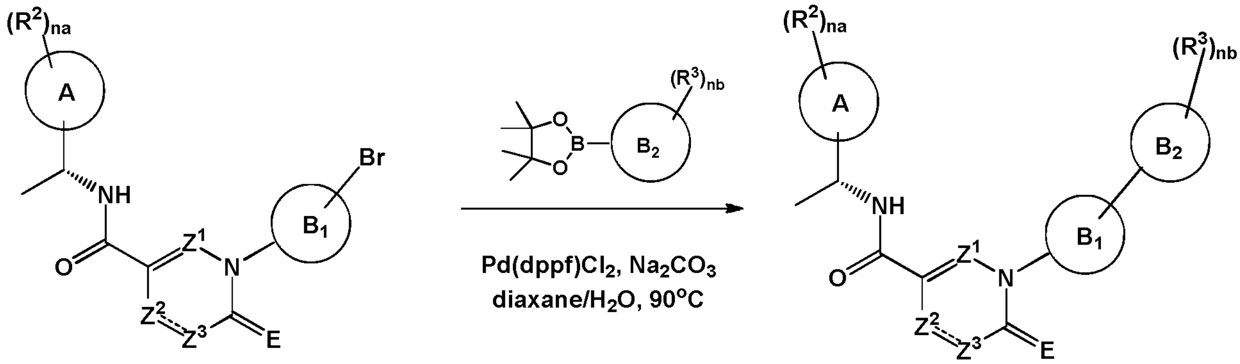


For example, according to Reaction Scheme 1F, a compound of Formula I can be prepared by coupling a starting material in which ring $B_1$ is halogenated with a pinacolborane or boronic acid compound of ring $B_2$ under an appropriate catalyst (for example, Pd(dppf)$Cl_2$).

In one embodiment, the $R^1$ group of Formula I may be introduced after Intermediate b is coupled with Intermediate a. For example, as illustrated in Reaction Scheme 2 below, when $R^1$ is CN, a compound of Formula I in which CN is substituted can be prepared using CuCN under an appropriate solvent (for example, N-methyl-2-pyrrolidone) after coupling an appropriate halogenated Intermediate a to which ring B is attached with Intermediate b in an appropriate solvent (for example, DCM, toluene), if necessary, in the presence of an appropriate catalyst (for example, $AlMe_3$).

[Reaction Scheme 2]

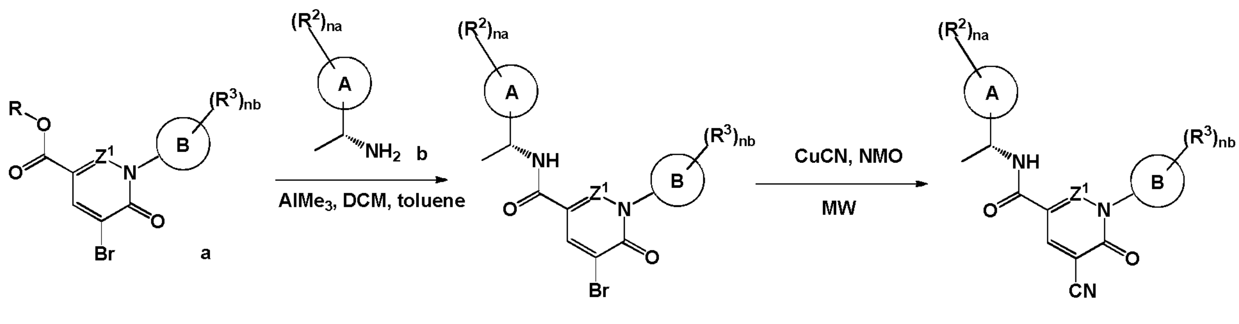


Compound of Formula 1

(In Reaction Scheme 2, R is H or alkyl.)

In one embodiment, Intermediate a in which $R^1$ is substituted can be prepared according to the method of Reaction Scheme 2A below.

[Reaction Scheme 2A]

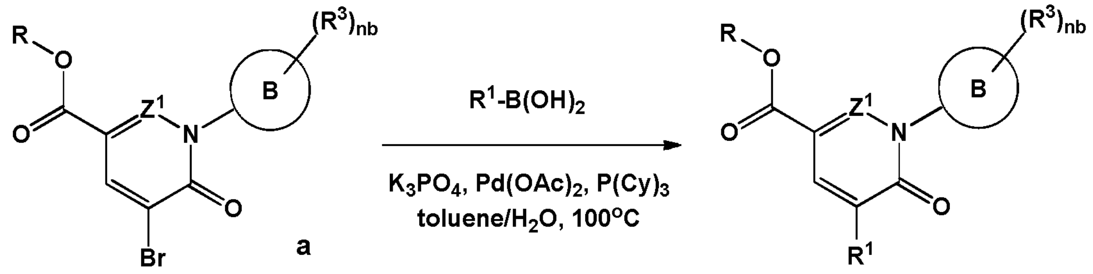


(In Reaction Scheme 2A, R is H or alkyl.)

For example, according to Reaction Scheme 2A, Intermediate a into which $R^1$ is introduced can be prepared by reacting an appropriate halogenated Intermediate a to which ring B is attached with a boronic acid compound of $R^1$.

In another embodiment, Intermediate a in which $R^1$ is alkyl can be prepared according to the method of Reaction Scheme 2B below.

[Reaction Scheme 2B]

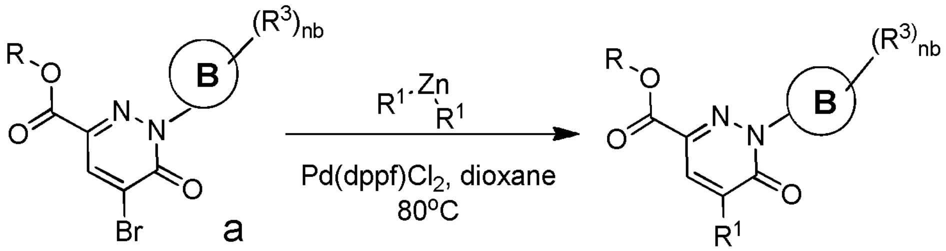


(In Reaction Scheme 2B, $R^1$ is alkyl.)

For example, Intermediate a into which an alkyl group is introduced as $R^1$ can be prepared by reacting an appropriate halogenated Intermediate a to which ring B is attached with a dialkyl zinc (Zn Negishi reaction) in an appropriate solvent (for example, THF, dioxane, etc.) in the presence of an appropriate catalyst (for example, $Pd(PPh_3)_4$, $Pd(dppf)Cl_2$, etc.).

In one embodiment, Intermediate a can be prepared according to the method of Reaction Scheme 3 below.

[Reaction Scheme 3]

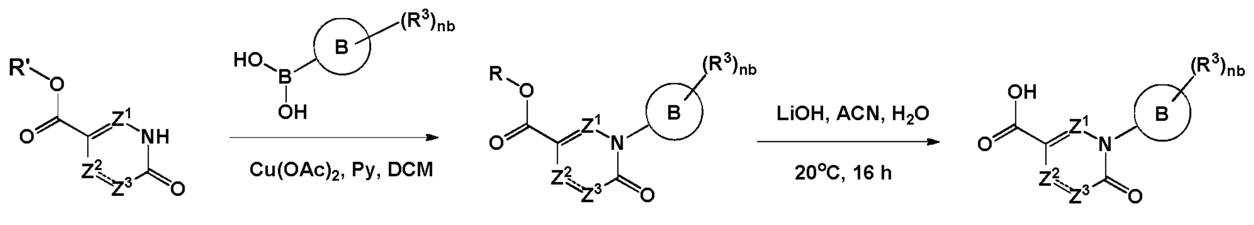


For example, Intermediate a can be prepared by dissolving an appropriate starting material in a solvent (for example, DCM), adding an appropriate amount of a base (for example, pyridine) and $Cu(OAc)_2$, then reacting with a boronic acid or 4,4,5,5-tetramethyl-1,3,2-dioxaborolane (pinacolborane) derivative of ring B, and hydrolyzing an ester group by addition of an appropriate base (for example, LiOH).

In another embodiment, Intermediate a can be prepared according to the method of Reaction Scheme 4 below.

[Reaction Scheme 4]

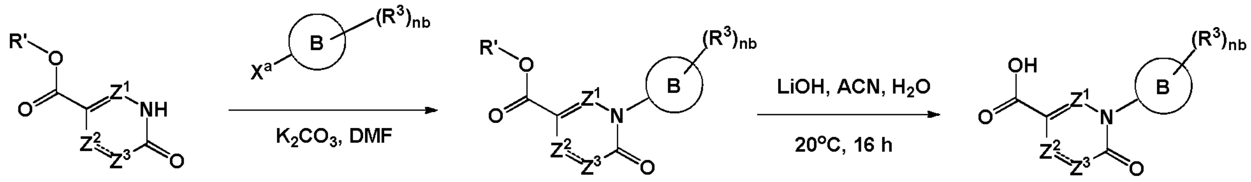


(In Reaction Scheme 4, $X^a$ is halogen, methylsulfonyloxy or trifluoromethylsulfonyloxy.)

For example, Intermediate a can be prepared by dissolving an appropriate starting material in a solvent (for example, DMF), adding an appropriate amount of a base (for example, $K_2CO_3$), then reacting with a halide, methylsulfonate or trifluoromethylsulfonate derivative of ring B, and hydrolyzing an ester group by addition of an appropriate base (for example, LiOH).

In another embodiment, Intermediate a can be prepared according to the method of Reaction Scheme 5 below.

[Reaction Scheme 5]

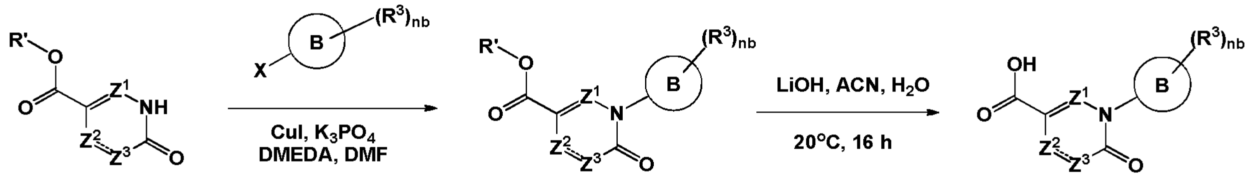


(In Reaction Scheme 5, X is halogen.)

For example, Intermediate a can be prepared by dissolving an appropriate starting material in a solvent (for example, DMF), adding an appropriate amount of DMEDA, $K_3PO_4$, and CuI, then reacting with a halide compound of ring B, and hydrolyzing an ester group by addition of an appropriate base (for example, LiOH).

In another embodiment, Intermediate a can be prepared according to the method of Reaction Scheme 6 below.

[Reaction Scheme 6]

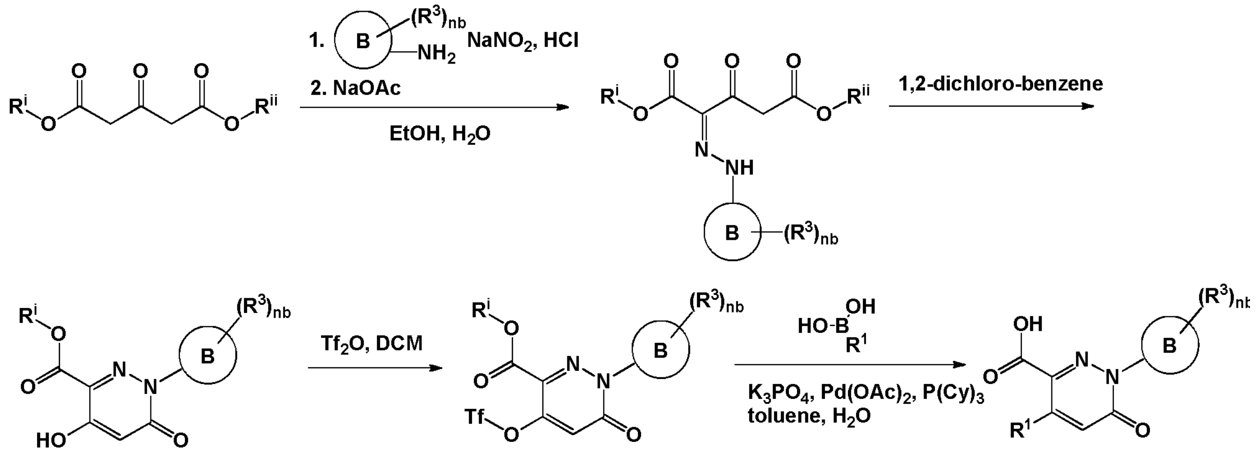


(In Reaction Scheme 6, $R^i$ and $R^{ii}$ are each alkyl.)

For example, Intermediate a can be prepared by treating a mixture of an aminated ring B compound, water and HCl with $NaNO_2$, adding to NaOAc and 3-oxopentandioate in an appropriate solvent (for example, EtOH, water) to form a hydrazone compound, then stirring in an appropriate solvent (for example, 1,2-dichlorobenzene) to form a hydroxy group substituted dihydropyridazinone ring, then adding $Tf_2O$ in an appropriate solvent (for example, DCM) to introduce a trifluoromethylsulfonyloxy group, and reacting with a boronic acid compound of $R^1$.

In one embodiment, Intermediate a can be prepared according to the method of Reaction Scheme 6A below.

[Reaction Scheme 6A]

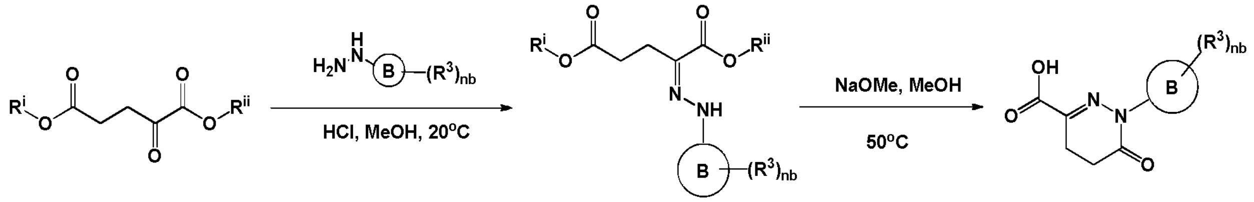


For example, Intermediate a can be prepared by reacting a hydrazinated ring B compound with 2-oxopentandioate in the presence of MeOH and HCl to form a hydrazone compound, and then stirring under NaOMe and MeOH to form a tetrahydropyridazinone ring.

In one embodiment, Intermediate a can be prepared according to the method of Reaction Scheme 6B below.

[Reaction Scheme 6B]

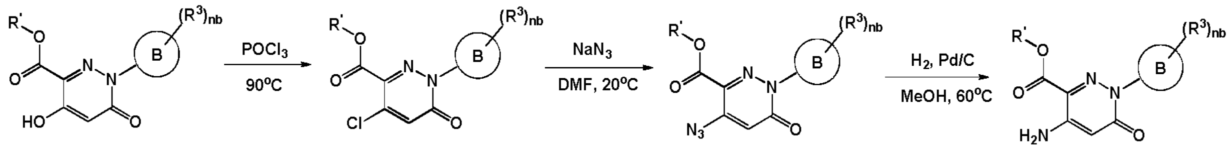


For example, according to Reaction Scheme 6B, Intermediate a into which an amino group is introduced as $R^1$ can be prepared by sequentially reacting a hydroxy substituted dihydropyridazinone ester compound with $POCl_3$ and $NaN_3$ to change a hydroxy group to a chloro group, and then to an azido group, and reducing the azido group under a Pd/C catalyst.

In one embodiment, Intermediate a can be prepared according to the method of Reaction Scheme 6C below.

[Reaction Scheme 6C]

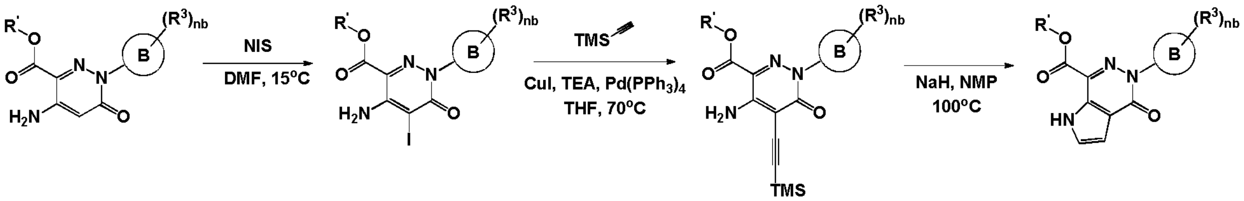


For example, a compound into which a trimethylsilylethynyl group is introduced can be synthesized by reacting Intermediate a prepared according to Reaction Scheme 6B with NIS to introduce an iodo group, and coupling with ethynyl(trimethyl)silane. Thereafter, Intermediate a having a pyrrolodihydropyridazinone core can be prepared through a cyclization reaction under NaH and NMP conditions.

In one embodiment, Intermediate a can be prepared according to the method of Reaction Scheme 6D below.

[Reaction Scheme 6D]

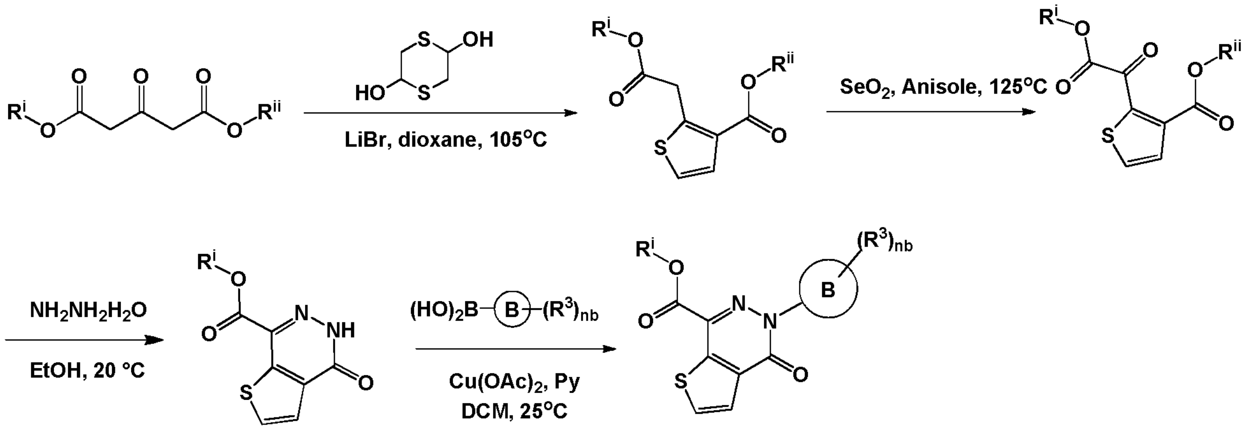


For example, according to Reaction Scheme 6D, a 3-oxopentandioate compound may be reacted with 1,4-dithiane-2,5-diol in the presence of LiBr to form a thiophene diester compound. Intermediate a having a thienodihydropyridazinone core can be prepared by reacting a thiophene diester compound with $SeO_2$ in an anisole solvent to additionally introduce an oxo group to the thiophene diester compound, and performing cyclization reaction with hydrazine, and then reacting with a boronic acid derivative of ring B.

In one embodiment, Intermediate a can be prepared according to the method of Reaction Scheme 6E below.

[Reaction Scheme 6E]

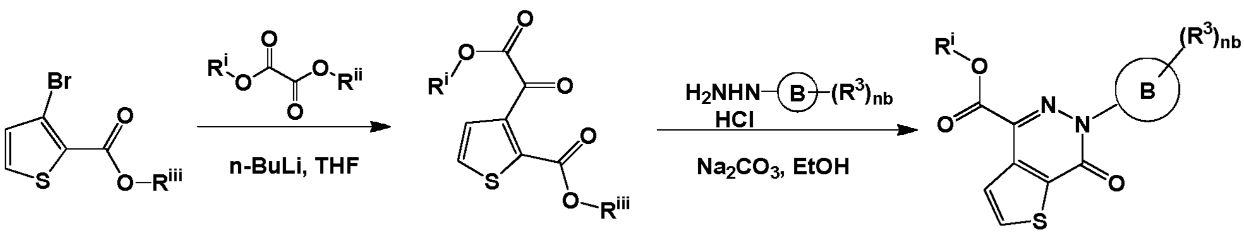


(In Reaction Scheme 6E, $R^i$ to $R^{iii}$ are alkyl.)

For example, according to Reaction Scheme 6E, Intermediate a having a thienodihydropyridazinone core can be prepared by reacting a brominated thiophene ester compound with oxalate in the presence of n-BuLi to synthesize a thiophene oxodiester compound, and then reacting with a ring B compound substituted with hydrazine.

In another embodiment, Intermediate a in which $R^1$ is alkyl can be prepared according to the method of Reaction Scheme 7 below.

[Reaction Scheme 7]

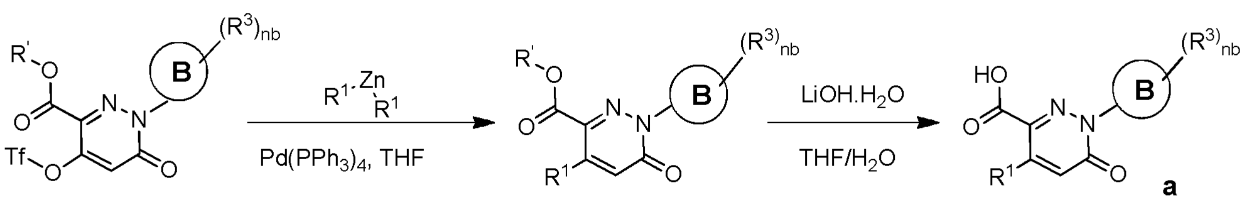


a

Intermediate a can be prepared by reacting a compound into which a trifluoromethylsulfonyloxy group is introduced, which is prepared in the method of Reaction Scheme 6, with a dialkyl zinc (Zn Negishi reaction) in an appropriate solvent (for example, THF, dioxane, etc.) in the presence of appropriate catalyst (for example, $Pd(PPh_3)_4$, $Pd(dppf)Cl_2$, etc.) to introduce an alkyl group into a dihydropyridazinone ring, and then hydrolyzing an ester group by addition of an appropriate base (for example, LiOH).

In one embodiment, Intermediate a can be prepared according to the method of Reaction Scheme 7A below.

[Reaction Scheme 7A]

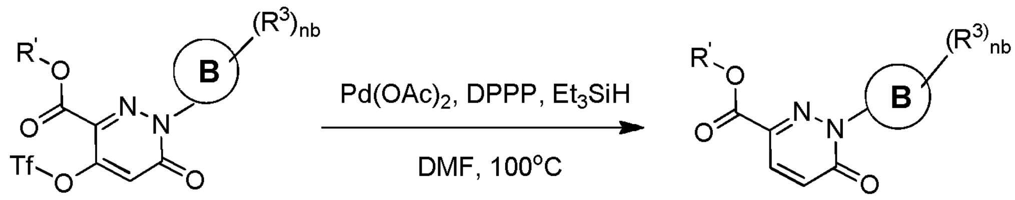


According to Reaction Scheme 7A, a compound into which a trifluoromethylsulfonyloxy group is introduced may be reacted with DPPP and $Et_3SiH$ in an appropriate solvent (for example, DMF) in the presence of an appropriate catalyst (for example, $Pd(OAc)_2$) to remove a trifluoromethylsulfonyloxy group.

In one embodiment, Intermediate a can be prepared according to the method of Reaction Scheme 8 below.

[Reaction Scheme 8]

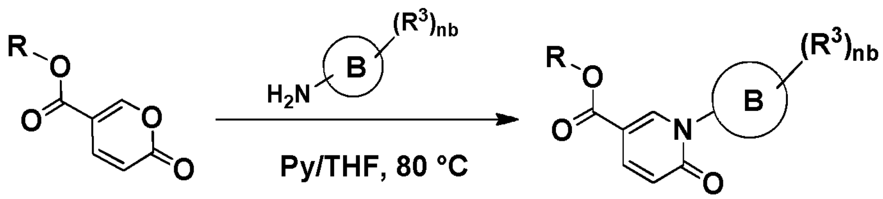


For example, according to Reaction Scheme 8, Intermediate a having a pyridinone core can be prepared by reacting a coumalate compound with an amine compound of ring B in the presence of pyridine.

In one embodiment, Intermediate b can be prepared according to the method of Reaction Scheme 9 below.

[Reaction Scheme 9]

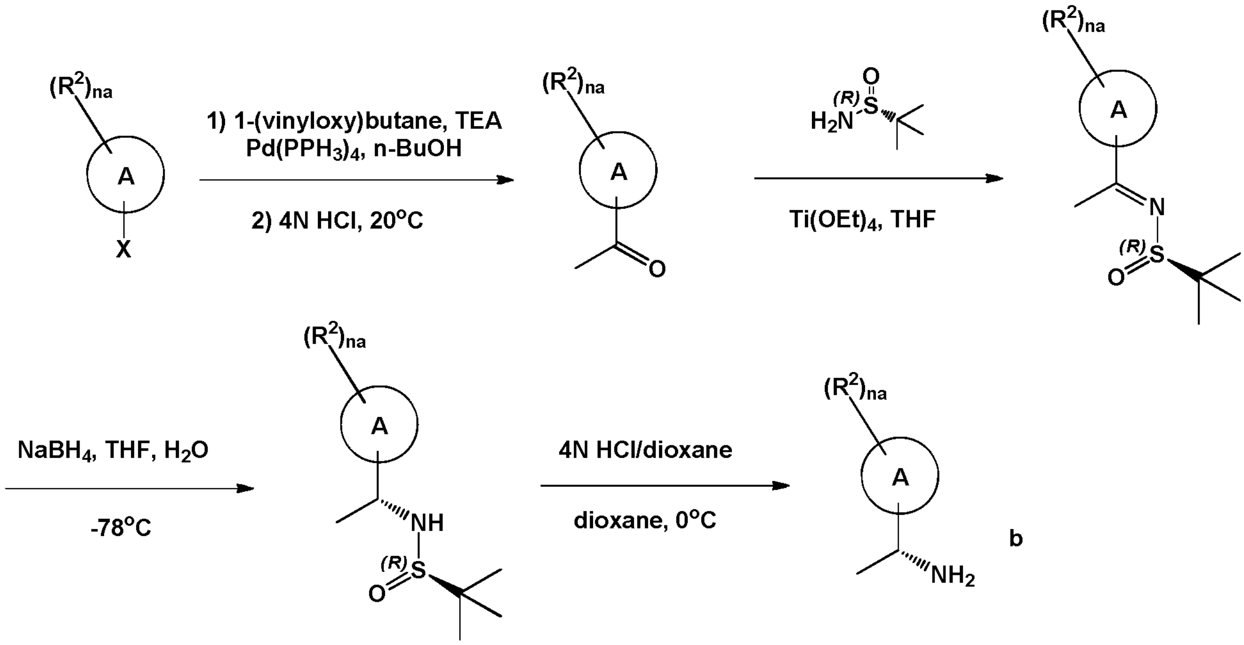


b

For example, a halogenated ring A compound is reacted with 1-vinyloxybutane or tributyl(1-ethoxyvinyl)stannane under Heck reaction conditions, followed by treatment with an acid to obtain an acetylated ring A compound. The reaction may be performed in the presence of $Pd(PP_3)_4$ or $Pd(PP_3)_2Cl_2$, and a solvent such as TEA, butanol, or dioxane may be used. Thereafter, the acetylated ring A compound may be reacted with tert-butyl sulfinamide having an (R) orientation in the presence of titanium alkoxide, and the imine bond may be reduced to an amine bond, and treated with an acid to prepare Intermediate b.

In one embodiment, when $R_2$ is an alkylsilane group, Intermediate b can be prepared according to the method of Reaction Scheme 10 below.

[Reaction Scheme 10]

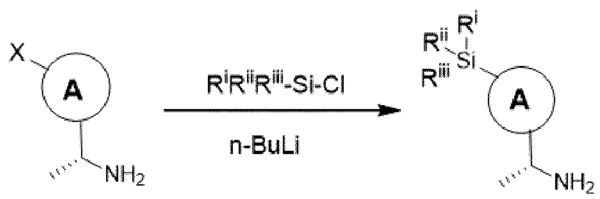


(In Reaction Scheme 10, $R^i$, $R^{ii}$ and $R^{iii}$ are each an alkyl group, and two of $R^i$, $R^{ii}$ and $R^{iii}$ may be optionally linked to each other to form cycloalkyl.)

For example, a halogenated ring A compound may be reacted with an appropriate alkyl silane halide compound in the presence of n-BuLi to introduce an alkylsilane group into ring A.

**Medicinal use, pharmaceutical composition, and administration method**

In another aspect, there is provided a pharmaceutical composition for preventing or treating a SOS 1 mediated disease, comprising the compound of Formula I, stereoisomer, solvate, or pharmaceutically acceptable salt. The compound of Formula I, stereoisomer, solvate, pharmaceutically acceptable salt are as described above.

In the present specification, the term "preventing" or "prevention" refers to preventing a disease, for example, preventing a disease, condition or disorder in a subject who may be predisposed to the disease, condition or disorder but has not yet experienced or exhibited the pathology or signs of the disease.

As used herein, the term "treating" or "treatment" refers to inhibiting a disease, for example, inhibiting a disease, condition or disorder in a subject who experiences or exhibits the pathology or signs of the disease, condition or disorder, i.e., preventing further development of the pathology and/or signs, or ameliorating the disease, for example, ameliorating the disease, condition or disorder in a subject who experiences or exhibits the pathology or signs of the disease, condition or disorder, i.e., reversing the pathology and/or signs, for example, reducing the severity of the disease.

The SOS1 mediated disease may include a disease that can be prevented or treated by inhibiting the interaction between SOS1 and RAS family proteins, or between SOS1 and RAC1. The SOS1 mediated disease may include a disease associated with the abnormal activity of SOS1 and/or RAS family proteins. The SOS1 mediated disease may be, for example, cancer. The cancer may be, for example, pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer or sarcoma. In one embodiment, the cancer may be pancreatic cancer, lung cancer (for example, non-small cell lung cancer), biliary tract cancer or colorectal cancer.

The cancer may be, for example, a cancer dependent on the RAS family and the MAPK signaling pathway. The cancer may include, for example, cancer having mutation of proteins or genes, gene amplification and/or overexpression in the RAS family and MAPK signaling pathway, e.g., KRAS, NRAS, HRAS, receptor tyrosine kinases (for example, EGFR, ErbB2, ErbB3, ErbB4, PDGFR-A/B, FGFR1/2/3, IGF1R, INSR, ALK, ROS, TrkA, TrkB, TrkC, RET, c-MET, VEGFR1/2/3, AXL), GAP (for example, NF1) and SOS1 (for example, mutation, amplification or overexpression of RAF, MEK). In addition, the cancer may be a RAC1 dependent cancer.

The SOS1 mediated disease may be, for example, a disease associated with dysregulation of RAS family protein pathways, i.e., RASopathy. The RASopathy may include neurofibromatosis type 1 (NF1), Noonan syndrome, Noonan syndrome with multiple lentigines (NSML, also referred to as Leopard syndrome), capillary malformation-arteriovenous malformation syndrome (CM-AVM), Costello syndrome, CFC syndrome (Cardio-Facio-Cutaneous syndrome), Legius syndrome (also referred to as NF1-like syndrome) or hereditary gingival fibromatosis.

When used in the treatment of cancer, the compound of the present invention may be administered alone or in combination with other anticancer therapies, such as radiation therapy, taxane derivatives (for example, paclitaxel, docetaxel), platinum compounds (for example, cisplatin, carboplatin), antimetabolites (for example, 5-FU, gemcitabine, cytarabine), CDK4/6 inhibitors (for example, abemaciclib, palbociclib), immunotherapeutic agents (for example, anti-CTLA4 antibody, anti-PD1 antibody), angiogenesis inhibitors (for example, bevacizumab), topoisomerase inhibitors (for example, irinotecan), ERK inhibitors (for example, ulixertinib), MDM2 inhibitors, PARP inhibitors, MCL-1 inhibitors, mTOR inhibitors (for example, rapamycin, temsirolimus), BET inhibitors, CDK9 inhibitors, IGF1/2 or IGF1-R inhibitors, PIK

inhibitors, EGFR inhibitors (for example, apatinib, osimertinib, cetuximab), ErbB2 (HER2) inhibitors (for example, trastuzumab), ALK inhibitors (for example, crizotinib, alectinib), MEK inhibitors (for example, trametinib), BCR-ABL inhibitors (for example, imatinib, nilotinib, dasatinib), FGFR1, FGFR2 or FGFR3 inhibitors (nintedanib), ROS1 inhibitors (for example, crizotinib, entrectinib), c-MET inhibitors, AXL inhibitors, NTRK1 inhibitors, RET inhibitors, KRAS G12C inhibitors (for example, sotorasib), SHP2 inhibitors, mutBRAF inhibitors or pan-RAF inhibitors, and the like.

According to one embodiment, a compound of Formula I can be used in the treatment of a disease associated with the abnormal activity of SOS1 or RAS family proteins, or dysregulation of RAS family protein pathways by inhibiting the interaction between SOS1 and RAS family proteins, or between SOS1 and RAC 1.

In one embodiment, the pharmaceutical composition may comprise conventional pharmaceutically acceptable carriers, excipients or additives. The pharmaceutical composition may be formulated according to a conventional method, and may be prepared as various oral dosage forms such as tablets, pills, powders, capsules, syrups, emulsions, microemulsions, or parenteral dosage forms such as intramuscular, intravenous or subcutaneous dosage form. The pharmaceutical composition may be a single composition or separate compositions. The pharmaceutical composition comprises the compound, stereoisomer, solvate, or pharmaceutically acceptable salt according to one aspect as an active ingredient of the pharmaceutical composition.

When the pharmaceutical composition is prepared in the form of an oral formulation, examples of additives or carriers used may include cellulose, calcium silicate, corn starch, lactose, sucrose, dextrose, calcium phosphate, stearic acid, magnesium stearate, calcium stearate, gelatin, talc, surfactant, suspending agent, emulsifying agent, diluent, and the like. When the pharmaceutical composition of the present invention is prepared in the form of an injection, the additive or carrier may include water, saline, aqueous glucose solution, similar aqueous sugar solution, alcohol, glycol, ether (for example, polyethylene glycol 400), oil, fatty acid, fatty acid ester, glyceride, surfactant, suspending agent, emulsifying agent, and the like.

The dosage of the pharmaceutical composition is an amount effective for treatment or prevention of a subject or patient, and may be administered orally or parenterally as desired. It may be administered in one to several divided doses to be administered in an amount of 0.01 to 1000 mg, more specifically 0.1 to 300 mg per kg of body weight daily based on the active ingredient when administered orally, or in an amount of 0.01 to 100 mg, more specifically 0.1 to 50 mg per kg of body weight daily based on the active ingredient when administered parenterally. The dose to be administered to a specific subject or patient should be determined in light of several related factors such as body weight, age, sex, health condition of the patient, diet, administration time, administration method, the severity of the disease, and the like, and it should be understood that it may be appropriately increased or decreased by a specialist. The above dosage is not intended to limit the scope of the present invention in any way. A physician or veterinarian of ordinary skill in the art may readily determine and prescribe the required effective amount of the pharmaceutical composition. For example, by a physician or veterinarian, a dose of the compound of the present invention used in a pharmaceutical composition may start at a level lower than that required to achieve the desired therapeutic effect, and may gradually increase until the desired effect is achieved.

In one embodiment, the pharmaceutical composition includes within its scope a pharmaceutical composition comprising, as an active ingredient, a therapeutically effective amount of at least one of the compounds according to one embodiment, alone or in combination with a pharmaceutical carrier. The term "therapeutically effective amount" or "effective amount" refers to an amount sufficient to produce a beneficial or desired clinical result, for example, an amount sufficient to alleviate, ameliorate, stabilize, reverse, slow or delay the progression of a disease.

Optionally, the compound according to one embodiment may be administered alone, in combination with the compound according to another embodiment, or simultaneously, separately, or sequentially in combination with one or more other therapeutic agents, for example, an anticancer agent or other pharmaceutically active substances. Examples of the anticancer agent that can be administered in combination are as described above.

In another aspect, there is provided a method for preventing or treating a SOS1 mediated disease, comprising administering to a subject a compound of Formula I, a solvate, stereoisomer or pharmaceutically acceptable salt thereof, or the pharmaceutical composition comprising the same.

Among the terms or elements mentioned in the description of the method, the same as those already mentioned are the same as described above.

The administration may be oral or parenteral administration. It may be administered in one to several divided doses to be administered in an amount of 0.01 to 1000 mg, more specifically 0.1 to 300 mg per kg of body weight daily based on the active ingredient when administered orally, or in an amount of 0.01 to 100 mg, more specifically 0.1 to 50 mg per kg of body weight daily based on the active ingredient when administered parenterally. The dose to be administered to a specific subject or patient should be determined in light of several related factors such as body weight, age, sex, health condition of the patient, diet, administration time, administration method, the severity of the disease, and the like, and it may be appropriately increased or decreased by a specialist.

As used herein, the term "subject" refers to a subject in need of treatment for a disease, and more specifically means a mammal such as a human or non-human primate, a mouse, a dog, a cat, a horse, and a cow.

In another aspect, there is provided a medicinal use of the compound of Formula I, solvate, stereoisomer or pharmaceutically acceptable salt thereof for the prevention or treatment of a SOS1 mediated disease; or a use of the compound of Formula I, solvate, stereoisomer or pharmaceutically acceptable salt thereof for the manufacture of a medicament for the prevention or treatment of a SOS1 mediated disease.

Among the terms or elements mentioned in the description of the method or use, the same as those already mentioned are the same as described above.

**Effects of Invention**

The compound of Formula I, solvate, stereoisomer or pharmaceutically acceptable salt thereof has an effective inhibitory activity against SOS1, in particular, inhibits the interaction between SOS1 and RAS family proteins, or between SOS1 and RAC1, and thus, it is useful for the prevention or treatment of a SOS1 mediated disease, specifically a disease associated with the abnormal activity of SOS1 and/or RAS family proteins.

**Detailed Description for Carrying out the Invention**

Hereinafter, the present invention will be described in more detail by way of the following examples. However, the following examples are only for illustrating the present invention, and the scope of the present invention is not limited thereto.

**[Preparation Examples]**

**Preparation Example 1: 6-oxo-1-phenyl-pyridazine-3-carboxylic acid**

Step 1: Synthesis of methyl 6-oxo-1-phenyl-pyridazine-3-carboxylate

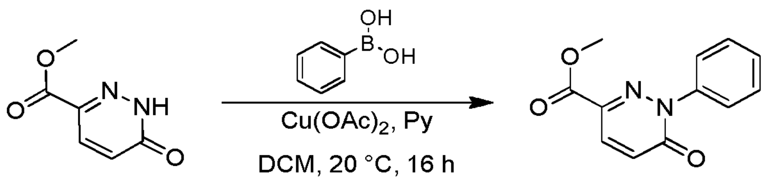


A mixture of phenylboronic acid (380 mg, 3.1 mmol), methyl 6-oxo-1H-pyridazine-3-carboxylate (504 mg), $Cu(OAc)_2$ (113 mg, 623 μmol), pyridine (1.6 g, 19.8 mmol) in DCM (10 mL) was degassed and purged with $N_2$ for 3 times, and then the mixture was stirred at 20 °C for 16 hours under $N_2$ atmosphere. The reaction mixture was poured into distilled water (20 mL) and extracted with EtOAc. The combined organic layer was washed with brine, dried over anhydrous $Na_2SO_4$ and concentrated under reduced pressure to obtain a crude product, which was purified by silica gel column chromatography (21% EtOAc in petroleum ether) to give methyl 6-oxo-1-phenyl-pyridazine-3-carboxylate (450 mg, 62.7% yield) as a yellow solid. $^1H$ NMR (400 MHz, DMSO-$d_6$) δ 7.93 (d, *J* = 10.0 Hz, 1H), 7.57-7.53 (m, 4H), 7.52-7.47 (m, 1H), 7.16 (d, *J* = 10.0 Hz, 1H), 3.86 (s, 3H); LC/MS (ESI) m/z = 231.0 $[M+H]^+$.

Step 2: Synthesis of 6-oxo-1-phenyl-pyridazine-3-carboxylic acid

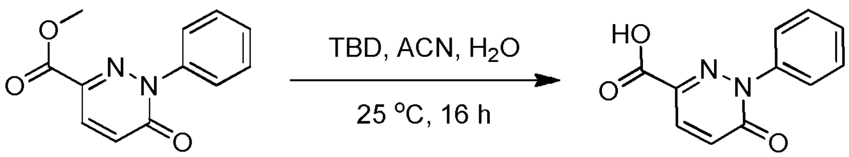


**Intermediate A**

To a solution of methyl 6-oxo-1-phenyl-pyridazine-3-carboxylate (450 mg, 2.0 mmol) in ACN (5 mL) and $H_2O$ (1 mL) was added 3, 4, 6, 7, 8, 9-hexahydro-2H-pyrimido[1, 2-a] pyrimidine (544 mg, 3.9 mmol), followed by stirring at

25 °C for 16 hours. The reaction mixture was concentrated under reduced pressure to obtain a residue, which was added with water (20 mL), acidified (pH = 2.0) with 1 N aqueous HCl solution and extracted with EtOAc. The combined organic layer was washed with brine, dried over anhydrous $Na_2SO_4$ and concentrated under reduced pressure to give Intermediate A (410 mg, crude, 95% yield) as a yellow solid. $^1H$ NMR (400 MHz, DMSO-$d_6$) δ 13.67 (bs, 1H), 7.91 (d, *J* = 10.0 Hz,1H), 7.52 (m, 5H), 7.13(d, *J* = 10.0 Hz, 1H); LC/MS (ESI) m/z = 217.0 [M+H]$^+$.

**Preparation Example 2: (1R)-1-[3-nitro-5-(trifluoromethyl)phenyl]ethanamine**

Step 1: Synthesis of 1-(3-nitro-5-(trifluoromethyl)phenyl]ethanone

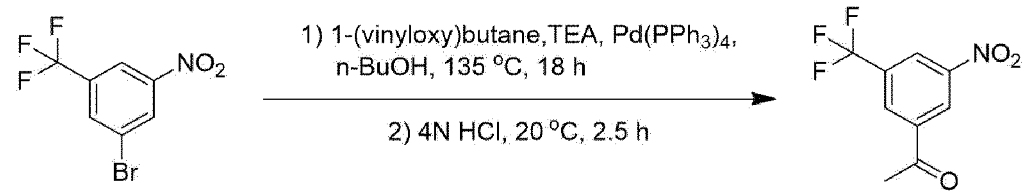


1-vinyloxybutane (74.2 g, 741 mmol) and TEA (11.2 g, 111 mmol) were added dropwise to a mixture of 1-bromo-3-nitro-5-(trifluoromethyl)benzene (20.0 g, 74.1 mmol) and $Pd(PPh_3)_4$ (4.3 g, 3.7 mmol) in n-BuOH (200 mL), and the mixture was degassed, purged with $N_2$ for 3 times, and then stirred at 135 °C for 18 hours under $N_2$ atmosphere. The mixture was added with 4N HCl (120 mL) and THF (100 mL) and stirred at 20 °C for 2.5 hours. The reaction mixture was poured into water (600 mL) and extracted with EtOAc. The combined organic layer was washed with brine, dried over anhydrous $Na_2SO_4$ and concentrated under reduced pressure to obtain a residue, which was purified by column chromatography on silica gel (3% EtOAc in PE) to give 1-[3-nitro-5-(trifluoromethyl)phenyl]ethanone (20.45 g, 47.37% yield) as yellow oil. $^1H$ NMR (400 MHz, CHLOROFORM-*d*) δ 8.94 (d, *J* = 1.6 Hz, 1H), 8.69 (s, 1H), 8.53 (s, 1H), 2.75 (s, 3H).

Step 2: Synthesis of (R)-2-methyl-N-[1-[3-nitro-5-(trifluoromethyl)phenyl]ethylidene]propane-2-sulfinamide

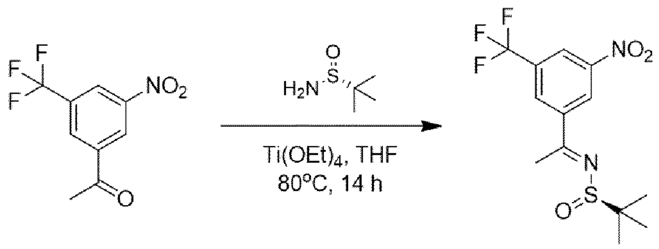


To a solution of 1-[3-nitro-5-(trifluoromethyl)phenyl]ethanone (20.5 g, 87.7 mmol) in THF (200 mL), $Ti(OEt)_4$ (50.0 g, 219 mmol) and (R)-2-methylpropane-2-sulfinamide (13.8 g, 114 mmol) were added, followed by stirring at 80 °C for 14 hours under $N_2$. The mixture was quenched with ice water (300 mL) at 20 °C, and the precipitate was dissolved in EtOAc (500 mL) and filtered out. The organic layer was concentrated in vacuo to obtain a residue, which was purified by silica gel column chromatography (15-20% EtOAc in PE) to give (R)-2-methyl-N-[1-[3-nitro-5-(trifluoromethyl)phenyl]ethylidene]propane-2-sulfinamide (20.4 g, 69.0% yield) as yellow oil. $^1H$ NMR (400 MHz, DMSO-$d_6$) δ 8.84 (s, 1H), 8.64 (s, 1H), 8.54 (s, 1H), 2.85 (s, 3H), 1.25 (s, 9H); LC/MS (ESI) m/z = 337.0 [M+H]$^+$.

Step 3: Synthesis of (R)-2-methyl-N-[(1R)-1-[3-nitro-5-(trifluoromethyl)phenyl]ethyl]propane-2-sulfinamide

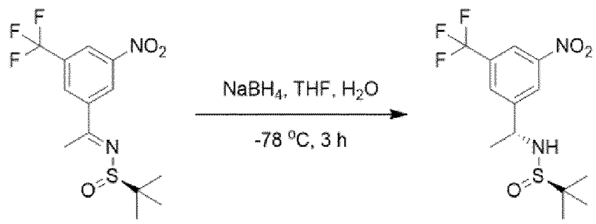


To a solution of (R)-2-methyl-N-[1-[3-nitro-5-(trifluoromethyl)phenyl]ethylidene]propane-2-sulfinamide (20.4 g, 60.5 mmol) in THF (200 mL) and $H_2O$ (4 mL) was added $NaBH_4$ (1.6 g, 42.4 mmol), followed by stirring at -78 °C for 3 hours under $N_2$. The reaction mixture was quenched with sat. aq. $NH_4Cl$ (150 mL) at 20 °C, diluted with EtOAc (100 mL) and extracted with EtOAc. The combined organic layer was washed with brine, dried over anhydrous $Na_2SO_4$, filtered and concentrated under reduced pressure to obtain a residue. Two diastereomers in the ratio of 95:5 were purified by silica gel column chromatography (20% EtOAc in petroleum ether) to give the main product, (R)-2-methyl-N-[(1R)-1-[3-nitro-5-(trifluoromethyl)phenyl]ethyl]propane-2-sulfinamide (14.3 g, 69.9% yield, >99% ee) as a light-yellow solid. $^1H$ NMR (400 MHz, DMSO-$d_6$) δ 8.63 (s, 1H), 8.37 (s, 1H), 8.29 (s, 1H), 6.07 (d, *J* = 8.8 Hz, 1H), 4.73-4.63 (m, 1H), 1.45 (d, *J* = 7.2 Hz, 3H), 1.13 (s, 9H); LC/MS (ESI) m/z = 339.0 $[M+H]^+$.

Step 4: Synthesis of (1R)-1-[3-nitro-5-(trifluoromethyl)phenyl]ethanamine

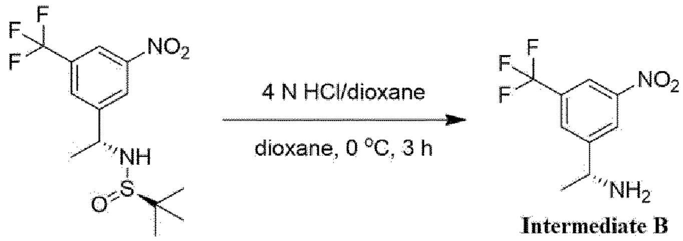


Intermediate B

To a solution of (R)-2-methyl-N-[(1R)-1-[3-nitro-5-(trifluoromethyl)phenyl]ethyl]propane-2-sulfinamide (14.3 g, 42.3 mmol) in dioxane (50 mL) was added 4N HCl/dioxane (50 mL) at 0 °C, followed by stirring at 0 °C for 3 hours. The mixture was concentrated under reduced pressure to obtain a residue, the residue was triturated with MTBE (200 mL) for 20 min at 20 °C, and the mixture was filtered to give Intermediate B (8.4 g, 73.4% yield, HCl salt) as an off-white solid. $^1H$ NMR (400 MHz, DMSO-$d_6$) δ 8.90 (br s, 3H), 8.79 (s, 1H), 8.50 (s, 2H), 4.75 (q, *J* = 6.8 Hz, 1H), 1.59 (d, *J* = 6.8 Hz, 3H); LC/MS (ESI) m/z = 235.1 $[M+H]^+$.

**Preparation Example 3: 3-[(1R)-1-aminoethyl]-5-(trifluoromethyl)aniline**

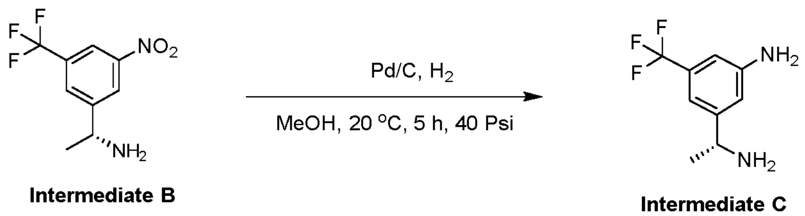


Intermediate B

Intermediate C

To a solution of Intermediate B (3.00g, 11.09 mmol, HCl salt) in MeOH (30 mL) was added Pd/C (600 mg, 10% purity), followed by stirring at 20°C for 5 hours under $H_2$ (40 Psi). The reaction mixture was filtered, and the filtrate was concentrated under reduced pressure to give Intermediate C (2.5 g, 93.72% yield, HCl salt) as a light yellow solid. $^1H$ NMR (400 MHz, DMSO-$d_6$) δ 8.49 (brs, 3H), 6.97 (s, 1H), 6.84 (brd, *J* = 5.2 Hz, 2H), 5.75 (s, 2H), 4.44-4.24 (m, 1H), 1.47 (d, *J* = 6.8 Hz, 3H); LC/MS (ESI) m/z = 205.0 $[M+H]^+$.

**Preparation Example 4: 2-[3-[(1R)-1-aminoethyl]phenyl]-2,2-difluoroethanol**

Step 1: Synthesis of ethyl 2-(3-acetylphenyl)-2,2-difluoro-acetate

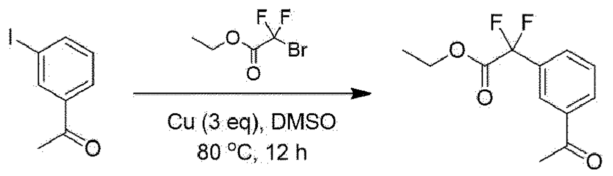


To a solution of 1-(3-iodophenyl)ethanone (5.0 g, 20.32 mmol) in DMSO (50 mL), Cu (3.87 g, 60.96 mmol) and ethyl 2-bromo-2, 2-difluoro-acetate (12.37 g, 60.96 mmol) were added, followed by stirring at 80 °C for 12 hours under $N_2$. The reaction mixture was poured into water (100 mL) and extracted with EtOAc. The combined organic layer was washed with brine, dried over anhydrous $Na_2SO_4$ and concentrated under reduced pressure to obtain a crude product. The product was purified by silica gel column chromatography (8% EtOAc in petroleum ether) to give ethyl 2-(3-acetylphenyl)-2, 2-difluoro-acetate (2.93 g, 52.38% yield) as colorless oil. $^1$H NMR (400 MHz, CHLOROFORM-*d*) δ 8.19 (s, 1H), 8.09 (d, *J* = 8.0 Hz, 1H), 7.81 (d, *J* = 8.0 Hz, 1H), 7.58 (t, *J* = 8.0 Hz, 1H), 4.31 (q, *J* = 7.2 Hz, 2H), 2.64 (s, 3H), 1.31 (t, *J* = 7.2 Hz, 3H); LC/MS (ESI) m/z = 243.0 $[M+H]^+$.

Step 2: Synthesis of ethyl 2-[3-[(Z)-N-[(R)-tert-butylsulfinyl]-C-methyl-carboimidoyl]phenyl]-2,2-difluoro-acetate

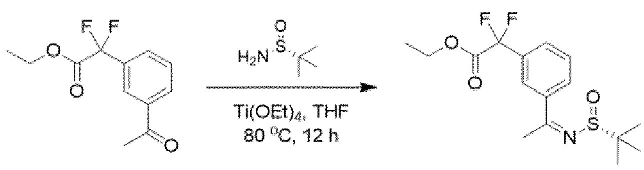


To a solution of ethyl 2-(3-acetylphenyl)-2,2-difluoro-acetate (2.93 g, 12.10 mmol) in THF (30 mL), $Ti(OEt)_4$ (6.90 g, 30.24 mmol) and (R)-2-methylpropane-2-sulfinamide (1.91 g, 15.73 mmol) were added, followed by stirring at 80 °C for 12 hours. The mixture was quenched with ice water (80 mL) at 20 °C, and the precipitate was dissolved in EtOAc (200 mL) and filtered out. The organic layer was concentrated in vacuo to obtain a residue, which was purified by silica gel column chromatography (12% EtOAc in petroleum ether) to give ethyl 2-[3-[(Z)-N-[(R)-tert-butylsulfinyl]-C-methyl-carboimidoyl]phenyl]-2,2-difluoro-acetate (3.0 g, 63.90% yield) as yellow oil. $^1$H NMR (400 MHz, CHLOROFORM-*d*) δ 8.10 (s, 1H), 8.02 (d, *J* = 8.0 Hz, 1H), 7.74 (d, *J* = 8.0 Hz, 1H), 7.54 (t, *J* = 8.0 Hz, 1H), 4.32 (q, *J* = 7.2 Hz, 2H), 2.80 (s, 3H), 1.34 (s, 12H); LC/MS (ESI) m/z = 346.0 $[M+H]^+$.

Step 3: Synthesis of (R)-N-[(1R)-1-[3-(1,1-difluoro-2-hydroxy-ethyl)phenyl]ethyl]-2-methyl-propane-2-sulfinamide

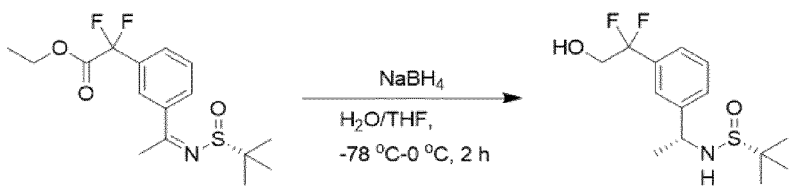


To a solution of ethyl 2-[3-[(Z)-N-[(R)-tert-butylsulfinyl]-C-methyl-carboimidoyl]phenyl]-2,2-difluoro-acetate (1 g, 2.90 mmol) in THF (10 mL) was added $NaBH_4$ (240.97 mg, 6.37 mmol) at -78 °C, followed by stirring at 0 °C for 2 hours. The reaction mixture was quenched with sat. aq. $NH_4Cl$ (40 mL) at 20 °C, diluted with EtOAc (30 mL) and extracted with EtOAc. The combined organic layer was washed with brine, dried over anhydrous $Na_2SO_4$, filtered and concentrated under reduced pressure to obtain a residue. Diastereomers produced in the ratio of about 3:1 were subjected to a first

purification with silica gel column chromatography (22% EtOAc in petroleum ether) to obtain a product. The product was subjected to prep-HPLC (Xtimate C18 150*40mm*10um; mobile phase: [water ($NH_3H_2O$)-ACN]; B%: 25%-55%, 10min) to separate a main product. Then, $CH_3CN$ was removed under reduced pressure, and the remaining solvent was removed by lyophilization to give (R)-N-[(1R)-1-[3-(1,1-difluoro-2-hydroxyethyl)phenyl]ethyl]-2-methyl-propane-2-sulfinamide (613 mg, 46.17% yield, 99.90% purity, 94.9% ee) as colorless oil. $^1H$ NMR (400 MHz, DMSO-$d_6$) δ 7.54 (s, 1H), 7.48-7.40 (m, 3H), 4.59-4.50 (m, 1H), 3.95 (t, *J* = 13.6 Hz, 2H), 3.52 (d, *J* = 5.2 Hz, 1H), 1.75 (s, 1H), 1.55 (d, *J* = 6.8 Hz, 3H), 1.23 (s, 9H); LC/MS (ESI) m/z = 306.3 $[M+H]^+$.

Step 4: Synthesis of 2-[3-[(1R)-1-aminoethyl]phenyl]-2,2-difluoro-ethanol

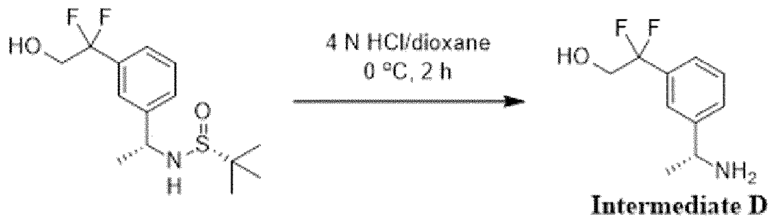


Intermediate D

To a solution of (R)-N-[(1R)-1-[3-(1,1-difluoro-2-hydroxy-ethyl)phenyl]ethyl]-2-methylpropane-2-sulfinamide (613 mg, 2.01 mmol) in dioxane (5 mL) was added 4N HCl/dioxane (5 mL), followed by stirring at 0 °C for 2 hours. The mixture was concentrated under reduced pressure to give Intermediate D (400 mg, crude) as light yellow oil. LC/MS (ESI) m/z = 202.0 $[M+H]^+$.

**Preparation Example 5: 2-[3-[(1R)-1-aminoethyl]-2-fluoro-phenyl]-2,2-difluoro-ethanol**

Step 1: Synthesis of 1-(2-fluoro-3-iodo-phenyl)ethanol

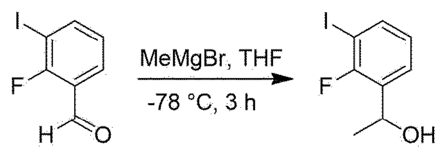


To a solution of 2-fluoro-3-iodo-benzaldehyde (4.0 g, 16.00 mmol) in THF (40 mL) was added MeMgBr (3 M, 8.00 mL) dropwise at -78 °C, followed by stirring for 3 hours. The reaction mixture was poured into sat. aq. $NH_4Cl$ (50 mL) and extracted with EtOAc. The combined organic layer was washed with brine, dried over anhydrous $Na_2SO_4$ and concentrated under reduced pressure to obtain a crude product. The product was purified by silica gel column chromatography (7% EtOAc in petroleum ether) to give 1-(2-fluoro-3-iodo-phenyl)ethanol (4.45 g, 83.63% yield) as yellow oil. $^1H$ NMR (400 MHz, CHLOROFORM-*d*) δ 7.72-7.64 (m, 1H), 7.55-7.45 (m, 1H), 6.93 (t, *J* = 7.6 Hz, 1H), 5.20 (q, *J* = 6.4 Hz, 1H), 1.52 (d, *J* = 6.4 Hz, 3H).

Step 2: Synthesis of 1-(2-fluoro-3-iodo-phenyl)ethanone

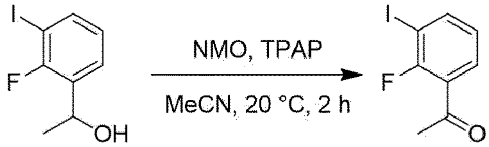


To a solution of 1-(2-fluoro-3-iodo-phenyl)ethanol (4.45 g, 16.73 mmol) in MeCN (50 mL), TPAP (587.80 mg, 1.67 mmol) and NMO (2.94 g, 25.09 mmol) were added, followed by stirring at 20 °C for 2 hours. The mixture was filtrated and concentrated under reduced pressure to obtain a crude product, which was purified by silica gel column chromatography (0% EtOAc in petroleum ether) to give 1-(2-fluoro-3-iodo-phenyl)ethanone (3.8 g, 12.95 mmol, 77.44% yield) as a white solid. $^{1}$H NMR (400 MHz, CHLOROFORM-d) δ 7.97-7.88 (m, 1H), 7.85-7.78 (dm, 1H), 7.00 (t, *J* = 7.6 Hz, 1H), 2.65 (d, *J* = 5.2 Hz, 3H).

Step 3: Synthesis of ethyl 2-(3-acetyl-2-fluoro-phenyl)-2,2-difluoro-acetate

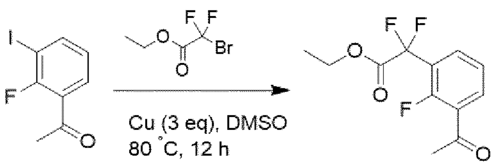


To a solution of 1-(2-fluoro-3-iodo-phenyl)ethanone (3.0 g, 11.36 mmol) and ethyl 2-bromo-2,2-difluoro-acetate (6.92 g, 34.09 mmol, 4.38 mL) in DMSO (30 mL) was added Cu (2.17 g, 34.09 mmol), followed by stirring at 80 °C for 12 hours. The reaction mixture was poured into water (50 mL) and extracted with EtOAc. The combined organic layer was washed with brine, dried over anhydrous $Na_2SO_4$ and concentrated under reduced pressure to obtain a crude product. The product was purified by silica gel column chromatography (5% EtOAc in petroleum ether) to give ethyl 2-(3-acetyl-2-fluoro-phenyl)-2,2-difluoro-acetate (1.8 g, 56.05% yield) as colorless oil. $^{1}$H NMR (400 MHz, CHLOROFORM-*d*) δ 8.08-8.00 (m, 1H), 7.87-7.81 (m, 1H), 7.36 (t, *J*= 7.6 Hz, 1H), 4.42-4.37 (m, 2H), 2.66 (d, *J* = 5.2 Hz, 3H), 1.35 (t, *J* = 7.2 Hz, 3H); LC/MS (ESI) m/z = 261.0 $[M+H]^+$.

Step 4: Synthesis of ethyl 2-[3-[[(R)-tert-butylsulfinyl]-C-methyl-carboimidoyl]-2-fluorophenyl]-2,2-difluoro-acetate

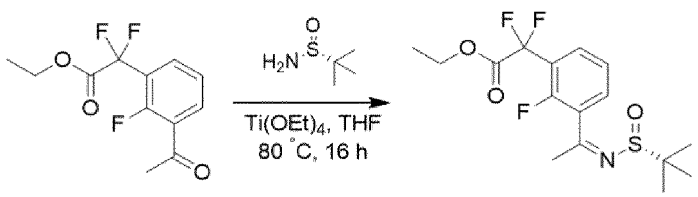


To a solution of ethyl 2-(3-acetyl-2-fluoro-phenyl)-2,2-difluoro-acetate (1.8 g, 6.92 mmol) and (R)-2-methylpropane-2-sulfinamide (1.26 g, 10.38 mmol) in THF (20 mL) was added $Ti(OEt)_4$ (4.73 g, 20.75 mmol), followed by stirring at 80 °C for 16 hours. The reaction mixture was poured into water (30 mL) and EtOAc (30 mL) and filtrated, and the filtrate was extracted with EtOAc. The combined organic layer was washed with brine, dried over anhydrous $Na_2SO_4$ and concentrated under reduced pressure to obtain a crude product. The crude product was purified by silica gel column chromatography (6% EtOAc in petroleum ether) to give ethyl 2-[3-[[(R)-tert-butylsulfinyl]-C-methyl-carboimidoyl]-2-fluorophenyl]-2,2-difluoro-acetate (1.9 g, 74.09% yield) as yellow oil. $^{1}$H NMR (400 MHz, CHLOROFORM-*d*) δ 7.86-7.72 (m, 2H), 7.32 (t, *J* = 7.6 Hz, 1H), 4.44-4.30 (m, 2H), 2.77 (s, 3H), 1.32 (s, 9H); LC/MS (ESI) m/z = 364.0 $[M+H]^+$.

Step 5: Synthesis of (R)-N-[(1R)-1-[3-(1,1-difluoro-2-hydroxyethyl)-2-fluorophenyl]ethyl]-2-methyl-propane-2- sulfinamide

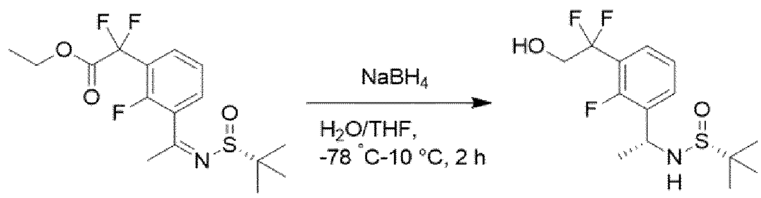

To a solution of ethyl 2-[3-[[(R)-tert-butylsulfinyl]-C-methyl-carboimidoyl]-2-fluorophenyl]-2,2-difluoro-acetate (900 mg, 2.48 mmol) in THF (10 mL) and $H_2O$ (0.2 mL) was added $NaBH_4$ (210 mg, 5.55 mmol) at -78 °C, and the mixture was warmed to 10 °C slowly and then stirred at 10 °C for 2 hours. The reaction mixture was poured into ice water (30 mL) and extracted with EtOAc. The combined organic layer was washed with brine, dried over anhydrous $Na_2SO_4$ and concentrated under reduced pressure to obtain a crude product. The product was subjected to a first purification with silica gel column chromatography (50% EtOAc in petroleum ether), and then two diastereomers (in the ratio about 3: 1) were separated and purified by using prep-HPLC (Xtimate C18 150*40mm*10um;mobile phase: [water($NH_3H_2O$)-ACN];B%: 25%-55%,10min). $CH_3CN$ was removed under reduced pressure, and the remaining solvent was removed by lyophilization to give a main product, (R)-N-[(1R)-1-[3-(1,1-difluoro-2-hydroxyethyl)-2-fluorophenyl]ethyl]-2-methyl-propane-2-sulfinamide (440 mg, 50.90% yield, 92.65% purity, >99% ee) as a white solid. $^1H$ NMR (400 MHz, DMSO-$d_6$) δ 7.70 (t, $J$ = 6.8 Hz, 1H), 7.48-7.39 (m, 1H), 7.35-7.25 (m, 1H), 5.87 (d, $J$ = 7.6 Hz, 1H), 5.70 (t, $J$ = 6.4 Hz, 1H), 4.68 (quin, $J$ = 7.2 Hz, 1H), 3.90 (dt, $J$ = 6.4, 14.4 Hz, 2H), 1.40 (d, $J$ = 6.8 Hz, 3H), 1.10 (s, 9H); LC/MS (ESI) m/z = 324.3 $[M+H]^+$.

Step 6: Synthesis of 2-[3-[(1R)-1-aminoethyl]-2-fluoro-phenyl]-2,2-difluoro-ethanol

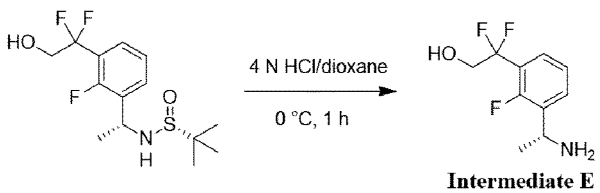


**Intermediate E**

To a solution of (R)-N-[(1R)-1-[3-(1,1-difluoro-2-hydroxy-ethyl)-2-fluoro-phenyl]ethyl]-2-methyl-propane-2-sulfinamide (440 mg, 1.36 mmol) in dioxane (4 mL) was added 4N HCl/dioxane (2 mL) at 0 °C, followed by stirring at 0 °C for 1 hour. The mixture was concentrated under reduced pressure to give Intermediate E (347 mg, 100% yield, HCl salt) as yellow oil. LC/MS (ESI) m/z = 220.0 $[M+H]^+$.

**Preparation Example 6: Methyl 5-bromo-6-oxo-1-phenyl-pyridazine-3-carboxylate**

Step 1: Synthesis of methyl 5-bromo-6-oxo-1H-pyridazine-3-carboxylate

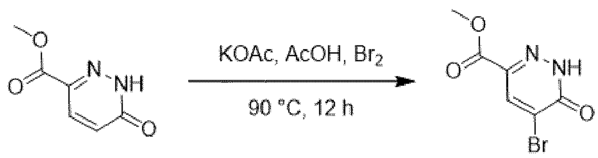


To a solution of methyl 6-oxo-1H-pyridazine-3-carboxylate (3.0 g, 19.46 mmol) in AcOH (60 mL), KOAc (6.69 g, 68.13 mmol) and $Br_2$ (6.84 g, 42.82 mmol, 2.21 mL) were added, followed by stirring at 90 °C for 12 hours. The mixture was quenched by the addition of aqueous NaHSOs solution (500 mL, 3 mol/L) and extracted with EtOAc. The combined organic layer was washed with brine, dried over anhydrous $Na_2SO_4$ and concentrated under reduced pressure to give methyl 5-bromo-6-oxo-1H-pyridazine-3-carboxylate (3.0 g, 54.24% yield) as a white solid. $^1H$ NMR (400MHz, DMSO-$d_6$) δ 13.94 (brs, 1H), 8.26 (s, 1H), 3.85 (s, 3H); LC/MS (ESI) m/z = 232.9 $[M+H]^+$.

Step 2: Synthesis of methyl 5-bromo-6-oxo-1-phenyl-pyridazine-3-carboxylate

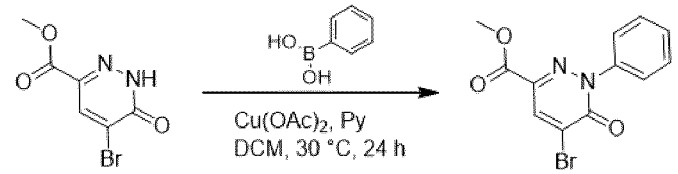


Intermediate F

To a solution of methyl 5-bromo-6-oxo-1H-pyridazine-3-carboxylate (3.00 g, 12.87 mmol) and phenylboronic acid (2.35 g, 19.31 mmol) in DCM (40 mL), pyridine (6.62 g, 83.68 mmol) and $Cu(OAc)_2$ (1.17 g, 6.44 mmol) were added, followed by stirring at 30 °C for 24 hours. The reaction mixture was poured into water (50 mL) and extracted with EtOAc, and the combined organic layer was washed with brine, dried over anhydrous $Na_2SO_4$ and concentrated under reduced pressure to obtain a crude product. The crude product was purified by silica gel column chromatography (15% EtOAc in petroleum ether) to give Intermediate F (2.5 g, 57.43% yield) as a light yellow solid. LC/MS (ESI) m/z = 309.0 $[M+H]^+$.

**Preparation Example 7: Methyl 1-(2-nitrophenyl)-6-oxopyridazine-3-carboxylate**

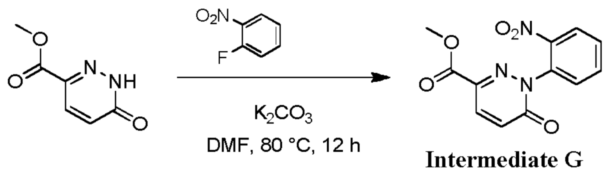


Intermediate G

A mixture of methyl 6-oxo-1H-pyridazine-3-carboxylate (1 g, 6.49 mmol), 1-fluoro-2-nitro-benzene (1.10 g, 7.79 mmol) and $K_2CO_3$ (1.35 g, 9.73 mmol) in DMF (10 mL) was degassed and purged with $N_2$ for 3 times, and then stirred at 80 °C for 12 hours under $N_2$ atmosphere. The reaction mixture was poured into water (10 mL) and extracted with EtOAc. The combined organic layer was washed with brine, dried over anhydrous $Na_2SO_4$ and concentrated under reduced pressure to obtain a crude product. The crude product was purified by flash silica gel chromatography (40% EtOAc in petroleum ether) to give Intermediate G (1.2 g, 57.34% yield) as a white solid. LC/MS (ESI) m/z = 275.9 $[M+H]^+$.

Further, the following Intermediate G-1 to Intermediate G-4 were prepared in a similar method to that of Intermediate G.

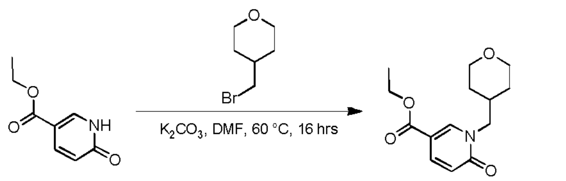


Intermediate G-1

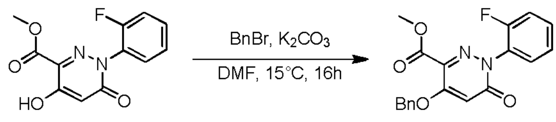


Intermediate AK

Intermediate G-2

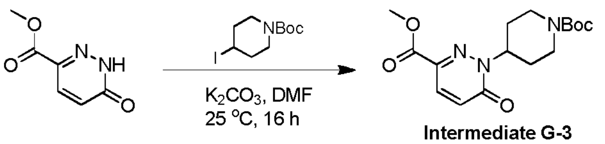


Intermediate G-3

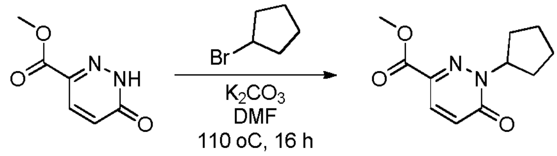


Intermediate G-4

**Preparation Example 8: Methyl 6-oxo-1-phenyl-4-(trifluoromethylsulfonyloxy)pyridazine-3-carboxylate**

Step 1: Synthesis of dimethyl 3-oxo-2-(phenylhydrazono)pentanedioate

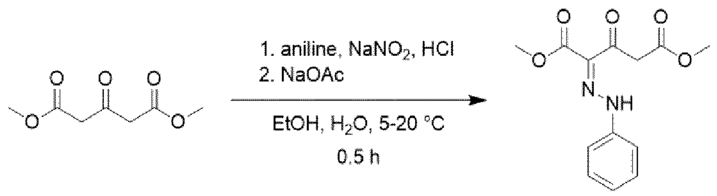


A mixture of HCl (10.20 g, 100.71 mmol, 10 mL, 36% purity), distilled water (20 mL) and aniline (1.86 g, 19.98 mmol, 1.82 mL) was treated with a solution of $NaNO_2$ (1.38 g, 19.98 mmol) in distilled water (15 mL) at 5 °C. The solution was poured into a mixture of dimethyl 3-oxopentanedioate (3.48 g, 19.98 mmol, 2.88 mL) in EtOH (12 mL) and NaOAc (12 g, 146.28 mmol) in distilled water (40 mL), and the reaction mixture was extracted with EtOAc. The combined organic layer was washed with brine, dried over anhydrous $Na_2SO_4$ and concentrated under reduced pressure to give dimethyl 3-oxo-2-(phenylhydrazono)pentanedioate (5.5 g, 89.02% yield) as yellow oil. $^1H$ NMR (400MHz, DMSO-$d_6$) $\delta$ 11.99 (s, 1H), 7.49-7.40 (m, 4H), 7.19-7.10 (m, 1H), 3.89 (s, 2H), 3.85 (s, 3H), 3.62 (s, 3H).

Step 2: Synthesis of methyl 4-hydroxy-6-oxo-1-phenylpyridazine-3-carboxylate

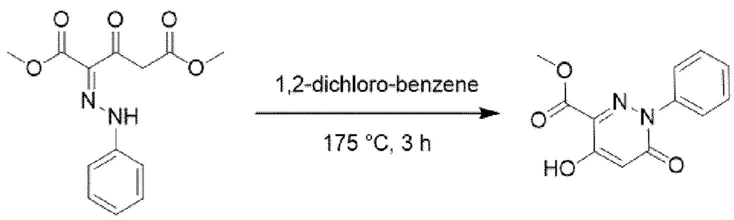


A solution of dimethyl 3-oxo-2-(phenylhydrazono)pentanedioate (5.30 g, 19.05 mmol) in 1,2-dichlorobenzene (50 mL) was stirred at 175 °C for 3 hours. The mixture was purified by silica gel column chromatography (35% EtOAc in petroleum ether) to give methyl 4-hydroxy-6-oxo-1-phenylpyridazine-3-carboxylate (2.6 g, 52.67% yield) as a yellow solid. $^1H$ NMR (400MHz, DMSO-$d_6$) $\delta$ 13.10-10.54 (m, 1H), 7.58-7.42 (m, 5H), 6.21 (s, 1H), 3.88-3.78 (m, 3H).

Step 3: Synthesis of methyl 6-oxo-1-phenyl-4-(trifluoromethylsulfonyloxy)pyridazine-3-carboxylate

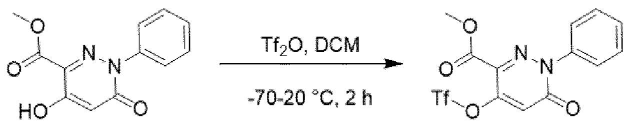


Intermediate H

To a solution of methyl 4-hydroxy-6-oxo-1-phenyl-pyridazine-3-carboxylate (200 mg, 812.29 μmol) in DCM (4 mL) was added trifluoromethanesulfonic acid anhydride ($Tf_2O$, 297.93 mg, 1.06 mmol, 174.23 μL) in DCM (10 mL) at -70 °C dropwise, and the mixture was stirred at 20 °C for 1 hour. The reaction mixture was poured into water (20 mL) and extracted with EtOAc, and the combined organic layer was washed with brine, dried over anhydrous $Na_2SO_4$ and concentrated under reduced pressure to obtain a crude product. The crude product was purified by silica gel column chromatography (12% EtOAc in petroleum ether) to give Intermediate H (220 mg, 71.60% yield) as a yellow solid. LC/MS (ESI) m/z = 379.0 [M+H]$^+$).

**Preparation Example 9: (1R)-1-(3-ethoxyphenyl)ethanamine**

Step 1: Synthesis of 1-(3-ethoxyphenyl)ethanone

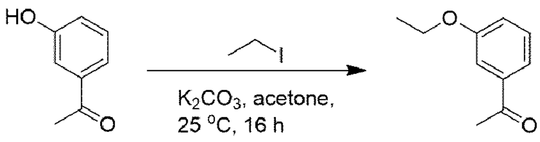


To a mixture of 1-(3-hydroxyphenyl)ethanone (5.00 g, 36.7 mmol) and iodoethane (10.5 g, 67.2 mmol) in acetone (50 mL) was added $K_2CO_3$ (10.2 g, 73.5 mmol), followed by stirring at 25 °C for 16 hours under $N_2$. The reaction mixture was filtered, and the filtrate was concentrated under reduced pressure to obtain a residue. The residue was purified by silica gel column chromatography (6% EtOAc in petroleum ether) to give 1-(3-ethoxyphenyl)ethanone (5.65 g, 93.69% yield) as white oil. $^1H$ NMR (400MHz, CHLOROFORM-d) δ = 7.52 (td, *J* = 1.2, 7.6 Hz, 1H), 7.49-7.45 (m, 1H), 7.36 (t, *J* = 8.0 Hz, 1H), 7.05-7.13 (m, 1H), 4.08 (q, *J* = 6.8 Hz, 2H), 2.60-2.58 (m, 3H), 1.43 (t, *J* = 6.8 Hz, 3H).

Step 2: Synthesis of (R)-N-[1-(3-ethoxyphenyl)ethylidene]-2-methyl-propane-2-sulfinamide

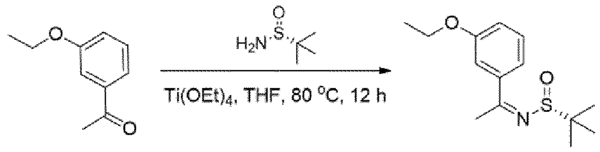


To a solution of 1-(3-ethoxyphenyl)ethanone (1.50 g, 9.14 mmol) and (R)-2-methylpropane-2-sulfinamide (1.66 g, 13.7 mmol) in THF (20 mL) was added $Ti(OEt)_4$ (6.25 g, 27.4 mmol), followed by stirring at 80 °C for 12 hours. The reaction mixture was poured into water (50 mL), then a large amount of white solid was obtained, and the mixture was filtrated. The filter cake was washed with EtOAc, then the filtrate was combined and separated, and the organic layer was dried over anhydrous $Na_2SO_4$ and concentrated under reduced pressure to obtain a residue. The residue was purified by silica gel column chromatography (0-18% EtOAc in petroleum ether) to give (R)-N-[1-(3-ethoxyphenyl)ethylidene]-2-methyl-propane-2-sulfinamide (2.20 g, 90.07% yield) as yellow oil. $^1H$ NMR (400MHz, CHLOROFORM-d) δ = 7.50-7.36 (m, 2H), 7.32 (t, *J* = 8.4 Hz, 1H), 7.02 (dd, *J* = 1.6, 8.0 Hz, 1H), 4.07 (q, *J* = 7.2 Hz, 2H), 2.75 (s, 3H), 1.43 (t, *J* = 7.2 Hz, 3H), 1.32 (s, 9H); LC/MS (ESI) m/z = 268.1 $[M+H]^+$.

Step 3: Synthesis of (R)-N-[(1R)-1-(3-ethoxyphenyl)ethyl]-2-methyl-propane-2-sulfinamide

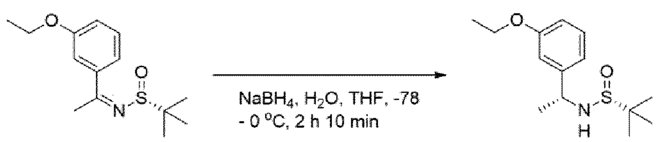


To a solution of (R)-N-[1-(3-ethoxyphenyl)ethylidene]-2-methyl-propane-2-sulfinamide (900 mg, 3.37 mmol) in THF (10 mL) and $H_2O$ (0.2 mL) was added $NaBH_4$ (285 mg, 7.53 mmol) dropwise at -78 °C, and then the mixture was stirred at -78 °C for 10 min and warmed to 0 °C. The resulting mixture was stirred at 0 °C for another 2 hours. The mixture was diluted with water (20 mL) and extracted with EtOAc. The combined organic layer was washed with brine, dried over anhydrous $Na_2SO_4$ and concentrated under reduced pressure to obtain a residue as a mixture of diastereomers. The residue was purified by silica gel column chromatography (0-17% EtOAc in petroleum ether) to give a main product, (R)-N-[(1R)-1-(3-ethoxyphenyl)ethyl]-2-methylpropane-2-sulfinamide (716 mg, 78.96% yield) as colorless oil. $^1H$ NMR (400MHz, CHLOROFORM-*d*) δ 7.29 (s, 1H), 7.28-7.30 (m, 1H), 6.92-6.97 (m, 2H), 6.84 (dd, *J* = 8.4, 2.0 Hz, 1H), 4.51-4.58 (m, 1H), 4.04-4.09 (m, 2H), 3.45 (brs, 1H), 1.29-1.79 (m, 15H); LC/MS (ESI) m/z = 270.1 $[M+H]^+$.

Step 4: Synthesis of (1R)-1-(3-ethoxyphenyl)ethanamine

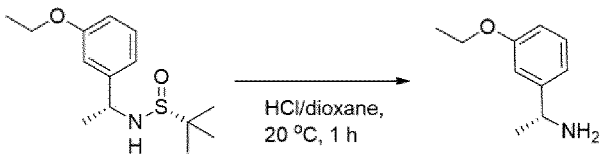


Intermediate I

A solution of (R)-N-[(1R)-1-(3-ethoxyphenyl)ethyl]-2-methyl-propane-2-sulfinamide (60.0 mg, 223 μmol) in 4 N HCl/dioxane (1 mL) was stirred at 20 °C for 1 hour. The reaction mixture was concentrated under reduced pressure to give Intermediate I (53 mg, crude, HCl salt) as colorless oil. LC/MS (ESI) m/z = 166.1 $[M+H]^+$.

**Preparation Example 10: (1R)-1-(3-trimethylsilylphenyl)ethanamine**

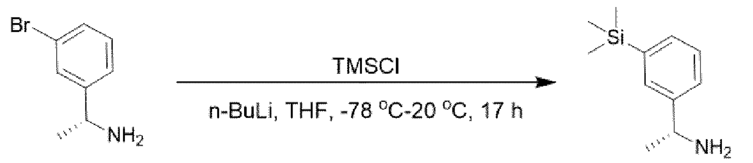


Intermediate J

To a solution of (1R)-1-(3-bromophenyl)ethanamine (100 mg, 499.81 μmol) in THF (2 mL) was added n-BuLi (2.5 M, 899.66 μL) dropwise at -78 °C, and the mixture was stirred for 1 hour and then added with trimethylsilyl chloride (135.75 mg, 1.25 mmol, 158.59 μL) at -78°C. The resulting mixture was stirred at 20 °C for 17 hours. The reaction mixture was poured into water (20 mL) and extracted with EtOAc, and the organic layer was washed with brine, dried over anhydrous $Na_2SO_4$ and concentrated under reduced pressure to obtain a crude product. The crude product was purified by silica gel column chromatography (25% EtOAc in petroleum ether) to give Intermediate J (22 mg, 22.76% yield) as light-yellow oil. $^1H$ NMR (400 MHz, CHLOROFORM-d) δ 7.49 (s, 1H), 7.44-7.40 (m, 1H), 7.38-7.32 (m, 2H), 4.14 (dd, *J* = 3.6, 6.8 Hz, 1H), 1.42 (d, *J* = 6.8 Hz, 3H), 0.28 (s, 9H).

**Preparation Example 11: Methyl 5-oxo-4-phenyl-pyrazine-2-carboxylate**

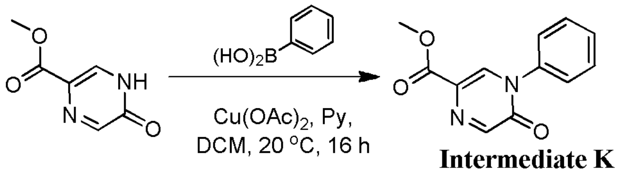


Intermediate K

Intermediate K was prepared in the same method as in Step 1 of Preparation Example 1, except that methyl 6-oxo-1H-pyrazine-3-carboxylate was used instead of methyl 6-oxo-1H-pyridazine-3-carboxylate. LC/MS (ESI) m/z = 231.0 $[M+H]^+$.

Further, the following Intermediates K-1 to K-22 were prepared in a similar method to that of Intermediate K.

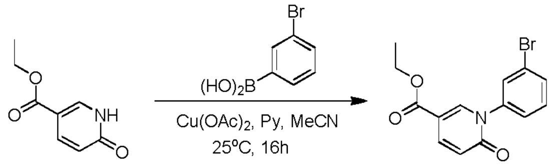


Intermediate K-1

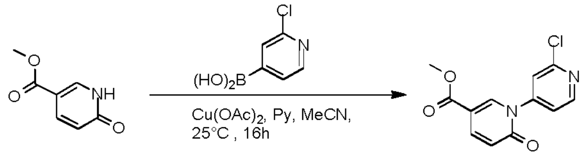


Intermediate K-2

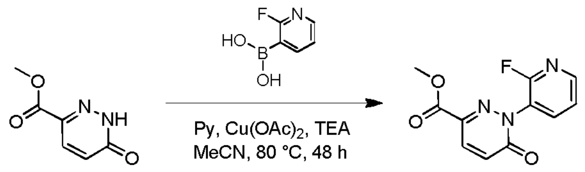


Intermediate K-3

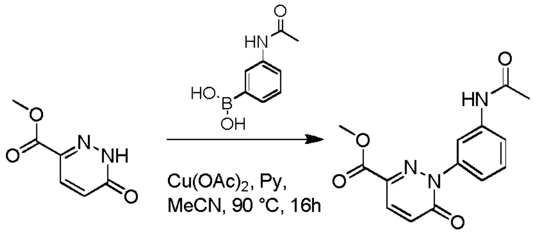


Intermediate K-4

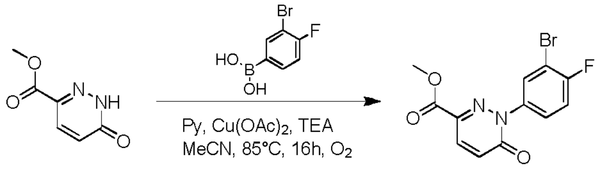


Intermediate K-5

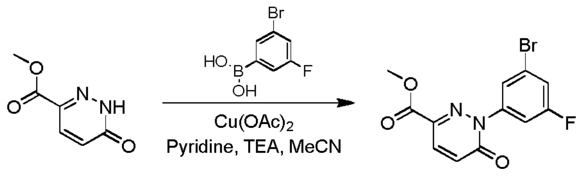


Intermediate K-6

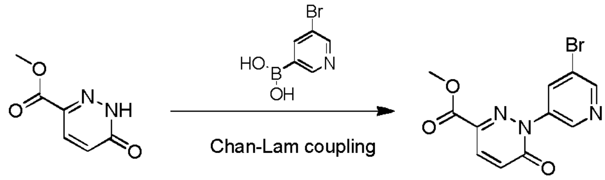


Intermediate K-7

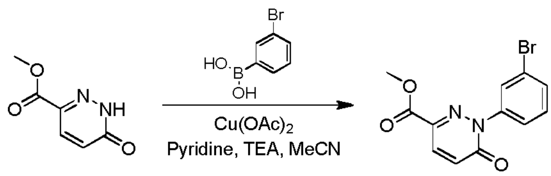


Intermediate K-8

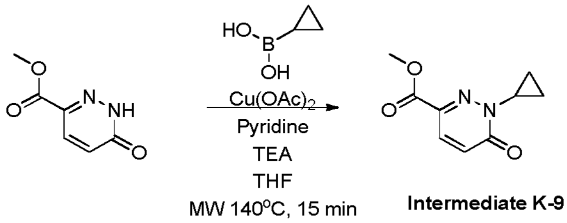


Intermediate K-9

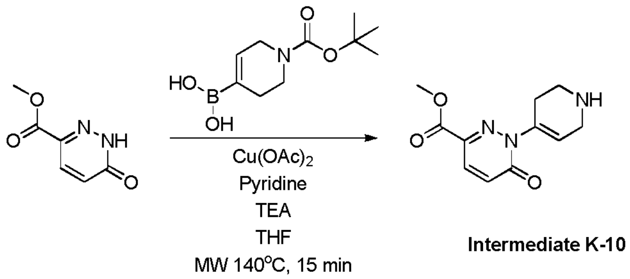


Intermediate K-10

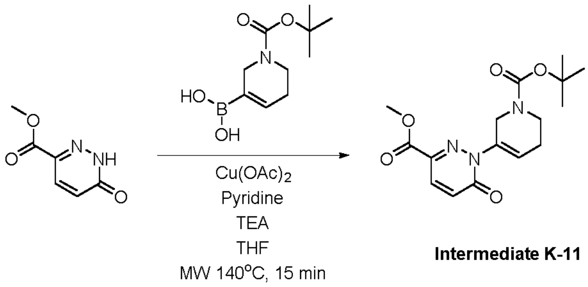


Intermediate K-11

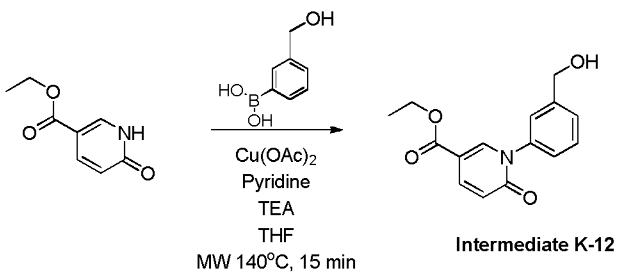


Intermediate K-12

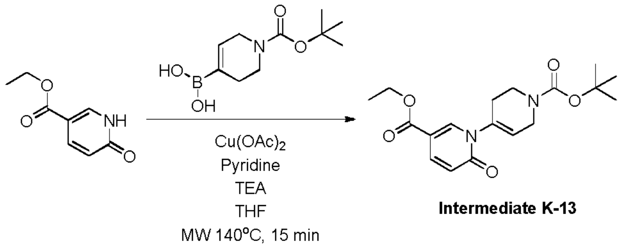


Intermediate K-13

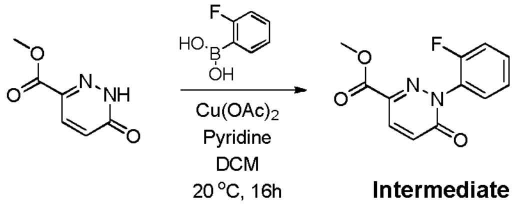


Intermediate K-14

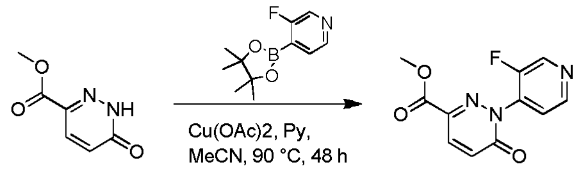


Intermediate K-15

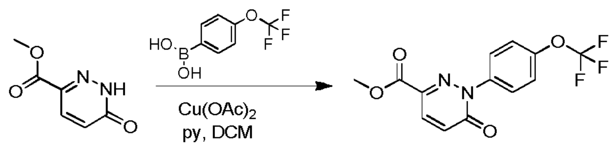


Intermediate K-16

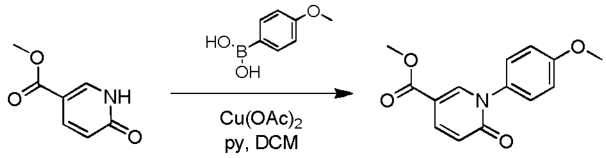


Intermediate K-17

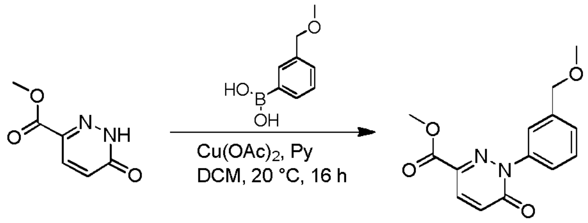


Intermediate K-18

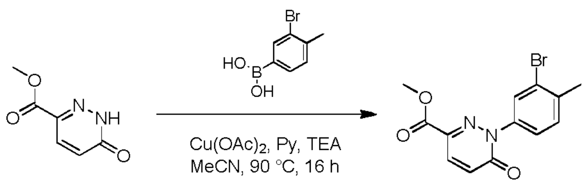


Intermediate K-19

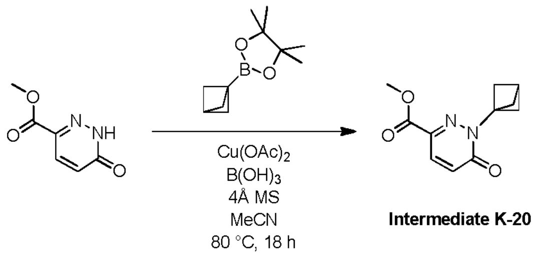


Intermediate K-20

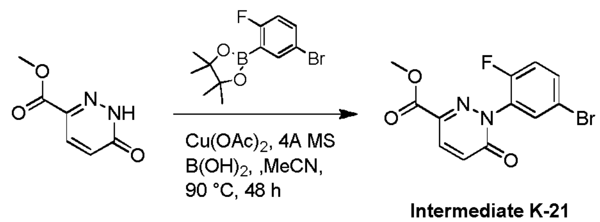


Intermediate K-21

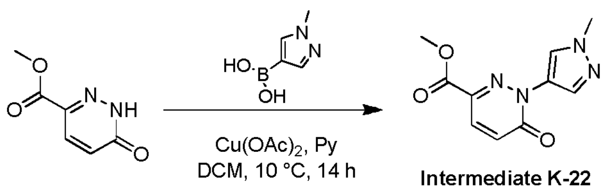


Intermediate K-22

**Preparation Example 12: (1R)-1-[(3-pentafluoro-$\lambda^6$-sulfanyl)phenyl]ethanamine**

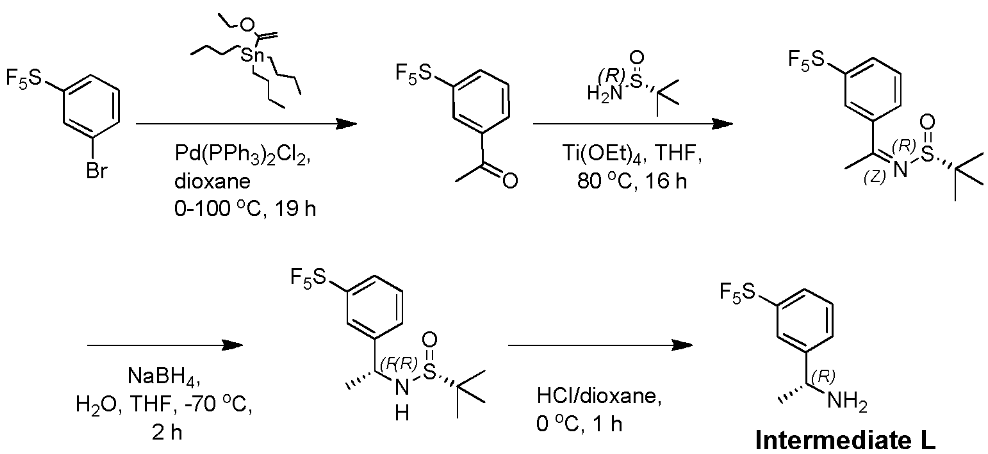


Intermediate L

Intermediate L was prepared in the same manner as in Steps 1 to 4 of Preparation Example 2 by changing the starting materials and the reagents used in Step 1 of Preparation Example 2 to 1-bromo-3-pentafluoro-$\lambda^6$-sulfanylben-

zene, tributyl(1-ethoxyvinyl)stannane, $Pd(PPh_3)_2Cl_2$ and dioxane. LC/MS (ESI) m/z = 247.1 $[M+H]^+$.

**Preparation Example 13: (R)-3-(1-aminoethyl)-2-fluorobenzonitrile**

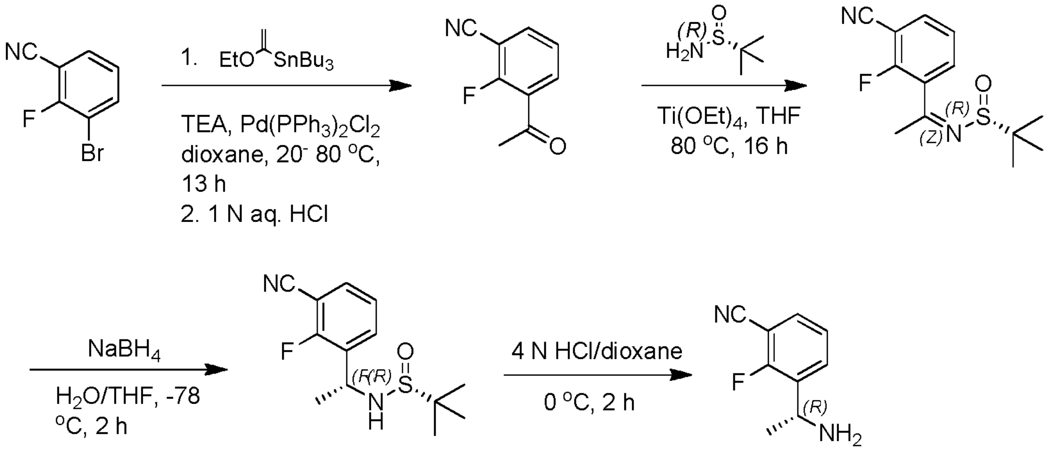


**Intermediate M**

Intermediate M was prepared in a similar method to Preparation Example 12. MS (ESI) m/z = 164.1 $[M+H]^+$.

**Preparation Example 14: (R)-1-(2-methyl-5-nitro-3-(trifluoromethyl)phenyl)ethanamine**

Step 1: Synthesis of 1-bromo-2-methyl-5-nitro-3-(trifluoromethyl)benzene

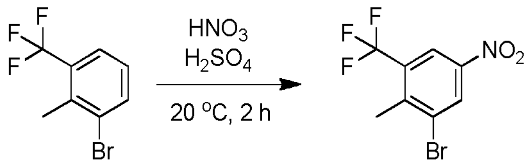


To a mixture of 1-bromo-2-methyl-3-(trifluoromethyl)benzene (10 g, 41.84 mmol) in $H_2SO_4$ (80 mL) was added $HNO_3$ (54.480 g, 864.59 mmol, 38.91 mL) slowly at 0 °C, followed by stirring at 20°C for 2 hours under $N_2$ atmosphere. The reaction mixture was quenched with ice-cold water (100 mL) and extracted with EtOAc (100 mL × 3). The combined organic layer was washed with brine (50 mL), dried over anhydrous $Na_2SO_4$ and concentrated under reduced pressure to obtain a crude product. The crude product was purified by flash silica gel chromatography (0% EtOAc in PE) to give 1-bromo-2-methyl-5-nitro-3-(trifluoromethyl)benzene (6 g, 50.49% yield) as colourless oil. $^1H$ NMR (400 MHz, DMSO-$d_6$) $\delta$ = 8.69 (d, $J$ = 2.4 Hz, 1H), 8.38 (d, $J$ = 2.4 Hz, 1H), 2.58 (d, $J$ = 1.2 Hz, 3H).

Steps 2 to 5: Synthesis of (R)-1-(2-methyl-5-nitro-3-(trifluoromethyl)phenyl)ethanamine

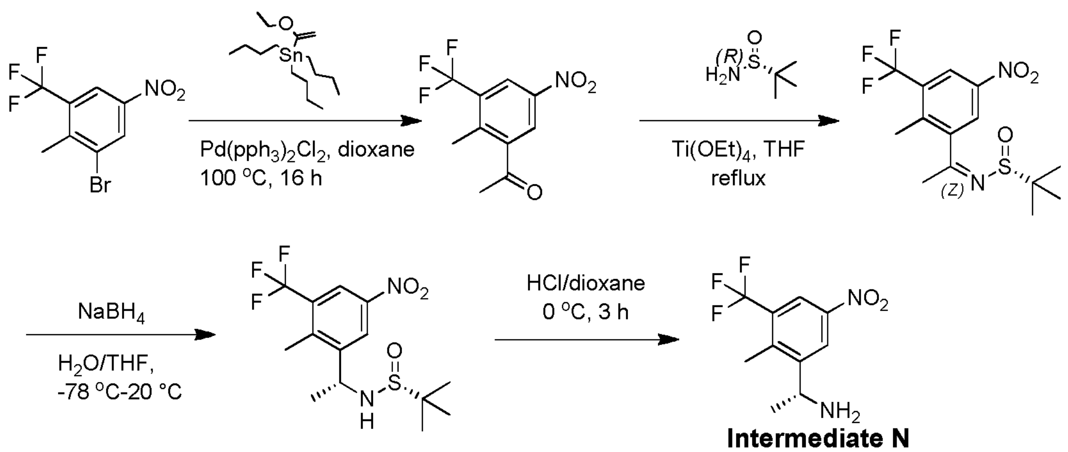


**Intermediate N**

Intermediate N was prepared in the same manner as in Preparation Example 12 by using 1-bromo-2-methyl-5-nitro-3-(trifluoromethyl)benzene as a starting material. MS (ESI) m/z = 248.08 $[M+H]^+$.

**Preparation Example 15: 1-[3-(dimethylcarbamoyl)-4-methoxy-phenyl]-6-oxo-pyridazine-3-carboxylic acid**

Step 1: Synthesis of 5-bromo-2-methoxy-N,N-dimethyl-benzamide

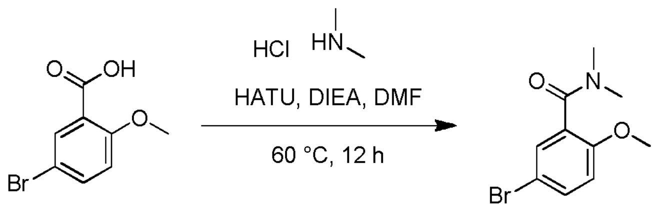


To a solution of 5-bromo-2-methoxy-benzoic acid (1 g, 4.33 mmol) in DMF (15 mL), DIEA (2.24 g, 17.31 mmol, 3.02 mL) and HATU (2.47 g, 6.49 mmol) were aded. The mixture was stirred at 25 °C for 0.5 hour. N-methylmethanamine;hydrochloride (1.06 g, 12.98 mmol) was added thereto, and the resulting mixture was stirred at 60 °C for 12 hours. The reaction mixture was poured into water (20 mL) and extracted with EtOAc (30 mL × 3). The combined organic layer was washed with brine (20 mL × 2), dried over anhydrous $Na_2SO_4$ and concentrated under reduced pressure to obtain a crude product. The crude product was purified by silica gel column chromatography (35% EtOAc in PE) to give 5-bromo-2-methoxy-N,N-dimethyl-benzamide (930 mg, 83.25% yield) as yellow oil. MS (ESI) m/z = 258.0 $[M+H]^+$.

Step 2: Synthesis of 1-[3-(dimethylcarbamoyl)-4-methoxy-phenyl]-6-oxo-pyridazine-3-carboxylic acid

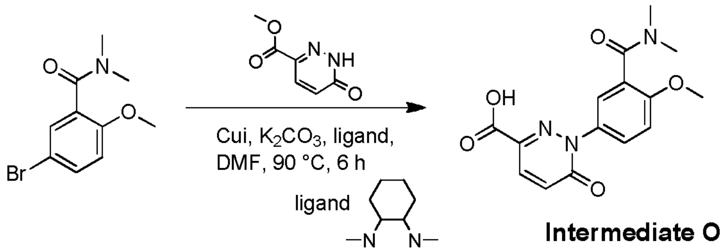


**Intermediate O**

A mixture of methyl 6-oxo-1H-pyridazine-3-carboxylate (89.57 mg, 581.14 μmol), 5-bromo-2-methoxy-N,N-dimethylbenzamide (100 mg, 387.43 μmol), CuI (73.79 mg, 387.43 μmol), (1R,2R)-N1,N2-dimethylcyclohexane-1,2-diamine (110.22 mg, 774.86 μmol) and $K_2CO_3$ (160.64 mg, 1.16 mmol) in DMF (3 mL) was degassed and purged with

$N_2$ for 3 times, and then the mixture was stirred at 90 °C for 6 hours under $N_2$ atmosphere. The reaction mixture was poured into water (20 mL) and extracted with EtOAc (20 mL ×3). The combined organic layer was discarded. The aqueous layer was adjusted by 1 N aq. HCl to pH = 3-4, and extracted with EtOAc (30 mL × 3). The combined organic layer was washed with brine (20 mL × 2), dried over anhydrous $Na_2SO_4$ and concentrated under reduced pressure to give Intermediate O (50 mg, 40.67% yield) as yellow oil. MS (ESI) m/z = 318.1 $[M+H]^+$.

**Preparation Example 16: (R)-1-(3-(1-aminoethyl)-2-fluorophenyl)-1,1-difluoro-2-methylpropan-2-ol**

Step 1: Synthesis of ethyl 2-[3-(1,1-dimethoxyethyl)-2-fluoro-phenyl]-2,2-difluoro-acetate

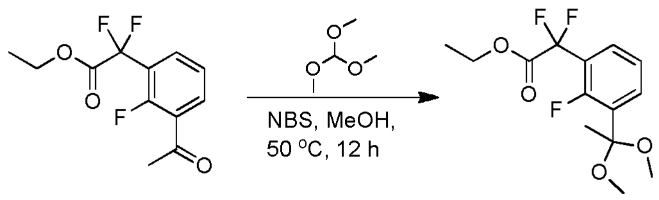


To a solution of ethyl 2-(3-acetyl-2-fluoro-phenyl)-2,2-difluoro-acetate (7.1 g, 27.29 mmol) obtained in Step 3 of Preparation Example 5 in MeOH (100 mL), trimethoxymethane (8.69 g, 81.86 mmol, 8.97 mL) and NBS (291.39 mg, 1.64 mmol) were added. The mixture was stirred at 50 °C for 12 hours. The mixture was concentrated under reduced pressure to obtain a residue. The residue was purified by flash silica gel chromatography (10% EtOAc in PE) to give ethyl 2-[3-(1,1-dimethoxyethyl)-2-fluoro-phenyl]-2,2-difluoro-acetate (6.42 g, 76.82% yield) as colorless oil. $^1H$ NMR (400 MHz, DMSO-$d_6$) $\delta$ = 7.80 (t, *J* = 7.2 Hz, 1H), 7.70 (t, *J* = 6.8 Hz, 1H), 7.40 (t, *J* = 7.6 Hz, 1H), 4.39 - 4.31 (m, 2H), 3.08 (s, 6H), 1.53 (s, 3H), 1.20 (t, *J* = 7.2 Hz, 3H).

Step 2: Synthesis of 1-[3-(1,1-dimethoxyethyl)-2-fluorophenyl]-1,1-difluoro-2-methyl-propan-2-ol

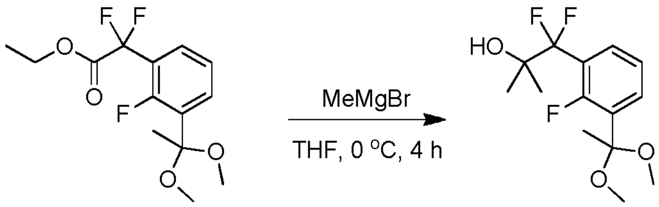


A mixture of ethyl 2-[3-(1,1-dimethoxyethyl)-2-fluoro-phenyl]-2,2-difluoro-acetate (6.42 g, 20.96 mmol) and MeMgBr (1 M, 62.88 mL) in THF (65 mL) was degassed and purged with $N_2$ for 3 times at 0 °C, and then the mixture was stirred at 0°C for 4 hours under $N_2$ atmosphere. The reaction mixture was quenched by addition of sat. aq. $NH_4Cl$ (100 mL) at 20°C, diluted with EtOAc (100 mL) and extracted with EtOAc (100 mL × 3). The mixture was dried over anhydrous $Na_2SO_4$ and concentrated under reduced pressure to give a crude product. The residue was purified by flash silica gel chromatography (10% EtOAc in PE) to give 1-[3-(1,1-dimethoxyethyl)-2-fluorophenyl]-1,1-difluoro-2-methyl-propan-2-ol (4.5 g, 73.45% yield) as colorless oil. $^1H$ NMR (400 MHz, DMSO-$d_6$) $\delta$ = 7.70 (t, *J* = 7.2 Hz, 1H), 7.43 (t, *J* = 6.8 Hz, 1H), 7.26 (t, *J* = 7.6 Hz, 1H), 5.33 (s, 1H), 3.11 - 3.07 (m, 6H), 1.54 (s, 3H), 1.20 (s, 6H).

Step 3: Synthesis of 1-[3-(1,1-dimethoxyethyl)-2-fluorophenyl]-1,1-difluoro-2-methyl-propan-2-ol

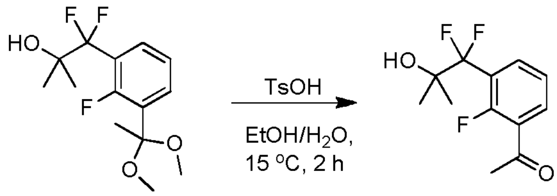


A solution of 1-[3-(1,1-dimethoxyethyl)-2-fluoro-phenyl]-1,1-difluoro-2-methyl-propan-2-ol (4.5 g, 15.40 mmol) and TsOH (5.30 g, 30.79 mmol) in $H_2O$ (4.5 mL) and EtOH (45 mL) was prepared. The reaction mixture was stirred at 15°C for 2 hours under $N_2$. The solution was concentrated under reduced pressure, and the reaction mixture was poured into water (50 mL) and extracted with EtOAc (50 mL × 3). The combined organic layer was washed with brine (50 mL), dried over anhydrous $Na_2SO_4$ and concentrated under reduced pressure to obtain a crude product. The crude product as a yellow oil, 1-[3-(1,1-difluoro-2-hydroxy-2-methyl-propyl)-2-fluorophenyl]ethanone (3.7 g, 97.61% yield) was used in the next step without further purification. $^1H$ NMR (400 MHz, DMSO-$d_6$) δ = 7.94 - 7.87 (m, 1H), 7.67 (t, *J* = 7.2 Hz, 1H), 7.42 - 7.38 (m, 1H), 5.42 (s, 1H), 2.60 - 2.57 (m, 3H), 1.22 (s, 6H).

<u>Steps 4 to 6: Synthesis of (R)-1-(3-(1-aminoethyl)-2-fluorophenyl)-1,1-difluoro-2-methylpropan-2-ol</u>

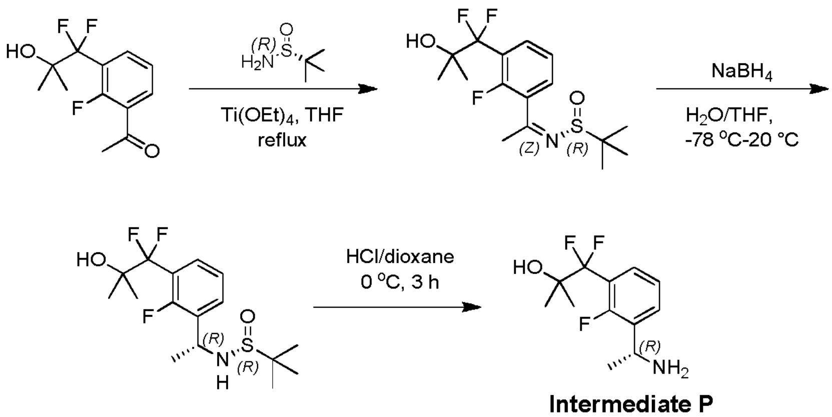


**Intermediate P**

Intermediate P was prepared in the same manner as in Steps 4 to 6 of Preparation Example 5. LC/MS (ESI) m/z = 247.1 $[M+H]^+$.

**Preparation Example 17: methyl 1-(2-methylthiazol-5-yl)-6-oxopyridazine-3-carboxylate**

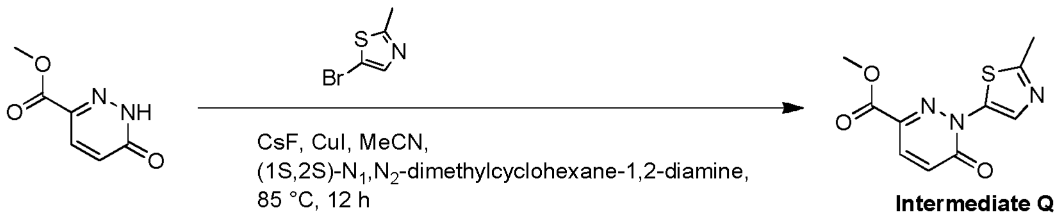


**Intermediate Q**

A mixture of methyl 6-oxo-1H-pyridazine-3-carboxylate (150 mg, 973.25 μmoL), 5-bromo-2-methyl-thiazole (207.94 mg, 1.17 mmol), CuI (18.54 mg, 97.32 μmoL), CsF (443.52 mg, 2.92 mmol, 107.65 μL) and (1S,2S)-N1,N2-dimethylcyclohexane-1,2-diamine (27.69 mg, 194.65 μmol) in MeCN (3 mL) was degassed and purged with $N_2$ for 3 times, and then the mixture was stirred at 85 °C for 12 hours under $N_2$ atmosphere. The reaction mixture was poured into water (20 mL) and extracted with EtOAc (30 mL × 3). The combined organic layer was washed with brine (20 mL×2), dried over anhydrous $Na_2SO_4$ and concentrated under reduced pressure to obtain a crude product. The product was purified by silica gel column chromatography (45% EtOAc in PE) to give Intermediate Q (70 mg, 16.89% yield) as

an off-white solid. MS (ESI) m/z = 252.1 $[M+H]^+$

The following Intermediates Q-1, Q-2, and Q-3 were prepared in a similar manner.

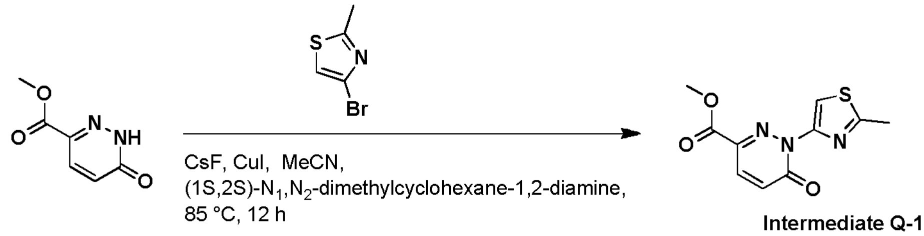


**Intermediate Q-1**

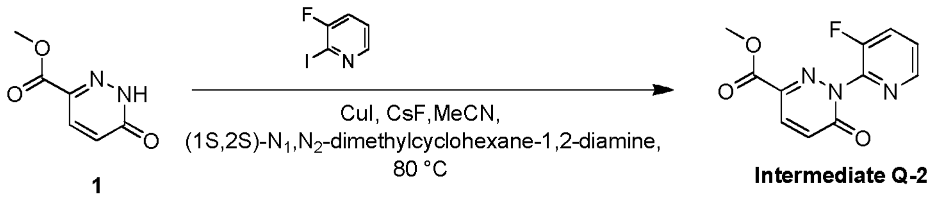


1

**Intermediate Q-2**

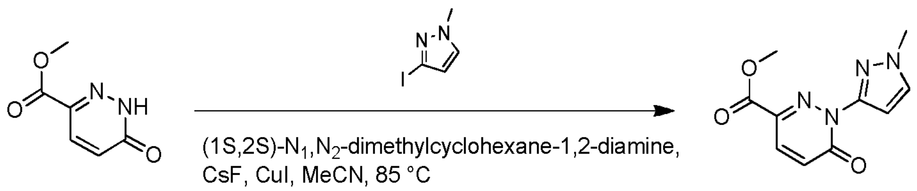


**Intermediate Q-3**

**Preparation Example 18: methyl 1-(1,3-dihydroisobenzofuran-5-yl)-6-oxo-pyridazine-3-carboxylate**

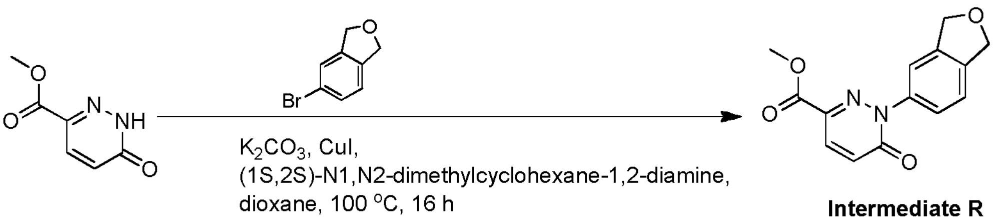


**Intermediate R**

A mixture of methyl 6-oxo-1H-pyridazine-3-carboxylate (150.99 mg, 979.65 μmol), 5-bromo-1,3-dihydroisobenzofuran(194.99 mg, 979.65 μmol), $K_2CO_3$ (406.18 mg, 2.94 mmol), CuI (186.58 mg, 979.65 μmol) and (1S,2S)-N1,N2-dimethylcyclohexane-1,2-diamine (278.70 mg, 1.96 mmol) in dioxane (3 mL) was degassed and purged with $N_2$ for 3 times, and then the mixture was stirred at 100 °C for 16 hours under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The crude product was purified by flash silica gel chromatography (32% EtOAc in PE) to give Intermediate R (60 mg, 14.32% yield) as a white solid. MS (ESI) m/z = 273.1 $[M+H]^+$.

**Preparation Example 19: methyl 1-(5-chloro-1-methyl-pyrazol-4-yl)-6-oxo-pyridazine-3-carboxylate**

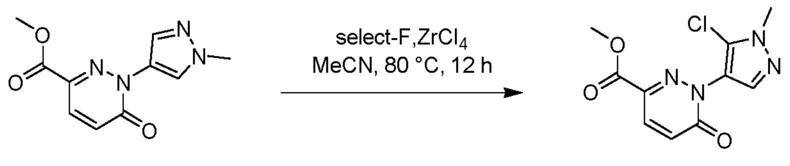


Intermediate K-22 Intermediate S

To a solution of methyl 1-(1-methylpyrazol-4-yl)-6-oxo-pyridazine-3-carboxylate, Intermediate K-22 (300 mg, 1.28 mmol) in MeCN (6 mL), Select F (680.66 mg, 1.92 mmol) and $ZrCl_4$ (59.70 mg, 256.18 μmol, 21.32 μL) were added. The mixture was stirred at 80 °C for 12 hours. The reaction mixture was poured into water (20 mL) and extracted with EtOAc (30 mL × 3). The combined organic layer was washed with brine (20 mL × 2), dried over anhydrous $Na_2SO_4$ and concentrated under reduced pressure to obtain a crude product. The product was purified by silica gel column chromatography (35% EtOAc in PE) to give Intermediate S (123 mg, 35.74% yield) as yellow oil. MS (ESI) m/z = 269.1 $[M+H]^+$.

**Preparation Example 20: methyl 4-anilno-1-(2-fluorophenyl)-6-oxo-pyridazine-3-carboxylate**

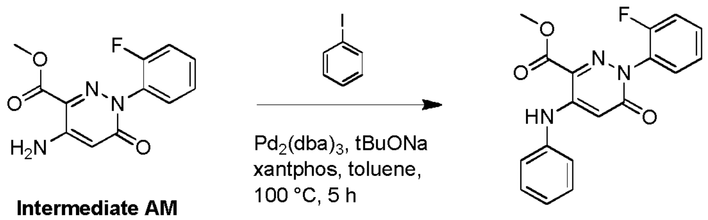


Intermediate AM

Intermediate T

To a mixture of iodobenzene (155.01 mg, 759.81 μmol, 84.70 μL) and methyl 4-amino-1-(2-fluorophenyl)-6-oxo-pyridazine-3-carboxylate (100 mg, 379.90 μmol) in toluene (5 mL), Xantphos (21.98 mg, 37.99 μmol), t-BuONa (54.77 mg, 569.86 μmol) and $Pd_2(dba)_3$ (34.79 mg, 37.99 μmol) were added, and the reaction mixture was stirred at 100 °C for 5 hours under $N_2$. The reaction mixture was poured into water (30 mL) and extracted with EtOAc (30 mL×3). The combined organic layer was washed with brine (50 mL), dried over anhydrous $Na_2SO_4$ and concentrated under reduced pressure to obtain a residue. The residue was purified by silica gel column chromatography (61% EtOAc in PE) to give Intermediate T (28 mg, 15.91% yield) as a yellow solid. MS (ESI) m/z = 340.0 $[M+1+H]^+$.

**Preparation Example 21: 1-[3-(4-methyl-1,2,4-triazol-3-yl)phenyl]-6-oxo-pyridine-3-carboxylate**

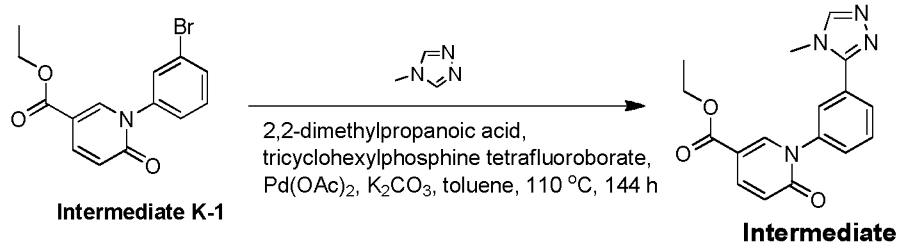


Intermediate K-1

Intermediate U

A mixture of Intermediate K-1 (200 mg, 620.82 μmol), 4-methyl-1,2,4-triazole (67.06 mg, 807.07 μmol), $Pd(OAc)_2$ (13.94 mg, 62.08 μmol), tricyclohexylphosphonium;tetrafluoroborate (45.72 mg, 124.16 μmol), 2,2-dimethylpropanoic acid (126.81 mg, 1.24 mmol, 142.64 μL) and $K_2CO_3$ (171.61 mg, 1.24 mmol) in toluene (2 mL) was degassed and purged with $N_2$ for 3 times, and then the mixture was stirred at 120 °C for 144 hours under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The crude product was purified by flash silica gel chromatography (8% MeOH in DCM) to give Intermediate U (180 mg, 21.38% yield) as a colorless oil. MS (ESI) m/z = 324.9 $[M+H]^+$.

Also, the following Intermediates U-1, U-2, U-3, U-4, and U-5 were prepared in a similar manner.

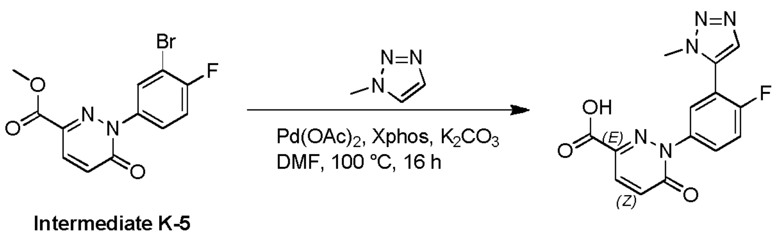


Intermediate K-5

Intermediate U-1

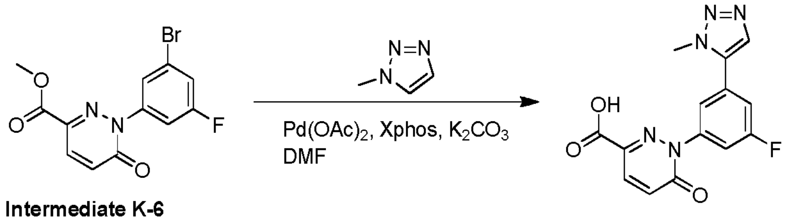


Intermediate K-6

Intermediate U-2

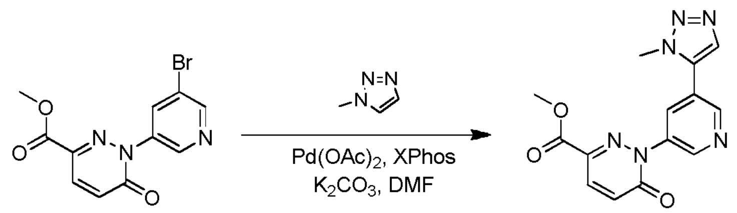


Intermediate K-7

Intermediate U-3

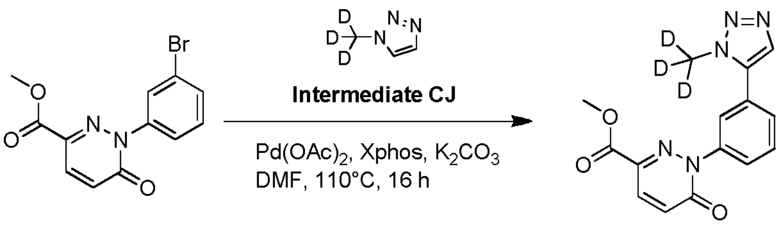


Intermediate K-8

Intermediate U-4

**Preparation Example 21A: 1-[4-methyl-3-(3-methyltriazol-4-yl)phenyl]-6-oxo-pyridazine-3-carboxylic acid**

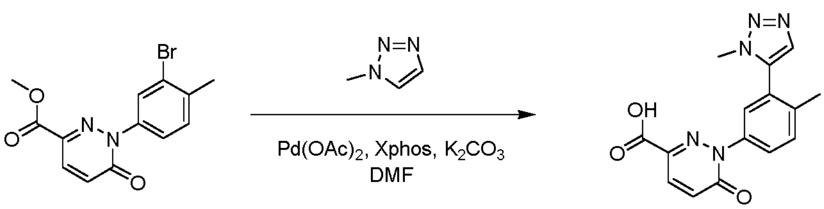


Intermediate K-19

Intermediate U-5

A mixture of methyl 1-(3-bromo-4-methyl-phenyl)-6-oxo-pyridazine-3-carboxylate (100 mg, 309.46 μmol), 1-methyltriazole (51.43 mg, 618.92 μmol), $K_2CO_3$ (85.54 mg, 618.92 μmol), $Pd(OAc)_2$ (6.95 mg, 30.95 μmol) and XPhos (29.51 mg, 61.89 μmol) in DMF (4 mL) was degassed and purged with $N_2$ for 3 times, and then the mixture was stirred

at 100 °C for 12 hours under $N_2$ atmosphere. The reaction mixture was poured into water (20 mL) and extracted with EtOAc (20 mL). The combined organic layer was discarded. The aqueous layer was adjusted by 1 N aq. HCl to pH = 2-3 and extracted with EtOAc (30 mL × 3). The combined organic layer was washed with brine (20 mL × 2), dried over anhydrous $Na_2SO_4$ and concentrated under reduced pressure to give Intermediate U-5 (80 mg, 29.26% yield) as yellow oil. MS (ESI) m/z = 321.1 [M+H]+).

**Preparation Example 22: Ethyl 1-[3-(1-methyltetrazol-5-yl)phenyl]-6-oxo-pyridine-3-carboxylate**

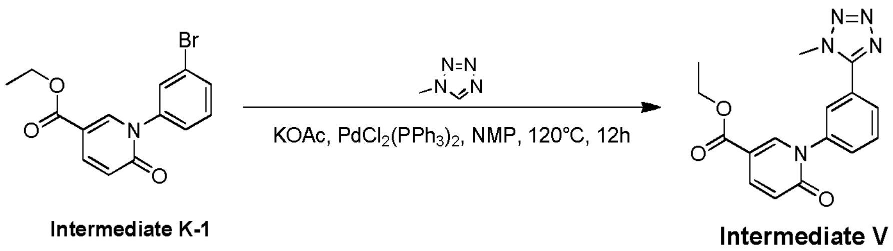


A mixture of Intermediate K-1 (104.55 mg, 324.54 μmol), 1-methyltetrazole (54.57 mg, 649.08 μmol), KOAc (63.70 mg, 649.08 μmol) and $Pd(PPh_3)_2Cl_2$ (22.78 mg, 32.45 μmol) in NMP (3 mL) was degassed and purged with $N_2$ for 3 times, and then the mixture was stirred at 120 °C for 12 hours under $N_2$ atmosphere. The reaction mixture was poured into water (50 mL) and extracted with EtOAc (50 mL × 3). The combined organic layer was washed with brine (30 mL × 2), dried over anhydrous $Na_2SO_4$ and concentrated under reduced pressure to obtain a crude product. The residue was purified by flash silica gel chromatography (48% EtOAc in PE) to give Intermediate V (154 mg, 68.66% yield) as yellow oil. MS (ESI) m/z = 326.0 [M+H]+.

**Preparation Example 23: (R)-1-(2-fluoro-3-(trifluoromethyl)phenyl)ethanamine**

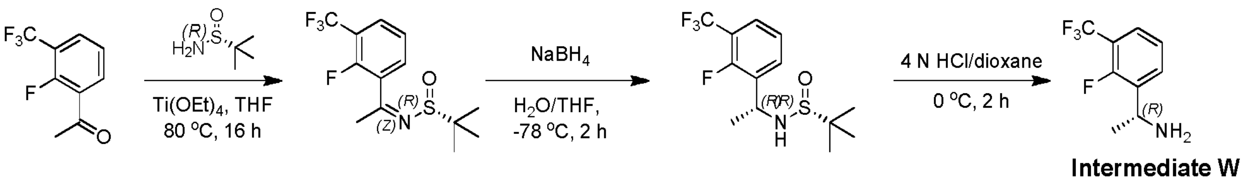


Intermediate W was prepared in the same manner as in Steps 2 to 4 of Preparation Example 2.

**Preparation Example 24: 1-(2-chloro-3-fluorophenyl)ethanamine**

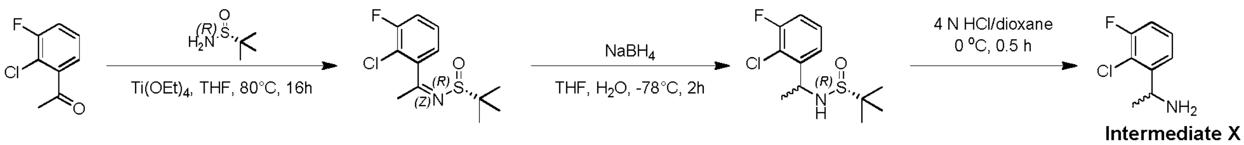


Intermediate X was prepared in the same manner as in Steps 2 to 4 of Preparation Example 2.

**Preparation Example 25: (R)-1-(1H-pyrazol-3-yl)ethane-1-amine**

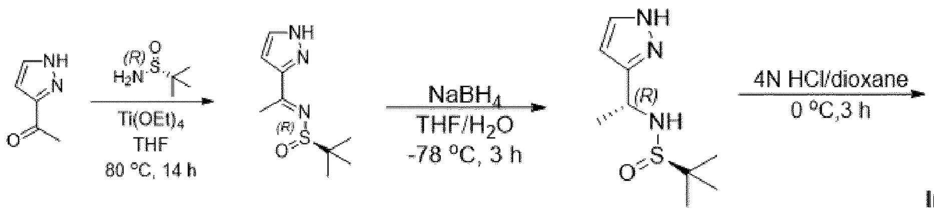


**Intermediate Y**

Intermediate Y was prepared in the same manner as in Steps 2 to 4 of Preparation Example 2. LC/MS m/z = 112.7 $[M+H]^+$.

**Preparation Example 26: (R)-1-(5-bromothiophen-2-yl)ethanamine**

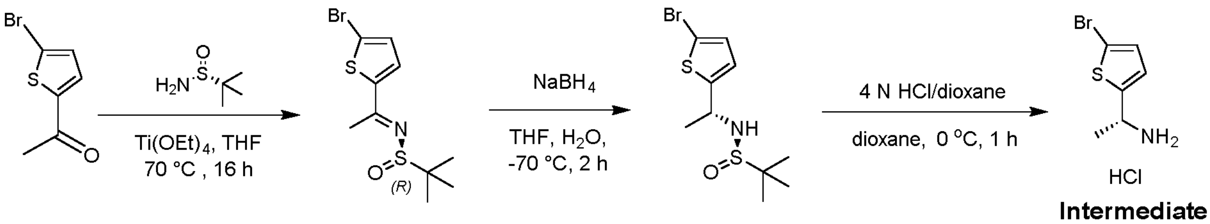


**Intermediate Z**

Intermediate Z was prepared as a yellow solid in the same manner as in Steps 2 to 4 of Preparation Example 2. $^1H$ NMR (400 MHz, DMSO-$d_6$) δ = 7.06 (d, *J*= 4.0 Hz, 1H), 6.89 (dd, *J* = 0.8, 4.0 Hz, 1H), 5.90 (d, *J* = 7.2 Hz, 1H), 4.57 (t, V = 6.8 Hz, 1H), 1.47 (d, *J* = 6.8 Hz, 3H), 1.12 (s, 9H); MS (ESI) m/z = 311.8 $[M-16+H]^+$.

**Preparation Example 27: (R)-1-(5-bromothiazol-2-yl)ethanamine**

Step 1: Synthesis of (NZ,S)-N-[1-(5-bromothiazol-2-yl)ethylidene]-2-methyl-propane-2-sulfinamide

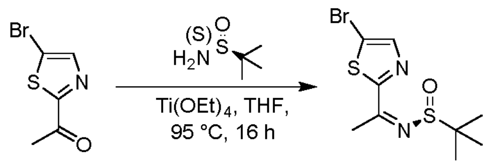


To a solution of 1-(5-bromothiazol-2-yl)ethanone (500 mg, 2.43 mmol) in THF (8 mL), $Ti(OEt)_4$ (8.31 g, 36.5 mmol) and (S)-2-methylpropane-2-sulfinamide (1.18 g, 9.72 mmol) were added, followed by stirring at 95 °C for 16 hours under $N_2$. The mixture was diluted with EtOAc (20 mL), quenched with water (30 mL) and extracted with EtOAc (10 mL × 2). The mixture was dried over anhydrous $Na_2SO_4$ and concentrated under reduced pressure to obtain a residue, which was purified by silica gel column chromatography (20% EtOAc in PE) to give (NZ,S)-N-[1-(5-bromothiazol-2-yl)ethylidene]-2-methyl-propane-2-sulfinamide (700 mg, 93.0% yield) as yellow oil. $^1H$ NMR (400 MHz, $CDCl_3$) δ 7.82 (s, 1H), 2.81 (s, 3H), 1.32 (s, 9H); LC/MS (EI) m/z = 310.9 $[M+H]^+$.

Step 2: Synthesis of (S)-N-[(1R)-1-(5-bromothiazol-2-yl)ethyl]-2-methylpropane-2-sulfinamide

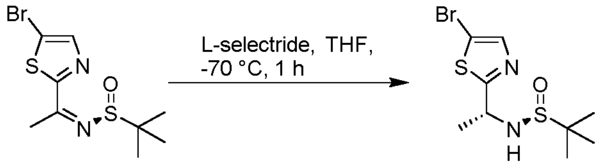


To a solution of (NZ,S)-N-[1-(2-bromothiazol-5-yl)ethylidene]-2-methyl-propane-2-sulfinamide (620 mg, 2.00 mmol) in THF (5 mL) was added L-selectride (1 M, 4.01 mL), followed by stirring at -70°C for 1 hour under $N_2$. The reaction mixture was quenched with sat. aq. $NH_4Cl$ (10 mL) at 20°C and extracted with EtOAc (10 mL × 3). The combined organic layer was dried over $Na_2SO_4$, filtered and concentrated under reduced pressure to obtain a residue. The residue was purified by silica gel column chromatography (25% EtOAc in PE) to give a main product, (S)-N-[(1R)-1-(5-bromothiazol-2-yl)ethyl]-2-methylpropane-2-sulfinamide (610 mg, 95% yield) as yellow oil. $^1$H NMR (400 MHz, $CDCl_3$) δ 7.62 (s, 1H), 4.84-4.75 (m, 1H), 3.55 (d, *J* = 6.4 Hz, 1H), 1.73 (d, *J* = 6.8 Hz, 3H), 1.30 (s, 9H); LC/MS (EI) m/z = 311.0 [M+H]$^+$.

Step 3: Synthesis of (R)-1-(5-bromothiazol-2-yl)ethyl]ethanamine

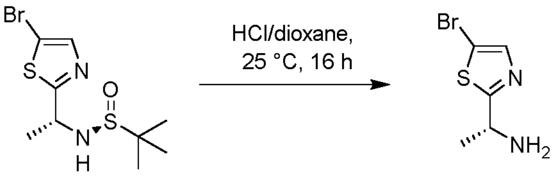


**Intermediate AA**

To a solution of (S)-N-[(1R)-1-(5-bromothiazol-2-yl)ethyl]-2-methylpropane-2-sulfinamide (200 mg, 643 μmol) in MeOH (2 mL) was added 4N HCl/dioxane solution (2.00 mL) at 0°C, followed by stirring at 20 °C for 1hour. The mixture was concentrated under reduced pressure to give the HCl salt of Intermediate AA as a yellow solid. $^1$H NMR (400 MHz, DMSO-$d_6$) δ 8.84 (s, 3H), 7.98 (s, 1H), 4.88-4.75 (m, 1H), 1.59 (d, *J* = 6.8 Hz, 3H)

**Preparation Example 27A: (R)-1-(2-bromothiazol-5-yl)ethanamine**

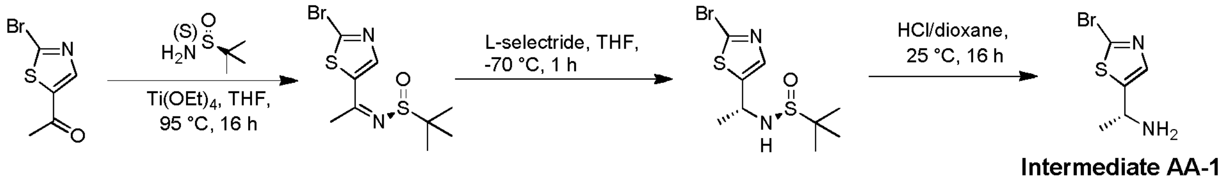


**Intermediate AA-1**

Intermediate AA-1 was prepared in the same manner as in Preparation Example 27. $^1$H NMR (400 MHz, DMSO-$d_6$) δ = 8.52 (s, 3H), 7.79 (s, 1H), 4.86 - 4.76 (m, 1H), 1.57 (d, *J* = 6.8 Hz, 3H).

**Preparation Example 28: methyl 1-[3-(dimethylcarbamoyl)-2-fluoro-phenyl]-4-hydroxy-6-oxo-pyridazine-3-carboxylate**

Step 1: Synthesis of 2-fluoro-N, N-dimethyl-3-nitrobenzamide

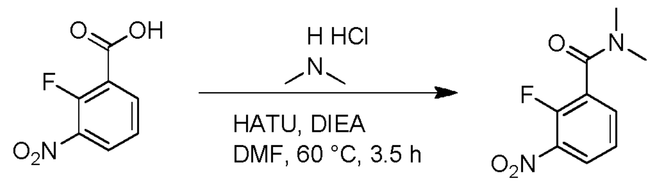


A mixture of 2-fluoro-3-nitro-benzoic acid (2.9 g, 15.67 mmol), N-methylmethanamine;hydrochloride (5.55 g, 47.00 mmol, HCl), DIEA (8.10 g, 62.67 mmol, 10.92 mL) and HATU (8.94 g, 23.50 mmol) in DMF (30 mL) was degassed and purged with $N_2$ for 3 times, and then the mixture was stirred at 60 °C for 3.5 hours under $N_2$ atmosphere. The reaction mixture was poured into water (250 mL) and extracted with EtOAc (100 mL × 3). The combined organic layer was washed with brine (50 mL × 4), dried over anhydrous $Na_2SO_4$ and concentrated under reduced pressure to obtain a crude product. The product was purified by flash silica gel chromatography (12% EtOAc in PE) to give 2-fluoro-N, N-dimethyl-3-nitrobenzamide (4.3 g, 68.56% yield) as a yellow solid. MS (ESI) m/z = 213.1 $[M+H]^+$.

Step 2: Synthesis of 3-amino-2-fluoro-N,N-dimethyl-benzamide

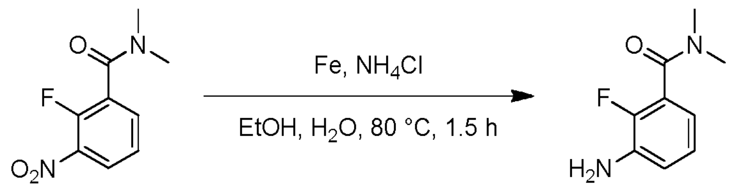


A mixture of 2-fluoro-N,N-dimethyl-3-nitro-benzamide (4.3 g, 20.27 mmol), Fe (11.32 g, 202.66 mmol) and $NH_4Cl$ (10.84 g, 202.66 mmol) in EtOH (40 mL) and $H_2O$ (8 mL) was degassed and purged with $N_2$ for 3 times, and then the mixture was stirred at 80 °C for 1.5 hours under $N_2$ atmosphere. The mixture was filtrated, and the filtrate was poured into water (50 mL) and extracted with EtOAc (50 mL × 4). The combined organic layer was washed with brine (50 mL × 2), dried over anhydrous $Na_2SO_4$ and concentrated under reduced pressure to give 3-amino-2-fluoro-N,N-dimethyl-benzamide (crude, 2.81 g, 71.75% yield). MS (ESI) m/z = 183.1 $[M+H]^+$.

Steps 3 and 4: Synthesis of methyl 1-[3-(dimethylcarbamoyl)-2-fluoro-phenyl]-4-hydroxy-6-oxo-pyridazine-3-carboxylate

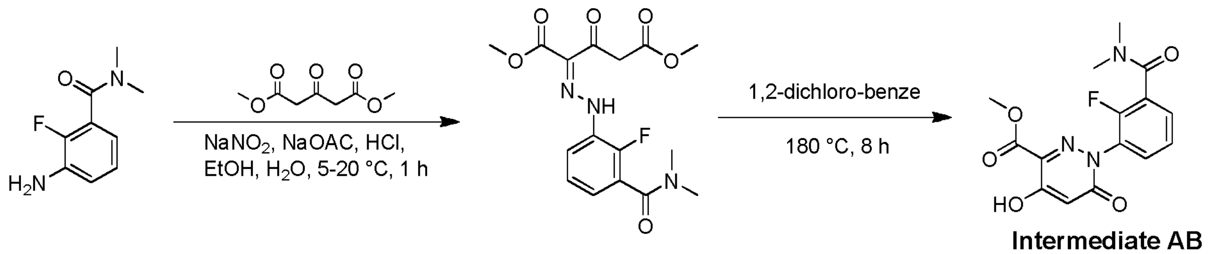


**Intermediate AB**

Intermediate AB was prepared in the same manner as in Steps 1 and 2 of Preparation Example 8 except that 3-amino-2-fluoro-N,N-dimethyl-benzamide was used instead of aniline in Step 1 of Preparation Example 8. $^1H$ NMR (400MHz, DMSO-$d_6$) $\delta$= 12.46 - 12.04 (m, 1H), 7.65 - 7.61 (m, 1H), 7.53 (ddd, $J$ = 1.6, 6.0, 7.6 Hz, 1H), 7.45 - 7.40 (m, 1H), 6.23 (s, 1H), 3.83 (s, 3H), 3.01 (s, 3H), 2.86 (s, 3H); MS (ESI) m/z = 336.1 $[M+H]^+$.

**Preparation Example 29: methyl 1-[3-(dimethylcarbamoyl)-2-fluoro-phenyl]-6-oxo-pyridazine-3-carboxylate**

Step 1: Synthesis of methyl 1-[3-(dimethylcarbamoyl)-2-fluoro-phenyl]-6-oxo-4-(trifluoromethylsulfonyloxy)pyridazine-3-carboxylate

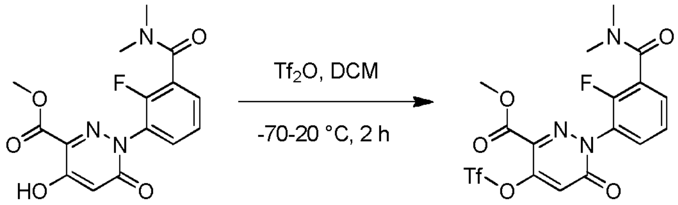


Intermediate AB

Methyl 1-[3-(dimethylcarbamoyl)-2-fluoro-phenyl]-6-oxo-4-(trifluoromethylsulfonyloxy)pyridazine-3-carboxylate (554 mg, 73.37% yield) was obtained as a yellow oil in the same manner as in Step 3 of Preparation Example 8. MS (ESI) m/z = 468.0 $[M+H]^+$.

Step 2: Synthesis of methyl 1-[3-(dimethylcarbamoyl)-2-fluoro-phenyl]-6-oxo-pyridazine-3-carboxylate

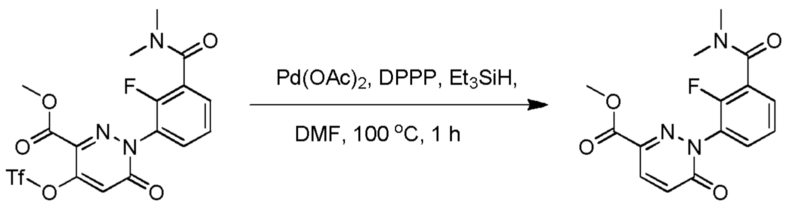


**Intermediate AC**

A mixture of methyl 1-[3-(dimethylcarbamoyl)-2-fluoro-phenyl]-6-oxo-4-(trifluoromethylsulfonyloxy)pyridazine-3-carboxylate (554 mg, 1.19 mmol), $Pd(OAc)_2$ (39.92 mg, 177.81 μmoL), DPPP (146.67 mg, 355.62 μmoL) and $Et_3SiH$ (179.19 mg, 1.54 mmol, 246.14 μL) in DMF (5 mL) was degassed and purged with $N_2$ for 3 times, and then the mixture was stirred at 100 °C for 1 hour under $N_2$ atmosphere. The reaction mixture was poured into water (30 mL) and extracted with EtOAc (30 mL × 3). The combined organic layer was washed with brine (30 mL × 2), dried over anhydrous $Na_2SO_4$ and concentrated under reduced pressure to obtain a residue. The residue was purified by flash silica gel chromatography (46% EtOAc in PE) to give Intermediate AC (215 mg, 50.51% yield) as yellow oil. MS (ESI) m/z = 320.0 $[M+H]^+$.

**Preparation Example 30: 5-(1-methylpyrazol-4-yl)-4-oxo-1H-pyrolo[2,3-d]pyridazine-7-carboxylic acid**

Steps 1 and 2: Synthesis of 4-hydroxy-1-(1-methyl-1H-pyrazol-4-yl)-6-oxo-1,6-dihydropyridazine-3-carboxylate

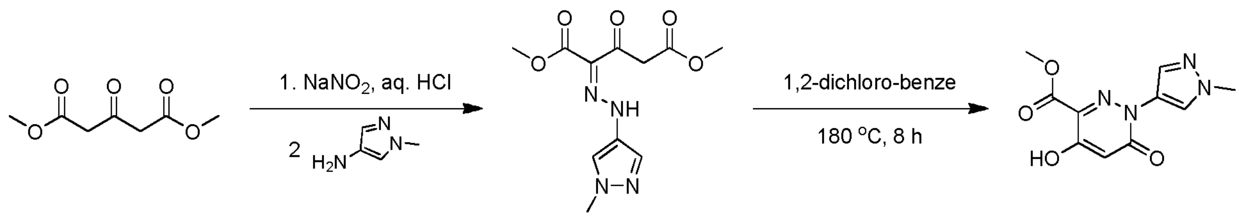


Methyl 4-hydroxy-1-(1-methyl-1H-pyrazol-4-yl)-6-oxo-1,6-dihydropyridazine-3-carboxylate was prepared in the same manner as in Steps 1 and 2 of Preparation Example 8 except that 1-methyl-1H-pyrazol-4-amine was used instead of aniline in Step 1 of Preparation Example 8. MS (ESI) m/z = 250.1 $[M+H]^+$.

Step 3: Synthesis of methyl 4-chloro-1-(1-methylpyrazol-4-yl)-6-oxo-pyridazine-3-carboxylate

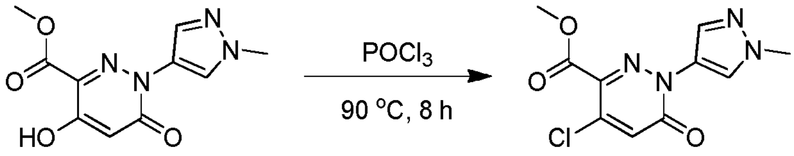


A mixture of methyl 4-hydroxy-1-(1-methylpyrazol-4-yl)-6-oxo-pyridazine-3-carboxylate (4.3 g, 17.19 mmol) in $POCl_3$ (50 mL) was degassed and purged with $N_2$ for 3 times, and then the mixture was stirred at 90 °C for 8 hours under $N_2$ atmosphere. The reaction mixture was poured into water (100 mL) and extracted with EtOAc (100 mL × 3). The combined organic layer was washed with brine (100 mL), dried over anhydrous $Na_2SO_4$ and concentrated under reduced pressure to obtain a crude product. Methyl 4-chloro-1-(1-methylpyrazol-4-yl)-6-oxo-pyridazine-3-carboxylate (crude, 4.1 g, 82.21% yield) as a yellow solid was used in the next step without further purification. $^1H$ NMR (400 MHz, DMSO-$d_6$) δ = 8.36 (s, 1H), 7.86 (s, 1H), 7.51 (s, 1H), 3.91 (s, 3H), 3.89 (s, 3H); MS (ESI) m/z = 268.9 $[M+H]^+$.

Step 4: Synthesis of methyl 4-azido-1-(1-methylpyrazol-4-yl)-6-oxo-pyridazine-3-carboxylate

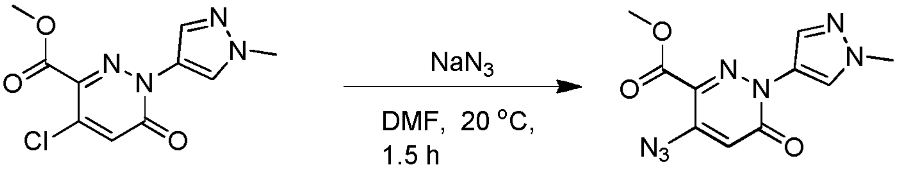


To a solution of methyl 4-chloro-1-(1-methylpyrazol-4-yl)-6-oxo-pyridazine-3-carboxylate (4.1 g, 15.26 mmol) in DMF (45 mL) was added $NaN_3$ (1.4 g, 21.54 mmol). The mixture was stirred at 20 °C for 5 hours. The reaction mixture was adjusted to PH > 9 with aq. $Na_2CO_3$, and extrated with EtOAc (100 mL × 3). The combined organic layer was washed with brine (100 mL), dried over anhydrous $Na_2SO_4$ and concentrated under reduced pressure to obtain a crude product. The aqueous layer was added with ice-cold water (aqueous layerwater = 1:50) and adjusted to pH= 11 with aq. NaOH. Then, the aqueous layer was added dropwise with sat. aq. NaClO (50 mL) and stirred at 20 °C for 12 hours. Methyl 4-azido-1-(1-methylpyrazol-4-yl)-6-oxo-pyridazine-3-carboxylate (crude, 4.2 g, 99.71% yield) as a yellow solid was used in the next step without further purification. MS (ESI) m/z = 275.9 $[M+H]^+$.

Step 5: Synthesis of methyl 4-amino-1-(1-methylpyrazol-4-yl)-6-oxopyridazine-3-carboxylate

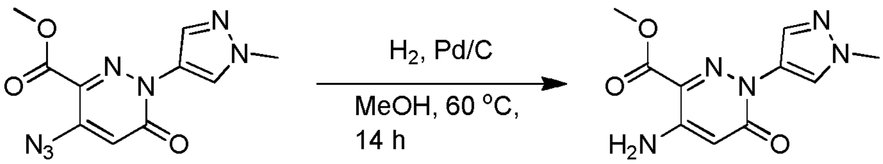


A mixture of methyl 4-azido-1-(1-methylpyrazol-4-yl)-6-oxo-pyridazine-3-carboxylate (4.2 g, 15.26 mmol) and Pd/C (1 g, 15.26 mmol, 10% purity) in MeOH (30 mL) and AcOH (30 mL) was degassed and purged with $H_2$ for 3 times, and then the mixture was stirred at 60 °C for 6 hours under $H_2$ atmosphere. The mixture was filtrated and concentrated under reduced pressure to give methyl 4-amino-1-(1-methylpyrazol-4-yl)-6-oxopyridazine-3-carboxylate (4 g, 68.17% yield) as a yellow solid. $^1H$ NMR (400 MHz, DMSO-$d_6$) δ= 8.23 (s, 1H), 7.76 (s, 1H), 7.02 (br s, 2H), 5.85 (s, 1H), 3.87 (s, 6H); MS (ESI) m/z = 249.9 $[M+H]^+$.

Step 6: Synthesis of methyl 4-amino-5-iodo-1-(1-methylpyrazol-4-yl)-6-oxo-pyridazine-3-carboxylate

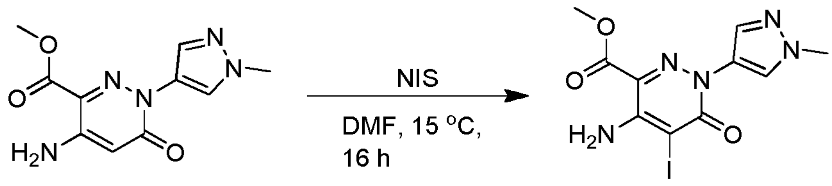


A mixture of methyl 4-amino-1-(1-methylpyrazol-4-yl)-6-oxo-pyridazine-3-carboxylate (4 g, 16.05 mmol), NIS (3.79 g, 16.85 mmol) in DMF (40 mL) was degassed and purged with $N_2$ for 3 times, and then the mixture was stirred at 20 °C for 3 hours under $N_2$ atmosphere. The mixture was filtered to obtain a residue. The residue was triturated with DCM (50 mL) for 30 min at 20 °C. The mixture was filtered to give methyl 4-amino-5-iodo-1-(1-methylpyrazol-4-yl)-6-oxo-pyridazine-3-carboxylate (2.4 g, 38.07% yield) as an off-white solid. $^1H$ NMR (400 MHz, DMSO-$d_6$) $\delta$ = 8.23 (br s, 1H), 7.78 (br s, 1H), 7.08 (br s, 2H), 3.88 (br s, 6H); MS (ESI) m/z = 375.8 $[M+H]^+$.

Step 7: Synthesis of methyl 4-amino-1-(1-methylpyrazol-4-yl)-6-oxo-5-(2-trimethylsilylethynyl)pyridazine-3-carboxylate

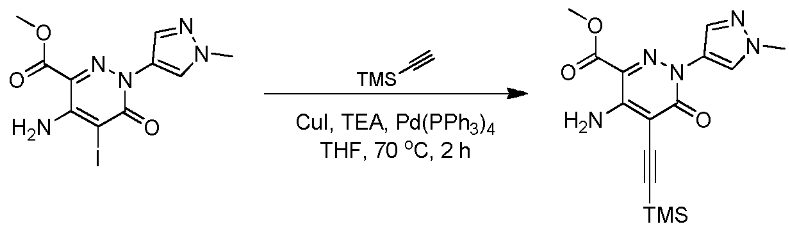


A mixture of methyl 4-amino-5-iodo-1-(1-methylpyrazol-4-yl)-6-oxo-pyridazine-3-carboxylate (900 mg, 2.40 mmol), ethynyl(trimethyl)silane (589.12 mg, 6.00 mmol), CuI (45.69 mg, 239.92 µmol), TEA (728.32 mg, 7.20 mmol, 1.00 mL) and $Pd(PPh_3)_2Cl_2$ (168.40 mg, 239.92 µmol) in THF (5 mL) was degassed and purged with $N_2$ for 3 times, and then the mixture was stirred at 70 °C for 2 hours under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The crude product was purified by flash silica gel chromatography (30% EtOAc in PE) to give methyl 4-amino-1-(1-methylpyrazol-4-yl)-6-oxo-5-(2-trimethylsilylethynyl)pyridazine-3-carboxylate (700 mg, 74.25% yield) as a yellow solid. MS (ESI) m/z = 346.1 $[M+H]^+$.

Step 8: Synthesis of 5-(1-methylpyrazol-4-yl)-4-oxo-1H-pyrolo[2,3-d]pyridazine-7-carboxylic acid

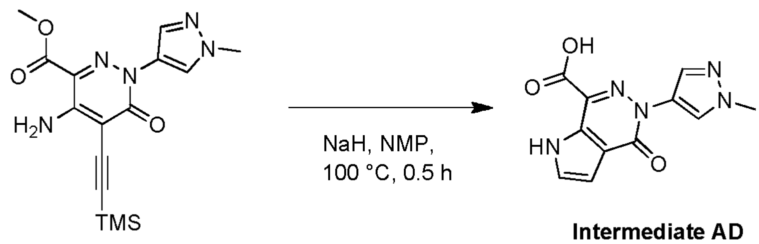


**Intermediate AD**

To a solution of methyl 4-amino-1-(1-methylpyrazol-4-yl)-6-oxo-5-(2-trimethylsilylethynyl)pyridazine-3-carboxylate (200 mg, 578.99 µmol) in NMP (2 mL) at 0 °C, was added NaH (27.79 mg, 694.79 µmol). The mixture was stirred at 100 °C for 0.5 hour under $N_2$ atmosphere. The reaction was quenched with EtOH slowly until no hydrogen was released. The reaction mixture was poured into water (30 mL) and extracted with EtOAc (30 mL × 3). The organic layer was discarded. The aqueous layer was adjusted to pH = 3-4 with aq.1 N HCl. The aqueous layer was purified by reversed-phase HPLC(10% MeOH in $H_2O$) to give Intermediate AD (110 mg, 65.52% yield) as a yellow solid. $^1H$ NMR (400 MHz, DMSO-$d_6$) $\delta$ = 11.83 (br s, 1H), 8.27 (br s, 1H), 7.89 (s, 1H), 7.40 (br s, 1H), 6.68 (br s,1H), 4.03 (q, *J* = 7.2 Hz, 1H), 3.88 (s, 3H); MS (ESI) m/z = 260.0 $[M+H]^+$.

Also, the following Intermediate AD-1 was prepared in the same manner as for Intermediate AD.

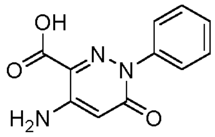

**Intermediate AD-1**

**Preparation Example 31: Methyl 4-oxo-5-phenyl-1H-pyrolo[2,3-d]pyridazine-7-carboxylate**

Steps 1 to 5: Synthesis of methyl 4-amino-6-oxo-1-phenyl-5-(2-trimethylsilylethynyl)pyridazine-3-carboxylate

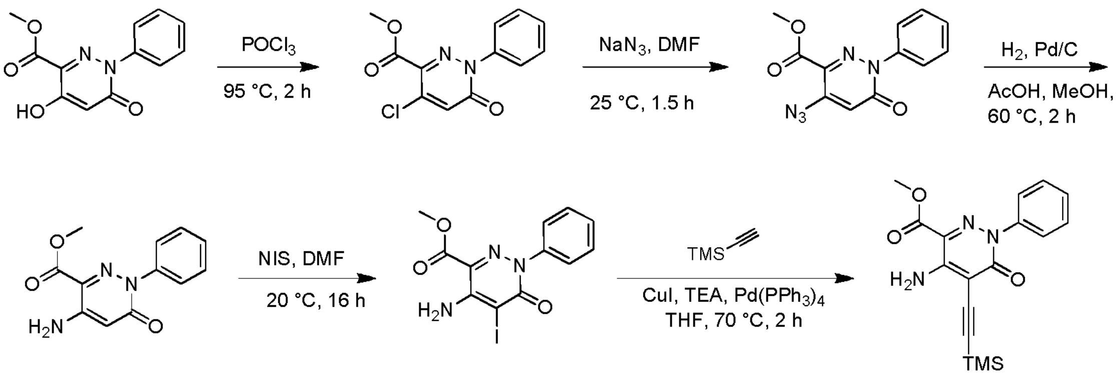


Methyl 4-amino-6-oxo-1-phenyl-5-(2-trimethylsilylethynyl)pyridazine-3-carboxylate was prepared in the same manner as in Steps 3 to 7 of Preparation Example 30, using methyl 4-hydroxy-6-oxo-1-phenyl-pyridazine-3-carboxylate obtained in Step 2 of Preparation Example 8 as a starting material.

Step 6: Synthesis of methyl 4-oxo-5-phenyl-1H-pyrolo[2,3-d]pyridazine-7-carboxylate

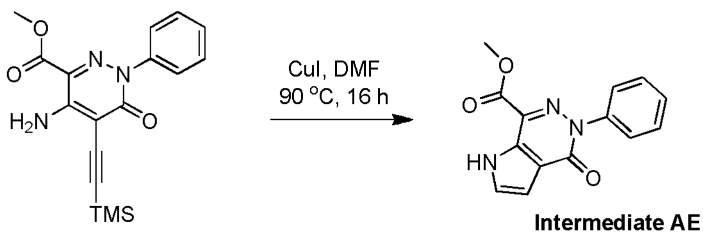


**Intermediate AE**

A mixture of methyl 4-amino-6-oxo-1-phenyl-5-(2-trimethylsilylethynyl)pyridazine-3-carboxylate (90 mg, 263.59 μmol) and CuI (25.10 mg, 131.80 μmol) in DMF (2 mL) was degassed and purged with $N_2$ for 3 times, and then the mixture was stirred at 100 °C for 16 hours under $N_2$ atmosphere. The reaction mixture was poured into water (30 mL) and extracted with EtOAc (30 mL × 3). The combined organic layer was washed with brine (30 mL), dried over anhydrous $Na_2SO_4$ and concentrated under reduced pressure to obtain a crude product. The residue was purified by flash silica gel chromatography (30% EtOAc in PE) to give Intermediate AE (20 mg, 25.99% yield) as a yellow solid. MS (ESI) m/z = 270.1 $[M+H]^+$

**Preparation Example 32: methyl 5-(2-fluorophenyl)-4-oxo-1H-pyrolo[2,3-d]pyridazine-7-carboxylate**

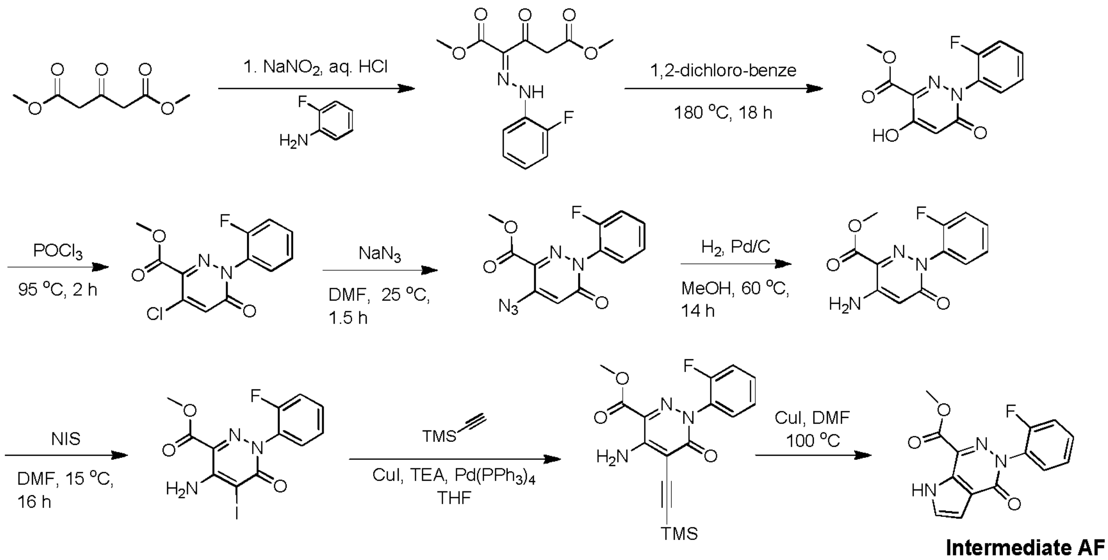


**Intermediate AF**

Intermediate AF (1.23 g, 58.08% yield) was prepared as a yellow solid in the same manner as in Preparation Example 30, using 2-fluoroaniline as a starting material. $^1$H NMR (400MHz, CBLOROFORM-*d*) $\delta$ = 10.09 (s, 1H), 7.39-7.51 (m, 2H), 7.33 (t, *J* = 2.8 Hz, 1H), 7.26-7.30 (m, 1H), 7.20-7.25 (m, 1H), 6.99 (t, J = 2.8 Hz, 1H), 4.02 (s, 3H); MS (ESI) m/z = 165.1 $[M+H]^+$.

**Preparation Example 33: Ethyl 4-oxo-5-phenyl-thieno[2,3-d]pyridazine-7-carboxylate**

Step 1: Synthesis of ethyl 2-(2-ethoxy-2-oxo-ethyl)thiophene-3-carboxylate

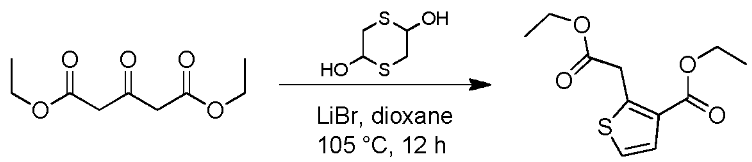


To a solution of 1,4-dithiane-2,5-diol (5.0 g, 32.84 mmol) and diethyl 3-oxopentanedioate (19.92 g, 98.53 mmol) in dioxane (50 mL) was added LiBr (855.75 mg, 9.85 mmol). The mixture was stirred at 105 °C for 12 hours. The mixture was concentrated under reduced pressure to obtain a residue. The residue was purified by silica gel column chromatography (10% EtOAc in PE) to give ethyl 2-(2-ethoxy-2-oxo-ethyl)thiophene-3-carboxylate (4.74 g, 59.56% yield) as colorless oil. $^1$H NMR (400 MHz, CHLOROFORM-d) $\delta$ = 7.45 (d, J=5.6 Hz, 1H), 7.14 (d, *J* = 5.6 Hz, 1H), 4.30 (q, *J*=7.2 Hz, 2H), 4.22-4.16 (m, 4H), 1.35 (t, *J* = 7.2 Hz, 3H), 1.27 (t, *J* = 7.2 Hz, 3H).

Step 2: Synthesis of ethyl 2-(2-ethoxy-2-oxoacetyl)thiophene-3-carboxylate

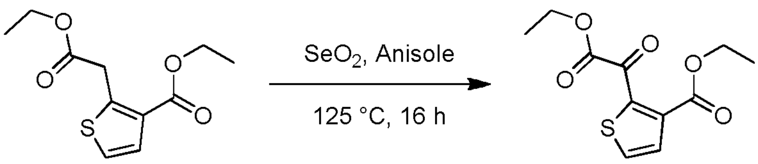


To a solution of ethyl 2-(2-ethoxy-2-oxo-ethyl)thiophene-3-carboxylate (2.0 g, 8.25 mmol) in anisole (50 mL) was added $SeO_2$ (2.29 g, 20.64 mmol). The mixture was stirred at 125 °C for 16 hours. The mixture was filtrated, and the filtrate was concentrated under reduced pressure to obtain a residue. The residue was purified by silica gel column chromatography (10% EtOAc in PE) to obtain a crude product. The product was purified by silica gel column chromatography (10% EtOAc in PE) to give ethyl 2-(2-ethoxy-2-oxoacetyl)thiophene-3-carboxylate (840 mg, 39.71% yield) as yellow oil. $^1$H NMR (400 MHz, CHLOROFORM-d) δ = 7.64 (d, *J* = 5.2 Hz, 1H), 7.47 (d, *J*= 5.2 Hz, 1H), 4.41-4.35 (m, 2H), 4.35-4.29 (m, 2H), 1.38 (td, J=7.2, 14.0 Hz, 6H).

Step 3: Synthesis of ethyl 4-oxo-5H-thieno[2,3-d]pyridazine-7-carboxylate

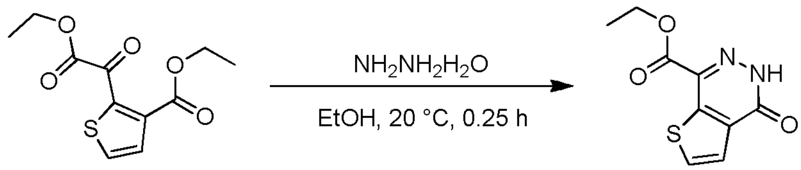


To a solution of ethyl 2-(2-ethoxy-2-oxo-acetyl)thiophene-3-carboxylate (640 mg, 2.50 mmol) in EtOH (7 mL) was added $NH_2NH_2 \cdot H_2O$ (160 mg, 3.13 mmol). The mixture was stirred at 20 °C for 0.25 hour. The reaction mixture was filtrated to obtain the filter cake. The product was purified by silica gel column chromatography (10% EtOAc in DCM) to give ethyl 4-oxo-5H-thieno[2,3-d]pyridazine-7-carboxylate (180 mg, 32.14% yield) as a white solid. $^1$H NMR (400 MHz, DMSO-$d_6$) δ = 13.95-12.84 (m, 1H), 8.16 (d, *J*=5.4 Hz, 1H), 7.66 (d, *J*=5.2 Hz, 1H), 4.40 (q, *J*=7.2 Hz, 2H), 1.36 (t, *J*=7.2 Hz, 3H); MS (ESI) m/z = 225.0 $[M+H]^+$.

Step 4: Synthesis of ethyl 4-oxo-5-phenyl-thieno[2,3-d]pyridazine-7-carboxylate

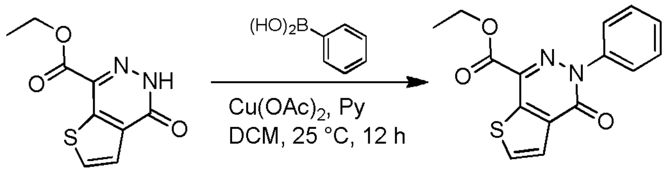


**Intermediate AG**

To a solution of ethyl 4-oxo-5H-thieno[2,3-d]pyridazine-7-carboxylate (180 mg, 802.73 μmol) and phenylboronic acid (146.81 mg, 1.20 mmol) in DCM (4 mL), pyridine (380.97 mg, 4.82 mmol) and $Cu(OAc)_2$ (29.16mg, 160.55 μmol) were added. The mixture was stirred at 25 °C for 12 hours under air. The mixture was concentrated under reduced pressure to obtain a residue. The residue was purified by silica gel column chromatography (100% EtOAc in PE) to give Intermediate AG (84 mg, 34.84% yield) as yellow oil. $^1$H NMR (400 MHz, DMSO-$d_6$) δ = 8.24 (d, *J* = 5.2 Hz, 1H), 7.74 (d, *J* = 5.2 Hz, 1H), 7.62-7.53 (m, 4H), 7.53-7.47 (m, 1H), 7.42-7.36 (m, 1H), 7.18-7.12 (m, 1H), 4.42 (q, *J* = 7.2 Hz, 2H), 1.34 (t, *J* = 7.2 Hz, 3H); MS (ESI) m/z = 300.9 $[M+H]^+$.

Also, the following Intermediate AG-1 was prepared in the same manner as for Intermediate AG.

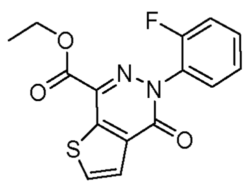

**Intermediate AG-1**

**Preparation Example 34: Ethyl 6-(2-fluorophenyl)-7-oxo-thieno[2,3-d]pyridazine-4-carboxylate**

Step 1: Synthesis of methyl 3-(2-ethoxy-2-oxo-acetyl)thiophene-2-carboxylate

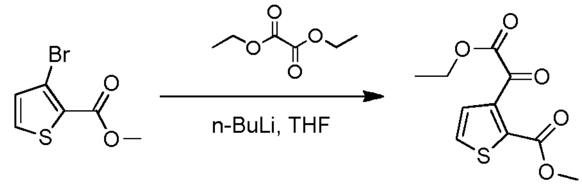


To a mixture of methyl 3-bromothiophene-2-carboxylate (5 g, 22.62 mmol) in THF (100 mL) were added n-BuLi (2.5 M, 9.95 mL) at -78 °C under $N_2$ atmosphere. After 10 minutes, the mixture was added wtih methyl 3-bromothiophene-2-carboxylate (5 g, 22.62 mmol) and diethyl oxalate (9.92 g, 67.85 mmol) in THF (100 mL) at -20 °C. After 10 minutes, the cold bath was removed and the reaction mixture was warmed to 15 °C. The mixture was diluted with sat. aq. $NH_4Cl$ (50 mL) and extracted with EtOAc (100 mL × 3). The combined organic layer was washed with brine (100 mL), dried over anhydrous $Na_2SO_4$ and concentrated under reduced pressure to obtain a residue. The residue was purified by silica gel column (PE/EtOAc = 10/1) to give methyl 3-(2-ethoxy-2-oxo-acetyl)thiophene-2-carboxylate (1.1 g) as yellow oil. $^1H$ NMR (400 MHz, DMSO-$d_6$) δ = 8.17 (d, *J*= 5.2 Hz, 1H), 7.65 (d, *J* = 5.2 Hz, 1H), 4.25 (s, 2H), 3.80 (s, 3H), 1.28 (s, 3H).

Step 2: Synthesis of ethyl 6-(2-fluorophenyl)-7-oxo-thieno[2,3-d]pyridazine-4-carboxylate

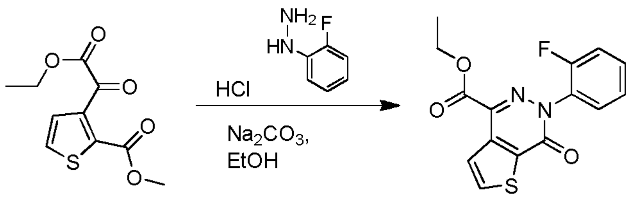


**Intermediate AH**

A mixture of methyl 3-(2-ethoxy-2-oxo-acetyl)thiophene-2-carboxylate (600 mg), (2-fluorophenyl)hydrazine (374.88 mg, 2.97 mmol) and $Na_2CO_3$ (525.03 mg, 4.95 mmol) in EtOH (15 mL) was degassed and purged with $N_2$ for 3 times, and then the mixture was stirred at 20 °C for 2 hours under $N_2$ atmosphere. The mixture was concentrated under reduced pressure to obtain a residue. The residue was purified by prep-HPLC (column: Phenomenex C18 75*30mm*3um;mobile phase: [water($NH_3H_2O$+$NH_4HCO_3$)-ACN];B%: 33%-63%,10min). The desired fraction was concentrated under reduced pressure, and the remaining solvent was removed by lyophilization to afford Intermediate AH (54 mg, 13.47% 2-steps yield) as a yellow solid. MS (ESI) m/z = 319.1 $[M+H]^+$.

**Preparation Example 35: 1-(difluoromethyl)-5-iodo-pyrazole**

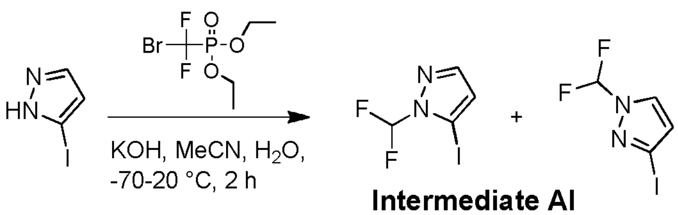


**Intermediate AI**

To a mixture of 5-iodo-1H-pyrazole (1.21 g, 6.24 mmol) and KOH (4.20 g, 74.90 mmol) in MeCN (10 mL)/$H_2O$ (10 mL) was added 1-[[bromo(difluoro)methyl]-ethoxy-phosphoryl]oxyethane (5 g, 18.73 mmol) at -70 °C. Then, the mixture was stirred at 20°C for 2 hours under $N_2$. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by silica gel column chromatography (3% EtOAc in PE) to give Intermediate AI (0.54 g, 34.71% yield) as a colorless oil. $^1H$ NMR (400 MHz, DMSO-$d_6$) δ ppm 6.76 (d, J=2.6 Hz, 1 H) 6.79 (d, *J* = 1.6 Hz, 1 H) 7.64 (s, 1 H) 7.70 (s, 1 H) 7.79 (s, 1 H) 7.80 (m, 1 H) 7.85 (s, 1 H) 7.94 (s, 1 H) 7.99 (s, 1 H) 8.15 (d, J=2.6 Hz, 1 H); MS (ESI) m/z = 244.9 $[M+1+H]^+$.

**Preparation Example 36: (R)-1-(3-(difluoromethyl)-5-nitrophenyl)ethanamine**

Step 1: Synthesis of 1-bromo-3-(difluoromethyl)-5-nitro-benzene

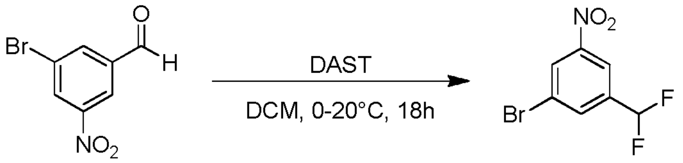


A mixture of 3-bromo-5-nitro-benzaldehyde (13.7 g, 59.56 mmol) and DAST (48.00 g, 297.81 mmol, 39.35 mL) in DCM (140 mL) was degassed and purged with $N_2$ for 3 times, stirred at 0-20 °C for 18 hours, and then the mixture was stirred at 0-20 °C for 18 hours under $N_2$ atmosphere. The resulting solution was poured over ice and extracted with dichloromethane (300 mL). Then, the reaction mixture was extracted with EtOAc (200 mL×3). The combined organic layer was washed with brine (200 mL×2), dried over anhydrous $Na_2SO_4$ and concentrated under reduced pressure to obtain a crude product. The product was purified by silica gel column chromatography (8% EtOAc in PE) to give 1-bromo-3-(difluoromethyl)-5-nitro-benzene (14.27 g, 56.62 mmol, 95.07% yield) as colorless oil. $^1$H NMR (400MHz, DMSO-$d_6$) δ = 8.57 (s, 1H), 8.40 (s, 1H), 8.29 (s, 1H), 7.34 - 7.05 (m, 1H)

Steps 2 to 5: Synthesis of (R)-1-(3-(difluoromethyl)-5-nitrophenyl)ethanamine

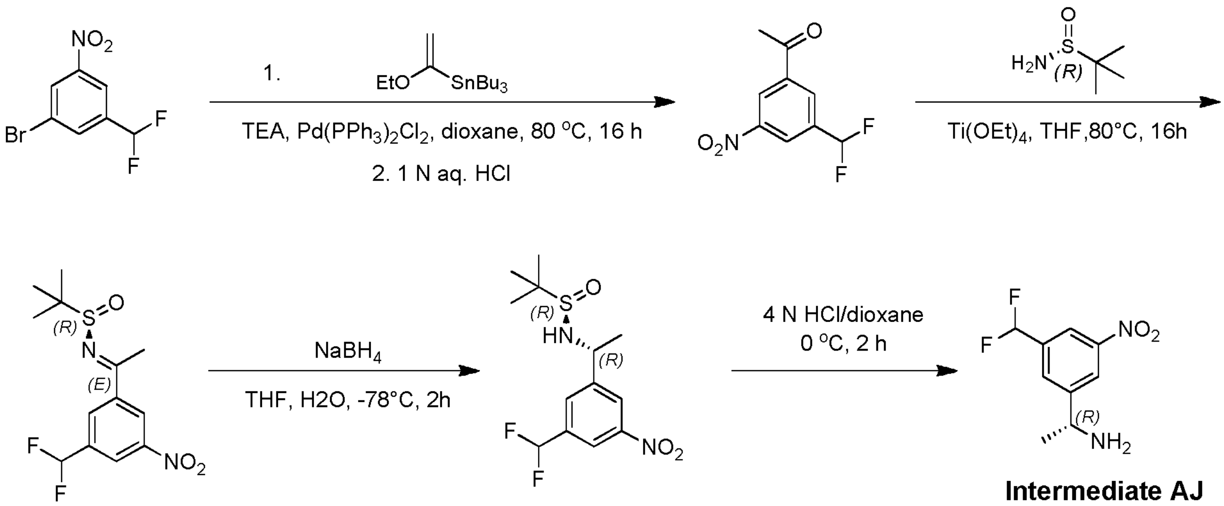


**Intermediate AJ**

Intermediate AJ was prepared in the same manner as in Preparation Example 13, using 1-bromo-3-(difluoromethyl)-5-nitro-benzene. MS (ESI) m/z = 216.1 [M+H]$^+$.

**Preparation Example 37: Methyl 1-(2-fluorophenyl)-4-hydroxy-6-oxo-1,6-dihydropyridazine-3-carboxylate**

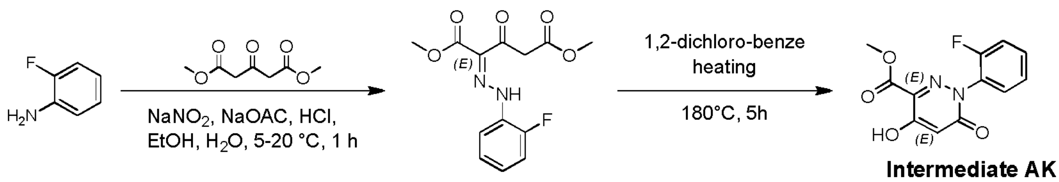


**Intermediate AK**

Intermediate AK was prepared in the same manner as in Steps 1 and 2 of Preparation Example 8, using 2-fluoroaniline instead of aniline. MS (ESI) m/z = 264.1 $[M+H]^+$.

**Preparation Example 38: methyl 1-(2-fluoro-4-methoxyphenyl)-6-oxo-4-(((trifluoromethyl)sulfonyl)oxy)-1,6-dihydropyridazine-3-carboxylate**

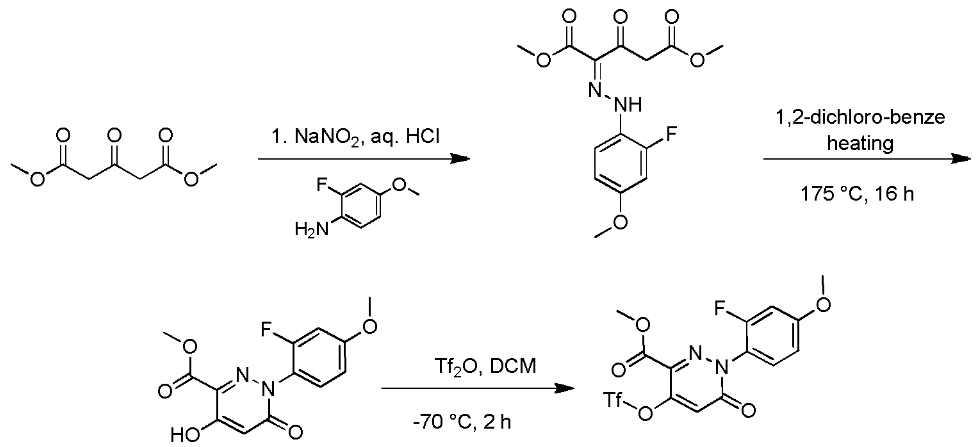


**Intermediate AL**

Intermediate AL was prepared in the same manner as in Preparation Example 8, using 2-fluoro-4-methoxyaniline. MS (ESI) m/z = 426.0 $[M+H]^+$.

**Preparation Example 39: methyl 4-amino-1-(2-fluorophenyl)-6-oxo-1,6-dihydropyridazine-3-carboxylate**

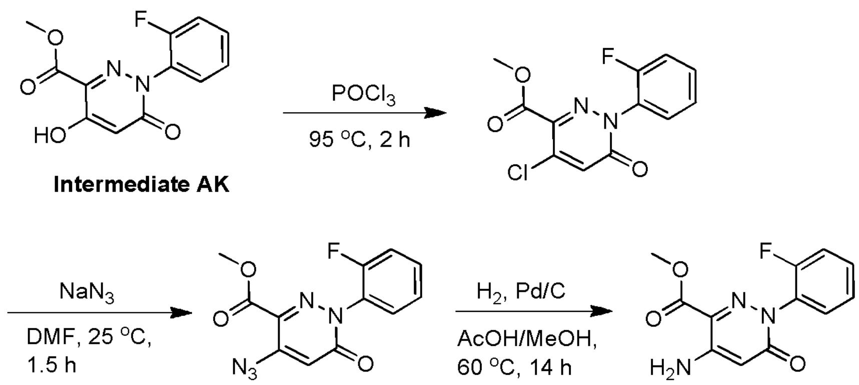


**Intermediate AK**

**Intermediate AM**

Intermediate AM was prepared in the same manner as in Steps 3 to 5 of Preparation Example 30. MS (ESI) m/z = 246.1 $[M+H]^+$

**Preparation Example** 40: **Methyl 5-amino-6-oxo-1-phenyl-pyridazine-3-carboxylate**

<u>Step 1: Synthesis of methyl 5-(tert-butoxycarbonylamino)-6-oxo-1-phenyl-pyridazine-3-carboxylate</u>

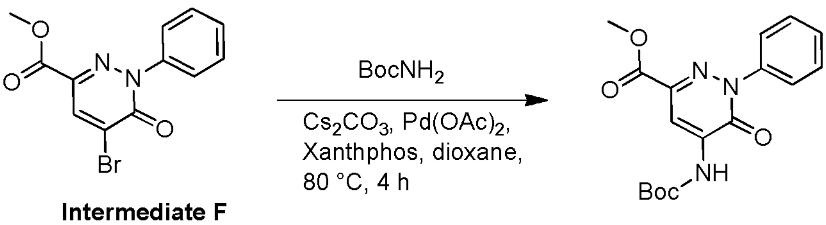


Intermediate F

A mixture of Intermediate F (200.00 mg, 647.01 μmol), tert-butyl carbamate (151.59 mg, 1.29 mmol), $Pd(OAc)_2$ (7.26 mg, 32.35 μmol), Xantphos (56.16 mg, 97.05 μmol) and $Cs_2CO_3$ (421.62 mg, 1.29 mmol) in dioxane (3 mL) was degassed and purged with $N_2$ for 3 times, and then the mixture was stirred at 80 °C for 4 hours under $N_2$ atmosphere. The reaction mixture was poured into water (10 mL) and extracted with EtOAc (20 mL × 3). The combined organic layer was washed with brine (30 mL), dried over anhydrous $Na_2SO_4$ and concentrated under reduced pressure to obtain a crude product. The crude product was purified by flash silica gel chromatography (9% EtOAc in PE) to give methyl 5-(tert-butoxycarbonylamino)-6-oxo-1-phenyl-pyridazine-3-carboxylate (120 mg, 44.49% yield) as a yellow oil. MS (ESI) m/z = 346.0 $[M+H]^+$.

Step 2: Synthesis of methyl 5-amino-6-oxo-1-phenyl-pyridazine-3-carboxylate

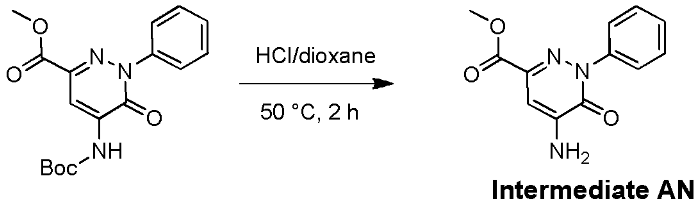


**Intermediate AN**

A mixture of methyl 5-(tert-butoxycarbonylamino)-6-oxo-1-phenyl-pyridazine-3-carboxylate (120.00 mg, 347.47 μmol) in HCl/dioxane (3 mL) and was stirred at 50 °C for 2 hours under air. The mixture was concentrated under reduced pressure to obtain a crude product. Intermediate AN (crude, 110 mg, 95.06% yield, HCl) as a yellow solid was used in the next step without further purification. MS (ESI) m/z = 246.0 $[M+H]^+$.

**Preparation Example 41: Methyl 5-methyl-6-oxo-1-phenylpyridazine-3-carboxylate**

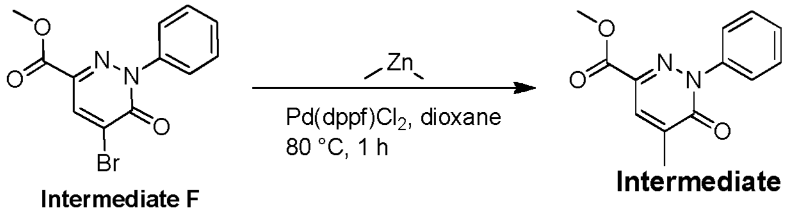


Intermediate F

**Intermediate AO**

A mixture of Intermediate F (200 mg, 647.01 μmol), dimethylzinc (30.88 mg, 323.51 μmol) and $Pd(dppf)Cl_2$ (94.68 mg, 129.40 μmol) in dioxane (3 mL) was degassed and purged with $N_2$ for 3 times, and then the mixture was stirred at 80 °C for 1 hour under $N_2$ atmosphere. The reaction mixture was poured into water (10 mL) and extracted with EtOAc (20 mL × 3). The combined organic layer was washed with brine (30 mL), dried over anhydrous $Na_2SO_4$ and concentrated under reduced pressure to obtain a crude product. The crude product was purified by flash silica gel chromatography (10% EtOAc in PE) to give Intermediate AO (55 mg, 32.89% yield) as a brown solid. MS (ESI) m/z = 245.0 $[M+H]^+$.

**Preparation Example 42: methyl 4-[tert-butoxycarbonyl(methyl)amino]-1-(2-fluorophenyl)-6-oxo-pyridazine-3-carboxylate**

Step 1: Synthesis of methyl 4-(tert-butoxycarbonylamino)-1-(2-fluorophenyl)-6-oxo-pyridazine-3-carboxylate

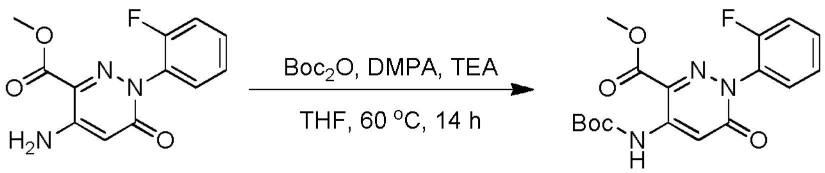


**Intermediate AM**

A mixture of Intermediate AM (300 mg, 1.14 mmol), DMAP (69.62 mg, 569.86 μmol), TEA (172.99 mg, 1.71 mmol) and $Boc_2O$ (298.49 mg, 1.37 mmol) in THF (2 mL) was stirred at 60 °C for 14 hours. The mixture was diluted with water (50 mL) and extracted with EtOAc (50 mL × 3). The combined organic layer was washed with brine (50 mL), dried over anhydrous $Na_2SO_4$ and concentrated under reduced pressure to give methyl 4-(tert-butoxycarbonylamino)-1-(2-fluorophenyl)-6-oxo-pyridazine-3-carboxylate (558 mg, crude) as yellow oil. MS (ESI) m/z = 364.1 $[M+H]^+$

Step 2: Synthesis of methyl 4-[tert-butoxycarbonyl(methyl)amino]-1-(2-fluorophenyl)-6-oxo-pyridazine-3 -carboxylate

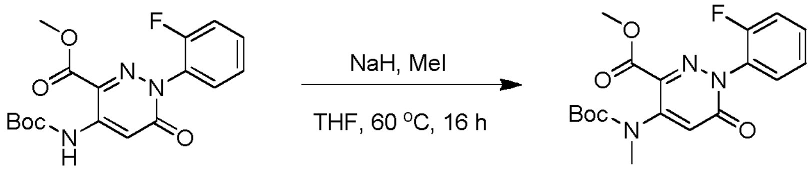


**Intermediate AP**

To a mixture of methyl 4-(tert-butoxycarbonylamino)-1-(2-fluorophenyl)-6-oxo-pyridazine-3-carboxylate (558 mg, crude) in THF (10 mL) wase added NaH (184.27 mg, 4.61 mmol, 60% purity) at 0 °C for 0.5 hour under $N_2$ atmosphere, then MeI (1.09 g, 7.68 mmol) was added thereto, and the mixture was stirred at 60 °C for 16 hours under $N_2$ atmosphere. The mixture was diluted with water (50 mL) and extracted with EtOAc (50 mL × 3). The combined organic layer was washed with brine (50 mL), dried over anhydrous $Na_2SO_4$ and concentrated under reduced pressure to obtain a residue. The residue was purified by silica gel column (PE/EtOAc = 5/1) to give Intermediate AP (220 mg, 51.14% 2-steps yield) as a yellow solid. $^1H$ NMR (400MHz, DMSO-$d_6$) δ = 7.55-7.62 (m, 2H), 7.36-7.48 (m, 2H), 7.12 (s, 1H), 3.78 (s, 3H), 3.26 (s, 3H), 1.40 (s, 9H); MS (ESI) m/z = 378.2 $[M+H]^+$.

**Preparation Example 43: 1-(2,3-difluorophenyl)-6-oxo-1,6-dihydropyridine-3-carboxylic acid**

Step 1: Synthesis of methyl 1-(2,3-difluorophenyl)-6-oxo-1,6-dihydropyridine-3-carboxylate

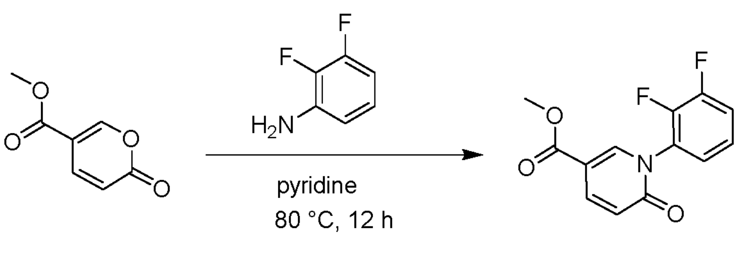


A mixture of methyl 2-oxo-2H-pyran-5-carboxylate (100 mg, 0.65 mmol) and 2,3-difluoroaniline (84 mg, 0.65

mmol) in pyridine (2 mL) was stirred at 80 °C for overnight. The mixture was added with DW and extracted with EtOAc. The organic layer was washed with water and 1N HCl, dried over $MgSO_4$, filtered and concentrated under reduced pressure to obtain a crude product. Methyl 1-(2,3-difluorophenyl)-6-oxo-1,6-dihydropyridine-3-carboxylate (51 mg, crude) was obtained as a yellow solid and used in the next step without further purification. LC/MS (ESI) m/z = 266.1 $[M+H]^+$.

Step 2: Synthesis of 1-(2,3-difluorophenyl)-6-oxo-1,6-dihydropyridine-3-carboxylic acid

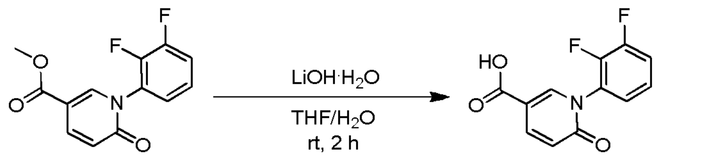


**Intermediate AQ**

To a solution of methyl 1-(2,3-difluorophenyl)-6-oxo-1,6-dihydropyridine-3-carboxylate (90mg, 0.34mmol) in THF (4 mL) and $H_2O$ (2 mL) was added $LiOH \cdot H_2O$ (35.6 mg, 0.84 mmol). The mixture was stirred at room temperature for 2 hours. The reaction mixture was poured into water and extracted with EtOAc. The aqueous layer was adjusted to pH = 3-4 with aq.1 N HCl and then extracted with EtOAc (10 mL × 2). The organic layer was washed with water (20 mL) and brine (20 mL), dried over $MgSO_4$, filtered and concentrated under reduced pressure to give Intermediate AQ (43.9 mg, 51.5% yield) as a white solid. LC/MS (ESI) m/z = 266.1 $[M+H]^+$.

Also Intermediates AQ-1, AQ-2, AQ-3, and AQ-4 were prepared in the same manner as for Intermediate AQ.

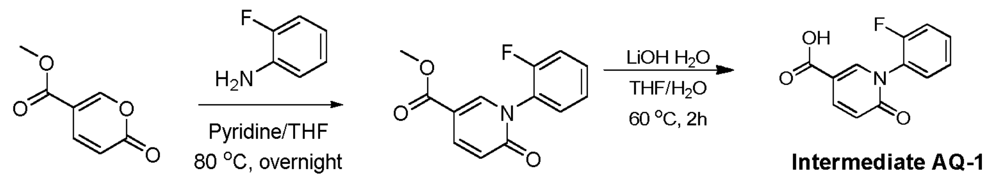


**Intermediate AQ-1**

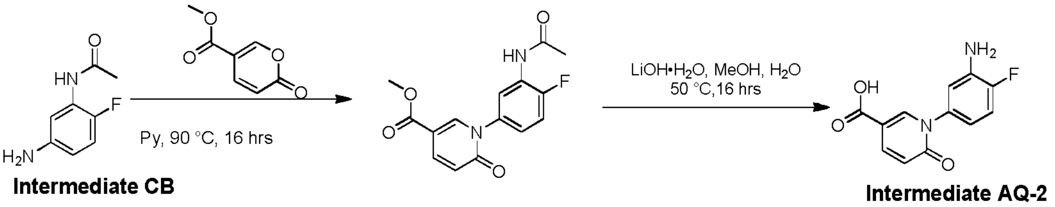


**Intermediate CB**

**Intermediate AQ-2**

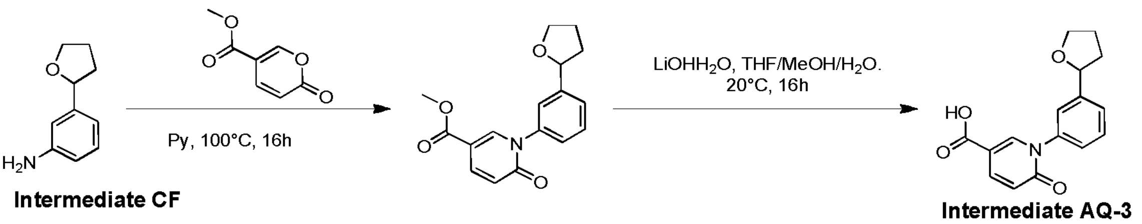


**Intermediate CF**

**Intermediate AQ-3**

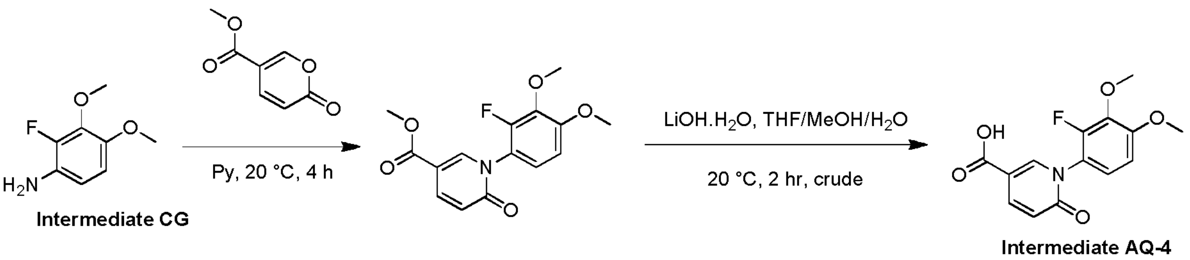


Intermediate CG

Intermediate AQ-4

Preparation Example 44: 1-(1-acetylpiperidin-4-yl)-6-oxo-1,6-dihydropyridazine-3-**carboxylic acid**

Step 1: Synthesis of methyl 1-(1-acetyl-4-piperidyl)-6-oxo-pyridazine-3-carboxylate

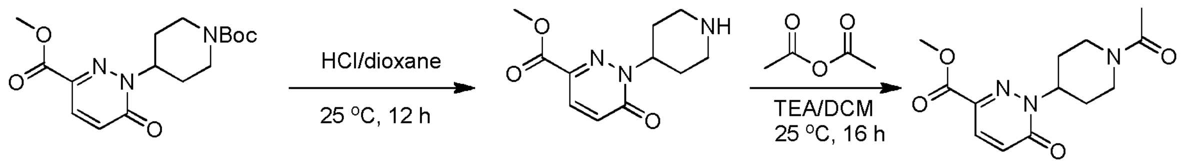


**Intermediate G-3**

The Boc group of Intermediate G-3 was deprotected. Subsequently, a mixture of methyl 6-oxo-1-(4-piperidyl)pyridazine-3-carboxylate (100 mg, 421.49 μmol), $Ac_2O$ (60.00 mg, 587.73 μmol, 55.05 μL) and TEA (127.95 mg, 1.26 mmol, 176.00 μL) in DCM (6 mL) was degassed and purged with $N_2$ for 3 times, and then stirred at 25 °C for 16 hours under $N_2$ atmosphere. The reaction mixture was poured into water (30 mL) and extracted with EtOAc (30 mL × 3). The combined organic layer was washed with brine (30 mL × 2), dried over anhydrous $Na_2SO_4$ and concentrated under reduced pressure to obtain a crude product. The product was purified by silica gel column chromatography (97% EtOAc in PE) to give methyl 1-(1-acetyl-4-piperidyl)-6-oxo-pyridazine-3-carboxylate (184.9 mg, 63.16% yield) as a white solid. MS (ESI) m/z = 280.0 $[M+H]^+$

Step 2: Synthesis of 1-(1-acetylpiperidin-4-yl)-6-oxo-L6-dihydropyridazine-3-carboxylic acid

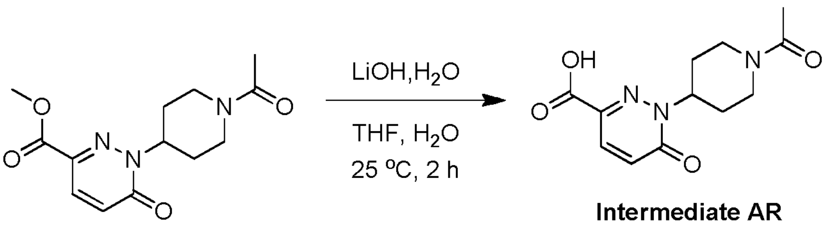


**Intermediate AR**

Intermediate AR was prepared in the same manner as in Step 2 of Preparation Example 43.

**Preparation Example 45: 1-(2-fluorophenyl)-6-oxo-1,6-dihydropyridazine-3-carboxylic acid**

Step 1: Synthesis of dimethyl (2Z)-2-(phenylhydrazono)pentanedioate

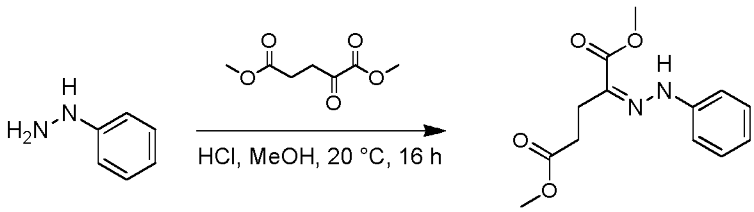


A mixture of dimethyl 2-oxopentanedioate (200 mg, 1.15 mmol), phenylhydrazine (124.36 mg, 1.15 mmol, 113.06 μL) and HCl (23.29 mg, 230.00 μmol, 22.84 μL) in MeOH (2 mL) was degassed and purged with $N_2$ for 3 times, and then the mixture was stirred at 20 °C for 16 hours under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a crude product. The residue was purified by flash silica gel chromatography (12% EtOAc in PE) to give dimethyl (2Z)-2-(phenylhydrazono)pentanedioate (400 mg, 64.88% yield) as a yellow oil. $^1H$ NMR (400 MHz, DMSO-$d_6$) δ = 8.36 (s, 1H), 7.86 (s, 1H), 7.51 (s, 1H), 3.91 (s, 3H),

3.89 (s, 3H); MS (ESI) m/z = 265.0 $[M+H]^+$.

Step 2: Synthesis of 6-oxo-1-phenyl-4,5-dihydropyridazine-3-carboxylic acid

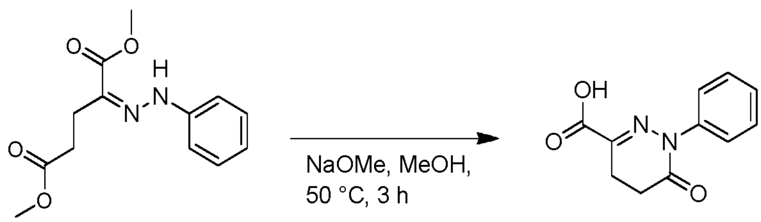


**Intermediate AS**

A mixture of dimethyl (2Z)-2-(phenylhydrazono)pentanedioate (270 mg, 1.02 mmol) and NaOMe (66.23 mg, 1.23 mmol) in MeOH (1 mL) was degassed and purged with $N_2$ for 3 times, and then the mixture was stirred at 50 °C for 3 hours under $N_2$ atmosphere. The reaction mixture was poured into water (30 mL) and extracted with EtOAc (30 mL × 3). The aqueous layer was adjusted to pH = 3-4 with aq.1 N HCl and then extracted with EtOAc (30 mL × 3). The organic layer was washed with water (30 mL) and brine (30 mL), dried over $Na_2SO_4$, filtered and concentrated under reduced pressure to obtain a residue. Intermediate AS (100 mg, 13.80% yield) was obtained as yellow oil and used in the next step without further purification. MS (ESI) m/z = 218.9 $[M+H]^+$.

**Preparation Example 46: methyl 6-oxo-1-[3-(5-trimethylsilylisoxazol-3-yl)phenyl]pyridazine-3-carboxylate**

Step 1: Synthesis of methyl 1-(3-formylphenyl)-6-oxo-1,6-dihydropyridazine-3-carboxylate

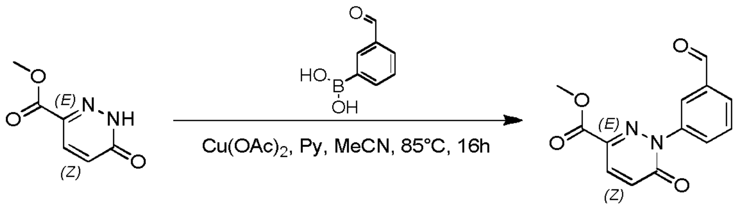


Methyl 1-(3-formylphenyl)-6-oxo-1,6-dihydropyridazine-3-carboxylate was prepared in the same manner as in Preparation Example 11. MS (ESI) m/z = 258.1 $[M+H]^+$.

Step 2: Synthesis of methyl 1-[3-(hydroxyiminomethyl)phenyl]-6-oxo-pyridazine-3-carboxylate

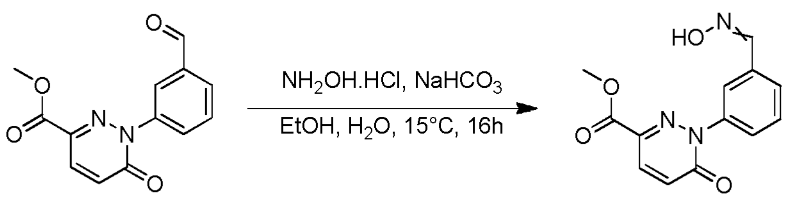


To a solution of $NaHCO_3$ (39.04 mg, 464.70 μmol, 18.07 μL) in $H_2O$ (5 mL) was added $NH_2OH.HCl$ (32.29 mg, 464.70 μmol). The resulting solution was then added to a vigorously stirred suspension of methyl 1-(3-formylphenyl)-6-oxo-pyridazine-3-carboxylate (100 mg, 387.25 μmol) in EtOH (5 mL) at 15 °C for 15 hours. The product was filtered. Then, the filtrate was poured into water (20 mL) and extracted with EtOAc (20 mL×4). The combined organic layer was washed with brine (520 mL×2), dried over anhydrous $Na_2SO_4$ and concentrated under reduced pressure to give methyl 1-[3-(hydroxyiminomethyl)phenyl]-6-oxo-pyridazine-3-carboxylate (crude, 100 mg, 317.70 μmol, 82.04% yield). MS (ESI)

m/z = 273.9 [M+H]+.

Step 3: Synthesis of methyl 6-oxo-1-[3-(5-trimethylsilylisoxazol-3-yl)phenyl]pyridazine-3-carboxylate

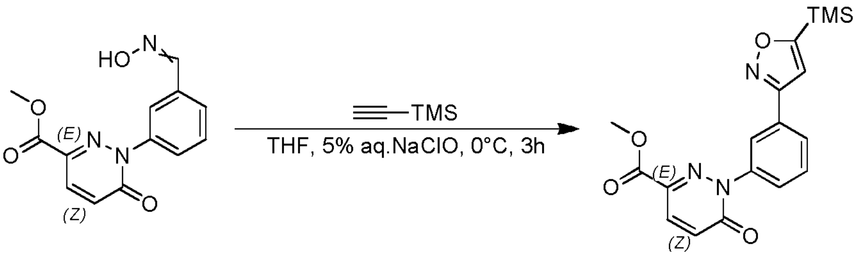


**Intermediate AT**

To a solution of ethynyl(trimethyl)silane (107.84 mg, 1.10 mmol, 152.10 μL) and NaClO (0.6 mL, 5% purity) in THF (1 mL) was added a solution of methyl 1-[3-(hydroxyiminomethyl)phenyl]-6-oxo-pyridazine-3-carboxylate (100 mg, 365.97 μmol) obtained in THF (1 mL) at 0°C, and the mixture was stirred for 3 hours. The reaction mixture was poured into water (50 mL) and extracted with EtOAc (50 mL×3). The combined organic layer was washed with brine (30 mL×2), dried over anhydrous $Na_2SO_4$ and concentrated under reduced pressure to obtain a crude product. The residue was purified by flash silica gel chromatography (18% EtOAc in PE) to give Intermediate AT (35 mg, 76.38 μmol, 20.87% yield) as a white solid. MS (ESI) m/z = 370.1 [M+H]+.

**Preparation Example 47: 2-[(1S)-1-aminomethyl]-6-(trifluoromethyl)pyridin-4-amine and 2-[(1R)-1-aminomethyl]-6-(trifluoromethyl)pyridin-4-amine**

Step 1: Synthesis of 1-[4-amino-6-(trifluoromethyl)-2-pyridyl]ethanone

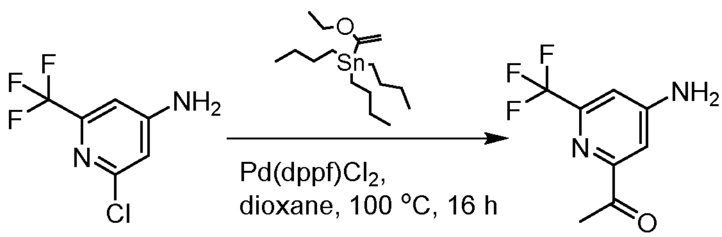


1-tributyl(1-ethoxyvinyl)stannane (2.23 g, 2.09 mmol) and Pd(dppf)$Cl_2$ (93.1 mg, 127 μmol) were added to a mixture of 2-chloro-6-(trifluoromethyl)pyridin-4-amine (500 mg, 2.54 mmol) in dioxane (5 mL), and the mixture was degassed, purged with N2 three times and stirred at 100 °C for 16 hours under $N_2$ atmosphere. The mixture was added with 1N HCl solution (3 mL) and stirred at 25 °C for 30 minutes. The mixture was quenched with sat. aq. CsF (30 mL) and extracted with EtOAc (10 mL X 3). The combined organic layer was washed with brine, dried over $Na_2SO_4$ and concentrated under reduced pressure to obtain a residue, which was purified by silica gel column chromatography (20% EtOAc in PE) to give 1-[4-amino-6-(trifluoromethyl)-2-pyridyl]ethanone (420 mg, 80.0% yield) as yellow oil. $^1H$ NMR (400 MHz, CHLOROFORM-d) δ 7.37 (d, J = 2.4 Hz, 1H), 7.02 (d, J = 2.4 Hz, 1H), 4.56 (s, 2H), 2.70 (s, 3H).

Steps 2 to 4: Synthesis of (R)-N-[(1S)-1-[4-amino-6-(trifluoromethyl)-2-pyridyl]ethyl]-2-methyl-propane)-2-sulfinamide and (R)-N-[(1R)-1-[4-amino-6-(trifluoromethyl)-2-pyridyl]ethyl]-2-methyl-propane)-2-sulfinamide

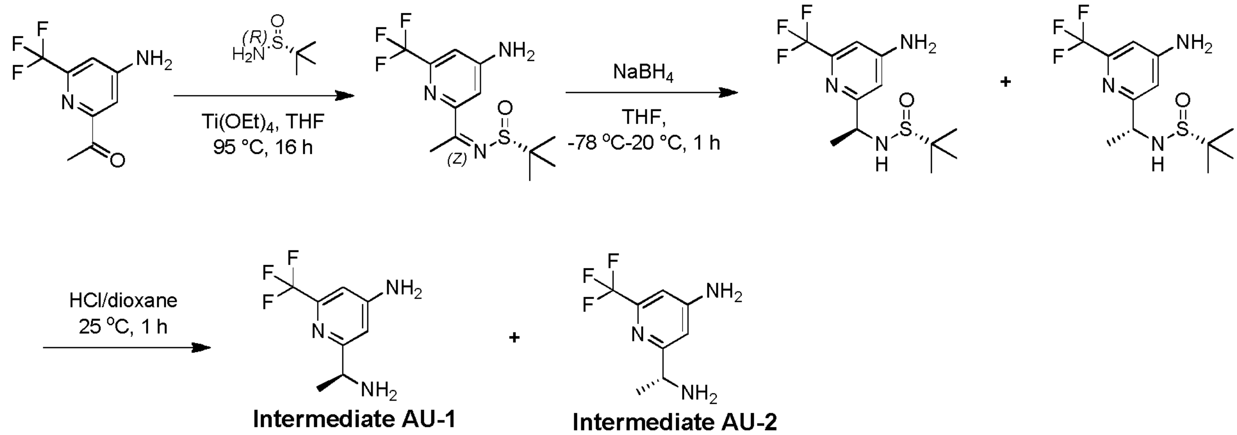


**Intermediate AU-1** **Intermediate AU-2**

Intermediate AU-1 and AU-2 were prepared in the same method as in Steps 2 to 4 of Preparation Example 2. $^1$H NMR (400 MHz, DMSO-$d_6$) δ = 8.28 (d, J = 1.2 Hz, 3H), 6.91 (d, J = 1.6 Hz, 1H), 6.71 (d, J = 1.6 Hz, 1H), 4.38 - 4.27 (m, 1H), 1.44 (d, J = 6.8 Hz, 3H). $^1$H NMR (400 MHz, DMSO-$d_6$) δ = 8.28 (d, J = 1.2 Hz, 3H), 6.91 (d, J = 1.6 Hz, 1H), 6.71 (d, J = 1.6 Hz, 1H), 4.38 - 4.27 (m, 1H), 1.44 (d, J = 6.8 Hz, 3H).

**Preparation Example I: 1-(5-bromothiophen-3-yl)-N-methylmethanamine**

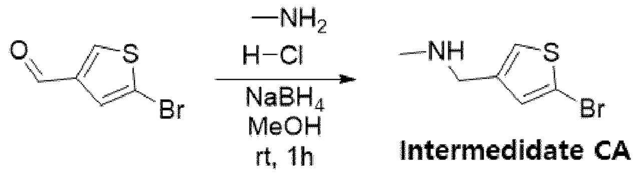


To a solution of 5-bromothiophene-3-carbaldehyde (0.3 ml, 2.75 mmol) in MeOH (5 mL) was added methylamine hydrochloride (223 mg, 3.30 mmol). The reaction mixture was stirred at room temperature for 15 min and cooled to 0 °C. To the mixture was added sodium borohydride (104 mg, 2.75 mmol). The mixture was stirred at room temperature for 1 hour. The mixture was quenched with water and concentrated under reduced pressure. The residue was extracted with DCM. The organic layer was dried over $Na_2SO_4$ and concentrated under reduced pressure. The product was purified by flash chromatography (0-5 % MeOH in DCM) to give Intermediate CA (58.7 mg, 10.4 % yield) as brown liquid. $^1$H NMR (500 MHz, MeOD) δ 8.26 (d, J = 2.6 Hz, 1H), 8.08 (dd, J = 9.7, 2.6 Hz, 1H), 7.62 (d, J = 1.9 Hz, 1H), 7.61 - 7.54 (m, 1H), 7.54 - 7.47 (m, 2H), 7.46 - 7.42 (m, 2H), 7.41 - 7.31 (m, 4H), 6.66 (d, J = 9.7 Hz, 1H), 5.20 (q, J = 7.1 Hz, 1H), 4.05 (s, 2H), 2.63 (s, 3H), 1.56 (d, J = 7.1 Hz, 3H); LC/MS m/z = 206 $[M+H]^+$.

**Preparation Example II: N-(5-amino-2-fluoro-phenyl)acetamide**

Step 1: Synthesis of N-(2-fluoro-5-nitro-phenyl)acetamide

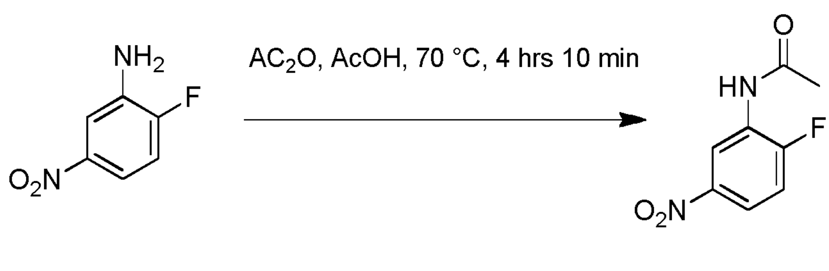


A mixture of 2-fluoro-5-nitro-aniline (2.0 g, 12.8 mmol) in AcOH (4 mL) was stirred at 70 °C for 10 min. The mixture was added with $Ac_2O$ (2.62 g, 25.6 mmol) and stirred at 70 °C for 4 hours. The mixture was filtered, and the

filtrate was concentrated in vacuo to N-(2-fluoro-5-nitro-phenyl)acetamide (2.27 g, 89% yield) as a white solid. $^1H$ NMR (400 MHz, DMSO-$d_6$) δ = 10.18 (s, 1H), 9.00 (dd, *J* = 2.8, 6.8 Hz, 1H), 8.05 - 8.00 (m, 1H), 7.56 (dd, *J*= 9.2, 10.4 Hz, 1H), 2.16 (s, 3H).

Step 2: Synthesis of N-(5-amino-2-fluoro-phenyl)acetamide

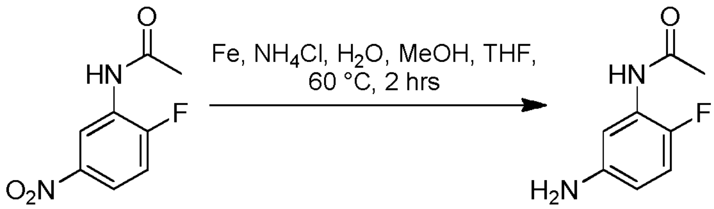


**Intermediate CB**

To a mixture of N-(2-fluoro-5-nitro-phenyl)acetamide (2.27 g, 11.4 mmol), Fe (3.20 g, 57.2 mmol) and $NH_4Cl$ (6.13 g, 114.5 mmol) in THF (8 mL), $H_2O$ (4 mL) and MeOH (32 mL) were added. The reaction mixture was stirred at 60 °C for 2 hours. The reaction mixture was diluted in MeOH (100 mL) and filtered through celite. The filtrate was concentrated in vacuo to give Intermediate CB (1.75 g, 90% yield) as a yellow solid. $^1H$ NMR (400 MHz, DMSO-$d_6$) δ = 9.40 (s, 1H), 7.12 (d, *J* = 4.8 Hz, 1H), 6.84 (t, *J* = 9.6 Hz, 1H), 6.32 - 6.18 (m, 1H), 4.96 (s, 2H), 2.04 (s, 3H).

**Preparation Example III: 1-acetylpiperidin-4-yl methanesulfonate**

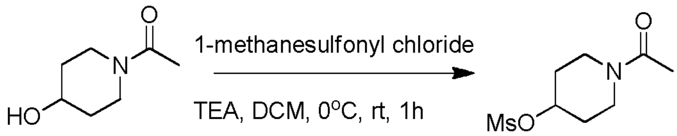


**Intermediate CC**

To a solution of 1-(4-hydroxypiperidin-1-yl)ethan-1-one (200 mg, 1.39 mmol) in DCM (3 mL) was added triethylamine (0.29 mL, 2.09 mmol). Methanesulfonyl chloride (0.11 mL, 1.39 mmol) was dropwise added to the reaction mixture at 0 °C. The reaction mixture was stirred at room temperature for 1 hour. The mixture was added with DW and extracted with MC. The combined organic layer was dried over $MgSO_4$, filtered and concentrated to give Intermediate CC (240 mg, 78 % yield). LC/MS m/z = 222.1 $[M+H]^+$.

**Preparation Example IV**: **1-methyl-1,2,3,6-tetrahydropyridin-4-yl trifluoromethanesulfonate**

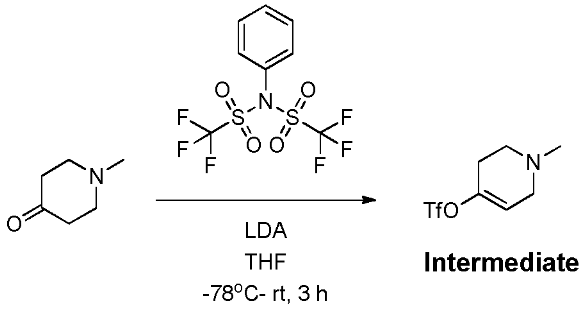


**Intermediate CD**

To a solution of 1-methylpiperidin-4-one (0.92 g, 8.13 mmol) in THF (10 mL) was added LDA (1.0 M in THF/Hexane, 8.13 mL) at -78 °C. The mixture was allowed to be warmed to room temperature and stirred for 30 min. The solution was cooled once more to -78 °C, and 1,1,1-trifluoro-N-phenyl-N-((trifluoromethyl)sulfonyl)methanesulfonamide (4.36 g,

12.2 mmol) was added in one portion. The solution was warmed to room temperature and stirred for 3 hours. The reaction mixture was poured into water and extracted with ether. The organic layer was dried over anhydrous $Na_2SO_4$ and concentrated under reduced pressure to obtain a crude product. The residue was purified by flash chromatography (0-20 % EtOAc in Hx) to give Intermediate CD (1.22 g, 61.3 %). LC/MS m/z = 246 $[M+H]^+$.

**Preparation Example V: N-(3-bromophenyl)cyclopropanecarboxamide**

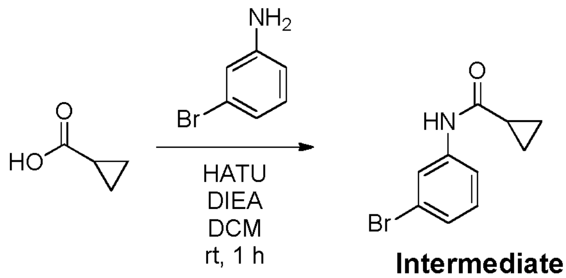


**Intermediate CE**

To a solution of cyclopropanecarboxylic acid (253 μL, 3.20 mmol) in DCM (5 mL), HATU (1.66 g, 4.36 mmol), DIEA (1.01 mL, 5.81 mmol) and 3-bromoaniline (0.5 g, 2.91 mmol) were added. The mixture was stirred at room temperature for 1 hour. The precipitate was collected by filtration and washed with DCM. The product was purified by flash chromatography (25-50 % EtOAc in Hx) to give Intermediate CE (0.98 g, 140 %). LC/MS m/z = 240 $[M+H]^+$.

**Preparation Example VI**: **3-tetrahydrofuran-2-ylaniline**

Step 1: Synthesis of 5-(3-nitrophenyl)-2,3-dihydrofuran

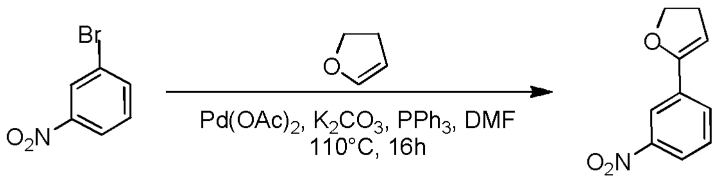


A mixture of 1-bromo-3-nitro-benzene (2.0 g, 9.90 mmol), 2,3-dihydrofuran (3.47 g, 49.5 mmol), $Pd(OAc)_2$ (222 mg, 990 μmol), $PPh_3$ (519 mg, 1.98 mmol) and $K_2CO_3$ (13.6 g, 99.0 mmol) in DMF (20 mL) was stirred at 110°C for 16 hours. The reaction mixture was filtered, and the filtrate was added with water (50 mL). The mixture was extracted with EA (3 × 20 mL). The organic layers were combined, washed with saturated NaCl solution (2 × 30 mL) and concentrated in vacuo. The residue was purified by column chromatography ($SiO_2$, Petroleum ether/Ethyl acetate = 1/0 to 10/1) to give 5-(3-nitrophenyl)-2,3-dihydrofuran (1.1 g, 58% yield) as yellow oil. $^1H$ NMR (400 MHz, $CDCl_3$) δ = 8.21 - 8.11 (m, 2H), 7.65 (d, J= 7.6 Hz, 1H), 7.57 - 7.48 (m, 1H), 6.12 (d, *J*= 5.6 Hz, 1H), 5.90 (d, *J*= 5.6 Hz, 2H), 5.02 - 4.89 (m, 1H), 4.87 - 4.78 (m, 1H).

Step 2: Synthesis of 3-tetrahydrofuran-2-ylaniline

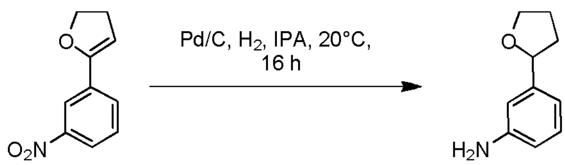


**Intermediate CF**

To a solution of 5-(3-nitrophenyl)-2,3-dihydrofuran (0.9 g, 4.71 mmol) in IPA (10 mL) was added Pd/C (90 mg, 5% purity) under $N_2$ atmosphere. The reaction mixture was stirred at 20 °C for 16 hours under $H_2$ at 15 psi. The reaction mixture was filtered, and the filtrate was concentrated in vacuo to give Intermediate CF (0.7 g, 91% yield) as yellow gum. $^1$H NMR (400 MHz, $CDCl_3$) δ = 7.12 (d, *J* = 7.6 Hz, 1H), 6.75 - 6.65 (m, 2H), 6.60 - 6.55 (m, 1H), 4.82 (t, *J* = 7.2 Hz, 1H), 4.13 - 4.04 (m, 1H), 3.96-3.88 (m,1H), 2.36 - 2.22 (m, 1H), 2.03 - 1.93 (m, 2H), 1.84-1.79 (m, 1H).

**Preparation Example VII: 2-fluoro-3,4-dimethoxy-aniline**

Step 1: Synthesis of 3-fluoro-1,2-dimethoxy-4-nitro-benzene

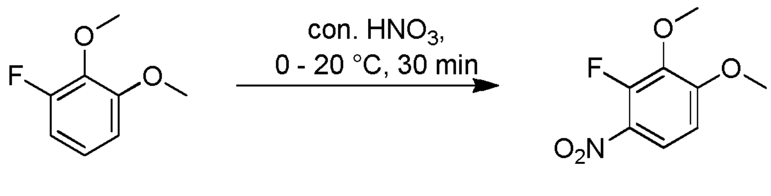


To 1-fluoro-2,3-dimethoxy-benzene (300 mg, 1.92 mmol) was added $HNO_3$ (5.6 mL) dropwise at 0 °C. The mixture was stirred at 0 °C for 15 min and stirred at 20 °C for another 15 min. The reaction mixture was poured into ice, and the resultant solid was filtered, washed with water and concentrated under reduced pressure to obtain a residue. The residue was purified by Prep-TLC ($SiO_2$, PE: EtOAc = 10: 1) to give 3-fluoro-1,2-dimethoxy-4-nitro-benzene (120 mg, 31% yield). $^1$H NMR (400 MHz, DMSO-$d_6$) δ = 7.97 (dd, *J*= 8.4, 9.6 Hz, 1H), 7.11 (dd, *J*= 1.6, 9.6 Hz, 1H), 3.96 (s, 3H), 3.85 (s, 3H).

Step 2: Synthesis of 2-fluoro-3,4-dimethoxy-aniline

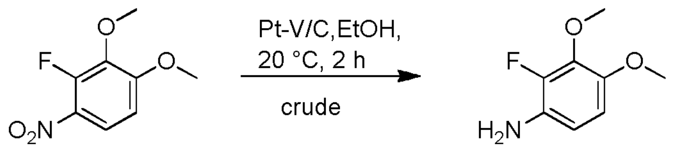


**Intermediate CG**

To a solution of 3-fluoro-1,2-dimethoxy-4-nitro-benzene (120 mg, 597 μmol) in EtOH (5 mL) was added Pt-V/C (16 mg) under $N_2$ atmosphere. The suspension was degassed and purged with $H_2$ for 3 times. Then, the mixture was stirred under $H_2$ (15 Psi) for 1 hour at 20 °C. The reaction mixture was filtered, and the filtrate was concentrated under reduced pressure to give Intermediate CG (100 mg, crude) as brown liquid. $^1$H NMR (400 MHz, DMSO-$d_6$) δ = 6.59 (dd, *J* = 2.0, 8.8 Hz, 1H), 6.47 - 6.38 (m, 1H), 4.70 (s, 2H), 3.75 (s, 3H), 3.68(s, 3H).

**Preparation Example VIII: 4-(4,4,5,5-tetramethyl-1,3,2-dioxaborolan-2-yl)bicyclo[4.2.0]octa-1,3,5-triene-3-carbaldehyde**

Step 1: Synthesis of 4-bromobicyclo[4.2.0]octa-1,3,5-triene-3-carbaldehyde

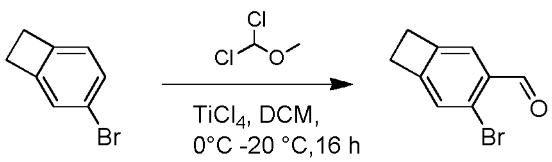


To a solution of dichloro(methoxy)methane (251.21 mg, 2.19 mmol) and $TiCl_4$ (497.40 mg, 2.62 mmol) in DCM (6 mL) was added 3-bromobicyclo[4.2.0]octa-1,3,5-triene (200 mg, 1.09 mmol) in DCM (2 mL) dropwise at 0 °C. The

reaction mixture was stirred at 20 °C for 16 hours under $N_2$. The mixture was added with a cold 5% aqueous HCl solution (20 mL) at 0 °C and stirred for 15 min. The mixture was extracted with $CH_2Cl_2$ (20 × 3 mL). The combined organic layer was washed with brine (50 mL), dried over anhydrous $Na_2SO_4$ and concentrated under reduced pressure to obtain a residue. The residue was purified by silica gel column chromatography (1% EtOH in PE) to give 4-bromobicyclo[4.2.0]octa-1,3,5-triene-3-carbaldehyde (180 mg , 33.77% yield) as a yellow solid. $^1H$ NMR (400 MHz, DMSO-$d_6$) δ = 10.23 (s, 1H), 7.53 (d, *J*= 7.6 Hz, 2H), 3.26 - 3.21 (m, 2H), 3.18 - 3.13 (m, 2H); MS (ESI) m/z = 212.8 $[M+1+H]^+$.

Step 2: Synthesis of 4-(4,4,5,5-tetramethyl-L3,2-dioxaborolan-2-yl)bicyclo[4.2.0]octa-1,3,5-triene-3-carbaldehyde

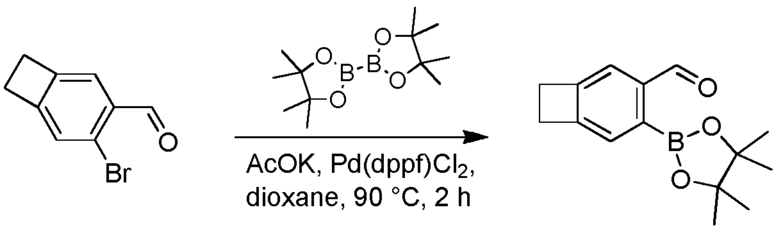


**Intermediate CH**

To a mixture of 4-bromobicyclo[4.2.0]octa-1,3,5-triene-3-carbaldehyde (50 mg, 236.91 μmol) and 4,4,5,5-tetramethyl-2-(4,4,5,5-tetramethyl-1,3,2-dioxaborolan-2-yl)-1,3,2-dioxaborolane (120.32 mg, 473.81 μmol) in dioxane (2 mL), AcOK (69.75 mg, 710.72 μmol) and Pd(dppf)$Cl_2$ (17.33 mg, 23.69 μmol) were added. The mixture was stirred at 90 C for 2 hours. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by silica gel column chromatography (2% EtOAc in PE) to give Intermediate CH (52 mg, 67.44% yield) as yellow oil. $^1H$ NMR (400 MHz, DMSO-$d_6$) δ = 10.26 (s, 1H), 7.62 (d, *J* = 0.8 Hz, 1H), 7.40 (s, 1H), 3.22 (s, 4H), 1.33 (s, 12H); MS (ESI) m/z = 259.0 $[M+2+H]^+$.

**Preparation Example IX: 5-methyl-1-(3-(4,4,5,5-tetramethyl-1,3,2-dioxaborolan-2-yl)phenyl)- 1H-1,2,3-triazole**

Step 1: Synthesis of 1-azido-3-bromobenzene

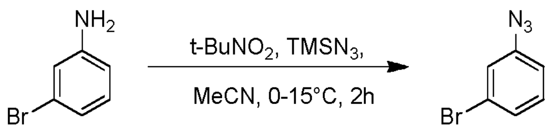


A solution of 3-bromoaniline (2 g, 11.63 mmol, 1.27 mL) in MeCN (20 mL) was cooled to 0 °C in an ice bath, t-BuO$NO_2$ (1.44 g, 13.95 mmol, 1.66 mL) was added thereto, followed by adding $TMSN_3$ (1.61 g, 13.95 mmol, 1.83 mL) slowly while stirring. After the resulting solution was stirred at 15 °C for 2 hours, TLC (EtOAc: Petroleum ether= 0: 1) indicated that the reaction had completed. Without workup, the product was concentrated under reduced pressure and purified by silica gel chromatography (0% EtOAc in Petroleum ether) to give 1-azido-3-bromobenzene (1.56 g, 67.4% yield) as yellow oil. $^1H$ NMR (400MHz, DMSO-$d_6$) δ 7.40 - 7.34 (m, 2H), 7.34 - 7.30 (m, 1H), 7.13 (td, *J* = 2.0, 7.2 Hz, 1H); LC/MS (ESI) m/z = 318.3 $[M+H]^+$

Step 2: Synthesis of 1-(3-bromophenyl)-5-methyl-triazole

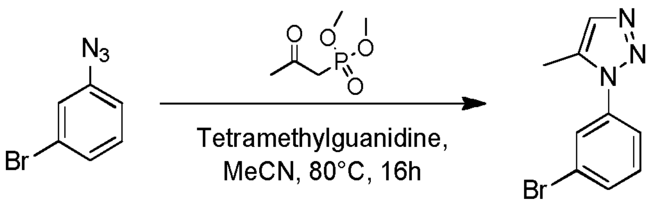


To a solution of 1-azido-3-bromo-benzene (800 mg, 4.04 mmol) in MeCN (10 mL), tetramethylguanidine (1.40 g, 12.12 mmol) and 1-dimethoxyphosphorylpropan-2-one (671.09 mg, 4.04 mmol, 554.62 μL) were added. The mixture was stirred at 80 °C for 16 hours. The reaction mixture was poured into water (50 mL) and extracted with EtOAc (30 mL×3). The combined organic layer was washed with brine (30 mL), dried over anhydrous $Na_2SO_4$ and concentrated under reduced pressure to obtain a residue. The residue was purified by flash silica gel chromatography (15% EtOAc in PE) to give 1-(3-bromophenyl)-5-methyl-triazole (350 mg, 1.32 mmol, 32.73% yield) as a yellow solid. $^1H$ NMR (400MHz, DMSO-$d_6$) δ = 7.89 (t, *J* = 2.0 Hz, 1H), 7.81 - 7.77 (m, 1H), 7.71 (d, *J* = 0.8 Hz, 1H), 7.67 - 7.63 (m, 1H), 7.60 - 7.55 (m, 1H), 2.34 (d, *J*= 0.4 Hz, 3H); MS (ESI) m/z = 238.0 $[M+H]^+$.

Step 3: Synthesis of 5-methyl-1-(3-(4,4,5,5-tetramethyl-1,3,2-dioxaborolan-2-yl)phenyl)-1H-1 2,3-triazole

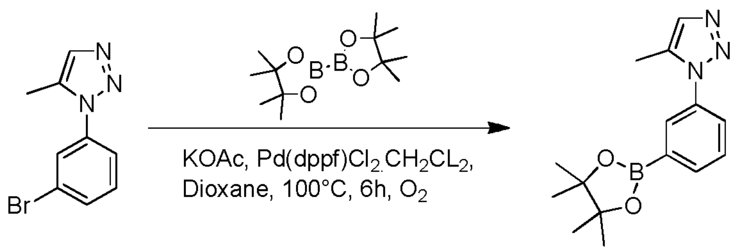


**Intermediate CI**

Intermediate CI was prepared in the same method as in Step 2 of Preparation Example VIII. MS (ESI) m/z = 285.2 $[M+H]^+$.

**Preparation Example X: 1-(trideuteriomethyl)triazole**

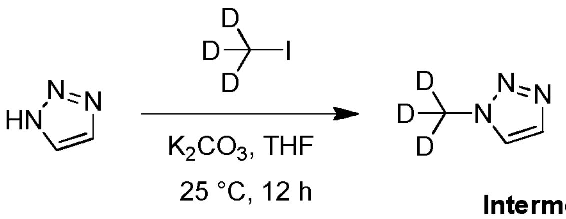


**Intermediate CJ**

To a solution of 1H-triazole (1.99 g, 28.74 mmol) in THF (25 mL), $K_2CO_3$ (7.95 g, 57.49 mmol) and trideuterio(iodo)methane (5 g, 34.49 mmol) were added. The mixture was stirred at 25 °C for 12 hours. The reaction mixture was poured into water (20 mL) and extracted with EtOAc (30 mL×3). The combined organic layer was washed with brine (20 mL × 2), dried over anhydrous $Na_2SO_4$ and concentrated under reduced pressure to obtain a crude product (800 mg, 32.32% yield) as yellow oil. $^1H$ NMR (400MHz, DMSO-$d_6$) δ = 8 8.06 (s, 1H), 7.70 (s, 1H).

**[Examples]**

**Example 1: N-[(1R)-1-(3-chlorophenyl)ethyl]-6-oxo-1-phenyl-pyridazine-3-carboxamide**

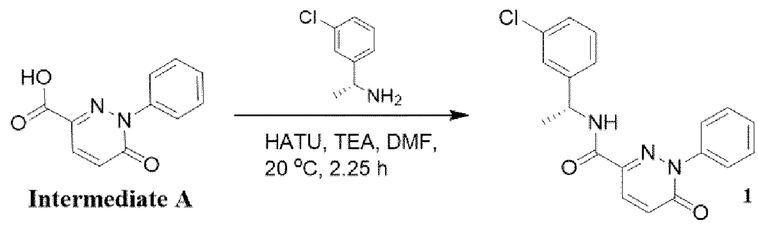


1

To a solution of Intermediate A (60 mg, 278 μmol) in DMF (1.5 mL), HATU (158 mg, 416 μmol) and TEA (84.3 mg, 833 μmol) were added, and the mixture was stirred at 20 °C for 15 min. The mixture was added with (1R)-1-(3-chlorophenyl)ethanamine (51.8 mg, 333 μmol) and stirred at 20 °C for about 2 hours under $N_2$. The reaction mixture was poured into water (10 mL) and extracted with EtOAc. The combined organic layer was washed with brine (10 mL × 3), dried over anhydrous $Na_2SO_4$ and concentrated under reduced pressure to obtain a crude product, which was purified by prep-HPLC (Phenomenex Luna C18 100*30mm*3μm; mobile phase: [water (0.225%FA)-ACN]; B%: 45%-75%, 8min). $CH_3CN$ was removed under reduced pressure, and the remaining solvent was removed by lyophilization to give the compound of Example 1 (46.1 mg, 47.0% yield) as a white solid. $^1H$ NMR (400 MHz, DMSO-$d_6$) δ 8.89 (d, *J*= 8.4 Hz, 1H), 7.88 (d, *J*= 10.0 Hz, 1H), 7.70-7.64 (m, 2H), 7.57-7.52 (m, 2H), 7.50-7.44 (m, 2H), 7.35 (d, *J*= 5.2 Hz, 2H), 7.31-7.26 (m, 1H), 7.14 (d, *J*= 10.0 Hz, 1H), 5.17-5.08 (m, 1H), 1.47 (d, *J* = 7.2 Hz, 3H); LC/MS (ESI) m/z = 354.3 $[M+H]^+$.

**Example 2**: **N-[(1R)-1-(3-bromophenyl)ethyl]-6-oxo-1-phenyl-pyridazine-3-carboxamide**

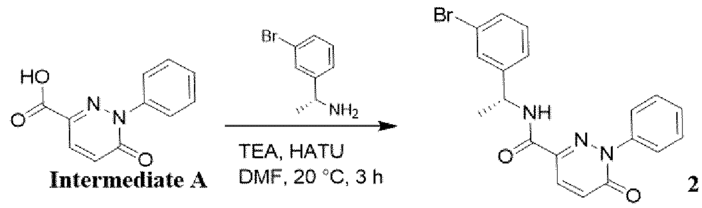


2

To a solution of Intermediate A (1.00 g, 4.63 mmol) in DMF (10 mL), HATU (2.64 g, 6.94 mmol) and TEA (1.40 g, 13.88 mmol) were added. The mixture was stirred at 20 °C for 15 min. The mixture was added with (1R)-1-(3-bromophenyl)ethanamine (1.11 g, 5.55 mmol) and then was stirred at 20 °C for about 3 hours under $N_2$ atmosphere. The reaction mixture was poured into water (25 mL) and extracted with EtOAc (30 mL × 3). The combined organic layer was washed with brine (30 mL), dried over anhydrous $Na_2SO_4$ and concentrated under reduced pressure to obtain a crude product (1.00 g). 50 mg of the crude product was purified by prep-HPLC (column: Phenomenex Luna C18 100*30mm*3um; mobile phase: [water (0.225%FA)-ACN]; B%: 50%-80%, 8 min). $CH_3CN$ was removed under reduced pressure, and the remaining solvent was removed by lyophilization to give the compound of Example 2 (9.9 mg, 10.76% yield) as a white solid. $^1H$ NMR (400 MHz, DMSO-$d_6$) δ 8.92 (d, *J*= 8.0 Hz, 1H), 7.89 (d, *J*= 9.6 Hz, 1H), 7.71-7.64 (m, 2H), 7.60 (s, 1H), 7.56 (t, *J*= 7.2 Hz, 2H), 7.51-7.45 (m, 1H), 7.45-7.37 (m, 2H), 7.34-7.23 (m, 1H), 7.14 (d, *J*= 9.6 Hz, 1H), 5.12 (quin, *J* = 7.2, 14.8 Hz 1H), 1.47 (d, *J* = 6.8 Hz, 3H); LC/MS (ESI) m/z = 400.3 $[M+H]^+$.

**Example 3 to Example 10**

The compounds shown in the following table were prepared in the same method as in Example 2 by replacing (1R)-1-(3-bromophenyl)ethanamine with appropriate amine compounds.

[Table 1]

| No. | Structure/Name | Spectral Data |
|---|---|---|
| 3 | 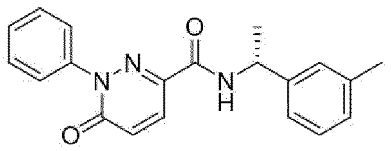 (R)-6-oxo-1-phenyl-N-(1-(m-tolyl)ethyl)-1,6-dihydropyridazine-3-carboxamide | $^1$H NMR (400 MHz, DMSO-$d_6$) δ 8.77 (d, *J* = 8.4 Hz, 1H), 7.89 (d, J = 10.0 Hz, 1H), 7.67 (d, *J* = 8.0 Hz, 2H), 7.57-7.51 (m, 2H), 7.50-7.44 (m, 1H), 7.23-7.11 (m, 4H), 7.03 (br d, *J* = 6.8 Hz, 1H), 5.10 (quin, *J* = 7.2 Hz, 1H), 2.28 (s, 3H), 1.46 (d, *J* = 7.2 Hz, 3H); LC/MS (ESI) m/z = 334.3 $[M+H]^+$ |
| 4 | 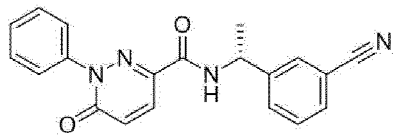 (R)-N-(1-(3-cyanophenyl)ethyl)-6-oxo-1-phenyl-1,6-dihydropyridazine-3-carboxamide | $^1$H NMR (400 MHz, DMSO-$d_6$) δ 8.90 (d, *J* = 8.4 Hz, 1H), 7.90-7.84 (m, 2H), 7.72 (d, *J* = 4.4 Hz, 1H), 7.69-7.66 (m, 2H), 7.63 (s, 1H), 7.54 (d, *J* = 6.4 Hz, 3H), 7.50-7.45 (m, 1H), 7.14 (d, *J*= 9.6 Hz, 1H), 5.17 (quin, *J* = 7.2 Hz, 1H), 1.49 (d, *J* = 7.2 Hz, 3H); LC/MS (ESI) m/z = 345.3 $[M+H]^+$ |
| 5 | 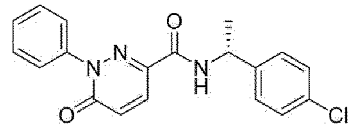 (R)-N-(1-(4-chlorophenyl)ethyl)-6-oxo-1-phenyl-1,6-dihydropyridazine-3-carboxamide | $^1$H NMR (400 MHz, DMSO-$d_6$) δ 8.86 (d, *J* = 8.0 Hz, 1H), 7.88 (d, J = 9.6 Hz, 1H), 7.66 (d, *J* = 7.6 Hz, 2H), 7.55 (t, *J* = 7.6 Hz, 2H), 7.49-7.45 (m, 1H), 7.43-7.35 (m, 4H), 7.14 (d, *J* = 9.6 Hz, 1H), 5.12 (quin, *J* = 7.2 Hz, 1H), 1.46 (d, *J* = 7.2 Hz, 3H); LC/MS (ESI) m/z = 354.3 $[M+H]^+$ |
| 6 | 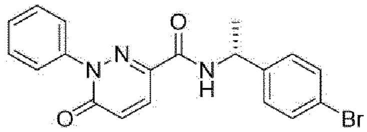 (R)-N-(1-(4-bromophenyl)ethyl)-6-oxo-1-phenyl-1,6-dihydropyridazine-3-carboxamide | $^1$H NMR (400 MHz, DMSO-$d_6$) δ 8.92-8.82 (m, 1H), 7.88 (d, *J* = 9.6 Hz, 1H), 7.66 (d, *J* = 8.0 Hz, 2H), 7.59-7.43 (m, 5H), 7.34 (d, *J* = 8.4 Hz, 2H), 7.14 (d, *J* = 9.6 Hz, 1H), 5.10 (quin, *J* = 7.2 Hz, 1H), 1.46 (d, *J* = 7.2 Hz, 3H); LC/MS (ESI) m/z = 398.2 $[M+H]^+$ |
| 7 | 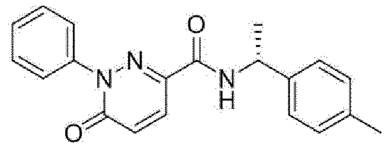 (R)-6-oxo-1-phenyl-N-(1-(p-tolyl)ethyl)-1,6-dihydropyridazine-3-carboxamide | $^1$H NMR (400 MHz, DMSO-$d_6$) δ 8.73 (d, *J* = 8.4 Hz, 1H), 7.88 (d, J = 9.6 Hz, 1H), 7.69-7.63 (m, 2H), 7.57-7.50 (m, 2H), 7.49-7.43 (m, 1H), 7.26 (d, *J* = 8.0 Hz, 2H), 7.16-7.08 (m, 3H), 5.09 (quin, *J* = 7.3 Hz, 1H), 2.25 (s, 3H), 1.45 (d, *J* = 7.2 Hz, 3H); LC/MS (ESI) m/z = 334.4 $[M+H]^+$ |
| 8 | 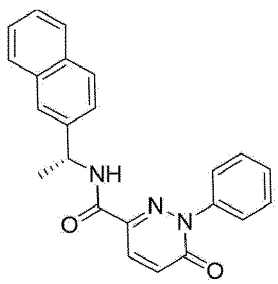 N-[(1R)-1-(2-naphthyl)ethyl]-6-oxo-1-phenyl-pyridazine-3-carboxamide | $^1$H NMR (400 MHz, DMSO-$d_6$) δ 8.93 (d, *J* = 8.4 Hz, 1H), 7.92-7.85 (m, 5H), 7.70-7.66 (m, 2H), 7.59-7.45 (m, 6H), 7.14 (d, *J* = 9.6 Hz, 1H), 5.31 (quin, *J* = 7.2 Hz, 1H), 1.58 (d, *J* = 7.2 Hz, 3H); LC/MS (ESI) m/z = 370.4 $[M+H]^+$ |

(continued)

| No. | Structure/Name | Spectral Data |
|---|---|---|
| 9 | 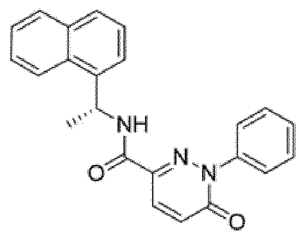 N-[(1R)-1-(1-naphthyl)ethyl]-6-oxo-1-phenyl-pyridazine-3-carboxamide | $^1$H NMR (400 MHz, DMSO-$d_6$) δ 8.99 (d, *J* = 8.0 Hz, 1H), 8.19 (d, *J* = 8.4 Hz, 1H), 7.96-7.87 (m, 2H), 7.83 (d, *J* = 8.0 Hz, 1H), 7.68-7.60 (m, 3H), 7.60-7.50 (m, 4H), 7.50-7.41 (m, 2H), 7.14 (d, *J* = 10.0 Hz, 1H), 5.93 (quin, *J* = 7.2, 14.4 Hz, 1H), 1.61 (d, *J* = 6.8 Hz, 3H); LC/MS (ESI) m/z = 370.3 [M+H]$^+$ |
| 10 | 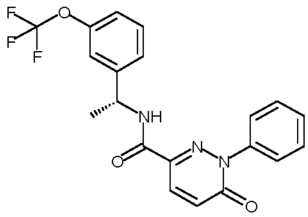 6-oxo-1-phenyl-N-[(1R)-1-[3-(trifluoromethoxy)phenyl]ethyl] pyridazine-3-carboxamide | $^1$H NMR (400 MHz, DMSO-$d_6$) δ 8.94 (d, J = 8.4 Hz, 1H), 7.88 (d, J = 10.0 Hz, 1H), 7.67 (d, J = 7.6 Hz, 2H), 7.55 (t, J = 7.2 Hz, 2H), 7.49 - 7.38 (m, 4H), 7.22 (br d, J = 7.6 Hz, 1H), 7.14 (d, J = 10.4 Hz, 1H), 5.17 (quin, J = 7.2 Hz, 1H), 1.48 (d, J = 7.2 Hz, 3H); LC/MS (ESI) m/z = 404.3 [M+H]$^+$ |

**Example 11: N-[(1R)-1-(3-methylsulfonylphenyl)ethyl]-6-oxo-1-phenyl-pyridazine-3-carboxamide**

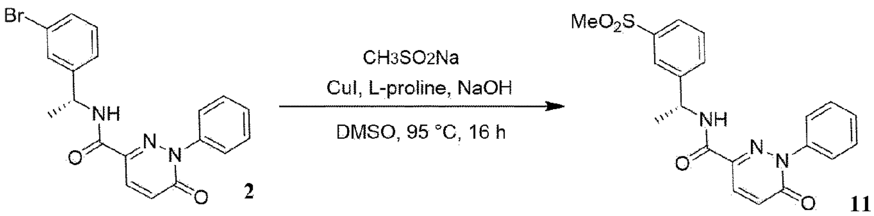


2

11

A mixture of the compound of Example 2 (50 mg, 125.55 μmol), $CH_3SO_2Na$ (15.38 mg, 150.66 μmol), CuI (2.39 mg, 12.55 μmol), L-proline (2.89 mg, 25.11 μmol) and NaOH (1.00 mg, 25.11 μmol) in DMSO (1.5 mL) was degassed and purged with $N_2$ for 3 times, and then the mixture was stirred at 95 °C for 16 hours under $N_2$ atmosphere. The reaction mixture was poured into water (10 mL) and extracted with EtOAc. The combined organic layer was washed with brine, dried over anhydrous $Na_2SO_4$ and concentrated under reduced pressure to obtain a crude product. The product was purified by prep-HPLC (Phenomenex Luna C18 100*30mm*3um; mobile phase: [water (0.225%FA)-ACN]; B%: 35%-65%, 8min). $CH_3CN$ was removed under reduced pressure, and the remaining solvent was removed by lyophilization to give the compound of Example 11 (14.5 mg, 29.06% yield). $^1$H NMR (400 MHz, DMSO-$d_6$) δ 9.02 (d, *J*= 8.3 Hz, 1H), 7.96 (s, 1H), 7.89 (d, *J* = 9.6 Hz, 1H), 7.80 (d, *J* = 7.6 Hz, 1H), 7.75 (d, *J* = 8.0 Hz, 1H), 7.70-7.64 (m, 2H), 7.64-7.58 (m, 1H), 7.58-7.51 (m, 2H), 7.50-7.44 (m, 1H), 7.14 (d, *J*= 9.6 Hz, 1H), 5.24 (quin, J= 7.3 Hz, 1H), 3.20 (s, 3H), 1.51 (d, *J* = 7.2 Hz, 3H); LC/MS (ESI) m/z = 398.3 [M+H]$^+$.

**Example 12: N-[(1R)-1-(4-methylsulfonylphenyl)ethyl]-6-oxo-1-phenyl-pyridazine-3-carboxamide**

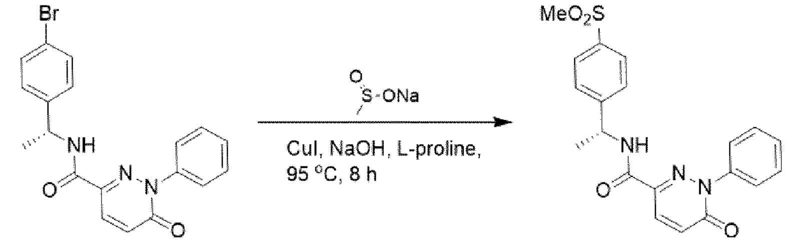


12

The compound of Example 12 was prepared as a white solid in the same method as in Example 11, except that N-[(1R)-1-(4-bromophenyl)ethyl]-6-oxo-1-phenyl-pyridazine-3-carboxamide was used instead of the compound of Example 2. $^1$H NMR (400 MHz, DMSO-$d_6$) δ 8.97 (d, *J* = 6.8 Hz, 1H), 7.87 (d, *J* = 5.6 Hz, 3H), 7.66 (s, 4H), 7.55 (s, 2H), 7.14 (d, *J* = 8.8 Hz, 1H), 5.20 (s, 1H), 3.19-3.16 (m, 3H), 1.50 (d, *J*= 5.2 Hz, 3H); LC/MS (ESI) m/z = 398.3 $[M+H]^+$.

**Example 13: 6-oxo-N-[(1R)-1-(3-phenoxyphenyl)ethyl]-1-phenyl-pyridazine-3-carboxamide**

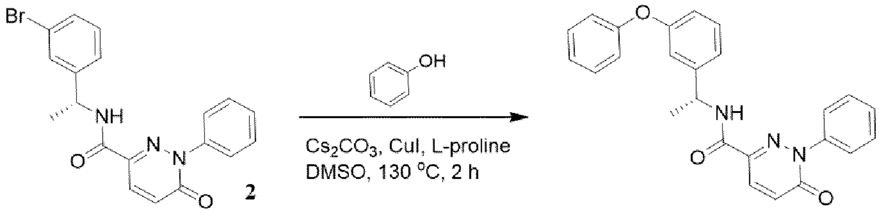


2

13

A mixture of the compound of Example 2 (100 mg, 251.10 μmol), phenol (35.45 mg, 376.65 μmol), CuI (9.56 mg, 50.22 μmol), $CS_2CO_3$ (245.44 mg, 753.29 μmol) and L-proline (5.78 mg, 50.22 μmol) in DMSO (2 mL) was stirred at 130 °C for 2 hr under $N_2$ atmosphere and microwave. The reaction mixture was poured into water (10 mL) and extracted with EtOAc, and the combined organic layer was washed with brine, dried over anhydrous $Na_2SO_4$ and concentrated under reduced pressure to obtain a crude product. The crude product was purified by prep-HPLC (Gemini NX C18 5um*10*150mm;mobile phase: [ACN/EtOH(0.1%$NH_3H_2O$)];B%:25%-75%, 30min), $CH_3CN$ was removed under reduced pressure, and the remaining solvent was removed by lyophilization to give the compound of Example 13 (2.2 mg, 2.12% yield) as a white solid. $^1$H NMR (400 MHz, DMSO-$d_6$) δ 8.88-8.79 (m, 1H), 7.89 (d, *J* = 9.6 Hz, 1H), 7.93-7.85 (m, 1H), 7.92-7.84 (m, 1H), 7.68-7.62 (m, 2H), 7.57-7.51 (m, 2H), 7.48 (d, *J*= 7.2 Hz, 1H), 7.39-7.31 (m, 3H), 7.18-7.08 (m, 4H), 7.01-6.95 (m, *J*= 8.0 Hz, 2H), 6.86-6.80 (m, 1H), 5.13 (quin, *J* = 7.6 Hz, 1H), 1.47 (d, *J*= 7.2 Hz, 3H); LC/MS (ESI) m/z = 412.3 $[M+H]^+$.

**Example 14: N-[(1R)-1-(3-cyclopropylphenyl)ethyl]-6-oxo-1-phenyl-pyridazine-3-carboxamide**

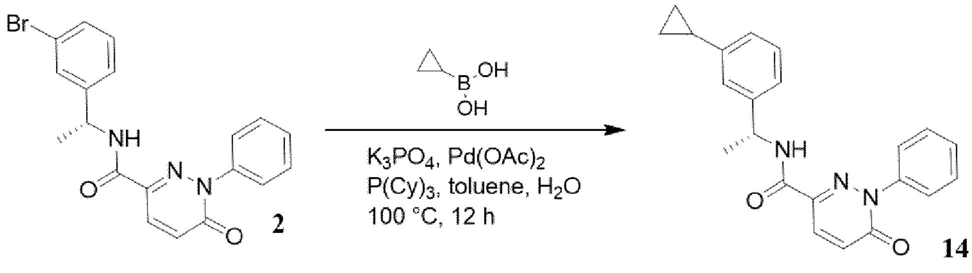


2

14

A mixture of the compound of Example 2 (50 mg, 125.55 μmol), cyclopropylboronic acid (14.02 mg, 163.21 μmol), $K_3PO_4$ (93.27 mg, 439.42 μmol), $Pd(OAc)_2$ (2.82 mg, 12.55 μmol) and $P(Cy)_3$ (7.04 mg, 25.11 μmol) in toluene (1 mL) and $H_2O$ (0.1 mL) was degassed and purged with $N_2$ for 3 times, and then the mixture was stirred at 100 °C for 12 hours under $N_2$ atmosphere. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by prep-HPLC (Phenomenex Luna C18 100*30mm*3μm, mobile

phase: [water(0.225%FA)-ACN];B%: 48%-78%,15min), $CH_3CN$ was removed under reduced pressure, and the remaining solvent was removed by lyophilization to give the compound of Example 14 (15.8 mg, 35.01% yield) as a white solid. $^1H$ NMR (400MHz, 400MHz, DMSO-$d_6$) δ 8.79 (d, *J*= 8.6 Hz, 1H), 7.88 (d, *J*= 9.6 Hz, 1H), 7.71-7.64 (m, 2H), 7.60-7.50 (m, 2H), 7.50-7.44 (m, 1H), 7.21-7.08 (m, 4H), 6.90 (d, *J* = 7.6 Hz, 1H), 5.09 (quin, *J* = 7.6 Hz, 1H), 1.96-1.80 (m, 1H), 1.45 (d, *J* = 7.2 Hz, 3H), 0.99-0.84 (m, 2H), 0.69-0.57 (m, 2H); LC/MS (ESI) m/z = 360.3 $[M+H]^+$.

**Example 15: 6-oxo-1-phenyl-N-[(1R)-1-[3-(trifluoromethyl)phenyl]ethyl]pyridazine-3-carboxamide**

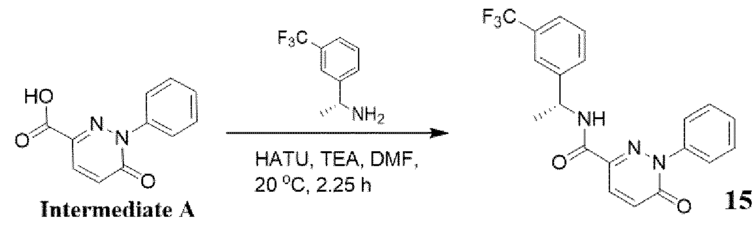

Intermediate A
15

To a solution of Intermediate A (40 mg, 185 μmol) in DMF (1.5 mL), HATU (106 mg, 278 μmol) and TEA (56.2 mg, 555 μmol) were added, followed by stirring at 20 °C for 15 min. Then, the mixture was added with (1R)-1-[3-(trifluoromethyl)phenyl]ethanamine (42.0 mg, 222 μmol) and stirred at 20 °C for 2 hours under $N_2$. The reaction mixture was poured into water (10 mL) and extracted with EtOAc. The combined organic layer was washed with brine, dried over anhydrous $Na_2SO_4$ and concentrated under reduced pressure to obtain a crude product, which was purified by prep-HPLC (Phenomenex Luna C18 100*30mm*3μm, mobile phase: [water (0.225%FA)-ACN]; B%: 50%-80%, 8min). $CH_3CN$ was removed under reduced pressure, and the remaining solvent was removed by lyophilization to give the compound of Example 15 (34.4 mg, 48.0% yield) as a white solid. $^1H$ NMR (400 MHz, DMSO-$d_6$) δ 8.99 (d, *J* = 8.4 Hz, 1H), 7.88 (d, *J* = 9.6 Hz, 1H), 7.76 (s, 1H), 7.72-7.65 (m, 3H), 7.61-7.52 (m, 4H), 7.50-7.45 (m, 1H), 7.14 (d, *J* = 9.6 Hz, 1H), 5.17-5.26 (m, 1H), 1.50 (d, *J* = 7.2 Hz, 3H); LC/MS (ESI) m/z = 388.3 $[M+H]^+$.

**Example 16: N-[(1R)-1-[3-amino-5-(trifluoromethyl)phenyl]ethyl]-6-oxo-1-phenyl-pyridazine-3-carboxamide**

Step 1: Synthesis of N-[(1R)-1-[3-nitro-5-(trifluoromethyl)phenyl]ethyl]-6-oxo-1-phenyl-pyridazine-3-carboxamide

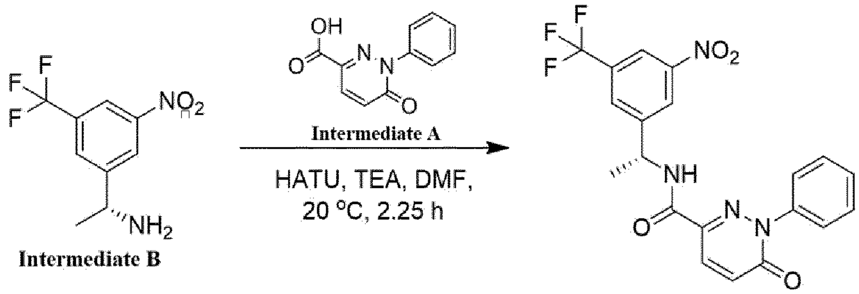


To a solution of Intermediate A (65 mg, 300 μmol) in DMF (1.5 mL), HATU (171 mg, 451 μmol) and TEA (91.3 mg, 902 μmol) were added, followed by stirring at 20 °C for 15 min. Then, the mixture was added with Intermediate B (84.5 mg, 361 μmol) and stirred at 20 °C for 2 hours under $N_2$. The reaction mixture was poured into water (15 mL) and extracted with EtOAc. The combined organic layer was washed with brine, dried over anhydrous $Na_2SO_4$ and concentrated under reduced pressure to obtain a crude product, which was purified by silica gel column chromatography (25% EtOAc in petroleum ether) to give N-[(1R)-1-[3-nitro-5-(trifluoromethyl)phenyl]ethyl]-6-oxo-1-phenyl-pyridazine-3-carboxamide (99 mg, 76.2% yield) as yellow oil. LC/MS (ESI) m/z = 433.0 $[M+H]^+$.

Step 2: Synthesis of N-[(1R)-1-[3-amino-5-(trifluoromethyl)phenyl]ethyl]-6-oxo-1-phenyl-pyridazine-3 -carboxamide

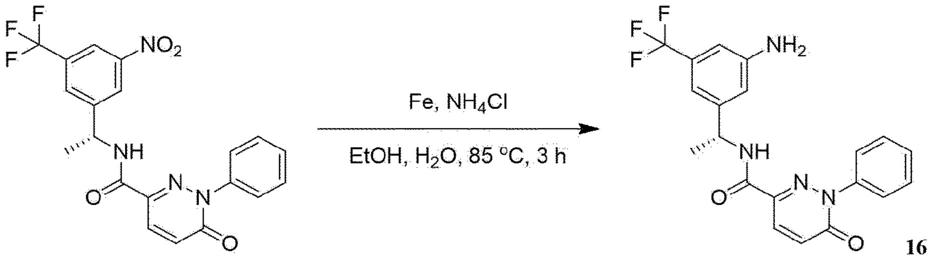


16

To a solution of N-[(1R)-1-[3-nitro-5-(trifluoromethyl)phenyl]ethyl]-6-oxo-1-phenyl-pyridazine-3-carboxamide (99 mg, 229 μmol) in EtOH (2 mL) and $H_2O$ (0.2 mL), Fe (63.9 mg, 1.1 mmol) and $NH_4Cl$ (98 mg, 1.8 mmol) were added, followed by stirring at 85 °C for 3 hours. The reaction mixture was adjusted to pH=8-9 with aq. NaHCOs and extracted with EtOAc. The combined organic layer was washed with brine, dried over anhydrous $Na_2SO_4$ and concentrated under reduced pressure to obtain a crude product, which was purified by prep-HPLC (Phenomenex Gemini-NX C18 75*30mm*3μm, mobile phase: [water(0.225%FA)-ACN]; B%: 50%-80%, 8min). $CH_3CN$ was removed under reduced pressure, and the remaining solvent was removed by lyophilization to give the compound of Example 16 (24.5 mg, 26.6% yield) as a white solid. $^1H$ NMR (400 MHz, DMSO-$d_6$) δ 8.85 (d, *J*= 8.4 Hz, 1H), 7.89 (d, *J* = 10.0 Hz, 1H), 7.70-7.63 (m, 2H), 7.57-7.51 (m, 2H), 7.50-7.44 (m, 1H), 7.14 (d, *J* = 9.6 Hz, 1H), 6.82 (s, 1H), 6.78 (s, 1H), 6.70 (s, 1H), 5.54 (s, 2H), 4.98-5.07 (m, 1H), 1.44 (d, *J*= 7.2 Hz, 3H); LC/MS (ESI) m/z = 403.3 $[M+H]^+$.

**Example 17: N-[(1R)-1-(3-chlorophenyl)ethyl]-6-oxo-1-tetrahydropyran-4-yl-pyridazine-3-carboxamide**

Step 1: Synthesis of methyl 6-oxo-1-tetrahydropyran-4-yl-pyridazine-3-carboxylate

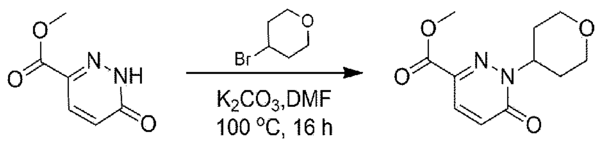


To a solution of methyl 6-oxo-1H-pyridazine-3-carboxylate (300 mg, 1.95 mmol) in DMF (5 mL), $K_2CO_3$ (538.0 mg, 3.89 mmol) and 4-bromotetrahydropyran (481.8 mg, 2.92 mmol) were added, followed by stirring at 100 °C for 16 hours. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by silica gel column chromatography (28% EtOAc in petroleum ether) to give methyl 6-oxo-1-tetrahydropyran-4-yl-pyridazine-3-carboxylate (266.0 mg, 57.36% yield) as a white solid. $^1H$ NMR (400 MHz, DMSO-$d_6$) δ 7.87-7.82 (m, 1H), 7.05-7.00 (m, 1H), 5.12-4.95 (m, 1H), 3.95-3.99 (m, 2H), 3.89-3.85 (m, 3H), 3.53-3.43 (m, 2H), 1.97-1.83 (m, 2H), 1.73-1.77 (m, 2H); LC/MS (ESI) m/z = 239.0 $[M+H]^+$.

Step 2: Synthesis of 6-oxo-1-tetrahydropyran-4-yl-pyridazine-3-carboxylic acid

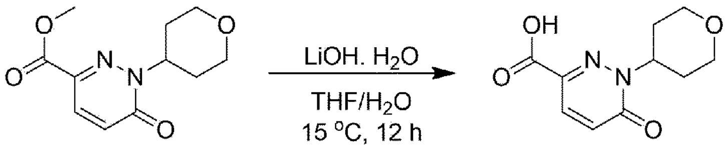


To a solution of methyl 6-oxo-1-tetrahydropyran-4-yl-pyridazine-3-carboxylate (100 mg, 419.8 μmol) in THF (2 mL) and $H_2O$ (1 mL) was added LiOH·$H_2O$ (70.46 mg, 1.68 mmol), followed by stirring at 15 °C for 12 hours. The reaction mixture was acidified with aq. 1N HCl (pH = 3-4) and then extracted with EtOAc. The organic layer was washed with water and brine, dried over $Na_2SO_4$, filtered and concentrated under reduced pressure to give 6-oxo-1-tetrahydropyran-4-yl-pyridazine-3-carboxylic acid (88 mg, crude) as a white solid. LC/MS (ESI) m/z = 225.1 $[M+H]^+$.

Step 3: Synthesis of N-[(1R)-1-(3-chlorophenyl)ethyl]-6-oxo-1-tetrahydropyran-4-yl-pyridazine-3-carboxamide

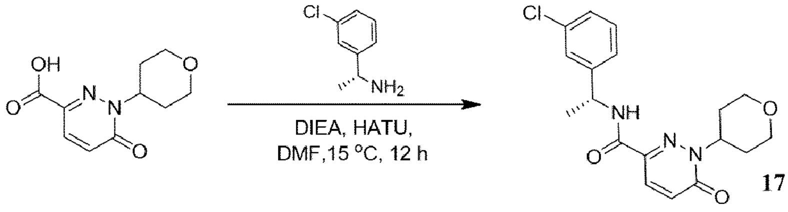


17

To a solution of 6-oxo-1-tetrahydropyran-4-yl-pyridazine-3-carboxylic acid (88 mg, 392.5 μmol) in DMF (1.5 mL), (1R)-1-(3-chlorophenyl)ethanamine (73.30 mg, 471.0 μmol), HATU (194.0 mg, 510.2 μmol) and DIEA (152.2 mg, 1.18 mmol) were added, followed by stirring at 15 °C for 12 hours. The reaction mixture was poured into water (20 mL) and extracted with EtOAc. The combined organic layer was washed with brine, dried over anhydrous $Na_2SO_4$ and concentrated under reduced pressure to obtain a crude product, which was purified by prep-HPLC (Phenomenex Luna C18 100*30mm*3μm, mobile phase: [water (0.225%FA)-ACN]; B%: 40%-70%, 8min). $CH_3CN$ was removed under reduced pressure, and the remaining solvent was removed by lyophilization to give the compound of Example 17 (38.5 mg, 27.11% yield). $^1H$ NMR (400 MHz, DMSO-$d_6$) δ 8.85 (d, *J* = 8.4 Hz, 1H), 7.81 (d, *J* = 9.6 Hz, 1H), 7.49 (s, 1H), 7.42-7.34 (m, 2H), 7.34-7.27 (m, 1H), 7.01 (d, *J*= 9.6 Hz, 1H), 5.17 (quin, *J* = 7.2 Hz, 1H), 5.10-4.99 (m, 1H), 3.99-4.03 (m, 2H), 3.56-3.47 (m, 2H), 2.30-2.18 (m, 2H), 1.74-1.66 (m, 2H), 1.53 (d, *J*= 7.2 Hz, 3H); LC/MS (ESI) m/z = 362.3 $[M+H]^+$.

**Example 18: 1-(1-acetyl-4-piperidyl)-N-[(1R)-1-(3-chlorophenyl)ethyl]-6-oxo-pyridazine-3-carboxamide**

Steps 1 to 3: Synthesis of tert-butyl 4-[3-[[(1R)-1-(3-chlorophenyl)ethyl]carbamoyl]-6-oxo-pyridazin-1-yl]piperidine-1-carboxylate

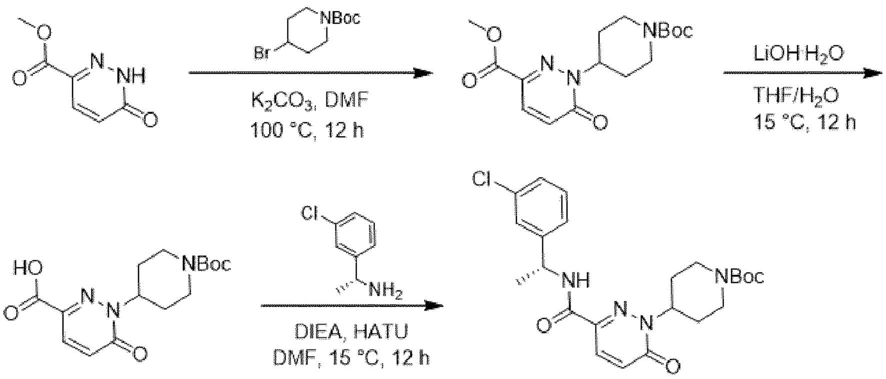


Tert-butyl 4-[3-[[(1R)-1-(3-chlorophenyl)ethyl]carbamoyl]-6-oxo-pyridazin-1-yl]piperidine-1-carboxylate was obtained as yellow oil in the same method as in Steps 1 to 3 of Example 17, except that tert-butyl 4-bromopiperidine-1-carboxylate was used instead of 4-bromotetrahydropyran in Step 1. $^1H$ NMR (400 MHz, CHLOROFORM-*d*) δ 7.94 (d, *J* = 9.6 Hz, 1H), 7.34 (s, 1H), 7.31-7.27 (m, 2H), 7.25-7.22 (m, 1H), 7.14 (d, *J* = 8.0 Hz, 1H), 6.98 (d, *J* = 9.6 Hz, 1H), 5.23 (quin, *J* = 7.2 Hz, 1H), 5.14-5.03 (m, 1H), 4.30 (br s, 2H), 2.95-2.87 (m, 2H), 1.94-1.88 (m, 4H), 1.61 (d, *J* = 7.2 Hz, 3H), 1.48 (s, 9H).

Step 4: Synthesis of N-[(1R)-1-(3-chlorophenyl)ethyl]-6-oxo-1-(4-piperidyl)pyridazine-3-carboxamide

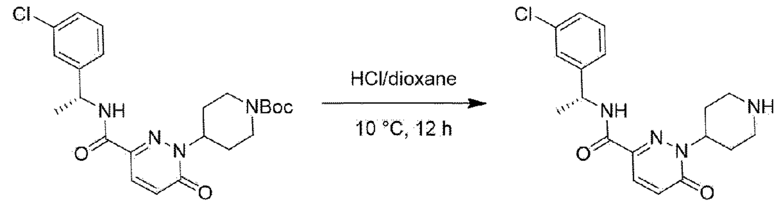


To a solution of tert-butyl 4-[3-[[(1R)-1-(3-chlorophenyl)ethyl]carbamoyl]-6-oxo-pyridazin-1-yl]piperidine-1-carboxylate (417 mg, 904.65 μmol) in dioxane (3 mL) was added HCl/dioxane (4 M, 3 mL), followed by stirring at 10 °C for 12 hours. The mixture was concentrated under reduced pressure to give N-[(1R)-1-(3-chlorophenyl)ethyl]-6-oxo-1-(4-piperidyl)pyridazine-3-carboxamide (350 mg, 100% yield, HCl salt) as a yellow solid. LC/MS (ESI) m/z = 261.0 $[M+H]^+$.

Step 5: Synthesis of 1-(1-acetyl-4-piperidyl)-N-[(1R)-1-(3-chlorophenyl)ethyl]-6-oxo-pyridazine-3-carboxamide

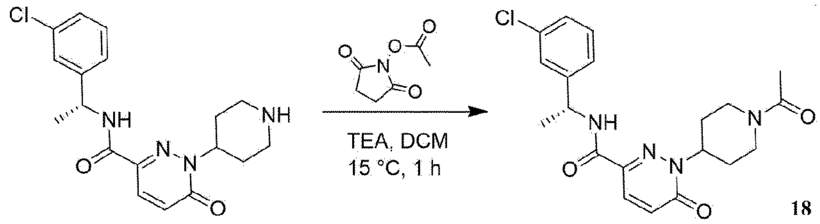

18

To a solution of N-[(1R)-1-(3-chlorophenyl)ethyl]-6-oxo-1-(4-piperidyl)pyridazine-3-carboxamide (50 mg, 125.85 μmol, HCl salt) in DCM (1 mL), TEA (38.20 mg, 377.55 μmol) and (2,5-dioxopyrrolidin-1-yl) acetate (20 mg, 127.29 μmol) were added, and the mixture was stirred at 15 °C for 1 hour. The reaction mixture was poured into water (20 mL) and extracted with EtOAc. The combined organic layer was washed with brine, dried over anhydrous $Na_2SO_4$ and concentrated under reduced pressure to obtain a crude product. The crude product was purified by prep-HPLC (Phenomenex Luna C18 100*30mm*3μm, mobile phase: [water (0.225%FA)-ACN]; B%: 30%-90%, 8 min). $CH_3CN$ was removed under reduced pressure, and the remaining solvent was removed by lyophilization to give the compound of Example 18 (19.2 mg, 37.87% yield,) as a white solid. $^1H$ NMR (400 MHz, DMSO-$d_6$) δ 8.78 (br d, *J* = 8.8 Hz, 1H), 7.80 (d, *J* = 9.6 Hz, 1H), 7.47 (br d, *J* = 4.4 Hz, 1H), 7.40-7.33 (m, 2H), 7.30 (td, J=2.8, 5.6 Hz, 1H), 7.01 (d, *J* = 9.6 Hz, 1H), 5.20-5.10 (m, 1H), 5.05-4.95 (m, 1H), 4.58 (d, *J* = 13.6 Hz, 1H), 3.99 (d, *J* = 11.6 Hz, 1H), 3.22 (s, 1H), 2.75-2.67 (m, 1H), 2.04 (s, 3H), 2.04-1.92 (m, 2H), 1.89-1.76 (m, 2H), 1.52 (d, *J*= 7.2 Hz, 3H); LC/MS (ESI) m/z = 403.3 $[M+H]^+$.

**Example 19: (R)-N-(1-(3-chlorophenyl)ethyl)-1-(1-(methylsulfonyl)piperidin-4-yl)-6-oxo-1,6-dihydropyridazine-3-carboxamide**

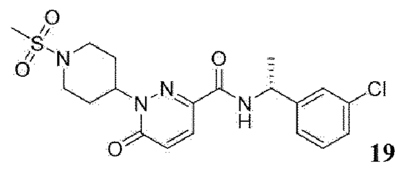
19

The compound of Example 19 was obtained in the same method as in Example 18, except that methanesulfonyl chloride was used instead of (2,5-dioxopyrrolidin-1-yl)acetate in Step 5. $^1H$ NMR (400MHz, DMSO-$d_6$) δ 8.84 (d, J = 8.4 Hz, 1H), 7.81 (d, J = 9.6 Hz, 1H), 7.47 (s, 1H), 7.41-7.34 (m, 2H), 7.34-7.27 (m, 1H), 7.01 (d, J = 9.6 Hz, 1H), 5.16 (quin, J = 7.2 Hz, 1H), 4.98-4.87 (m, 1H), 3.72 (d, J = 12.0 Hz, 2H), 3.01-2.93 (m, 2H), 2.92 (s, 3H), 2.25 (q, J = 12.0 Hz, 2H), 1.89 (d, J = 11.2 Hz, 2H), 1.53 (d, J = 7.2 Hz, 3H). LC/MS (ESI) m/z = 403.3 $[M+H]^+$.

**Example 20: N-[(1R)-1-[3-amino-5-(trifluoromethyl)phenyl]ethyl]-6-oxo-1-(2-pyridyl)pyridazine-3-carboxamide**

Step 1: Synthesis of methyl 6-oxo-1-(2-pyridyl)pyridazine-3-carboxylate

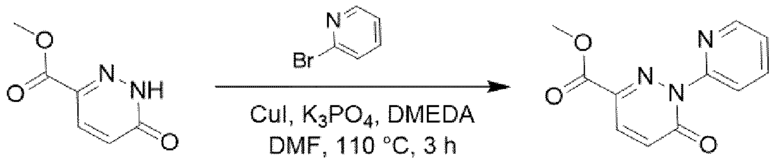


A mixture of methyl 6-oxo-1H-pyridazine-3-carboxylate (200 mg, 1.30 mmol), 2-bromopyridine (410.05 mg, 2.60 mmol), DMEDA (68.63 mg, 778.60 μmol), CuI (123.57 mg, 648.83 μmol) and $K_3PO_4$ (688.62 mg, 3.24 mmol) in DMF (5 mL) was degassed and purged with $N_2$ for 3 times, and then the mixture was stirred at 110 °C for 3 hours under $N_2$ atmosphere. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by silica gel column chromatography (59% EtOAc in petroleum ether) to give methyl 6-oxo-1-(2-pyridyl)pyridazine-3-carboxylate (100 mg, 30.66% yield) as yellow oil. LC/MS (ESI) m/z = 232.0 $[M+H]^+$.

Step 2: Synthesis of 6-oxo-1-(2-pyridyl)pyridazine-3-carboxylic acid

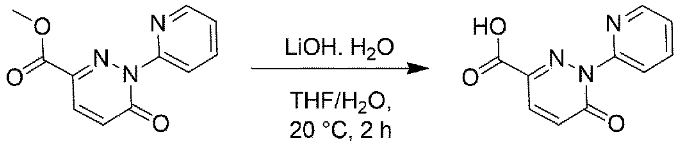


To a solution of methyl 6-oxo-1-(2-pyridyl)pyridazine-3-carboxylate (100 mg, 432.51 μmol) in THF (2 mL), $LiOH \cdot H_2O$ (72.60 mg, 1.73 mmol) and $H_2O$ (1 mL) were added, followed by stirring at 20 °C for 2 hours. The mixture was adjusted to pH = 3-4 and concentrated under reduced pressure to obtain a residue, which was purified by prep-HPLC (Phenomenex Luna C18 100*30mm*3μm, mobile phase: [water(0.225%FA)-ACN];B%: 0%-30%,15min). $CH_3CN$ was removed under reduced pressure, and the remaining solvent was removed by lyophilization to give 6-oxo-1-(2-pyridyl)pyridazine-3-carboxylic acid (40 mg, 40.45% yield) as a white solid. LC/MS (ESI) m/z = 218.0 $[M+H]^+$.

Step 3: Synthesis of N-[(1R)-1-[3-nitro-5-(trifluoromethyl)phenyl]ethyl]-6-oxo-1-(2-pyridyl)pyridazine-3-carboxamide

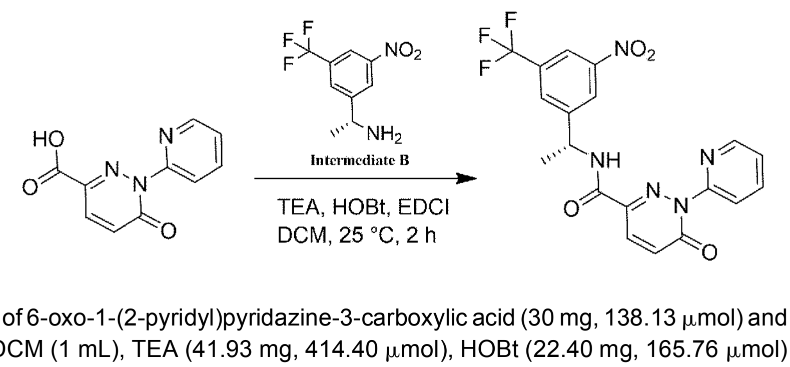


To a solution of 6-oxo-1-(2-pyridyl)pyridazine-3-carboxylic acid (30 mg, 138.13 μmol) and Intermediate B (37.38 mg, 138.13 μmol) in DCM (1 mL), TEA (41.93 mg, 414.40 μmol), HOBt (22.40 mg, 165.76 μmol) and EDCI (31.78 mg, 165.76 μmol) were added, followed by stirring at 25 °C for 2 hours. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by silica gel column chromatography (80% EtOAc in petroleum ether) to give N-[(1R)-1-[3-nitro-5-(trifluoromethyl)phenyl]ethyl]-6-oxo-1-(2-pyridyl)pyridazine-3-carboxamide (59 mg, 89.34% yield) as colorless solid. LC/MS (ESI) m/z = 434.0 [M+H]+.

Step 4: Synthesis of N-[(1R)-1-[3-amino-5-(trifluoromethyl)phenyl]ethyl]-6-oxo-1-(2-pyridyl)pyridazine-3-carboxamide

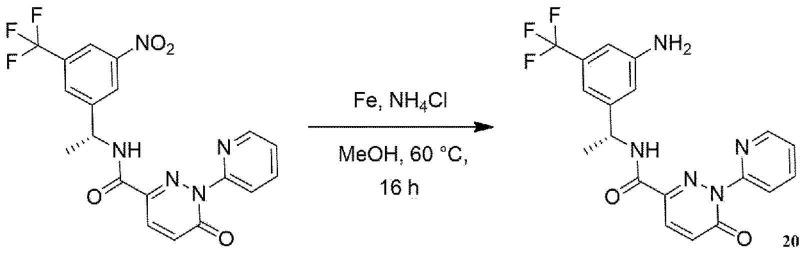


20

To a solution of N-[(1R)-1-[3-nitro-5-(trifluoromethyl)phenyl]ethyl]-6-oxo-1-(2-pyridyl)pyridazine-3-carboxamide (59 mg, 136.15 μmol) in sat. aq. $NH_4Cl$ (1 mL) and MeOH (3 mL) was added Fe (60.83 mg, 1.09 mmol), followed by stirring at 60 °C for 16 hours. The mixture was filtrated, and the filtrate was concentrated under reduced pressure to obtain a crude product, which was then purified by prep-HPLC (Phenomenex Gemini-NX C18 75*30mm*3μm, mobile phase:[water(0.05%$NH_3H_2O$+10mM $NH_4HCO_3$)-ACN];B%: 21%-41%,10min). $CH_3CN$ was removed under reduced pressure, and the remaining solvent was removed by lyophilization to give the compound of Example 20 (7.3 mg, 12.70% yield) as a light-yellow solid. $^1H$ NMR (400 MHz, DMSO-$d_6$) δ 8.87 (d, *J*= 8.4 Hz, 1H), 8.64 (dd, *J*= 1.2, 4.8 Hz, 1H), 8.11-8.03 (m, 1H), 7.94 (d, *J*= 10.0 Hz, 1H), 7.69 (d, *J*= 8.0 Hz, 1H), 7.61-7.54 (m, 1H), 7.17 (d, *J*= 10.0 Hz, 1H), 6.79 (d, *J*= 15.2 Hz, 2H), 6.69 (s, 1H), 5.54 (s, 2H), 5.02 (quin, *J* = 7.2 Hz, 1H), 1.42 (d, *J*= 7.2 Hz, 3H); LC/MS (ESI) m/z = 404.3 [M+H]+.

**Example 21: N-[(1R)-1-[3-amino-5-(trifluoromethyl)phenyl]ethyl]-6-oxo-1-(4-pyridyl)pyridazine-3-carboxamide**

Step 1: Synthesis of methyl 6-oxo-1-(4-pyridyl)pyridazine-3-carboxylate

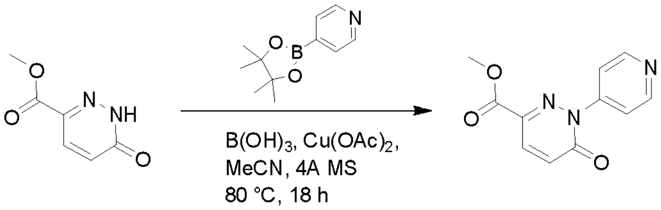


A mixture of methyl 6-oxo-1H-pyridazine-3-carboxylate (300 mg, 1.95 mmol), 4-(4,4,5,5-tetramethyl-1,3,2-dioxaborolan-2-yl)pyridine (478.98 mg, 2.34 mmol), $Cu(OAc)_2$ (70.71 mg, 389.30 μmol), 4A MS (300 mg) and boric acid (240.72 mg, 3.89 mmol) in $CH_3CN$ (8 mL) was degassed and purged with $O_2$ for 3 times, and then the mixture was stirred at 80 °C for 18 hours under $O_2$ atmosphere. The reaction mixture was poured into water (20 mL) and extracted with EtOAc. The combined organic layer was washed with brine, dried over anhydrous $Na_2SO_4$ and concentrated under reduced pressure to obtain a crude product, methyl 6-oxo-1-(4-pyridyl)pyridazine-3-carboxylate (380 mg, 67.55% yield) as a white solid. LC/MS (ESI) m/z = 232.0 [M+H]+.

Step 2: Synthesis of 6-oxo-1-(4-pyridyl)pyridazine-3-carboxylic acid

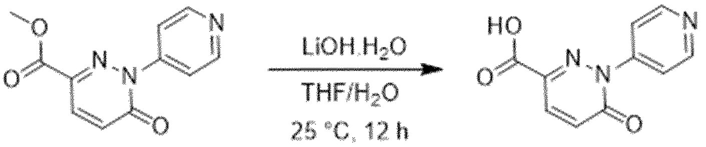

To a solution of methyl 6-oxo-1-(4-pyridyl)pyridazine-3-carboxylate (180 mg, 778.52 μmol) in THF (2 mL), $LiOH \cdot H_2O$ (65.34 mg, 1.56 mmol) and $H_2O$ (1 mL) were added, and the mixture was stirred at 25 °C for 12 hours. The reaction mixture was acidified with aq. 1 N HCl (pH = 3-4) and extracted with EtOAc, and the material precipitated in the aqueous layer was filtrated to obtain 6-oxo-1-(4-pyridyl)pyridazine-3-carboxylic acid (50 mg, 28.09% yield) as a white solid (filter cake). LC/MS (ESI) m/z = 218.0 $[M+H]^+$.

Step 3: Synthesis of N-[(1R)-1-[3-amino-5-(trifluoromethyl)phenyl]ethyl]-6-oxo-1-(4-pyridyl)pyridazine-3-carboxamide

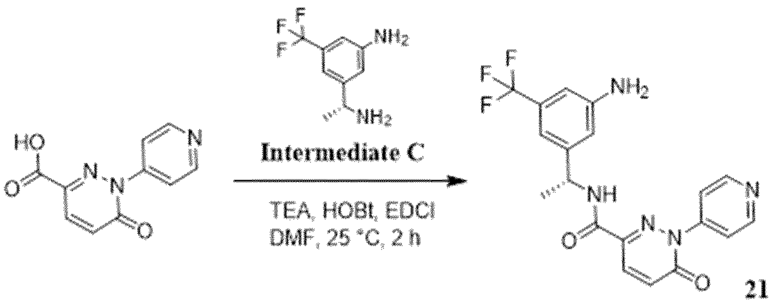


21

A solution of 6-oxo-1-(4-pyridyl)pyridazine-3-carboxylic acid (40 mg, 184.18 μmol) and Intermediate C (44.32 mg, 184.18 μmol, HCl salt) in DMF (1 mL), TEA (55.91 mg, 552.54 μmol), HOBt (29.86 mg, 221.01 μmol) and EDCI (42.37 mg, 221.01 μmol) were added, followed by stirring at 25 °C for 2 hours. The reaction mixture was poured into water (20 mL) and extracted with EtOAc. The combined organic layer was washed with brine, dried over anhydrous $Na_2SO_4$ and concentrated under reduced pressure to obtain a crude product, which was purified by prep-HPLC (Phenomenex C18 75*30mm*3μm, mobile phase: [water($NH_3H_2O$+$NH_4HCO_3$)-ACN];B%: 21%-51%,11 min). $CH_3CN$ was removed under reduced pressure, and the remaining solvent was removed by lyophilization to give the compound of Example 21 (11.6 mg, 14.66% yield) as a white solid. $^1H$ NMR (400MHz, DMSO-$d_6$) δ 8.96 (d, *J* = 8.4 Hz, 1H), 8.78-8.73 (m, 2H), 7.92-7.87 (m, 3H), 7.19 (d, *J* = 9.6 Hz, 1H), 6.81 (d, *J*= 14.4 Hz, 2H), 6.71 (s, 1H), 5.55 (s, 2H), 5.06 (quin, *J* = 7.2 Hz, 1H), 1.47 (d, *J*= 7.2 Hz, 3H); LC/MS (ESI) m/z = 404.3 $[M+H]^+$.

**Example 22: N-[(1R)-1-[3-amino-5-(trifluoromethyl)phenyl]ethyl]-1-(5-methyl-2-thienyl)-6-oxo-pyridazine-3-carboxamide**

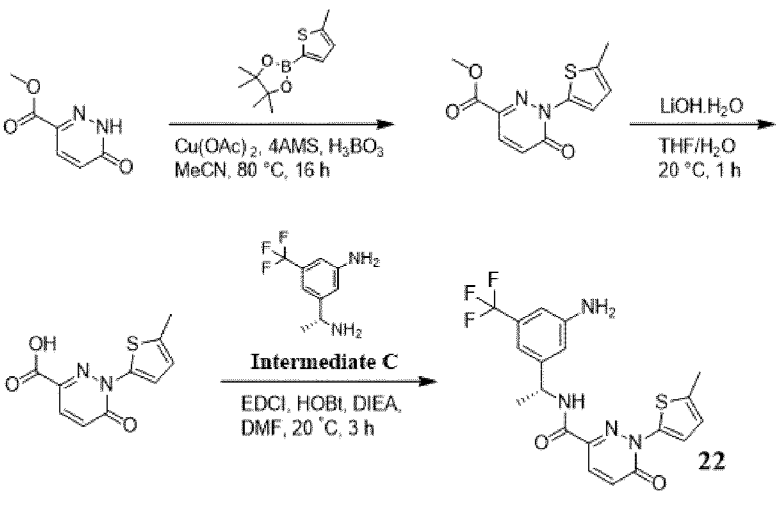


22

The compound of Example 22 was prepared in the same method in Example 21, except that 4,4,5,5-tetramethyl-2-(5-methyl-2-thienyl)-1,3,2-dioxaborolane was used instead of 4-(4,4,5,5-tetramethyl-1,3,2-dioxaborolan-2-yl)pyridine in Step 1. $^1H$ NMR (400 MHz, DMSO-$d_6$) δ 8.98 (d, *J* = 8.4 Hz, 1H), 7.89 (d, *J* = 9.6 Hz, 1H), 7.84 (d, *J* = 4.0 Hz, 1H), 7.23 (d, *J* = 9.6 Hz, 1H), 6.88-6.81 (m, 3H), 6.72 (s, 1H), 5.56 (s, 2H), 5.09 (quin, *J* = 7.2 Hz, 1H), 2.46 (s, 3H), 1.51 (d,

$J$= 7.2 Hz, 3H); LC/MS (ESI) m/z = 423.3 $[M+H]^+$.

**Example 23: N-[(1R)-1-[3-amino-5-(trifluoromethyl)phenyl]ethyl]-1-(2-methoxyphenyl)-6-oxo-pyridazine-3-carboxamide**

Step 1: Synthesis of methyl 1-(2-methoxyphenyl)-6-oxopyridazine-3-carboxylate

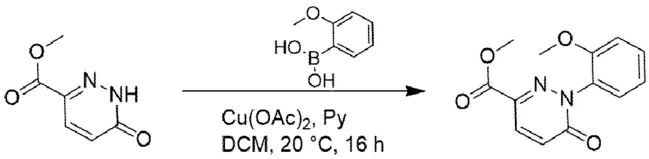


A mixture of methyl 6-oxo-1H-pyridazine-3-carboxylate (200 mg, 1.30 mmol), (2-methoxyphenyl)boronic acid (236.62 mg, 1.56 mmol), $Cu(OAc)_2$ (47.14 mg, 259.53 μmol) and pyridine (667.19 mg, 8.43 mmol) in DCM (3 mL) was stirred at 20 °C for 16 hours under air. The reaction mixture was poured into water (10 mL) and extracted with EtOAc. The combined organic layer was washed with brine, dried over anhydrous $Na_2SO_4$ and concentrated under reduced pressure to obtain a crude product. The product was purified by flash silica gel chromatography (30% EtOAc in petroleum ether) to give methyl 1-(2-methoxyphenyl)-6-oxopyridazine-3-carboxylate (180 mg, 47.16% yield) as a white oil. LC/MS (ESI) m/z = 261.0 $[M+H]^+$.

Step 2: Synthesis of 1-(2-methoxyphenyl)-6-oxo-pyridazine-3-carboxylic acid

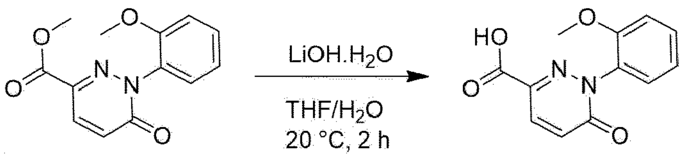


A mixture of methyl 1-(2-methoxyphenyl)-6-oxo-pyridazine-3-carboxylate (180 mg, 691.66 μmol) and $LiOH{\cdot}H_2O$ (87.07 mg, 2.07 mmol) in THF (3 mL) and $H_2O$ (1.5 mL) was stirred at 20 °C for 2 hours under air. The reaction mixture was acidified with aq.1 N HCl (pH = 3-4) and extracted with EtOAc. The organic layer was washed with water and brine, dried over $Na_2SO_4$, filtered and concentrated under reduced pressure to give 1-(2-methoxyphenyl)-6-oxo-pyridazine-3-carboxylic acid (160 mg, 81.91% yield) as a crude product of a yellow solid. LC/MS (ESI) m/z = 247.0 $[M+H]^+$.

Step 3: Synthesis of N-[(1R)-1-[3-amino-5-(trifluoromethyl)phenyl]ethyl]-1-(2-methoxyphenyl)-6-oxo-pyridazine-3-carboxamide

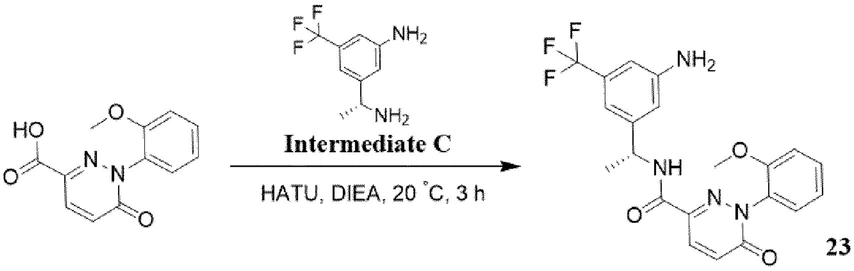


23

A mixture of 1-(2-methoxyphenyl)-6-oxo-pyridazine-3-carboxylic acid (50 mg, 203.07 μmol), Intermediate C (45.61 mg, 223.38 μmol), HATU (115.82 mg, 304.61 μmol) and DIPEA (78.74 mg, 609.22μmol) in DMF (2 mL) was degassed and purged with $N_2$ for 3 times, and then stirred at 20 °C for 3 hours under $N_2$ atmosphere. The reaction

mixture was poured into water (10 mL) and extracted with EtOAc. The combined organic layer was washed with brine, dried over anhydrous $Na_2SO_4$ and concentrated under reduced pressure to obtain a crude product, which was then purified by prep-HPLC (Phenomenex Luna C18 100*30mm*3μm, mobile phase: [water(0.225%FA)-ACN];B%: 38%-68%,7min). $CH_3CN$ was removed under reduced pressure, and the remaining solvent was removed by lyophilization to give the compound of Example 23 (26.6 mg, 30.28% yield) as a white solid. $^1H$ NMR (400 MHz, DMSO-$d_6$) δ 8.82 (d, *J* = 8.4 Hz, 1H), 7.90 (d, *J* = 10.0 Hz, 1H), 7.51-7.47 (m, 1H), 7.43 (dd, *J* = 1.6, 7.6 Hz, 1H), 7.21 (d, *J* = 8.0 Hz, 1H), 7.12-7.08 (m, 2H), 6.82 (s, 1H), 6.78 (s, 1H), 6.71 (s, 1H), 6.48-5.10 (m, 2H), 5.02 (t, *J* = 7.6 Hz, 1H), 3.76 (s, 3H), 1.42 (d, *J*= 7.2 Hz, 3H); LC/MS (ESI) m/z = 433.3 $[M+H]^+$.

**Example 24 to Example 45**

The compounds of Examples 24 to 45 were prepared in a similar method to Example 23, using starting materials and intermediates corresponding to the respective structures of the desired compounds. Meanwhile, the coupling reagent used in Step 3 was changed to HOBt and EDCI when the compounds of Examples 40 and 41 were prepared.

[Table 2]

| No. | Structure/Name | Spectral Data |
|---|---|---|
| 24 | 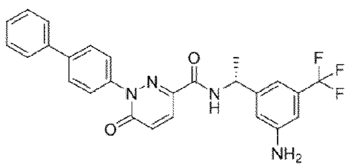 (R)-1-([1,1'-biphenyl]-4-yl)-N-(1-(3-amino-5-(trifluoromethyl)phenyl)ethyl)-6-oxo-1,6-dihydropyridazine-3 -carboxamide | $^1H$ NMR (400 MHz, DMSO-$d_6$) δ8.91 (d, J = 8.4 Hz, 1H), 7.91 (d, J = 9.6 Hz, 1H), 7.84 - 7.67 (m, 6H), 7.51 (t, J = 7.6 Hz, 2H), 7.46 - 7.38 (m, 1H), 7.17 (d, J = 10.0 Hz, 1H), 6.83 (s, 1H), 6.80 (s, 1H), 6.71 (s, 1H), 5.57 (br s, 2H), 5.05 (quin, J = 7.2 Hz, 1H), 1.46 (d, J = 6.8 Hz, 3H); LC/MS (ESI) m/z = 403.3 |
| 25 | 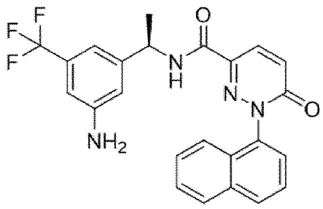 (R)-N-(1-(3-amino-5-(trifluoromethyl)phenyl) ethyl)-1-(naphthalen-1-yl)-6-oxo-1,6-dihydropyridazine-3-carboxamide | $^1H$ NMR (400 MHz, DMSO-$d_6$) δ 8.82 (br d, J = 8.4 Hz, 1H), 8.17 - 8.02 (m, 3H), 7.77 - 7.59 (m, 3H), 7.55 (s, 1H), 7.50 - 7.37 (m, 1H), 7.23 (d, J = 10.0 Hz, 1H), 6.75 (br s, 2H), 6.68 (br s, 1H), 6.33 - 5.13 (m, 2H), 5.02 (quin, J = 7.2 Hz, 1H), 1.37 (br d, J = 6.8 Hz, 3H), 1.05 (br d, J = 5.6 Hz, 1H); LC/MS (ESI) m/z = 453.3 $[M+H]^+$ |
| 26 | 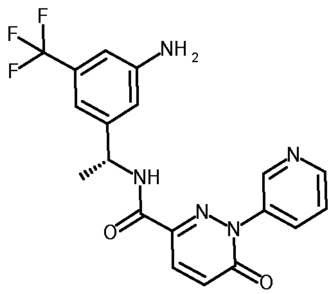 (R)-N-(1-(3-amino-5-(trifluoromethyl)phenyl) ethyl)-6-oxo-1-(pyridin-3-yl)-1,6-dihydropyridazine-3-carboxamide | $^1H$ NMR (400 MHz, DMSO-$d_6$) δ 8.98 - 8.93 (m, 2H), 8.65 (dd, J = 1.6, 4.8 Hz, 1H), 8.18 (d, J = 7.6 Hz, 1H), 7.91 (d, J = 9.6 Hz, 1H), 7.60 (dd, J = 4.8, 8.4 Hz, 1H), 7.18 (d, J = 9.6 Hz, 1H), 6.82 (s, 1H), 6.78 (s, 1H), 6.70 (s, 1H), 5.59 - 5.51 (m, 2H), 5.05 (t, J = 7.6 Hz, 1H), 1.45 (d, J = 6.8 Hz, 2H); LC/MS (ESI) m/z = 404.3 $[M+H]^+$ |

(continued)

| No. | Structure/Name | Spectral Data |
|---|---|---|
| 27 | 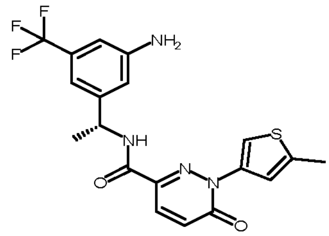 (R)-N-(1-(3-amino-5-(trifluoromethyl)phenyl) ethyl)-1-(5-methylthiophen-3-yl)-6-oxo-1,6-dihydropyridazine-3-carboxamide | $^1$H NMR (400 MHz, CHLOROFORM-d) δ 7.97 (d, J = 9.6 Hz, 1H), 7.73 - 7.66 (m, 1H), 7.14 (s, 1H), 7.09 (d, J = 9.6 Hz, 1H), 6.97 (s, 1H), 6.82 (s, 2H), 5.20 (quin, J = 7.3 Hz, 1H), 4.50 - 3.26 (m, 2H), 2.55 - 2.52 (m, 3H), 1.59 (d, J = 7.0 Hz, 3H); LC/MS (ESI) m/z = 423.3 $[M+H]^+$ |
| 28 | 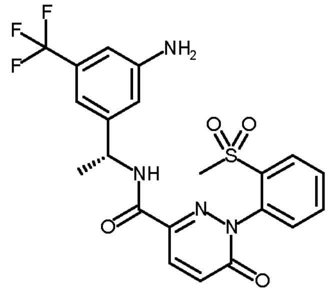 (R)-N-(1-(3-amino-5-(trifluoromethyl)phenyl) ethyl)-1-(2-(methylsulfonyl)phenyl)-6-oxo-1,6-dihydropyridazine-3 -carboxamide | $^1$H NMR (400 MHz, DMSO-$d_6$) δ9.40 (d, J = 8.4 Hz, 1H), 8.24 (d, J = 9.2 Hz, 1H), 7.99 (dd, J = 1.4, 8.0 Hz, 1H), 7.85 (t, J = 8.0 Hz, 1H), 7.76 (d, J = 8.8 Hz, 1H), 7.63 - 7.57 (m, 2H), 6.87 (s, 1H), 6.80 (s, 1H), 6.69 (s, 1H), 5.55 (s, 2H), 5.12 - 5.05 (m, 1H), 3.34 - 3.34 (m, 3H), 1.47 (d, J = 6.8 Hz, 3H); LC/MS (ESI) m/z = 481.3 $[M+H]^+$ |
| 29 | 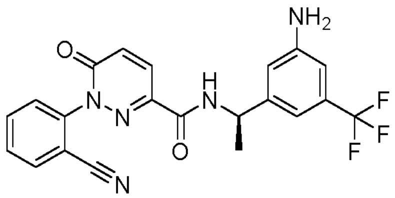 N-[(1R)-1-[3-amino-5-(trifluoromethyl)phenyl] ethyl]-1-(2-cyanophenyl)-6-oxo-pyridazine-3-carboxamide | $^1$H NMR (400 MHz, DMSO-$d_6$) δ8.94 (d, J = 8.4 Hz, 1H), 8.08 (dd, J = 1.2, 7.6 Hz, 1H), 8.00-7.92 (m, 2H), 7.85 (d, J = 7.6 Hz, 1H), 7.73 (t, J = 7.2 Hz, 1H), 7.25 (d, J = 10.0 Hz, 1H), 6.80 (s, 1H), 6.78 (s, 1H), 6.70 (s, 1H), 5.53 (s, 2H),5.03 (quin, J = 7.2 Hz, 1H), 1.42 (d, J = 6.8 Hz, 3H); LC/MS (ESI) m/z = 428.3 $[M+H]^+$ |
| 30 | 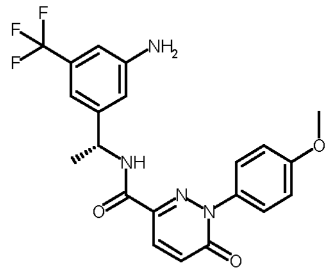 N-[(1R)-1-[3-amino-5-(trifluoromethyl)phenyl] ethyl]-1-(4-methoxyphenyl)-6-oxo-pyridazine-3-carboxamide | $^1$H NMR (400 MHz, DMSO-$d_6$) δ8.82 (d, J = 8.4 Hz, 1H), 7.87 (d, J = 9.6 Hz, 1H), 7.60 - 7.55 (m, 2H), 7.11 (d, J = 9.6 Hz, 1H), 7.08 - 7.04 (m, 2H), 6.82 (s, 1H), 6.78 (s, 1H), 6.70 (s, 1H), 5.55 (s, 2H), 5.06 - 4.99 (m, 1H), 3.82 (s, 3H), 1.44 (d, J = 7.2 Hz, 3H); LC/MS (ESI) m/z = 433.3 $[M+H]^+$ |

(continued)

| No. | Structure/Name | Spectral Data |
|---|---|---|
| 31 | 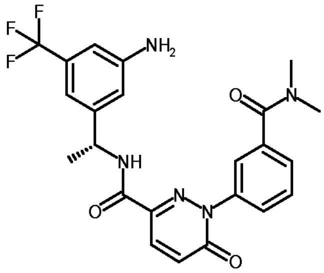 (R)-N-(1-(3-amino-5-(trifluoromethyl)phenyl) ethyl)-1-(3 -(dimethylcarbamoyl)phenyl)-6-oxo-1,6-dihydropyridazine-3-carboxamide | $^{1}H$ NMR (400 MHz, DMSO-$d_6$) $\delta$8.89 (d, J = 8.4 Hz, 1H), 7.89 (d, J = 9.6 Hz, 1H), 7.79 - 7.72 (m, 2H), 7.60 (t, J = 7.6 Hz, 1H), 7.50 (d, J = 7.6 Hz, 1H), 7.15 (d, J = 9.6 Hz, 1H), 6.80 (d, J = 12.8 Hz, 2H), 6.70 (s, 1H), 5.54 (s, 2H), 5.04 (quin, J = 7.2 Hz, 1H), 2.98 (d, J = 15.6 Hz, 6H), 1.44 (d, J = 7.2 Hz, 3H); LC/MS (ESI) m/z = 474.4 $[M+H]^+$ |
| 32 | 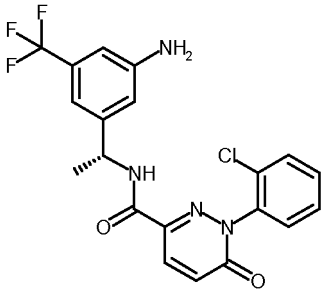 (R)-N-(1-(3-amino-5-(trifluoromethyl)phenyl) ethyl)-1-(2-chlorophenyl)-6-oxo-1,6-dihydropyridazine-3- carboxamide | $^{1}H$ NMR (400 MHz, DMSO-$d_6$) $\delta$8.89 (d, J= 8.4 Hz, 1H), 7.96 (d, J = 9.8 Hz, 1H), 7.72 - 7.63 (m, 2H), 7.58 - 7.53 (m, 2H), 7.19 (d, J = 10.0 Hz, 1H), 6.79 (d, J = 11.6 Hz, 2H), 6.70 (s, 1H), 5.54 (s, 2H), 5.03 (quin, J = 7.2 Hz, 1H), 1.42 (d, J = 7.2 Hz, 3H) ; LC/MS (ESI) m/z = 437.2 $[M+H]^+$ |
| 33 | 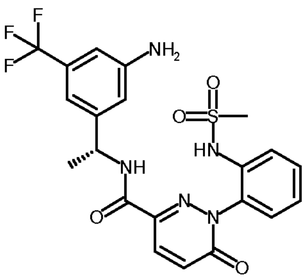 N-[(1R)-1-[3-amino-5-(trifluoromethyl)phenyl] ethyl]-1-[2-(methanesulfonamido)phenyl]-6-oxo-pyridazine-3-carboxamide | $^{1}H$ NMR (400 MHz, DMSO-$d_6$) $\delta$ 9.40 (br s, 1H), 8.77 (d, J = 8.4 Hz, 1H), 7.89 (d, J = 10.0 Hz, 1H), 7.65 (dd, J = 1.2, 8.4 Hz, 1H), 7.48 (t, J = 7.6 Hz, 1H), 7.41 (dd, J = 1.6, 8.0 Hz, 1H), 7.29 (t, J = 7.2 Hz, 1H), 7.12 (d, J = 9.6 Hz, 1H), 6.79 (s, 1H), 6.76 (s, 1H), 6.69 (s, 1H), 5.53 (s, 2H), 5.03 (quin, J = 14.4 Hz, 1H), 3.00 - 2.85 (m, 3H), 1.40 (d, J = 6.8 Hz, 3H) ; LC/MS (ESI) m/z = 496.3 $[M+H]^+$ |
| 34 | 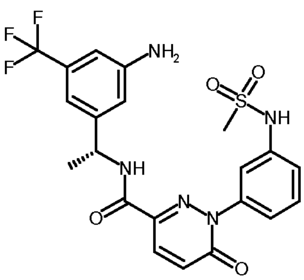 (R)-N-(1-(3-amino-5-(trifluoromethyl)phenyl) ethyl)-1-(3-(methylsulfonamido)phenyl)-6-oxo-1,6-dihydropyridazine-3-carboxamide | $^{1}H$ NMR (400 MHz, DMSO-$d_6$) $\delta$ 10.20 - 9.77 (m, 1H), 8.85 (d, J = 8.4Hz, 1H), 7.89 (d, J = 9.6Hz, 1H), 7.52 - 7.46 (m, 2H), 7.42 - 7.37 (m, 1H), 7.30 - 7.26 (m, 1H), 7.14 (d, J = 9.6Hz, 1H), 6.80 (d, J = 14.4Hz, 2H), 6.70 (s, 1H), 5.54 (s, 2H), 5.03 (quin, J = 7.2Hz, 1H), 3.06 (s, 3H), 1.44 (d, J = 7.2Hz, 3H) ; LC/MS (ESI) m/z = 496.3 $[M+H]^+$ |

(continued)

| No. | Structure/Name | Spectral Data |
|---|---|---|
| 35 | 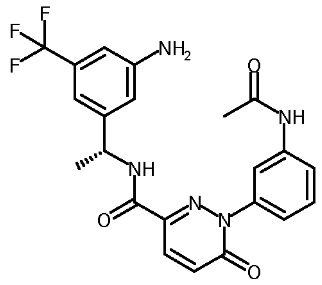 (R)-1-(3-acetamidophenyl)-N-(1-(3-amino-5-(trifluoromethyl)phenyl)ethyl)-6-oxo-1,6-dihydropyridazine-3-carboxamide | 1H NMR (400 MHz, DMSO-$d_6$) δ10.18 (s, 1H), 8.84 (d, J = 8.4Hz, 1H), 7.93 - 7.83 (m, 2H), 7.62 (d, J = 8.4 Hz, 1H), 7.45 (t, J = 8.0Hz, 1H), 7.30 (d, J = 8.0Hz, 1H), 7.14 (d, J = 9.6 Hz, 1H), 6.80 (d, J = 14.0 Hz, 2H), 6.70 (s, 1H), 5.56 (s, 2H), 5.03 (quin, J = 7.2Hz, 1H), 2.06 (s, 3H), 1.43 (d, J = 7.2Hz, 3H) ; LC/MS (ESI) m/z = 460.4 $[M+H]^+$ |
| 36 | 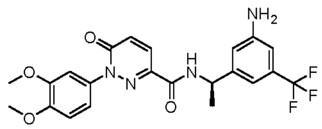 N-[(1R)-1-[3-amino-5-(trifluoromethyl)phenyl] ethyl]-1-(3,4-dimethoxyphenyl)-6-oxo-pyridazine-3- carboxamide | $^1H$ NMR (400 MHz, DMSO-$d_6$) δ8.84 (d, J = 8.4 Hz, 1H), 7.88 (d, J = 9.6 Hz, 1H), 7.28-7.14 (m, 2H), 7.11 (d, J = 9.6 Hz, 1H), 7.06 (d, J = 8.4 Hz, 1H), 6.82 (s, 1H), 6.78 (s, 1H), 6.69 (s, 1H), 5.56 (brs, 2H), 5.09-4.96 (m, 1H), 3.82 (s, 3H), 3.77 (s, 3H), 1.44 (d, J = 7.2 Hz, 3H); LC/MS (ESI) m/z = 463.3 $[M+H]^+$ |
| 37 | 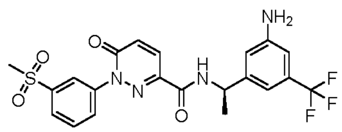 N-[(1R)-1-[3-amino-5-(trifluoromethyl)phenyl] ethyl]-1-(3-methylsulfonylphenyl)-6-oxo-pyridazine-3- carboxamide | $^1H$ NMR (400 MHz, DMSO-$d_6$) δ8.96 (d, J = 8.4 Hz, 1H), 8.29 (t, J = 1.6 Hz, 1H), 8.10-8.02 (m, 2H), 7.91 (d, J = 9.6 Hz, 1H), 7.84 (t, J = 7.6 Hz, 1H), 7.19 (d, J = 9.6 Hz, 1H), 6.82 (s, 1H), 6.78 (s, 1H), 6.70 (s, 1H), 5.56 (s, 2H), 5.04 (t, J = 7.6 Hz, 1H), 3.29 (s, 3H), 1.44 (d, J = 7.2 Hz, 3H) ; LC/MS (ESI) m/z = 481.3 $[M+H]^+$ |
| 38 | 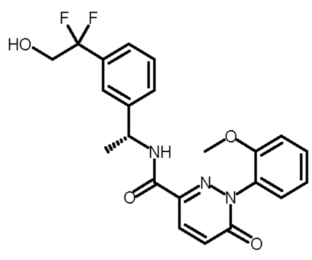 N-[(1R)-1-[3-(1,1-difluoro-2-hydroxyethyl)phenyl] ethyl]-1-(2-methoxyphenyl)-6-oxo-pyridazine-3-carboxamide | $^1H$ NMR (400 MHz, DMSO-$d_6$) δ8.89 (d, J = 8.0 Hz, 1H), 7.90 (d, J = 10.0 Hz, 1H), 7.53 - 7.47 (m, 3H), 7.44 - 7.36 (m, 3H), 7.21 (dd, J = 0.8, 8.4 Hz, 1H), 7.13 - 7.08 (m, 2H), 5.60 (br s, 1H), 5.17 (quin, J = 7.2 Hz, 1H), 3.82 (br t, J = 14.4 Hz, 2H), 3.76 (s, 3H), 1.46 (d, J = 7.2 Hz, 3H) ; LC/MS (ESI) m/z = 430.3 $[M+H]^+$ |
| 39 | 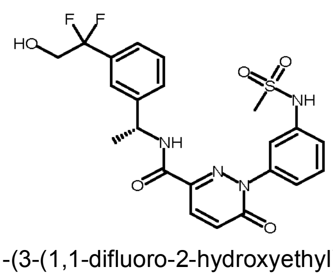 (R)-N-(1-(3-(1,1-difluoro-2-hydroxyethyl)phenyl) ethyl)-1-(3- (methylsulfonamido)phenyl)-6-oxo-1,6-dihydropyridazine-3-carboxamide | $^1H$ NMR (400 MHz, DMSO-$d_6$) δ 10.25 - 9.77 (m, 1H), 8.93 (d, J = 8.4 Hz, 1H), 7.88 (d, J = 9.6 Hz, 1H), 7.55 - 7.51 (m, 2H), 7.50 - 7.41 (m, 3H), 7.40 - 7.37 (m, 2H), 7.29 - 7.26 (m, 1H), 7.14 (d, J = 9.6 Hz, 1H), 5.83 - 5.41 (m, 1H), 5.22 - 5.15 (m, 1H), 3.83 (t, J = 14.0 Hz, 2H), 3.06 (s, 3H), 1.48 (d, J = 7.2 Hz, 3H) ; LC/MS (ESI) m/z = 493.3 $[M+H]^+$ |

(continued)

| No. | Structure/Name | Spectral Data |
|---|---|---|
| 40 | 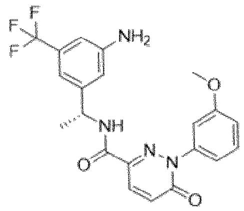 N-[(1R)-1-[3-amino-5-(trifluoromethyl)phenyl] ethyl]-1-(3-methoxyphenyl)-6-oxo-pyridazine-3-carboxamide | $^1$H NMR (400MHz, DMSO-$d_6$) δ 8.86 (d, *J* = 8.4 Hz, 1H), 7.88 (d, J = 9.6 Hz, 1H), 7.44 (t, *J* = 8.0 Hz, 1H), 7.28-7.20 (m, 2H), 7.13 (d, *J* = 9.6 Hz, 1H), 7.08-7.02 (m, 1H), 6.80 (d, *J* = 16.4 Hz, 2H), 6.70 (s, 1H), 5.55 (s, 2H), 5.03 (quin, *J*=7.2 Hz, 1H), 3.80 (s, 3H), 1.44 (d, *J* = 7.2 Hz, 3H) ; LC/MS (ESI) m/z = 433.3 $[M+H]^+$ |
| 41 | 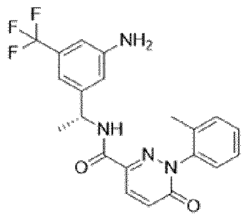 N-[(1R)-1-[3-amino-5-(trifluoromethyl)phenyl] ethyl]-1-(o-tolyl)-6-oxo-pyridazine-3 -carboxamide | $^1$H NMR (400 MHz, DMSO-$d_6$) δ 8.85 *(d, J=* 8.8 Hz, 1H), 7.94 (d, *J*= 9.6 Hz, 1H), 7.43-7.34 (m, 4H), 7.16 (d, *J* = 9.6 Hz, 1H), 6.80 (s, 1H), 6.76 (s, 1H), 6.69 (s, 1H), 5.54 (s, 2H), 5.03 (quin, *J* = 7.2 Hz, 1H), 2.09 (s, 3H), 1.42 (d, *J* =7.2 Hz, 3H) ; LC/MS (ESI) m/z = 417.3 $[M+H]^+$ |
| 42 | 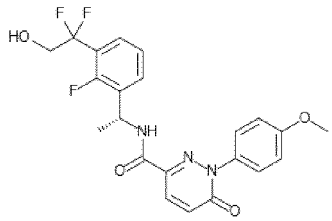 (R)-N-(1-(3-(1,1-difluoro-2-hydroxyethyl)-2-fluorophenyl)ethyl)-1 -(4-methoxyphenyl)-6-oxo-1,6-dihydropyridazine-3 -carboxamide | $^1$H NMR (400MHz, DMSO-$d_6$) δ 8.94 (d, J=8.0 Hz, 1H), 7.86 (d, J=9.6 Hz, 1H), 7.70 - 7.54 (m, 3H), 7.44 (br t, J=6.8 Hz, 1H), 7.33 - 7.23 (m, 1H), 7.10 (dd, J=9.2, 12.4 Hz, 3H), 5.72 (t, J=6.4 Hz, 1H), 5.47 - 5.35 (m, 1H), 3.92 (dt, J=6.4, 14.4 Hz, 2H), 3.83 (s, 3H), 1.48 (d, J=7.2 Hz, 3H) ; LC/MS (ESI) m/z = 448.3 $[M+H]^+$ |
| 43 | 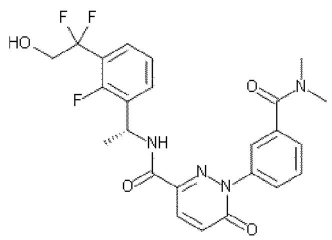 N-[(1R)-1-[3-(1,1-difluoro-2-hydroxy-ethyl)-2-fluorophenyl]ethyl]-1-[3-(dimethylcarbamoyl) phenyl]-6-oxo-pyridazine-3-carboxamide | $^1$H NMR (400 MHz, DMSO-$d_6$) δ 9.00 (d, J = 8.0 Hz, 1H), 7.87 (d, J = 9.6 Hz, 1H), 7.82-7.69 (m, 2H), 7.66-7.59 (m, 2H), 7.52 (d, J = 7.2 Hz, 1H), 7.43 (t, J = 6.8 Hz, 1H), 7.27 (t, J = 7.7 Hz, 1H), 7.15 (d, J = 9.8 Hz, 1H), 5.71 (t, J = 6.4 Hz, 1H), 5.40 (quin, J = 7.2 Hz, 1H), 3.91 (dt, J = 6.4, 14.4 Hz, 2H), 2.99 (br d, J = 13.6 Hz, 6H), 1.48 (d, J = 7. Hz, 3H); LC/MS (ESI) m/z = 489.4 $[M+H]^+$ |

(continued)

| No. | Structure/Name | Spectral Data |
|---|---|---|
| 44 | 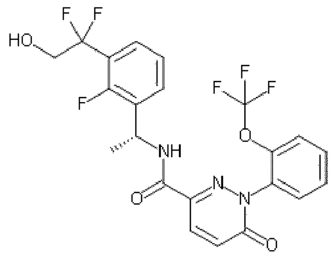 (R)-N-(1-(3-(1,1-difluoro-2-hydroxyethyl)-2-fluorophenyl)ethyl)-6-oxo-1 -(2-(trifluoromethoxy) phenyl)-1,6-dihydropyridazine-3-carboxamide | $^{1}$H NMR (400MHz, DMSO-$d_6$) δ 8.96 (d, J=8.1 Hz, 1H), 7.94 (d, J=9.8 Hz, 1H), 7.83 - 7.75 (m, 1H), 7.71 - 7.65 (m, 1H), 7.64 - 7.57 (m, 3H), 7.42 (t, J = 6.8 Hz, 1H), 7.29 - 7.22 (m, 1H), 7.19 (d, J = 9.6 Hz, 1H), 5.71 (t, J=6.4 Hz, 1H), 5.40 (quin, J=7.2 Hz, 1H), 3.90 (dt, J=6.4, 14.4 Hz, 2H), 1.45 (d, J=7.2 Hz, 3H) ; LC/MS (ESI) m/z = 502.3 $[M+H]^+$ |
| 45 | 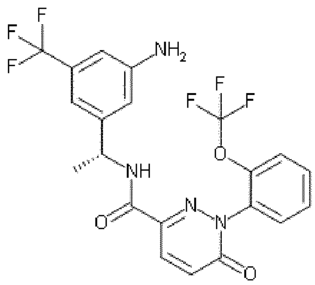 N-[(1R)-1-[3-amino-5-(trifluoromethyl)phenyl] ethyl]-6-oxo-1-[2-(trifluoromethoaη )phenyl] pyridazine-3-carboxamide | $^{1}$H NMR (400 MHz, DMSO-$d_6$) δ 8.84 (d, J = 8.4 Hz, 1H), 7.95 (d, J = 10.0 Hz, 1H), 7.80-7.72 (m, 1H), 7.70-7.64 (m, 1H), 7.63-7.57 (m, 2H), 7.19 (d, J = 9.6 Hz, 1H), 6.76 (br s, 2H), 6.69 (s, 1H), 5.54 (s, 2H), 5.03 (quin, J = 7.2 Hz, 1H), 1.42 (d, J = 7.2 Hz, 3H); LC/MS (ESI) m/z = 487.3 $[M+H]^+$ |

**Example 46: (R)-N-(1-(3-amino-5-(trifluoromethyl)phenyl)ethyl)-1-(2-fluorophenyl)-6-oxo-1,6-dihydropyridazine-3-carboxamide**

Step 1: Synthesis of 1-[3-nitro-5-(trifluoromethyl)phenyl]ethanone

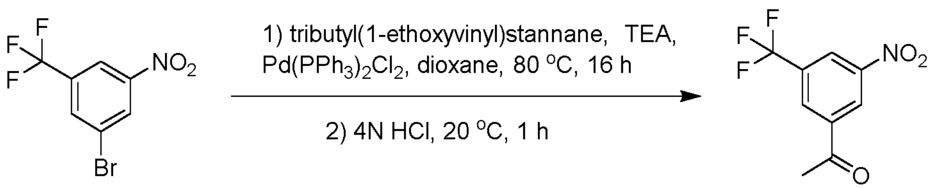


A mixture of 1-bromo-3-nitro-5-(trifluoromethyl)benzene (50 g, 185.18 mmol), tributyl(1-ethoxyvinyl)stannane (70.2 g, 194.38 mmol, 65.61 mL), $Pd(PPh_3)_2Cl_2$ (13.00 g, 18.52 mmol) and TEA (37.48 g, 370.37 mmol, 51.55 mL) in dioxane (500 mL) was degassed and purged with $N_2$ for 3 times, and then the mixture was stirred at 80 °C for 16 hours under $N_2$ atmosphere. The mixture was quenched with 6 N HCl (200 mL) and stirred at 20 °C for 1 hour. The reaction mixture was poured into water (200 mL) and extracted with EtOAc (150 mL × 3). The combined organic layer was washed with brine (100 mL × 3), dried over anhydrous $Na_2SO_4$ and concentrated under reduced pressure to obtain a residue, which was purified by flash silica gel chromatography (5% EtOAc in PE) to give 1-[3-nitro-5-(trifluoromethyl)phenyl]ethanone (66 g, 76.43% yield) as yellow oil. $^{1}$H NMR (400 MHz, DMSO-$d_6$) δ 8.85 (s, 1H), 8.73 (s, 1H), 8.63 (s, 1H), 2.76 (s, 3H).

Step 2: Synthesis of (R)-2-methyl-N-[1-[3-nitro-5-(trifluoromethyl)phenyl]ethylidene]propane-2-sulfinamide

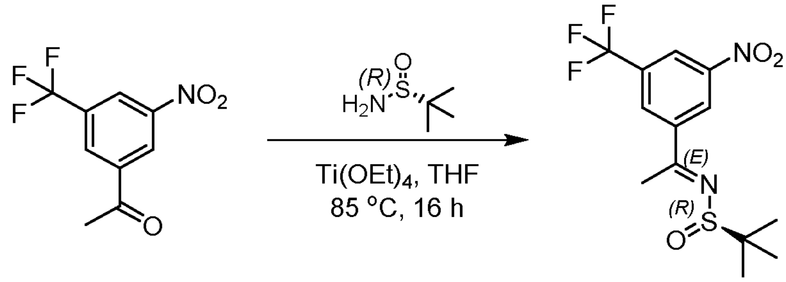


To a solution of 1-[3-nitro-5-(trifluoromethyl)phenyl]ethanone (66 g, 283.09 mmol) in THF (650 mL), $Ti(OEt)_4$ (161.44 g, 707.72 mmol, 146.76 mL) and (R)-2-methylpropane-2-sulfinamide (44.60 g, 368.01 mmol) were added, followed by stirring at 85 °C for 16 hours under $N_2$ atmosphere. The reaction mixture was poured into water (200 mL) and EtOAc (200 mL) and filtrated, and the filtrate was extracted with EtOAc (200 mL × 3). The combined organic layer was washed with brine (200 mL × 2), dried over anhydrous $Na_2SO_4$ and concentrated under reduced pressure to obtain a residue, which was purified by silica gel column chromatography (12% EtOAc in PE) to give (R,E)-2-methyl-N-[1-[3-nitro-5-(trifluoromethyl)phenyl]ethylidene]propane-2-sulfinamide (77 g, 71.07% yield) as yellow oil. $^1H$ NMR (400 MHz, DMSO-$d_6$) δ 8.85 (s, 1H), 8.73 (s, 1H), 8.63 (s, 1H), 2.76 (s, 3H); LC/MS (ESI) m/z = 336.9 $[M+H]^+$.

Step 3: Synthesis of (R)-2-methyl-N-[(1R)-1-[3-nitro-5-(trifluoromethyl)phenyl]ethyl]propane-2-sulfinamide

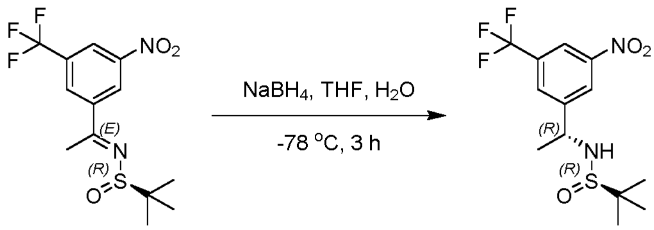


To a solution of (R)-2-methyl-N-[1-[3-nitro-5-(trifluoromethyl)phenyl]ethylidene]propane-2-sulfinamide (38.5 g, 114.47 mmol) in THF (350 mL) and $H_2O$ (7 mL) was added $NaBH_4$ (3.27 g, 86.43 mmol) for 3 batches, and the mixture was stirred at -78 °C for 3 hours under $N_2$. The reaction mixture was quenched with sat. aq. $NH_4Cl$ (150 mL) at 20°C, poured into water (200 mL) and extracted with EtOAc (200 mL × 3). The combined organic layer was washed with brine (100 mL), dried over anhydrous $Na_2SO_4$, filtered and concentrated under reduced pressure to obtain a residue. The residue was purified by silica gel column chromatography (15% EtOAc in PE) to give a main product, (R)-2-methyl-N-[(1R)-1-[3-nitro-5-(trifluoromethyl)phenyl]ethyl]propane-2-sulfinamide (30 g, 32.01% yield) as a green solid. $^1H$ NMR (400 MHz, DMSO-$d_6$) δ 8.63 (s, 1H), 8.37 (s, 1H), 8.29 (s, 1H), 6.07 (d, *J* = 8.8 Hz, 1H), 4.73-4.63 (m, 1H), 1.45 (d, *J*= 7.2 Hz, 3H), 1.13 (s, 9H); LC/MS (ESI) m/z = 338.9 $[M+H]^+$.

Step 4: Synthesis of (1R)-1-[3-nitro-5-(trifluoromethyl)phenyl]ethanamine

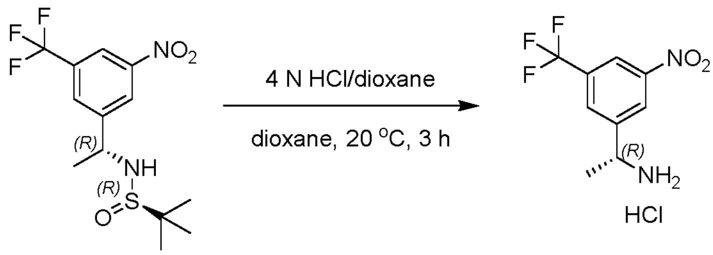


To a solution of (R)-2-methyl-N-[(1R)-1-[3-nitro-5-(trifluoromethyl)phenyl]ethyl]propane-2-sulfinamide (30 g, 88.67 mmol) in dioxane (30 mL) was added 4N HCl/dioxane (60 mL) at 0 °C, followed by stirring at 20 °C for 3 hours.

The mixture was concentrated under reduced pressure to obtain a residue, which was triturated with MTBE (100 mL) for 12 hours at 20 °C and then filtered to give the HCl salt of (1R)-1-[3-nitro-5-(trifluoromethyl)phenyl]ethanamine (23 g, 91.02% yield) as a white solid. $^1$H NMR (400 MHz, DMSO-$d_6$) δ 8.93 - 8.61 (m, 4H), 8.51 (s, 1H), 8.45 (s, 1H), 4.74 (q, *J*= 6.8 Hz, 1H), 1.58 (d, *J*= 6.8 Hz, 3H); LC/MS (ESI) m/z = 234.9 [M+H]$^+$.

Step 5: Synthesis of 3-[(1R)-1-aminoethyl]-5-(trifluoromethyl)aniline

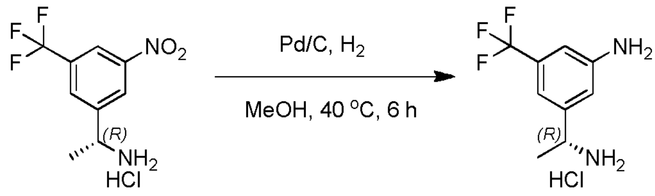


**Intermediate C**

To a solution of (1R)-1-[3-nitro-5-(trifluoromethyl)phenyl]ethanamine (10 g, 42.70 mmol, HCl salt) in MeOH (100 mL) was added Pd/C (2 g, 10% purity), followed by stirring at 40 °C for 6 hours under $H_2$ atmosphere. The reaction mixture was filtered, and the filtrate was concentrated under reduced pressure to give Intermediate C (8.5 g, 93.87% yield) as a yellow solid, which was used in the next step without further purification. $^1$H NMR (400 MHz, DMSO-$d_6$) δ 8.44 (br s, 3H), 6.97 (s, 1H), 6.84 (s, 2H), 5.75 (s, 2H), 4.30 (q, *J* = 6.8 Hz, 1H), 1.47 (d, *J* = 6.8 Hz, 3H); LC/MS (ESI) m/z = 204.9 [M+H]$^+$.

Step 6: Synthesis of 1-(2-fluorophenyl)-6-oxo-pyridazine-3-carboxylate

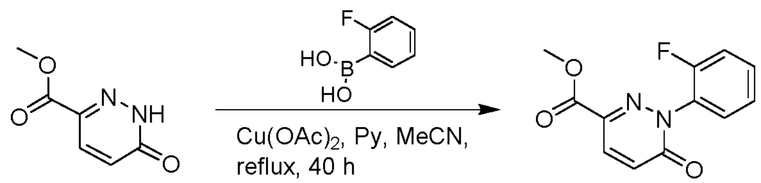


A mixture of (2-fluorophenyl)boronic acid (17.70 g, 126.52 mmol), methyl 6-oxo-1H-pyridazine-3-carboxylate (15 g, 97.32 mmol), $Cu(OAc)_2$ (5.30 g, 29.20 mmol) and pyridine (50.04 g, 632.61 mmol, 51.06 mL) in MeCN (500 mL) was degassed and purged with $O_2$ three times, and stirred at 110 °C for 40 hours under $O_2$ atmosphere. The reaction mixture was concentrated under reduced pressure, poured into distilled water (500 mL) and extracted with EtOAc. The combined organic layer was washed with brine (500 mL), dried over $Na_2SO_4$ and concentrated under reduced pressure to obtain a crude product, which was purified by silica gel column chromatography (30% EtOAc in petroleum ether) to give 1-(2-fluorophenyl)-6-oxo-pyridazine-3-carboxylate (20 g, 20.35% yield) as a white solid. $^1$H NMR (400 MHz, DMSO-$d_6$) δ 7.97 (d, *J*= 10.0 Hz, 1H), 7.60 (ddquin, 2H), 7.46 (t, *J*= 9.6 Hz, 1H), 7.40 (dt, *J*= 1.2, 7.6 Hz, 1H), 7.21 (d, *J*= 9.6 Hz, 1H), 3.85 (s, 3H); LC/MS (ESI) m/z = 249.1 [M+H]$^+$.

Step 7: Synthesis of 1-(2-fluorophenyl)-6-oxo-pyridazine-3-carboxylic acid

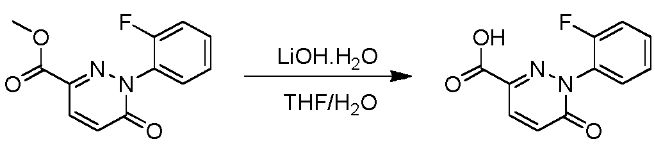


**Intermediate DA**

To a solution of 1-(2-fluorophenyl)-6-oxo-pyridazine-3-carboxylate (20 g, 80.58 mmol) in THF (180 mL), Li-

OH·$H_2O$ (10.14 g, 241.73 mmol) and $H_2O$ (20 mL) were added. The mixture was stirred at 20°C for 1 hour. The mixture was poured into water (200 mL) and extracted with EtOAc (100 mL × 3). After the organic layer was removed, the mixture was adjusted to pH 3-4 with aq. 1N HCl and then extracted with EtOAc (200 mL × 3). The combined organic layer was washed with brine (30 mL × 2), dried over $Na_2SO_4$ and concentrated under reduced pressure to give Intermediate DA (18 g, 92.36% yield) as a white solid. $^1H$ NMR (400 MHz, DMSO-$d_6$) δ 13.79 (br s, 1H), 7.95 (d, *J*= 9.6 Hz, 1H), 7.65 - 7.53 (m, 2H), 7.46 (t, *J*= 9.2 Hz, 1H), 7.42 - 7.36 (m, 1H), 7.18 (d, *J* = 10.0 Hz, 1H); LC/MS (ESI) m/z = 235.0 $[M+H]^+$.

Step 8: (R)-N-(1-(3-amino-5-(trifluoromethyl)phenyl)ethyl)-1-(2-fluorophenyl)-6-oxo-1,6-dihydropyridazine-3-carboxamide

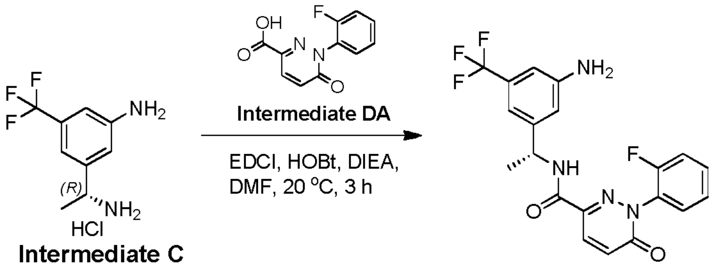


**Intermediate C** **46**

To a solution of Intermediate DA (6.45 g, 27.54 mmol) and Intermediate C (7.29 g, 30.30 mmol, HCl salt) in DMF (65 mL), DIEA (10.68 g, 82.63 mmol, 14.39 mL), HOBt (7.44 g, 55.09 mmol) and EDCI (10.56 g, 55.09 mmol) were added, and the mixture was was degassed and purged with $N_2$ for 3 times, and then stirred at 20 °C for 2 hours under $N_2$ atmosphere. The reaction mixture was poured into water (100 mL) and extracted with EtOAc (100 mL × 3). The combined organic layer was washed with brine (100 mL), dried over anhydrous $Na_2SO_4$ and concentrated under reduced pressure to obtain a crude product, which was was purified by silica gel column chromatography (40% EtOAc in PE) to give a main product, the compound of Example 46 (7.1 g, 60.75% yield) as a white solid. $^1H$ NMR (400MHz, DMSO-$d_6$) δ 8.91 (d, *J* = 8.4 Hz, 1H), 7.94 (d, *J* = 9.6 Hz, 1H), 7.67 (dt, *J* = 1.6, 7.6 Hz, 1H), 7.61 - 7.54 (m, 1H), 7.47 - 7.37 (m, 2H), 7.18 (d, *J* = 9.6 Hz, 1H), 6.80 (s, 1H), 6.77 (s, 1H), 6.70 (s, 1H), 5.54 (s, 2H), 5.02 (quin, *J* = 7.2 Hz, 1H), 1.42 (d, *J*= 7.2 Hz, 3H); LC/MS (ESI) m/z = 421.3 $[M+H]^+$.

**Example 47: N-[(1R)-1-[3-amino-5-(trifluoromethyl)phenyl]ethyl]-1-(2-hydroxyphenyl)-6-oxo-pyridazine-3-carboxamide**

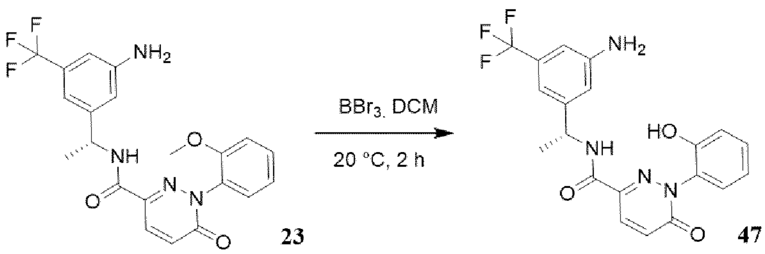


**23** **47**

A mixture of the compound of Example 23 (30 mg, 69.38 μmol) and $BBr_3$ (1 M, 346.91 μL) was stirred at 20 °C for 2 hous under air, poured onto ice-water (5 mL) and extracted with DCM. The combined organic layer was washed with brine, dried over anhydrous $Na_2SO_4$ and concentrated under reduced pressure to obtain a crude product. The product was purified by prep-HPLC (Phenomenex Gemini-NX C18 75*30mm*3μm, mobile phase:[water(0.05%$NH_3H_2O$+10mM $NH_4HCO_3$)-ACN]; B%: 18%-38%, 10min). $CH_3CN$ was removed under reduced pressure, and the remaining solvent was removed by lyophilization to give the compound of Example 47 (12.9 mg, 43.87% yield). $^1H$ NMR (400 MHz, DMSO-$d_6$) δ 9.79 (br s, 1H), 8.78 (d, *J* = 8.4 Hz, 1H), 7.89 (d, *J* = 9.6 Hz, 1H), 7.34-7.27 (m, 2H), 7.09 (d, *J* = 9.6 Hz, 1H), 6.98 (d, *J*= 7.6 Hz, 1H), 6.93 (t, *J*= 7.6 Hz, 1H), 6.81 (s, 1H), 6.77 (s, 1H), 6.69 (s, 1H), 5.54 (s, 2H), 5.06-4.98 (m, 1H), 1.42 (d, *J*= 6.8 Hz, 3H); LC/MS (ESI) m/z = 419.3 $[M+H]^+$.

**Example 48: (R)-N-(1-(3-amino-5-(trifluoromethyl)phenyl)ethyl)-5-hydroxy-6-oxo-1-phenyl-1,6-dihydropyridazine-3-carboxamide**

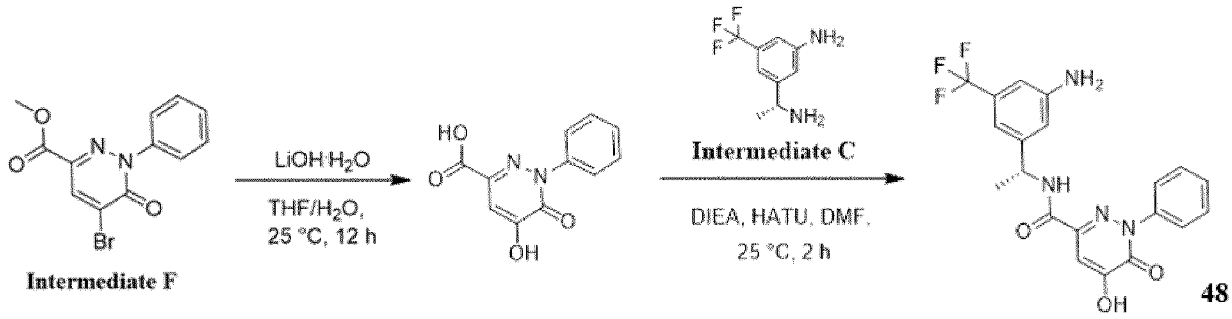


48

The compound of Example 48 was obtained by reacting Intermediate F in the same method as in Steps 2 and 3 of Example 23. $^1$H NMR (400 MHz, DMSO-$d_6$) δ 8.60 (brs, 1H), 7.55-7.49 (m, 2H), 7.49-7.38 (m, 3H), 7.18 (brs, 1H), 7.01 (brs, 1H), 6.83 (s, 1H), 6.79 (s, 1H), 6.69 (s, 1H), 5.56 (br s, 2H), 5.04 (quin, J = 7.2 Hz, 1H), 1.43 (d, J = 7.2 Hz, 3H); LC/MS (ESI) m/z = 419.3 $[M+H]^+$.

**Example 49: N-[(1R)-1-[3-amino-5-(trifluoromethyl)phenyl]ethyl]-4-methyl-6-oxo-1-phenyl-pyridazine-3-carboxamide**

Step 1: Synthesis of methyl 4-methyl-6-oxo-1-phenyl-pyridazine-3-carboxylate

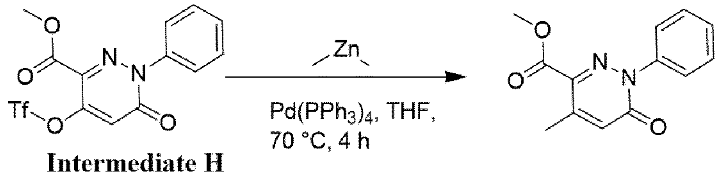


Intermediate H

A mixture of Intermediate H (80 mg, 211.48 μmol), dimethylzinc (2 M, 52.87 μL) and $Pd(PPh_3)_4$ (48.88 mg, 42.30 μmol) in THF (2 mL) was degassed and purged with $N_2$ for 3 times, and then stirred at 70 °C for 4 hours under $N_2$ atmosphere. The reaction mixture was poured into distilled water (10 mL) and extracted with EtOAc, and the combined organic layer was washed with brine, dried over anhydrous $Na_2SO_4$ and concentrated under reduced pressure to obtain a crude product. The product was purified by flash silica gel chromatography (20% EtOAc in petroleum ether) to give methyl 4-methyl-6-oxo-1-phenyl-pyridazine-3-carboxylate (60 mg, 79.08% yield) as a white solid. LC/MS (ESI) m/z = 245.0 $[M+H]^+$.

Steps 2 and 3: Synthesis of N-[(1R)-1-[3-amino-5-(trifluoromethyl)phenyl]ethyl]-4-methyl-6-oxo-1-phenyl-pyridazine-3-carboxamide

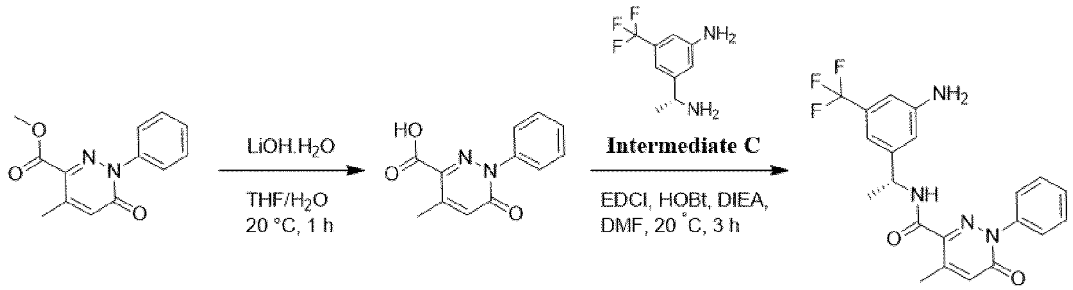


49

The compound of Example 49 was obtained by reacting methyl 4-methyl-6-oxo-1-phenyl-pyridazine-3-carboxylate in the same method as in Steps 2 and 3 of Example 23, except that the coupling reagent used in Step 3 of Example 23 was changed to HOBt and EDCI. $^1H$ NMR (400 MHz, DMSO-$d_6$) $\delta$ 9.01 (br d, J = 8.0 Hz, 1H), 7.64 (br d, J = 7.6 Hz, 2H), 7.51 (br t, J = 7.6 Hz, 2H), 7.47-7.41 (m, 1H), 6.98 (s, 1H), 6.80 (s, 1H), 6.77 (br s, 1H), 6.71 (br s, 1H), 5.58 (br s, 2H), 4.98 (br t, J = 7.2 Hz, 1H), 2.25 (s, 3H), 1.40 (br d, J = 6.8 Hz, 3H); LC/MS (ESI) m/z = 417.3 $[M+H]^+$.

**Example 50: N-[(1R)-1-[3-amino-5-(trifluoromethyl)phenyl]ethyl]-4-ethynyl-6-oxo-1-phenyl-pyridazine-3-carboxamide**

Step 1: Synthesis of methyl 6-oxo-1-phenyl-4-(2-trimethylsilylethynyl)pyridazine-3-carboxylate

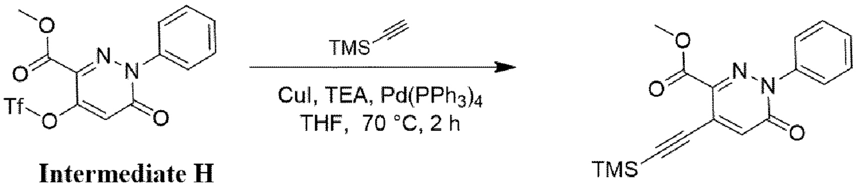


Intermediate H

A mixture of Intermediate H (200 mg, 528.71 μmol), ethynyl(trimethyl)silane (129.82 mg, 1.32 mmol, 183.11 μL), CuI (10.07 mg, 52.87 μmol), TEA (160.50 mg, 1.59 mmol, 220.77 μL) and $Pd(PPh_3)_4$ (61.10 mg, 52.87 μmol) in THF (2 mL) was degassed and purged with $N_2$ for 3 times, and then stirred at 70 °C for 2 hours under $N_2$ atmosphere. The reaction mixture was poured into water (20 mL) and extracted with EtOAc, and the combined organic layer was washed with brine, dried over anhydrous $Na_2SO_4$ and concentrated under reduced pressure to obtain a crude product. The product was purified by flash silica gel chromatography (10% EtOAc in petroleum ether) to give methyl 6-oxo-1-phenyl-4-(2-trimethylsilylethynyl)pyridazine-3-carboxylate (50 mg, 17.85% yield) as a white solid. LC/MS (ESI) m/z = 327.0 $[M+H]^+$.

Steps 2 and 3: Synthesis of N-[(1R)-1-[3-amino-5-(trifluoromethyl)phenyl]ethyl]-4-ethynyl-6-oxo-1-phenyl-pyridazine-3-carboxamide

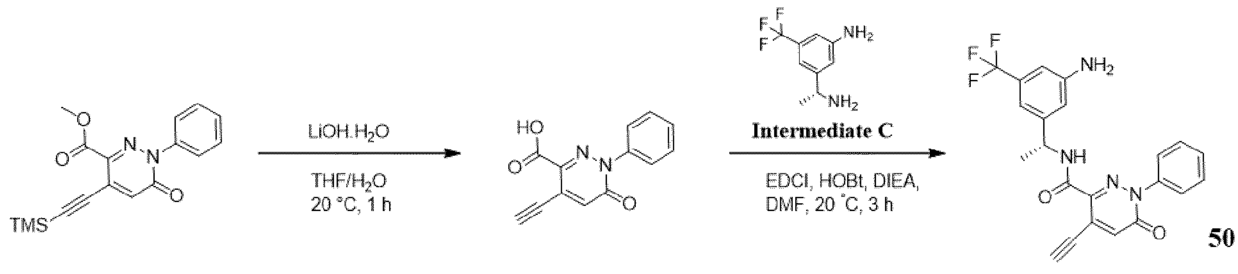


50

The compound of Example 50 was obtained by reacting methyl 6-oxo-1-phenyl-4-(2-trimethylsilylethynyl)pyridazine-3-carboxylate in the same method as in Steps 2 and 3 of Example 23, except that the coupling reagent used in

Step 3 of Example 23 was changed to HOBt and EDCI. $^1H$ NMR (400 MHz, DMSO-$d_6$) δ 9.05 (d, J = 8.0 Hz, 1H), 7.63 (d, J = 7.6 Hz, 2H), 7.54-7.49 (m, 2H), 7.48-7.43 (m, 1H), 7.34 (s, 1H), 6.80 (s, 1H), 6.76 (s, 1H), 6.71 (s, 1H), 5.56 (s, 2H), 5.01-4.94 (m, 1H), 4.86 (s, 1H), 1.40 (d, J = 7.2 Hz, 3H); LC/MS (ESI) (m/z) = 427.3 $[M+H]^+$.

**Example 51: (R)-N-(1-(3-amino-5-(trifluoromethyl)phenyl)ethyl)-1-(2-aminophenyl)-6-oxo-1,6-dihydropyridazine-3-carboxamide**

Steps 1 and 2: Synthesis of 1-(2-nitrophenyl)-N-[(1R)-1-[3-nitro-5-(trifluoromethyl)phenyl]ethyl]-6-oxo-pyridazine-3-carboxamide

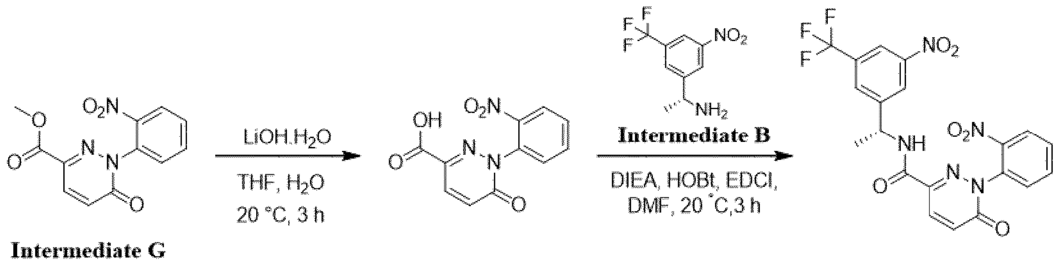


1-(2-nitrophenyl)-N-[(1R)-1-[3-nitro-5-(trifluoromethyl)phenyl]ethyl]-6-oxo-pyridazine-3-carboxamide was obtained as a crude product of a yellow solid by reacting Intermediate G in the same method as in Steps 2 and 3 of Example 23, except that, in Step 3, Intermediate C was replaced with Intermediate B, and the coupling reagent was changed to HOBt and EDCI. LC/MS (ESI) m/z = 278.1 $[M+H]^+$.

Step 3: Synthesis of (R)-N-(1-(3-amino-5-(trifluoromethyl)phenyl)ethyl)-1-(2-aminophenyl)-6-oxo-1,6-dihydropyridazine-3 -carboxamide

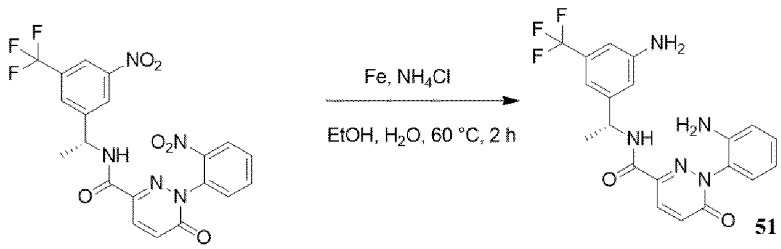


51

A mixture of 1-(2-nitrophenyl)-N-[(1R)-1-[3-nitro-5-(trifluoromethyl)phenyl]ethyl]-6-oxo-pyridazine-3-carboxamide (100 mg, 209.49 μmol) and Fe (117.00 mg, 2.09 mmol) in sat. aq $NH_4Cl$ (1 mL) and EtOH (3 mL) was stirred at 60 °C for 2 hours under air. The reaction mixture was poured into distilled water (10 mL) and extracted with EtOAc. The combined organic layer was washed with brine, dried over anhydrous $Na_2SO_4$ and concentrated under reduced pressure to obtain a crude product, which was purified by prep-HPLC (Phenomenex C18 75*30mm*3μm, mobile phase: [water($NH_3H_2O$+$NH_4HCO_3$)-ACN]; B%: 23%-63%, 15min). $CH_3CN$ was removed under reduced pressure, and the remaining solvent was removed by lyophilization to give the compound of Example 51 (49 mg, 55.84% yield) as a yellow solid. $^1H$ NMR (400 MHz, DMSO-$d_6$) δ 8.72 (d, *J* = 8.4 Hz, 1H), 7.87 (d, *J* = 9.6 Hz, 1H), 7.12 (br d, *J* = 1.6 Hz, 1H), 7.11-7.08 (m, 1H), 7.08-7.06 (m, 1H), 6.83-6.77 (m, 3H), 6.69 (s, 1H), 6.62 (t, *J* = 7.2 Hz, 1H), 5.53 (s, 2H), 5.12 (s, 2H), 5.05-4.99 (m, 1H), 1.43 (d, *J*= 7.2 Hz, 3H). LC/MS (ESI) m/z = 418.4 $[M+H]^+$.

**Example 52: (R)-1-(2-acetamidophenyl)-N-(1-(3-amino-5-(trifluoromethyl)phenyl)ethyl)-6-oxo-1,6-dihydropyridazine-3-carboxamide**

Step 1: Synthesis of methyl 1-(2-aminophenyl)-6-oxo-pyridazine-3-carboxylate

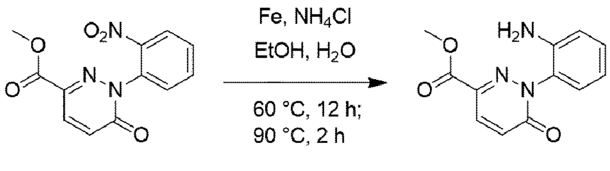


**Intermediate G**

To a solution of Intermediate G (400 mg, 1.45 mmol) in MeOH (6 mL) and sat. aq. $NH_4Cl$ (2 mL) was added Fe (405.83 mg, 7.27 mmol), followed by stirring at 60 °C for 12 hours. The mixture was concentrated under reduced pressure, and then EtOH (6 mL) was added thereto. The resulting mixture was stirred at 90 °C for 2 hours. The mixture was filtrated, and the filtrate was concentrated under reduced pressure, added with water (10 mL) and extracted with EtOAc. The combined organic layer was washed with brine, dried over anhydrous $Na_2SO_4$ and concentrated under reduced pressure to give methyl 1-(2-aminophenyl)-6-oxo-pyridazine-3-carboxylate (180 mg, 21.21% yield) as yellow oil. LC/MS (ESI) m/z = 246.0 $[M+H]^+$.

Step 2: Synthesis of methyl 1-(2-acetamidophenyl)-6-oxopyridazine-3-carboxylate

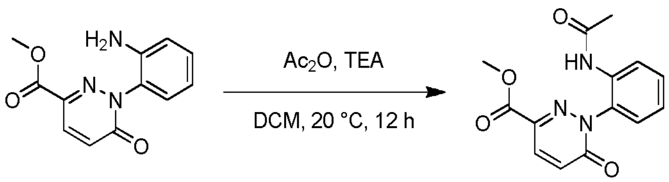


To a solution of methyl 1-(2-aminophenyl)-6-oxo-pyridazine-3-carboxylate (180 mg, 733.99 μmol) in DCM (3 mL), TEA (222.82 mg, 2.20 mmol) and $Ac_2O$ (112.40 mg, 1.10 mmol) were added, followed by stirring at 20 °C for 12 hours. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by silica gel column chromatography (50% EtOAc in petroleum ether) to give methyl 1-(2-acetamidophenyl)-6-oxopyridazine-3-carboxylate (180 mg, 32.44% yield) as a white solid. LC/MS (ESI) m/z = 288.0 $[M+H]^+$.

Steps 3 and 4: Synthesis of (R)-1-(2-acetamidophenyl)-N-(1-(3-amino-5-(trifluoromethyl)phenyl)ethyl)-6-oxo-1,6-dihydropyridazine-3-carboxamide

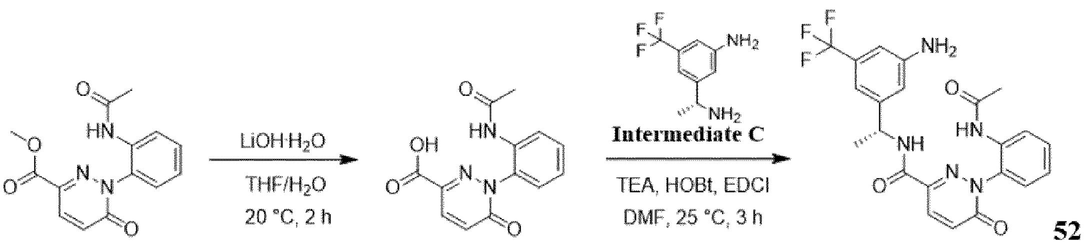


52

The compound of Example 52 was obtained by reacting methyl 1-(2-acetamidophenyl)-6-oxopyridazine-3-carboxylate in the same method as in Steps 2 and 3 of Example 23, except that the coupling reagent used in Step 3 of Example 23 was changed to HOBt and EDCI. $^1H$ NMR (400MHz, DMSO-$d_6$) δ 9.29 (s, 1H), 8.71 (d, *J* = 8.4 Hz, 1H), 7.97-7.84 (m, 2H), 7.50-7.40 (m, 2H), 7.32-7.21 (m, 1H), 7.12 (d, *J* = 10.0 Hz, 1H), 6.79 (d, *J* = 16.4 Hz, 2H), 6.69 (s, 1H), 5.55 (s, 2H), 5.04 (quin, *J* = 7.2 Hz, 1H), 1.91 (s, 3H), 1.42 (d, *J* = 7.2 Hz, 3H); LC/MS (ESI) m/z = 460.3 $[M+H]^+$.

**Example 53: 1-(3-acetamidophenyl)-N-[(1R)-1-[3-(1,1-difluoro-2-hydroxyethyl)phenyl]ethyl]-6-oxo-pyridazine-3-carboxamide**

Step 1: Synthesis of methyl 1-(3-acetamidophenyl)-6-oxo-pyridazine-3-carboxylate

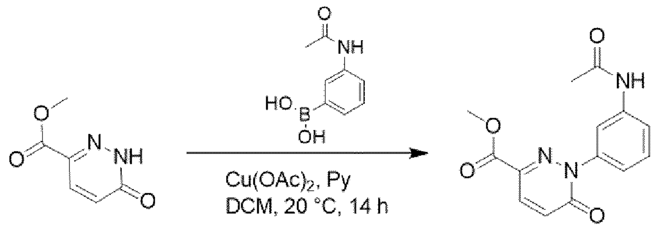


A mixture of methyl 6-oxo-1H-pyridazine-3-carboxylate (500 mg, 3.24 mmol), (3-acetamidophenyl) boronic acid (754.83 mg, 4.22 mmol), $Cu(OAc)_2$ (294.62 mg, 1.62 mmol) and pyridine (1.67 g, 21.09 mmol, 1.70 mL) in DCM (5 mL) was stirred at 20 °C for 14 hours under air atmosphere. The reaction mixture was poured into water (20 mL) and extracted with EtOAc, and the combined organic layer was washed with brine, dried over anhydrous $Na_2SO_4$ and concentrated under reduced pressure to obtain a crude product. The product was purified by silica gel column chromatography (65% EtOAc in petroleum ether) to give methyl 1-(3-acetamidophenyl)-6-oxo-pyridazine-3-carboxylate (400 mg, 35.62% yield) as a light-yellow solid. LC/MS (ESI) m/z = 288.0 $[M+H]^+$.

Steps 2 and 3: Synthesis of 1-(3-acetamidophenyl)-N-[(1R)-1-[3-(1,1-difluoro-2-hydroxy-ethyl)phenyl]ethyl]-6-oxo-pyridazine-3-carboxamide

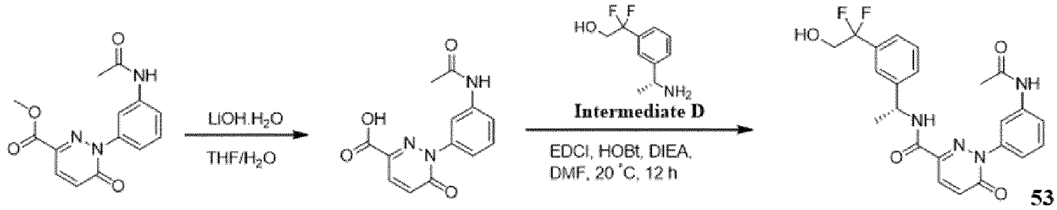


53

The compound of Example 53 was obtained by reacting methyl 1-(3-acetamidophenyl)-6-oxo-pyridazine-3-carboxylate in the same method as in Steps 2 and 3 of Example 23, except that, in Step 3, Intermediate C was replaced with Intermediate D, and the coupling reagent was changed to HOBt and EDCI. $^1H$ NMR (400 MHz, DMSO-$d_6$) $\delta$ 10.18 (s, 1H), 8.91 (d, $J$ = 8.4 Hz, 1H), 7.88 (d, $J$ = 9.6 Hz, 1H), 7.85 (s, 1H), 7.63 (d, $J$ = 8.4 Hz, 1H), 7.54-7.48 (m, 2H), 7.41 (s, 2H), 7.39-7.36 (m, 1H), 7.29 (br d, $J$ = 7.6 Hz, 1H), 7.13 (d, $J$ = 9.6 Hz, 1H), 5.63 (br s, 1H), 5.18 (quin, $J$= 7.2 Hz, 1H), 3.82 (br t, $J$= 14.4 Hz, 2H), 2.06 (s, 3H), 1.49-1.44 (m, 3H); LC/MS (ESI) m/z = 457.4 $[M+H]^+$.

**Example 54: (R)-N-(1-(3-amino-5-(trifluoromethyl)phenyl)ethyl)-5-cyclopropyl-6-oxo-1-phenyl-1,6-dihydropyridazine-3-carboxamide**

Step 1: Synthesis of 5-cyclopropyl-6-oxo-1-phenyl-pyridazine-3-carboxylic acid

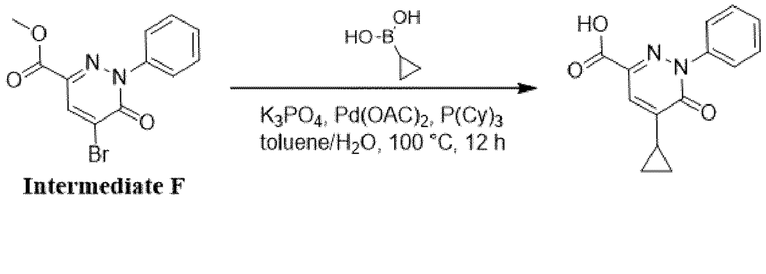


Intermediate F

A mixture of Intermediate F (50 mg, 161.75 μmol), cyclopropylboronic acid (18.06 mg, 210.28 μmol), $K_3PO_4$ (120.17 mg, 566.13 μmol), $Pd(OAc)_2$ (3.63 mg, 16.18 μmol) and $P(Cy)_3$ (9.07 mg, 32.35 μmol) in toluene (1 mL) and $H_2O$ (0.1 mL) was degassed and purged with $N_2$ for 3 times, and then stirred at 100 °C for 12 hours under $N_2$ atmosphere. The reaction mixture was poured into water (20 mL) and extracted with EtOAc, and the combined organic layer was discarded. The aqueous layer was acidified with 1N aq. HCl (pH = 3-4) and extracted with EtOAc. The combined organic layer was washed with brine, dried over anhydrous $Na_2SO_4$ and concentrated under reduced pressure to give 5-cyclopropyl-6-oxo-1-phenyl-pyridazine-3-carboxylic acid (41 mg, 84.67% yield) as a yellow solid. LC/MS (ESI) m/z = 257.1 $[M+H]^+$.

Step 2: Synthesis of (R)-N-(1-(3-amino-5-(trifluoromethyl)phenyl)ethyl)-5-cyclopropyl-6-oxo-1-phenyl-1,6-dihydropyridazine-3-carboxamide

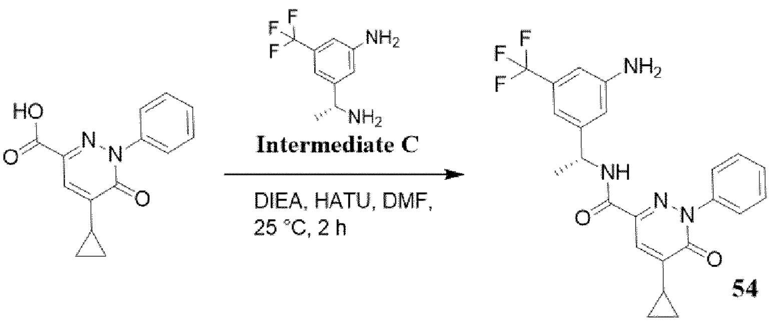

54

The compound of Example 54 was obtained by reacting 5-cyclopropyl-6-oxo-1-phenyl-pyridazine-3-carboxylic acid in the same method as in Step 3 of Example 23. $^1H$ NMR (400MHz, DMSO-$d_6$) δ8.78 (d, *J* = 8.4 Hz, 1H), 7.69-7.58 (m, 2H), 7.57-7.49 (m, 2H), 7.49-7.43 (m, 1H), 7.34 (s, 1H), 6.79 (d, *J* = 16.4 Hz, 2H), 6.69 (s, 1H), 5.53 (s, 2H), 5.01 (quin, *J* = 7.2 Hz, 1H), 2.26-2.13 (m, 1H), 1.43 (d, *J* = 7.2 Hz, 3H), 1.13-1.05 (m, 2H), 0.97-0.88 (m, 2H). LC/MS (ESI) m/z = 443.3 $[M+H]^+$.

**Example 55: (R)-N-(1-(3-amino-5-(trifluoromethyl)phenyl)ethyl)-4-cyclopropyl-6-oxo-1-phenyl-1,6-dihydropyridazine-3-carboxamide**

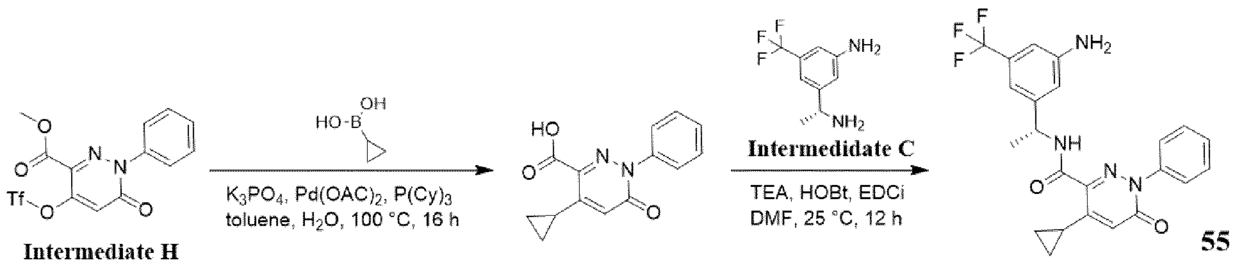

Intermediate H
55

The compound of Example 55 was obtained in the same method as in Example 54, except that Intermediate F was replaced with Intermediate H in Step 1 of Example 54, and the coupling reagent used in Step 2 was changed to HOBt and EDCI. $^1H$ NMR (400MHz, DMSO-$d_6$) δ 9.13 (d, J = 8.0 Hz, 1H), 7.66-7.58 (m, 2H), 7.53-7.47 (m, 2H), 7.45-7.40 (m, 1H), 6.79 (d, J = 14.4 Hz, 2H), 6.71 (s, 1H), 6.60 (s, 1H), 5.56 (s, 2H), 4.99 (quin, J =7.2 Hz, 1H), 2.05-1.95 (m, 1H), 1.40 (d, J = 7.2 Hz, 3H), 1.03-0.81 (m, 4H); LC/MS (m/z) = 443.3 $[M+H]^+$.

**Example 56: N-[(1R)-1-[3-amino-5-(trifluoromethyl)phenyl]ethyl]-5-cyano-6-oxo-1-phenyl-pyridazine-3-carboxamide**

Step 1: Synthesis of 5-bromo-N-[(1R)-1-[3-nitro-5-(trifluoromethyl)phenyl]ethyl]-6-oxo-1-phenyl-pyridazine-3-carboxamide

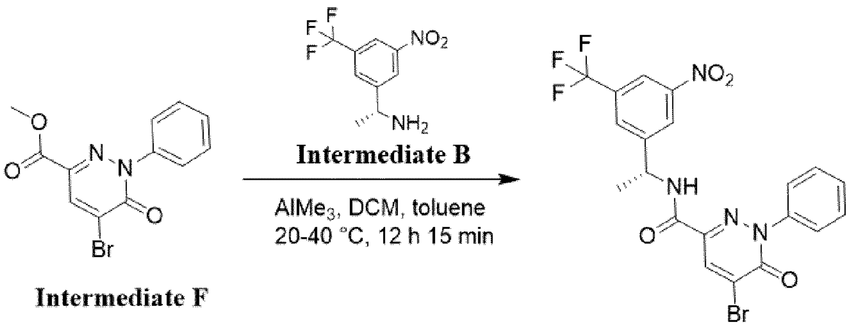


To a stirred solution of Intermediate B (400 mg, 1.71 mmol) in DCM (2 mL) and toluene (2 mL) was added $AlMe_3$ (2.67 mL, 4.27 mmol, 1.6 M solution in toluene) dropwise, and the mixture was stirred at 20 °C for 15 min under $N_2$ atmosphere. Then, Intermediate F (528 mg, 1.71 mmol) was added thereto, and the reaction mixture was stirred at 40 °C under $N_2$ atmosphere for another 12 hours. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by silica gel column chromatography (0-20% EtOAc in petroleum ether) to give 5-bromo-N-[(1R)-1-[3-nitro-5-(trifluoromethyl)phenyl]ethyl]-6-oxo-1-phenyl-pyridazine-3-carboxamide (253 mg, 28.97% yield) as a yellow solid. $^1H$ NMR (400MHz, CHLOROFORM-d) $\delta$ 8.39 (s, 3H), 7.93 (s, 1H), 7.59-7.54 (m, 4H), 7.53-7.50 (m, 1H), 7.35 (d, $J$ = 7.2 Hz, 1H), 5.38-5.30 (m, 1H), 1.65 (d, $J$ = 7.2 Hz, 3H); LC/MS (ESI) m/z = 511.1 $[M+H]^+$.

Step 2: Synthesis of 5-cyano- N-[(1R)-1-[3-nitro-5-(trifluoromethyl)phenyl]ethyl]-6-oxo-1-phenyl-pyridazine-3 -carboxamide

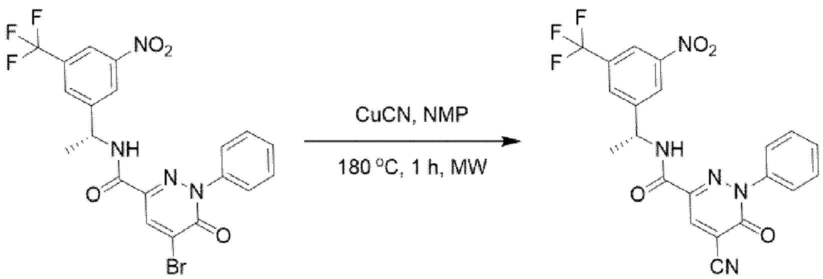


To a solution of 5-bromo-N-[(1R)-1-[3-nitro-5-(trifluoromethyl)phenyl]ethyl]-6-oxo-1-phenyl-pyridazine-3-carboxamide (163 mg, 318.83 μmol) in NMP (2 mL) was added CuCN (142.78 mg, 1.59 mmol, 348.24 μL), and the mixture was stirred at 180 °C for 1 hour in microwave under $N_2$. The reaction mixture was poured into water (20 mL) and extracted with EtOAc, and the combined organic layer was washed with brine, dried over anhydrous $Na_2SO_4$ and concentrated under reduced pressure to obtain a crude product. The product was purified by silica gel column chromatography (15% EtOAc in petroleum ether) to give 5-cyano-N-[(1R)-1-[3-nitro-5-(trifluoromethyl)phenyl]ethyl]-6-oxo-1-phenyl-pyridazine-3-carboxamide (140 mg, 81.99% yield) as yellow oil. LC/MS (ESI) m/z = 456.1 $[M+H]^+$.

Step 3: Synthesis of N-[(1R)-1-[3-amino-5-(trifluoromethyl)phenyl]ethyl]-5-cyano-6-oxo-1-phenyl-pyridazine-3-carboxamide

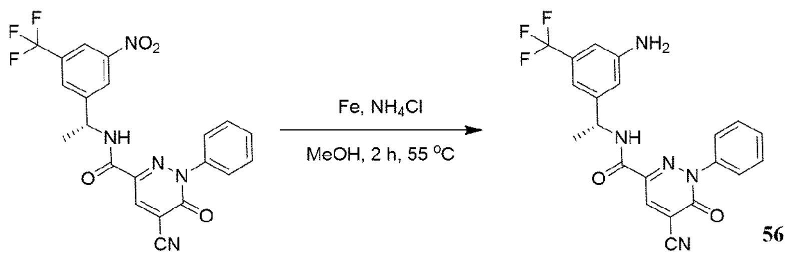

56

To a solution of 5-cyano-N-[(1R)-1-[3-nitro-5-(trifluoromethyl)phenyl]ethyl]-6-oxo-1-phenyl-pyridazine-3-carboxamide (140 mg, 306.10 μmol) in MeOH (3 mL) and $NH_4Cl$ (1 mL) was added Fe (170.94 mg, 3.06 mmol), followed by stirring at 55 °C for 2 hours. The reaction mixture was poured into water (30 mL) and extracted with EtOAc, and the combined organic layer was washed with brine, dried over anhydrous $Na_2SO_4$ and concentrated under reduced pressure to obtain a residue. The residue was purified by prep-HPLC (Phenomenex C18 75*30mm*3μm, mobile phase: [water ($NH_3H_2O$+$NH_4HCO_3$)-ACN]; B%: 31%-71%, 14min). Most of $CH_3CN$ was removed under reduced pressure, and the remaining solvent was removed by lyophilization to give the compound of Example 56 (9.6 mg, 7.27% yield) as a yellow solid. $^1$H NMR (400 MHz, DMSO-$d_6$) δ 9.02 (d, *J* = 8.0 Hz, 1H), 8.58 (s, 1H), 7.68 (d, *J* = 7.2 Hz, 2H), 7.59-7.49 (m, 3H), 6.82 (s, 1H), 6.78 (s, 1H), 6.70 (s, 1H), 5.55 (s, 2H), 5.05 (quin, *J* = 7.2 Hz, 1H), 1.44 (d, *J* = 7.2 Hz, 3H); LC/MS (ESI) m/z = 428.3 $[M+H]^+$.

**Example 57: N-[(1R)-1-[3-(1,1-difluoro-2-hydroxy-ethyl)phenyl]ethyl]-6-oxo-1-phenyl-pyridazine-3-carboxamide**

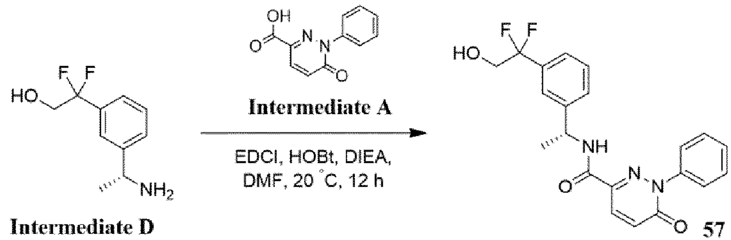

57

A mixture of Intermediate A (70 mg, 323.79 μmol), Intermediate D (91.21 mg, 453.30 μmol), EDCI (124.14 mg, 647.57 μmol), HOBt (87.50 mg, 647.57 μmol) and DIEA (104.62 mg, 809.46 μmol, 140.99 μL) in DMF (2 mL) was degassed and purged with $N_2$ for 3 times, and then was stirred at 20 °C for 12 hours under $N_2$ atmosphere. The reaction mixture was poured into water (20 mL) and extracted with EtOAc. The combined organic layer was washed with brine, dried over $Na_2SO_4$ and concentrated under reduced pressure to obtain a residue. The residue was purified by prep-HPLC (Phenomenex C18 75*30mm*3μm, mobile phase: [water ($NH_3H_2O$+$NH_4HCO_3$)-ACN]; B%: 22%-52%, 10min). $CH_3CN$ was removed under reduced pressure, and the remaining solvent was removed by lyophilization to give the compound of Example 57 (28.0 mg, 15.32% yield) as a white solid. $^1$H NMR(400 MHz, DMSO-$d_6$) δ 8.92(d, *J* = 8.4 Hz, 1H), 7.89(d, *J* = 9.8 Hz, 1H), 7.70-7.64(m, 2H), 7.57-7.50(m, 4H), 7.50-7.46(m, 1H), 7.45-7.37(m, 2H), 7.14(d, *J* = 9.8 Hz, 1H), 5.60(t, *J* = 6.4 Hz, 1H), 5.19(quin, *J* = 7.2 Hz, 1H), 3.83(dt, *J* = 6.4, 14.4 Hz, 2H), 1.49(d, *J* = 7.2 Hz, 3H); LC/MS (ESI) m/z = 400.3 $[M+H]^+$.

**Example 58: N-[(1R)-1-[3-(1,1-difluoro-2-hydroxy-ethyl)phenyl]ethyl]-1-(3-methylsulfonylphenyl)-6-oxo-pyridazine-3-carboxamide**

Step 1: Synthesis of methyl 1-(3-methylsulfonylphenyl)-6-oxo-pyridazine-3-carboxylate

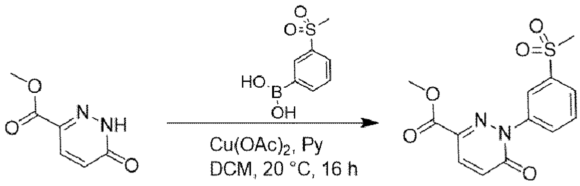


A mixture of methyl 6-oxo-1H-pyridazine-3-carboxylate (150 mg, 973.25 μmol), (3-methylsulfonylphenyl)boronic acid (214.14 mg, 1.07 mmol), $Cu(OAc)_2$ (35.35 mg, 194.65 μmol) and pyridine (500.39 mg, 6.33 mmol) in DCM (3 mL) was degassed and purged with $N_2$ for 3 times, and then stirred at 20 °C for 16 hours under $N_2$ atmosphere. The reaction mixture was poured into water (10 mL) and extracted with EtOAc. The combined organic layer was washed with brine, dried over $Na_2SO_4$ and concentrated under reduced pressure to obtain a crude product. The product was purified by flash silica gel chromatography (60% EtOAc in petroleum ether) to give methyl 1-(3-methylsulfonylphenyl)-6-oxo-pyridazine-3-carboxylate (300 mg, 41.67% yield) as a white solid. LC/MS (ESI) m/z = 308.9 $[M+H]^+$.

Step 2: Synthesis of N-[(1R)-1-[3-(1,1-difluoro-2-hydroxy-ethyl)phenyl]ethyl]-1-(3-methylsulfonylphenyl)-6-oxo-pyridazine-3-carboxamide

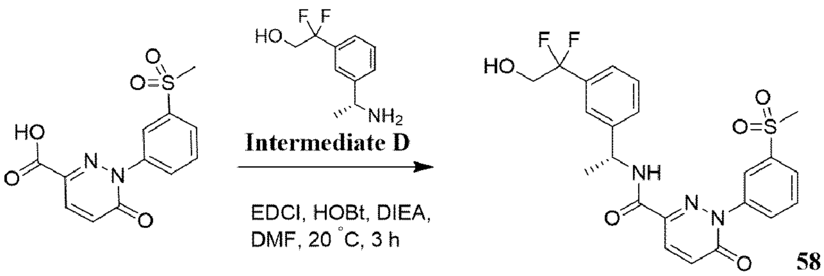


58

The compound of Example 58 was obtained in the same method as in Example 57, except that Intermediate A was replaced with 1-(3-methylsulfonylphenyl)-6-oxo-pyridazine-3-carboxylic acid. $^1$H NMR (400 MHz, DMSO-$d_6$) δ 9.03 (d, *J* = 8.4 Hz, 1H), 8.31 (t, *J* = 2.0 Hz, 1H), 8.12 - 8.01 (m, 2H), 7.92 (d, *J* = 9.6 Hz, 1H), 7.86 (t, *J* = 8.0 Hz, 1H), 7.57-7.52 (m, 2H), 7.45 (t, *J* = 7.2 Hz, 1H), 7.41-7.38 (m, 1H), 7.20 (d, *J* = 9.6 Hz, 1H), 5.63 (br s, 1H), 5.21 (quin, *J* = 7.2 Hz, 1H), 3.84 (t, *J* = 14.4 Hz, 2H), 3.41-3.31 (s, 3H), 1.51 (d, *J* = 7.2 Hz, 3H); LC/MS (ESI) m/z = 478.3 $[M+H]^+$.

**Example 59: N-[(1R)-1-[3-(1,1-difluoro-2-hydroxy-ethyl)-2-fluoro-phenyl]ethyl]-6-oxo-1-phenyl-pyridazine-3-carboxamide**

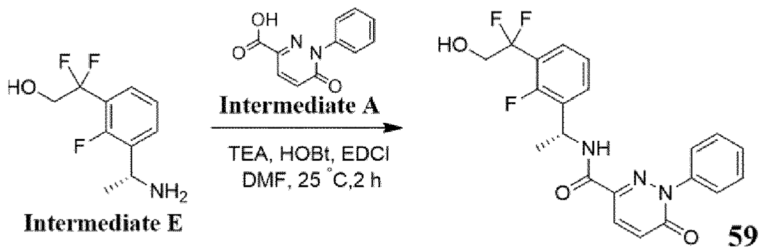


59

The compound of Example 59 was obtained in the same method as in Example 57, except that Intermediate D was replaced with Intermediate E. $^1$H NMR (400MHz, DMSO-$d_6$) δ 8.97 (d, *J* = 8.2 Hz, 1H), 7.88 (d, *J* = 9.6 Hz, 1H), 7.72-7.60 (m, 3H), 7.56 (t, *J* = 7.6 Hz, 2H), 7.51-7.39 (m, 2H), 7.32-7.25 (m, 1H), 7.14 (d, *J* = 9.6 Hz, 1H), 5.72 (t, *J* = 6.4 Hz, 1H), 5.40 (quin, *J* = 7.2 Hz, 1H), 3.91 (dt, *J* = 6.4, 14.4 Hz, 2H), 1.47 (d, *J* = 7.2 Hz, 3H); LC/MS (ESI) m/z = 418.3 $[M+H]^+$.

**Example 60: (R)-N-(1-(3-ethoxyphenyl)ethyl)-6-oxo-1-phenyl-1,6-dihydropyridazine-3-carboxamide**

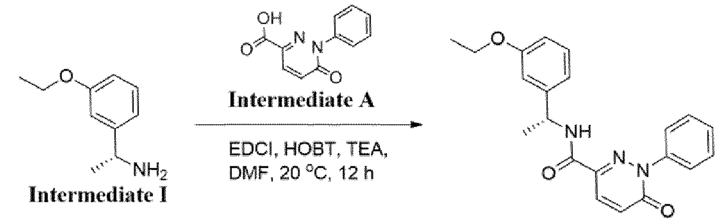


60

The compound of Example 60 was obtained in the same method as in Example 57, except that Intermediate D was replaced with Intermediate I. $^1H$ NMR (400MHz, DMSO-$d_6$) δ 8.80 (brd, *J* = 8.4 Hz, 1H), 7.88 (d, *J* = 9.6 Hz, 1H), 7.67 (d, *J* = 7.6 Hz, 2H), 7.54 (t, *J* = 7.6 Hz, 2H), 7.50-7.43 (m, 1H), 7.20 (t, *J* = 7.6 Hz, 1H), 7.14 (d, *J* = 9.6 Hz, 1H), 6.98-6.88 (m, 2H), 6.77 (d, *J* = 8.0 Hz, 1H), 5.10 (quin, *J* =7 .2 Hz, 1H), 3.97 (q, *J* = 6.8 Hz, 2H), 1.45 (d, *J* = 6.8 Hz, 3H), 1.29 (t, *J* = 6.8 Hz, 3H); LC/MS (ESI) m/z = 364.3 $[M+H]^+$.

**Example 61: 6-oxo-1-phenyl-N-[(1R)-1-(3-trimethylsilylphenyl)ethyl]pyridazine-3-carboxamide**

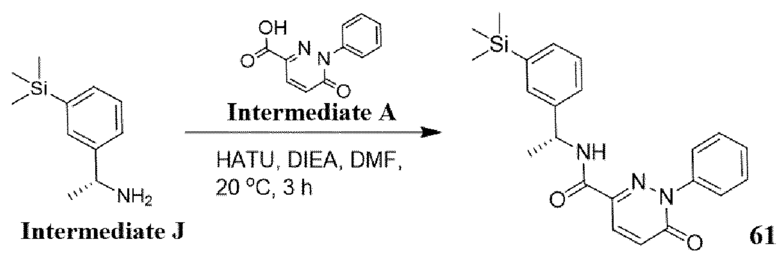


61

To a solution of Intermediate A (24.60 mg, 113.78 μmol) in DMF (2 mL), HATU (64.89 mg, 170.67 μmol) and TEA (34.54 mg, 341.33 μmol, 47.51 μL) were added, and the mixture was stirred at 20 °C for 15 min and added with Intermediate J (22 mg, 113.78 μmol), followed by stirring at 20 °C for 2.75 hours under $N_2$. The reaction mixture was poured into water (20 mL) and extracted with EtOAc. The combined organic layer was washed with brine, dried over anhydrous $Na_2SO_4$ and concentrated under reduced pressure to obtain a crude product. The product was purified by prep-HPLC (C18-6 100*30mm*5μm, mobile phase: [water (FA)-ACN]; B%: 62%-92%, 15min). Most of $CH_3CN$ was removed under reduced pressure, and the remaining solvent was removed by lyophilization to give the compound of Example 61 (11.4 mg, 23.65% yield) as an off-white solid. $^1H$ NMR (400 MHz, DMSO-$d_6$) δ 8.84 (d, *J* = 8.4Hz, 1H), 7.88 (d, *J* = 9.6Hz, 1H), 7.70-7.64 (m, 2H), 7.56-7.50 (m, 3H), 7.49-7.44 (m, 1H), 7.40-7.35 (m, 2H), 7.32-7.27 (m, 1H), 7.14 (d, *J* = 9.6Hz, 1H), 5.14 (quin, *J* = 7.2Hz, 1H), 1.47 (d, *J* = 7.2Hz, 3H), 0.22 (s, 9H); LC/MS (ESI) m/z = 392.4 $[M+H]^+$.

**Example 62: N-[(1R)-1-[3-amino-5-(trifluoromethyl)phenyl]ethyl]-6-oxo-1-phenylpyridine-3-carboxamide**

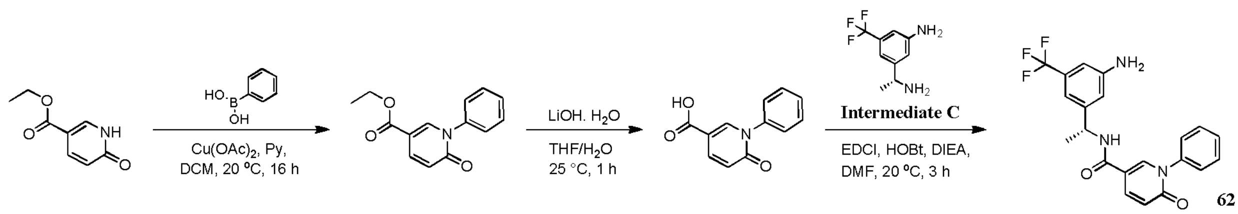


62

The compound of Example 62 was obtained in the same method as in Example 23, except that ethyl 6-oxo-1H-pyridine-3-carboxylate and phenylboronic acid were used as starting materials in Step 1 of Example 23, and the coupling reagent used in Step 3 was changed to HOBt and EDCI. $^1H$ NMR (400 MHz, DMSO-$d_6$) δ 8.59 (d, *J* = 7.6 Hz,

1H), 8.38 (d, *J* = 2.4 Hz, 1H), 7.95 (dd, *J* = 2.6, 9.6 Hz, 1H), 7.59-7.53 (m, 2H), 7.53-7.46 (m, 3H), 6.75 (s, 2H), 6.69 (s, 1H), 6.54 (d, *J* = 9.6 Hz, 1H), 5.56 (s, 2H), 5.01 (quin, *J* = 7.2 Hz, 1H), 1.39 (d, *J* = 7.2 Hz, 3H); LC/MS (ESI) m/z = 402.3 $[M+H]^+$.

**Example 63: N-[(1R)-1-[3-amino-5-(trifluoromethyl)phenyl]ethyl]-5-oxo-4-phenyl-pyrazine-2-carboxamide**

Step 1: Synthesis of N-[(1R)-1-[3-nitro-5-(trifluoromethyl)phenyl]ethyl]-5-oxo-4-phenyl-pyrazine-2-carboxamide

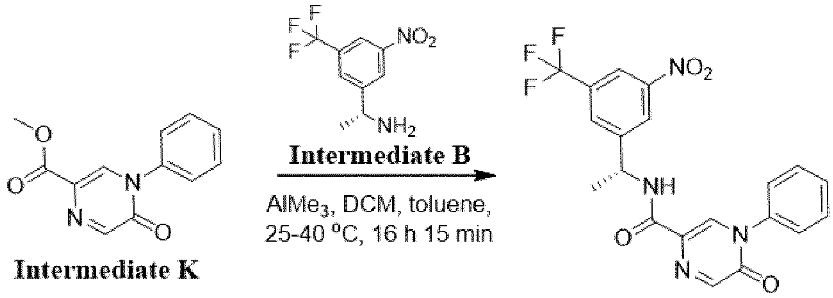


N-[(1R)-1-[3-nitro-5-(trifluoromethyl)phenyl]ethyl]-5-oxo-4-phenyl-pyrazine-2-carboxamide was obtained in the same method as in Step 1 of Example 56, with replacing Intermediate F with Intermediate K. LC/MS (ESI) m/z = 433.0 $[M+H]^+$.

Step 2: Synthesis of N-[(1R)-1-[3-amino-5-(trifluoromethyl)phenyl]ethyl]-5-oxo-4-phenyl-pyrazine-2-carboxamide

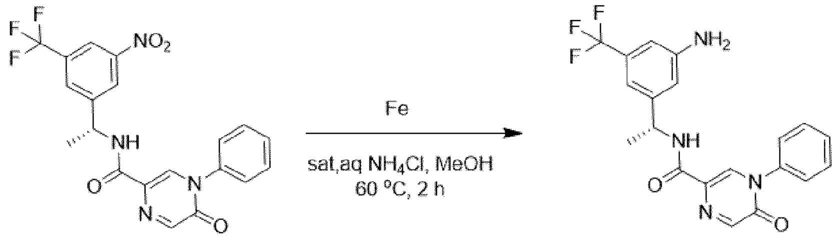


63

The compound of Example 63 was obtained by reacting N-[(1R)-1-[3-nitro-5-(trifluoromethyl)phenyl]ethyl]-5-oxo-4-phenyl-pyrazine-2-carboxamide in the same method as in Step 3 of Example 56. $^1H$ NMR (400 MHz, DMSO-$d_6$) δ 8.82 (d, *J* = 8.4 Hz, 1H), 8.14 (s, 1H), 8.03 (s, 1H), 7.58-7.50 (m, 5H), 6.87 (s, 1H), 6.80 (s, 1H), 6.70 (s, 1H), 5.55 (s, 2H), 5.08-5.00 (m, 1H), 1.47 (d, *J* = 7.2 Hz, 3H); LC/MS (ESI) m/z = 403.3 $[M+H]^+$.

**Example 64: N-[(1R)-1-[3-amino-5-(trifluoromethyl)phenyl]ethyl]-1-cyclopropyl-6-oxo-1,6-dihydropyridazine-3-carboxamide**

Step 1: Synthesis of 1-cyclopropyl-6-oxo-1,6-dihydropyridazine-3-carboxylic acid

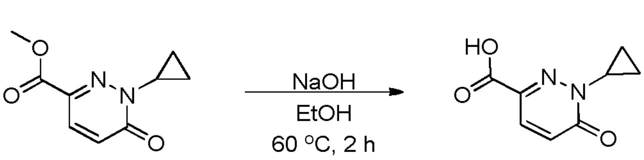


**Intermediate K-9**

To a solution of Intermediate K-9 (57.6 mg, 0.30 mmol) in EtOH (1.3 mL) was added NaOH (2N, 297 μL). The

mixture was stirred at 60 °C for 2 hours. The reaction mixture was cooled to room temperature and extracted with EtOAc. The aqueous layer was adjusted to pH = 2-3 with aq. 2 N HCl, and then extracted with EtOAc. The combined organic layer was washed with brine, dried over anhydrous $Na_2SO_4$ and concentrated under reduced pressure to give 1-cyclopropyl-6-oxo-1,6-dihydropyridazine-3-carboxylic acid (65.7 mg, crude). LC/MS (m/z) = 181.1 $[M + H]^+$.

Step 2: Synthesis of N-[(1R)-1-[3-amino-5-(trifluoromethyl)phenyl]ethyl]-1-cyclopropyl-6-oxo-1,6-dihydropyridazine-3-carboxamide

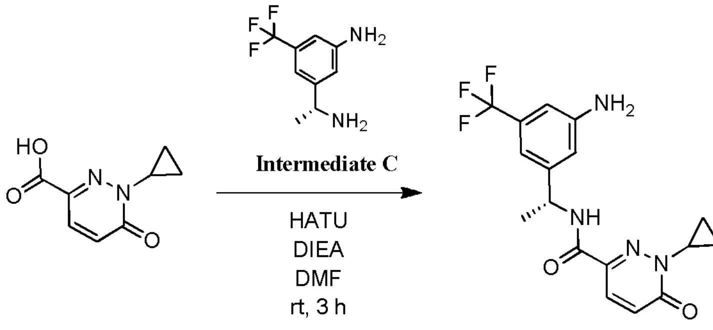

64

A mixture of 1-cyclopropyl-6-oxo-1,6-dihydropyridazine-3-carboxylic acid (65.7 mg, 0.36 mmol), Intermediate C (82 mg, 0.40 mmol), HATU (208 mg, 0.55 mmol) and DIEA (191 μL, 1.09 mmol) in DMF (2 mL) was degassed and purged with $N_2$ for 3 times, and then stirred at room temperature for 3 hours under $N_2$ atmosphere. The reaction mixture was poured into water and extracted with EtOAc. The combined organic layer was washed with brine, dried over anhydrous $Na_2SO_4$ and concentrated under reduced pressure to obtain a crude product. The residue was purified by prep-HPLC. The solvent was removed under reduced pressure to give the compound of Example 64 (20.3 mg, 15.2 % yield). $^1H$ NMR (400 MHz, MeOD) δ 7.90 (d, J = 9.6 Hz, 1H), 7.06 - 6.98 (m, 2H), 6.96 (s, 1H), 6.88 (s, 1H), 5.17 - 5.10 (m, 1H), 4.05 (tt, J = 7.6, 3.9 Hz, 1H), 2.19 (t, J = 7.6 Hz, 1H), 2.03 (d, J = 6.1 Hz, 1H), 1.56 (d, J = 7.0 Hz, 3H), 1.13 - 1.04 (m, 2H). LC/MS (m/z) = 367.1 $[M + H]^+$.

**Example 65 and Example 66**

The compounds shown in the following table were prepared in the same manner as in Example 64 by using starting materials corresponding to the respective structures of the desired compounds based on Preparation Example 11.

[Table 3]

| No. | Structure/Name | Spectral Data |
|---|---|---|
| 65 | 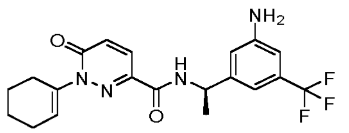 N-[(1R)-1-[3-amino-5-(trifluoromethyl)phenyl]ethyl]-1-(cyclohex-1-en-1-yl)-6-oxo-1,6-dihydropyridazine-3-carboxamide | $^1H$ NMR (400 MHz, DMSO) δ 8.81 (d, J = 8.3 Hz, 1H), 7.80 (d, J = 9.7 Hz, 1H), 7.01 (d, J = 9.7 Hz, 1H), 6.88 (s, 1H), 6.83 (s, 1H), 6.75 (s, 1H), 6.05 - 5.98 (m, 1H), 5.08 - 5.00 (m, 1H), 3.96 (s, 1H), 2.37 - 2.30 (m, 1H), 2.21 (dd, J = 6.6, 3.4 Hz, 2H), 1.79 - 1.70 (m, 2H), 1.68 - 1.59 (m, 2H), 1.48 (d, J = 7.1 Hz, 3H); LC/MS (ESI) m/z = 407.1 $[M+H]^+$ |
| 66 | 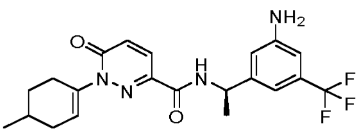 N-[(1R)-1-[3-amino-5-(trifluoromethyl)phenyl]ethyl]-1-(4-methylcyclohex-1-en-1yl)-6-oxo-1,6-dihydropyridazine-3-carboxamide | $^1H$ NMR (400 MHz, DMSO) δ 8.78 (d, J = 8.3 Hz, 1H), 7.80 (d, J = 9.7 Hz, 1H), 7.01 (d, J = 9.7 Hz, 1H), 6.81 (d, J = 16.6 Hz, 2H), 6.71 (s, 1H), 5.99 (s, 1H), 5.57 (s, 2H), 5.07 - 4.99 (m, 1H), 2.43 (s, 1H), 2.38 - 2.25 (m, 2H), 1.80 (s, 2H), 1.75 (s, 1H), 1.47 (d, J = 7.1 Hz, 3H), 1.40 (dt, J = 11.9, 5.5 Hz, 1H), 1.01 (d, J = 6.3 Hz, 3H); LC/MS (ESI) m/z = 421.2 $[M+H]^+$ |

**Example 67: N-[(1R)-1-[3-amino-5-(trifluoromethyl)phenyl]ethyl]-1-(1-methanesulfonyl-1,2,3,6-tetrahydropyridin-4-yl)-6-oxo-1,6-dihydropyridazine-3-carboxamide**

Step 1: Synthesis of methyl 1-(1-(methylsulfonyl)-1,2,3,6-tetrahydropyridin-4-yl)-6-oxo-1,6-dihydropyridazine-3-carboxylate

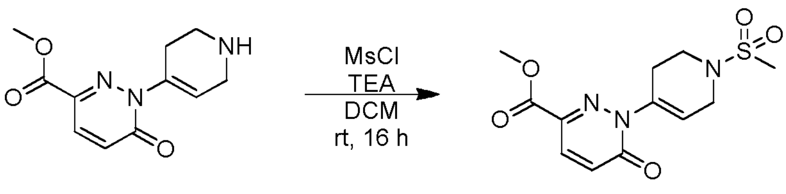


**Intermediate K-10**

To a solution of Intermediate K-10 (48.5 mg, 0.21 mmol) in DCM (200 μL) was added TEA (86 μL, 0.62 mmol). The mixture was cooled to 0-5 °C and then added slowly dropwise with methanesulfonyl chloride (19.15 μL, 0.25 mmol), maintaining the reaction temperature to be lower than 20 °C. After the addition, the mixture was stirred at room temperature for 16 hours. The reaction was quenched by slow addition of $H_2O$, and the layers were separated. The aqueous layer was extracted with DCM. The combined organic layer was washed successively with sat. $NH_4C1$, sat. $NaHCO_3$, sat. $NH_4Cl$ and brine, dried over anhydrous $Na_2SO_4$, and concentrated under reduced pressure to obtain a crude product. The residue was purified by prep-HPLC. The solvent was removed under reduced pressure to give methyl 1-(1-(methylsulfonyl)-1,2,3,6-tetrahydropyridin-4-yl)-6-oxo-1,6-dihydropyridazine-3-carboxylate (7.8 mg, 12.1 % yield). LC/MS (m/z) = 314.1 $[M + H]^+$.

Steps 2 and 3: Synthesis of N-[(1R)-1-[3-amino-5-(trifluoromethyl)phenyl]ethyl]-1-(1-methanesulfonyl-1,2,3,6-tetrahydropyridin-4-yl)-6-oxo-1,6-dihydropyridazine-3-carboxamide

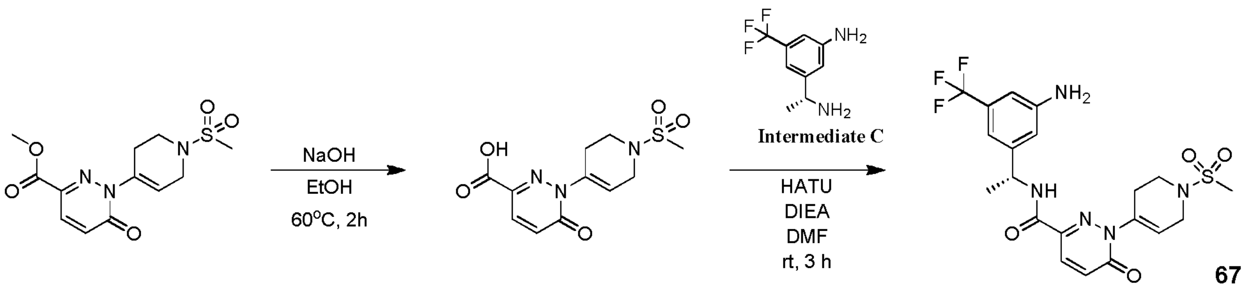


67

The compound of Example 67 (2.6 mg, 31.4 % yield) was obtained in the same manner as in Example 64. $^1H$ NMR (400 MHz, DMSO) δ 8.84 (d, J = 8.3 Hz, 1H), 7.83 (d, J = 9.6 Hz, 1H), 7.05 (d, J = 9.8 Hz, 1H), 6.83 (s, 1H), 6.79 (s, 1H), 6.70 (d, J = 7.2 Hz, 3H), 6.28 (s, 2H), 5.04 (s, 1H), 3.94 (s, 2H), 3.42 (d, J = 5.4 Hz, 1H), 2.99 (s, 3H), 2.67 (s, 2H), 1.47 (d, J = 6.9 Hz, 3H); LC/MS m/z = 486.2 $[M+H]^+$.

**Example 68: 1-(1-acetyl-1,2,3,6-tetrahydropyridin-4-yl)-N-[(1R)-1-[3-amino-5-(trifluoromethyl)phenyl]ethyl]-6-oxo-1,6-dihydropyridazine-3-carboxamide**

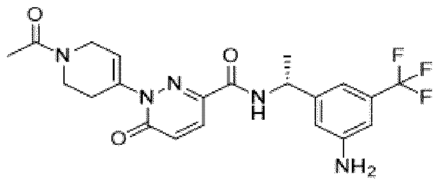

68

The compound of Example 68 (5.3 mg, 15 % yield) was obtained in the same manner as in Example 67, except that methanesulfonyl chloride was replaced with acetic anhydride in Step 1 of Example 67. $^1$H NMR (400 MHz, MeOD) δ 7.94 (dd, J = 9.7, 1.3 Hz, 1H), 7.06 (dd, J = 9.7, 1.3 Hz, 1H), 6.90 (d, J = 9.7 Hz, 2H), 6.81 (s, 1H), 6.18 (s, 1H), 5.13 (q, J = 7.1 Hz, 1H), 4.31 - 4.25 (m, 2H), 3.90 - 3.77 (m, 2H), 2.67 (s, 1H), 2.59 (s, 1H), 2.18 (d, J = 7.6 Hz, 3H), 1.54 (d, J = 7.1 Hz, 3H); LC/MS m/z = 450.2 $[M+H]^+$.

**Example 69: N-[(1R)-1-[3-amino-5-(trifluoromethyl)phenyl]ethyl]-1-(1-methanesulfonyl-1,2,5,6-tetrahydropyridin-3-yl)-6-oxo-1,6-dihydropyridazine-3-carboxamide**

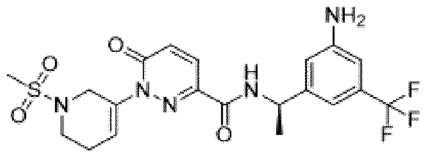

69

Intermediate K-11 used as a starting material was adidified, and the compound of Example 69 (3.5 mg, 11.5 % yield) was then obtained in the same manner as in Example 67. $^1$H NMR (400 MHz, MeOD) δ 7.94 (d, J = 9.7 Hz, 1H), 7.26 (s, 1H), 7.18 (s, 1H), 7.11 - 7.03 (m, 2H), 6.30 (dq, J = 4.2, 2.0 Hz, 1H), 5.18 (q, J = 6.9 Hz, 1H), 4.14 (d, J = 2.2 Hz, 1H), 3.53 (t, J = 5.8 Hz, 2H), 3.00 (s, 3H), 2.53 (dq, J = 5.9, 3.2 Hz, 2H), 1.57 (d, J = 7.1 Hz, 3H); LC/MS m/z = 486.2 $[M+H]^+$.

**Example 70: N-[(1R)-1-[3-amino-5-(trifluoromethyl)phenyl]ethyl]-1-fbicyclo[1.1.1]pentan-1-yll-6-oxo-1,6-dihydropyridazine-3-carboxamide**

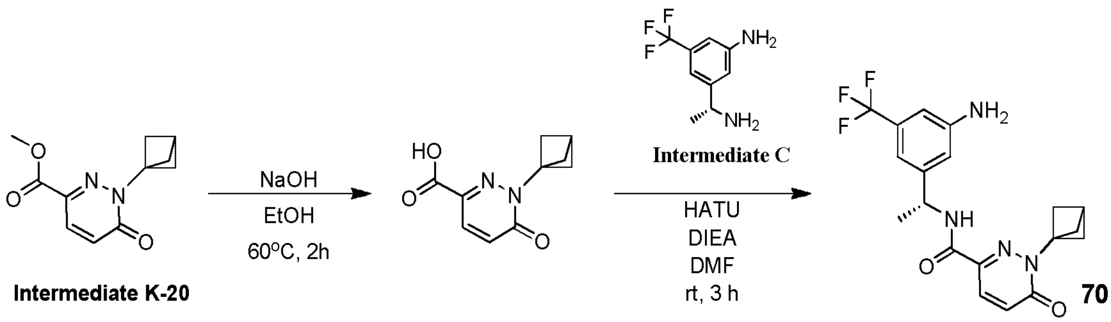


70

The compound of Example 70 (2.9 mg, 90 % yield) was obtained in the same manner as in Example 64. $^1$H NMR (400 MHz, MeOD) δ 7.61 (s, 1H), 7.29 (s, 1H), 7.20 (s, 1H), 7.12 (s, 1H), 5.16 (q, J = 7.0 Hz, 1H), 2.58 (s, 1H), 2.19 (s, 6H), 1.56 (d, J = 7.1 Hz, 3H); LC/MS m/z = 393.2 $[M+H]^+$.

**Example 71: N-[(1R)-1-[3-amino-5-(trifluoromethyl)phenyl]ethyl]-1-[3-(hydroxymethyl)phenyl]-6-oxo-1,6-dihydropyridine-3-carboxamide**

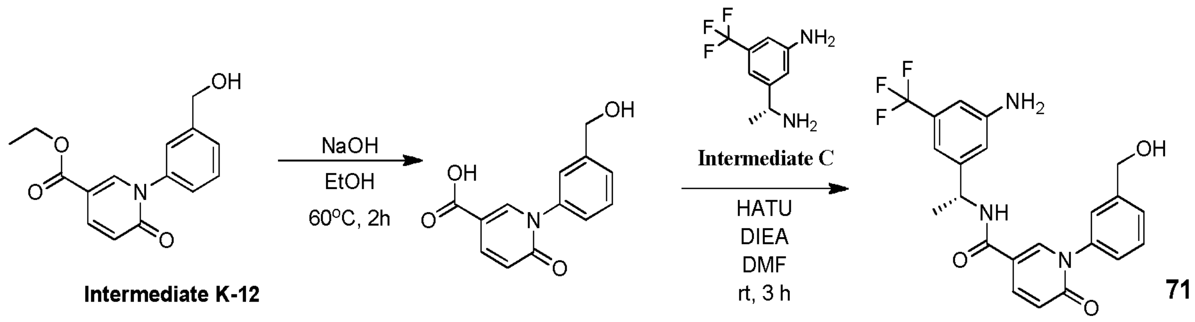


Intermediate K-12

71

The compound of Example 71 (38.1 mg, 87 % yield) was obtained in the same manner as in Example 64. $^1$H NMR (400 MHz, DMSO) δ 8.59 (d, J = 7.7 Hz, 1H), 8.35 (d, J = 2.6 Hz, 1H), 7.96 (dd, J = 9.6, 2.7 Hz, 1H), 7.55 - 7.30 (m, 5H), 6.77 (s, 2H), 6.71 (s, 1H), 6.53 (d, J = 9.6 Hz, 1H), 5.01 (t, J = 7.3 Hz, 1H), 4.58 (s, 2H), 1.39 (d, J = 7.1 Hz, 3H); LC/MS m/z = 432.2 $[M+H]^+$.

**Example 72 and Example 73**

The compounds shown in the table below were prepared in the same manner as in Example 71 by using appropriate starting materials corresponding to the respective structures of the desired compounds based on Preparation Example 7.

[Table 4]

| No. | Structure/Name | Spectral Data |
|---|---|---|
| 72 | 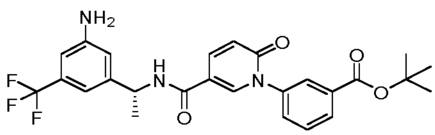 Tert-butyl3-(5-{[(1R)-1-[3-amino-5-(trifluoromethyl) phenyl]ethyl]carbamoyl}-2-oxo-1,2-dihydropyridin-1-yl)benzoate | $^1$H NMR (400 MHz, MeOD) δ 8.30 (dd, J = 7.6, 2.7 Hz, 1H), 8.13 - 7.99 (m, 3H), 7.66 (dd, J = 4.9, 2.0 Hz, 3H), 6.99 (d, J = 13.3 Hz, 2H), 6.90 (s, 1H), 6.66 (d, J = 9.6 Hz, 1H), 5.12 (d, J = 7.0 Hz, 1H), 1.60 (s, 9H), 1.51 (d, J = 7.1 Hz, 3H). LC/MS (m/z) = 502.2 $[M+H]^+$ |
| 73 | 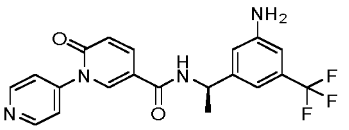 N-[(1R)-1-[3-amino-5-(trifluoromethyl)phenyl]ethyl]-2-oxo-2H-[1,4'-bipyridine]-5-carboxamide | $^1$H NMR (400 MHz, MeOD) δ 8.78 - 8.72 (m, 2H), 8.30 (d, J = 2.6 Hz, 1H), 8.04 (dd, J = 9.6, 2.6 Hz, 1H), 7.65 - 7.59 (m, 2H), 6.91 - 6.85 (m, 2H), 6.80 (d, J = 2.0 Hz, 1H), 6.65 (d, J = 9.6 Hz, 1H), 5.19 - 5.05 (m, 1H), 1.51 (d, J = 7.1 Hz, 3H). LC/MS (m/z) = 403.2 [M+H]+. LC/MS (m/z) = 403.2 $[M+H]^+$ |

**Example 74: N-[(1R)-1-[3-amino-5-(trifluoromethyl)phenyl]ethyl]-1'-methanesulfonyl-6-oxo-2',3'-dihydro-1'H,6H,6'H-[1,4'-bipyridine]-3-carboxamide**

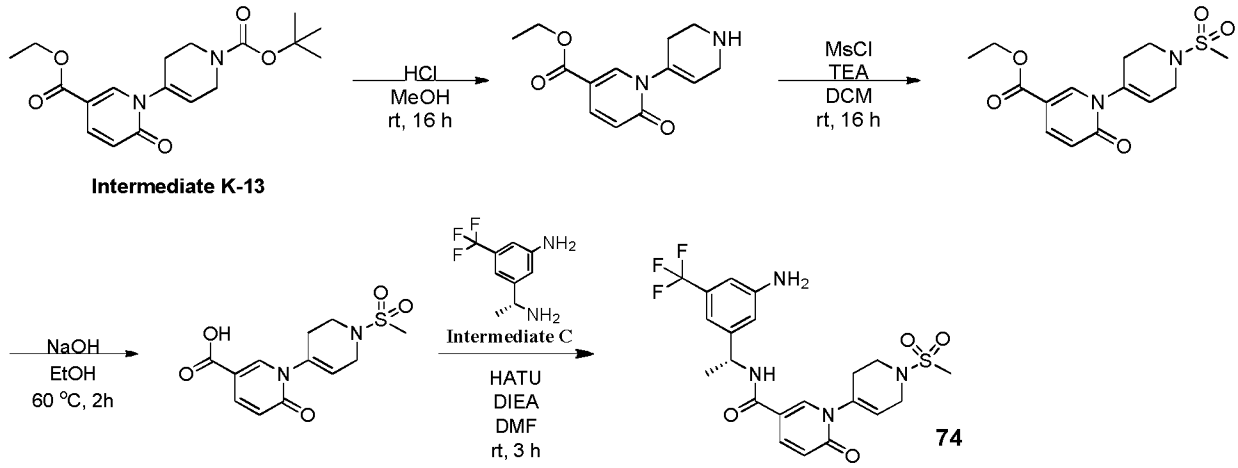


**Intermediate K-13**

74

Intermediate K-13 was acidified, and the compound of Example 74 (7.2 mg, 7.3 % yield) was then obtained in the same manner as in Example 67. $^1H$ NMR (400 MHz, MeOD) δ 8.18 (d, J = 2.5 Hz, 1H), 7.99 (dd, J = 9.6, 2.6 Hz, 1H), 6.88 (q, J = 1.7 Hz, 2H), 6.80 (d, J = 2.0 Hz, 1H), 6.54 (d, J = 9.6 Hz, 1H), 6.04 - 5.97 (m, 1H), 5.10 (q, J = 7.0 Hz, 1H), 4.01 (q, J = 2.9 Hz, 2H), 3.61 - 3.53 (m, 2H), 2.95 (s, 3H), 2.61 (dq, J = 6.1, 3.1 Hz, 2H), 1.51 (d, J = 7.1 Hz, 3H); LC/MS m/z = 485.2 $[M+H]^+$.

**Example 75: N-[(1R)-1-[3-amino-5-(trifluoromethyl)phenyl]ethyl]-1-[3-(morpholine-4-carbonyl)phenyl]-6-oxo-1,6-dihydropyridine-3-carboxamide**

Step 1: Synthesis of (R)-3-(5-((1-(3-amino-5-(trifluoromethyl)phenyl)ethyl)carbamoyl)-2-oxopyridin-1(2H)-yl)benzoic acid

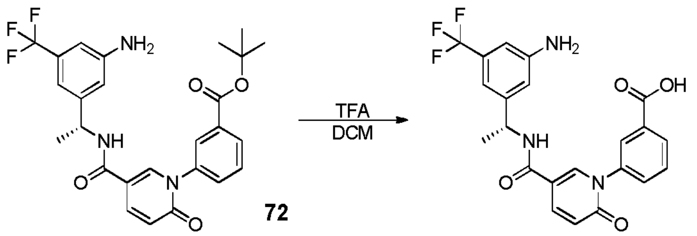


72

To a solution of the compound of Example 72 (10.2 mg, 0.020 mmol) in DCM (1 mL) was added TFA (7.83 μL, 0.10 mmol). The mixture was stirred at room temperature. The mixture was concentrated under reduced pressure to give (R)-3-(5-((1-(3-amino-5-(trifluoromethyl)phenyl)ethyl)carbamoyl)-2-oxopyridin-1(2H)-yl)benzoic acid (crude, 10 mg, 0.022 mmol), which was used in the next step without further purification. LC/MS m/z = 446.2 $[M+H]^+$.

Step 2: Synthesis of N-[(1R)-1-[3-amino-5-(trifluoromethyl)phenyl]ethyl]-1-[3-(morpholine-4-carbonyl) phenyl]-6-oxo-1,6-dihydropyridine-3-carboxamide

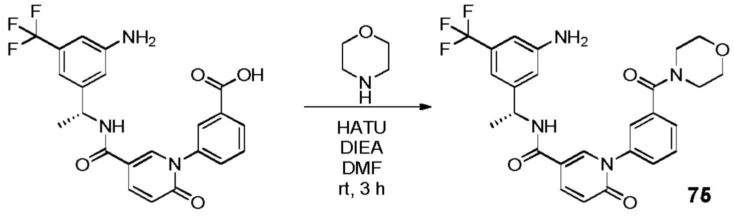


75

A mixture of (R)-3-(5-((1-(3-amino-5-(trifluoromethyl)phenyl)ethyl)carbamoyl)-2-oxopyridin-1(2H)-yl)benzoic

acid (10 mg, 0.022 mmol), morpholine (2.15 mg, 0.025 mmol), HATU (12.81 mg, 0.034 mmol) and DIEA (8.6 µL, 0.067 mmol) in DMF (0.1 mL) was degassed and purged with $N_2$ for 3 times, and then stirred at room temparature for 3 hours under $N_2$ atmosphere. The reaction mixture was poured into water and extracted with EtOAc. The combined organic layer was washed with brine, dried over anhydrous $Na_2SO_4$ and concentrated under reduced pressure to obtain a crude product. The residue was purified by prep-HPLC. The solvent was removed under reduced pressure to give the compound of Example 75 (0.9 mg, 7.8% yield). $^1$H NMR (400 MHz, MeOD) δ 8.31 (d, J = 2.7 Hz, 1H), 8.05 (dd, J = 9.6, 2.6 Hz, 1H), 7.71 - 7.63 (m, 1H), 7.58 (d, J = 7.4 Hz, 3H), 7.11 (s, 1H), 7.06 (s, 1H), 6.99 (s, 1H), 6.66 (d, J = 9.6 Hz, 1H), 5.14 (q, J = 7.1 Hz, 1H), 3.76 (s, 5H), 3.65 (s, 3H), 3.52 (s, 2H), 1.52 (d, J = 7.1 Hz, 3H); LC/MS m/z = 515.2 $[M+H]^+$ .

**Example 76: N-(1-(3-chlorophenyl)cyclopropyl)-1-(2-fluorophenyl)-6-oxo-1,6-dihydropyridazine-3-carboxamide**

Step 1: Synthesis of 1-(2-fluorophenyl)-6-oxo-1,6-dihydropyridazine-3-carboxylic acid

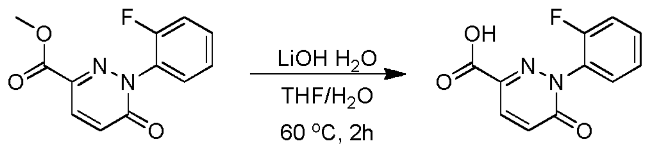


**Intermediate K-14**

To a solution of Intermediate K-14 (91.3 mg, 0.37 mmol) in THF (1 mL), LiOH•$H_2O$ (30.9 mg, 0.74 mmol) and $H_2O$ (0.5 mL) were added. The mixture was stirred at 25 °C for 3 hours. The reaction mixture was adjusted to pH = 3-4 with aq. 1 N HCl and extracted with EtOAc. The aqueous layer was filtrated to give 1-(2-fluorophenyl)-6-oxo-1,6-dihydropyridazine-3-carboxylic acid (79.9 mg, 0.37 mmol, 93 % yield) as a yellow solid. LC/MS (m/z) = 235.1 $[M+H]^+$.

Step 2: Synthesis of N-(1-(3-chlorophenyl)cyclopropyl)-1-(2-fluorophenyl)-6-oxo-1,6-dihydropyridazine-3-carboxamide

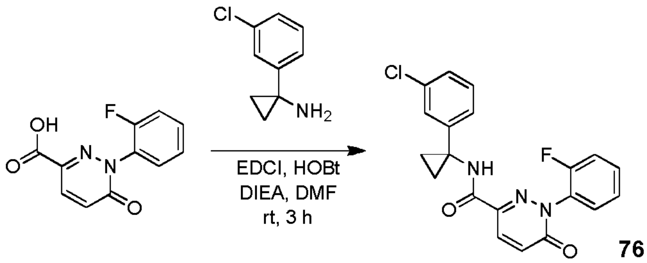


**76**

To a solution of 1-(2-fluorophenyl)-6-oxo-1,6-dihydropyridazine-3-carboxylic acid (25 mg, 0.11 mmol) and 1-(3-chlorophenyl)cyclopropan-1-amine (15.9 µL, 0.12 mmol) in DMF (1 mL) EDCI (21.7 mg, 0.14 mmol), HOBt (18.9 mg, 0.14 mmol) and DIEA (55.8 µL, 0.32 mmol) were added, and the mixture was degassed and purged with $N_2$ for 3 times, and then stirred at 25 °C for 1 hour under $N_2$ atmosphere. The reaction mixture was partitioned between $H_2O$ and EtOAc. The organic layer was separated, washed with brine, dried over $Na_2SO_4$, filtered and concentrated under reduced pressure to obtain a residue. The residue was purified by prep-HPLC to give the compound of Example 76 (9.4 mg, 22.9 % yield). LC/MS m/z = 384.1 $[M+H]^+$.

**Example 77: N-[(1R)-1-(3-chlorophenyl)propyl]-1-(2-fluorophenyl)-6-oxo-1,6-dihydropyridazine-3-carboxamide**

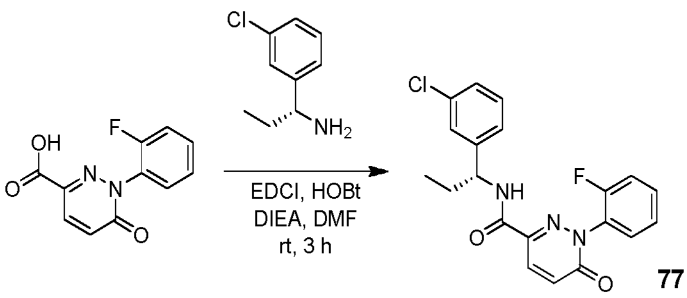


77

The compound of Example 77 (17.5 mg, 38.6 % yield) was obtained in the same manner as in Example 76, except that (R)-1-(3-chlorophenyl)propan-1-amine was used instead of 1-(3-chlorophenyl)cyclopropan-1-amine. LC/MS m/z = 386.1 [M+H]$^+$.

**Example 78: N-[(1R)-1-[3-(1,1-difluoro-2-hydroxyethyl)-2-fluorophenyl]ethyl]-1-[3-(1-methyl-1H-pyrazol-5-yl)phenyl]-6-oxo-1,6-dihydropyridine-3-carboxamide**

Steps 1 and 2: Synthesis of (R)-1-(3-bromophenyl)-N-(1-(3-(1,1-difluoro-2-hydroxyethyl)-2-fluorophenyl)ethyl)-6-oxo-1,6-dihydropyridine-3-carboxamide

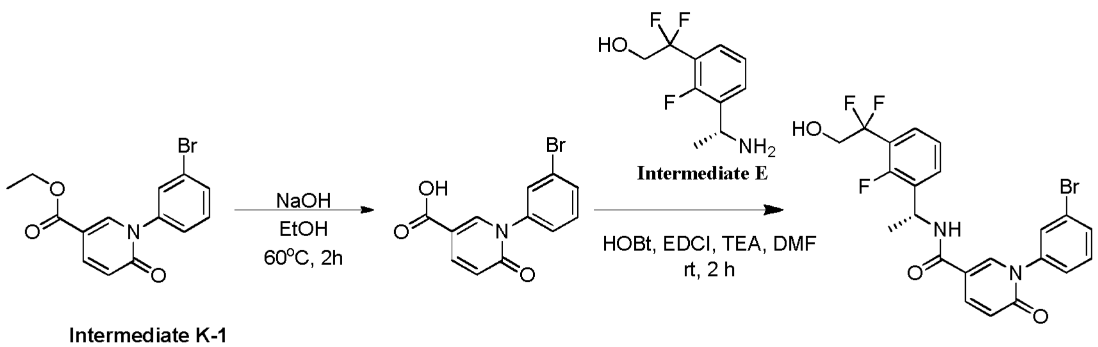


Intermediate K-1

(R)-1-(3-bromophenyl)-N-(1-(3-(1,1-difluoro-2-hydroxyethyl)-2-fluorophenyl)ethyl)-6-oxo-1,6-dihydropyridine-3-carboxamide (20.2 mg, 55.3 % yield) was obtained in a similar manner to Example 64, with changing the coupling reagents used in Step 2 to HOBt and EDCI. LC/MS m/z = 495 [M+H]$^+$.

Step 3: Synthesis of N-[(1R)-1-[3-(1,1-difluoro-2-hydroxyethyl)-2-fluorophenyl]ethyl]-1-[3-(1-methyl-1H-pyrazol-5-yl)phenyl]-6-oxo-1,6-dihydropyridine-3-carboxamide

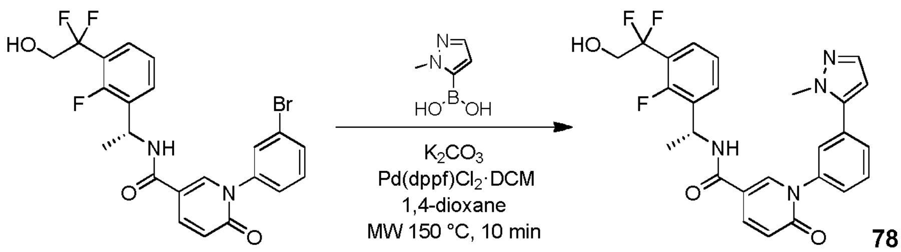


78

To a solution of (R)-1-(3-bromophenyl)-N-(1-(3-(1,1-difluoro-2-hydroxyethyl)-2-fluorophenyl)ethyl)-6-oxo-1,6-dihydropyridine-3-carboxamide (13.4 mg, 0.027 mmol) in 1,4-dioxane (0.5 mL) and water (0.1 mL), (1-methyl-1H-pyrazol-5-yl)boronic acid (3.75 mg, 0.030 mmol), potassium carbonate (14.96 mg, 0.11 mmol) and $Pd(dppf)Cl_2 \cdot DCM$ (2.21 mg, 2.71 μmol) were added. The mixture was heated by microwave at 150 °C for 10 min. The mixture was cooled to room temperature, added with water and extracted with DCM. The organic layer was washed with brine, dried over anhydrous

$Na_2SO_4$, and concentrated under reduced pressure to obtain a crude product. The residue was purified by prep-HPLC. The solvent was removed under reduced pressure to give the compound of Example 78 (3.7 mg, 27.5 % yield). $^1H$ NMR (400 MHz, MeOD) δ 8.35 (d, J = 2.6 Hz, 1H), 8.05 (dd, J = 9.6, 2.6 Hz, 1H), 7.73 - 7.61 (m, 3H), 7.54 (dt, J = 9.1, 1.9 Hz, 2H), 7.32 (s, 1H), 7.24 (s, 1H), 7.16 (s, 1H), 6.67 (dd, J = 9.6, 0.7 Hz, 1H), 6.47 (d, J = 2.0 Hz, 1H), 5.16 (q, J = 7.1 Hz, 1H), 3.92 (s, 3H), 1.54 (d, J = 7.1 Hz, 3H); LC/MS m/z = 482.2 $[M+H]^+$.

**Example 79: N-[(1R)-1-[3-amino-5-(trifluoromethyl)phenyl]ethyl]-1-[3-(methylamino)phenyl]-6-oxo-1,6-dihydropyridine-3-carboxamide**

Step 1: Synthesis of ethyl 1-[3-[tert-butoxycarbonyl(methyl)amino]phenyl]-6-oxo-pyridine-3-carboxylate

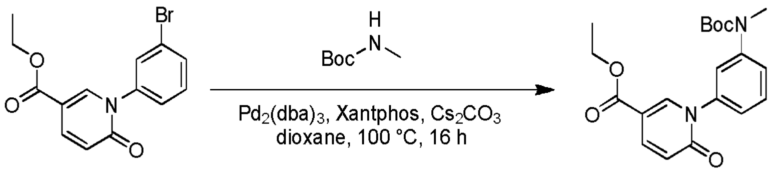


**Intermediate K-1**

A mixture of Intermediate K-1 (300 mg, 931 μmol), tert-butyl N-methylcarbamate (146 mg, 1.12 mmol), $Pd_2(dba)_3$ (85.2 mg, 93.1 μmol), Xantphos (53.8 mg, 93.1 μmol) and $Cs_2CO_3$ (758 mg, 2.33 mmol) in dioxane (6 mL) was stirred at 100 °C for 16 hours. The mixture was concentrated in vacuo. The residue was purified by column chromatography ($SiO_2$, PE: EA = 2: 1 to 5: 1) to give ethyl 1-[3-[tert-butoxycarbonyl(methyl)amino]phenyl]-6-oxo-pyridine-3-carboxylate (200 mg, 57% yield) as yellow oil. $^1H$ NMR (400 MHz, DMSO-$d_6$) δ = 8.21 (d, *J* = 2.0 Hz, 1H), 7.88 (dd, *J* = 2.4, 9.6 Hz, 1H), 7.54 - 7.41 (m, 3H), 7.26 (br d, *J* = 7.2 Hz, 1H), 6.56 (d, *J* = 9.6 Hz, 1H), 4.25 (q, *J* = 7.2 Hz, 2H), 3.23 (s, 3H), 1.42 (s, 9H), 1.26 (t, *J* = 7.2 Hz, 3H).

Steps 2 and 3: Synthesis of tert-butyl N-[3-[5-[[(1R)-1-[3-nitro-5-(trifluoromethyl)phenyl]ethyl]carbamoyl]-2-oxo-1-pyridyl]phenyl]-N-methyl-carbamate

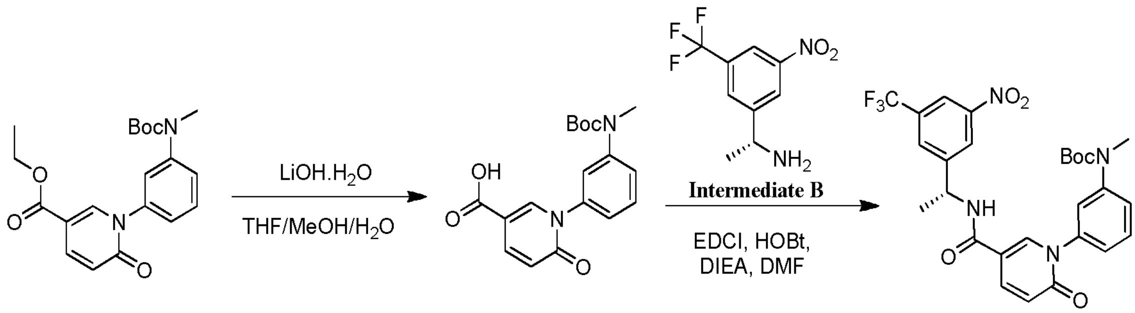


Tert-butyl N-[3-[5-[[(1R)-1-[3-nitro-5-(trifluoromethyl)phenyl]ethyl]carbamoyl]-2-oxo-1-pyridyl]phenyl]-N-methyl-carbamate (150 mg, 76 % yield) was obtained in the same manner as in Example 76. $^1H$ NMR (400 MHz, DMSO-$d_6$) δ = 8.79 (d, *J* = 7.2 Hz, 1H), 8.52 (s, 1H), 8.36 (d, *J* = 2.4 Hz, 2H), 8.22 (s, 1H), 7.97 - 7.88 (m, 1H), 7.54 - 7.48 (m, 1H), 7.46 - 7.41 (m, 2H), 7.28 (d, *J* = 7.6 Hz, 1H), 6.54 (d, *J* = 9.6 Hz, 1H), 5.31 (t, *J* = 6.8 Hz, 1H), 3.32 (s, 3H), 1.49 (d, *J* = 7.2 Hz, 3H), 1.42 (s, 9H).

Step 4: Synthesis of tert-butyl N-[3-[5-[[(1R)-1-[3-amino-5-(trifluoromethyl)phenyl]ethyl]carbamoyl]-2-oxo-1-pyridyl]phenyl]-N-methyl-carbamate

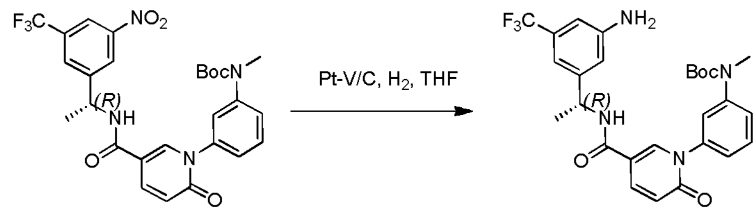


A mixture of tert-butyl N-methyl-N-[3-[5-[[(1R)-1-[3-nitro-5-(trifluoromethyl)phenyl]ethyl]carbamoyl]-2-oxo-1-pyridyl]phenyl]carbamate (100 mg, 178 μmol) and Pt-V/C (100 mg, 5% purity) in THF (1 mL) was stirred at 25 °C under $H_2$ (15 psi) for 1 hour. The mixture was filtrated, and the filtrate was concentrated in vacuo to give tert-butyl N-[3-[5-[[(1R)-1-[3-amino-5-(trifluoromethyl)phenyl]ethyl]carbamoyl]-2-oxo-1-pyridyl]phenyl]-N-methyl-carbamate (90 mg, 95% yield) as a colorless solid. MS (EI) m/z: 553.2 $[M+Na]^+$.

<u>Step 5: Synthesis of N-[(1R)-1-[3-amino-5-(trifluoromethyl)phenyl]ethyl]-1-[3-(methylamino)phenyl]-6-oxo-pyridine-3-carboxamide</u>

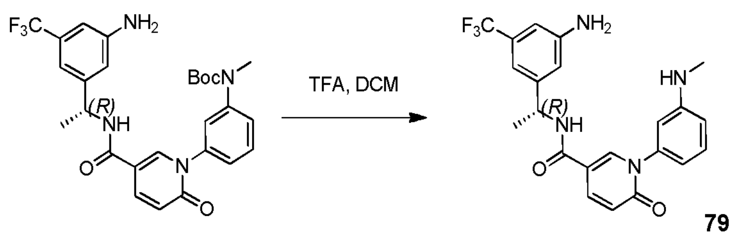


**79**

To a mixture of tert-butyl N-[3-[5-[[(1R)-1-[3-amino-5-(trifluoromethyl)phenyl]ethyl]carbamoyl]-2-oxo-1-pyridyl]phenyl]-N-methyl-carbamate (50 mg, 94.2 μmol) in TFA (0.3 mL) was added DCM (1 mL) at 0 °C, followed by stirring at 0 °C for 1 hour. The mixture was purified by Prep-HPLC (column: Phenomenex luna C18 150*25mm*10μm, mobile phase: [water(FA)-ACN];B%: 24%-54%,10min) to give the compound of Example 79 (13.9 mg, 33% yield) as a white solid. $^1H$ NMR (400 MHz, DMSO-$d_6$) δ = 8.58 (d, *J* = 7.6 Hz, 1H), 8.35 (d, *J* = 2.4 Hz, 1H), 7.94 (dd, *J* = 2.4, 9.6 Hz, 1H), 7.23 (t, *J* = 8.0 Hz, 1H), 6.77 (s, 2H), 6.70 (s, 1H), 6.65 (dd, *J* = 1.6, 8.4 Hz, 1H), 6.57 - 6.48 (m, 3H), 6.16 - 5.37 (m, 2H), 5.01 (t, *J* = 7.2 Hz, 1H), 2.70 (s, 3H), 1.40 (d, *J*= 7.2 Hz, 3H). MS (EI) m/z: 431.1 $[M+H]^+$.

**Example 80: N-[(1R)-1-[3-amino-5-(trifluoromethyl)phenyl]ethyl]-1-(3-methanesulfonamidophenyl)-6-oxo-1,6-dihydropyridine-3-carboxamide**

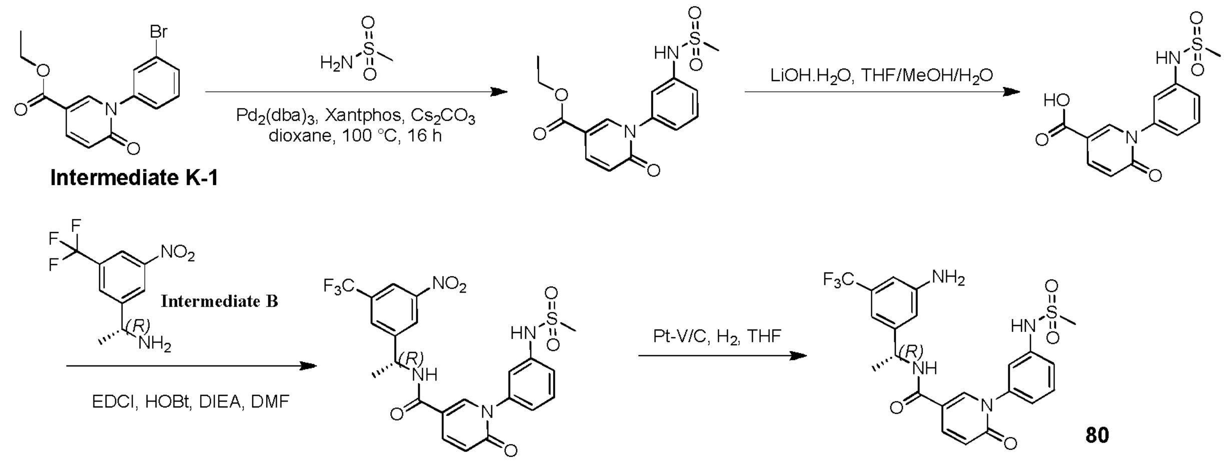


Intermediate K-1

**80**

The compound of Example 80 (16.94 mg, 35 % yield) was obtained as a white solid in the same manner as in

Steps 1 to 4 of Example 79, except that methanesulfonamide was used instead of tert-butyl N-methylcarbamate in Step 1. $^1$H NMR (400 MHz, DMSO-$d_6$) δ = 10.16 - 9.90 (m, 1H), 8.58 (d, *J* = 7.6 Hz, 1H), 8.35 (d, *J* = 2.4 Hz, 1H), 7.97 (dd, *J* = 2.8, 9.6 Hz, 1H), 7.58 - 7.45 (m, 1H), 7.30 (d, *J* = 8.0 Hz, 1H), 7.24 (t, *J* = 2.0 Hz, 1H), 7.19 (d, *J* = 8.8 Hz, 1H), 6.76 (s, 2H), 6.69 (s, 1H), 6.53 (d, *J* = 9.6 Hz, 1H), 5.54 (s, 2H), 5.01 (t, *J* = 7.2 Hz, 1H), 3.07 (s, 3H), 1.40 (d, *J* = 7.2 Hz, 3H); MS (EI) m/z: 495.1 $[M+H]^+$.

**Example 81 to Example 88**

The compounds shown in the following table were prepared in the same manner as in Steps 1 to 4 of Example 79, with replacing tert-butyl N-methylcarbamate with secondary amines in Step 1 of Example 79.

[Table 5]

| No. | Structure/Name | Spectral Data |
|---|---|---|
| 81 | 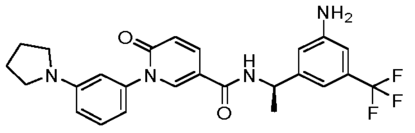 N-[(1R)-1-[3-amino-5-(trifluoromethyl)phenyl]ethyl]-6-oxo-1-[3-(pyrrolidin-1-yl)phenyl]-1,6-dihydropyridine-3-carboxamide | $^1$H NMR (400 MHz, $CDCl_3$) δ = 8.09 (d, J = 2.4 Hz, 1H), 7.68 (dd, J = 2.8, 9.6 Hz, 1H), 7.30 (t, J = 8.0 Hz, 1H), 6.95 (s, 1H), 6.81 (d, J = 3.6 Hz, 2H), 6.71 - 6.54 (m, 3H), 6.46 (d, J = 2.0 Hz, 1H), 6.09 (d, J = 7.2 Hz, 1H), 5.21 (t, J = 7.2 Hz, 1H), 3.29 (t, J = 6.4 Hz, 4H), 2.05 - 1.98 (m, 4H), 1.55 (d, J = 7.2 Hz, 3H); LC/MS (ESI) m/z = 471.2 $[M+H]^+$ |
| 82 | 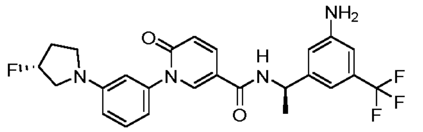 N-[(1R)-1-[3-amino-5-(trifluoromethyl)phenyl]ethyl]-1-{3-[(3R)-3-fluoropyrrolidin-1-yl]phenyl}-6-oxo-1,6-dihydropyridine-3-carboxamide | $^1$H NMR (400 MHz, $CDCl_3$) δ = 8.11 - 8.03 (m, 1H), 7.68 (dd, J = 2.8, 9.6 Hz, 1H), 7.33 (t, J = 8.0 Hz, 1H), 7.00 - 6.91 (m, 2H), 6.81 (s, 1H), 6.69 - 6.58 (m, 3H), 6.48 (s, 1H), 6.24 - 6.12 (m, 1H), 5.49 - 5.36 (m, 1H), 5.34 - 5.16 (m, 2H), 3.89 (t, J = 6.8 Hz, 1H), 3.62 - 3.45 (m, 4H), 2.44 - 2.19 (m, 2H), 1.55 (d, J = 6.8 Hz, 3H; LC/MS (ESI) m/z = 489.2 $[M+H]^+$ |
| 83 | 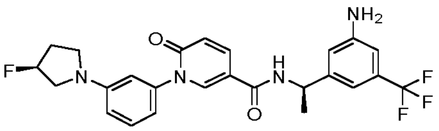 N-[(1R)-1-[3-amino-5-(trifluoromethyl)phenyl]ethyl]-1-{3-[(3S)-3-fluoropyrrolidin-1-yl]phenyl}-6-oxo-1,6-dihydropyridine-3-carboxamide | $^1$H NMR (400 MHz, $CDCl_3$) δ = 8.08 (d, J = 2.4 Hz, 1H), 7.68 (dd, J = 2.8, 9.6 Hz, 1H), 7.34 (t, J = 8.0 Hz, 1H), 6.95 (s, 1H), 6.84-6.78 (m, 2H), 6.72 - 6.58 (m, 3H), 6.48 (s, 1H), 6.08 (d, J = 7.2 Hz, 1H), 5.47 - 5.29 (m, 1H), 5.22 - 5.18 (m, 1H), 3.89 (br s, 2H), 3.66 - 3.40 (m, 4H), 2.45 - 2.32 (m, 1H), 2.28 - 2.06 (m, 1H), 1.57 - 1.49 (m, 3H); LC/MS (ESI) m/z = 489.1 $[M+H]^+$ |
| 84 | 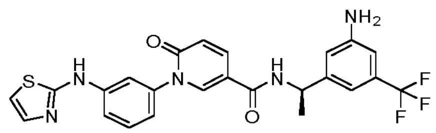 N-[(1R)-1-[3-amino-5-(trifluoromethyl)phenyl]ethyl]-6-oxo-1-{3-[(1,3-thiazol-2-yl)amino]phenyl}-1,6-dihydropyridine-3-carboxamide | $^1$H NMR (400 MHz, DMSO-$d_6$) δ = 10.46 (s, 1H), 8.59 (d, J = 7.6 Hz, 1H), 8.38 (d, J = 2.4 Hz, 1H), 7.97 (dd, J = 2.4, 9.6 Hz, 1H), 7.87 (s, 1H), 7.61 (dd, J = 1.2, 8.0 Hz, 1H), 7.47 (t, J = 8.0 Hz, 1H), 7.27 (d, J = 3.6 Hz, 1H), 7.00 (dd, J = 1.2, 7.6 Hz, 1H), 6.96 (d, J = 3.6 Hz, 1H), 6.75 (s, 2H), 6.69 (s, 1H), 6.54 (d, J = 9.6 Hz, 1H), 5.54 (d, J = 7.2 Hz, 1H), 5.01 (t, J = 7.2 Hz, 1H), 1.39 (d, J = 7.2 Hz, 3H); LC/MS (ESI) m/z = 500.1 $[M+H]^+$ |

(continued)

| No. | Structure/Name | Spectral Data |
|---|---|---|
| 85 | 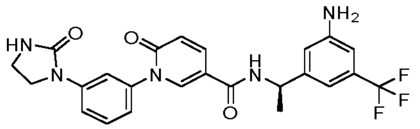 N-[(1R)-1-[3-amino-5-(trifluoromethyl)phenyl]ethyl]-6-oxo-1-[3-(2-oxoimidazolidin-1-yl)phenyl]-1,6-dihydropyridine-3-carboxamide | $^1$H NMR (400 MHz, DMSO-$d_6$) δ = 8.58 (d, J = 7.8 Hz, 1H), 8.35 (d, J = 2.4 Hz, 1H), 7.96 (dd, J = 2.4, 9.6 Hz, 1H), 7.72 (t, J = 2.0 Hz, 1H), 7.62 - 7.55 (m, 1H), 7.47 (t, J = 8.0 Hz, 1H), 7.12 (s, 1H), 7.06 (d, J = 7.6 Hz, 1H), 6.75 (s, 2H), 6.69 (s, 1H), 6.52 (d, J = 9.6 Hz, 1H), 5.53 (d, J = 7.2 Hz, 1H), 5.05 - 4.97 (m, 1H), 3.93 - 3.79 (m, 2H), 3.42 (t, J = 8.0 Hz, 2H), 1.39 (d, J = 7.2 Hz, 3H); LC/MS (ESI) m/z = 486.2 $[M+H]^+$ |
| 86 | 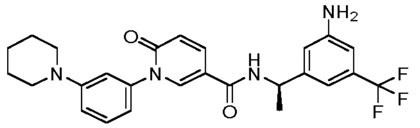 N-[(1R)-1-[3-amino-5-(trifluoromethyl)phenyl]ethyl]-6-oxo-1-[3-(piperidin-1-yl)phenyl]-1,6-dihydropyridine-3-carboxamide | $^1$H NMR (400 MHz, DMSO-$d_6$) δ = 8.56 (d, J = 8.0 Hz, 1H), 8.35 (d, J = 2.4 Hz, 1H), 7.93 (dd, J = 2.4, 9.6 Hz, 1H), 7.33 (t, J = 8.0 Hz, 1H), 7.02 (dd, J = 2.4, 8.4 Hz, 1H), 6.95 (t, J = 2.0 Hz, 1H), 6.75 (d, J = 2.4 Hz, 3H), 6.69 (s, 1H), 6.50 (d, J = 9.6 Hz, 1H), 5.54 (d, J = 7.2 Hz, 1H), 5.00 (J = 7.2 Hz, 1H), 3.26 - 3.14 (m, 4H), 1.66 - 1.51 (m, 6H), 1.39 (d, J = 7.2 Hz, 3H); LC/MS (ESI) m/z = 485.3 $[M+H]^+$ |
| 87 | 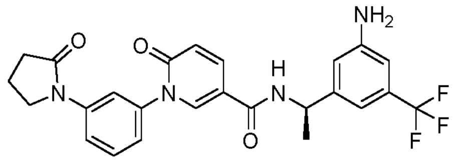 N-[(1R)-1-[3-amino-5-(trifluoromethyl)phenyl]ethyl]-6-oxo-1-[3-(2-oxopyrrolidin-1-yl)phenyl]pyridine-3-carboxamide | $^1$H NMR (400 MHz, DMSO-$d_6$) δ = 8.58 (d, J = 7.6 Hz, 1H), 8.35 (d, J = 2.8 Hz, 1H), 8.06 - 7.90 (m, 1H), 7.90 - 7.70 (m, 1H), 7.78 - 7.70 (m, 1H), 7.60 - 7.50 (m, 1H), 7.26 - 7.20 (m, 1H), 6.76 (s, 2H), 6.70 (s, 1H), 6.58 - 6.50 (m, 1H), 5.85 - 5.24 (m, 1H), 7.60 - 5.46 (m, 1H), 7.94 - 3.80 (m, 2H), 3.47 - 3.43 (m, 2H), 2.16 - 2.00 (m, 2H), 1.39 (d, J = 7.6 Hz, 3H); LC/MS (ESI) m/z = 485.1 $[M+H]^+$ |
| 88 | 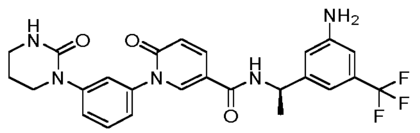 N-[(1R)-1-[3-amino-5-(trifluoromethyl)phenyl]ethyl]-6-oxo-1-[3-(2-oxohexahydropyrimidin-1-yl)phenyl] pyridine-3-carboxamide | $^1$H NMR (400 MHz, DMSO-$d_6$) δ = 8.61 (d, J = 7.6 Hz, 1H), 8.36 (d, J = 2.8 Hz, 1H), 8.00- 7.90 (m, 1H), 7.50 - 7.40 (m, 3H), 7.28 - 7.10 (m, 1H), 6.76 (s, 2H), 6.80 - 6.60 (m, 2H), 6.53 (d, J = 9.6 Hz, 1H), 5.59 - 5.49 (m, 2H), 5.06 - 4.95 (m, 1H), 3.76 - 3.60 (m, 2H), 3.25 - 3.19 (m, 2H), 2.00 - 1.93 (m, 2H), 1.39 (d, J = 7.2 Hz, 3H); LC/MS (ESI) m/z = 500.1 $[M+H]^+$ |

**Example 89: N-[(1R)-1-[3-amino-5-(trifluoromethyl)phenyl]ethyl]-1-[3-(3-methyl-2-oxo-imidazolidin-1-yl)phenyl]-6-oxo-pyridine-3-carboxamide**

Step 1: Synthesis of ethyl 1-[3-(3-methyl-2-oxo-imidazolidin-1-yl)phenyl]-6-oxo-pyridine-3-carboxylate

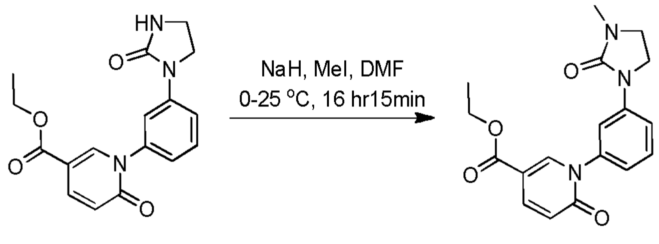


A mixture of ethyl 6-oxo-1-[3-(2-oxoimidazolidin-1-yl)phenyl]pyridine-3-carboxylate (200 mg, 611 μmol, which was synthesized in the same manner as in Step 1 of Example 79, in DMF (4 mL) was added NaH (36.6 mg, 916 μmol,

60% purity) at 0 °C, and the mixture was stirred at 0 °C for 15 min. Then, the mixture was added with MeI (130 mg, 916 μmol) and stirred at 25 °C for 16 hours. The mixture was quenched with ice water (10 mL) and extracted with EA (3 × 10 mL). The combined organic layer was dried over $Na_2SO_4$ and filtered, and the filtrate was concentrated in vacuo. The residue was purified by reversed-phase HPLC (0.1% FA condition) to give ethyl 1-[3-(3-methyl-2-oxo-imidazolidin-1-yl)phenyl]-6-oxo-pyridine-3-carboxylate (150 mg, 71% yield) as a yellow solid. MS (EI) m/z: 342.1 $[M+H]^+$.

Steps 2 to 4: Synthesis of N-[(1R)-1-[3-amino-5-(trifluoromethyl)phenyl]ethyl]-1-[3-(3-methyl-2-oxo-imidazolidin-1-yl)phenyl]-6-oxo-pyridine-3-carboxamide

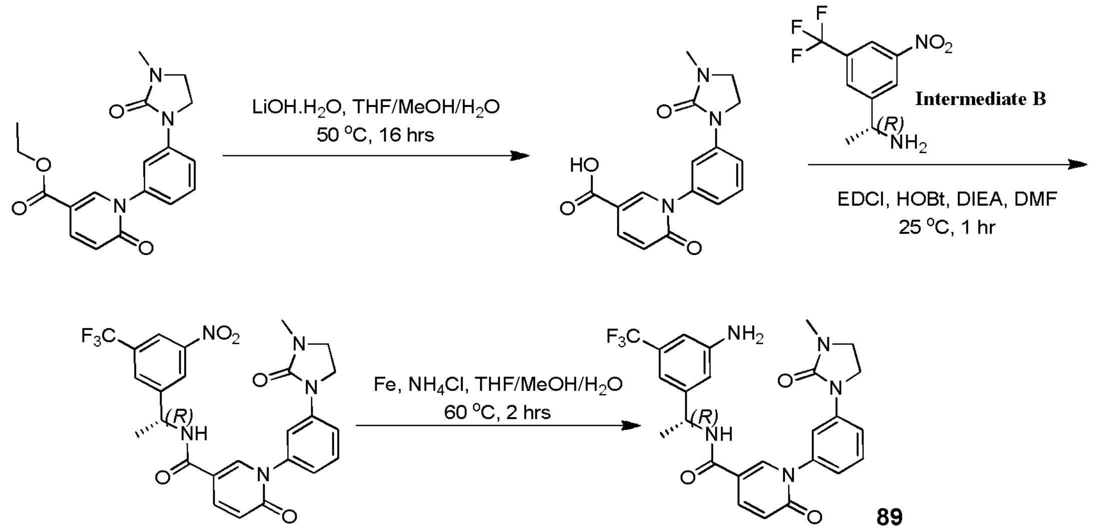


**89**

The compound of Example 89 (5.97 mg, 41% yield) was obtained in the same manner as in Steps 2 to 4 of Example 79, except that the reagents for the reduction reaction were changed in Step 4. $^1H$ NMR (400 MHz, DMSO-$d_6$) δ = 8.59 (d, *J* = 7.6 Hz, 1H), 8.36 (d, *J* = 2.4 Hz, 1H), 7.96 (dd, *J* = 2.8, 9.6 Hz, 1H), 7.70 (t, *J* = 2.0 Hz, 1H), 7.63 (dd, *J* = 1.6, 8.4 Hz, 1H), 7.48 (t, *J* = 8.0 Hz, 1H), 7.07 (dd, *J* = 1.2, 7.6 Hz, 1H), 6.76 (s, 2H), 6.69 (s, 1H), 6.53 (d, *J* = 9.6 Hz, 1H), 5.54 (d, *J* = 7.2 Hz, 1H), 5.07 - 4.94 (m, 1H), 3.86 - 3.75 (m, 2H), 3.46 (t, *J* = 8.0 Hz, 2H), 2.77 (s, 3H), 1.39 (d, *J* = 7.2 Hz, 3H); MS (EI) m/z: 500.2 $[M+H]^+$.

**Example 90 and Example 91: N-[(1R)-1-[3-amino-5-(trifluoromethyl)phenyl]ethyl]-6-oxo-1-{3-[((2S)-1,1,1-trifluoropropan-2-yl)amino]phenyl}-1,6-dihydropyridine-3-carboxamide and N-[(1R)-1-[3-amino-5-(trifluoromethyl)phenyl]ethyl]-6-oxo-1-{3-[((2R)-1,1,1-trifluoropropan-2-yl)amino]phenyl}-1,6-dihydropyridine-3-carboxamide**

Step 1: Synthesis of ethyl 6-oxo-1-[3-[(1,1,1-trifluoropropan-2-yl)amino]phenyl]dihydropyridine-3-carboxylate

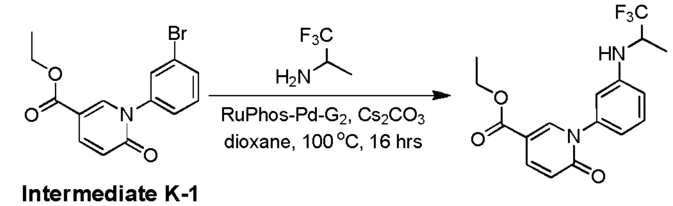


**Intermediate K-1**

A mixture of 1,1,1-trifluoropropan-2-amine (510 mg, 3.41 mmol, HCl), Intermediate EA (1.0 g, 3.10 mmol), RuPhos Pd $G_2$ (241 mg, 310 μmol) and $Cs_2CO_3$ (5.06 g, 15.5 mmol) in dioxane (5 mL) was stirred at 100 °C for 16 hours. The mixture was added with water (20 mL) and extracted with EA (3 X15 mL). The combined organic layer was dried over $Na_2SO_4$. The residue was purified by reversed-phase HPLC (0.1% FA condition) to give ethyl 6-oxo-1-[3-[(1,1,1-trifluoropropan-2-yl)amino]phenyl]dihydropyridine-3-carboxylate (800 mg, 72% yield) as yellow oil. MS (EI)

m/z: 355.2 [M+H]+.

Steps 2 and 3: Synthesis of N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide

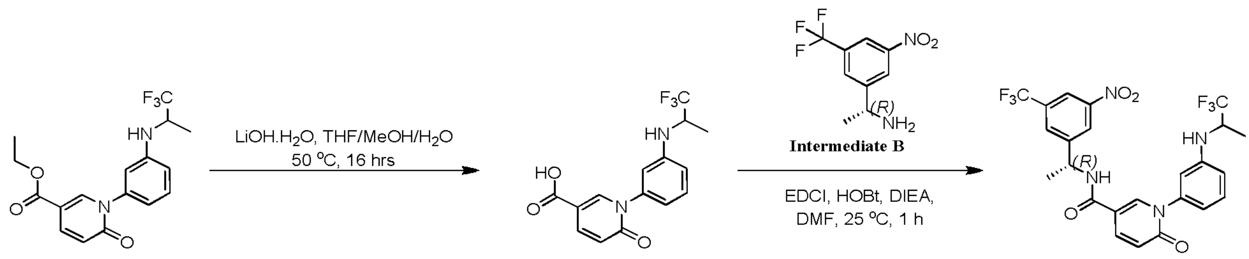


N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide (140 mg, 55 % yield) was obtained in the same manner as in Example 76. MS (EI) m/z: 543.4 [M+H]+.

Step 4: Separation of N-[(1R)-1-[3-nitro-5-(trifluoromethyl)phenyl]ethyl]-6-oxo-1-[3-[[(2S)-1,1,1-trifluoropropan-2-yl]amino]phenyl]dihydropyridine-3-carboxamide and N-[(1R)-1-[3-nitro-5-(trifluoromethyl)phenyl]ethyl]-6-oxo-1-[3-[[(2R)-1,1,1-trifluoropropan-2-yl]amino]phenyl]dihydropyridine-3-carboxamide

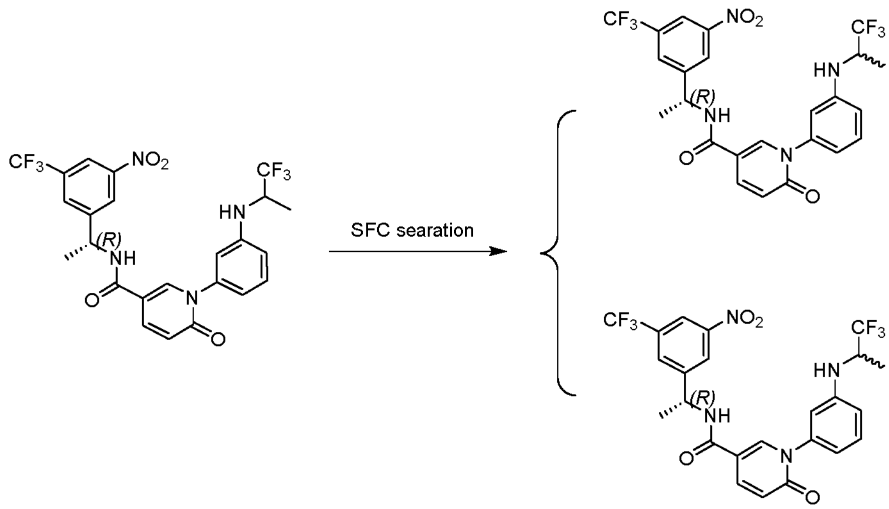


N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide (140 mg) was purified by Prep-HPLC (column: DAICEL CHIRALCEL OJ-H(250mm*30mm, 5μm); mobile phase: [0.1% $NH_3H_2O$ IPA]; B%: 25%-25%, 2.65min) to give one of the diastereomers, N-[(1R)-1-[3-nitro-5-(trifluoromethyl)phenyl]ethyl]-6-oxo-1-[3-[[(2S)-1,1,1-trifluoropropan-2-yl]amino]phenyl]dihydropyridine-3-carboxamide (30 mg) as a yellow solid.

N-[(1R)-1-[3-nitro-5-(trifluoromethyl)phenyl]ethyl]-6-oxo-1-[3-[(1,1,1-trifluoropropan-2-yl)amino]phenyl]dihydropyridine-3-carboxamide (140 mg) was purified by Prep-HPLC (column: DAICEL CHIRALCEL OJ-H(250mm*30mm, 5μm); mobile phase: [0.1% $NH_3H_2O$ IPA]; B%: 25%-25%, 2.65min) to give the other of the diastereomers, N-[(1R)-1-[3-nitro-5-(trifluoromethyl)phenyl]ethyl]-6-oxo-1-[3-[[(2R)-1,1,1-trifluoropropan-2-yl]amino]phenyl]dihydropyridine-3-carboxamide (30 mg) as a yellow solid.

Step 5: Synthesis of N-[(1R)-1-[3-amino-5-(trifluoromethyl)phenyl]ethyl]-6-oxo-1-{3-[((2S)-1,1,1-trifluoropropan-2-yl)amino]phenyl}-1,6-dihydropyridine-3-carboxamide and N-[(1R)-1-[3-amino-5-(trifluoromethyl)phenyl]ethyl]-6-oxo-1-{3-[((2R)-1,1,1-trifluoropropan-2-yl)amino]phenyl}-1,6-dihydropyridine-3-carboxamide

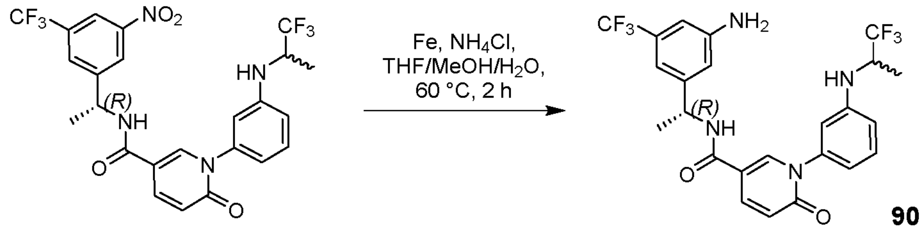


90

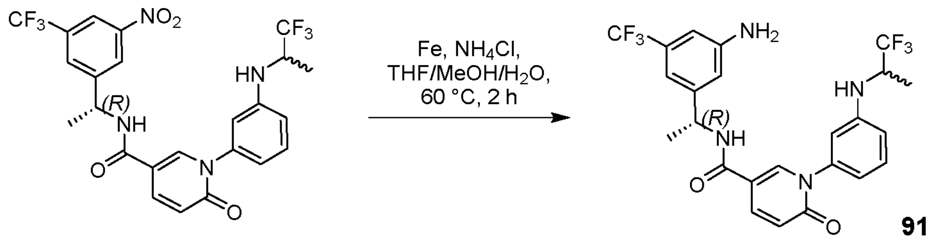


91

The compound of Example 90 (5.64 mg, 23% yield) and the compound of Example 91 (5.47 mg, 23% yield) were obtained by reacting each of the two diastereomers in the same manner as in Step 4 of Example 89.

$^1H$ NMR (400 MHz, DMSO-$d_6$) $\delta$ = 8.63 - 8.51 (m, 1H), 8.35 (s, 1H), 7.95 (d, *J* = 8.8 Hz, 1H), 7.32 - 7.18 (m, 1H), 6.87 - 6.83 (m, 1H), 6.77 (s, 3H), 6.70 (s, 1H), 6.67 - 6.61 (m, 1H), 6.55 - 6.46 (m, 1H), 6.37 - 6.27 (m, 1H), 5.55 (d, *J* = 7.6 Hz, 1H), 5.08 - 4.94 (m, 1H), 4.50 - 4.35 (m, 1H), 1.39 (d, *J* = 6.8 Hz, 3H), 1.31 (d, *J* = 6.4 Hz, 3H); MS (EI) m/z: 513.2 $[M+H]^+$.

$^1H$ NMR (400 MHz, DMSO-$d_6$) $\delta$ = 8.63 - 8.50 (m, 1H), 8.34 (d, *J* = 0.8 Hz, 1H), 7.98 - 7.89 (m, 1H), 7.30 - 7.21 (m, 1H), 6.84 (d, *J* = 7.2 Hz, 1H), 6.79 - 6.73 (m, 3H), 6.69 (s, 1H), 6.64 (d, *J* = 7.2 Hz, 1H), 6.50 (d, *J* = 9.6 Hz, 1H), 6.32 (d, *J* = 8.8 Hz, 1H), 5.54 (d, *J* = 6.4 Hz, 2H), 5.00 (t, *J* = 6.4 Hz, 1H), 4.43 (d, *J* = 7.6 Hz, 1H), 1.39 (d, *J* = 7.2 Hz, 3H), 1.30 (d, *J* = 6.4 Hz, 3H); MS (EI) m/z: 513.2 $[M+H]^+$.

**Example 92: Methyl N-[3-[5-[[(1R)-1-[3-amino-5-(trifluoromethyl)phenyl]ethyl]carbamoyl]-2-oxo-1-pyridyl]phenyl]carbamate**

Step 1: Synthesis of ethyl 1-[3-(tert-butoxycarbonylamino)phenyl]-6-oxo-pyridine-3-carboxylate

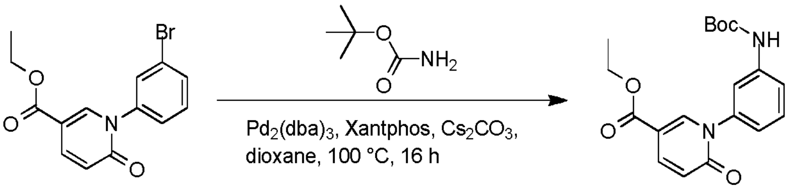


**Intermediate K-1**

Ethyl 1-[3-(tert-butoxycarbonylamino)phenyl]-6-oxo-pyridine-3-carboxylate (200 mg, 35 % yield) was obtained as a yellow solid by using tert-butyl N-carbamate in the same manner as in Step 1 of Example 79. MS (EI) m/z: 359.0 $[M+H]^+$.

Step 2: Synthesis of ethyl 1-(3-aminophenyl)-6-oxo-pyridine-3-carboxylate

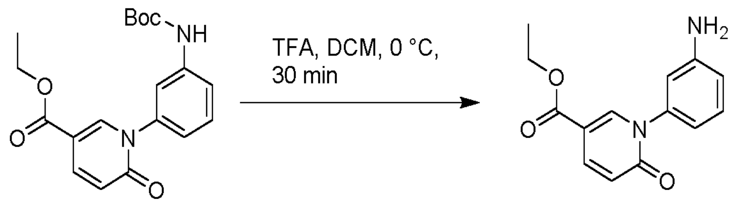


A mixture of ethyl 1-[3-(tert-butoxycarbonylamino)phenyl]-6-oxo-pyridine-3-carboxylate (800 mg, 1.34 mmol) in DCM (20 mL) was added TFA (600 μL) at 0 °C, and the mixture was stirred at 0 °C for 30 min. The mixture was concentrated in vacuo. The residue was purified by reversed-phase HPLC (0.1% FA condition) to give ethyl 1-(3-aminophenyl)-6-oxo-pyridine-3-carboxylate (320 mg, 92% yield) as yellow oil. $^1H$ NMR (400 MHz, DMSO-$d_6$) δ =8.15 (s, 1H), 7.87 - 7.83 (m, 1H), 7.14 (t, *J* = 8.0 Hz, 1H), 6.66 - 6.58 (m, 1H), 6.54 - 6.49 (m, 3H), 5.42 - 5.38 (m, 2H), 4.27 - 4.21 (m, 2H), 1.26 (t, *J* = 7.2 Hz, 3H).

Step 3: Synthesis of ethyl 1-[3-(methoxycarbonylamino)phenyl]-6-oxo-pyridine-3-carboxylate

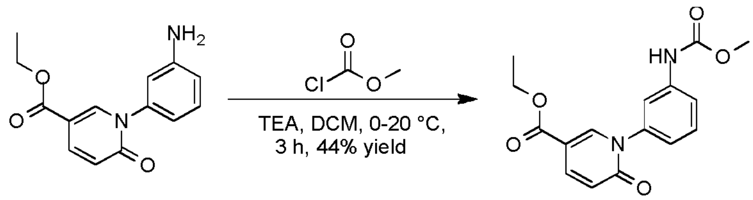


To a solution of ethyl 1-(3-aminophenyl)-6-oxo-pyridine-3-carboxylate (240 mg, 929 μmol) and TEA (470 mg, 4.65 mmol) in DCM (5 mL) was added methyl carbonochloridate (0.53 g, 5.61 mmol) at 0 °C. The reaction mixture was warmed to 20 °C and stirred for 3 hours. The reaction mixture was added to water (30 mL) and then adjusted to pH 9 with $Na_2CO_3$, and extracted with DCM (3 × 50 mL). The combined organic layer was concentrated in vacuo. The residue was purified by reversed-phase HPLC (0.1% FA condition) to give ethyl 1-[3-(methoxycarbonylamino)phenyl]-6-oxo-pyridine-3-carboxylate (130 mg, 44% yield) as a white solid. $^1H$ NMR (400 MHz, DMSO-$d_6$) δ = 9.92 (s, 1H), 8.22 (d, *J* = 2.4 Hz, 1H), 7.96 - 8.80 (m, 1H), 7.61 - 7.50 (m, 2H), 7.48 - 7.39 (m, 1H), 7.16 - 7.68 (m, 1H), 6.55 (d, *J* = 9.6 Hz, 1H), 4.28 - 4.20 (m, 2H), 3.68 (s, 3H),1.26 (t, *J*= 7.2 Hz, 3H).

Steps 4 to 6: Synthesis of methyl N-[3-[5-[[(lR)-1-[3-amino-5-(trifluoromethyl)phenyl]ethyl]carbamoyl]-2-oxo-1-pyridyl]phenyl]carbamate

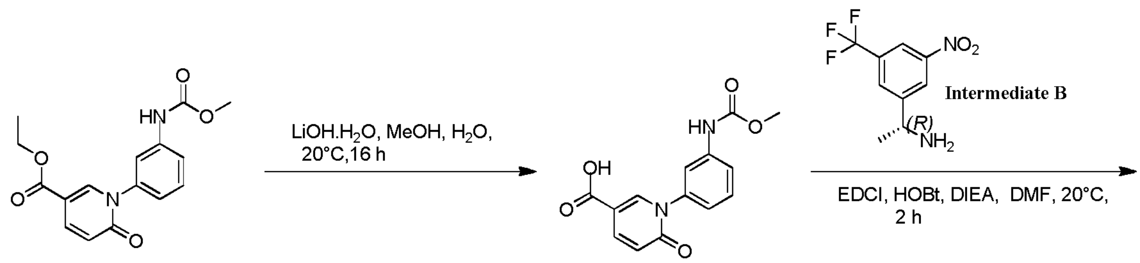


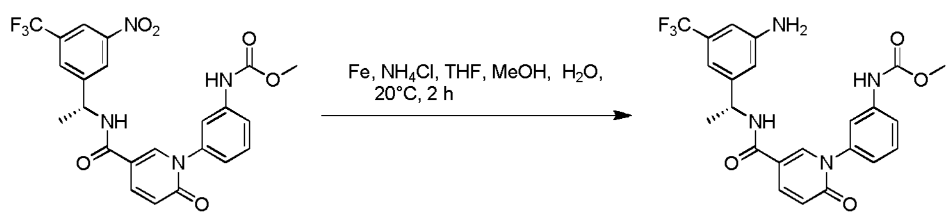


92

The compound of Example 92 (23.0 mg, 39% yield) was obtained in the same manner as in Steps 2 to 4 of Example 89 as a white solid. $^1$H NMR (400 MHz, DMSO-$d_6$) δ = 9.92 (s, 1H), 8.59 (d, *J* = 7.6 Hz, 1H), 8.39 - 8.31 (m, 1H), 8.03 - 7.89 (m, 1H), 7.59 - 7.44 (m, 3H), 7.15 - 7.04 (m, 1H), 6.80 - 6.75 (m, 2H), 6.69 (s, 1H), 5.59 - 5.51 (m, 2H), 5.11 - 4.92 (m, 1H), 3.71 - 3.65 (m, 3H), 1.39 (d, *J* = 7.6 Hz, 3H); MS (EI) m/z: 475.1 $[M+H]^+$.

**Example 93: N-[(1R)-1-(3-amino-5-trifluoromethyl)phenyl]ethyl]-6-oxo-1-[3-(2-oxo-1H-imidazol-3-yl)phenyl] pyridine-3-carboxamide**

Step 1: Synthesis of ethyl 1-[3-(2,2-diethoxyethylcarbonylamino)phenyl]-6-oxo-pyridine-3-carboxyl(2-fluorophenyl)-6-oxo-1,6-dihydropyridazine-3-carboxylate

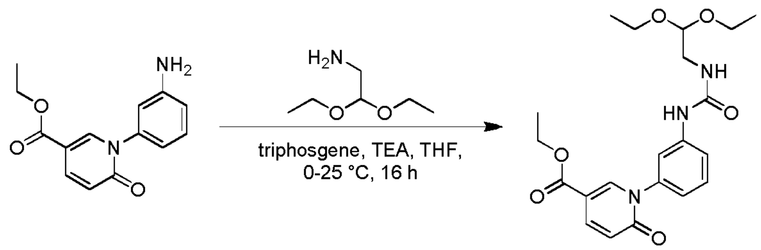


To a mixture of ethyl 1-(3-aminophenyl)-6-oxo-pyridine-3-carboxylate (50 mg, 193 μmol) obtained from Step 2 of Example 92 and TEA (58.7 mg, 580 μmol) in THF (2 mL) was added triphosgene (11.4 mg, 38.7 μmol ) at 0 °C for 30 min, and 2,2-diethoxyethanamine (30.9 mg, 232 μmol) and TEA (58.7 mg, 580 μmol) were subsequently added thereto. The reaction mixture was stirred at 25 °C for 16 hours. The reaction mixture was quenched with 1N $K_2CO_3$ aqueous solution (5 mL), adjusted to pH 9 and extracted with EtOAc (10 mL X 2). The combined organic layer was dried over anhydrous $Na_2SO_4$ and concentrated under reduced pressure to give ethyl 1-[3-(2,2-diethoxyethylcarbonylamino)phenyl]-6-oxo-pyridine-3-carboxyl(2-fluorophenyl)-6-oxo-1,6-dihydropyridazine-3-carboxylate (80 mg, crude) as a yellow solid. $^1$H NMR (400 MHz, DMSO-$d_6$) δ = 8.88 (s, 1H), 8.20 (d, *J* = 2.4 Hz, 1H), 7.80 - 7.96 (m, 1H), 7.59 (s, 1H), 7.39 - 7.35 (m, 2H), 6.90 - 7.04 (m, 1H), 6.55 (d, *J* = 9.6 Hz, 1H), 6.20 - 7.24 (m, 1H), 4.40 - 4.58 (m, 1H), 4.26 - 4.22 (m, 2H), 3.65 - 3.59 (m, 2H), 3.40 - 3.58 (m, 2H), 3.10 - 3.26 (m, 2H), 1.29 - 1.24 (m, 3H), 1.15 - 1.11 (m, 6H).

Step 2: Synthesis of ethyl 6-oxo-1-[3-(2-oxo-1H-imidazol-3-yl)phenyl]pyridine-3-carboxylate

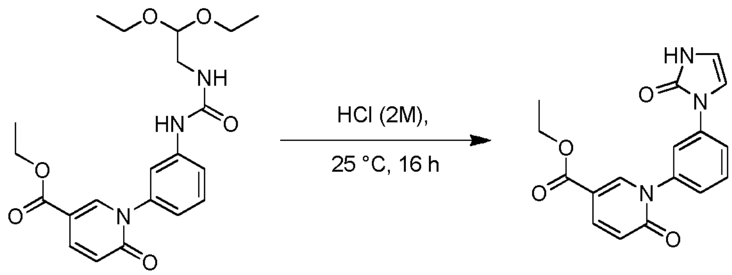


Ethyl 1-[3-(2,2-diethoxyethylcarbonylamino)phenyl]-6-oxo-pyridine-3-carboxyl(2-fluorophenyl)-6-oxo-1,6-dihydropyridazine-3-carboxylate (80 mg, 191 μmol) in 2M HCl (2 mL) was stirred at 25°C for 16 hours. The reaction mixture was diluted with water (8 mL), adjusted to pH 9 with $Na_2CO_3$ aqueous solution and then extracted with EtOAc (10 mL X 3). The combined organic layer was dried over anhydrous $Na_2SO_4$ and concentrated under reduced pressure to give ethyl 6-oxo-1-[3-(2-oxo-1H-imidazol-3-yl)phenyl]pyridine-3-carboxylate (52 mg, 83 % yield) as a yellow solid. 1H NMR (400 MHz, DMSO-$d_6$) δ = 8.29 (d, J = 2.4 Hz, 1H), 7.93 - 7.88 (m, 3H), 7.63 - 7.52 (m, 2H), 7.29 (s, 1H), 7.07 (d, J = 3.2 Hz, 1H), 6.64 (d, J = 3.2 Hz, 1H), 6.57 (d, J = 9.6 Hz, 1H), 4.25 (d, J = 7.2 Hz, 2H), 1.27 - 1.25 (m, 3H).

Steps 3 and 4: Synthesis of N-[(1R)-1-(3-amino-5-trifluoromethyl)phenyl]ethyl]-6-oxo-1-[3-(2-oxo-1H-imidazol-3-yl)phenyl]pyridine-3-carboxamide

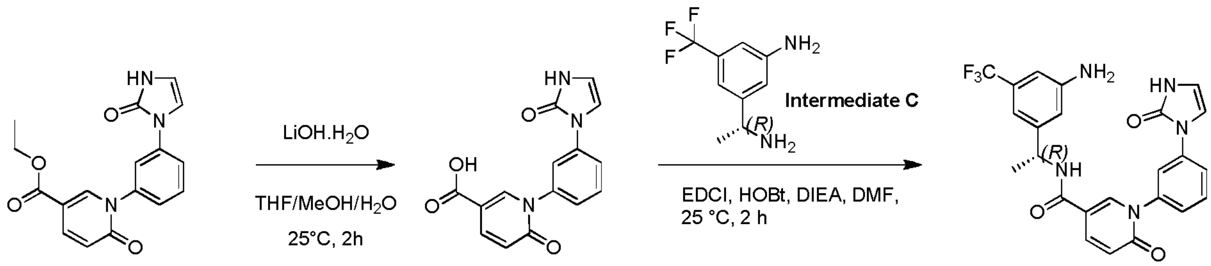


93

The compound of Example 93 (4.99 mg, 28% yield) was obtained in the same manner as in Example 76 as a white solid. $^1$H NMR (400 MHz, $CDCl_3$) δ = 8.35 (d, J = 2.2 Hz, 1H), 8.00 - 8.10 (m, 1H), 7.86 - 7.73 (m, 2H), 7.60 - 7.68 (m, 1H), 7.30 - 7.46 (m, 1H), 6.99 - 6.86 (m, 3H), 6.80 (s, 1H), 6.65 (d, J = 9.7 Hz, 1H), 6.58 (d, J = 2.8 Hz, 1H), 5.00 - 5.20 (m, 1H), 1.40 - 1.60(m, 3H). MS (EI) m/z: $[M+H]^+$ 484.1.

**Example 94: N-[(1R)-1-[3-amino-5-(trifluoromethyl)phenyl]ethyl]-1-(2,3-difluorophenyl)-6-oxo-1,6-dihydropyridine-3-carboxamide**

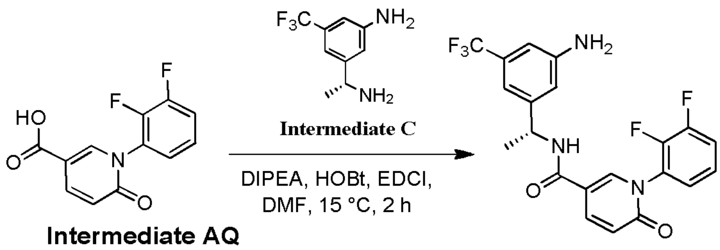


Intermediate AQ

94

A solution of Intermediate C (22 mg, 0.10 mmol), Intermediate AQ (30 mg, 0.12 mmol), HOBt (32.9 mg, 0.21 mmol), EDCI (32.1 mg, 0.16 mmol) and DIPEA (42 μL, 0.24 mmol) in DMF (1 mL) was prepared. The reaction mixture was stirred at room temperature for 2 hours. The mixture was added with water and sat. $NaHCO_3$ solution and extracted with EA. The organic layer was dried over $MgSO_4$. The solvent was removed under reduced pressure and the crude residue was purified by Prep/LC to give the compound of Example 94 (24.7 mg, 47.3% yield). $^1$H NMR (400 MHz, DMSO) δ 8.58 (d, J = 7.7 Hz, 1H), 8.38 (d, J = 2.6 Hz, 1H), 8.01 (dd, J = 9.7, 2.6 Hz, 1H), 7.66 (q, J = 7.9 Hz, 1H), 7.53 - 7.38 (m, 2H), 6.77 (s, 2H), 6.71 (s, 1H), 6.59 (d, J = 9.7 Hz, 1H), 5.01 (t, J = 7.2 Hz, 1H), 1.40 (d, J = 7.1 Hz, 3H); LC/MS (ESI) m/z = 438.1 $[M+H]^+$.

**Example 95 to Example 99**

The compounds shown in the following table were prepared in the same manner as in Example 94 by using starting materials corresponding to the respective structures of the desired compounds based on Preparation Example 43 or by replacing Intermediate C with Intermediate E.

[Table 6]

| No. | Structure/Name | Spectral Data |
|---|---|---|
| 95 | 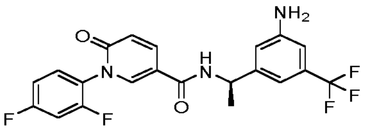 N-[(1R)-1-[3-amino-5-(trifluoromethyl)phenyl]ethyl]-1-(2,4-difluorophenyl)-6-oxo-1,6-dihydropyridine-3-carboxamide | $^1$H NMR (400 MHz, DMSO) δ 8.61 (d, J = 7.7 Hz, 1H), 8.36 (d, J = 2.6 Hz, 1H), 8.00 (dd, J = 9.7, 2.6 Hz, 1H), 7.71 (td, J = 8.8, 5.9 Hz, 1H), 7.57 (ddd, J = 11.5, 9.1, 2.8 Hz, 1H), 7.37 - 7.27 (m, 1H), 6.76 (d, J = 1.9 Hz, 2H), 6.70 (d, J = 2.0 Hz, 1H), 6.57 (d, J = 9.7 Hz, 1H), 5.57 (s, 2H), 5.00 (p, J = 7.1 Hz, 1H), 1.40 (d, J = 7.1 Hz, 3H); LC/MS (ESI) m/z = 438.1 [M+H]$^+$ |
| 96 | 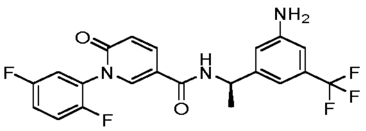 N-[(1R)-1-[3-amino-5-(trifluoromethyl)phenyl]ethyl]-1-(2,5-difluorophenyl)-6-oxo-1,6-dihydropyridine-3-carboxamide | $^1$H NMR (400 MHz, DMSO) δ 8.56 (d, J = 7.7 Hz, 1H), 8.37 (d, J = 2.6 Hz, 1H), 7.99 (dd, J = 9.7, 2.6 Hz, 1H), 7.68 (ddd, J = 8.7, 5.8, 3.1 Hz, 1H), 7.60 - 7.43 (m, 2H), 6.76 (d, J = 1.9 Hz, 2H), 6.70 (s, 1H), 6.58 (d, J = 9.7 Hz, 1H), 5.56 (s, 2H), 5.01 (t, J = 7.2 Hz, 1H), 1.40 (d, J = 7.0 Hz, 3H); LC/MS (ESI) m/z = 438.1 [M+H]$^+$ |
| 97 | 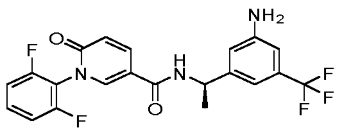 N-[(1R)-1-[3-amino-5-(trifluoromethyl)phenyl]ethyl]-1-(2,6-difluorophenyl)-6-oxo-1,6-dihydropyridine-3-carboxamide | $^1$H NMR (400 MHz, DMSO) δ 8.58 (d, J = 7.6 Hz, 1H), 8.42 (d, J = 2.6 Hz, 1H), 8.04 (dd, J = 9.7, 2.6 Hz, 1H), 7.73 - 7.61 (m, 1H), 7.41 (t, J = 8.6 Hz, 2H), 6.76 (d, J = 1.9 Hz, 2H), 6.70 (d, J = 1.9 Hz, 1H), 6.64 (d, J = 9.7 Hz, 1H), 5.57 (s, 2H), 5.00 (t, J = 7.2 Hz, 1H), 1.40 (d, J = 7.0 Hz, 3H); LC/MS (ESI) m/z =438.1 [M+H]$^+$ |
| 98 | 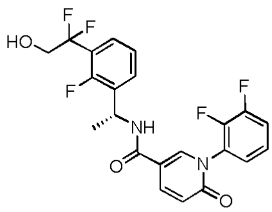 N-[(1R)-1-[3-(1,1-difluoro-2-hydroxyethyl)-2-fluorophenyl]ethyl]-1-(2,3-difluorophenyl)-6-oxo-1,6-dihydropyridine-3-carboxamide | $^1$H NMR (400 MHz, DMSO) δ 8.70 (d, J = 7.3 Hz, 1H), 8.43 (d, J = 2.6 Hz, 1H), 8.00 (dd, J = 9.6, 2.6 Hz, 1H), 7.70 - 7.62 (m, 1H), 7.58 (t, J = 7.3 Hz, 1H), 7.50 (t, J = 7.0 Hz, 1H), 7.44 (q, J = 7.5 Hz, 2H), 7.28 (t, J = 7.7 Hz, 1H), 6.59 (d, J = 9.7 Hz, 1H), 5.73 (t, J = 6.4 Hz, 1H), 5.35 (t, J = 7.1 Hz, 1H), 3.92 (td, J = 14.4, 6.4 Hz, 2H), 1.44 (d, J = 7.1 Hz, 3H); LC/MS (ESI) m/z = 453.2 [M+H]$^+$ |
| 99 | 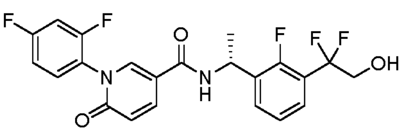 N-[(1R)-1-[3-(1,1-difluoro-2-hydroxyethyl)-2-fluorophenyl]ethyl]-1-(2,4-difluorophenyl)-6-oxo-1,6-dihydropyridine-3-carboxamide | $^1$H NMR (400 MHz, DMSO) δ 8.68 (d, J = 7.4 Hz, 1H), 8.40 (d, J = 2.6 Hz, 1H), 7.98 (dd, J = 9.7, 2.6 Hz, 1H), 7.78 - 7.68 (m, 1H), 7.58 (s, 2H), 7.43 (t, J = 7.4 Hz, 1H), 7.38 - 7.24 (m, 2H), 6.57 (d, J = 9.8 Hz, 1H), 5.73 (t, J = 6.4 Hz, 1H), 5.35 (t, J = 7.1 Hz, 1H), 3.92 (td, J = 14.5, 6.5 Hz, 2H), 1.43 (d, J = 7.0 Hz, 3H); LC/MS (ESI) m/z = 453.1 [M+H]$^+$ |

**Example 100: 1-(2-fluorophenyl)-N-[(1R)-1-{2'-[(methylamino)methyl]-[1,1'-biphenyl]-3-yl}ethyl]-6-oxo-1,6-dihydropyridine-3-carboxamide**

Step 1: Synthesis of (R)-N-(1-(3-bromophenyl)ethyl)-1-(2-fluorophenyl)-6-oxo-1,6-dihydropyridine-3-carboxamide

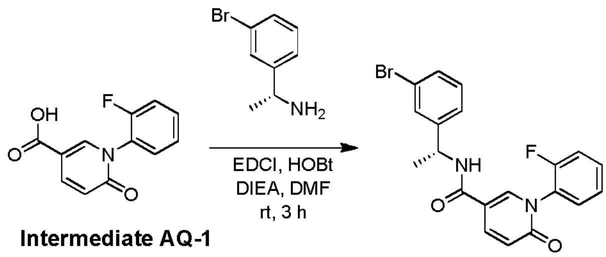


**Intermediate AQ-1**

(R)-N-(1-(3-bromophenyl)ethyl)-1-(2-fluorophenyl)-6-oxo-1,6-dihydropyridine-3-carboxamide (1.01g, 55.3% yield) was obtained in the same manner as in Step 2 of Example 76. LC/MS m/z = 415.1 $[M+H]^+$.

Step 2: Synthesis of 1-(2-fluorophenyl)-N-[(1R)-1-{2'-[(methylamino)methyl]-[1,1'-biphenyl]-3-yl}ethyl]-6-oxo-1,6-dihydropyridine-3-carboxamide

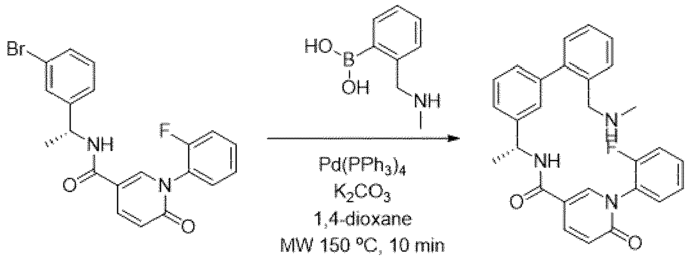


**100**

To a solution of (R)-N-(1-(3-bromophenyl)ethyl)-1-(2-fluorophenyl)-6-oxo-1,6-dihydropyridine-3-carboxamide (50 mg, 0.12 mmol) in 1,4-dioxane (2.5 mL) and distilled water (0.5 mL), (2-((methylamino)methyl)phenyl)boronic acid (23.8 mg, 0.14 mmol), $Pd(PPh_3)_4$ (13.9 mg, 0.012 mmol) and $K_2CO_3$ (49.6 mg, 0.36 mmol) were added. The reaction mixture was heated by microwave at 150 °C for 10 min. The mixture was cooled, added with water, and extracted with DCM. The organic layer was washed with brine, dried over $Na_2SO_4$ and concentrated under reduced pressure. The residue was purified by prep-HPLC to give the compound of Example 100 (27.8 mg, 50.7 % yield). $^1H$ NMR (500 MHz, MeOD) δ 8.27 (d, J = 2.6 Hz, 1H), 8.07 (dd, J = 9.6, 2.6 Hz, 1H), 7.57 (dd, J = 6.1, 2.9 Hz, 2H), 7.50 (dd, J = 5.9, 3.6 Hz, 4H), 7.47 (d, J = 6.6 Hz, 2H), 7.40 - 7.32 (m, 5H), 7.23 (dt, J = 6.8, 2.0 Hz, 1H), 6.65 (d, J = 9.6 Hz, 1H), 5.16 (q, J = 7.1 Hz, 1H), 4.18 (s, 2H), 3.98 (s, 1H), 2.53 (s, 3H), 1.57 (d, J = 7.1 Hz, 3H); LC/MS m/z = 456.2 $[M+H]^+$.

**Example 101 to Example 107**

The compounds shown in the following table were prepared in the same method as in Example 100 by using appropriate boronic acid derivatives in Step 2.

[Table 7]

| No. | Structure/Name | Spectral Data |
|---|---|---|
| 101 | 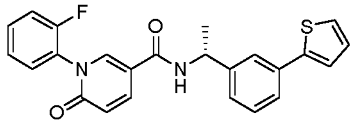 1-(2-fluorophenyl)-6-oxo-N-[(1R)-1-[3-(thiophen-2-yl)phenyl]ethyl] -1,6-dihydropyridine-3-carboxamide | $^1H$ NMR (500 MHz, MeOD) δ 8.24 (d, J = 2.6 Hz, 1H), 8.06 (dd, J = 9.7, 2.6 Hz, 1H), 7.62 (d, J = 1.8 Hz, 1H), 7.56 (tdd, J = 7.4, 5.0, 1.7 Hz, 1H), 7.52 - 7.45 (m, 2H), 7.38 - 7.35 (m, 3H), 7.34 (d, J = 2.1 Hz, 2H), 7.33 (d, J = 2.3 Hz, 1H), 7.31 - 7.27 (m, 1H), 7.07 (dd, J = 5.1, 3.6 Hz, 1H), 6.64 (d, J = 9.7 Hz, 1H), 5.19 (td, J = 7.7, 5.7 Hz, 1H), 1.55 (d, J = 7.2 Hz, 3H); LC/MS (ESI) m/z = 419.1 $[M+H]^+$ |

(continued)

| No. | Structure/Name | Spectral Data |
|---|---|---|
| 102 | 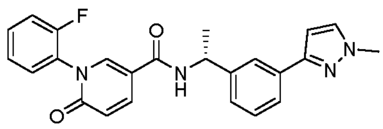 1-(2-fluorophenyl)-N-[(1R)-1-[3-(1-methyl-1H-pyrazol-3-yl)phenyl]ethyl]-6-oxo-1,6-dihydropyridine-3-carboxamide | $^1$H NMR (500 MHz, MeOD) δ 8.25 (d, J = 2.6 Hz, 1H), 8.06 (dd, J = 9.6, 2.6 Hz, 1H), 7.58 - 7.54 (m, 1H), 7.51 (dd, J = 5.0, 2.0 Hz, 1H), 7.50 - 7.46 (m, 4H), 7.37 (t, J = 7.7 Hz, 5H), 6.65 (d, J = 9.6 Hz, 1H), 6.38 (d, J = 2.0 Hz, 1H), 5.22 (d, J = 7.1 Hz, 1H), 3.85 (s, 3H), 1.57 (d, J = 7.1 Hz, 3H); LC/MS (ESI) m/z = 417.2 $[M+H]^+$ |
| 103 | 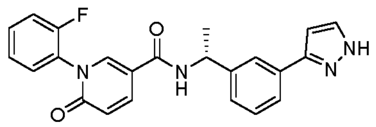 1-(2-fluorophenyl)-6-oxo-N-[(1R)-1-[3-(1H-pyrazol-3-yl)phenyl]ethyl]-1,6-dihydropyridine-3-carboxamide | $^1$H NMR (500 MHz, MeOD) δ 8.26 (d, J = 2.7 Hz, 1H), 8.11 - 8.03 (m, 1H), 7.78 (s, 1H), 7.74 (d, J = 1.9 Hz, 1H), 7.64 (dd, J = 7.4, 1.6 Hz, 1H), 7.60 - 7.53 (m, 1H), 7.50 (t, J = 7.5 Hz, 1H), 7.43 - 7.32 (m, 4H), 6.72 (d, J = 1.9 Hz, 1H), 6.65 (d, J = 9.7 Hz, 1H), 5.22 (q, J = 7.0 Hz, 1H), 1.57 (d, J = 7.1 Hz, 3H); LC/MS (ESI) m/z = 403.2 $[M+H]^+$ |
| 104 | 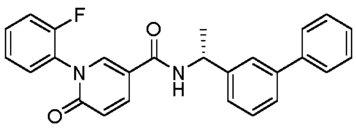 N-[(1R)-1-{[1,1'-biphenyl]-3-yl}ethyl]-1-(2-fluorophenyl)-6-oxo-1,6-dihydropyridine-3-carboxamide | $^1$H NMR (500 MHz, MeOD) δ 8.25 (d, J = 2.6 Hz, 1H), 8.08 (dd, J = 9.6, 2.6 Hz, 1H), 7.67 - 7.63 (m, 1H), 7.60 (ddt, J = 9.5, 6.1, 1.4 Hz, 4H), 7.55 (ddd, J = 11.0, 5.6, 3.1 Hz, 2H), 7.49 (dt, J = 7.6, 1.6 Hz, 2H), 7.41 (q, J = 7.6 Hz, 4H), 7.35 (ddd, J = 16.3, 6.7, 3.8 Hz, 4H), 6.65 (d, J = 9.6 Hz, 1H), 5.29 - 5.20 (m, 1H), 1.58 (d, J = 7.1 Hz, 3H); LC/MS (ESI) m/z = 413.2 $[M+H]^+$ |
| 105 | 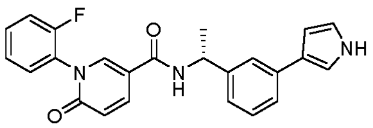 1-(2-fluorophenyl)-6-oxo-N-[(1R)-1-[3-(1H-pyrrol-3-yl)phenyl]ethyl]-1,6-dihydropyridine-3-carboxamide | $^1$H NMR (500 MHz, MeOD) δ 8.22 (d, J = 2.6 Hz, 1H), 8.05 (dd, J = 9.7, 2.6 Hz, 1H), 7.58 - 7.49 (m, 2H), 7.44 (td, J = 7.8, 1.8 Hz, 1H), 7.38 (dt, J = 7.8, 1.4 Hz, 1H), 7.37 - 7.29 (m, 2H), 7.23 (t, J = 7.7 Hz, 1H), 7.10 (dt, J = 7.6, 1.5 Hz, 1H), 7.08 (t, J = 1.8 Hz, 1H), 6.74 (dd, J = 2.8, 1.9 Hz, 1H), 6.63 (d, J = 9.6 Hz, 1H), 6.42 (dd, J = 2.8, 1.6 Hz, 1H), 5.16 (q, J = 7.0 Hz, 1H), 1.53 (d, J = 7.0 Hz, 3H); LC/MS (ESI) m/z = 402.2 $[M+H]^+$ |
| 106 | 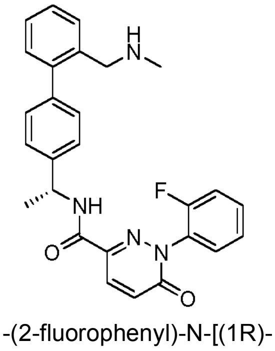 1-(2-fluorophenyl)-N-[(1R)-1-[4-[2-(methylaminomethyl)phenyl]phenyl]ethyl]-6-oxo-pyridazine-3-carboxamide | $^1$H NMR (400 MHz, DMSO-$d_6$) δ = 8.93 (d, *J* = 8.4 Hz, 1H), 7.97 (d, *J* = 10.0 Hz, 1H), 7.69 (dt, *J* = 1.6, 7.6 Hz, 1H), 7.62 - 7.55 (m, 1H), 7.51 (d, *J* = 7.2 Hz, 1H), 7.47 - 7.39 (m, 4H), 7.38 - 7.31 (m, 3H), 7.30 - 7.24 (m, 1H), 7.21 - 7.15 (m, 2H), 5.28 - 5.13 (m, 1H), 3.52 (s, 2H), 2.19 (s, 3H), 1.51 (d, *J* = 7.2 Hz, 3H); LC/MS (ESI) m/z: 457.4 $[M+H]^+$ |

(continued)

| No. | Structure/Name | Spectral Data |
|---|---|---|
| 107 | 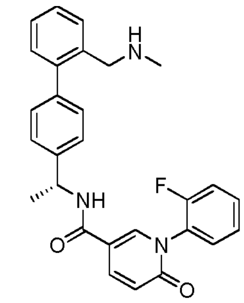 1-(2-fluorophenyl)-N-[(1R)-1-[4-[2-(methylaminomethyl)phenyl]phenyl]ethyl]-6-oxo-pyridine-3-carboxamide | $^1$H NMR (400 MHz, DMSO-$d_6$) $\delta$ = 8.62 (br d, *J* = 8.0 Hz, 1H), 8.41 (d, *J* = 2.4 Hz, 1H), 8.02 (dd, *J* = 2.4, 9.6 Hz, 1H), 7.67 - 7.44 (m, 4H), 7.44 - 7.27 (m, 7H), 7.19 (br d, *J* = 7.6 Hz, 1H), 6.57 (d, *J* = 10.0 Hz, 1H), 5.18 (quin, *J* = 7.2 Hz, 1H), 3.55 (s, 2H), 2.21 (s, 3H), 2.05 - 1.94 (m, 1H), 1.48 (br d, *J* = 7.2 Hz, 3H); LC/MS (ESI) m/z: 456.3 [M+H]$^+$ |

**Example 108: 1-(2-fluorophenyl)-N-[(1R)-1-[2'-(methylamino)-[1,1'-biphenyl]-3-yl]ethyl]-6-oxo-1,6-dihydropyridine-3-carboxamide**

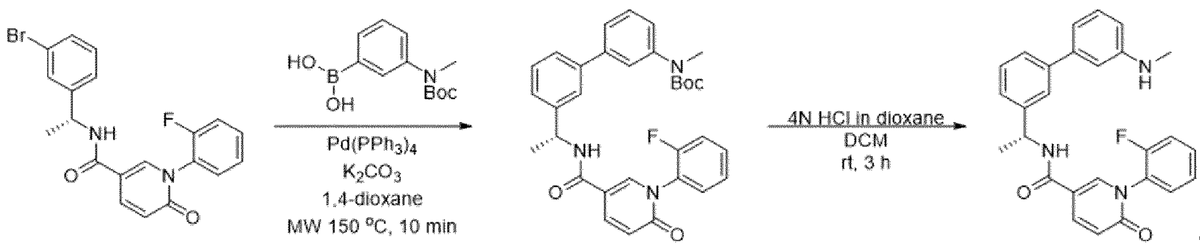


**108**

To a solution of (R)-N-(1-(3-bromophenyl)ethyl)-1-(2-fluorophenyl)-6-oxo-1,6-dihydropyridine-3-carboxamide obtained from Step 1 of Example 100 (50 mg, 0.12 mmol) in 1,4-dioxane (2.5 mL) and distilled water (0.5 mL), (3-((tert-butoxycarbonyl)(methyl)amino)phenyl)boronic acid (35.2 mg, 0.14 mmol), $Pd(PPh_3)_4$ (13.9 mg, 0.012 mmol) and $K_2CO_3$ (49.7 mg, 0.36 mmol) were added. The reaction mixture was heated by microwave at 150 °C for 10 min. The mixture was cooled, added with water, and extracted with DCM. The organic layer was washed with brine, dried over $Na_2SO_4$ and concentrated under reduced pressure. The residue was dissolved in DCM, and 4N HCl (85 $\mu$L) in dioxane was added thereto. The reaction mixture was stirred at room temperature for 3 hours. The mixture was concentrated and basified with sat. $NaHCO_3$ solution. The aqueous layer was extracted with EA. The combined organic layer was dried over $Na_2SO_4$, filtered and concentrated. The mixture was purified by prep-HPLC to give the compound of Example 108 (9.6 mg, 18.1 % yield). $^1$H NMR (500 MHz, MeOD) $\delta$ 8.25 (d, J = 2.6 Hz, 1H), 8.08 (dd, J = 9.6, 2.6 Hz, 1H), 7.56 (d, J = 6.4 Hz, 2H), 7.52 - 7.47 (m, 1H), 7.45 (d, J = 7.5 Hz, 1H), 7.37 (dd, J = 8.5, 6.5 Hz, 3H), 7.32 (d, J = 7.9 Hz, 1H), 7.18 (t, J = 7.8 Hz, 1H), 6.86 (d, J = 7.6 Hz, 1H), 6.82 (t, J = 2.1 Hz, 1H), 6.65 (d, J = 9.6 Hz, 1H), 6.61 (dd, J = 7.9, 2.4 Hz, 1H), 5.23 (q, J = 7.1 Hz, 1H), 2.80 (s, 3H), 1.57 (d, J = 7.1 Hz, 3H); LC/MS m/z = 442.2 [M+H]$^+$.

**Example 109: 1-(2-fluorophenyl)-N-[(1R)-1-(3-{4-[(methylamino)methyl]thiophen-2-yl}phenyl)ethyl]-6-oxo-1,6-dihydropyridine-3-carboxamide**

Step 1: Synthesis of (R)-1-(2-fluorophenyl)-6-oxo-N-(1-(3-(4,4,5,5-tetramethyl-1,3,2-dioxaborolan-2-yl)phenyl)ethyl)-1,6-dihydropyridine-3-carboxamide

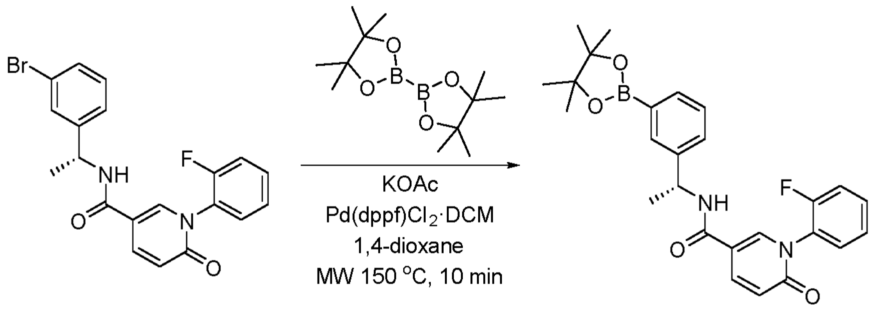


To a solution of (R)-N-(1-(3-bromophenyl)ethyl)-1-(2-fluorophenyl)-6-oxo-1,6-dihydropyridine-3-carboxamide obtained from Step 1 of Example 100 (100 mg, 0.24 mmol) in 1,4-dioxane (3 mL), bis(pinacolato)diborane (70.9 mg, 0.72 mmol), Pd(dppf)$Cl_2$·DCM (19.7 mg, 0.024 mmol) and KOAc (70.6 mg, 0.72 mmol) were added. The reaction mixture was heated by microwave at 150 °C for 10 min. The mixture was cooled and extracted with EtOAc. The organic layer was dried over anhydrous $Na_2SO_4$ and concentrated in vacuo to obtain a crude product. The product was purified by flash chromatography (0-5% MeOH in DCM) to give (R)-1-(2-fluorophenyl)-6-oxo-N-(1-(3-(4,4,5,5-tetramethyl-1,3,2-dioxaborolan-2-yl)phenyl)ethyl)-1,6-dihydropyridine-3-carboxamide (113.4 mg, 102 % yield) as brown liquid. LC/MS m/z = 463.3 $[M+H]^+$.

Step 2: Synthesis of 1-(2-fluorophenyl)-N-[(1R)-1-(3-{4-[(methylamino)methyl]thiophen-2-yl}phenyl)ethyl]-6-oxo-1,6-dihydropyridine-3-carboxamide

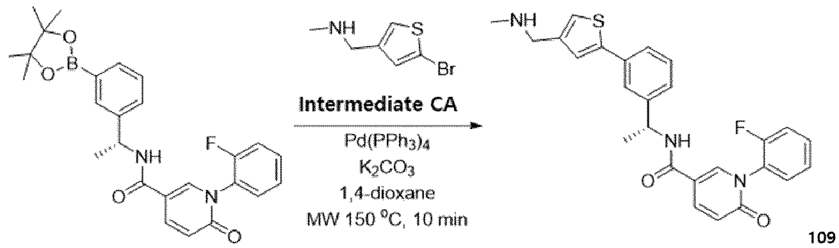

109

The compound of Example 109 (2.3 mg, 3.0% yield) was obtained in the same manner as in Step 2 of Example 100. $^1H$ NMR (500 MHz, MeOD) δ 8.26 (d, J = 2.6 Hz, 1H), 8.08 (dd, J = 9.7, 2.6 Hz, 1H), 7.62 (d, J = 1.9 Hz, 1H), 7.61 - 7.54 (m, 1H), 7.54 - 7.47 (m, 2H), 7.46 - 7.42 (m, 2H), 7.41 - 7.31 (m, 4H), 6.66 (d, J = 9.7 Hz, 1H), 5.20 (q, J = 7.1 Hz, 1H), 4.05 (s, 2H), 2.63 (s, 3H), 1.56 (d, J = 7.1 Hz, 3H); LC/MS m/z = 462.2 $[M+H]^+$.

**Example 110: 1-(2-fluorophenyl)-N-[(1R)-(1-(3-(3-[(methylamino)methyl]thiopen-2-yl)phenyl)ethyl)]-6-oxo-1,6-dihydropyridine-3-carboxamide**

Step 1: Synthesis of (R)-1-(2-fluorophenyl)-N-(1-(3-(3-formylthiophen-2-yl)phenyl)ethyl)-6-oxo-1,6-dihydropyridine-3-carboxamide

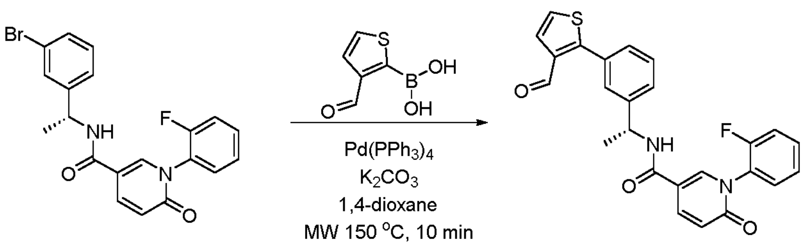


(R)-1-(2-fluorophenyl)-N-(1-(3-(3-formylthiophen-2-yl)phenyl)ethyl)-6-oxo-1,6-dihydropyridine-3-carboxamide (295.9 mg, 92% yield) was obtained by using (3-formylthiophen-2-yl)boronic acid in the same manner as in Step 2 of

Example 100. LC/MS (m/z) = 447.1 [M+H]$^+$.

Step 2: Synthesis of 1-(2-fluorophenyl)-N-[(1R)-(1-(3-(3-[(methylamino)methyl]thiophen-2-yl)phenyl)ethyl)]-6-oxo-1,6-dihydropyridine 3-carboxamide

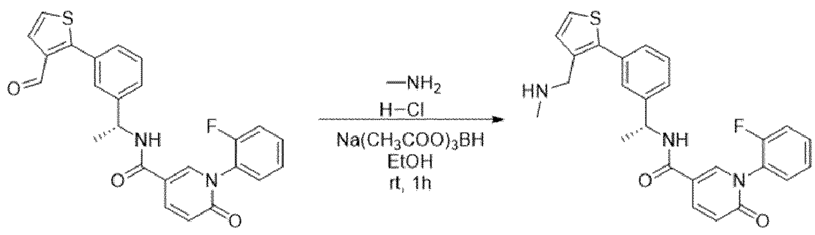


**110**

To a solution of (R)-1-(2-fluorophenyl)-N-(1-(3-(3-formylthiophen-2-yl)phenyl)ethyl)-6-oxo-1,6-dihydropyridine-3-carboxamide (176.2 mg, 0.39 mmol) in EtOH (4 mL) was added methylamine hydrochloride (40.0 mg, 0.59 mmol). The reaction mixture was stirred at room temperature for 15 min and cooled to 0 °C. To the mixture was added sodium triacetoxyborohydride (167 mg, 0.79 mmol). The mixture was stirred at room temperature for 1 hour. The mixture was quenched with water and concentrated under reduced pressure. The residue was extracted with DCM. The organic layer was dried over $Na_2SO_4$ and concentrated under reduced pressure. The residue was purified by prep-HPLC and pTLC to give the compound of Example 110 (26 mg, 14.27 % yield). $^1$H NMR (500 MHz, MeOD) δ 8.26 (d, J = 2.6 Hz, 1H), 8.08 (dt, J = 9.7, 1.8 Hz, 1H), 7.56 (t, J = 6.6 Hz, 1H), 7.49 (t, J = 7.5 Hz, 1H), 7.44 (s, 1H), 7.43 - 7.38 (m, 4H), 7.38 (s, 1H), 7.36 (s, 1H), 7.35 - 7.30 (m, 2H), 7.18 (d, J = 5.3 Hz, 1H), 6.65 (d, J = 9.6 Hz, 1H), 5.21 (q, J = 7.1 Hz, 1H), 3.81 (s, 2H), 2.34 (s, 3H), 1.57 (d, J = 7.1 Hz, 3H); LC/MS m/z = 462.2 [M+H]$^+$.

**Example 111: 1-(2-fluorophenyl)-N-[(1R)-1-(3-(2-[(methylamino)methyl]thiophen-3-yl)phenyl)ethyl]-6-oxo-1,6-dihydropyridine-3-carboxamide**

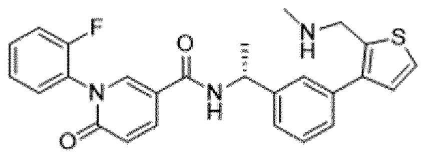

**111**

The compound of Example 111 (33 mg, 13.0% yield) was obtained by using (2-formylthiophen-3-yl)boronic acid in Step 1 in the same manner as in Example 110. $^1$H NMR (500 MHz, MeOD) δ 8.25 (d, J = 2.6 Hz, 1H), 8.07 (dd, J = 9.7, 2.6 Hz, 1H), 7.56 (ddd, J = 7.7, 5.1, 1.8 Hz, 1H), 7.52 - 7.44 (m, 1H), 7.43 - 7.30 (m, 6H), 7.26 (d, J = 7.7 Hz, 1H), 7.05 (d, J = 5.1 Hz, 1H), 6.65 (d, J = 9.7 Hz, 1H), 5.20 (q, J = 7.0 Hz, 1H), 3.93 (s, 2H), 2.32 (s, 2H), 1.56 (d, J = 7.0 Hz, 3H); LC/MS m/z = 462.2 [M+H]$^+$.

**Example 112: (R)-1-(2-fluorophenyl)-N-(1-(1-(2-((methylamino)methyl)phenyl)-1H-pyrazol-3-yl)ethyl)-6-oxo-1,6-dihydropyridine-3-carboxamide**

Step 1: Synthesis of (R)-N-(1-(1H-pyrazol-3-yl)ethyl)-1-(2-fluorophenyl)-6-oxo-1,6-dihydropyridine-3-carboxamide

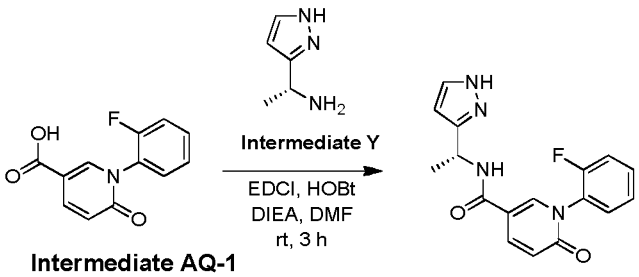


Intermediate AQ-1

(R)-N-(1-(1H-pyrazol-3-yl)ethyl)-1-(2-fluorophenyl)-6-oxo-1,6-dihydropyridine-3-carboxamide (20.1 mg, 17.0% yield) was obtained in the same manner as in Step 2 of Example 76. LC/MS m/z = 327.7 $[M+H]^+$.

Step 2: Synthesis of (R)-1-(2-fluorophenyl)-N-(1-(1-(2-((methylamino)methyl)phenyl)-1H-pyrazol-3-yl)ethyl)-6-oxo-1,6-dihydropyridine-3-carboxamide

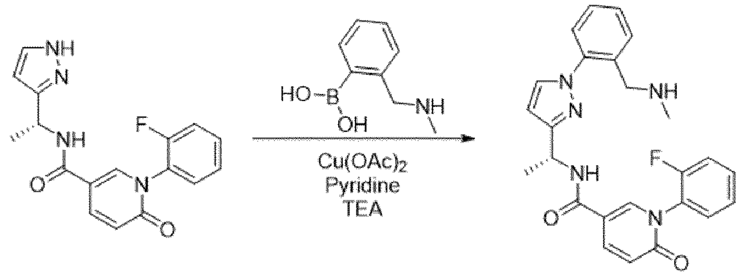


112

To a solution of (R)-N-(1-(1H-pyrazol-3-yl)ethyl)-1-(2-fluorophenyl)-6-oxo-1,6-dihydropyridine-3-carboxamide (20.1 mg, 0.062 mmol) in anhydrous THF (0.2 mL), 2-((methylamino)methyl)phenylboronic acid (12.2 mg, 0.074 mmol), $Cu(OAc)_2$ (22.4 mg, 0.12 mmol), pyridine (39.7 μL, 0.49 mmol) and TEA (42.9 μl, 0.31 mmol) were added. The reaction mixture was heated by microwave at 140 °C for 10 min. The mixture was cooled to room temperature, and filtered through celite and rinsed with MeOH. The filtrate was concentrated, and the concentrate was purified by prep-HPLC to give the compound of Example 112 (1.9 mg, 6.9 % yield). $^1H$ NMR (500 MHz, MeOD) δ 8.25 (d, J = 2.6 Hz, 1H), 8.10 (d, J = 2.6 Hz, 1H), 8.07 (dd, J = 9.6, 2.6 Hz, 1H), 7.63 (ddd, J = 15.1, 7.5, 1.5 Hz, 2H), 7.62 - 7.52 (m, 2H), 7.49 (qd, J = 7.5, 1.6 Hz, 2H), 7.37 (q, J = 8.6 Hz, 2H), 6.65 (d, J = 9.6 Hz, 1H), 6.60 (d, J = 2.5 Hz, 1H), 5.40 - 5.31 (m, 1H), 4.13 (d, J = 3.6 Hz, 2H), 2.87 (s, 3H), 1.65 (d, J = 7.2 Hz, 3H); LC/MS m/z = 446.2 $[M+H]^+$.

**Example 113: 1-(3-acetamido-4-fluoro-phenyl)-N-[(1R)-1-[3-amino-5-(trifluoromethyl)phenyl]ethyl]-6-oxo-pyridine-3-carboxamide**

Step 1: Synthesis of 1-(3-acetamido-4-fluoro-phenyl)-6-oxo-pyridine-3-carboxylic acid

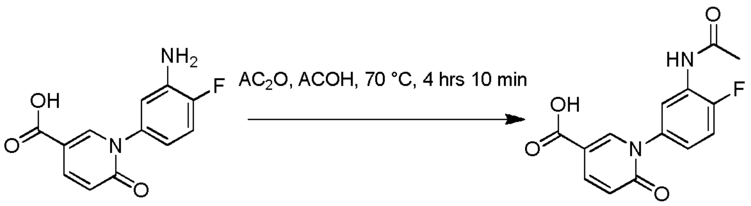


Intermediate AQ-2

A mixture of Intermediate AQ-2 (250 mg, 1.01 mmol) in AcOH (2 mL) was stirred at 70 °C for 10 min. The mixture was added with $Ac_2O$ (514 mg, 5.04 mmol) and stirred at 70 °C for 16 hours. The mixture was filtrated, and the filtrate was concentrated in vacuo to give 1-(3-acetamido-4-fluoro-phenyl)-6-oxo-pyridine-3-carboxylic acid (180 mg, 61% yield) as a white solid. $^1H$ NMR (400 MHz, DMSO-$d_6$) δ = 9.72 (s, 1H), 8.16 (d, *J* = 2.4 Hz, 1H), 8.02 (dd, *J* = 2.4,

6.8 Hz, 1H), 7.86 (dd, $J$ = 2.4, 9.6 Hz, 1H), 7.38 (dd, $J$ = 8.8, 10.4 Hz, 1H), 7.28 - 7.16 (m, 1H), 6.54 (d, $J$ = 9.6 Hz, 1H), 2.12 (s, 3H).

Steps 2 and 3: Synthesis of 1-(3-acetamido-4-fluoro-phenyl)-N-[(1R)-1-[3-amino-5-(trifluoromethyl)phenyl]ethyl]-6-oxo-pyridine-3-carboxamide

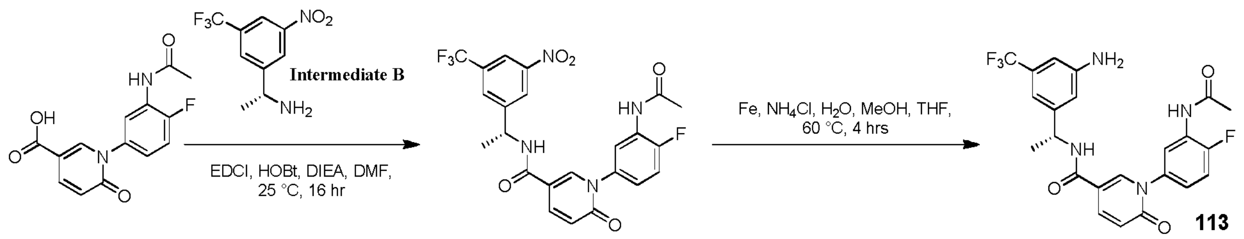


113

The compound of Example 113 (20.3 mg, 53% yield) was obtained in the same manner as in Steps 3 and 4 of Example 89 as a white solid. $^1H$ NMR (400 MHz, DMSO-$d_6$) $\delta$ = 9.98 (s, 1H), 8.61 - 8.55 (m, 1H), 8.36 - 8.32 (m, 1H), 8.04 (d, $J$= 4.8 Hz, 1H), 7.98 - 7.92 (m, 1H), 7.48 - 7.38 (m, 1H), 7.28 - 7.22 (m, 1H), 6.88 - 6.82 (m, 1H), 6.76 (s, 1H), 6.68 (s, 1H), 6.56 - 6.52 (m, 1H), 5.58 - 5.52 (m, 1H), 5.06 - 4.96 (m, 1H), 2.14 - 2.09 (m, 3H), 1.42 (d, $J$ = 7.2 Hz, 3H); MS (EI) m/z: 477.1 $[M+H]^+$.

**Example 114: 1-(3-acetamido-2-fluoro-phenyl)-N-[(1R)-1-[3-amino-5-(trifluoromethyl)phenyl]ethyl]-6-oxo-pyridine-3-carboxamide**

Step 1: Synthesis of methyl 1-(3-bromo-2-fluoro-phenyl)-6-oxo-pyridine-3-carboxylate

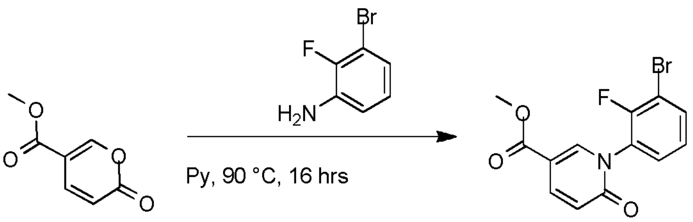


Methyl 1-(3-bromo-2-fluoro-phenyl)-6-oxo-pyridine-3-carboxylate (1.89 g, 22% yield) was obtained as a yellow solid in the same manner as in Step 1 of Preparation Example 43. $^1H$ NMR (400 MHz, DMSO-$d_6$) $\delta$ = 8.44 (d, $J$ = 2.4 Hz, 1H), 7.94 (dd, $J$ = 2.4, 9.6 Hz, 1H), 7.89 - 7.81 (m, 1H), 7.66 - 7.60 (m, 1H), 7.34 (dd, $J$ = 1.2, 8.0 Hz, 1H), 6.60 (d, $J$ = 9.6 Hz, 1H), 3.78 (s, 3H).

Step 2: Synthesis of methyl 1-(3-acetamido-2-fluoro-phenyl)-6-oxo-pyridine-3-carboxylate

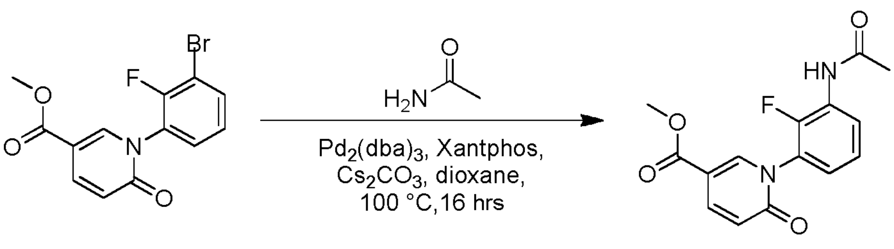


A solution of methyl 1-(3-bromo-2-fluoro-phenyl)-6-oxo-pyridine-3-carboxylate (500 mg, 1.53 mmol), acetamide

(226 mg, 3.83 mmol), $Pd_2(dba)_3$ (140 mg, 153 μmol), Xantphos (88.7 mg, 153 μmol) and $Cs_2CO_3$ (1.25 g, 3.83 mmol) in dioxane (10 mL) was stirred at 100 °C for 16 hours. The reaction mixture was concentrated in vacuo. The residue was purified by silica gel column chromatograpy (PE/EA = 1/0 to 2/1) to give methyl 1-(3-acetamido-2-fluoro-phenyl)-6-oxo-pyridine-3-carboxylate (380 mg, 81% yield) as a yellow solid. $^1H$ NMR (400 MHz, DMSO-$d_6$) δ = 9.94 (s, 1H), 8.36 (d, *J* = 2.4 Hz, 1H), 7.98 - 7.86 (m, 2H), 7.32 - 7.28 (m, 2H), 6.64 - 6.58 (m, 1H), 3.78 (s, 3H), 2.11 (s, 3H).

Steps 3 to 5: Synthesis of 1-(3-acetamido-2-fluoro-phenyl)-N-[(1R)-1-[3-amino-5-(trifluoromethyl)phenyl]ethyl]-6-oxo-pyridine-3-carboxamide

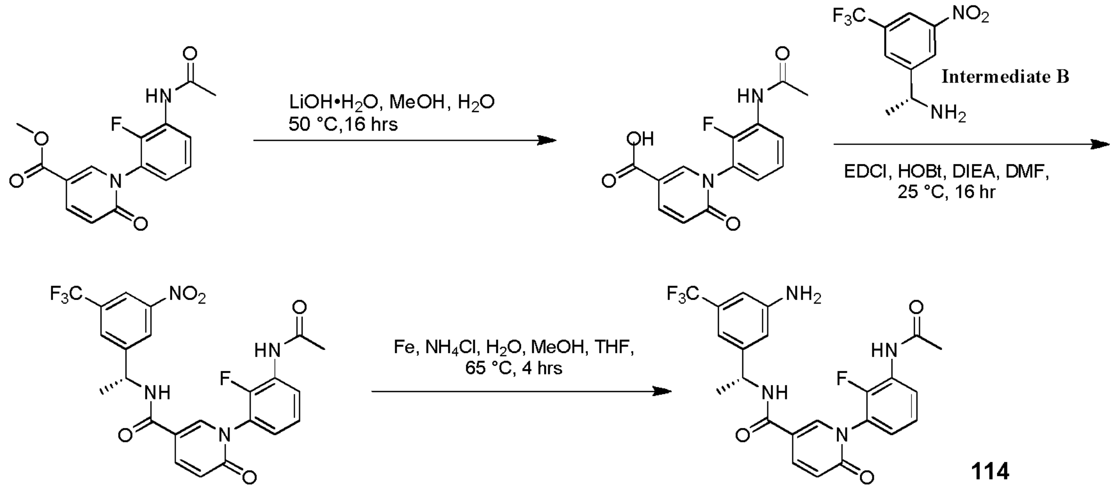


**114**

The compound of Example 114 (15.4 mg, 47% yield) was obtained as a white solid in the same manner as in Steps 2 to 4 of Example 89. $^1H$ NMR (400 MHz, DMSO-$d_6$) δ = 9.97 (s, 1H), 8.57 (d, *J* = 7.6 Hz, 1H), 8.39 (d, *J* = 2.4 Hz, 1H), 8.08 - 7.96 (m, 2H), 7.34 (d, *J* = 5.6 Hz, 2H), 6.76 (s, 2H), 6.71 (s, 1H), 6.58 (d, *J* = 9.6 Hz, 1H), 5.60 - 5.52 (m, 2H), 5.01 (t, *J* = 7.2 Hz, 1H), 2.12 (s, 3H), 1.40 (d, *J* = 7.2 Hz, 3H); MS (EI) m/z: 477.1 $[M+H]^+$.

**Example 115: N-[(1R)-1-[3-amino-5-(trifluoromethyl)phenyl]ethyl]-1-cyclopentyl-6-oxo-1,6-dihydropyridazine-3-carboxamide**

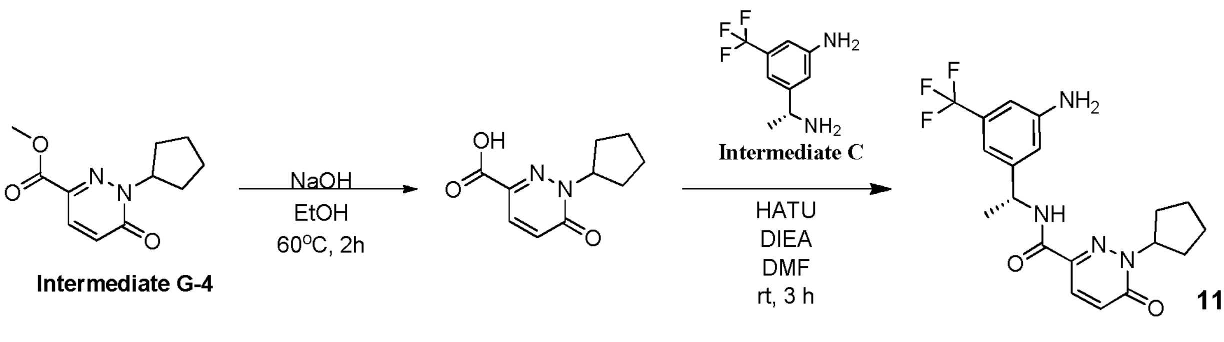


**Intermediate G-4**

**115**

The compound of Example 115 (9 mg, 51.6% yield) was obtained in the same manner as in Example 64. $^1H$ NMR (400 MHz, DMSO) δ 8.52 (d, J = 8.3 Hz, 1H), 7.78 (d, J = 9.7 Hz, 1H), 6.98 (d, J = 9.7 Hz, 1H), 6.88 (s, 1H), 6.83 (s, 1H), 6.76 (s, 1H), 5.24 (p, J = 7.7 Hz, 1H), 5.09 - 5.00 (m, 1H), 1.97 (d, J = 8.9 Hz, 5H), 1.83 (t, J = 6.2 Hz, 2H), 1.64 (d, J = 6.9 Hz, 2H), 1.49 (d, J = 7.0 Hz, 3H); LC/MS m/z = 395.2 $[M+H]^+$.

**Example 116 and Example 117**

The compounds shown in the following table were prepared in the same manner as in Example 115 by preparing

appropriate starting materials corresponding to the respective structures of the desired compounds based on Preparation Example 7.

[Table 8]

| No. | Structure/Name | Spectral Data |
|---|---|---|
| 116 | 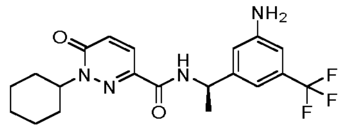 N-[(1R)-1-[3-amino-5-(trifluoromethyl)phenyl]ethyl]-1-cyclohexyl-6-oxo-1,6-dihydropyridazine-3-carboxamide | $^1$H NMR (400 MHz, DMSO) δ 8.65 (d, J = 8.4 Hz, 1H), 7.79 (d, J = 9.6 Hz, 1H), 6.99 (d, J = 9.7 Hz, 1H), 6.91 (s, 1H), 6.84 (s, 1H), 6.77 (d, J = 1.8 Hz, 1H), 5.08 (p, J = 7.3 Hz, 1H), 4.76 (td, J = 11.6, 3.9 Hz, 1H), 1.89 (tdd, J = 15.6, 9.4, 5.0 Hz, 3H), 1.77 (d, J = 7.8 Hz, 2H), 1.68 (d, J = 12.8 Hz, 1H), 1.54 - 1.34 (m, 4H), 1.34 1.22 (m, 1H); LC/MS (ESI) m/z = 409.2 [M+H]$^+$ |
| 117 | 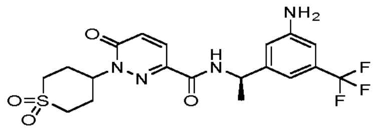 N-[(1R)-1-[3-amino-5-(trifluoromethyl)phenyl]ethyl]-1-(1,1-dioxo-1λ$^6$-thian-4-yl)-6-oxo-1,6-dihydropyridazine-3-carboxamide | $^1$H NMR (400 MHz, DMSO) δ 8.82 (d, J = 8.4 Hz, 1H), 7.81 (d, J = 9.7 Hz, 1H), 7.03 (d, J = 9.7 Hz, 1H), 6.81 (d, J = 9.1 Hz, 2H), 6.72 (s, 1H), 5.57 (s, 2H), 5.16 (t, *J* = 11.4 Hz, 1H), 5.05 (t, J = 7.5 Hz, 1H), 3.50 (t, J = 13.5 Hz, 2H), 2.61 (s, 1H), 2.14 (d, J = 13.1 Hz, 2H), 1.50 (d, J = 7.1 Hz, 3H); LC/MS (ESI) m/z = 459.2 [M+H]$^+$ |

**Example 118: 1-(1-acetylpiperidin-4-yl)-N-[(1R)-1-[3-amino-5-(trifluoromethyl)phenyl]ethyl]-6-oxo-1,6-dihydropyridazine-3-carboxamide**

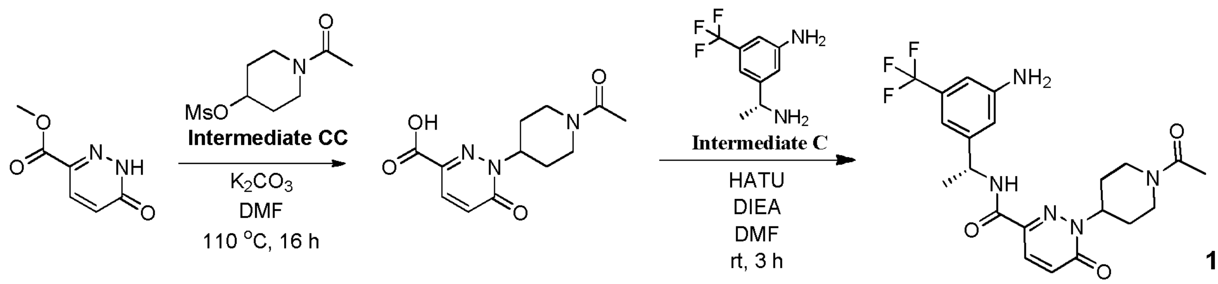


**118**

The compound of Example 118 (10 mg, 15% yield) was obtained in a similar manner to Preparation Example 7 and Step 2 of Example 115. $^1$H NMR (400 MHz, DMSO) δ 8.77 (d, J = 8.5 Hz, 1H), 7.81 (dd, J = 9.7, 1.9 Hz, 1H), 7.02 (d, J = 9.7 Hz, 1H), 6.87 - 6.76 (m, 2H), 6.70 (d, J = 8.9 Hz, 2H), 5.56 (d, J = 9.7 Hz, 2H), 5.11 - 5.01 (m, 1H), 4.98 (d, J = 12.5 Hz, 1H), 4.58 (d, J = 13.3 Hz, 1H), 3.97 (s, 1H), 3.30 - 3.14 (m, 2H), 2.74 - 2.65 (m, 1H), 2.04 (d, J = 3.9 Hz, 3H), 1.87 - 1.75 (m, 2H), 1.49 (d, J = 7.1 Hz, 3H); LC/MS m/z = 425.2 [M+H]$^+$.

**Example 119 and Example 120**

The compounds shown in the following table were prepared in the same manner as in Example 118 by replacing Intermediate CC with appropriate methanesulfonate compounds corresponding to the respective structures of the desired compounds.

[Table 9]

| No. | Structure/Name | Spectral Data |
|---|---|---|
| 119 | 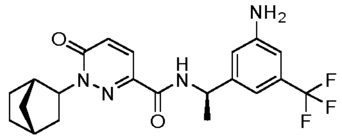 N-[(1R)-1-[3-amino-5-(trifluoromethyl)phenyl]ethyl]-1-[(1S, 4R)-bicyclo[2.2.1]heptan-2-yl]-6-oxo-1,6-dihydropyridazine-3-carboxamide | $^1$H NMR (400 MHz, DMSO) δ 8.36 (d, J = 8.1 Hz, 1H), 7.82 (d, J = 9.6 Hz, 1H), 7.00 (d, J = 9.6 Hz, 1H), 6.83 (d, J = 13.8 Hz, 2H), 6.72 (s, 1H), 5.57 (s, 2H), 5.12 (d, J = 11.7 Hz, 1H), 5.00 (t, J = 7.4 Hz, 1H), 2.34 (s, 2H), 1.82 (t, J = 12.5 Hz, 1H), 1.63 (d, J = 9.7 Hz, 1H), 1.49 (d, J = 7.0 Hz, 4H), 1.39 (d, J = 9.6 Hz, 1H), 1.23 (s, 3H), 1.08 (s, 1H); LC/MS (ESI) m/z = 421.2 [M+H]$^+$ |
| 120 | 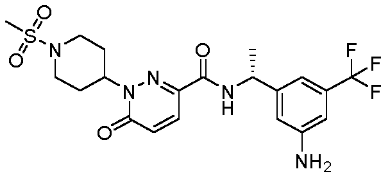 N-[(1R)-1-[3-amino-5-(trifluoromethyl)phenyl]ethyl]-1-(1-methanesulfonylpiperidin-4-yl)-6-oxo-1,6-dihydropyridazine-3-carboxamide | $^1$H NMR (400 MHz, DMSO) δ 8.88 (d, J = 8.4 Hz, 1H), 7.82 (d, J = 9.7 Hz, 1H), 7.02 (d, J = 9.7 Hz, 1H), 6.86 (s, 1H), 6.82 (s, 1H), 6.74 (s, 1H), 5.12 - 5.04 (m, 1H), 4.91 (s, 1H), 3.71 (d, J = 11.8 Hz, 3H), 3.01 - 2.93 (m, 2H), 2.91 (s, 3H), 2.26 (d, J = 11.7 Hz, 2H), 1.83 (m, 2H), 1.50 (d, J = 7.1 Hz, 3H); LC/MS (ESI) m/z = 488.2 [M+H]$^+$ |

**Example 121 and Example 122: N-[(1R)-1-[5-amino-2-chloro-3-(trifluoromethyl)phenyl]ethyl]-1-(2-fluorophenyl)-6-oxo-pyridazine-3-carboxamide and N-[(1S)-1-[5-amino-2-chloro-3-(trifluoromethyl)phenyl]ethyl]-1-(2-fluorophenyl)-6-oxo-pyridazine-3-carboxamide**

Step 1: Synthesis of 1-bromo-2-chloro-5-nitro-3-(trifluoromethyl)benzene

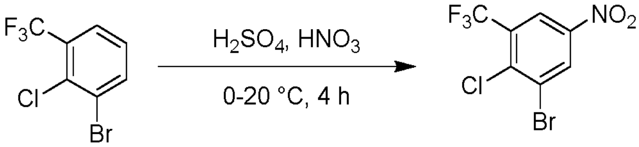


To a solution of 1-bromo-2-chloro-3-(trifluoromethyl)benzene (8.3 g, 31.99 mmol) in $H_2SO_4$ (15 mL) was added $HNO_3$ (10.270 g, 159.72 mmol, 7.34 mL, 98% purity) at 0°C for 40 min, followed by stirring at 20°C for 4 hours. The reaction mixture was poured onto ice water (30 mL) and extracted wtih EtOAc (40 mL×3). The combined organic layer was washed with brine (50 mL), dried over $Na_2SO_4$ and concentrated under reduced pressure to obtain a crude product, which was purified by silica gel column chromatography (1% EtOAc in PE) to give 1-bromo-2-chloro-5-nitro-3-(trifluoromethyl)benzene (9.3 g, 95.49% yield) as yellow oil. $^1$H NMR (400MHz, DMSO-$d_6$) δ 8.50 (d, *J* = 2.8 Hz, 1 H) 8.90 (d, *J* = 2.8 Hz, 1 H).

Step 2: Synthesis of 1-(2-chloro-5-nitro-3-(trifluoromethyl)phenyl)ethane-1-one

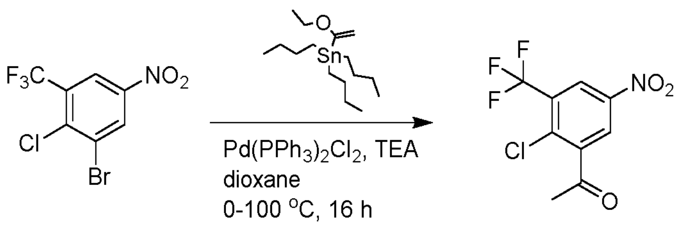

To a mixture of 1-bromo-2-chloro-5-nitro-3-(trifluorophenyl)benzene (3 g, 9.85 mmol), tributyl(1-ethoxyvinyl)stannane (3.340 g, 9.25 mmol, 3.12 mL) in dioxane (40 mL), TEA (1.99 g, 19.71 mmol, 2.74 mL) and $Pd(PPh_3)_2Cl_2$ (691.64 mg, 985.39 μmol) were added, followed by stirring at 100°C for 16 hours under nitrogen atmosphere. The reaction mixture was cooled to 0°C, treated with 4 M HCl, and stirred for 3 hours. The mixture was poured into water (50 mL) and extracted with EtOAc (50 mL × 3). The mixture was dried over $Na_2SO_4$ and filtered, and the filtrate was concentrated. The aqueous layer was adjusted to pH 9 with NaOH aqueous solution, added slowly with sodium hypochlorite (100 mL), and stirred. The product was purified by silica gel column chromatography (5% EtOAc in PE) to give 1-[2-chloro-5-nitro-3-(trifluoromethyl)phenyl]ethanone (1.47 g, 55.75% yield) as a yellow solid. $^1H$ NMR (400MHz, DMSO-$d_6$) δ 2.68 (m, 3 H) 8.59 (d, *J* = 2.8 Hz, 1 H) 8.81 (d, *J* = 2.8 Hz, 1 H).

Step 3: Synthesis of (R,Z)-N-(1-(2-chloro-5-nitro-3-(trifluoromethyl)phenyl)ethylidene)-2-methylpropane-2-sulfinamide

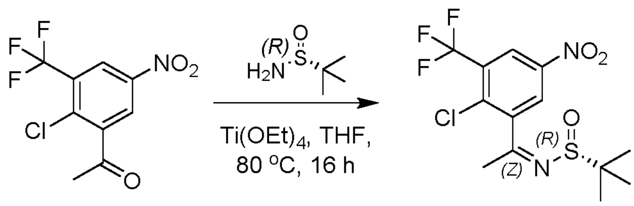


To a solution of 1-[2-chloro-5-nitro-3-(trifluoromethyl)phenyl]ethanone (1.47 g, 5.49 mmol) and (R)-2-methylpropane-2-sulfinamide (665.82 mg, 5.49 mmol) in THF (35 mL) was added $Ti(OEt)_4$ (3.76 g, 16.48 mmol, 3.42 mL). The reaction mixture was stirred 80 °C for 16 hours under nitrogen atmosphere. The reaction mixture was poured into water (30 mL) and extracted with EtOAc (30 mL × 3). The combined organic layer was washed with brine (50 mL), dried over $Na_2SO_4$, and concentrated under reduced pressure to obtain a crude product, which was purified by silica gel column chromatography (6% EtOAc in PE) to obtain (NZ,R)-N-[1-[2-chloro-5-nitro-3-(trifluoromethyl)phenyl]ethylidene]-2-methyl-propane-2-sulfinamide (1.66 g, 81.50% yield) as a yellow solid. $^1H$ NMR (400MHz, DMSO-$d_6$) δ 1.20 (br d, *J* = 17.2 Hz, 9 H) 2.50 (s, 2 H) 2.69 (s, 1 H) 8.53 (m, 1 H) 8.67 (br s, 1 H).

Step 4: (R)-N-[(1R)-1-[2-chloro-5-nitro-3-(trifluoro)phenyl]ethyl]-2-methyl-propane-2-sulfinamide and (R)-N-[(1S)-1-[2-chloro-5-nitro-3-(trifluoromethyl)phenyl]ethyl]-2-methylpropane-2-sulfinamide

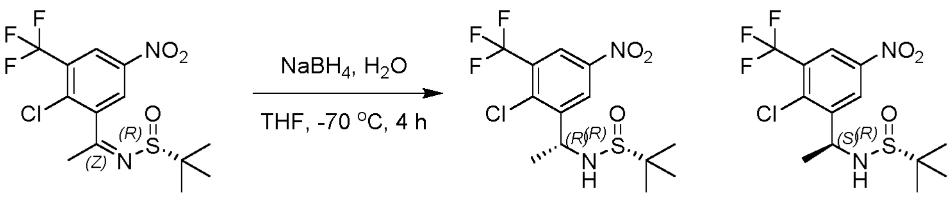


To a solution of (NZ,R)-N-[1-[2-chloro-5-nitro-3-(trifluoro)phenyl]ethylidene]-2-methylpropane-2-sulfinamide (480 mg, 1.29 mmol) in THF (12 mL) and $H_2O$ (0.5 mL) was added $NaBH_4$ (0.210 g, 5.55 mmol), followed by stirring at -70 °C for 4 hours under $N_2$ atmosphere. The mixture was quenched with aqueous ammonium chloride solution at room temperature, diluted with EtOAc (50 mL) and extracted with EtOAc (50 mL * 3). The organic layer was dried over $Na_2SO_4$ and filtrated under reduced pressure to obtain a crude product. The product was purified by silica column chromatography (28 % EtOAc in PE) to obtain (R)-N-[(1R)-1-[2-chloro-5-nitro-3-(trifluoro)phenyl]ethyl]-2-methyl-propane-2-sulfinamide (70 mg, 14.50% yield, 100% ee) and (R)-N-[(1S)-1-[2-chloro-5-nitro-3-(trifluoromethyl)phenyl]ethyl]-2-methyl-propane-2-sulfinamide (100 mg, 20.72% yield, 98.76% ee) as a yellow solid.

(R)_$^1H$ NMR (400 MHz, DMSO-$d_6$) δ ppm 1.12 (s, 9 H) 1.44 (d, *J*= 6.8 Hz, 3 H) 4.95 (m, 1 H) 6.34 (d, *J* = 8.8 Hz, 1 H) 8.45 (d, *J* = 2.8 Hz, 1 H) 8.84 (d, *J* = 2.8 Hz, 1 H); MS (ESI) m/z = 372.9 $[M+H]^+$. LC/MS $t_R$ = 0.571 min in 5-95AB_1min. (RP-18, 5um, 3.0*25mm), MS (ESI) m/z = 372.9 $[M+H]^+$; SFC (EB5303-244-P1S1): $t_R$ = 0.503 min in IC_3_IPA_DEA_40_25ML, e.e = 100%.

(S)_$^1H$ NMR (400 MHz, DMSO-$d_6$) δ ppm 1.14 (s, 9 H) 1.53 (d, *J* = 6.8 Hz, 3 H) 4.97 (quin, *J* = 6.8 Hz, 1 H) 5.92 (d, *J* = 6.4 Hz, 1 H) 8.44 (d, *J* = 2.8 Hz, 1 H) 8.73 (d, *J* = 2.8 Hz, 1 H); LC/MS (EB5303-244-P1A2): $t_R$ = 0.576 min

in 5-95AB_1min. (RP-18, 5um, 3.0*25mm), MS (ESI) m/z = 372.9 $[M+H]^+$; SFC (EB5303-244-P1S2): $t_R$ = 0.421 min in IC_3_IPA_DEA_40_25ML, e.e = 98.76%.

Step 5: (1R)-1-[2-chloro-5-nitro-3-(trifluoromethyl)phenyl]ethanamine hydrochloride and (1S)-1-[2-chloro-5-nitro-3-(trifluoromethyl)phenyl]ethanamine

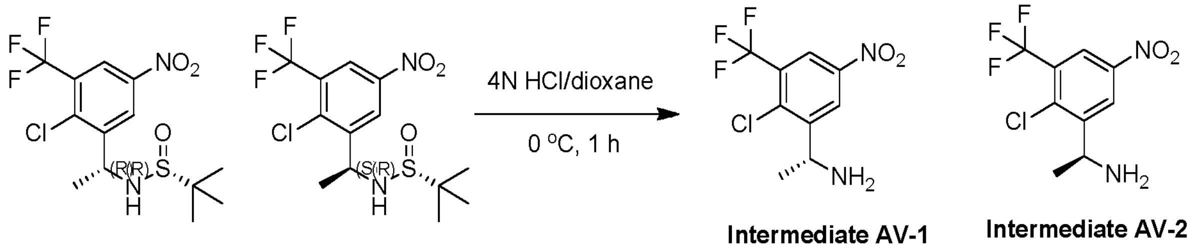


Intermediate AV-1 Intermediate AV-2

(R)-N-[(1R)-1-[2-chloro-5-nitro-3-(trifluoromethyl)phenyl]ethyl]-2-methyl-propane-2-sulfinamide (70 mg, 187.77 μmol) was added to 4N HCl/dioxane (3 mL) and stirred at 0 °C for 1 hour. The mixture was filtrated under reduced pressure to give Intermediate AV-1 (50 mg, 87.28%, HCl salt) as a yellow solid. LC/MS (ESI) m/z = 268.9 $[M+H]^+$.

Intermediate AV-2 (89 mg, 210.19 μmol, 78.36% yield, HCl salt) was obtained as a yellow solid in the same method as above. LC/MS (ESI) m/z = 269.0 $[M+H]^+$.

Step 6-1: Synthesis of N-[(1R)-1-[2-chloro-5-nitro-3-(trifluoromethyl)phenyl]ethyl]-1-(2-fluorophenyl)-6-oxo-pyridazine-3-carboxamide

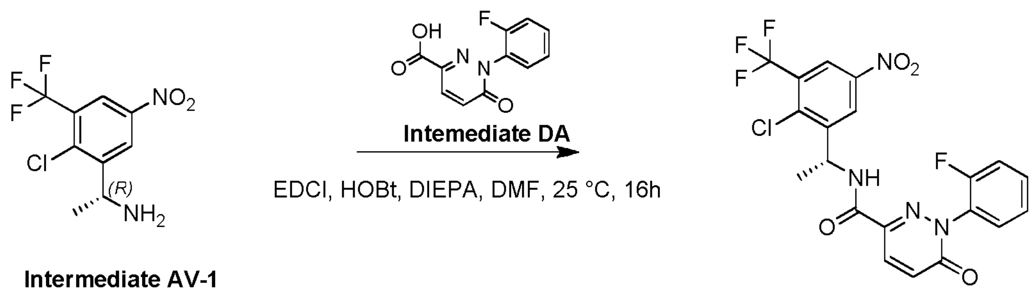


Intermediate AV-1

N-[(1R)-1-[2-chloro-5-nitro-3-(trifluoromethyl)phenyl]ethyl]-1-(2-fluorophenyl)-6-oxo-pyridazine-3-carboxamide was obtained in the same manner as in Example 94. MS (ESI) m/z = 485.1 $[M+H]^+$.

Step 7-1: Synthesis of N-[(1R)-1-[5-amino-2-chloro-3-(trifluoromethyl)phenyl]ethyl]-1-(2-fluorophenyl)-6-oxo-pyridazine-3-carboxamide

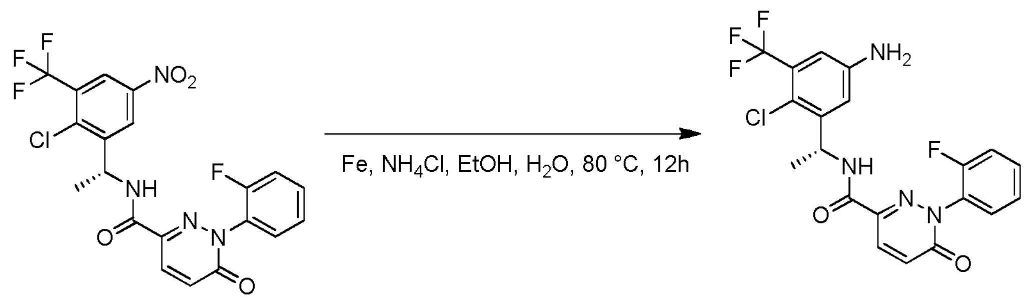


121

The compound of Example 121 was obtained in the same manner as in Step 3 of Example 56, except that EtOH and $H_2O$ were used instead of MeOH in Step 3 of Example 56. $^1H$ NMR (400 MHz, DMSO-$d_6$) $\delta$ ppm 1.40 (d, J = 7.2 Hz, 3 H) 5.31 (q, J = 7.2 Hz, 1 H) 6.88 (dd, J = 17.6, 2.8 Hz, 2 H) 7.19 (d, J = 10.0 Hz, 1 H) 7.44 (m, 2 H) 7.59 (m, 1 H) 7.71 (td, J = 7.6, 1.6 Hz, 1 H) 7.92 (d, J = 10.0 Hz, 1 H) 9.04 (d, J = 7.2 Hz, 1 H); MS (ESI) m/z = 455.3 $[M+H]^+$.

Step 6-2: N-[(1S)-1-[2-chloro-5-nitro-3-(trifluoromethyl)phenyl]ethyl]-1-(2-fluorophenyl)-6-oxo-pyridazine-3-carboxamide

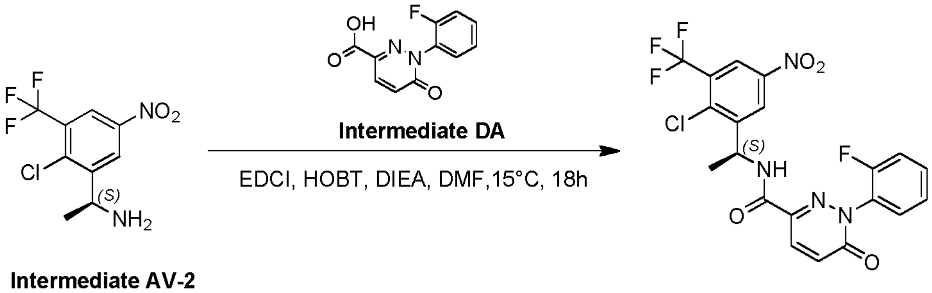


**Intermediate AV-2**

N-[(1S)-1-[2-chloro-5-nitro-3-(trifluoromethyl)phenyl]ethyl]-1-(2-fluorophenyl)-6-oxo-pyridazine-3-carboxamide was obtained in the same manner as in Example 94. MS (ESI) m/z = 485.1 $[M+H]^+$

Step 7-2: N-[(1S)-1-[5-amino-2-chloro-3-(trifluoromethyl)phenyl]ethyl]-1-(2-fluorophenyl)-6-oxo-pyridazine-3-carboxamide

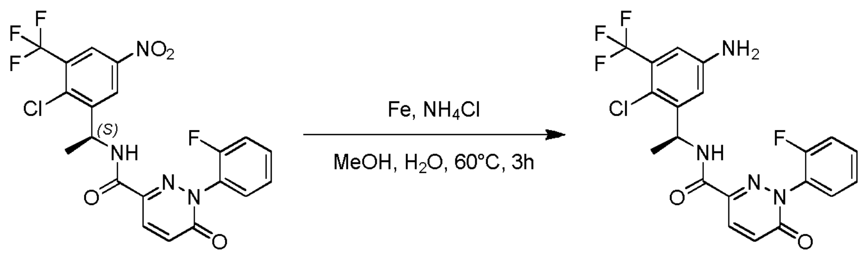


**122**

The compound of Example 122 was obtained in the same manner as in Step 3 of Example 56, except that $H_2O$ was added in Step 3 of Example 56. $^1H$ NMR (400 MHz, DMSO-$d_6$) $\delta$ = 9.03 (d, *J*= 7.2 Hz, 1H), 7.94 - 7.90 (m, 1H), 7.73 - 7.67 (m, 1H), 7.63 - 7.56 (m, 1H), 7.49 - 7.45 (m, 1H), 7.44 - 7.40 (m, 1H), 7.19 (d, *J* = 9.6 Hz, 1H), 6.90 (d, *J* = 2.8 Hz, 1H), 6.85 (d, *J* = 2.4 Hz, 1H), 5.68 (s, 2H), 5.31 (quin, *J* = 7.2 Hz, 1H), 1.40 (d, *J* = 7.2 Hz, 3H); MS (ESI) m/z = 455.3 $[M+H]^+$.

**Example 123: N-[(1R)-1-[3-amino-5-(trifluoromethyl)phenyl]ethyl]-6-oxo-1-(tetrahydropyran-4-ylmethyl)pyridine-3-carboxamide**

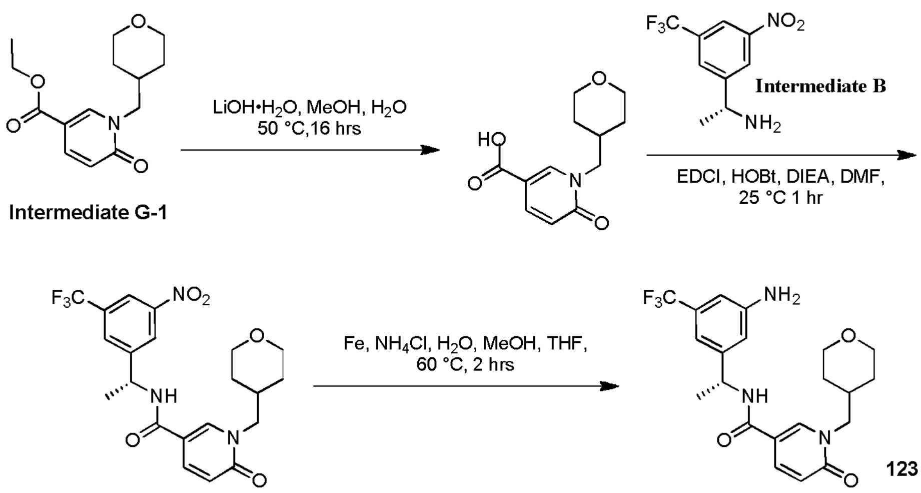


123

The compound of Example 123 (50 mg, 89% yield) was obtained in the same manner as in Steps 2 to 4 of Example 89 as a white solid. $^1H$ NMR (400 MHz, DMSO-$d_6$) δ = 8.51 (d, *J* = 8.0 Hz, 1H), 8.31 (d, *J* = 2.4 Hz, 1H), 7.93 (dd, *J* = 2.8, 9.6 Hz, 1H), 6.79 (s, 1H), 6.78 (s, 1H), 6.71 (s, 1H), 6.42 (d, *J* = 9.6 Hz, 1H), 5.02 (*J* = 7.2 Hz, 1H), 3.92 - 3.78 (m, 4H), 3.22 (t, *J* = 11.6 Hz, 2H), 2.08 - 1.95 (m, 1H), 1.42 (d, *J* = 7.2 Hz, 3H), 1.39 (s, 2H), 1.29 - 1.23 (m, 2H). MS (EI) m/z: 424.2 $[M+H]^+$.

**Example 124 and Example 125: N-((R)-1-(3-amino-5-(trifluoromethyl)phenyl)ethyl)-1-((1R,2R)-2-hydroxycyclohexyl)-6-oxo-1,6-dihydropyridazine-3-carboxamide and N-((R)-1-(3-amino-5-(trifluoromethyl)phenyl)ethyl)-1-((1S,2S)-2-hydroxycyclohexyl)-6-oxo-1,6-dihydropyridazine-3-carboxamide**

Step 1: Synthesis of 1-(2-hydroxycyclohexyl)-6-oxo-1,6-dihydropyridazine-3-carboxylic acid

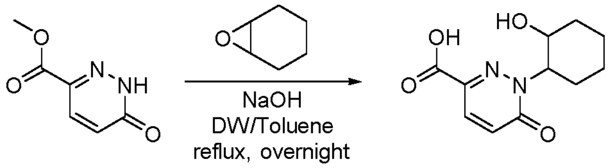


A mixture of methyl 6-oxo-1,6-dihydropyridazine-3-carboxylate (150 mg, 0.97 mmol), 7-oxabicyclo[4.1.0]heptane (478 mg, 4.87 mmol), sodium hydroxide (44.8 mg, 1.12 mmol) and benzyltriethylammonium chloride (22.2 mg, 0.09 mmol) in a mixture of water (2 mL) and toluene (2 mL) was refluxed for 18 hours overnight. The reaction mixture was cooled to room temperature. The mixture was added with distilled water and extracted with EA. The aqueous layer was acidified with 1N HCl and extracted with EA. The organic layer was dried over $MgSO_4$, filtered and concentrated under reduced pressure to give 1-(2-hydroxycyclohexyl)-6-oxo-1,6-dihydropyridazine-3-carboxylic acid (182 mg, 78 %) as a solid. LC/MS m/z = 239.1 $[M+H]^+$.

Step 2: Synthesis of N-((R)-1-(3-amino-5-(trifluoromethyl)phenyl)ethyl)-1-((1R,2R)-2-hydroxycyclohexyl)-6-oxo-1,6-dihydropyridazine-3-carboxamide and N-((R)-1-(3-amino-5-(trifluoromethyl)phenyl)ethyl)-1-((1S,2S)-2-hydroxycyclohexyl)-6-oxo-1,6-dihydropyridazine-3-carboxamide

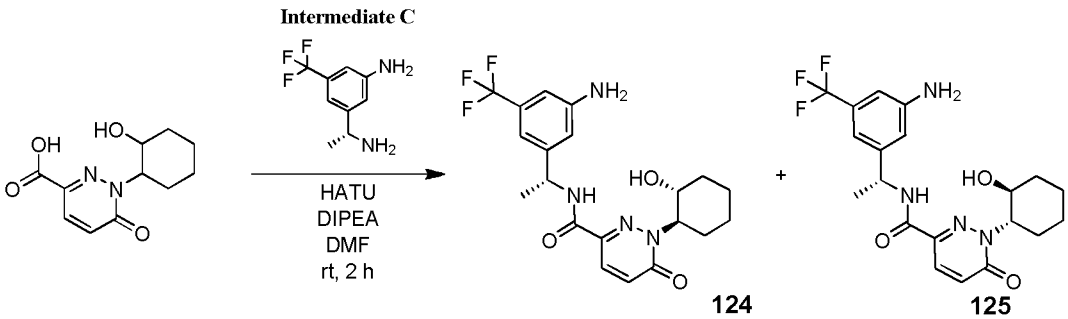


124 125

A solution of (R)-3-(1-aminoethyl)-5-(trifluoromethyl)aniline, 1-(2-hydroxycyclohexyl)-6-oxo-1,6-dihydropyridazine-3-carboxylic acid (30 mg, 0.12 mmol), HATU (47.9 mg, 0.12 mmol) and DIPEA (44 μL, 0.25 mmol) in DMF (2 mL) was prepared. The reaction mixture was stirred at room temperature for 2 hours. The mixture was added with distilled water and sat. $NaHCO_3$ solution and extracted with EA. The organic layer was dried over $MgSO_4$. The solvent was removed under reduced pressure, and the crude residue was purified by Prep/LC to give the compound of Example 124 (10 mg, 18.71%) and the compound of Example 125 (6.5 mg, 12.16%).

Compound of Example 124: $^1H$ NMR (400 MHz, DMSO-$d_6$) δ 8.63 (d, J = 8.5 Hz, 1H), 7.77 (d, J = 9.7 Hz, 1H), 6.97 (d, J = 9.6 Hz, 1H), 6.88 (s, 1H), 6.83 (s, 1H), 6.75 (t, J = 2.8 Hz, 1H), 5.09 (p, J = 7.2 Hz, 1H), 4.69 (t, J = 9.2 Hz, 1H), 3.99 (s, 1H), 1.98 (s, 1H), 1.78 (d, J = 17.4 Hz, 2H), 1.72 (s, 2H), 1.51 (d, J = 7.0 Hz, 3H), 1.36 (s, 3H), 1.29 - 1.21 (m, 1H); LC/MS m/z = 425.2 $[M+H]^+$.

Compound of Example 125: $^1H$ NMR (400 MHz, DMSO) δ 8.61 (d, J = 8.5 Hz, 1H), 7.77 (d, J = 9.6 Hz, 1H), 6.97 (d, J = 9.6 Hz, 1H), 6.89 (s, 1H), 6.83 (s, 1H), 6.77 - 6.71 (m, 2H), 5.13 - 5.04 (m, 1H), 4.68 (s, 1H), 3.97 (d, J = 4.9 Hz, 1H), 1.97 (s, 1H), 1.82 (s, 1H), 1.73 (t, J = 10.8 Hz, 3H), 1.51 (d, J = 7.1 Hz, 3H), 1.36 (d, J = 7.6 Hz, 2H), 1.29 - 1.21 (m, 1H); LC/MS m/z = 425.2 $[M+H]^+$.

**Example 126: N-[(1R)-1-[3-amino-5-(trifluoromethyl)phenyl]ethyl]-1-(1-methyl-1,2,3,6-tetrahydropyridin-4-yl)-6-oxo-1,6-dihydropyridazine-3-carboxamide**

Step 1: Synthesis of methyl 1-(1-methyl-1,2,3,6-tetrahydropyridin-4-yl)-6-oxo-1,6-dihydropyridazine-3-carboxylate

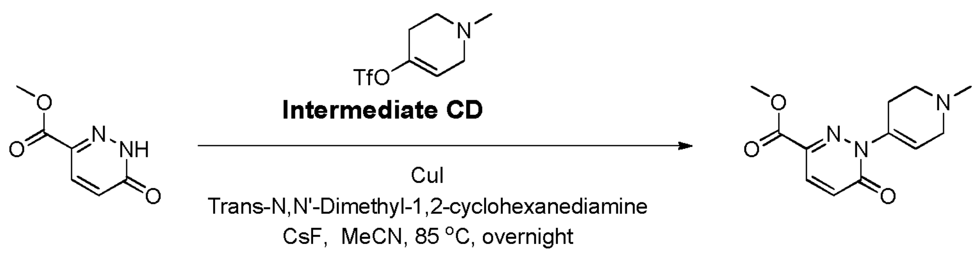


To a solution of methyl 6-oxo-1,6-dihydropyridazine-3-carboxylate (188 mg, 1.22 mmol) in MeCN (3 mL), Intermediate CD (450 mg, 1.84 mmol), CuI (233 mg, 1.23 mmol), trans-N,N'-dimethyl-1,2-cyclohexanediamine (174 mg, 1.22 mmol) and CsF (372 mg, 2.45 mmol) were added. The reaction mixture was stirred at 85 °C overnight under $N_2$ atmosphere and concentrated under reduced pressure. The residue was purified by prep-HPLC. The solvent was removed under reduced pressure to give methyl 1-(1-methyl-1,2,3,6-tetrahydropyridin-4-yl)-6-oxo-1,6-dihydropyridazine-3-carboxylate (173.6 mg, 56.9 % yield). LC/MS m/z = 250.1 $[M+H]^+$.

Steps 2 and 3: Synthesis of -N-[(1R)-1-[3-amino-5-(trifluoromethyl)phenyl]ethyl]-1-(1-methyl-1,2,3,6-tetrahydropyridin-4-yl)-6-oxo-1,6-dihydropyridazine-3-carboxamide

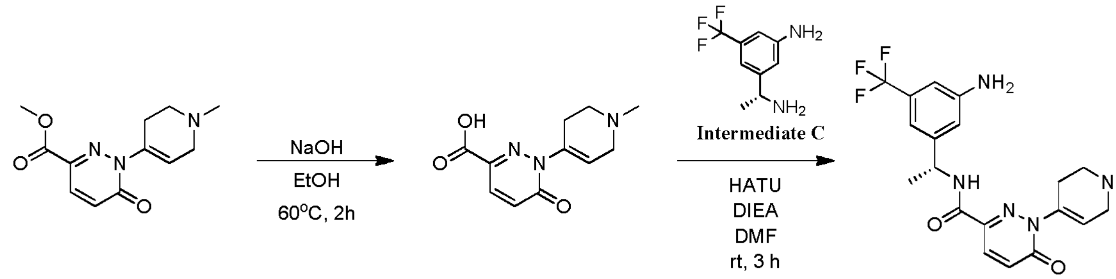


126

The compound of Example 126 (31.1 mg, 6.4% yield) was obtained in the same manner as in Example 64. $^1$H NMR (400 MHz, MeOD) δ 7.93 (d, J = 9.7 Hz, 1H), 7.04 (d, J = 9.7 Hz, 1H), 6.90 (d, J = 7.7 Hz, 2H), 6.81 (s, 1H), 5.13 (q, J = 7.0 Hz, 1H), 3.26 (d, J = 3.3 Hz, 2H), 2.84 (t, J = 5.8 Hz, 2H), 2.47 (s, 3H), 1.55 (d, J = 7.1 Hz, 3H); LC/MS m/z = 422.2 [M+H]$^+$.

**Example 127 and Example 128**

The compounds shown in the following table were prepared in the same manner as in Example 126 by replacing Intermediate CD with appropriate intermediates corresponding to the respective structures of the desired compounds.

[Table 10]

| No. | Structure/Name | Spectral Data |
|---|---|---|
| 127 | 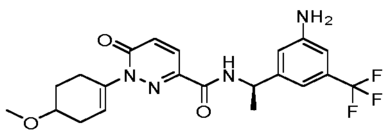 N-[(1R)-1-[3-amino-5-(trifluoromethyl)phenyl]ethyl]-1-(4-methoxycyclohex-1-en-1-yl)-6-oxo-1,6-dihydropyridazine-3-carboxamide | $^1$H NMR (400 MHz, DMSO) δ 8.83 (d, J = 8.2Hz, 1H), 7.80 (d, J = 9.7 Hz, 1H), 7.01 (d, J = 9.7 Hz, 1H), 6.85 - 6.77 (m, 2H), 6.71 (d, J = 1.9 Hz, 1H), 6.00 - 5.93 (m, 1H), 5.57 (s, 2H), 5.04 (t, J = 7.5 Hz, 1H), 3.54 (d, J = 7.4 Hz, 1H), 2.45 (s, 2H), 2.20 - 2.11 (m, 1H), 1.98 (m, 1H), 1.74 (m, 1H), 1.47 (d, J = 7.1 Hz, 3H); LC/MS (ESI) m/z = 437.1 [M+H]$^+$ |
| 128 | 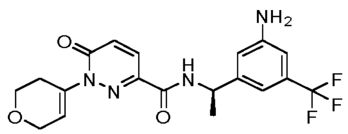 N-[(1R)-1-[3-amino-5-(trifluoromethyl)phenyl]ethyl] -1-(3,6-dihydro-2H-pyran-4-yl)-6-oxo-1,6-dihydropyridazine-3-carboxamide | $^1$H NMR (400 MHz, DMSO) δ 8.83 (d, J = 8.3 Hz, 1H), 7.81 (d, J = 9.7 Hz, 1H), 7.04 (d, J = 9.7 Hz, 1H), 6.81 (d, J = 17.5 Hz, 2H), 6.71 (s, 1H), 6.31 (s, 1H), 5.57 (s, 2H), 5.08 - 5.00 (m, 1H), 4.28 (d, J = 2.9 Hz, 2H), 3.85 (t, J = 5.5 Hz, 2H), 2.54 (s, 2H), 1.97 (m, 1H), 1.47 (d, J = 7.1 Hz, 3H); LC/MS (ESI) m/z = 409.1 [M+H]$^+$ |

**Example 129: N-[(1R)-1-[3-amino-5-(trifluoromethyl)phenyl]ethyl]-1-(3-acetamidophenyl)-6-oxo-1,6-dihydropyridine-3-carboxamide**

Step 1: Synthesis of ethyl 1-(3-acetamidophenyl)-6-oxo-1,6-dihydropyridine-3-carboxylate

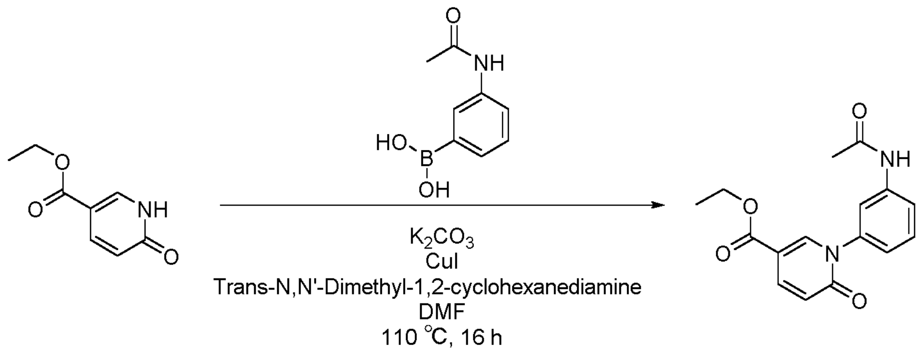


To a solution of ethyl 6-oxo-1,6-dihydropyridine-3-carboxylate (100 mg, 0.60 mmol), (3-acetamidophenyl)boronic acid (154 mg, 0.72 mmol), potassium carbonate (165 mg, 1.20 mmol) and copper(I) iodide (114 mg, 0.60 mmol) in DMF (1.1 mL) was added trans-N,N'-dimethyl-1,2-cyclohexanediamine (95 μL, 0.60 mmol). The reaction mixture was heated at 110 °C for 16 hours. The mixture was quenched and extracted with DCM. The organic layer was dried over anhydrous $Na_2SO_4$ and concentrated under reduced pressure to obtain a crude product. The residue was purified by flash chromatography (0-5 % MeOH in DCM) to give ethyl 1-(3-acetamidophenyl)-6-oxo-1,6-dihydropyridine-3-carboxylate (117.7 mg, 65.5 % yield). LC/MS m/z = 301.1 [M+H]+.

Steps 2 and 3: Synthesis of -N-[(1R)-1-[3-amino-5-(trifluoromethyl)phenyl]ethyl]-1-(1 methyl-12,3,6-tetrahydropyridin-4-yl)-6-oxo-1,6-dihydropyridazine-3-carboxamide

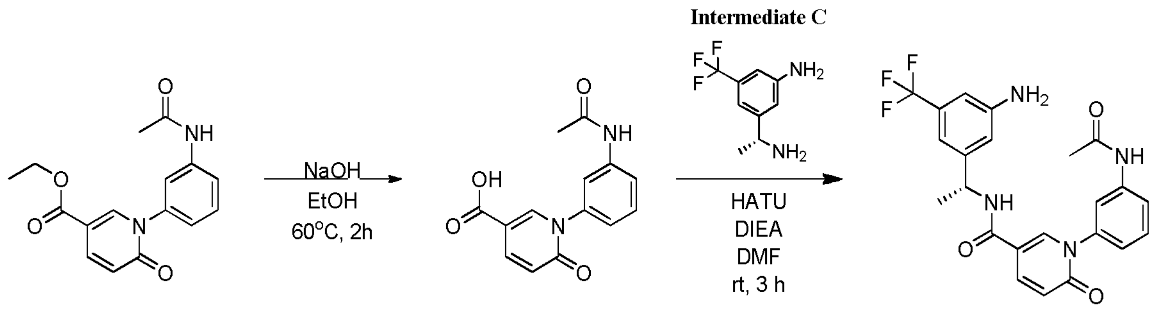

**129**

The compound of Example 129 (2.5 mg, 1.9% yield) was obtained in the same manner as in Example 64. $^1H$ NMR (400 MHz, MeOD) δ 8.28 (d, J = 2.6 Hz, 1H), 8.04 (dd, J = 9.6, 2.7 Hz, 1H), 7.78 (t, J = 2.1 Hz, 1H), 7.60 (ddd, J = 8.2, 2.0, 1.0 Hz, 1H), 7.48 (t, J = 8.1 Hz, 1H), 7.16 (ddd, J = 7.9, 2.1, 1.0 Hz, 1H), 6.90 - 6.85 (m, 2H), 6.80 (d, J = 2.0 Hz, 1H), 6.63 (d, J = 9.6 Hz, 1H), 5.10 (q, J = 7.0 Hz, 1H), 2.14 (s, 3H), 1.50 (d, J = 7.1 Hz, 3H); LC/MS m/z = 459.2 [M+H]+.

**Example 130: N-[(1R)-1-[3-amino-5-(trifluoromethyl)phenyl]ethyl]-1-(3-cyclopropaneamidophenyl)-6-oxo-1,6-dihydropyridine-3-carboxamide**

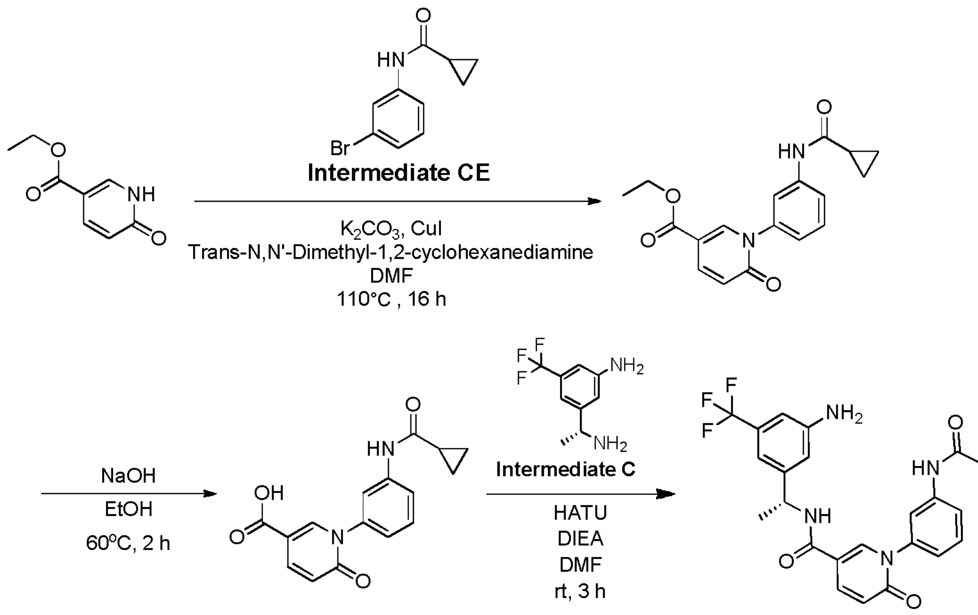


130

The compound of Example 130 (11.2 mg, 45.1%) was obtained in the same manner as in Example 129. $^1H$ NMR (400 MHz, MeOD) $\delta$ 8.29 (d, J = 2.5 Hz, 1H), 8.04 (dd, J = 9.6, 2.6 Hz, 1H), 7.80 (s, 1H), 7.60 (d, J = 8.2 Hz, 1H), 7.48 (t, J = 8.1 Hz, 1H), 7.15 (d, J = 7.8 Hz, 1H), 6.97 (d, J = 12.2 Hz, 2H), 6.88 (s, 1H), 6.64 (d, J = 9.6 Hz, 1H), 5.14 - 5.08 (m, 1H), 1.51 (d, J = 7.0 Hz, 3H), 0.98 - 0.83 (m, 3H); LC/MS m/z = 485.2 $[M+H]^+$.

**Example 131: N-[(1R)-1-[3-amino-5-(trifluoromethyl)phenyl]ethyl]-1-(3-cyclopropaneamidophenyl)-6-oxo-1,6-dihydropyridine-3-carboxamide**

Steps 1 to 3: Synthesis of 1-(2-acetamido-4-pyridyl)-N-[(IR)-1-[3-nitro-5-(trifluoromethyl)phenyl]ethyl]-6-oxo-pyridine-3-carboxamide

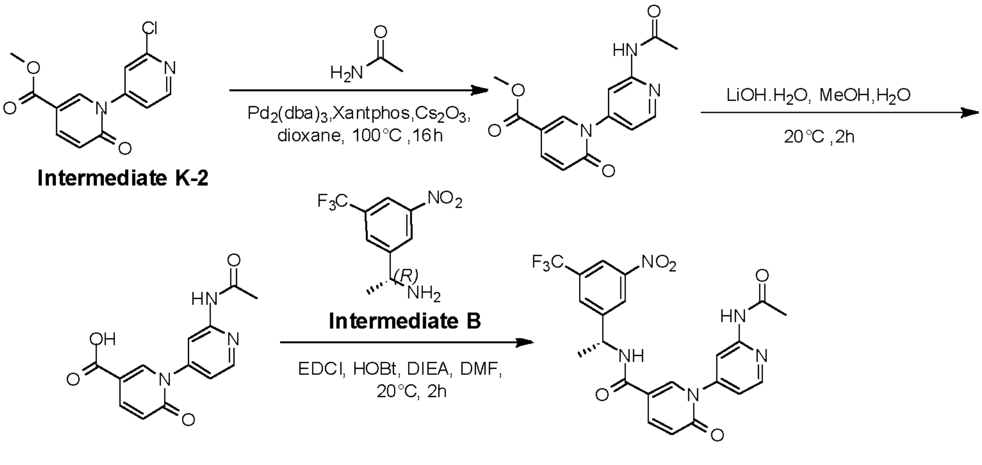


1-(2-acetamido-4-pyridyl)-N-[(1R)-1-[3-nitro-5-(trifluoromethyl)phenyl]ethyl]-6-oxo-pyridine-3-carboxamide (36 mg, 50% yield) was obtained as a white solid in the same manner as in Steps 1 to 3 of Example 79. $^1H$ NMR (400 MHz, DMSO-$d_6$) $\delta$ = 10.65 (s, 1H), 9.18 (br d, *J*= 7.6 Hz, 1H), 8.72 (s, 1H), 8.58 (s, 1H), 8.41 - 8.33 (m, 2H), 8.30 - 8.25 (m, 2H), 7.86 (s, 1H), 7.29 (d, *J* = 8.4 Hz, 1H), 6.94 - 6.90 (m, 1H),5.44 - 5.36 (m, 1H), 2.07 (s, 3H), 1.55 (d, *J*= 7.2 Hz, 3H).

Step 4: Synthesis of 1-(2-acetamido-4-pyridyl)-N-[(1R)-1-[3-amino-5-(trifluoromethyl)phenyl]ethyl]-6-oxo-pyridine-3-carboxamide

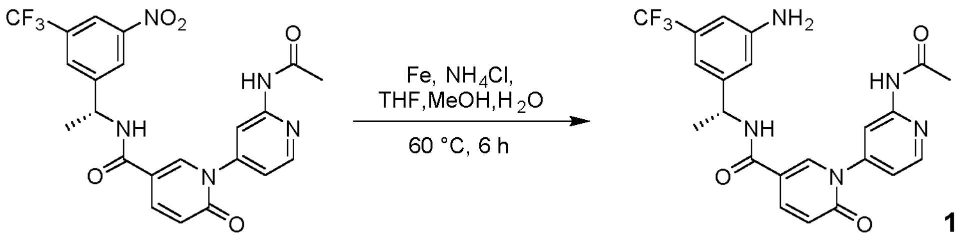


131

The compound of Example 131 (8.79 mg, 30% yield) was obtained as a white solid in the same manner as in Step 4 of Example 89. $^1$H NMR (400 MHz, DMSO-$d_6$) δ = 10.66 (s, 1H), 8.98 (d, *J* = 7.6 Hz, 1H), 8.83 - 8.63 (m, 1H), 8.47 - 8.21 (m, 2H), 7.85 (s, 1H), 7.37 - 7.19 (m, 1H), 7.01 - 6.87 (m, 2H), 6.83 - 6.69 (m, 2H), 5.64 - 5.51 (m, 1H), 5.07 (d, *J* = 7.6, 14.4 Hz, 1H), 2.07 (s, 3H), 1.45 (d, *J* = 7.6 Hz, 3H); MS (EI) m/z: 460.3 [M+H]$^+$.

**Example 132: N-[(1R)-1-[3-amino-5-(trifluoromethyl)phenyl]ethyl]-6-oxo-1-(3-tetrahydrofuran-2-ylphenyl)pyridine-3-carboxamide**

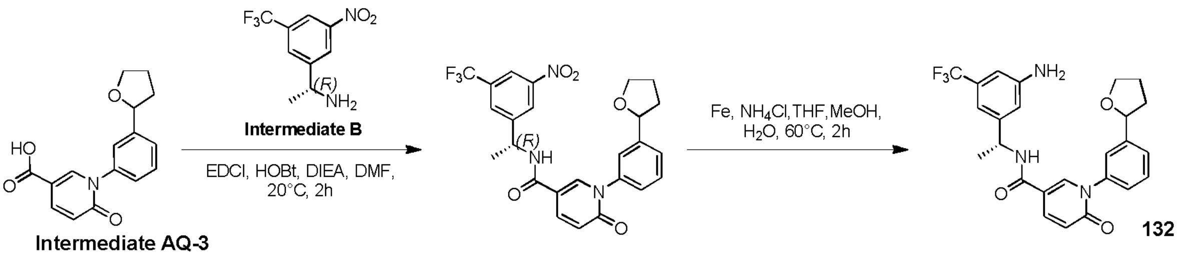


132

The compound of Example 132 (19.68 mg, 42% yield) was obtained as a white solid in the same manner as in Steps 3 and 4 of Example 89. $^1$H NMR (400 MHz, DMSO-$d_6$) δ = 8.58 (d, *J* = 7.6 Hz, 1H), 8.41 - 8.31 (m, 1H), 8.01 - 7.89 (m, 1H), 7.56 - 7.31 (m, 4H), 6.96 - 6.67 (m, 3H), 6.57 - 6.47 (m, 1H), 5.61 - 5.50 (m, 1H), 5.10 - 4.97 (m, 1H), 4.92 - 4.82 (m, 1H), 4.05 - 3.93 (m, 1H), 3.87 - 3.76 (m, 1H), 2.39 - 2.31 (m, 1H), 1.95 (d, *J*= 7.2 Hz, 2H), 1.76 - 1.63 (m, 1H), 1.39 (d, *J*= 7.2 Hz, 3H); MS (EI) m/z: 472.1 [M+H]$^+$.

**Example 133: N-[(1R)-1-[3-amino-5-(trifluoromethyl)phenyl]ethyl]-6-oxo-1-[3-(2-oxopiperazin-1-yl)phenyl]pyridine-3-carboxamide**

Steps 1 to 3: Synthesis of tert-butyl 4-[3-[5-[[(1R)-1-[3-nitro-5-(trifluoromethyl)phenyl]ethyl]carbamoyl]-2-oxo-1-pyridyl]phenyl]-3-oxo-piperazine-1-carboxylate

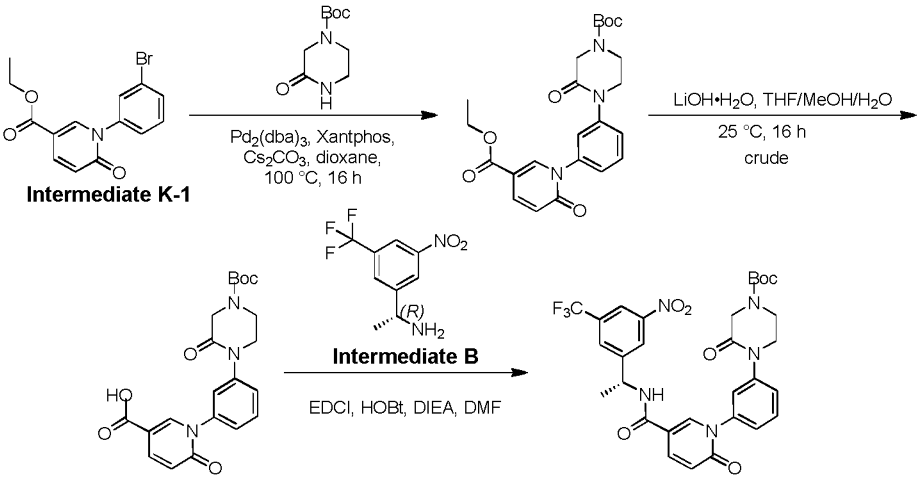


Tert-butyl 4-[3-[5-[[(1R)-1-[3-nitro-5-(trifluoromethyl)phenyl]ethyl]carbamoyl]-2-oxo-1-pyridyl]phenyl]-3-oxo-piperazine-1-carboxylate (70 mg, 44% yield) was obtained as a light yellow solid in the same manner as in Steps 1 to 3 of Example 79. $^1$H NMR (400 MHz, CDCl$_3$) $\delta$ = 8.33 (d, $J$ = 16.0 Hz, 2H), 8.05 (d, $J$ = 2.0 Hz, 1H), 7.88 (s, 1H), 7.76 - 7.69 (m, 1H), 7.47 - 7.39 (m, 1H), 7.35 (s, 1H), 7.31 - 7.26 (m, 1H), 7.20 (s, 1H), 7.19 - 7.17 (m, 1H), 6.47 (d, $J$ = 9.6 Hz, 1H), 5.32 - 5.20 (m, 1H), 4.16 (s, 2H), 3.72 (s, 4H), 1.50 (d, $J$ = 6.4 Hz, 3H), 1.43 (s, 9H); MS (EI) m/z: 630.3 [M+H]$^+$.

Step 4: Synthesis of tert-butyl 4-[3-[5-[[(1R)-1-[3-amino-5-(trifluoromethyl)phenyl]ethyl]carbamoyl]-2-oxo-1-pyridyl]phenyl]-3-oxo-piperazine-1-carboxylate

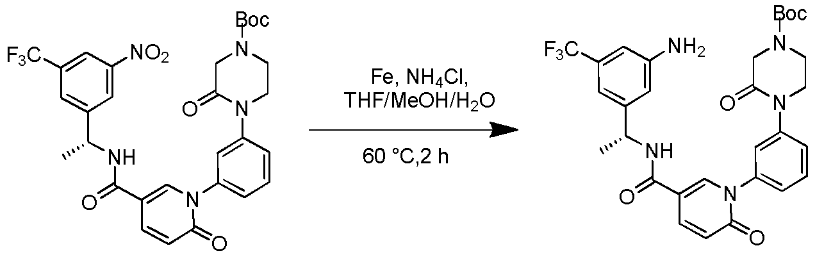


Tert-butyl 4-[3-[5-[[(1R)-1-[3-amino-5-(trifluoromethyl)phenyl]ethyl]carbamoyl]-2-oxo-1-pyridyl]phenyl]-3-oxo-piperazine-1-carboxylate (60 mg, 86% yield) was obtained as a yellow solid in the same manner as in Step 4 of Example 89. $^1$H NMR (400 MHz, CDCl$_3$) $\delta$ = 8.07 (s, 1H), 7.77 (d, $J$ = 8.0 Hz, 1H), 7.58 - 7.47 (m, 1H), 7.40 (s, 1H), 7.34 (d, $J$ = 7.2 Hz, 1H), 7.29 (s, 1H), 6.97 (s, 1H), 6.82 ( d, $J$ = 15.2 Hz, 2H), 6.58 (d, $J$ = 9.6 Hz, 1H), 5.19 (t, $J$ = 7.2 Hz, 1H), 4.23 (s, 2H), 3.78 (s, 4H), 1.53 (s, 3H), 1.51 (s, 9H).

Step 5: Synthesis of N-[(1R)-1-[3-amino-5-(trifluoromethyl)phenyl]ethyl]-6-oxo-1-[3-(2-oxopiperazin-1-yl)phenyllpyridine-3-carboxamide

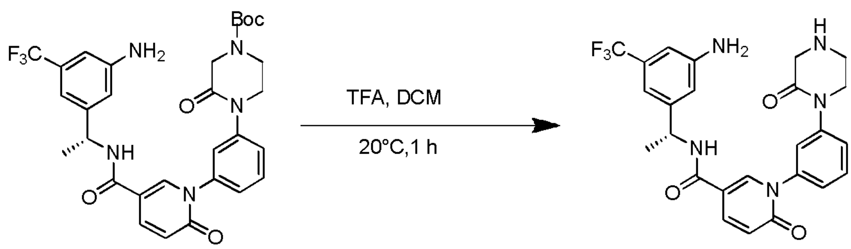


133

The compound of Example 133 (5.06 mg, 19% yield) was obtained as an off-white solid in the same manner as in Step 5 of Example 79. $^1$H NMR (400 MHz, DMSO-$d_6$) $\delta$ = 8.61 (d, $J$ = 7.6 Hz, 1H), 8.37 (d, $J$ = 2.4 Hz, 1H), 8.19 - 8.13 (m, 1H), 7.96 (dd, $J$ = 2.4, 9.6 Hz, 1H), 7.59 - 7.53 (m, 1H), 7.52 - 7.45 (m, 2H), 7.37 (d, $J$ = 8.0 Hz, 1H), 6.76 (s, 2H), 6.70 (s, 1H), 6.54 (d, $J$ = 9.6 Hz, 1H), 5.55 (s, 2H), 5.04 - 4.97 (m, 1H), 3.66 (t, $J$ = 5.4 Hz, 2H), 3.42 (s, 2H), 3.04 (t, $J$ = 5.3 Hz, 2H), 1.39 (d, $J$ = 7.2 Hz, 3H); MS (EI) m/z: 500.2 [M+H]$^+$.

**Example 134: N-[(1R)-1-[3-amino-5-(trifluoromethyl)phenyl]ethyl]-1-[3-[(3-methyloxetan-3-yl)amino]phenyl]-6-oxo-pyridine-3-carboxamide**

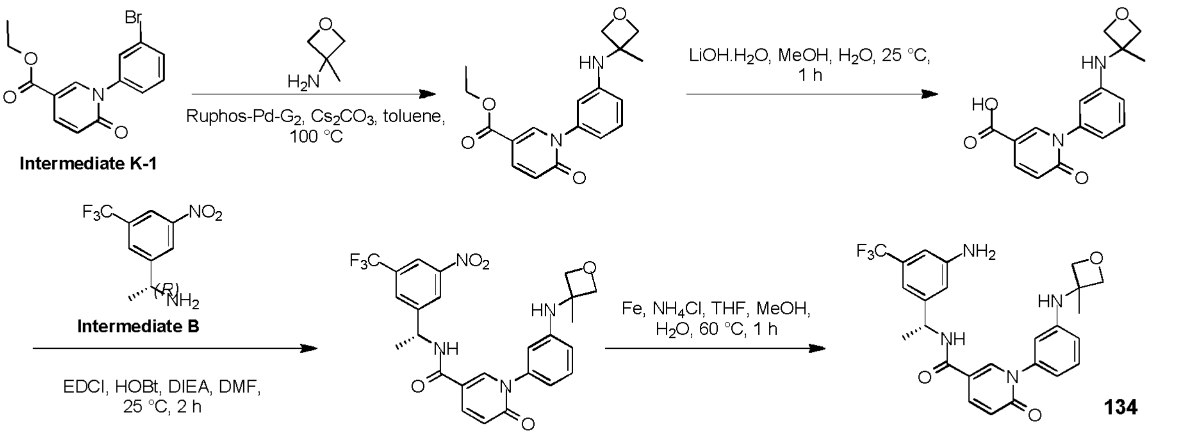


The compound of Example 134 (25.4 mg, 88% yield) was obtained as a white solid in a similar manner to Example 90, except that 3-methyloxetan-3-amine was used as a starting material and Step 4 of Example 90 was omitted. $^1$H NMR (400 MHz, DMSO-$d_6$) $\delta$ =8.69 - 8.50 (m, 1H), 8.39 - 8.32 (m, 1H), 7.96 - 7.90 (m, 1H),7.28 - 7.20 (m, 1H), 6.75 (s, 1H), 6.69 (s, 1H), 6.68 - 6.50 (m, 1H), 6.49 (d, $J$ = 9.6 Hz, 1H), 6.46 (s, 1H), 6.43 - 6.38 (m, 2H), 5.60 - 5.47 (m, 2H), 5.08 - 4.94 (m, 1H), 4.60 (d, $J$ = 5.6 Hz, 2H), 4.48 (d, $J$ = 5.6 Hz, 2H), 1.57 (s, 3H), 1.39 (d, $J$ = 7.2 Hz, 3H); MS (EI) m/z: 487.2 [M+H]$^+$.

**Example 135: N-[(1R)-1-[3-amino-5-(trifluoromethyl)phenyl]ethyl]-1-[3-[(3-methyloxetan-3-yl)amino]phenyl]-6-oxo-pyridine-3-carboxamide**

Step 1: Synthesis of ethyl 6-oxo-1-[3-(2-trimethylsilylethynyl)phenyl]pyridine-3-carboxylate

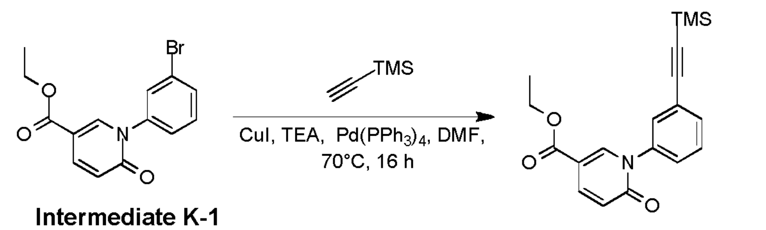


**Intermediate K-1**

A solution of Intermediate K-1 (400 mg, 1.24 mmol), CuI (23.6 mg, 124 $\mu$mol), TEA (37.6 mg, 372 $\mu$mol), $Pd(PPh_3)_4$ (143 mg, 124 $\mu$mol) and ethynyl(trimethyl)silane (182 mg, 1.86 mmol) in DMF (8 mL) was stirred at 70°C for 16 hours under $N_2$ atmosphere. The reaction mixture was quenched with water (10 mL) and extracted with PE (3 $\times$ 50 mL). The combined organic layer was washed with saturated NaCl solution (2 $\times$ 50 mL) and concentrated in vacuo. The residue was purified by column chromatography ($SiO_2$, petroleum ether/ethyl acetate = 1/0 to 3/1) to give ethyl 6-oxo-1-[3-(2-trimethylsilylethynyl)phenyl]pyridine-3-carboxylate (400 mg, 94% yield) as yellow oil. $^1$H NMR (400 MHz, DMSO-$d_6$) $\delta$ = 8.25 - 8.28 (m, 1H), 7.85 - 7.90 (m, 1H), 7.44 - 7.59 (m, 4H), 6.53 - 6.57 (m, 1H), 4.25 (q, $J$ = 7.2 Hz, 2H), 1.27 (t, $J$ = 7.2 Hz, 3H), 0.23 (s, 9H).

Step 2: Synthesis of ethyl 1-(3-ethynylphenyl)-6-oxo-pyridine-3-carboxylate

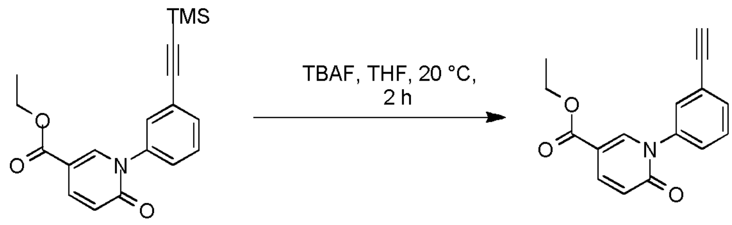


To a solution of ethyl 6-oxo-1-[3-(2-trimethylsilylethynyl)phenyl]pyridine-3-carboxylate (480 mg, 1.41 mmol) in THF (10 mL) was added TBAF (1 M, 3.54 mL). The reaction mixture was stirred at 20°C for 2 hours. The reaction mixture was filtered, and the filtrate was concentrated in vacuo. The residue was purified by column chromatography ($SiO_2$, petroleum ether/ethyl acetate = 1/0 to 3/1) to give ethyl 1-(3-ethynylphenyl)-6-oxo-pyridine-3-carboxylate (260 mg, 68% yield) as a yellow solid. $^1$H NMR (400 MHz, DMSO-$d_6$) δ = 8.26 - 8.29 (m, 1H), 7.80 - 7.98 (m, 1H), 7.69 - 7.46 (m, 4H), 6.56 (d, *J* = 9.6 Hz, 1H), 4.34 (s, 1H), 4.20 - 4.30 (m, 2H), 1.20 - 1.34 (m, 3H).

Step 3: Synthesis of ethyl 6-oxo-1-[3-(1H-triazol-5-yl)phenyl]pyridine-3-carboxylate

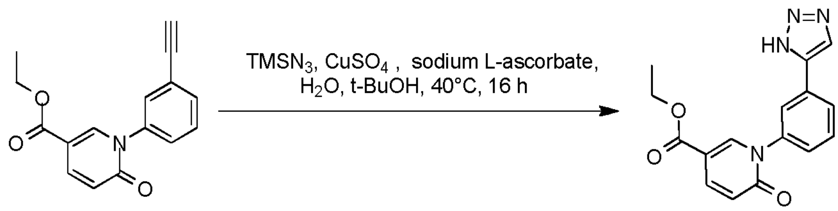


A solution of ethyl 1-(3-ethynylphenyl)-6-oxo-pyridine-3-carboxylate (200 mg, 748 μmol), $TMSN_3$ (344 mg, 2.99 mmol), sodium;(2R)-2-[(1S)-1,2-dihydroxyethyl]-4-hydroxy-5-oxo-2H-furan-3-olate (59.3 mg, 299 μmol) and $CuSO_4$ (23.8 mg, 149 μmol) in a mixture of t-BuOH (3 mL) and $H_2O$ (3 mL) was stirred at 40 °C for 16 hours. The reaction mixture was concentrated in vacuo. The residue was purified by reversed-phase HPLC (0.1% FA condition) to give ethyl 6-oxo-1-[3-(1H-triazol-5-yl)phenyl]pyridine-3-carboxylate (40 mg, 17 % yield) as a white solid. $^1$H NMR (400 MHz, DMSO-$d_6$) δ = 8.43 (s, 1H), 8.32 (d, *J* = 2.4 Hz, 1H), 7.99 (d, *J* = 8.4 Hz, 1H), 7.87 - 7.96 (m, 2H), 7.60 - 7.64 (m, 1H), 7.40 - 7.48 (m, 1H), 6.58 (d, *J* = 9.6 Hz, 1H), 4.30 - 4.20 (m, 2H), 1.32 - 1.20 (m, 3H).

Steps 4 to 6: Synthesis of N-[(1R)-1-[3-amino-5-(trifluoromethyl)phenyl]ethyl]-6-oxo-1-[3-(1H-triazol-5-yl)phenyl]pyridine-3-carboxamide

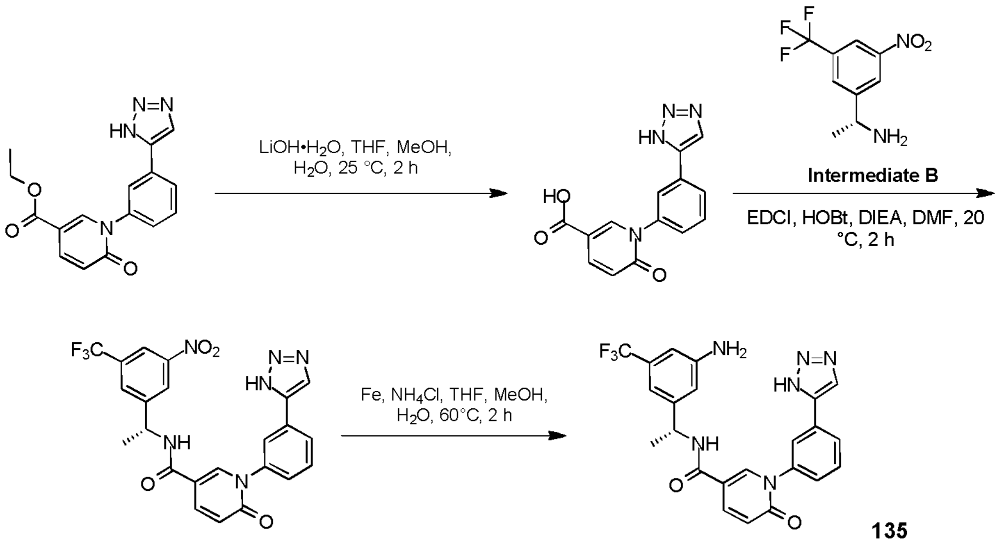


135

The compound of Example 135 (1.31 mg, 12% yield) was obtained as a white solid in the same manner as in Steps 2 to 4 of Example 89. $^1$H NMR (400 MHz, $CDCl_3$) δ = 8.40 - 8.58 (m, 1H), 8.35 (d, *J* = 2.4 Hz, 1H), 8.20 - 8.30 (m, 1H), 8.00 - 8.14 (m, 1H), 8.03 - 8.00 (m, 1H), 7.96 (d, *J* = 1.6 Hz, 1H), 7.67 - 7.63 (m, 1H), 7.49 - 7.45 (m, 1H), 7.36 - 7.32 (m, 1H), 6.89 (d, *J* = 2.4 Hz, 2H), 6.80 (s, 1H), 6.68 (d, *J* = 9.6 Hz, 1H), 5.07 - 5.18 (m, 1H), 1.51 (d, *J* = 7.6 Hz, 3H); MS (EI) m/z: 469.3 $[M+H]^+$.

**Example 136: 1-[3-[acetyl(methyl)amino]phenyl]-N-[(1R)-1-[3-amino-5-(trifluoromethyl)phenyl]ethyl]-6-oxo-pyridine-3-carboxamide**

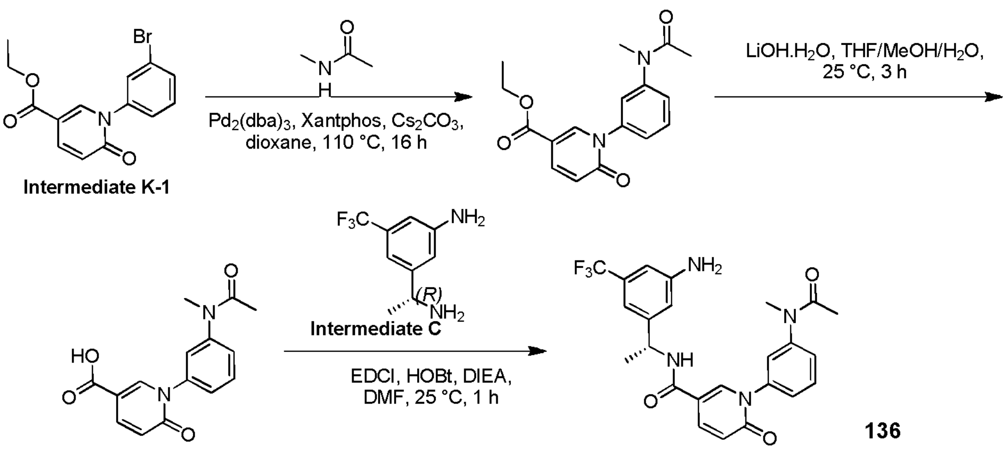


136

The compound of Example 136 (13.3 mg, 13% yield) was obtained as an off-white solid in the same manner as in Steps 1 to 3 of Example 79. $^1$H NMR (400 MHz, DMSO-$d_6$) δ = 8.60 (d, *J* = 7.6 Hz, 1H), 8.36 (d, *J* = 2.4 Hz, 1H), 7.96 (dd, *J* = 2.4, 9.6 Hz, 1H), 7.65 - 7.40 (m, 4H), 6.78 (s, 2H), 6.72 (s, 1H), 6.55 (d, *J*= 9.6 Hz, 1H), 5.07 - 4.96 (m, 1H), 3.20 (s, 3H), 1.87 (s, 3H), 1.40 (d, *J* = 7.2 Hz, 3H); MS (EI) m/z: 473.2 $[M+H]^+$.

**Example 137: N-[(1R)-1-[5-[5-chloro-2-(methylaminomethyl)phenyl]thiazol-2-yl]ethyl]-1-(2-fluorophenyl)-6-oxo-pyridazine-3-carboxamide**

Step 1: Synthesis of N-[(1R)-1-(5-bromothiazol-2-yl)ethyl]-1-(2-fluorophenyl)-6-oxo-pyridazine-3-carboxamide

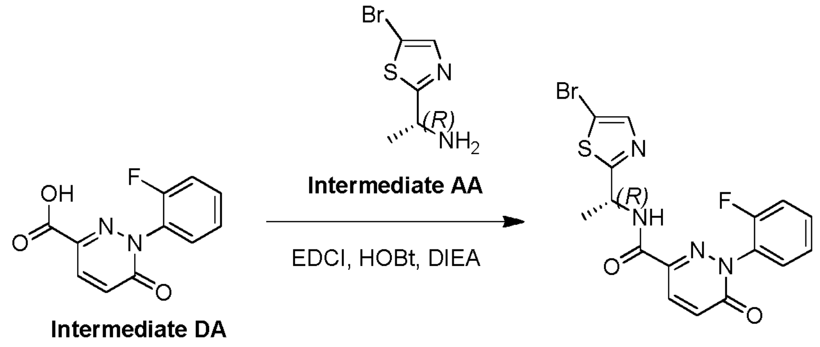


To a solution of Intermediate DA (48.1 mg, 205 μmol) in DMF (1 mL), DIPEA (79.6 mg, 616 μmol), EDCI (47.2 mg, 246 μmol) and HOBt (33.3 mg, 246 μmol) were added. The mixture was stirred at 20 °C for 10 min. Then, the mixture was added with Intermediate AA (50 mg, 205 μmol, HCl) and stirred at 20 °C for 15 hours 50 min. The reaction mixture was diluted with $H_2O$ (10 mL) and extracted with EtOAc (3 × 5 mL). The combined organic layer was washed with $H_2O$ (3 × 5 mL), dried over $Na_2SO_4$, filtered and concentrated under reduced pressure to obtain a residue. The residue was purified by Prep-TLC ($SiO_2$, PE: EtOAc = 1: 3) and reversed-phase HPLC (0.1% FA condition) to give N-[(1R)-1-(5-bromothiazol-2-yl)ethyl]-1-(2-fluorophenyl)-6-oxo-pyridazine-3-carboxamide (50 mg, 57% yield) as a light yellow solid. $^1H$ NMR (400 MHz, $CDCl_3$) δ = 8.07 (d, *J* = 9.6 Hz, 1H), 7.60 (s, 1H), 7.55 - 7.44 (m, 3H), 7.36 - 7.28 (m, 2H), 7.14 (d, *J* = 9.6 Hz, 1H), 5.52 - 5.43 (m, 1H), 1.69 (d, *J* = 7.2 Hz, 3H); MS (EI) m/z: 425.0 $[M+H]^+$.

Step 2: Synthesis of N-[(1R)-1-[5-(5-chloro-2-formyl-phenyl)thiazol-2-yl]ethyl]-1-(2-fluorophenyl)-6-oxo-pyridazine-3-carboxamide

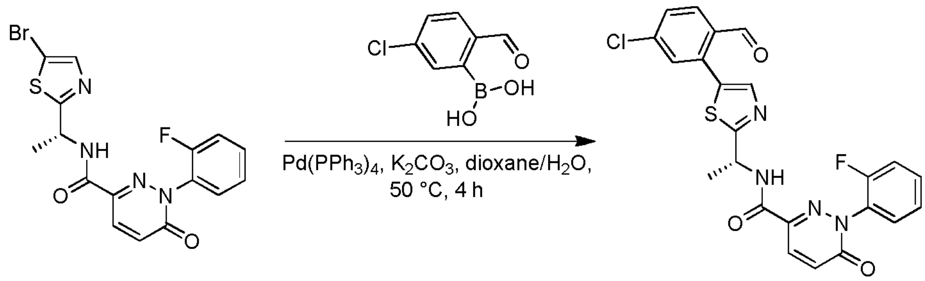


A mixture of N-[(1R)-1-(5-bromothiazol-2-yl)ethyl]-1-(2-fluorophenyl)-6-oxo-pyridazine-3-carboxamide (30 mg, 70.9 μmol), (5-chloro-2-formyl-phenyl)boronic acid (11.8 mg, 63.8 μmol), $K_2CO_3$ (29.4 mg, 213 μmol) and $Pd(PPh_3)_4$ (8.19 mg, 7.09 μmol) in dioxane (1.5 mL) and $H_2O$ (0.5 mL) was degassed and purged with $N_2$ for 3 times, and then the mixture was stirred at 50 °C for 4 hours under $N_2$ atmosphere. The reaction mixture was diluted with $H_2O$ (10 mL) and extracted with EtOAc (3 × 5 mL). The combined organic layer was washed with $H_2O$ (3 × 5 mL), dried over $Na_2SO_4$, filtered and concentrated under reduced pressure to obtain a residue. The residue was purified by Prep-TLC ($SiO_2$, PE: EtOAc = 1: 1) to give N-[(1R)-1-[5-(5-chloro-2-formyl-phenyl)thiazol-2-yl]ethyl]-1-(2-fluorophenyl)-6-oxo-pyridazine-3-carboxamide (30 mg, 84% yield) as a light yellow solid. $^1H$ NMR (400 MHz, $CDCl_3$) δ = 10.11 (s, 1H), 8.09 (d, *J* = 10.0 Hz, 1H), 7.97 (d, *J* = 8.4 Hz, 1H), 7.67 - 7.62 (m, 2H), 7.54 - 7.52 (m, 1H), 7.50 (d, *J* = 1.6 Hz, 1H), 7.49 - 7.47 (m, 2H), 7.39 - 7.29 (m, 2H), 7.17 - 7.13 (m, 1H), 5.66 - 5.55 (m, 1H), 1.78 (d, *J* = 7.2 Hz, 3H); MS (EI) m/z: 483.0 $[M+H]^+$.

Step 3: Synthesis of N-[(1R)-1-[5-[5-chloro-2-(methylaminomethyl)phenyl]thiazol-2-yl]ethyl]-1-(2-fluorophenyl)-6-oxo-pyridazine-3-carboxamide

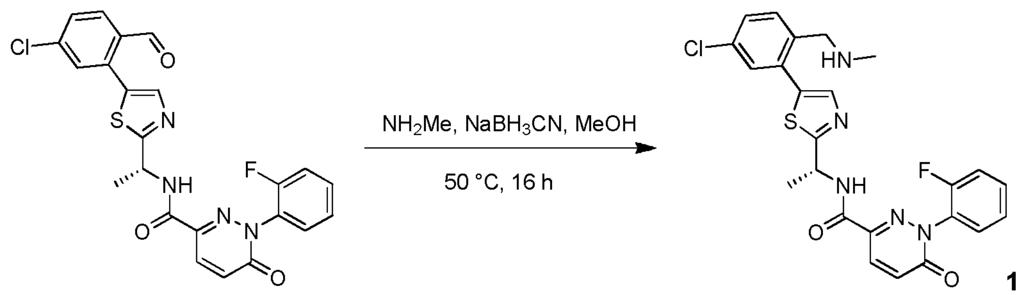


**137**

To a solution of N-[(1R)-1-[5-(5-chloro-2-formyl-phenyl)thiazol-2-yl]ethyl]-1-(2-fluorophenyl)-6-oxo-pyridazine-3-carboxamide (25 mg, 51.8 μmol) in MeOH (0.5 mL), $MeNH_2$ (2 M, 6.76 mL, THF solution) and $NaBH_3CN$ (6.51 mg, 103 μmol) were added. The mixture was stirred at 50 °C for 16 hours. The reaction mixture was concentrated under reduced pressure to obtain a residue. The residue was purified by Prep-HPLC (column: Waters Xbridge 150*25mm* 5μm; mobile phase: [water($NH_4HCO_3$)-ACN];B%: 36%-66%,8min) to give the compound of Example 137 (7.25 mg, 27% yield) as yellow gum. $^1$H NMR (400 MHz, $CDCl_3$) δ = 8.10 (d, *J* = 9.6 Hz, 1H), 7.76 (s, 1H), 7.66 (d, *J* = 8.4 Hz, 1H), 7.55 - 7.46 (m, 2H), 7.44 - 7.40 (m, 1H), 7.38 - 7.28 (m, 4H), 7.14 (d, *J* = 9.6 Hz, 1H), 5.64 - 5.55 (m, 1H), 3.71 (s, 2H), 2.41 (s, 3H), 1.76 (d, *J* = 6.8 Hz, 3H); MS (EI) m/z: 498.1 [M+H]$^+$.

**Example 138: N-[(1R)-1-[5-[5-chloro-2-(methylaminomethyl)phenyl]thiazol-2-yl]ethyl]-1-(2-fluorophenyl)-6-oxo-pyridine-3-carboxamide**

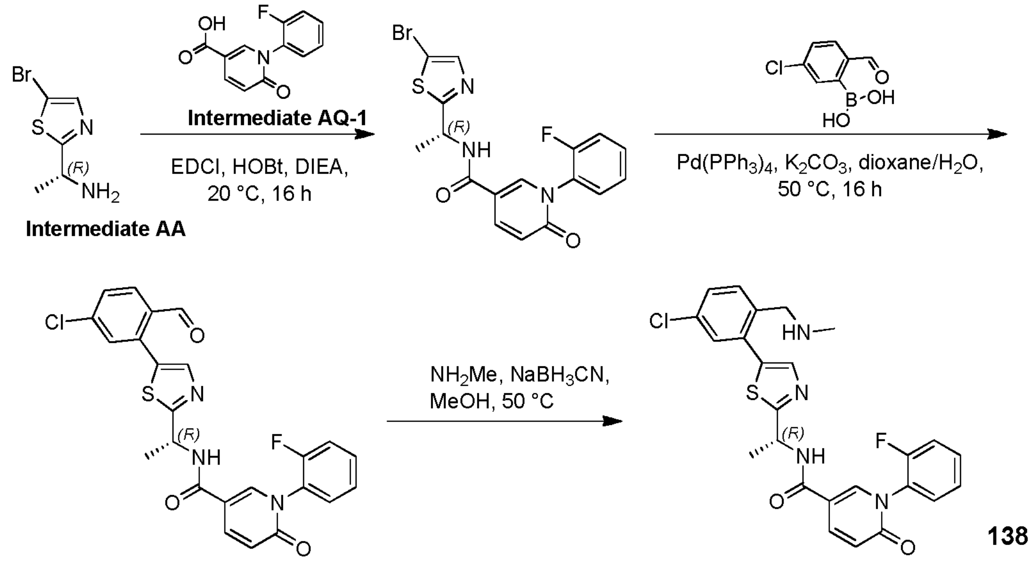


**138**

The compound of Example 138 (7.64 mg, 49% yield) was obtained as yellow gum in the same manner as in Example 137 using Intermediate AQ-1. $^1$H NMR (400 MHz, $CDCl_3$) δ = 8.08 (d, *J* = 2.4 Hz, 1H), 7.82 - 7.78 (m, 1H), 7.76 (s, 1H), 7.51 - 7.44 (m, 1H), 7.42 - 7.38 (m, 1H), 7.38 - 7.28 (m, 4H), 7.26 - 7.24 (m, 1H), 7.03 (d, *J* = 7.6 Hz, 1H), 6.67 (d, *J* = 9.6 Hz, 1H), 5.64 - 5.53 (m, 1H), 3.69 (s, 2H), 2.41 (s, 3H), 1.73 (d, *J* = 6.8 Hz, 3H); MS (EI) m/z: 497.1 [M+H]$^+$.

**Example 139: N-[(1R)-1-[3-amino-5-(trifluoromethyl)phenyl]ethyl]-1-(2-fluoro-3,4-dimethoxy-phenyl)-6-oxo-pyridine-3-carboxamide**

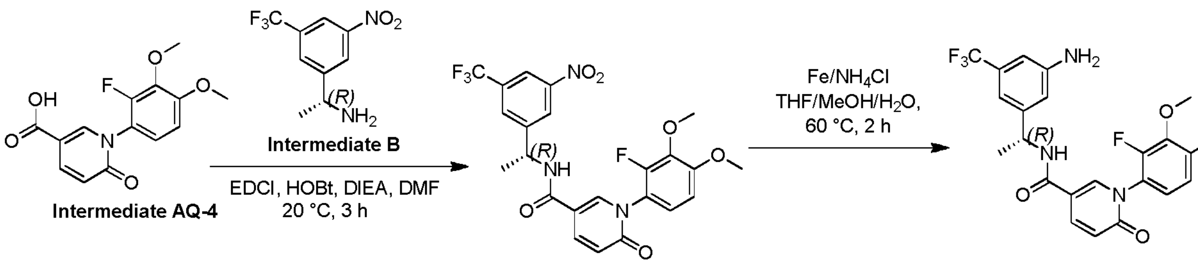

139

The compound of Example 139 (6.97 mg, 28% yield) was obtained as a white solid in the same manner as in Example 132. $^1H$ NMR (400 MHz, DMSO-$d_6$) δ = 8.55 (d, *J* = 7.6 Hz, 1H), 8.31 (d, *J* = 2.4 Hz, 1H), 7.97 (dd, *J* = 2.4, 9.7 Hz, 1H), 7.27 (t, *J* = 8.4 Hz, 1H), 7.05 (dd, *J* = 1.6, 9.2 Hz, 1H), 6.75 (s, 2H), 6.70 (s, 1H), 6.54 (d, *J* = 9.6 Hz, 1H), 5.55 (s, 2H), 5.03 - 4.97 (m, 1H), 3.90 (s, 3H), 3.84 (s, 3H), 1.39 (d, *J* = 7.2 Hz, 3H); MS (EI) m/z: 480.2 $[M+H]^+$.

**Example 140: N-[(1R)-1-[3-(1,1-difluoro-2-hydroxy-ethyl)-2-fluoro-phenyl]ethyl]-1-(2-fluoro-3,4-dimethoxy-phenyl)-6-oxo-pyridine-3-carboxamide**

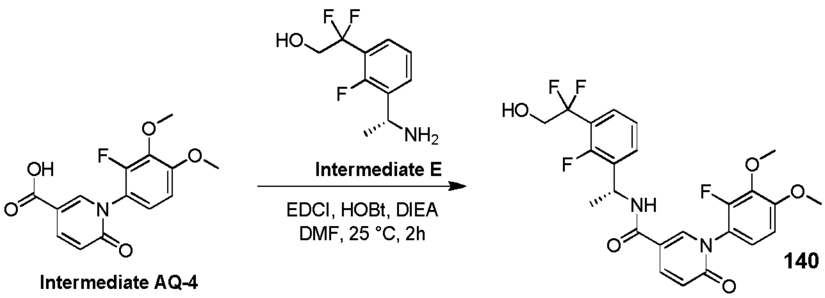

140

The compound of Example 140 (9.12 mg, 14% yield) was obtained as a white solid in the same manner as in Step 1 of Example 139 by replacing Intermediate B with Intermediate E. $^1H$ NMR (400 MHz, DMSO-$d_6$) δ = 8.68 (d, *J* = 7.2 Hz, 1H), 8.37 (d, *J* = 2.4 Hz, 1H), 7.97 (dd, *J* = 2.4, 9.6 Hz, 1H), 7.58 (t, *J* = 6.8 Hz, 1H), 7.44 (t, *J* = 6.8 Hz, 1H), 7.32 - 7.26 (m, 2H), 7.07 (dd, *J* = 1.6, 9.2 Hz, 1H), 6.55 (d, *J* = 9.6 Hz, 1H), 5.73 (t, *J* = 6.4 Hz, 1H), 5.37 - 5.32 (m, 1H), 3.96 - 3.88 (m, 5H), 3.84 (s, 3H), 1.44 (d, *J* = 7.2 Hz, 3H); MS (EI) m/z: 495.2 $[M+H]^+$.

**Example 141: N-[(1R)-1-[3-(1,1-difluoro-2-hydroxy-ethyl)-2-fluorophenyl]ethyl]-1-(2-fluoro-3-pyridyl)-6-oxo-pyridazine-3-carboxamide**

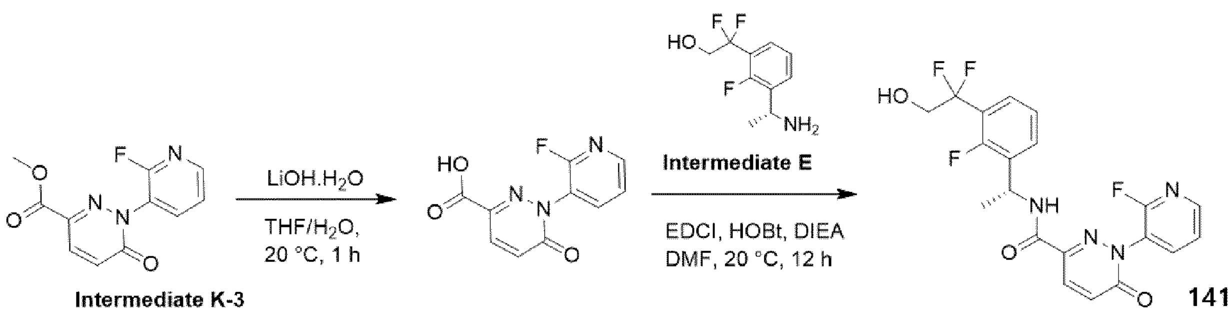

141

The compound of Example 141 (18.8 mg 33.77% yield) was obtained as a white solid in the same manner as in Example 76. $^1H$ NMR (400MHz, DMSO-$d_6$) δ = 9.07 (d, *J* = 8.0 Hz, 1H), 8.42 (d, *J* = 4.4 Hz, 1H), 8.38 - 8.29 (m, 1H), 7.94 (d, *J* = 9.6 Hz, 1H), 7.68 - 7.57 (m, 2H), 7.43 (br t, *J* = 6.8 Hz, 1H), 7.30 - 7.24 (m, 1H), 7.21 (d, *J* = 10.0 Hz, 1H), 6.06 (br s, 1H), 5.40 (quin, *J* = 7.2 Hz, 1H), 3.91 (br t, *J* = 14.4 Hz, 2H), 1.46 (d, *J* = 7.2 Hz, 3H); MS (ESI) m/z = 437.3 $[M+H]^+$.

**Example 142 to Example 214**

The compounds shown in the following table were prepared by using appropriate starting materials and intermediates corresponding to the respective structures of the desired compounds based on Example 141.

[Table 11]

| No. | Structure/Name | Spectral Data |
|---|---|---|
| 142 | 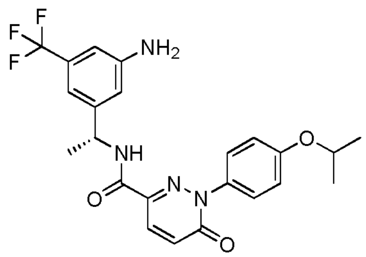 N-[(1R)-1-[3-amino-5-(trifluoromethyl)phenyl] ethyl]-1-(4-isopropoxyphenyl)-6-oxo-pyridazine-3-carboxamide | $^1$H NMR (400MHz, DMSO-$d_6$) $\delta$ = 8.83 (d, *J* = 8.2 Hz, 1H), 7.87 (d, *J* = 9.6 Hz, 1H), 7.60 - 7.51 (m, 2H), 7.11 (d, *J* = 9.6 Hz, 1H), 7.06 - 7.00 (m, 2H), 6.80 (d, *J* = 15.2 Hz, 2H), 6.70 (s, 1H), 5.55 (s, 2H), 5.03 (quin, *J* = 7.2 Hz, 1H), 4.69 (td, *J* = 6.0, 12.1 Hz, 1H), 1.44 (d, *J* = 7.2 Hz, 3H), 1.30 (d, *J* = 6.0 Hz, 6H); MS (ESI) m/z = 461.3 [M +H]$^+$. |
| 143 | 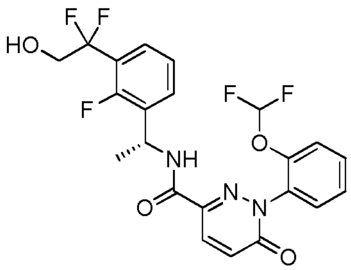 N-[(1R)-1-[3-(1,1-difluoro-2-hydroxy-ethyl)-2-fluoro-phenyl]ethyl]-1-[2-(difluoromethoxy) phenyl]-6-oxo-pyridazine-3- carboxamide | $^1$H NMR (400 MHz, DMSO-$d_6$) $\delta$ = 8.97 (br d, *J* = 8.0 Hz, 1H), 7.93 (d, *J* = 10.0 Hz, 1H), 7.85-7.57 (m, 1H), 7.65 - 7.57 (m, 2H), 7.49 - 7.40 (m, 3H), 7.35 (s, 0.25H), 7.29 - 7.22 (m, 1H), 7.19 (s, 0.5H), 7.17 (d, *J* = 4.0 Hz, 1H), 6.99 (s, 0.25H), 5.71 (s, 1H), 5.40 (quin, *J* = 7.2 Hz, 1H), 3.91 (br t, *J* = 14.4 Hz, 2H), 1.45 (d, *J* = 7.2 Hz, 3H); LC/MS (ESI) m/z: 484.3 [M+H]$^+$ |
| 144 | 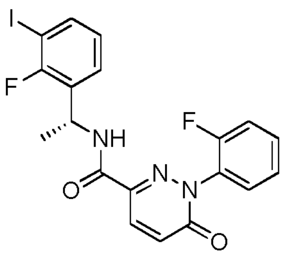 N-[(1R)-1-(2-fluoro-3-iodo-phenyl)ethyl]-1-(2-fluorophenyl)-6-oxo-pyridazine-3-carboxamide | $^1$H NMR (400 MHz, DMSO-$d_6$) $\delta$ = 9.02 (d, *J* = 8.0 Hz, 1H), 7.91 (d, *J* = 10.0 Hz, 1H), 7.75 - 7.64 (m, 2H), 7.64 - 7.55 (m, 1H), 7.49 - 7.39 (m, 3H), 7.17 (d, *J* = 9.8 Hz, 1H), 6.97 (t, *J* = 7.6 Hz, 1H), 5.32 (quin, *J* = 7.2 Hz, 1H), 1.44 (d, *J* = 7.2 Hz, 3H); LC/MS (ESI) m/z: 482.3 [M+H]$^+$ |
| 145 | 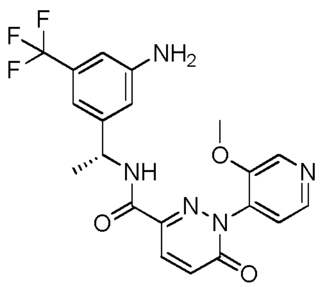 N-[(1R)-1-[3-amino-5-(trifluoromethyl)phenyl] ethyl]-1-(3-methoxy-4-pyridyl)-6-oxo-pyridazine-3-carboxamide | $^1$H NMR (400 MHz, DMSO-$d_6$) $\delta$ = 8.88 (d, *J* = 8.4 Hz, 1H), 8.63 (s, 1H), 8.41 (d, *J* = 4.8 Hz, 1H), 7.92 (d, *J* = 10.0 Hz, 1H), 7.58 (d, *J* = 5.2 Hz, 1H), 7.15 (d, *J* = 10.0 Hz, 1H), 6.78 (br d, *J* = 13.6 Hz, 2H), 6.69 (s, 1H), 5.54 (s, 2H), 5.02 (quin, *J* = 7.2 Hz, 1H), 3.90 (s, 3H), 1.42 (d, *J* = 7.2 Hz, 3H); LC/MS (ESI) m/z: 434.3 [M+H]$^+$ |

(continued)

| No. | Structure/Name | Spectral Data |
|---|---|---|
| 146 | 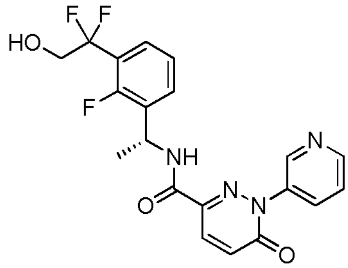 N-[(1R)-1-[3-(1,1-difluoro-2-hydroxy-ethyl)-2-fluoro-phenyl]ethyl]-6-oxo-1-(3- pyridyl) pyridazine-3-carboxamide | $^1$H NMR (400 MHz, DMSO-$d_6$) δ = 9.05 (d, *J* = 8.0 Hz, 1H), 8.97 (d, *J* = 2.4 Hz, 1H), 8.66 (d, *J* = 4.8 Hz, 1H), 8.23 - 8.16 (m, 1H), 7.90 (d, *J* = 9.6 Hz, 1H), 7.69 - 7.57 (m, 2H), 7.43 (t, *J* = 7.2 Hz, 1H), 7.33 -7.25 (m, 1H), 7.18 (d, *J* = 9.6 Hz, 1H), 5.72 (t, *J* = 6.4 Hz, 1H), 5.42 (quin, *J* = 7.2 Hz, 1H), 3.92 (dt, *J* = 6.2, 14.4 Hz, 2H), 1.48 (d, *J* = 7.2 Hz, 3H) ; LC/MS (ESI) m/z: 419.3 $[M+H]^+$ |
| 147 | 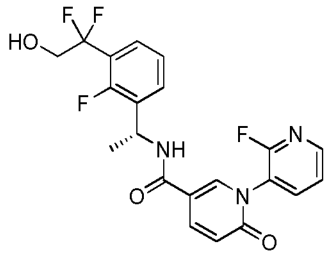 N-[(1R)-1-[3-(1,1-difluoro-2-hydroxy-ethyl)-2-fluoro-phenyl]ethyl]-1-(2-fluoro-3-pyridyl)-6-oxo-pyridine-3-carboxamide | $^1$H NMR (400 MHz, DMSO-$d_6$) δ = 9.03 (d, *J* = 7.6 Hz, 1H), 8.60 (d, *J* = 2.0 Hz, 1H), 8.35 (dd, *J* = 2.4, 8.4 Hz, 1H), 8.14 (td, *J* = 1.6, 4.8 Hz, 1H), 7.98 (ddd, *J* = 1.6, 8.0, 9.6 Hz, 1H), 7.62 (t, *J* = 6.8 Hz, 1H), 7.48 (ddd, *J* = 0.8, 4.8, 7.6 Hz, 1H), 7.43 (t, *J* = 6.8 Hz, 1H), 7.34 - 7.25 (m, 2H), 5.71 (t, *J* = 6.4 Hz, 1H), 5.40 (quin, *J* = 7.2 Hz, 1H), 3.92 (dt, *J* = 6.4, 14.4 Hz, 2H), 1.47 (d, *J* = 7.2 Hz, 3H); LC/MS (ESI) m/z: 436.6 $[M+H]^+$ |
| 148 | 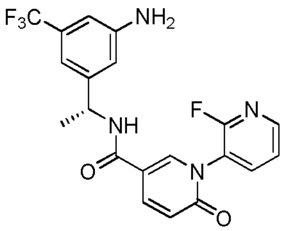 N-[(1R)-1-[3-amino-5-(trifluoromethyl)phenyl] ethyl]-1-(2-fluoro-3-pyridyl)-6-oxo-pyridine-3-carboxamide | $^1$H NMR (400 MHz, DMSO-$d_6$) δ = 8.90 (d, *J* = 7.6 Hz, 1H), 8.59 (d, *J* = 2.0 Hz, 1H), 8.34 (dd, *J* = 2.4, 8.4 Hz, 1H), 8.17 - 8.10 (m, 1H), 7.98 (ddd, *J* = 1.6, 8.0, 9.6 Hz, 1H), 7.52 - 7.44 (m, 1H), 7.31 (d, *J* = 8.4 Hz, 1H), 6.79 (s, 2H), 6.70 (s, 1H), 5.56 (s, 2H), 5.05 (quin, *J* = 7.2 Hz, 1H), 1.43 (d, *J* = 7.2 Hz, 3H) ; LC/MS (ESI) m/z: 421.3 $[M+H]^+$ |
| 149 | 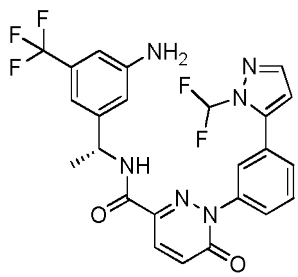 N-[(1R)-1-[3-amino-5-(trifluoromethyl)phenyl] ethyl]-1-[3-[2-(difluoromethyl)pyrazol-3-yl] phenyl]-6-oxo-pyridazine-3-carboxamide | $^1$H NMR (400 MHz, DMSO-$d_6$) δ = 8.90 (d, *J* = 8.4 Hz, 1H), 7.96 (s, 1H), 7.93 - 7.83 (m, 4H), 7.81 (s, 1H), 7.71 (t, *J* = 8.0 Hz, 1H), 7.67 (s, 1H), 7.61 (d, *J* = 7.6 Hz, 1H), 7.18 (d, *J* = 10.0 Hz, 1H), 6.80 (d, *J* = 12.4 Hz, 2H), 6.75 (d, *J* = 1.6 Hz, 1H), 6.70 (s, 1H), 5.55 (s, 2H), 5.04 (t, *J* = 7.2 Hz, 1H), 1.45 (d, *J* = 7.2 Hz, 3H); LC/MS (ESI) m/z: 519.4 $[M+H]^+$ |

(continued)

| No. | Structure/Name | Spectral Data |
|---|---|---|
| 150 | 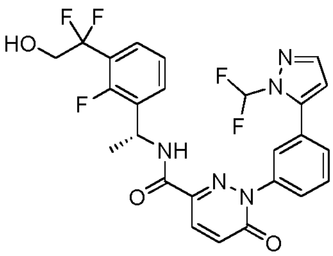<br>N-[(1R)-1-[3-(1,1-difluoro-2-hydroxy-ethyl)-2-fluoro-phenyl]ethyl]-1-[3-[2-(difluoromethyl) pyrazol-3-yl]phenyl]-6-oxo-pyridazine-3-carboxamide | $^1$H NMR (400 MHz, DMSO-$d_6$) δ = 9.02 (d, *J* = 8.0 Hz, 1H), 7.99 (s, 1H), 7.94 - 7.87 (m, 3H), 7.87 - 7.81 (m, 2H), 7.75 - 7.69 (m, 1H), 7.67 - 7.61 (m, 2H), 7.43 (t, *J* = 6.4 Hz, 1H), 7.26 (t, *J* = 7.6 Hz, 1H), 7.18 (d, *J* = 9.6 Hz, 1H), 6.76 (d, *J* = 1.6 Hz, 1H), 5.72 (br s, 1H), 5.41 (quin, *J* = 7.2 Hz, 1H), 3.91 (br t, *J* = 14.4 Hz, 2H), 1.48 (d, *J* = 7.2 Hz, 3H) ; LC/MS (ESI) m/z: 534.3 $[M+H]^+$ |
| 151 | 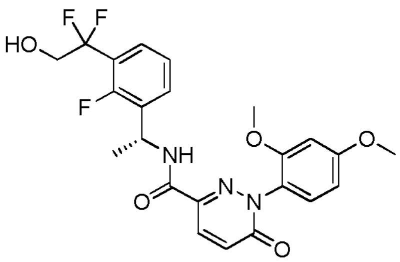<br>N-[(1R)-1-[3-(1,1-difluoro-2-hydroxy-ethyl)-2-fluoro-phenyl]ethyl]-1-(2,4-dimethoxyphenyl)-6-oxo-pyridazine-3-carboxamide | $^1$H NMR (400 MHz, DMSO-$d_6$) δ = 8.91 ( d, *J* = 8.2 Hz, 1H), 7.96 - 7.78 (m, 1H), 7.62 (t, *J* = 6.8 Hz, 1H), 7.42 (t, *J* = 7.2 Hz, 1H), 7.38 - 7.30 (m, 1H), 7.26 (t, *J* = 7.8 Hz, 1H), 7.13 - 7.02 (m, 1H), 6.74 (br s, 1H), 6.72 - 6.61 (m, 1H), 5.71 (br s, 1H), 5.47 - 5.32 (m, 1H), 3.91 (t, *J* = 14.4 Hz, 2H), 3.84 (s, 3H), 3.75 (s, 3H), 1.45 (d, *J* = 6.8 Hz, 3H) ; LC/MS (ESI) m/z: 478.4 $[M+H]^+$ |
| 152 | 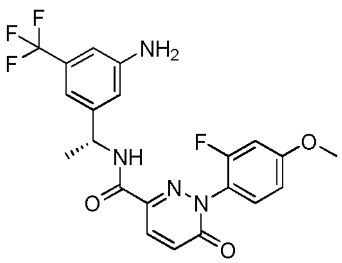<br>N-[(1R)-1-[3-amino-5-(trifluoromethyl)phenyl] ethyl]-1-(2-fluoro-4-methoxy-phenyl)-6-oxo-pyridazine-3-carboxamide | $^1$H NMR (400 MHz, DMSO-$d_6$) δ ppm 1.43 (d, *J* = 7.2 Hz, 3 H) 3.84 (s, 3 H) 5.02 (q, *J* = 7.2 Hz, 1 H) 5.54 (m, 2 H) 6.70 (s, 1 H) 6.77 (s, 1 H) 6.80 (s, 1 H) 6.95 (dd, *J* = 8.8, 2.25 Hz, 1 H) 7.06 (dd, *J* = 12.4, 2.63 Hz, 1 H) 7.2 (d, *J* = 9.76 Hz, 1 H) 7.55 (t, *J* = 8.8 Hz, 1 H) 7.92 (d, *J* = 10.0 Hz, 1 H) 8.87 (d, *J* = 8.4 Hz, 1 H) ; LC/MS (ESI) m/z: 451.2 $[M+H]^+$ |
| 153 | 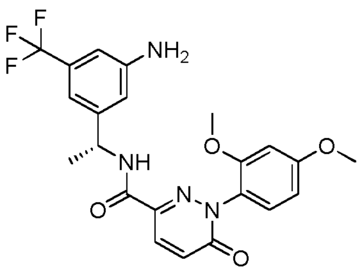<br>N-[(1R)-1-[3-amino-5-(trifluoromethyl)phenyl] ethyl]-1-(2,4-dimethoxyphenyl)-6-oxo-pyridazine-3-carboxamide | $^1$H NMR (400 MHz, DMSO-$d_6$) δ = 8.79 (br d, *J* = 8.0 Hz, 1H), 7.88 (d, *J* = 10.0 Hz, 1H), 7.32 (d, *J* = 8.8 Hz, 1H), 7.07 (d, *J* = 10.0 Hz, 1H), 6.85-6.66 (m, 4H), 6.64 (dd, *J* = 2.4, 8.8 Hz, 1H), 5.55 (s, 2H), 5.01 (br t, *J* = 7.6 Hz, 1H), 3.83 (s, 3H), 3.74 (s, 3H), 1.42 (d, *J* = 7.2 Hz, 3H) ; LC/MS (ESI) m/z: 463.3 $[M+H]^+$ |

(continued)

| No. | Structure/Name | Spectral Data |
|---|---|---|
| 154 | 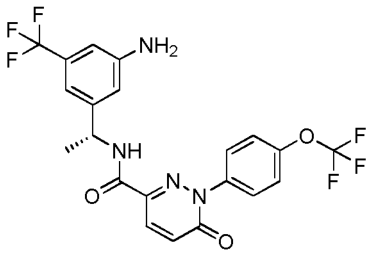 N-[(1R)-1-[3-amino-5-(trifluoromethyl)phenyl] ethyl]-6-oxo-1-[4-(trifluoromethoxy)phenyl] pyridazine-3-carboxamide | $^1$H NMR (400 MHz, DMSO-$d_6$) δ = 8.92 (d, *J* = 8.0 Hz, 1H), 7.90 (d, *J* = 9.8 Hz, 1H), 7.87 - 7.82 (m, 2H), 7.56 (d, *J* = 8.4 Hz, 2H), 7.17 (d, *J* = 10.0 Hz, 1H), 6.80 (br d, *J* = 14.8 Hz, 2H), 6.70 (s, 1H), 5.56 (s, 2H), 5.04 (quin, *J* = 7.2 Hz, 1H), 1.45 (d, *J* = 7.2 Hz, 3H) ; LC/MS (ESI) m/z: 486.3 $[M+H]^+$ |
| 155 | 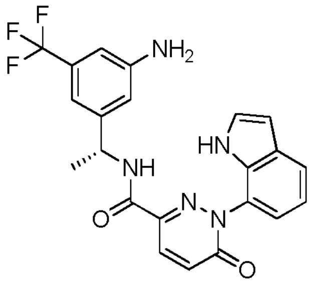 N-[(1R)-1-[3-amino-5-(trifluoromethyl)phenyl] ethyl]-1-(1H-indole-7-yl)-6-oxo-pyridazine-3-carboxamide | $^1$H NMR (400 MHz, DMSO-$d_6$) δ ppm 1.39 (d, *J* = 7.00 Hz, 3 H) 5.02 (quin, *J* = 7.6 Hz, 1 H) 5.53 (s, 2 H) 6.55 (m, 1 H) 6.68 (s, 1 H) 6.76 (s, 1 H) 6.80 (s, 1 H) 7.17 (m, 3 H) 7.38 (t, *J* = 2.8 Hz, 1 H) 7.68 (d, *J* = 7.6 Hz, 1 H) 7.95 (d, *J* = 9.6 Hz, 1 H) 8.80 (d, *J* = 8.4 Hz, 1 H) 11.17 (br s, 1 H) ; LC/MS (ESI) m/z: 442.3 $[M+H]^+$ |
| 156 | 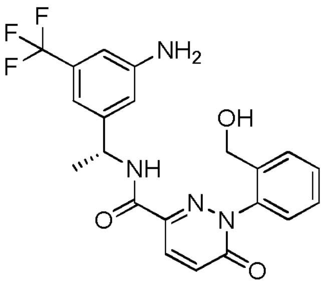 N-[(1R)-1-[3-amino-5-(trifluoromethyl)phenyl] ethyl]-1-[2-(hydroxymethyl)phenyl]-6-oxo-pyridazine-3-carboxamide | $^1$H NMR (400 MHz, DMSO-$d_6$) δ ppm 1.42 (br d, *J* = 7.2 Hz, 3 H) 4.35 (br d, *J* = 5.2 Hz, 2 H) 5.02 (quin, *J* = 7.2 Hz, 1 H) 5.20 (t, *J* = 5.6 Hz, 1 H) 5.54 (s, 2 H) 6.69 (s, 1H) 6.78 (brd, *J* = 13.2 Hz, 2 H) 7.14 (d, *J* = 9.6 Hz, 1 H) 7.42 (m, 2 H) 7.51 (m, 1 H) 7.63 (br d, *J* = 7.6 Hz, 1 H) 7.93 (d, *J* = 9.6 Hz, 1 H) 8.84 (br d, *J* = 8.4 Hz, 1 H) ; LC/MS (ESI) m/z: 433.3 $[M+H]^+$ |
| 157 | 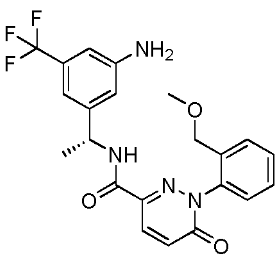 N-[(1R)-1-[3-amino-5-(trifluoromethyl)phenyl] ethyl]-1-[2-(methoxymethyl)phenyl]-6-oxo-pyridazine-3- carboxamide | $^1$H NMR (400 MHz, DMSO-$d_6$) δ ppm 1.42 (d, *J* = 7.2 Hz, 3 H) 3.15 (s, 3 H) 4.31 (s, 2 H) 5.03 (quin, *J* = 7.6 Hz, 1 H) 5.54 (s, 2 H) 6.69 (s, 1 H) 6.76 (s, 1 H) 6.79 (s, 1 H) 7.14 (d, *J* = 9.6 Hz, 1 H) 7.47 (d, *J* = 1.6 Hz, 1 H) 7.48 (d, *J* = 1.6 Hz, 1 H) 7.51 (td, *J* = 6.8, 2.81 Hz, 1 H) 7.56 (m, 1 H) 7.93 (d, *J* = 9.6 Hz, 1 H) 8.83 (d, *J* = 8.4 Hz, 1 H) ; LC/MS (ESI) m/z: 447.3 $[M+H]^+$ |

(continued)

| No. | Structure/Name | Spectral Data |
|---|---|---|
| 158 | 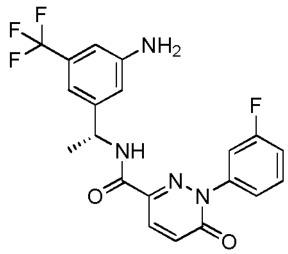 N-[(1R)-1-[3-amino-5-(trifluoromethyl)phenyl] ethyl]-1-(3-fluorophenyl)-6-oxo-pyridazine-3-carboxamide | $^{1}$H NMR (400 MHz, DMSO-$d_6$) $\delta$ = 8.91 (d, $J$ = 8.2 Hz, 1H), 7.90 (d, $J$ = 9.6 Hz, 1H), 7.66 (d, $J$ = 10.0 Hz, 1H), 7.63 - 7.55 (m, 2H), 7.34 (dtd, 1H), 7.17 (d, $J$ = 9.6 Hz, 1H), 6.83 (s, 1H), 6.79 (s, 1H), 6.71 (s, 1H), 5.56 (s, 2H), 5.05 (quin, $J$ = 7.2 Hz, 1H), 3.57 (s, 1H), 1.46 (d, $J$ = 7.2 Hz, 3H) ; LC/MS (ESI) m/z: 421.0 $[M+H]^+$ |
| 159 | 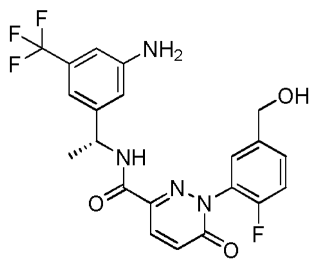 N-[(1R)-1-[3-amino-5-(tritluoromethyl)pheny 1] ethyl]-1-[2-fluoro-5-(hydroxymethyl)phenyl] -6-oxo-pyridazine-3-carboxamide | $^{1}$H NMR (400 MHz, DMSO-$d_6$) $\delta$ = 8.92 (d, $J$ = 8.4 Hz, 1H), 7.93 (d, $J$ = 10.0 Hz, 1H), 7.63 - 7.46 (m, 2H), 7.44 - 7.32 (m, 1H), 7.17 (d, $J$ = 9.6 Hz, 1H), 6.79 (br d, $J$ = 14.4 Hz, 2H), 6.69 (s, 1H), 5.54 (s, 2H), 5.48 - 5.30 (m, 1H), 5.02 (t, $J$ = 7.6 Hz, 1H), 4.54 (s, 2H), 1.42 (d, $J$ = 7.2 Hz, 3H) LC/MS (ESI) m/z: 451.1$[M+H]^+$ |
| 160 | 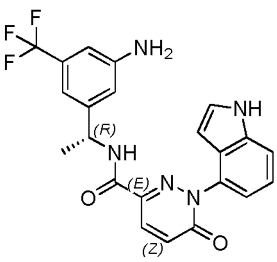 N-[(1R)-1-[3-amino-5-(trifluoromethyl)phenyl] ethyl]-1-(1H-indol-4-yl)-6-oxo-pyridazine-3-carboxamide | $^{1}$H NMR (400 MHz, DMSO-$d_6$) $\delta$ = 11.40 (br s, 1H), 8.74 (br d, $J$ = 8.0 Hz, 1H), 7.95 (d, $J$ = 9.6 Hz, 1H), 7.53 (d, $J$ = 8.0 Hz, 1H), 7.40 (br s, 1H), 7.25 - 7.15 (m, 3H), 6.79 (s, 1H), 6.75 (s, 1H), 6.68 (s, 1H), 6.18 (br s, 1H), 5.54 (s, 2H), 5.01 (br t, $J$ = 7.2 Hz, 1H), 1.39 (br d, $J$ = 6.8 Hz, 3H) ; LC/MS (ESI) m/z: 442.1 $[M+H]^+$ |
| 161 | 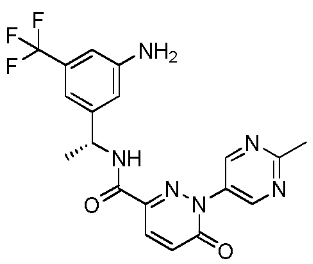 N-[(1R)-1-[3-amino-5-(trifluoromethyl)phenyl] ethyl]-1-(2-methylpyrimidin-5-yl)-6-oxo-pyridazine-3-carboxamide | $^{1}$H NMR (400 MHz, DMSO-$d_6$) $\delta$ = 9.15 (s, 2H), 8.99 (d, $J$ = 8.4 Hz, 1H), 7.92 (d, $J$ = 10.0 Hz, 1H), 7.20 (d, $J$ = 9.6 Hz, 1H), 6.82 (s, 1H), 6.79 (s, 1H), 6.71 (s, 1H), 5.56 (s, 2H), 5.06 (quin, $J$ = 7.2 Hz, 1H), 2.71 (s, 3H), 1.46 (d, $J$ = 6.8 Hz, 3H) ; LC/MS (ESI) m/z: 419.3 $[M+H]^+$ |

(continued)

| No. | Structure/Name | Spectral Data |
|---|---|---|
| 162 | 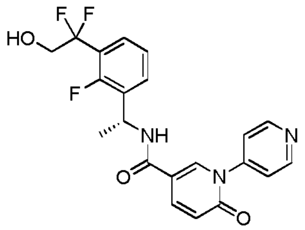 N-[(1R)-1-[3-(1,1-difluoro-2-hydroxy-ethyl)-2-fluoro-phenyl]ethyl]-6-oxo-1-(4-pyridyl)pyridine-3-carboxamide | $^1$H NMR (400 MHz, DMSO-$d_6$) $\delta$ = 8.81 - 8.76 (m, 2H), 8.71 (d, *J* = 7.2 Hz, 1H), 8.43 (d, *J* = 2.4 Hz, 1H), 7.95 (dd, *J* = 2.4, 9.6 Hz, 1H), 7.66 - 7.62 (m, 2H), 7.58 (t, *J* = 6.8 Hz, 1H), 7.46 - 7.40 (m, 1H), 7.31 - 7.24 (m, 1H), 6.58 (d, *J* = 9.6 Hz, 1H), 5.72 (t, *J* = 6.4 Hz, 1H), 5.36 (quin, *J* = 7.2 Hz, 1H), 3.92 (dt, *J* = 6.4, 14.4 Hz, 2H), 1.44 (d, *J* = 7.2 Hz, 3H) ; LC/MS (ESI) m/z: 418.3 $[M+H]^+$ |
| 163 | 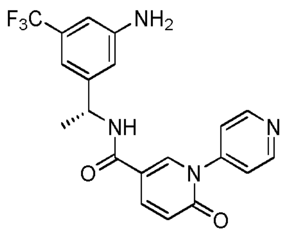 N-[(1R)-1-[3-amino-5-(trifluoromethyl)phenyl] ethyl]-6-oxo-1-(4-pyridyl)pyridine-3-carboxamide | $^1$H NMR (400 MHz, DMSO-$d_6$) $\delta$ = 8.81 - 8.74 (m, 2H), 8.59 (d, *J* = 7.6 Hz, 1H), 8.37 (d, *J* = 2.4 Hz, 1H), 7.97 (dd, *J* = 2.4, 9.6 Hz, 1H), 7.68 - 7.58 (m, 2H), 6.76 (s, 2H), 6.70 (s, 1H), 6.58 (d, *J* = 9.6 Hz, 1H), 5.55 (s, 2H), 5.01 (quin, *J* = 7.2 Hz, 1H), 1.40 (d, *J* = 7.2 Hz, 3H) ; LC/MS (ESI) m/z: 403.3 $[M+H]^+$ |
| 164 | 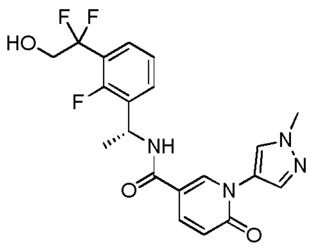 N-[(1R)-1-[3-(1,1-difluoro-2-hydroaη-ethyl)-2-fluoro-phenyl]ethyl]-1-(1-methylpyrazol-4-yl)-6-oxo-pyridine-3-carboxamide | $^1$H NMR (400 MHz, DMSO-$d_6$) $\delta$ = 8.73 (d, *J* = 7.2 Hz, 1H), 8.47 (d, *J* = 2.4 Hz, 1H), 8.26 (s, 1H), 7.90 (dd, *J* = 2.4, 9.6 Hz, 1H), 7.85 (s, 1H), 7.60 (br t, *J* = 6.8 Hz, 1H), 7.48 - 7.38 (m, 1H), 7.34 - 7.23 (m, 1H), 6.54 (d, *J* = 9.6 Hz, 1H), 5.72 (t, *J* = 6.4 Hz, 1H), 5.37 (quin, *J* = 7.2 Hz, 1H), 3.98 - 3.90 (m, 2H), 3.90 (s, 3H), 1.46 (d, *J* = 7.2 Hz, 3H) ; LC/MS (ESI) m/z: 421.3 $[M+H]^+$ |
| 165 | 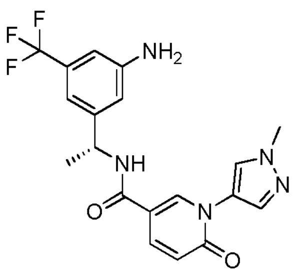 N-[(1R)-1-[3-amino-5-(trifluoromethyl)phenyl] ethyl]-1-(1-methylpyrazol-4-yl)-6-oxo-pyridine-3-carboxamide | $^1$H NMR (400 MHz, DMSO-$d_6$) $\delta$ = 8.61 (d, *J* = 7.6 Hz, 1H), 8.42 (d, *J* = 2.4 Hz, 1H), 8.25 (s, 1H), 7.91 (dd, *J* = 2.8, 9.6 Hz, 1H), 7.84 (s, 1H), 6.77 (s, 2H), 6.70 (s, 1H), 6.54 (d, *J* = 9.6 Hz, 1H), 5.55 (s, 2H), 5.02 (quin, *J* = 7.2 Hz, 1H), 3.89 (s, 3H), 1.42 (d, *J* = 7.2 Hz, 3H) ; LC/MS (ESI) m/z: 406.3 $[M+H]^+$ |

(continued)

| No. | Structure/Name | Spectral Data |
|---|---|---|
| 166 | 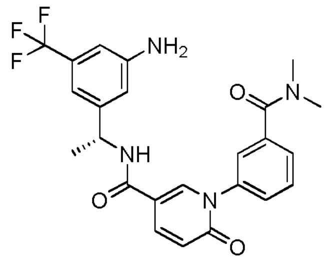 N-[(1R)-1-[3-amino-5-(trifluoromethyl)phenyl] ethyl]-1-[3-(dimethylcarbamoyl)phenyl]-6-oxo-pyridine-3-carboxamide | $^1$H NMR (400 MHz, DMSO-$d_6$) $\delta$ = 8.59 (d, $J$ = 7.6 Hz, 1H), 8.38 (d, $J$ = 2.4 Hz, 1H), 7.96 (dd, $J$ = 2.4, 9.6 Hz, 1H), 7.64 - 7.49 (m, 4H), 6.76 (s, 2H), 6.69 (s, 1H), 6.54 (d, $J$ = 9.6 Hz, 1H), 5.55 (s, 2H), 5.01 (quin, $J$ = 7.2 Hz, 1H), 2.97 (br d, $J$ = 16.0 Hz, 6H), 1.40 (d, $J$ = 7.2 Hz, 3H) ; LC/MS (ESI) m/z: 473.3 $[M+H]^+$ |
| 167 | 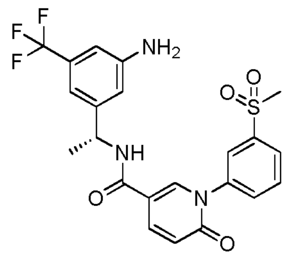 N-[(1R)-1-[3-amino-5-(trifluoromethyl)phenyl] ethyl]-1-(3-methylsulfonylphenyl)-6-oxo-pyridine-3-carboxamide | $^1$H NMR (400 MHz, DMSO-$d_6$) $\delta$ = 8.61 (d, $J$ = 7.6 Hz, 1H), 8.40 (d, $J$ = 2.4 Hz, 1H), 8.09 (s, 1H), 8.05 (d, $J$ = 7.6 Hz, 1H), 7.99 (dd, $J$ = 2.4, 9.6 Hz, 1H), 7.91 - 7.82 (m, 2H), 6.76 (s, 2H), 6.69 (s, 1H), 6.58 (d, $J$ = 9.6 Hz, 1H), 5.56 (s, 2H), 5.02 (quin, $J$ = 7.2 Hz, 1H), 3.31 - 3.29 (m, 3H), 1.40 (d, $J$ = 7.2 Hz, 3H) ; LC/MS (ESI) m/z: 480.3 $[M+H]^+$ |
| 168 | 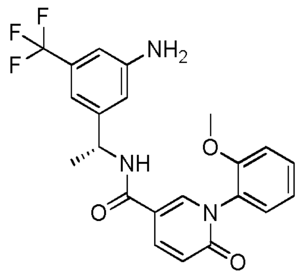 N-[(1R)-1-[3-amino-5-(trifluoromethyl)phenyl] ethyl]-1-(2-methoxyphenyl)-6-oxo-pyridine-3-carboxamide | $^1$H NMR (400 MHz, DMSO-$d_6$) $\delta$ = 8.53 (br d, $J$ = 7.2 Hz, 1H), 8.25 (s, 1H), 7.95 (dd, $J$ = 2.4, 9.6 Hz, 1H), 7.50 (t, $J$ = 8.0 Hz, 1H), 7.37 (d, $J$ = 7.6 Hz, 1H), 7.24 (d, $J$ = 8.4 Hz, 1H), 7.10 (t, $J$ = 7.6 Hz, 1H), 6.75 (s, 2H), 6.69 (s, 1H), 6.50 (d, $J$ = 9.6 Hz, 1H), 5.56 (s, 2H), 5.04 - 4.93 (m, 1H), 3.76 (s, 3H), 1.38 (d, $J$ = 7.2 Hz, 3H) ; LC/MS (ESI) m/z: 432.3 $[M+H]^+$ |
| 169 | 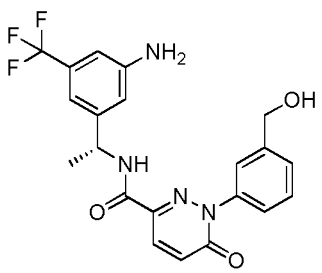 N-[(1R)-1-[3-amino-5-(trifluoromethyl)phenyl] ethyl]-1-[3-(hydroxymethyl)phenyl]-6-oxo-pyridazine-3-carboxamide | $^1$H NMR (400 MHz, DMSO-$d_6$) $\delta$ = 8.85 (d, $J$ = 8.4 Hz, 1H), 7.90 (d, $J$ = 9.6 Hz, 1H), 7.55 (s, 1H), 7.49 (d, $J$ = 5.2 Hz, 2H), 7.44 - 7.39 (m, 1H), 7.14 (d, $J$ = 9.6 Hz, 1H), 6.82 (s, 1H), 6.78 (s, 1H), 6.70 (s, 1H), 5.55 (s, 2H), 5.36 (br t, $J$ = 5.2 Hz, 1H), 5.03 (quin, $J$ = 7.2 Hz, 1H), 4.58 (d, $J$ = 4.8 Hz, 2H), 1.44 (d, $J$ = 7.2 Hz, 3H) ; LC/MS (ESI) m/z: 433.3 $[M+H]^+$ |

(continued)

| No. | Structure/Name | Spectral Data |
|---|---|---|
| 170 | 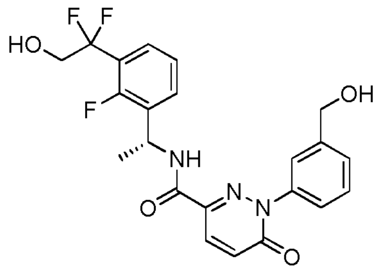 N-[(1R)-1-[3-(1,1-difluoro-2-hydroxy-ethyl)-2-fluoro-phenyl]ethyl]-1-[3-(hydroxymethyl) phenyl]-6-oxo-pyridazine-3-carboxamide | $^1$H NMR (400 MHz, DMSO-$d_6$) δ = 8.96 (d, *J* = 8.0 Hz, 1H), 7.87 (d, *J* = 9.6 Hz, 1H), 7.63 (t, *J* = 6.8 Hz, 1H), 7.56 (s, 1H), 7.54 - 7.47 (m, 2H), 7.45 - 7.40 (m, 2H), 7.27 (t, *J* = 7.6 Hz, 1H), 7.14 (d, *J* = 9.6 Hz, 1H), 5.71 (br s, 1H), 5.51 - 5.24 (m, 2H), 4.58 (s, 2H), 3.91 (t, *J* = 14.4 Hz, 2H), 1.47 (d, *J* = 7.2 Hz, 3H) ; LC/MS (ESI) m/z: 448.3 $[M+H]^+$ |
| 171 | 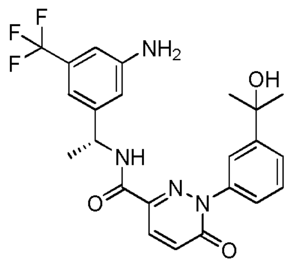 N-[(1R)-1-[3-amino-5-(trifluoromethyl)phenyl] ethyl]-1-[3-(1-hydroxy-1-methyl-ethyl)phenyl]-6-oxo-pyridazine-3-carboxamide | $^1$H NMR (400 MHz, DMSO-$d_6$) δ = 8.84 (d, *J* = 8.4 Hz, 1H), 7.89 (d, *J* = 9.6 Hz, 1H), 7.67 (s, 1H), 7.55 (d, *J* = 6.8 Hz, 1H), 7.49 - 7.42 (m, 2H), 7.13 (d, *J* = 9.6 Hz, 1H), 6.82 (s, 1H), 6.78 (s, 1H), 6.69 (s, 1H), 5.55 (s, 2H), 5.17 (s, 1H), 5.02 (quin, *J* = 7.2 Hz, 1H), 1.45 (s, 6H), 1.43 (d, *J* = 7.2 Hz, 3H) ; LC/MS (ESI) m/z: 443.4 $[M+H]^+$ |
| 172 | 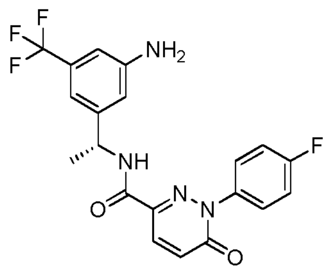 N-[(1R)-1-[3-amino-5-(trifluoromethyl)phenyl] ethyl]-1-(4-fluorophenyl)-6-oxo-pyridazine-3-carboxamide | $^1$H NMR (400 MHz, DMSO-$d_6$) δ = 8.86 (d, *J* = 8.4 Hz, 1H), 7.89 (d, *J* = 9.6 Hz, 1H), 7.78 - 7.69 (m, 2H), 7.38 (t, *J* = 8.4 Hz, 2H), 7.14 (d, *J* = 9.6 Hz, 1H), 6.82 (s, 1H), 6.78 (s, 1H), 6.70 (s, 1H), 5.54 (s, 2H), 5.04 (quin, *J* = 7.2 Hz, 1H), 1.45 (d, *J* = 7.2 Hz, 3H); LC/MS (ESI) m/z: 436.3 $[M+H]^+$ |
| 173 | 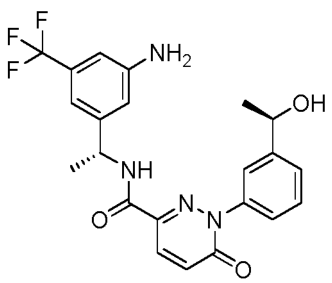 N-[(1R)-1-[3-amino-5-(trifluoromethyl)phenyl] ethyl]-1-[3-[(1R)-1-hydroxyethyl]phenyl] -6-oxo-pyridazine-3- carboxamide | $^1$H NMR (400 MHz, DMSO-$d_6$) δ = 8.84 (d, *J* = 8.4 Hz, 1H), 7.89 (d, *J* = 9.6 Hz, 1H), 7.57 (s, 1H), 7.52 - 7.39 (m, 3H), 7.14 (d, *J* = 9.6 Hz, 1H), 6.82 (s, 1H), 6.78 (s, 1H), 6.70 (s, 1H), 5.55 (s, 2H), 5.32 (br s, 1H), 5.03 (quin, *J* = 7.2 Hz, 1H), 4.80 (q, *J* = 6.4 Hz, 1H), 1.43 (d, *J* = 7.2 Hz, 3H), 1.35 (d, *J* = 6.4 Hz, 3H); LC/MS (ESI) m/z: 447.3 $[M+H]^+$ |

(continued)

| No. | Structure/Name | Spectral Data |
|---|---|---|
| 174 | 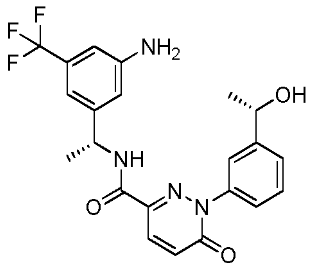 N-[(1R)-1-[3-amino-5-(trifluoromethyl)phenyl] ethyl]-1-[3-[(1S)-1-hydroxyethyl]phenyl]-6-oxo-pyridazine-3- carboxamide | $^1H$ NMR (400 MHz, DMSO-$d_6$) $\delta$ = 8.85 (d, *J* = 8.4 Hz, 1H), 7.89 (d, *J* = 9.6 Hz, 1H), 7.57 (s, 1H), 7.49 - 7.43 (m, 3H), 7.14 (d, *J* = 9.6 Hz, 1H), 6.82 (s, 1H), 6.77 (s, 1H), 6.69 (s, 1H), 5.55 (s, 2H), 5.32 (d, *J* = 4.4 Hz, 1H), 5.03 (quin, *J* = 7.2 Hz, 1H), 4.82 - 4.76 (m, 1H), 1.43 (d, *J* = 7.2 Hz, 3H), 1.35 (d, *J* = 6.4 Hz, 3H); LC/MS (ESI) m/z: 447.3 $[M+H]^+$ |
| 175 | 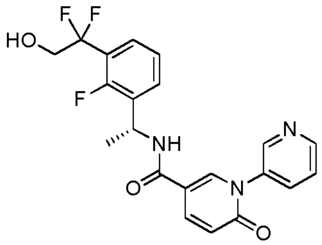 N-[(1R)-1-[3-(1,1-difluoro-2-hydroaη-ethyl)-2-fluoro-phenyl]ethyl]-6-oxo-1-(3-pyridyl)pyridine-3-carboxamide | $^1H$ NMR (400 MHz, DMSO-$d_6$) $\delta$ = 9.02 (d, *J* = 7.2 Hz, 1H), 8.63 (d, *J* = 2.4 Hz, 1H), 8.49 (d, *J* = 2.4 Hz, 1H), 8.47 (dd, *J* = 1.2, 4.8 Hz, 1H), 8.34 (dd, *J* = 2.4, 8.8 Hz, 1H), 7.69 (d, *J* = 8.8 Hz, 1H), 7.61 (t, *J* = 7.2 Hz, 1H), 7.50 (dd, *J* = 4.8, 8.4 Hz, 1H), 7.43 (t, *J* = 6.8 Hz, 1H), 7.28 (t, *J* = 7.2 Hz, 1H), 7.24 (d, *J* = 8.8 Hz, 1H), 5.72 (t, *J* = 6.4 Hz, 1H), 5.40 (quin, *J* = 7.2 Hz, 1H), 3.92 (dt, *J* = 6.4, 14.4 Hz, 2H), 1.47 (d, *J* = 7.2 Hz, 3H); LC/MS (ESI) m/z: 418.3 $[M+H]^+$ |
| 176 | 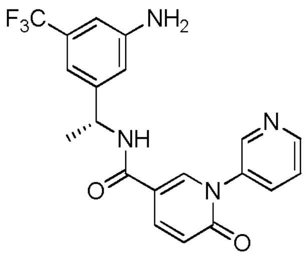 N-[(1R)-1-[3-amino-5-(trifluoromethyl)phenyl] ethyl]-6-oxo-1-(3-pyridyl)pyridine-3-carboxamide | $^1H$ NMR (400 MHz, DMSO-$d_6$) $\delta$ = 8.90 (d, *J* = 8.0 Hz, 1H), 8.62 (d, *J* = 2.4 Hz, 1H), 8.52 - 8.44 (m, 2H), 8.32 (dd, *J* = 2.4, 8.8 Hz, 1H), 7.68 (ddd, *J* = 1.4, 2.8, 8.4 Hz, 1H), 7.50 (dd, *J* = 4.4, 8.4 Hz, 1H), 7.23 (d, *J* = 8.8 Hz, 1H), 6.79 (s, 2H), 6.70 (s, 1H), 5.57 (s, 2H), 5.05 (t, *J* = 7.2 Hz, 1H), 1.44 (d, *J* = 7.2 Hz, 3H) ; LC/MS (ESI) m/z: 403.3 $[M+H]^+$ |
| 177 | 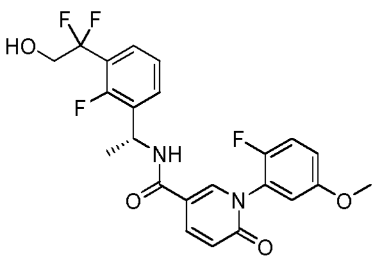 N-[(1R)-1-[3-(1,1-difluoro-2-hydroaη-ethyl)-2-fluoro-phenyl]ethyl]-1-(2-fluoro-5-methoxyphenyl)-6-oxo-pyridine-3-carboxamide | $^1H$ NMR (400 MHz, DMSO-$d_6$) $\delta$ = 8.66 (br d, *J* = 7.2 Hz, 1H), 8.40 (d, *J* = 2.4 Hz, 1H), 7.96 (dd, *J* = 2.4, 9.6 Hz, 1H), 7.58 (br t, *J* = 6.8 Hz, 1H), 7.45 - 7.35 (m, 2H), 7.28 (t, *J* = 7.6 Hz, 1H), 7.24 (dd, *J* = 3.2, 6.0 Hz, 1H), 7.12 (td, *J* = 3.4, 9.2 Hz, 1H), 6.55 (d, *J* = 9.6 Hz, 1H), 5.71 (t, *J* = 6.4 Hz, 1H), 5.35 (t, *J* = 7.2 Hz, 1H), 3.92 (dt, *J* = 6.4, 14.4 Hz, 2H), 3.80 (s,3H), 1.43 (d, *J* = 7.2 Hz, 3H) ; LC/MS (ESI) m/z: 465.3 $[M+H]^+$ |

(continued)

| No. | Structure/Name | Spectral Data |
|---|---|---|
| 178 | 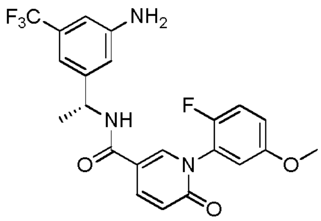 N-[(1R)-1-[3-amino-5-(trifluoromethyl)phenyl] ethyl]-1-(2-fluoro-5-methoxy-phenyl)-6-oxo-pyridine-3- carboxamide | $^1$H NMR (400 MHz, DMSO-$d_6$) $\delta$ = 8.55 (d, *J* = 7.6 Hz, 1H), 8.35 (d, *J* = 2.4 Hz, 1H), 7.98 (dd, *J* = 2.4, 9.6 Hz, 1H), 7.38 (t, *J* = 9.2 Hz, 1H), 7.22 (dd, *J* = 3.2, 6.0 Hz, 1H), 7.11 (td, *J* = 3.6, 9.0 Hz, 1H), 6.76 (s, 2H), 6.70 (s, 1H), 6.56 (d, *J* = 9.6 Hz, 1H), 5.55 (s, 2H), 5.00 (quin, *J* = 7.2 Hz, 1H), 3.79 (s, 3H), 1.40 (d, *J* = 7.2 Hz, 3H) ; LC/MS (ESI) m/z: 450.4 [M+H]$^+$ |
| 179 | 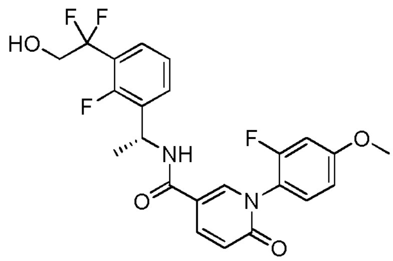 N-[(1R)-1-[3-(1,1-difluoro-2-hydroxy-ethyl)-2-fluoro-phenyl]ethyl]-1-(2-fluoro-4-methoxyphenyl)-6-oxo-pyridine-3-carboxamide | $^1$H NMR (400 MHz, DMSO-$d_6$) $\delta$ = 8.67 (d, *J* = 7.2 Hz, 1H), 8.37 (d, *J* = 2.4 Hz, 1H), 7.95 (dd, *J* = 2.8, 9.6 Hz, 1H), 7.60 - 7.47 (m, 2H), 7.43 (br t, *J* = 6.4 Hz, 1H), 7.31 - 7.25 (m, 1H), 7.10 (dd, *J* = 2.4, 12.0 Hz, 1H), 6.95 (dd, *J* = 2.4, 8.8 Hz, 1H), 6.61 - 6.48 (m, 1H), 5.74 (br d, *J* = 14.4 Hz, 1H), 5.39 - 5.31 (m, 1H), 3.91 (br t, *J* = 14.4 Hz, 2H), 3.84 (s, 3H), 1.43 (d,J = 7.2 Hz, 3H) ) ; LC/MS (ESI) m/z: 465.3 [M+H]$^+$ |
| 180 | 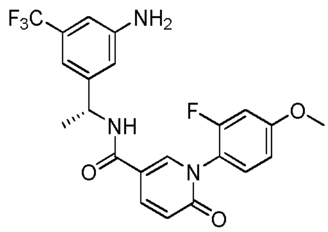 N-[(1R)-1-[3-amino-5-(trifluoromethyl)phenyl] ethyl]-1-(2-fluoro-4-methoxy-phenyl)-6-oxo-pyridine-3- carboxamide | $^1$H NMR (400 MHz, DMSO-$d_6$) $\delta$= 8.21 (d, *J* = 2.4 Hz, 1H), 8.06 (dd, *J* = 2.4, 9.6 Hz, 1H), 7.40 - 7.35(m, 1H), 6.99 - 6.91 (m, 2H), 6.88 (br s, 2H), 6.80 (s, 1H), 6.64 (d, *J* = 9.6 Hz, 1H), 5.11 (d, *J* = 7.2 Hz, 1H), 3.87 (s, 3H), 1.51 (d, *J* = 7.2 Hz, 3H) ; LC/MS (ESI) m/z: 450.4 [M+H]$^+$ |
| 181 | 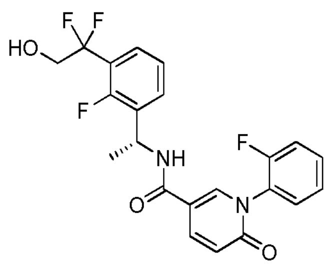 N-[(1R)-1-[3-(1,1-difluoro-2-hydroxy-ethyl)-2-fluoro-phenyl]ethyl]-1-(2-fluorophenyl)-6-oxo-pyridine-3-carboxamide | $^1$H NMR (400 MHz, DMSO-$d_6$) $\delta$ = 8.68 (d, *J* = 7.2 Hz, 1H), 8.42 (d, *J* = 2.4 Hz, 1H), 7.97 (dd, *J* = 2.8, 9.6 Hz, 1H), 7.65 -7.55 (m, 3H), 7.50 -7.39 (m, 3H), 7.30 - 7.24 (m, 1H), 6.57 (d, *J* = 9.6 Hz, 1H), 5.72 (br t, *J* = 6.4 Hz, 1H), 5.35 (quin, *J* = 7.2 Hz, 1H), 3.91 (dt, *J* = 5.8, 14.4 Hz, 2H), 1.43 (d, *J* = 7.2 Hz, 3H) ; LC/MS (ESI) m/z: 4 35.3 [M+H]$^+$ |

(continued)

| No. | Structure/Name | Spectral Data |
|---|---|---|
| 182 | 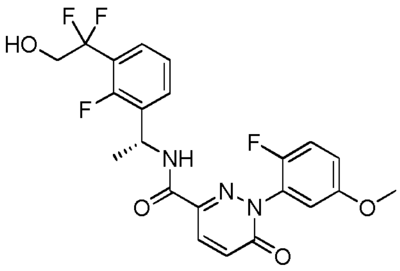<br>N-[(1R)-1-[3-(1,1-difluoro-2-hydroxy-ethyl)-2-fluoro-phenyl]ethyl]-1-(2-fluoro-5-methoxyphenyl)-6-oxo-pyridazine-3-carboxamide | $^1$H NMR (400 MHz, DMSO-$d_6$) δ = 9.01 (d, *J* = 8.0 Hz, 1H), 7.91 (d, *J* = 9.6 Hz, 1H), 7.62 (brt, *J* = 7.2 Hz, 1H), 7.45 - 7.35 (m, 2H), 7.30 - 7.24 (m, 2H), 7.20 - 7.11 (m, 2H), 5.71 (br s, 1H), 5.40 (t, *J* = 7.2 Hz, 1H), 3.91 (dt, *J* = 5.2, 14.4 Hz, 2H), 3.80 (s, 3H), 1.46 (d, *J* = 7.2 Hz, 3H) ; LC/MS (ESI) m/z: 466.3 $[M+H]^+$ |
| 183 | 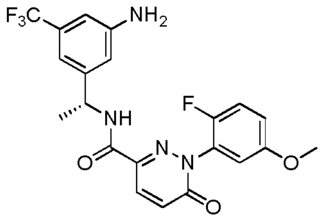<br>N-[(1R)-1-[3-amino-5-(trifluoromethyl)phenyl] ethyl]-1-(2-fluoro-5-methoxy-phenyl)-6-oxo-pyridazine-3-carboxamide | $^1$H NMR (400 MHz, DMSO-$d_6$) δ = 8.21 (d, *J* = 2.4 Hz, 1H), 8.06 (dd, *J* = 2.4, 9.6 Hz, 1H), 7.40 - 7.35 (m, 1H), 6.99 - 6.91 (m, 2H), 6.88 (br s, 2H), 6.80 (s, 1H), 6.64 (d, *J* = 9.6 Hz, 1H), 5.11 (d, *J* = 7.2 Hz, 1H), 3.87 (s, 3H), 1.51 (d, *J* = 7.2 Hz, 3H) ; LC/MS (ESI) m/z: 451.3 $[M+H]^+$ |
| 184 | 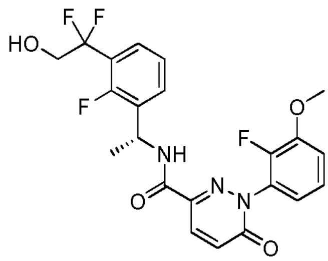<br>N-[(1R)-1-[3-(1,1-difluoro-2-hydroaη-ethyl)-2-fluoro-phenyl]ethyl]-1-(2-fluoro-3-methoxyphenyl)-6-oxo-pyridazine-3-carboxamide | $^1$H NMR (400 MHz, DMSO-$d_6$) δ = 9.01 (d, *J* = 8.0 Hz, 1H), 7.91 (d, *J* = 9.6 Hz, 1H), 7.61 (brt, *J* = 7.2 Hz, 1H), 7.42 (t, *J* = 6.8 Hz, 1H), 7.37 - 7.30 (m, 2H), 7.29 - 7.24 (m, 1H), 7.23 - 7.15 (m, 2H), 5.71 (t, *J* = 6.4 Hz, 1H), 5.39 (quin, *J* = 7.2 Hz, 1H), 3.95 - 3.87 (m, 5H), 1.45 (d, *J* = 7.2 Hz, 3H) ; LC/MS (ESI) m/z: 466.3 $[M+H]^+$ |
| 185 | 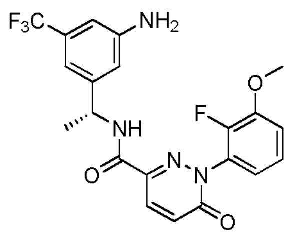<br>N-[(1R)-1-[3-amino-5-(trifluoromethyl)phenyl] ethyl]-1-(2-fluoro-3- methoxy-phenyl)-6-oxo-pyridazine-3-carboxamide | $^1$H NMR (400 MHz, DMSO-$d_6$) δ = 8.89 (br d, *J* = 8.0 Hz, 1H), 7.93 (d, *J* = 9.6 Hz, 1H), 7.37 - 7.29 (m, 2H),7.21 - 7.13 (m, 2H), 6.80 (s, 1H), 6.77 (s, 1H), 6.69 (s, 1H), 5.54 (s, 2H), 5.02 (quin, *J* = 7.2 Hz, 1H), 3.91 (s, 3H), 1.42 (d, *J* = 7.2 Hz, 3H) ; LC/MS (ESI) m/z: 45 1.3 $[M+H]^+$ |

(continued)

| No. | Structure/Name | Spectral Data |
|---|---|---|
| 186 | 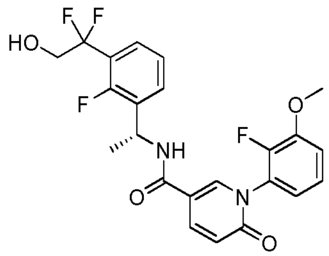 N-[(1R)-1-[3-(1,1-difluoro-2-hydroxy-ethyl)-2-fluoro-phenyl]ethyl]-1-(2-fluoro-3-methoxyphenyl)-6-oxo-pyridine-3-carboxamide | $^1$H NMR (400 MHz, DMSO-$d_6$) $\delta$ = 8.68 (d, *J* = 7.2 Hz, 1H), 8.39 (d, *J* = 2.4 Hz, 1H), 7.96 (dd, *J* = 2.4, 9.6 Hz, 1H), 7.57 (t, *J* = 7.2 Hz, 1H), 7.45 - 7.25 (m, 4H), 7.14 (dt, *J* = 2.0, 6.8 Hz, 1H), 6.56 (d, *J* = 9.6 Hz, 1H), 5.72 (t, *J* = 6.4 Hz, 1H), 5.34 (quin, *J* = 7.2 Hz, 1H), 3.96 - 3.86 (m, 5H), 1.43 (d, *J* = 7.2 Hz, 3H) ; LC/MS (ESI) m/z: 465.3 $[M+H]^+$ |
| 187 | 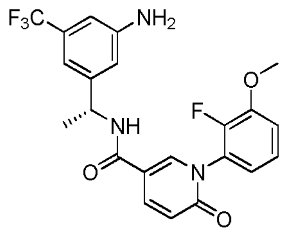 N-[(1R)-1-[3-amino-5-(trifluoromethyl)phenyl] ethyl]-1-(2-fluoro-3- methoxy-phenyl)-6-oxo-pyridine-3 - carboxamide | $^1$H NMR (400 MHz, DMSO-$d_6$) $\delta$ = 8.56 (d, *J* = 7.6 Hz, 1H), 8.34 (d, *J* = 2.4 Hz, 1H), 7.98 (dd, *J* = 2.8, 9.6 Hz, 1H), 7.34 (quin, *J* = 8.0 Hz, 2H), 7.13 (dt, *J* = 2.0, 6.8 Hz, 1H), 6.75 (s, 2H), 6.69 (s, 1H), 6.56 (d, *J* = 9.6Hz, 1H), 5.56 (s, 2H), 5.04 - 4.95 (m, 1H), 3.91 (s, 3H), 1.39 (d, *J* = 7.2 Hz, 3H) ; LC/MS (ESI) m/z: 450.3 $[M+H]^+$ |
| 188 | 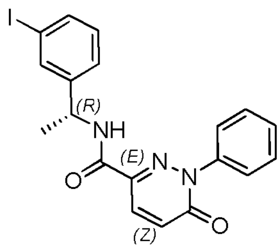 (R)-N-(1-(3-iodophenyl)ethyl)-6-oxo-1-phenyl-1,6-dihydropyridazine-3-carboxamide | $^1$H NMR (400 MHz, DMSO-$d_6$) $\delta$ = 8.92 (d, *J* = 8.0 Hz, 1H), 7.89 (s, 1H), 7.86 (s, 1H), 7.76 (s, 1H), 7.68 - 7.57 (m, 2H), 7.54-7.41 (m, 3H), 7.30-7.24 (m, 1H), 7.20-7.18 (d, 1H), 7.17-7.10 (m 2H), 5.07 (quin, *J* = 7.2 Hz, 1H), 1.46 (d, *J* = 7.2 Hz, 3H) ; LC/MS (ESI) m/z: 446.2 $[M+H]^+$ |
| 189 | 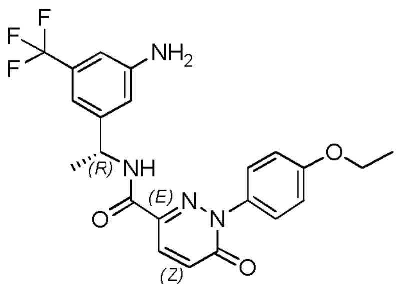 (R)-N-(1-(3-amino-5-(trifluoromethyl)phenyl) ethyl)-1-(4-ethoxyphenyl)-6-oxo-1,6-dihydropyridazine-3-carboxamide | $^1$H NMR (400 MHz, DMSO-$d_6$) $\delta$ = 8.86-8.84 (m, 1H), 8.87-8.84 (m, 1H), 7.60-7.50 (m, 2H), 7.11 (d, *J* = 9.6 Hz, 1H), 7.10-7.04 (m, 2H), 7.03-6.80 (m, 2H), 6.69 (m, 1H), 5.54 (s, 1H), 5.07-4.98 (m, 1H), 4.09 (quin, *J* = 7.2 Hz, 1H), 1.44 (d, *J* = 7.2 Hz, 2H), 1.35 (t, *J* = 6.8 Hz, 3H) ; LC/MS (ESI) m/z: 447.3 $[M+H]^+$ |

(continued)

| No. | Structure/Name | Spectral Data |
|---|---|---|
| 190 | 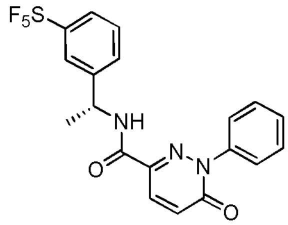 6-oxo-N-[(1R)-1-[3-(pentafluoro-λ6-sulfanyl) phenyl]ethyl]-1-phenyl-pyndazine-3-carboxamide | $^1$H NMR (400 MHz, DMSO-$d_6$) δ =1.50 (d, J = 7.2 Hz, 3 H) 5.22 (q, J = 7.6 Hz, 1 H) 7.14 (d, J = 10.0 Hz, 1 H) 7.48 (m, 1 H) 7.55 (m, 3 H) 7.67 (m, 3 H) 7.76 (dd, J = 8.4, 1.6 Hz, 1 H) 7.87 (d, J = 10.0 Hz, 1 H) 7.93 (s, 1 H) 9.03 (br d, J = 8.4 Hz, 1 H) ; LC/MS (ESI) m/z: 446.3 $[M+H]^+$ |
| 191 | 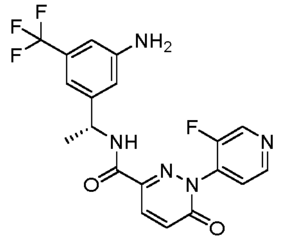 N-[(1R)-1-[3-(1,1-difluoro-2-hydroaη-ethyl)-2-fluorophenyl]ethyl]-1-(2-fluoro-3-pyridyl)-6-oxo-pyridazine-3-carboxamide | $^1$H NMR (400 MHz, DMSO-$d_6$) δ = 8.97 (d, J = 8.4 Hz, 1H), 8.84 (d, J = 1.6 Hz, 1H), 8.69 (d, J = 5.2 Hz, 1H), 7.95 (d, J = 10.0 Hz, 1H), 7.86 (t, J = 5.6 Hz, 1H), 7.23 (d, J = 9.6 Hz, 1H), 6.79 (d, J = 13.2 Hz, 2H), 6.70 (s, 1H), 5.55 (s, 2H), 5.09 - 4.97 (m, 1H), 1.43 (d, J = 7.2 Hz, 3H) ; LC/MS (ESI) m/z = 422.3 $[M+H]^+$ |
| 192 | 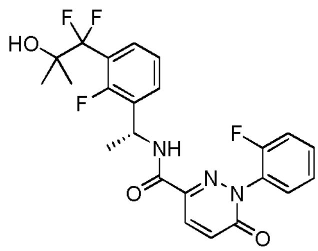 N-[(1R)-1-[3-(1,1-difluoro-2-hydroxy-2-methyl-propyl)-2-fluoro-phenyl]ethyl]-1-(2-fluorophenyl)-6-oxo-pyridazine-3-carboxamide | $^1$H NMR (400 MHz, DMSO-$d_6$) δ = 9.00 (br d, J = 8.0 Hz, 1H), 7.92 (d, J = 9.6 Hz, 1H), 7.69 (br t, J = 7.2 Hz, 1H), 7.62 - 7.53 (m, 2H), 7.49 - 7.40 (m, 2H), 7.32 (t, J = 6.8 Hz, 1H), 7.25 - 7.15 (m, 2H), 5.38 (br t, J = 7.2 Hz, 1H), 5.33 (s, 1H), 1.45 (br d, J = 6.8 Hz, 3H), 1.19 (br s, 6H) ; LC/MS (ESI) m/z: 464.3 $[M+H]^+$ |
| 193 | 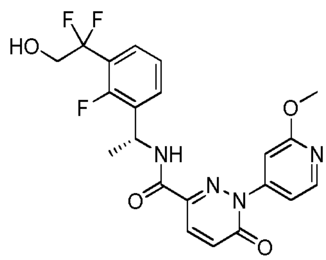 N-[(1R)-1-[3-(1,1-difluoro-2-hydroxy-ethyl)-2-fluoro-phenyl]ethyl]-1-(2-methoxy-4-pyridyl)-6-oxo-pyridazine-3-carboxamide | $^1$H NMR (400 MHz, DMSO-$d_6$) δ = 9.05 (d, *J* = 8.0 Hz, 1H), 8.33 (d, *J* = 5.6 Hz, 1H), 7.86 (d, *J* = 9.6 Hz, 1H), 7.65 (t, *J* = 7.2 Hz, 1H), 7.48 (dd, *J* = 1.6, 5.6 Hz, 1H), 7.46 - 7.41 (m, 1H), 7.36 (d, *J* = 1.6 Hz, 1H), 7.28 - 7.28 (m, 1H), 7.29 (t, *J* = 7.6 Hz, 1H), 7.17 (d, *J* = 9.6 Hz, 1H), 5.72 (t, *J* = 6.4 Hz, 1H), 5.41 (quin, *J* = 7.2 Hz, 1H), 3.96 - 3.89 (m, 5H), 1.50 (d, *J* = 7.2 Hz, 3H) ; LC/MS (ESI) m/z: 449.3 $[M+H]^+$ |

(continued)

| No. | Structure/Name | Spectral Data |
|---|---|---|
| 194 | 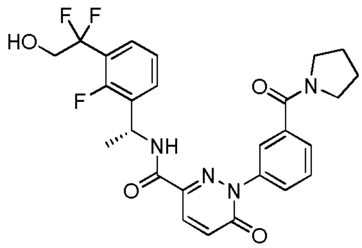 N-[(1R)-1-[3-(1,1-difluoro-2-hydroaη-ethyl)-2-fluoro-phenyl]ethyl]-6-oxo-1-[3-(pyrrolidine-1-carbonyl)phenyl]pyridazine-3-carboxamide | $^1$H NMR (400 MHz, DMSO-$d_6$) $\delta$ = 9.00 (d, *J* = 8.0 Hz, 1H), 7.91-7.82 (m, 2H), 7.78 (dt, *J* = 2.4, 4.4 Hz, 1H), 7.67-7.58 (m, 3H), 7.53-7.53 (m, 1H), 7.43 (br t, *J* = 6.8 Hz, 1H), 7.31-7.23 (m, 1H), 7.15 (d, *J* = 9.6 Hz, 1H), 5.71 (t, *J* = 6.4 Hz, 1H), 5.40 (quin, *J* = 7.2 Hz, 1H), 3.96-3.86 (m, 2H), 3.47 (td, *J* = 6.4, 15.6 Hz, 4H), 1.91-1.78 (m, 4H), 1.47 (d, *J* = 7.2 Hz, 3H) ; LC/MS (ESI) m/z: 515.4 $[M+H]^+$ |
| 195 | 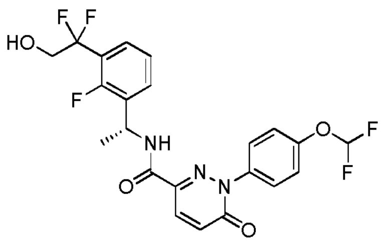 N-[(1R)-1-[3-(1,1-difluoro-2-hydroxy-ethyl)-2-fluoro-phenyl]ethyl]-1-[4-(difluoromethoxy) phenyl]-6-oxo-pyridazine-3- carboxamide | $^1$H NMR (400 MHz, DMSO-$d_6$) $\delta$ = 8.97 (d, *J* = 8.0 Hz, 1H), 7.87 (d, *J* = 9.6 Hz, 1H), 7.79-7.71 (m, 2H), 7.63 ( t, *J* = 6.8 Hz, 1H), 7.52 (s, 1H), 7.46-7.39 (m, 1H), 7.38 -7.31 (m, 3H), 7.27 (t, *J* = 7.6 Hz, 1H), 7.17-7.10 (m, 1 H), 5.72 (s, 1H), 5.40 (quin, *J* = 7.2 Hz, 1H), 3.92 (t, *J* = 14.4 Hz, 2H), 1.47 (d, *J* = 7.2 Hz, 3H) ; LC/MS (ESI) m/z: 484.3 $[M+H]^+$ |
| 196 | 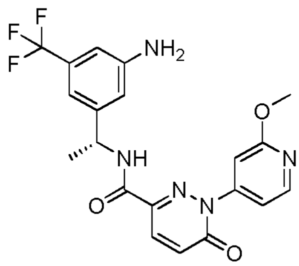 N-[(1R)-1-[3-amino-5-(trifluoromethyl)phenyl] ethyl]-1-(2-methoxy-4-pyridyl)-6-oxo-pyridazine-3-carboxamide | $^1$H NMR (400 MHz, DMSO-$d_6$) $\delta$ = 8.95 (d, *J* = 8.4 Hz, 1H), 8.31 (d, *J* = 5.6 Hz, 1H), 7.88 (d, *J* = 9.6 Hz, 1H), 7.48 (dd, *J* = 1.6, 5.6 Hz, 1H), 7.35 (d, *J* = 1.6 Hz, 1H), 7.17 (d, *J* = 9.6 Hz, 1H), 6.81 (d, *J* = 14.0 Hz, 2H), 6.71 (s, 1H), 5.55 (s, 2H), 5.05 (quin, *J* = 7.6 Hz, 1H), 3.92 (s, 3H), 1.47 (d, *J* = 7.2 Hz, 3H), 1.41-1.41 (m, 1H) ; LC/MS (ESI) m/z: 434.3 $[M+H]^+$ |
| 197 | 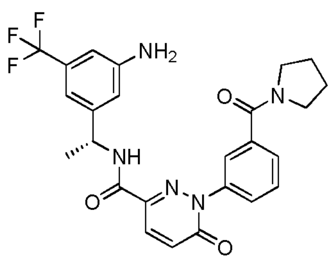 N-[(1R)-1-[3-amino-5-(trifluoromethyl)phenyl] ethyl]-6-oxo-1-[3-(pyrrolidine-1-carbonyl)phenyl] pyridazine-3- carboxamide | $^1$H NMR (400 MHz, DMSO-$d_6$) $\delta$ = 8.89 (d, *J* = 8.4 Hz, 1H), 7.89 (d, *J* = 9.6 Hz, 1H), 7.85 (s, 1H), 7.78 (dt, *J* = 2.0, 4.4 Hz, 1H), 7.63-7.58 (m, 2H), 7.15 (d, *J* = 9.6 Hz, 1H), 6.80 (d, *J* = 12.0 Hz, 2H), 6.70 (s, 1H), 5.55 (s, 2H), 5.07-4.99 (m, 1H), 3.49-3.43 (m, 4H), 1.90-1.79 (m, 4H), 1.44 (d, *J* = 7.2 Hz, 3H) ; LC/MS (ESI) m/z: 500.4 $[M+H]^+$ |

(continued)

| No. | Structure/Name | Spectral Data |
|---|---|---|
| 198 | 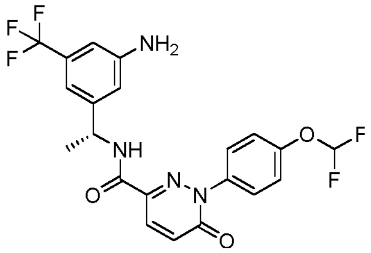 N-[(1R)-1-[3-amino-5-(trifluoromethyl)phenyl] ethyl]-1-[4-(difluoromethoxy)phenyl]-6-oxo-pyridazine-3-carboxamide | $^1$H NMR (400 MHz, DMSO-$d_6$) $\delta$ = 8.88 (d, *J* = 8.4 Hz, 1H), 7.89 (d, *J* = 9.6 Hz, 1H), 7.77-7.72 (m, 2H), 7.52 (s, 0.25H), 7.36-7.31 (m, 2.50H), 7.16 (s, 0.47H), 7.15 (s, 0.25H), 7.14 (s, 0.52H), 6.82 (s, 1H), 6.78 (s, 1H), 6.70 (s, 1H), 5.56 (s, 2H), 5.04 (quin, *J* = 7.2 Hz, 1H), 1.44 (d, *J* = 7.2 Hz, 3H) ; LC/MS (ESI) m/z: 469.3 $[M+H]^+$ |
| 199 | 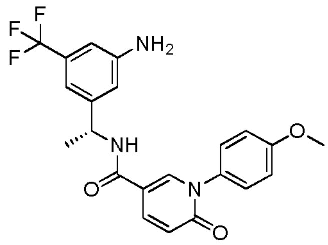 N-[(1R)-1-[3-amino-5-(trifluoromethyl)phenyl] ethyl]-1-(4-methoxyphenyl)-6-oxo-pyridine-3-carboxamide | $^1$H NMR (400 MHz, DMSO-$d_6$) $\delta$ = 8.57 (d, *J* = 7.6 Hz, 1H), 8.33 (d, *J* = 2.4 Hz, 1H), 7.94 (dd, *J* = 2.4, 9.6 Hz, 1H), 7.43-7.34 (m, 2H), 7.11-7.04 (m, 2H), 6.75 (s, 2H), 6.69 (s, 1H), 6.51 (d, *J* = 9.6 Hz, 1H), 5.54 (s, 2H), 5.00 (quin, *J* = 7.2 Hz, 1H), 3.81 (s, 3H), 1.39 (d, *J* = 7.2 Hz, 3H) ; LC/MS (ESI) m/z: 432.3 $[M+H]^+$ |
| 200 | 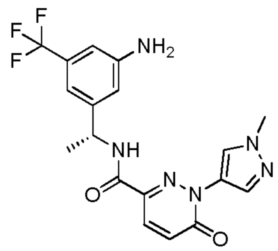 N-[(1R)-1-[3-amino-5-(trifluoromethyl)phenyl] ethyl]-1-(1-methylpyrazol-4-yl)-6-oxo-pyridazine-3-carboxamide | $^1$H NMR (400 MHz, DMSO-$d_6$) $\delta$ = 8.90 (d, *J* = 8.4 Hz, 1H), 8.55 (s, 1H), 8.36 (s, 1H), 7.87 (d, *J* = 9.6 Hz, 1H), 7.16 (d, *J* = 9.6 Hz, 1H), 6.86 (s, 1H), 6.82 (s, 1H), 6.72 (s, 1H), 5.57 (s, 2H), 5.10 (quin, *J* = 7.2 Hz, 1H), 3.91 (s, 3H), 1.52 (d, *J* = 7.2 Hz, 3H) ; LC/MS (ESI) m/z: 40 7.3 $[M+H]^+$ |
| 201 | 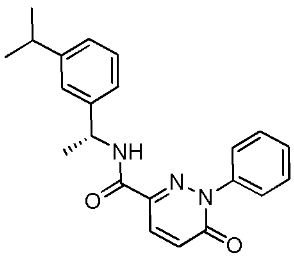 N-[(1R)-1-(3-isopropylphenyl)ethyl]-6-oxo-1-phenyl-pyridazine-3-carboxamide | $^1$H NMR (400 MHz, DMSO-$d_6$) $\delta$ = 8.79 (d, *J* = 8.4 Hz, 1H), 7.89 (d, *J* = 9.6 Hz, 1H), 7.71-7.63 (m, 2H), 7.56-7.50 (m, 2H), 7.49-7.43 (m, 1H), 7.25 (s, 1H), 7.24-7.16 (m, 2H), 7.14 (d, *J* = 9.6 Hz, 1H), 7.10 (d, *J* = 7.2 Hz, 1H), 5.12 (quin, *J* =7.2 Hz, 1H), 2.85 (td, *J* = 6.8, 13.6 Hz, 1H), 1.47 (d, *J* = 7.2 Hz, 3H), 1.18 (d, *J* = 6.8 Hz, 6H) ; LC/MS (ESI) m/z: 362.4 $[M+H]^+$ |

(continued)

| No. | Structure/Name | Spectral Data |
|---|---|---|
| 202 | 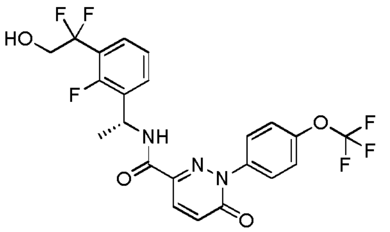 N-[(1R)-1-[3-(1,1-difluoro-2-hydroxy-ethyl)-2-fluoro-phenyl]ethyl]-6-oxo-1-[4-(trifluoromethoxy)phenyl]pyridazine-3-carboxamide | $^1$H NMR (400 MHz, DMSO-$d_6$) δ = 9.00 (d, *J* = 8.0 Hz, 1H), 7.94-7.80 (m, 3H), 7.64 (t, *J* = 7.0 Hz, 1H), 7.57 (d, *J* = 8.4 Hz, 2H), 7.43 (t, *J* = 6.8 Hz, 1H), 7.31-7.24 (m, 1H), 7.16 (d, *J* = 10.0 Hz, 1H), 5.71 (br s, 1H), 5.41 (q, *J* = 7.2 Hz, 1H), 3.92 (t, *J* = 14.4 Hz, 2H), 1.48 (d, *J* = 7.2 Hz, 3H); LC/MS (ESI) m/z: 502.1 $[M+H]^+$ |
| 203 | 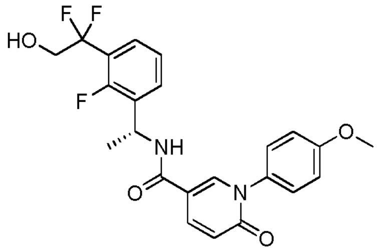 N-[(1R)-1-[3-(1,1-difluoro-2-hydroxy-ethyl)-2 -fluoro-phenyl]ethyl]-1-(4-methoxyphenyl)-6-oxo-pyridine-3-carboxamide | $^1$H NMR (400 MHz, DMSO-$d_6$) δ = 8.70 (br d, *J* = 7.6 Hz, 1H), 8.39 (d, *J* = 2.8 Hz, 1H) , 7.92 (dd, *J* = 2.8, 9.6 Hz, 1H), 7.57 (t, *J* = 7.2 Hz, 1H) , 7.46-7.37 (m, 3H), 7.31-7.24 (m, 1H), 7.11-7.06 (m, 2H), 6.51 (d, *J* = 9.6 Hz, 1H), 5.75 (br s, 1H), 5.35 (quin, *J* = 7.2 Hz, 1H), 3.92 (t, *J* = 14.4 Hz, 2H), 3 .82 (s, 3H), 1.43 (d, *J* = 7.2 Hz, 3H) LC/MS (ESI) m/z: 447.3 $[M+H]^+$ |
| 204 | 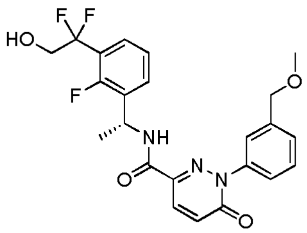 N-[(1R)-1-[3-(1,1-difluoro-2-hydroxy-ethyl)-2-fluoro-phenyl]ethyl]-1-[3-(methoxymethyl) phenyl]-6-oxo-pyridazine-3-carboxamide | $^1$H NMR (400 MHz, CHLOROFORM-*d*) δ = 8.00 (d, *J* = 9.6 Hz, 1H), 7.40-7.58 (m, 7H), 7.16-7.22 (m, 1H), 7.09 (d, *J* = 9.6 Hz, 1H), 5.38-5.48 (m, 1H), 4.53-4.60 (m, 2H), 3.98-4.13 (m, 2H), 3.44 (s, 3H), 2.64-2.72 (m, 1H), 1.59 (s, 3H) ; LC/MS (ESI) m/z: 462.2 $[M+H]^+$ |
| 205 | 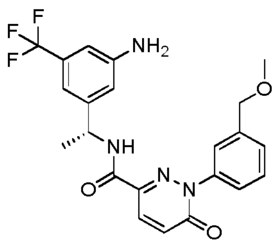 N-[(1R)-1-[3-amino-5-(trifluoromethyl)phenyl] ethyl]-1-[3-(methoxymethyl)phenyl]-6-oxo-pyridazine-3-carboxamide | 1H NMR (400 MHz, CHLOROFORM-d) δ = 8.01 (d, J = 9.6 Hz, 1H), 7.41-7.55 (m, 4H), 7.29 (d, J = 8.0 Hz, 1H), 7.09 (d, J = 9.8 Hz, 1H), 7.00 (s, 1H), 6.89 (d, J = 11.2 Hz, 2H), 5.19 (q, J = 7.2 Hz, 1H), 4.53-4.55 (m, 2H), 3.43 (s, 3H), 1.54 (d, J = 6.8 Hz, 3H); LC/MS (ESI) m/z: 447.2 $[M+H]^+$ |

(continued)

| No. | Structure/Name | Spectral Data |
|---|---|---|
| 206 | 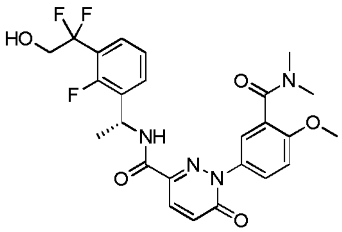 N-[(1R)-1-[3-(1,1-difluoro-2-hydroxy-ethyl)-2-fluoro-phenyl]ethyl]-1-[3-(dimethylcarbamoyl)-4-methoxy-phenyl]-6-oxo-pyridazine-3-carboxamide | 1H NMR (400 MHz, DMSO-$d_6$) $\delta$ = 8.98 (d, J = 8.0 Hz, 1H), 7.84 (d, J = 9.6 Hz, 1H), 7.69 (dd, J = 2.4, 8.8 Hz, 1H), 7.63 (t, J = 7.2 Hz, 1H), 7.51 (d, J = 2.8 Hz, 1H), 7.43 (t, J = 7.2 Hz, 1H), 7.31 -7.21 (m, 2H), 7.12 (d, J = 9.6 Hz, 1H), 5.72 (t, J = 6.6 Hz, 1H), 5.44 - 5.34 (m, 1H), 3.97 - 3.88 (m, 2H), 3.87 (s, 3H), 2.98 (s, 3H), 2.82 (s, 3H), 1.47 (d, J = 7.1 Hz, 3H); LC/MS (ESI) m/z: 519.4 $[M+H]^+$ |
| 207 | 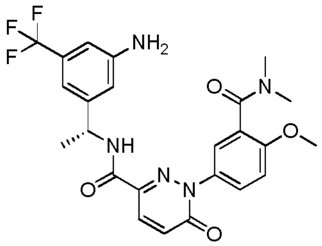 N-[(1R)-1-[3-amino-5-(trifluoromethyl)phenyl] ethyl]-1-[3-(dimethylcarbamoyl)-4-methoxy-phenyl]-6-oxo-pyridazine-3-carboxamide | 1H NMR (400 MHz, DMSO-$d_6$) $\delta$ = 8.87 (d, J = 8.3 Hz, 1H), 7.86 (d, J = 9.6 Hz, 1H), 7.68 (dd, J = 2.4, 8.8 Hz, 1H), 7.51 (d, J = 2.4 Hz, 1H), 7.22 (d, J = 9.2 Hz, 1H), 7.12 (d, J = 9.6 Hz, 1H), 6.80 (br d, J = 13.2 Hz, 2H), 6.70 (s, 1H), 5.55 (s, 2H), 5.02 (quin, J = 7.2 Hz, 1H), 3.86 (s, 3H), 2.97 (s, 3H), 2.80 (s, 3H), 1.44 (d, J = 7.0 Hz, 3H) ; LC/MS (ESI) m/z: 504.4 $[M+H]^+$ |
| 208 | 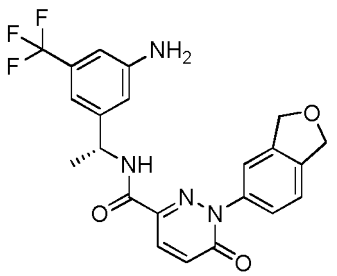 N-[(1R)-1-[3-amino-5-(trifluoromethyl)phenyl] ethyl]-1-(1,3-dihydroisobenzofuran-5-yl)-6-oxo-pyridazine-3-carboxamide | $^1$H NMR (400 MHz, DMSO-$d_6$) $\delta$ = 8.83 (d, *J* = 8.4 Hz, 1H), 7.89 (d, *J* = 9.6 Hz, 1H), 7.62 - 7.49 (m, 2H), 7.46 (d, *J* = 7.6 Hz, 1H), 7.14 (d, *J* = 9.6 Hz, 1H), 6.82 (s, 1H), 6.77 (s, 1H), 6.70 (s, 1H), 5.55 (s, 2H), 5.07 - 4.99 (m, 5H), 1.44 (d, *J* = 7.2 Hz, 3H) ; LC/MS (ESI) m/z: 445.4 $[M+H]^+$ |
| 209 | 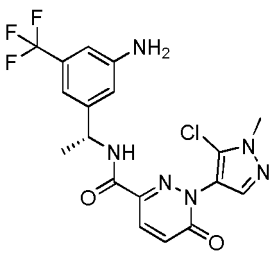 N-[(1R)-1-[3-amino-5-(trifluoromethyl)phenyl] ethyl]-1-(5-chloro-1-methyl-pyrazol-4-yl)-6-oxo-pyridazine-3-carboxamide | $^1$H NMR (400 MHz, DMSO-$d_6$) $\delta$ = 8.85 (d, *J* = 8.4 Hz, 1H), 7.92 (s, 1H), 7.89 (d, *J* = 9.6 Hz, 1H), 7.16 (d, *J* = 9.6 Hz, 1H), 6.81 (s, 1H), 6.77 (s, 1H), 6.70 (s, 1H), 5.56 (s, 2H), 5.01 (quin, *J* = 7.2 Hz, 1H), 3.88 (s, 3H), 1.43 (d, *J* = 7.2 Hz, 3H) ; LC/MS (ESI) m/z: 441.3 $[M+H]^+$ |

(continued)

| No. | Structure/Name | Spectral Data |
|---|---|---|
| 210 | 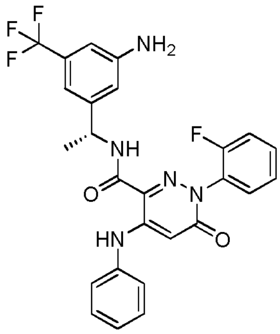 N-[(1R)-1-[3-amino-5-(trifluoromethyl)phenyl] ethyl] -4-anilino-1-(2-fluorophenyl)-6-oxo-pyridazine-3-carboxamide | $^1$H NMR (400 MHz, DMSO-$d_6$) δ ppm 1.45 (d, *J* = 7.2 Hz, 3 H) 5.06 (m, 1 H) 5.57 (s, 2 H) 6.05 (s, 1 H) 6.71 (s, 1 H) 6.80 (br d, *J* = 11.6 Hz, 2 H) 7.24 (m, 1 H) 7.40 (m, 6 H) 7.54 (m, 1 H) 7.62 (m, 1 H) 9.15 (m, 1 H) 10.11 (s, 1 H) ; LC/MS (ESI) m/z: 512.4 $[M+H]^+$ |
| 211 | 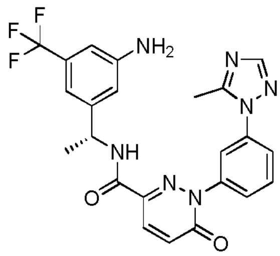 N-[(1R)-1-[3-amino-5-(trifluoromethyl)phenyl] ethyl]-1-[3-(5-methyl-1,2,4-triazol-1-yl)phenyl]-6-oxo-pyridazine-3-carboxamide | $^1$H NMR (400 MHz, DMSO-$d_6$) δ = 9.20 (s, 1H), 8.91 (d, J = 8.4 Hz, 1H), 8.14 (d, J = 1.2 Hz, 1H), 7.97 - 7.88 (m, 2H), 7.74 - 7.67 (m, 2H), 7.18 (d, J = 9.6 Hz, 1H), 6.80 (br d, J = 13.6 Hz, 2H), 6.70 (s, 1H), 5.55 (s, 2H), 5.04 (quin, J = 7.2 Hz, 1H), 2.37 (s, 3H), 1.44 (d, J = 7.2 Hz, 3H) ; LC/MS (ESI) m/z: 484.4 $[M+H]^+$ |
| 212 | 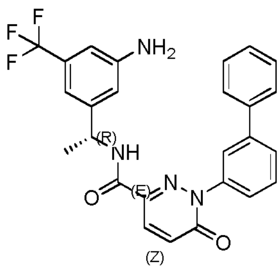 N-[(1R)-1-[3-amino-5-(trifluoromethyl)phenyl] ethyl]-6-oxo-1-(3-phenylphenyl)pyridazine-3-carboxamide | $^1$H NMR (400 MHz, DMSO-$d_6$) δ = 8.90 (d, *J* = 8.4 Hz, 1H), 7.95 - 7.89 (m, 2H), 7.80 - 7.75 (m, 1H), 7.72 (d, *J* = 7.2 Hz, 2H), 7.67 - 7.61 (m, 2H), 7.52 - 7.47 (m, 2H), 7.43 -7.38 (m, 1H), 7.17 (d, *J* = 9.6 Hz, 1H), 6.82 (s, 1H), 6.79 (s, 1H), 6.70 (s, 1H), 5.53 (s, 2H), 5.04 (quin, *J* = 7.2 Hz, 1H), 1.44 (d, *J* = 6.8 Hz, 3H) ; LC/MS (ESI) m/z: 351.4 $[M+H]^+$ |
| 213 | 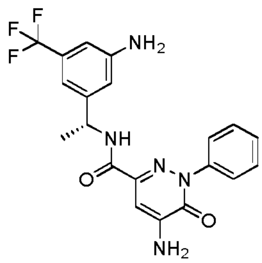 5-amino-N-[(1R)-1-[3-amino-5-(trifluoromethyl) phenyl]ethyl]-6-oxo-1-phenyl-pyridazine-3-carboxamide | $^1$H NMR (400 MHz, DMSO-$d_6$) δ = 8.51 (d, *J* = 8.4 Hz, 1H), 7.67 (d, *J* = 7.6 Hz, 2H), 7.52 (t, *J* = 7.2 Hz, 2H), 7.45-7.40 (m, 1H), 7.00-6.77 (m, 4H), 6.76 (s, 1H), 6.69 (s, 1H), 5.54 (s, 2H), 5.00 (quin, *J* = 7.2 Hz, 1H), 1.42 (d, *J* = 7.2 Hz, 3H); MS (ESI) m/z = 418.4 $[M+H]^+$. |

(continued)

| No. | Structure/Name | Spectral Data |
|---|---|---|
| 214 | 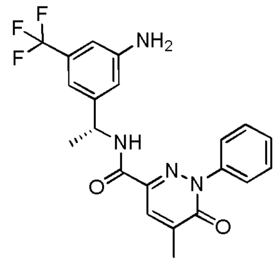 N-[(1R)-1-[3-amino-5-(trifluoromethyl)phenyl] ethyl]-5-methyl-6-oxo-1-phenyl-pyridazine-3-carboxamide | $^1$H NMR (400 MHz, DMSO-$d_6$) $\delta$ = 8.79 (d, $J$ = 8.4 Hz, 1H), 7.82 (d, $J$ = 1.2 Hz, 1H), 7.66 (d, $J$ = 7.6 Hz, 2H), 7.53 (t, $J$ = 7.2 Hz, 2H), 7.48 - 7.43 (m, 1H), 6.81 (s, 1H), 6.78 (s, 1H), 6.70 (s, 1H), 5.54 (s, 2H), 5.03 (quin, $J$ = 7.2 Hz, 1H), 2.19 - 2.17 (m, 3H), 1.44 (d, $J$ = 7.2 Hz, 3H); MS (ESI) m/z = 417.2 [M+H]$^+$. |

**Example 215: N-[(1R)-1-[3-(1,1-difluoro-2-hydroxy-ethyl)-2-fluoro-phenyl]ethyl]-1-(2-fluoro-4-methoxy-phenyl)-6-oxo-pyridazine-3-carboxamide**

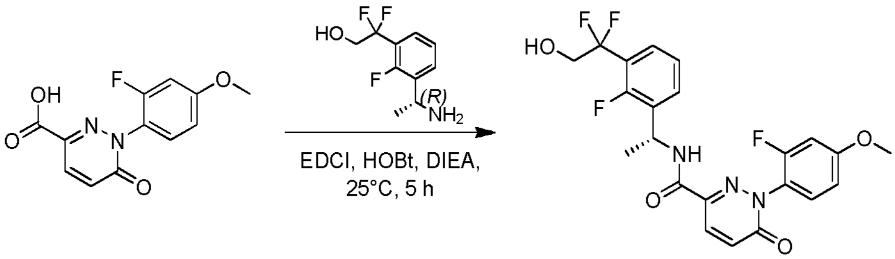


215

To a solution of 1-(2-fluoro-4-methoxy-phenyl)-6-oxo-pyridazine-3-carboxylic acid (125 mg, 473.11 µmol) and 2-[3-[(1R)-1-aminoethyl]-2-fluoro-phenyl]-2,2-difluoro-ethanol (120.96 mg, 473.11 µmol, HCl salt) in DMF (4 mL), DIEA (183.44 mg, 1.42 mmol, 247.22 µL), HOBt (127.86 mg, 946.22 µmol) and EDCI (181.39 mg, 946.22 µmol) were added, and the mixture was degassed, purged with $N_2$ for three times, and stirred at 25°C for 5 hours under $N_2$ atmosphere. The reaction mixture was poured into water (30 mL) and extracted with EtOAc (30 mL × 3). The combined organic layer was washed with brine (50 mL), dried over $Na_2SO_4$ and concentrated under reduced pressure to obtain a crude product, which was purified by silica gel column chromatography (1% MeOH in DCM) to give yellow oil. The product was purified by prep-HPLC (column: Welch Xtimate C18 150*30mm*5µm; mobile phase: [water ($NH_3H_2O$+$NH_4HCO_3$)-ACN];B%: 20%-60%, 28min). $CH_3CN$ was removed under reduced pressure, and the remaining solvent was removed by lyophilization to obtain the compound of Example 215 (20 mg, 9.08% yield) as a white solid. $^1$H NMR (400MHz, DMSO-$d_6$) $\delta$ 8.99 (br d, $J$ = 8.0 Hz, 1H), 7.91 (d, $J$ = 10.0 Hz, 1H), 7.67 - 7.54 (m, 2H), 7.43 (br t, $J$ = 6.8 Hz, 1H), 7.31 - 7.24 (m, 1H), 7.16 (d, $J$ = 10.0 Hz, 1H), 7.08 (dd, $J$ = 2.8, 12.0 Hz, 1H), 6.98 (dd, $J$ = 2.4, 9.2 Hz, 1H), 5.71 (t, $J$ = 6.4 Hz, 1H), 5.41 (br t, $J$ = 7.6 Hz, 1H), 3.92 (br d, $J$ = 6.8 Hz, 2H), 3.86 (s, 3H), 1.47 (d, $J$ = 7.2 Hz, 3H); LC/MS (ESI) m/z = 466.3 [M+H]$^+$.

**Example 216: N-[(1R)-1-(5-amino-3-cyano-2-fluoro-phenyl)ethyl]-1-(2-fluorophenyl)-6-oxo-pyridazine-3-carboxamide**

Step 1: Synthesis of 3-bromo-2-fluoro-5-nitro-benzonitrile

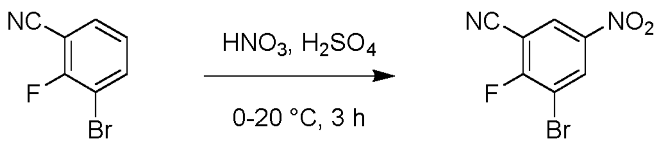

To a solution of 3-bromo-2-fluoro-benzonitrile (10 g, 50.00 mmol) in $H_2SO_4$ (40 mL) was added $HNO_3$ (17.520 g, 278.04 mmol, 12.51 mL) slowly at 0°C for 40 minutes, and the reaction mixture was stirred at 20 °C for 3 hours. The reaction mixture was poured into ice-cold water (70 mL) and extracted with EtOAc (40 mL × 3). The combined organic layer was washed with brine (50 mL), dried over $Na_2SO_4$ and concentrated under reduced pressure to obtain a crude product. The product was purified by silica gel column chromatography (14% MeOH in DCM) to give 3-bromo-2-fluoro-5-nitro-benzonitrile (7.57 g, 61.80% yield) as yellow oil. $^1$H NMR (400MHz, DMSO-$d_6$) δ 8.92 (m, 1 H) 8.95 (m, 1 H).

Step 2: Synthesis of 3-acetyl-2-fluoro-5-nitro-benzonitrile

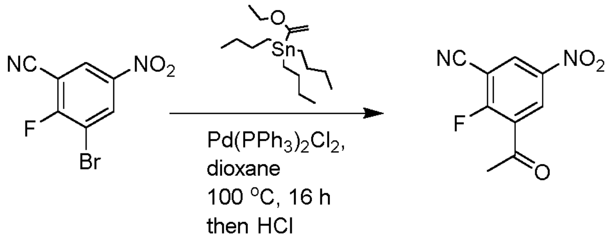


To a mixture of 3-bromo-2-fluoro-5-nitro-benzonitrile (4.5 g, 18.37 mmol) and tributyl(1-ethoxyvinyl)stannane (7.67 g, 21.23 mmol, 7.16 mL) in dioxane (50 mL), $Pd(PPh_3)_2Cl_2$ (1.29 g, 1.84 mmol) and TEA (3.72 g, 36.74 mmol, 5.11 mL) were added, and the reaction mixture was stirred at 100°C for 16 hours under $N_2$ atmosphere. Then, the reaction mixture was cooled to 0°C, treated with 4M HCl (30 mL) and stirred for 3 hours. The reaction mixture was diluted with water (50 mL) and extracted with EtOAc (50 mL × 3). The product was dried over $Na_2SO_4$ and filtered, and the filtrate was concentrated. The aqueous layer was adjusted to pH= 9 with aq. NaOH, and then sodium hypochlorite (100 mL) was added slowly under stirring. The product was purified by silica gel column chromatography (14% EtOAc in PE) to give 3-acetyl-2-fluoro-5-nitrobenzonitrile (2.58 g, 67.45% yield) as a yellow solid. $^1$H NMR (400MHz, DMSO-$d_6$) δ 2.69 (d, *J* = 4.0 Hz, 3H) 8.77 (dd, *J* = 6.0, 3.2 Hz, 1H) 9.14 (dd, *J* = 4.8, 2.8 Hz, 1H).

Step 3: Synthesis of (NZ,R)-N-[1-(3-cyano-2-fluoro-5-nitro-phenyl)ethylidene]-2-methyl-propane-2-sulfinamide

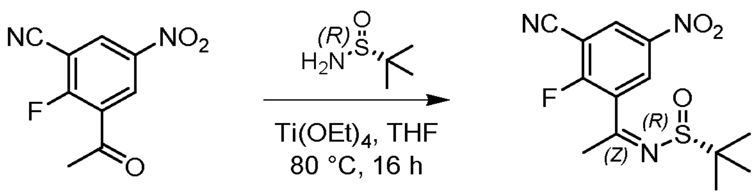


To a solution of 3-acetyl-2-fluoro-5-nitro-benzonitrile (2.58 g, 12.40 mmol) and (R)-2-methylpropane-2-sulfinamide (2.25 g, 18.59 mmol) in THF (25 mL) was added $Ti(OEt)_4$ (8.48 g, 37.19 mmol, 7.71 mL), and the reaction mixture was stirred at 80 °C for 16 hours under $N_2$ atmosphere. The reaction mixture was poured into water (30 mL) and extracted with EtOAc (30 mL×3). The combined organic layer was washed with brine (50 mL), dried over $Na_2SO_4$ and concentrated under reduced pressure to obtain a crude product, which was purified by silica gel column chromatography (14% EtOAc in PE) to give (NZ,R)-N-[1-(3-cyano-2-fluoro-5-nitrophenyl)ethylidene]-2-methyl-propane-2-sulfinamide (1.18 g, 30.58% yield) as yellow oil. $^1$H NMR (400MHz, DMSO-$d_6$) δ 1.24 (s, 9 H) 2.75 (d, *J* = 1.6 Hz, 3H) 8.76 (br dd, *J* = 5.6, 2.6 Hz, 1 H) 9.04 (dd, *J* = 4.4, 2.8 Hz, 1H).

Step 4: Synthesis of (NZ,R)-N-[1-(3-cyano-2-fluoro-5-nitro-phenyl)ethylidene]-2-methyl-propane-2-sulfinamide

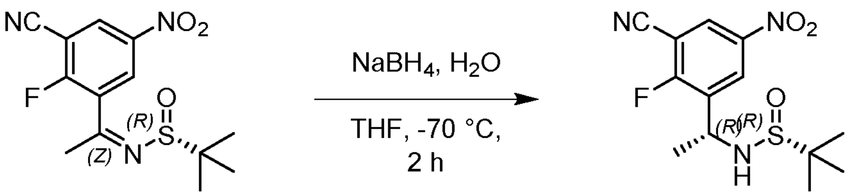


To a solution of (NZ,R)-N-[1-(3-cyano-2-fluoro-5-nitro-phenyl)ethylidene]-2-methylpropane-2-sulfinamide (1.18 g, 3.79 mmol) in THF (12 mL) and $H_2O$ (0.5 mL) was added $NaBH_4$ (170 mg, 4.49 mmol), and the reaction mixture was stirred at -70 °C for 2 hours under $N_2$ atmosphere. The reaction mixture was quenched with sat. aq. $NH_4Cl$ (50 mL) at 20°C, diluted with EtOAc (50 mL) and extracted with EtOAc (50 mL * 3). The organic layer was dried over $Na_2SO_4$ and filtered under reduced pressure to obtain a crude product. The product was purified by prep-HPLC (column: Phenomenex C18 75*30mm*3μm, mobile phase: [water($NH_3H_2O$+$NH_4HCO_3$)-ACN];B%: 24%-56%,11min). $CH_3CN$ was removed under reduced pressure, and the remaining solvent was removed by lyophilization to give (NZ,R)-N-[1-(3-cyano-2-fluoro-5-nitrophenyl)ethylidene]-2-methyl-propane-2-sulfinamide (156 mg, 13.08% yield) as a yellow solid. $^1H$ NMR (400MHz, DMSO-$d_6$) δ 1.13 (s, 9H) 1.45 (s, 3H) 4.03 (q, *J* = 7.2 Hz, 1 H) 8.80 (dd, *J* = 6.0, 2.8 Hz, 1 H) 8.85 (m, 1H); LC/MS (ESI) m/z = 314.1 $[M+H]^+$.

Step 5: Synthesis of 3-[(1R)-1-aminoethyl]-2-fluoro-5-nitro-benzonitrile

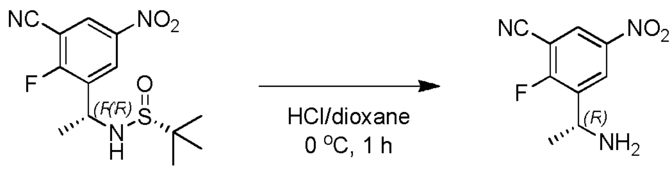


**Intermediate AW**

(NZ,R)-N-[1-(3-cyano-2-fluoro-5-nitro-phenyl)ethylidene]-2-methyl-propane-2-sulfinamide (156 mg, 497.85 μmol) was added to 4N HCl/dioxane (3 mL), and the mixture was stirred at 0 °C for 1 hour. The mixture was filtered under reduced pressure to give Intermediate AW (104 mg, 85.04% yield, HCl salt) as a yellow solid. LC/MS (ESI) m/z = 210.1 $[M+H]^+$.

Step 6: Synthesis of N-[(1R)-1-(3-cyano-2-fluoro-5-nitro-phenyl)ethyl]-1-(2-fluorophenyl)-6-oxo-pyridazine-3-carboxamide

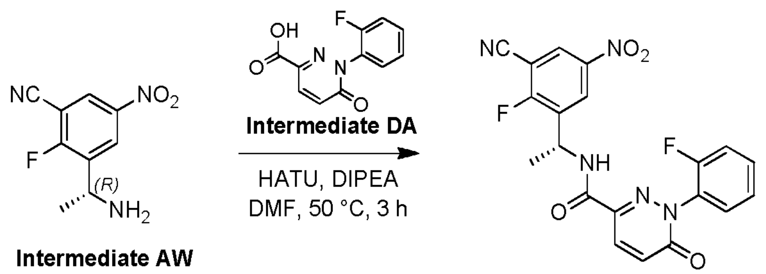


**Intermediate AW**

To a solution of Intermediate AW (40 mg, 162.84 μmol, HCl salt) and Intermediate DA (34.67 mg, 148.04 μmol) in DMF (3 mL), HATU (73.17 mg, 192.45 μmol) and DIEA (57.40 mg, 444.11 μmol, 77.36 μL) were added, and the reaction mixture was stirred at 50 °C for 3 hours under $N_2$. The reaction mixture was poured into water (30 mL) and extracted with EtOAc (30 mL×3). The combined organic layer was washed with brine (50 mL), dried over anhydrous $Na_2SO_4$ and concentrated under reduced pressure to give N-[(1R)-1-(3-cyano-2-fluoro-5-nitrophenyl)ethyl]-1-(2-fluorophenyl)-6-oxo-pyridazine-3-carboxamide (120 mg, 55.27% yield) as yellow oil. LC/MS (ESI) m/z = 426.0 $[M+H]^+$.

Step 7: Synthesis of N-[(1R)-1-(5-amino-3-cyano-2-fluoro-phenyl)ethyl]-1-(2-fluorophenyl)-6-oxo-pyridazine-3-carboxamide

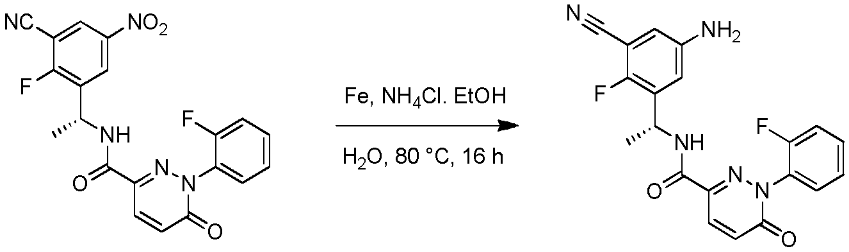


216

To a solution of N-[(1R)-1-(3-cyano-2-fluoro-5-nitro-phenyl)ethyl]-1-(2-fluorophenyl)-6-oxo-pyridazine-3-carboxamide (120.00 mg, 282.12 μmol) in EtOH (2.5 mL) and $H_2O$ (0.5 mL), Fe (78.78 mg, 1.41 mmol) and $NH_4Cl$ (75.46 mg, 1.41 mmol) were added, and the reaction mixture was stirred at 85 °C for 3 hours under $N_2$. The reaction mixture was filtered, and the filtrate was concentrated under reduced pressure and purified by prep-HPLC (column: Phenomenex C18 75*30mm*3μm, mobile phase: [water($NH_3H_2O$+$NH_4HCO_3$)-ACN];B%: 24%-54%,14 min). $CH_3CN$ was removed under reduced pressure, and the remaining solvent was removed by lyophilization to give the compound of Example 216 (6.8 mg, 16.02, 5.68% yield) as a yellow solid. $^1H$ NMR (400 MHz, DMSO-$d_6$) δ 1.42 (d, *J* = 7.2 Hz, 3 H) 5.18 (quin, *J* = 7.2 Hz, 1 H) 5.45 (s, 2 H) 6.72 (dd, *J* = 4.8, 2.81 Hz, 1 H), 6.87 (dd, *J* = 6.4, 2.75 Hz, 1 H) 7.19 (d, *J* = 10.0 Hz, 1 H) 7.44 (m, 2 H) 7.59 (m, 1 H) 7.69 (td, *J* = 7.6, 1.56 Hz, 1 H) 7.93 (d, *J* = 10.0 Hz, 1 H) 9.00 (d, *J* = 7.6 Hz, 1 H); LC/MS (ESI) m/z = 396.3 $[M+H]^+$.

**Example 217: N-[(1R)-1-[3-(1,1-difluoro-2-hydroxy-ethyl)-2-fluoro-phenyl]ethyl]-1-(2-fluorophenyl)-6-oxo-pyridazine-3-carboxamide**

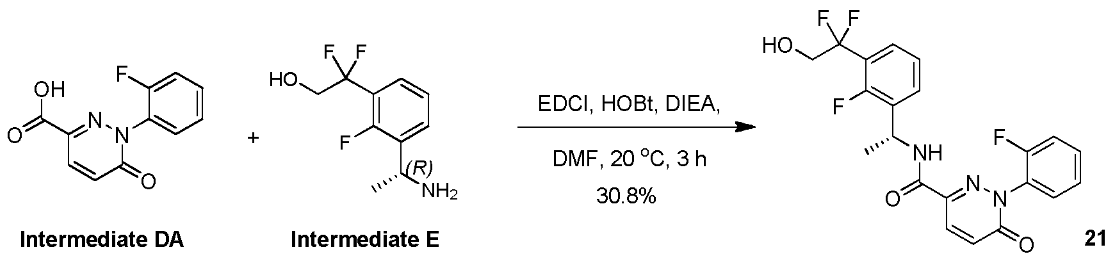


Intermediate DA

Intermediate E

217

To a solution of Intermediate DA (30.13 mg, 128.66 μmol) and Intermediate E (31.02 mg, 141.52 μmol) in DMF (2 mL), EDCI (49.33 mg, 257.32 μmol), HOBt (34.77 mg, 257.32 μmol) and DIEA (49.88 mg, 385.98 μmol, 67.23 μL) were added, vacumming of the reaction vessel and purging with nitrogen gas were performed three times, and then the mixture was stirred at 25 °C for 3 hours. After LC/MS confirmed the consumption of the starting materials and the MS of the product, the reaction mixture was poured into water (30 mL) and extracted with EtOAc. The combined organic layer was washed with brine, dried over $Na_2SO_4$ and concentrated under reduced pressure to obtain a crude product. The product was purified by prep-HPLC (column: Phenomenex C18 75*30mm*3μm, mobile phase: [water($NH_3H_2O$+$NH_4HCO_3$)-ACN];B%: 22%-52%,14 min), and the resulting mixture was subjected to the lyophilization condition, thereby obtaining the compound of Example 217 (17.3 mg, 30.8% yield) as a pink solid. $^1H$ NMR (400 MHz, DMSO-$d_6$) δ 9.03 (d, J = 8.0 Hz, 1H), 7.92 (d, J = 10.0 Hz, 1H), 7.69 (dt, J = 1.6, 7.6Hz, 1H), 7.64 - 7.56 (m, 2H), 7.49 - 7.40 (m, 3H), 7.27 (t, J = 7.6 Hz, 1H), 7.18 (d, J = 9.6 Hz, 1H), 5.71 (br s, 1H), 5.40 (quin, J = 7.2 Hz, 1H), 3.91 (t, J = 14.4 Hz, 2H), 1.46 (d, J = 7.2 Hz, 3H); LC/MS (ESI) m/z = 436.0 $[M+H]^+$

**Example 218: N-[(1R)-1-[3-amino-5-(trifluoromethyl)phenyl]ethyl]-1-(2-fluorophenyl)-6-oxo-pyridine-3-carboxamide**

Step 1: Synthesis of methyl 1-(2-fluorophenyl)-6-oxo-pyridine-3-carboxylate

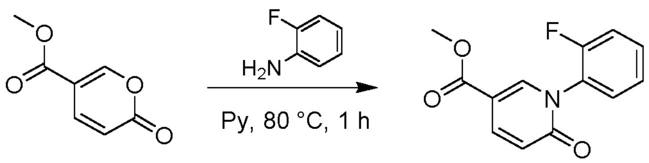


A mixture of methyl 6-oxopyran-3-carboxylate (18 g, 116.79 mmol) and 2-fluoroaniline (14.28 g, 128.47 mmol, 12.41 mL) in pyridine (180 mL) was stirred at 60°C for 1 hour. The reaction mixture was concentrated under reduced pressure to obtain a residue, which was purified by silica gel column chromatography(11% EtOAc in PE) to obtain methyl 1-(2-fluorophenyl)-6-oxo-pyridine-3-carboxylate (11 g, 21.19% yield, 55.61% purity) as yellow oil. LC/MS (ESI) m/z = 247.9 $[M+H]^+$.

Step 2: Synthesis of 1-(2-fluorophenyl)-6-oxo-pyridine-3-carboxylic acid

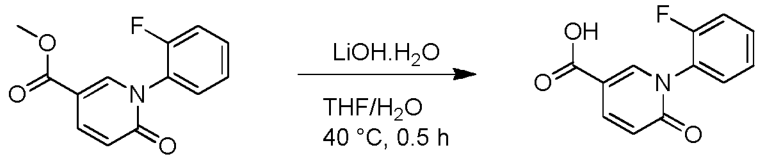


**Intermediate AQ-1**

To a solution of methyl 1-(2-fluorophenyl)-6-oxo-pyridine-3-carboxylate (10 g, 40.45 mmol) in THF (180 mL), $LiOH{\cdot}H_2O$ (5.09 g, 121.35 mmol) and $H_2O$ (40 mL) were added. The mixture was stirred at 40°C for 30 minutes. The reaction mixture was poured into water (50 mL) and extracted with EtOAc (40 mL × 2). The organic layer was discarded, and the mixture was adjusted to pH 3-4 with aq. 1N HCl and extracted with EtOAc (50 mL × 7). The combined organic layer was washed with brine (60 mL × 6), dried over $Na_2SO_4$ and concentrated under reduced pressure, and the crude product was recrystallized with EtOAc (20 mL) at 20 °C and filtered to give Intermediate AQ-1 (4.4 g, 38.52% yield, 82.59% purity) as a yellow solid. LC/MS (ESI) m/z = 234.1 $[M+H]^+$.

Step 3: Synthesis of N-[(1R)-1-[3-amino-5-(trifluoromethyl)phenyl]ethyl]-1-(2-fluorophenyl)-6-oxo-pyridine-3-carboxamide

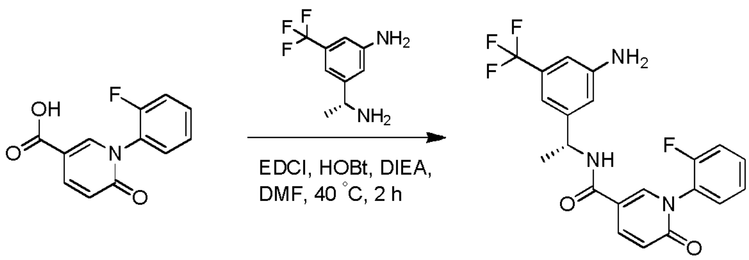


**218**

To a solution of 1-(2-fluorophenyl)-6-oxo-pyridine-3-carboxylic acid (4.4 g, 18.87 mmol) and 3-[(1R)-1-aminoethyl]-5-(trifluoromethyl)aniline (4.09 g, 16.98 mmol, HCl salt) in DMF (60 mL), DIEA (7.32 g, 56.61 mmol, 9.86 mL), HOBt (3.82 g, 28.30 mmol) and EDCI (5.43 g, 28.30 mmol) were added, and the mixture was degassed and purged with $N_2$

for three times, followed by stirring at 40 °C for 2 hours under $N_2$ atmosphere. The reaction mixture was poured into water (30 mL) and extracted with EtOAc (30 mL × 3). The combined organic layer was washed with brine (50 mL), dried over $Na_2SO_4$, and concentrated under reduced pressure to obtain a crude product, which was purified by silica gel column chromatography (73% EtOAc in PE) to give a main product, the compound of Example 218 (4.40 g, 55.27% yield) as an off-white solid. $^1H$ NMR (400MHz, DMSO-$d_6$) δ 8.59 (br d, *J* = 7.6 Hz, 1H), 8.38 (d, *J* = 2.4 Hz, 1H), 7.99 (dd, *J* = 2.4, 9.6 Hz, 1H), 7.66 - 7.53 (m, 2H), 7.52 - 7.34 (m, 2H), 6.76 (s, 2H), 6.70 (s, 1H), 6.57 (d, *J* = 9.6 Hz, 1H), 5.58 (s, 2H), 5.00 (br t, *J* = 7.2 Hz, 1H), 1.39 (d, *J* = 7.2 Hz, 3H); LC/MS (ESI) m/z = 421.3 $[M+H]^+$.

**Example 219: N-[(1R)-1-(3-fluoro-5-hydroxy-phenyl)ethyl]-6-oxo-1-phenyl-pyridazine-3-carboxamide**

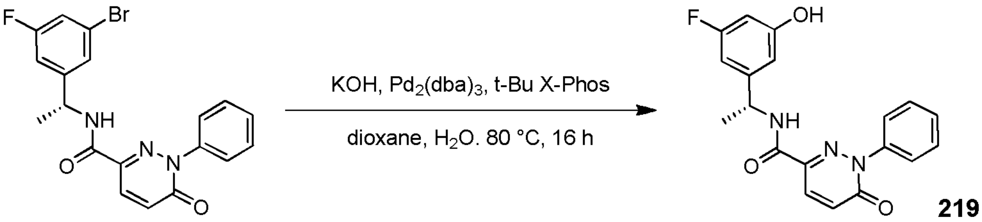


**219**

A mixture of N-[(1R)-1-(3-bromo-5-fluoro-phenyl)ethyl]-6-oxo-1-phenyl-pyridazine-3-carboxamide (125 mg, 0.300 mmol), KOH (84.2 mg, 1.50 mmol), t-Bu X-Phos (15.3 mg, 0.0360 mmol, 0.12 eq) and $Pd_2(dba)_3$ (11.0 mg, 0.0120 mmol, 0.04 *eq*) in dioxane (1 mL) and $H_2O$ (0.6 mL) was degassed and purged with $N_2$ for 3 times, and then the mixture was stirred at 80 °C for 16 hours under $N_2$ atmosphere. The reaction mixture was poured into water (20 mL) and extracted with EtOAc (30 mL × 3). The combined organic layer was washed with brine (20 mL ×3), dried over anhydrous $Na_2SO_4$ and concentrated under reduced pressure to obtain a crude product. The product was purified by silica gel column chromatography (50% EtOAc in PE) to obtain a residue, which was purified by prep-HPLC (C18-6 100*30mm*5μm; mobile phase: [water (FA)-ACN]; B%: 32%-62%, 15min). Most of $CH_3CN$ was removed under reduced pressure, and the remaining solvent was removed by lyophilization to give the compound of Example 219 (15.4 mg, 13.54% yield) as a light-yellow solid. $^1H$ NMR (400MHz, DMSO-$d_6$) δ = 9.84 (s, 1H), 8.80 (d, *J* = 8.4 Hz, 1H), 7.89 (d, *J* = 9.6 Hz, 1H), 7.70-7.62 (m, 2H), 7.57-7.51 (m, 2H), 7.50-7.44 (m, 1H), 7.14 (d, *J* = 9.6 Hz, 1H), 6.65-6.58 (m, 2H), 6.40 (td, *J* = 2.4, 10.8 Hz, 1H), 5.03 (quin, *J* = 7.2 Hz, 1H), 1.43 (d, *J* = 7.2 Hz, 3H); MS (ESI) m/z = 354.1$[M+H]^+$.

**Example 220: N-[(1R)-1-[3-amino-5-(trifluoromethyl)phenyl]ethyl]-4-chloro-6-oxo-1-phenyl-pyridazine-3-carboxamide**

Step 1: Synthesis of 4-hydroxy-6-oxo-1-phenyl-pyridazine-3-carboxylic acid

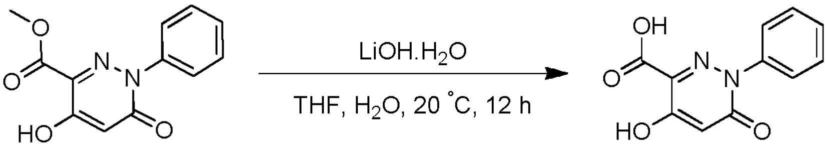


4-hydroxy-6-oxo-1-phenyl-pyridazine-3-carboxylic acid was prepared by reacting methyl 4-hydroxy-6-oxo-1-phenyl-pyridazine-3-carboxylate obtained from Step 2 of Preparation Example 8 in the same manner as in Step 1 of Example 76.

Step 2: Synthesis of 4-chloro-6-oxo-1-phenyl-pyridazine-3-carbonyl chloride

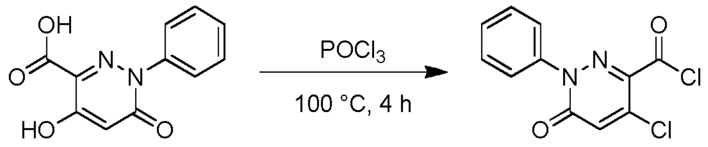


A solution of 4-hydroxy-6-oxo-1-phenyl-pyridazine-3-carboxylic acid (400 mg, 1.72 mmol) in $POCl_3$ (9.90 g, 64.6 mmol, 6.00 mL) was stirred at 100 °C for 4 hours under $N_2$. The reaction mixture was concentrated under reduced pressure to give 4-chloro-6-oxo-1-phenyl-pyridazine-3-carbonyl chloride (450 mg, crude) as brown oil, which was used in next step without further purification. MS (ESI) m/z = 265.0 $[M-2-H]^+$).

Steps 3 and 4: Synthesis of N-[(1R)-1-[3-amino-5-(trifluoromethyl)phenyl]ethyl]-4-chloro-6-oxo-1-phenyl-pyridazine-3-carboxamide

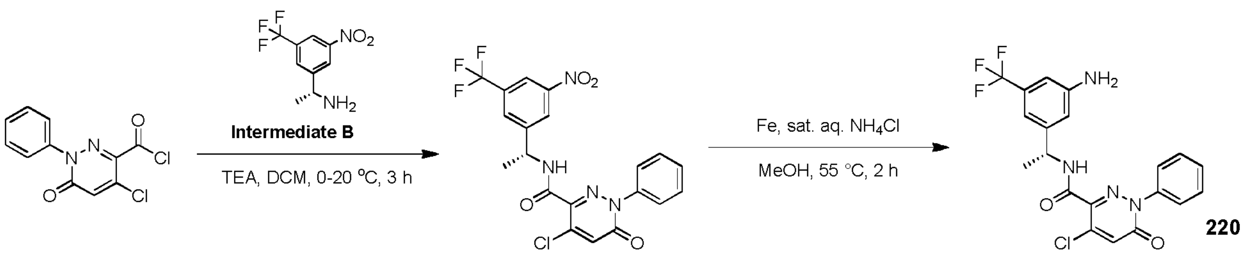


**220**

The compound of Example 220 (28.0 mg, 39.90% yield) was obtained in the same manner as in Steps 3 and 4 of Example 20 as a white solid. $^1H$ NMR (400MHz, DMSO-$d_6$) $\delta$ = 9.23 (d, *J* = 8.0 Hz, 1H), 7.63-7.57 (m, 2H), 7.56-7.49 (m, 3H), 7.49-7.44 (m, 1H), 6.79 (d, *J* = 14.0 Hz, 2H), 6.72 (s, 1H), 5.57 (s, 2H), 4.96 (quin, *J* = 7.2 Hz, 1H), 1.40 (d, *J* = 7.2 Hz, 3H); MS (ESI) m/z = 437.3 $[M+H]^+$.

**Example 221: N-[(1R)-1-[3-amino-5-(trifluoromethyl)phenyl]ethyl]-6-oxo-1H-pyridazine-3-carboxamide**

Step 1: Synthesis of methyl 6-oxo-1-(2-trimethylsilylethoxymethyl)pyridazine-3-carboxylate

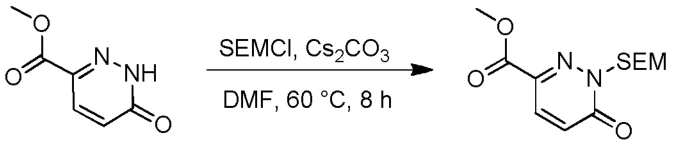


To a solution of methyl 6-oxo-1H-pyridazine-3-carboxylate (1 g, 6.49 mmol) in DMF (15 mL), $Cs_2CO_3$ (6.34 g, 19.46 mmol) and 2-(chloromethoxy)ethyl-trimethyl-silane (2.70 g, 16.22 mmol, 2.87 mL) were added. The mixture was stirred at 60 °C for 8 hours. The reaction mixture was poured into water (20 mL) and extracted with EtOAc (30 mL ×3). The combined organic layer was washed with brine (20 mL × 3), dried over anhydrous $Na_2SO_4$ and concentrated under reduced pressure to obtain a crude product. The product was purified by silica gel column chromatography (1% EtOAc in PE) to give methyl 6-oxo-1-(2-trimethylsilylethoxymethyl)pyridazine-3-carboxylate (650 mg, 34.22% yield) as light-yellow oil. $^1H$ NMR (400MHz, DMSO-$d_6$) $\delta$ = 7.83 (d, *J* = 4.4 Hz, 1H), 6.96 (d, *J* = 4.0 Hz, 1H), 5.54 (d, *J* = 2.4 Hz, 2H), 3.96 (s, 3H), 3.76-3.72 (m, 2H), 1.19-1.09 (m, 2H), 0.08 (s, 9H); MS (ESI) m/z = 227.0 $[M+H-58]^+$.

Steps 2 to 5: Synthesis of N-[(1R)-1-[3-amino-5-(trifluoromethyl)phenyl]ethyl]-6-oxo-1H-pyridazine-3 -carboxamide

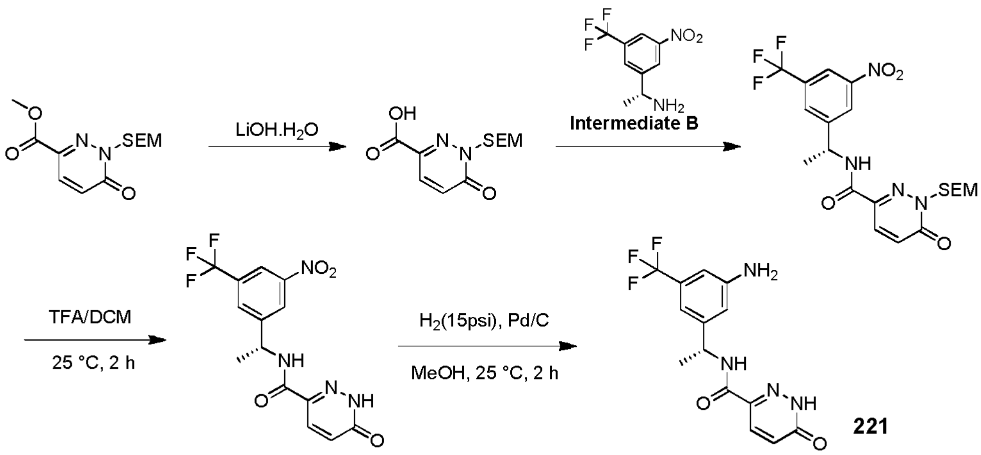


**221**

The compound of Example 221 (25.7 mg, 46.77% yield) was obtained as a white solid in a similar manner to Steps 2 to 5 of Example 79, except that Steps 4 and 5 of Example 79 were exchanged from each other and Pd/C catalyst was used instead of Pt-V/C in Step 4 of Example 79. $^1$H NMR (400MHz, DMSO-$d_6$) δ = 9.67 (d, *J* = 8.0 Hz, 1H), 9.12 (br s, 1H), 8.89 (s, 1H), 8.80 (s, 1H), 8.32 (d, *J* = 10.0 Hz, 1H), 7.54 (d, *J* = 10.0 Hz, 1H), 5.95 (q, *J* = 9.6 Hz, 2H), 2.08 (d, *J* = 7.2 Hz, 3H); MS (ESI) m/z = 327.3 $[M+H]^+$.

**Example 222: N-[(1R)-1-[3-amino-5-(trifluoromethyl)phenyl]ethyl]-1-(2-methoxyethyl)-6-oxo-pyridazine-3-carboxamide**

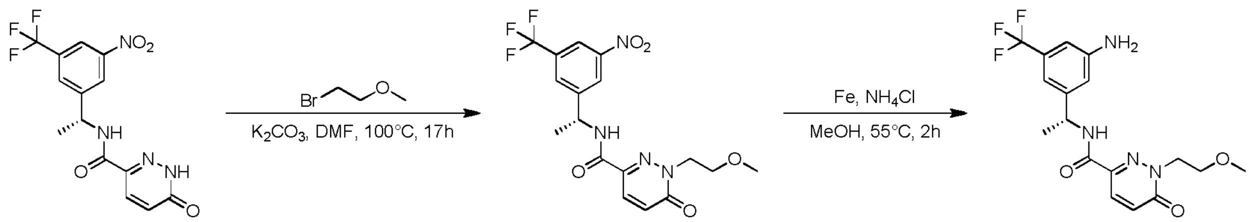


**222**

The compound of Example 222 (10.3 mg, 32.53% yield) was obtained as a white solid by reacting 1-[3-nitro-5-(trifluoromethyl)phenyl]ethyl]-6-oxo-lH-pyridazine-3 -carboxamide obtained from Step 4 of Example 221 and 1-bromo-2-methoxy-ethane in the same manner as in Step 1 of Example 118 and Step 4 of Example 89. $^1$H NMR (400MHz, DMSO-$d_6$) δ = 8.81 (d, *J* = 8.8 Hz, 1H), 7.81 (d, *J* = 9.6 Hz, 1H), 7.01 (d, *J* = 9.6 Hz, 1H), 6.81 (d, *J* = 16.4 Hz, 2H), 6.71 (s, 1H), 5.57 (s, 2H), 5.09 - 5.00 (m, 1H), 4.36 - 4.29 (m, 2H), 3.77 (t, *J* = 6.0 Hz, 2H), 3.25 (s, 3H), 1.47 (d, *J* = 7.2 Hz, 3H); MS (ESI) m/z = 385.1 $[M+H]^+$.

**Example 223 to Example 228**

The compounds of Examples 223 to 228 were obtained in the same manner as in Example 222 by using appropriate starting materials corresponding to the respective structures of the desired compounds.

[Table 12]

| No. | Structure/Name | Spectral Data |
|---|---|---|
| 223 | 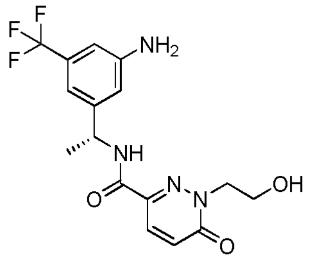 N-[(1R)-1-[3-amino-5-(trifluoromethyl)phenyl] ethyl]-1-(2-hydroxyethyl)-6-oxo-pyridazine-3-carboxamide | $^1$H NMR (400 MHz, DMSO-$d_6$) δ = 8.78 (br d, *J* = 8.2 Hz, 1H), 7.81 (d, *J* = 9.6 Hz, 1H), 7.00 (d, *J* = 9.6 Hz, 1H), 6.83 (s, 1H), 6.80 (s, 1H), 6.72 (s, 1H), 5.56 (s, 2H), 5.04 (quin, *J* = 7.2 Hz, 1H), 4.87 (t, *J* = 5.6 Hz, 1H), 4.28 - 4.16 (m, 2H), 3.80 (q, *J* = 6.0Hz, 2H), 1.48 (d, *J* = 7.2 Hz, 3H); LC/MS (ESI) m/z: 371.1 $[M+H]^+$ |
| 224 | 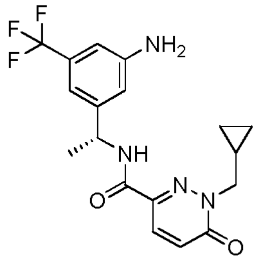 N-[(1R)-1-[3-amino-5-(trifluoromethyl)phenyl] ethyl]-1-(cyclopropylmethyl)-6-oxo-pyridazine-3-carboxamide | $^1$H NMR (400 MHz, DMSO-$d_6$) δ = 8.81 (d, *J* = 8.4 Hz, 1H), 7.82 (d, *J* = 10.0 Hz, 1H), 7.01 (d, *J* = 9.6 Hz, 1H), 6.83 (s, 1H), 6.78 (s, 1H), 6.71 (s, 1H), 5.57 (s, 2H), 5.09 - 4.99 (m, 1H), 4.07 - 3.93 (m, 2H), 1.47 (d, *J* = 7.2 Hz, 3H), 1.42 - 1.34 (m, 1H), 0.53 - 0.41 (m, 4H); LC/MS (ESI) m/z: 381.1 $[M+H]^+$ |
| 225 | 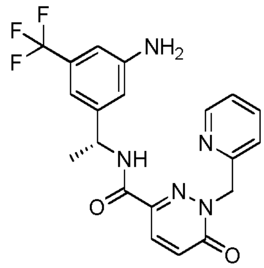 N-[(1R)-1-[3-amino-5-(trifluoromethyl)phenyl] ethyl]-6-oxo-1-(2-pyridylmethyl)pyridazine-3-carboxamide | $^1$H NMR (400 MHz, DMSO-$d_6$) δ ppm 1.44 (d, *J* = 7.00 Hz, 3H) 5.02 (t, *J* = 7.6 Hz, 1H) 5.41-5.51 (m, 2H) 5.55 (s, 2H) 6.70 (s, 1H) 6.79 (br d, *J* = 8.8 Hz, 2H) 7.06 (d, *J* = 10 Hz, 1H) 7.25-7.32 (m, 2H) 7.77 (td, *J* = 7.6, 1.69 Hz, 1H) 7.89 (d, *J* = 10 Hz, 1H) 8.48 (d, *J* = 4.4 Hz, 1H) 8.85 (d, *J* = 8.4 Hz, 1H); LC/MS (ESI) m/z: 418.3 $[M+H]^+$ |
| 226 | 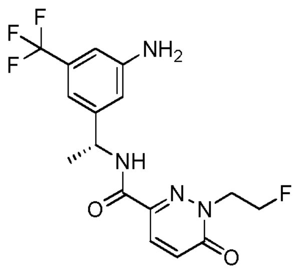 N-[(1R)-1-[3-amino-5-(trifluoromethyl)phenyl] ethyl]-1-(2-fluoroethyl)-6-oxo-pyridazine-3-carboxamide | $^1$H NMR (400 MHz, DMSO-$d_6$) δ = 8.86 - 8.77 (m, 1H), 7.89 - 7.80 (m, 1H), 7.04 (d, *J* = 9.6 Hz, 1H), 6.80 (br d, *J* = 11.2 Hz, 2H), 6.71 (s, 1H), 5.56 (s, 2H), 5.04 (t, *J* = 7.4 Hz, 1H), 4.95 (t, *J* = 4.8 Hz, 1H), 4.84 (t, *J* = 5.2 Hz, 1H), 4.52 - 4.40 (m, 2H), 1.47 (d, *J* = 7.2 Hz, 3H); LC/MS (ESI) m/z: 373.3 $[M+H]^+$ |

(continued)

| No. | Structure/Name | Spectral Data |
|---|---|---|
| 227 | 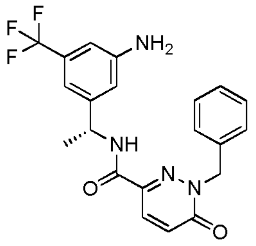 N-[(1R)-1-[3-amino-5-(trifluoromethyl)phenyl]ethyl]-1-benzyl-6-oxo-pyridazine-3-carboxamide | $^1$H NMR (400 MHz, DMSO-$d_6$) $\delta$ = 8.88 (d, $J$ = 8.4 Hz, 1H), 7.84 (d, $J$ = 10.0 Hz, 1H), 7.39 -7.29 (m, 5H), 7.03 (d, $J$ = 9.6 Hz, 1H), 6.84 (s, 1H), 6.79 (s, 1H), 6.71 (s, 1H), 5.57 (s, 2H), 5.33 (s, 2H), 5.04 (t, $J$ = 7.6 Hz, 1H), 1.48 (d, $J$ = 7.2 Hz, 3H); LC/MS (ESI) m/z: 417.3 $[M+H]^+$ |
| 228 | 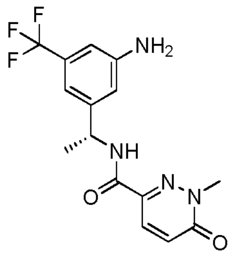 N-[(1R)-1-[3-amino-5-(trifluoromethyl)phenyl]ethyl]-1-methyl-6-oxo-pyridazine-3-carboxamide | $^1$H NMR (400 MHz, DMSO-$d_6$) $\delta$ = 8.85 (d, $J$ = 8.4 Hz, 1H), 7.82 (d, $J$ = 9.6 Hz, 1H), 7.00 (d, $J$ = 9.6 Hz, 1H), 6.83 (s, 1H), 6.79 (s, 1H), 6.70 (s, 1H), 5.56 (s, 2H), 5.07 - 4.97 (m, 1H), 3.75 (s, 3H), 1.46 (d, $J$ = 7.2 Hz, 3H); LC/MS (ESI) m/z: 341.3 $[M+H]^+$ |

**Example 229: N-[(1R)-1-[3-amino-5-(trifluoromethyl)phenyl]ethyl]-1-[2-(dimethylamino)ethyl]-6-oxo-pyridazine-3-carboxamide**

Step 1: Synthesis of 1-[2-(dimethylamino)ethyl]-N-[(1R)-1-[3-nitro-5-(trifluoromethyl)phenyl]ethyl]-6-oxo-pyridazine-3-carboxamide

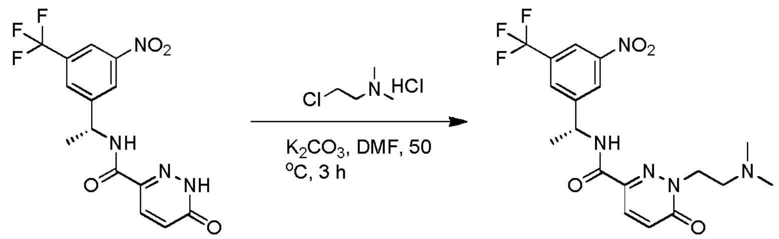


To a solution of N-[(1R)-1-[3-nitro-5-(trifluoromethyl)phenyl]ethyl]-6-oxo-1H-pyridazine-3-carboxamide (30.0 mg, 0.0842 mmol), which was obtained from Step 4 of Example 221, in DMF (1.5 mL), $K_2CO_3$ (34.9 mg, 0.253 mmol) and 2-chloro-N,N-dimethyl-ethanamine (18.2 mg, 0.126 mmol, HCl) were added. The mixture was stirred at 50 °C for 3 hours. The reaction mixture was poured into water (20 mL) and extracted with EtOAc (30 mL $\times$3). The combined organic layer was washed with brine (20 mL $\times$3), dried over anhydrous $Na_2SO_4$ and concentrated under reduced pressure to give 1-[2-(dimethylamino)ethyl]-N-[(1R)-1-[3-nitro-5-(trifluoromethyl)phenyl]ethyl]-6-oxo-pyridazine-3-carboxamide (35 mg, 94.33% yield) as yellow oil, which was used in next step without purification. MS (ESI) m/z = 428.2 $[M+H]^+$.

Step 2: Synthesis of N-[(1R)-1-[3-amino-5-(trifluoromethyl)phenyl]ethyl]-1-[2-(dimethylamino)ethyl]-6-oxo-pyridazine-3-carboxamide

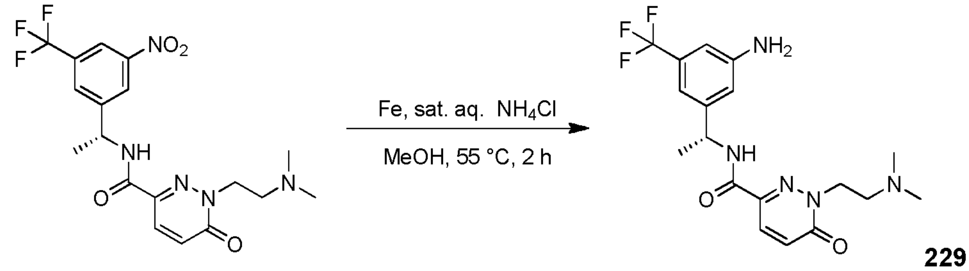


229

The compound of Example 229 (28.0 mg, 39.90% yield) was obtained as a white solid in the same manner as in Step 4 of Example 20. $^1$H NMR (400MHz, DMSO-$d_6$) $\delta$ = 8.82 (d, $J$ = 8.4 Hz, 1H), 7.81 (d, $J$ = 9.6 Hz, 1H), 7.01 (d, $J$ = 9.6 Hz, 1H), 6.83 (s, 1H), 6.78 (s, 1H), 6.71 (s, 1H), 5.57 (s, 2H), 5.04 (quin, $J$ = 7.2 Hz, 1H), 4.39-4.21 (m, 2H), 3.11-2.68 (m, 2H), 2.38-2.10 (m, 6H), 1.47 (d, $J$ = 7.2 Hz, 3H); MS (ESI) m/z = 398.4 $[M+H]^+$.

**Example 230: (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide**

Step 1: Synthesis of methyl (R)-2-fluoro-3-(3-((1-(3-nitro-5-(trifluoromethyl)phenyl)ethyl)carbamoyl)-6-oxopyridazin-1(6H)-yl)benzoate

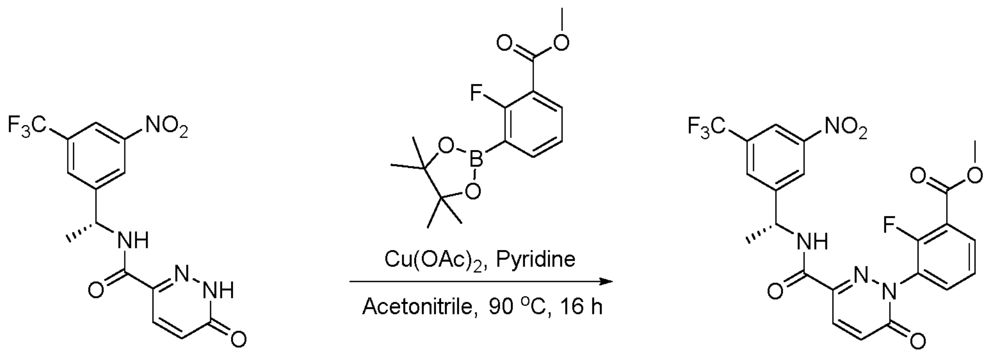


A mixture of the compound obtained from Step 4 of Example 221 (400 mg, 1.12 mmol), methyl 2-fluoro-3-(4,4,5,5-tetramethyl-1,3,2-dioxaborolan-2-yl)benzoate (472 mg, 1.68 mmol), $Cu(OAc)_2$ (204 mg, 1.12 mmol) and pyridine (362 $\mu$L, 4.49 mmol) in $CH_3CN$ (8.64 mL) was stirred at 90°C for 16 hours. The reaction mixture was poured into water (20 mL) and extracted with EtOAc. The combined organic layer was washed with aq. $CuSO_4.5H_2O$, dried over $Na_2SO_4$ and concentrated under reduced pressure to obtain a crude product. The product was purified by silica gel column chromatography (60% EtOAc in Hexane) to give methyl (R)-2-fluoro-3-(3-((1-(3-nitro-5-(trifluoromethyl)phenyl)ethyl)carbamoyl)-6-oxopyridazin-1(6H)-yl)benzoate (114 mg, 19.97% yield) as colorless oil. $^1$H NMR (400MHz, CHLOROFORM-*d*) $\delta$ 8.40 (s, 2H), 8.15-8.11 (m, 1H), 8.02 (d, $J$ = 10.0 Hz, 1H), 7.94 (s, 1H), 7.69-7.65 (m, 1H), 7.44-7.39 (m, 1H), 7.29 (d, $J$ = 7.6 Hz, 1H), 7.14 (d, $J$ = 10.0 Hz, 1H), 5.32 (quin, $J$ = 7.1 Hz, 1H), 1.65 (d, $J$ = 7.2 Hz, 3H); LC/MS (ESI) m/z = 509.0 $[M+H]^+$.

Step 2: Synthesis of (R)-2-fluoro-3-(3-((1-(3-nitro-5-(trifluoromethyl)phenyl)ethyl)carbamoyl)-6-oxopyridazin-1(6H)-yl)benzoic acid

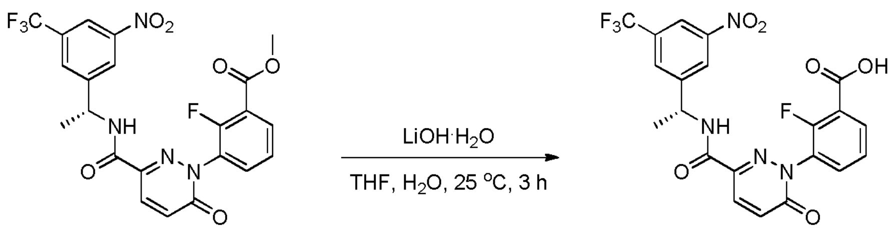


To a solution of methyl (R)-2-fluoro-3-(3-((1-(3-nitro-5-(trifluoromethyl)phenyl)ethyl)carbamoyl)-6-oxopyridazin-1(6H)-yl)benzoate (114 mg, 0.224 mmol) in THF (2.24 mL) and $H_2O$ (1.12 mL) was added LiOH·$H_2O$ (28.2 mg, 0.673 mmol). The mixture was stirred at room temperature for 3 hours. The reaction mixture was poured into water and extracted with EtOAc. The aqueous layer was adjusted to pH= 3-4 with aq. 1N HCl and extracted with EtOAc (10 mL × 2). The organic layer was washed with water (20 mL) and brine (20 mL), dried over $Na_2SO_4$, filtered and concentrated under reduced pressure to give (R)-2-fluoro-3-(3-((1-(3-nitro-5-(trifluoromethyl)phenyl)ethyl)carbamoyl)-6-oxopyridazin-1(6H)-yl)benzoic acid (112 mg, 100% yield) as a white solid. $^1H$ NMR (400MHz, DMSO-$d_6$) δ9.20 (d, *J* = 8.0 Hz, 1H), 8.59 (s, 1H), 8.37 (s, 1H), 8.27 (s, 1H), 8.02 (t, *J* = 6.8 Hz, 1H), 7.95-7.88 (m, 2H), 7.51 (t, *J* = 7.8 Hz, 1H), 7.20 (d, *J* = 10.0 Hz, 1H), 5.36 (quin, *J* = 7.2 Hz, 1H), 1.52 (d, *J* = 7.2 Hz, 3H); LC/MS (ESI) m/z = 493.0 $[M-H]^+$.

Step 3: Synthesis of (R)-1-(2-fluoro-3-(pyrrolidin-1-carbonyl)phenyl)-N-(1-(3-nitro-5-(tiifluoromethyl)phenyl)ethyl)-6-oxo-1,6-dihydropyridazine-3-carboxamide

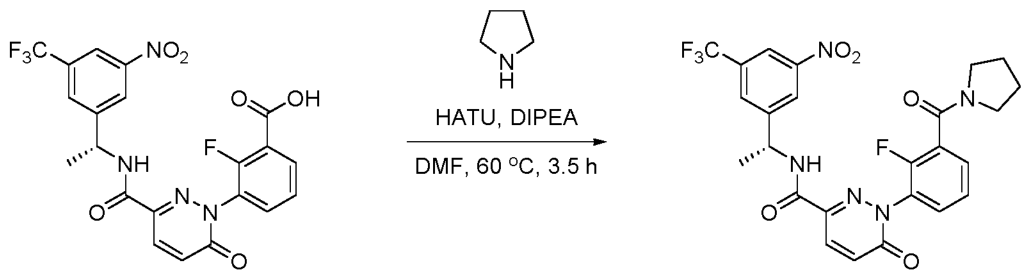


A mixture of (R)-2-fluoro-3-(3-((1-(3-nitro-5-(trifluoromethyl)phenyl)ethyl)carbamoyl)-6-oxopyridazin-1(6H)-yl)benzoic acid (19 mg, 38.4 μmol), pyrrolidine (9.47 μL, 115 μmol), DIPEA (26.8 μL, 154 μmol) and HATU (13.6 mg, 57.7 μmol) in DMF (192 μL) was degassed and purged with $N_2$ for 3 times, and then the mixture was stirred at 60 °C for 3.5 hours under $N_2$ atmosphere. The reaction mixture was poured into water (10 mL) and extracted with EtOAc (10 mL × 3). The combined organic layer was washed with brine (10 mL × 4), dried over anhydrous $Na_2SO_4$ and concentrated under reduced pressure to obtain a crude product. The product was purified by silica gel column chromatography (5% MeOH in DCM) to give (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide (19 mg, 90.30% yield) as a light-yellow solid. $^1H$ NMR (400MHz, CHLOROFORM-*d*) δ 8.41 (s, 2H), 8.02 (d, *J* = 9.6 Hz, 1H), 7.94 (s, 1H), 7.61-7.58 (m, 1H), 7.55-7.51 (m, 1H), 7.40 (t, *J* = 8.0 Hz, 1H), 7.34 (d, *J* = 6.8 Hz, 1H), 7.13 (d, *J* = 10.0 Hz, 1H), 5.31 (quin, *J* = 7.3 Hz, 1H), 3.67 (t, *J* = 6.8 Hz, 2H), 3.40 (t, *J* = 6.6 Hz, 2H), 2.02-1.90 (m, 4H), 1.66 (d, *J* = 7.2 Hz, 3H); LC/MS (ESI) m/z = 548.1 $[M+H]^+$.

Step 4: Synthesis of (R)-1-(2-fluoro-3-(pyrrolidine-1-carbonyl)phenyl)-N-(1-(3-nitro-5-(tiifluoromethyl)phenyl)ethyl)-6-oxo-1,6-dihydropyridazine-3-carboxamide

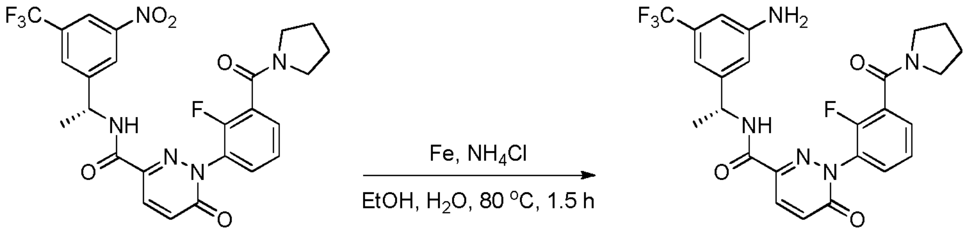


230

To a solution of (R)-1-(2-fluoro-3-(pyrrolidine-1-carbonyl)phenyl)-N-(1-(3-nitro-5-(trifluoromethyl)phenyl)ethyl)-6-oxo-1,6-dihydropyridazine-3-carboxamide (19 mg, 34.7 μmol) in EtOH (0.347 mL) and $H_2O$ (69.4 μL), Fe (19.4 mg, 0.347 mmol) and $NH_4Cl$ (18.6 mg, 0.347 mmol) were added, and the mixture was stirred at 80°C for 3 hours. The mixture was filtered, and the filtrate was concentrated under reduced pressure, followed by the addition of distilled water (10 mL) and then extracted with EtOAc. The combined organic layer was washed with brine, dried over anhydrous $Na_2SO_4$ and concentrated under reduced pressure to obtain a crude product. The product was purified by prep-HPLC (XBridge® Prep C18 19*250 mm*5 μm, mobile phase: [water-ACN]; B%: 50%-80%, 7min). $CH_3CN$ was removed under reduced pressure, and the remaining solvent was removed by lyophilization to give the compound of Example 230 (1.8 mg, 10.02% yield) as a white solid. $^1H$ NMR (400 MHz, DMSO-$d_6$) δ8.97 (d, *J* = 8.0 Hz, 1H), 7.95 (d, *J* = 9.6 Hz, 1H), 7.78-7.75 (m, 1H), 7.61-7.58 (m, 1H), 7.47 (t, *J* = 7.8 Hz, 1H), 7.19 (d, *J* = 9.6 Hz, 1H), 6.79 (br d, *J* = 14.4 Hz, 2H), 6.69 (s, 1H), 5.56 (s, 2H), 5.04-5.01 (m, 1H), 3.50-3.47 (m, 2H), 3.26-3.23 (m, 2H), 1.90-1.83 (m, 4H), 1.43 (d, *J* = 7.2 Hz, 3H); LC/MS (ESI) m/z = 518.2 $[M+H]^+$.

**Example 231 to Example 234**

The compounds shown in the following table were prepared in a similar manner to Example 230 by using starting materials and intermediates corresponding to the respective structures of the desired compounds, except that the purification method was changed from prep-HPLC to silica gel column chromatography when the compounds of Examples 232 to 234 were prepared.

[Table 13]

| No. | Structure/Name | Spectral Data |
|---|---|---|
| 231 | 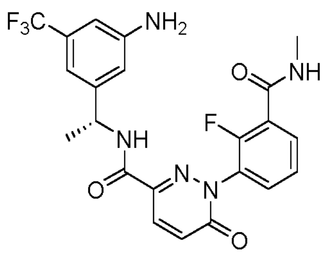 (R)-N-(1-(3-amino-5-(trifluoromethyl)phenyl)ethyl)-1-(2-fluoro-3 -(methylcarbamoyl)phenyl)-6-oxo-1,6-dihydropyridazine-3-carboxamide | $^1H$ NMR (400 MHz, DMSO-$d_6$) δ 8.94 (d, *J* = 8.4 Hz, 1H), 8.44-8.43 (m, 1H), 7.96 (d, *J* = 10.0 Hz, 1H), 7.80-7.73 (m, 2H), 7.46 (t, *J* = 7.8 Hz, 1H), 7.20 (d, *J* = 10.0 Hz, 1H), 6.79 (br d, *J* = 15.6 Hz, 2H), 6.69 (s, 1H), 5.56 (s, 2H), 5.02 (quin, *J* = 7.5 Hz, 1H), 2.78 (d, *J* = 4.8 Hz, 3H), 1.42 (d, *J* = 6.8 Hz, 3H); LC/MS (ESI) m/z = 478.2 $[M+H]^+$ |
| 232 | 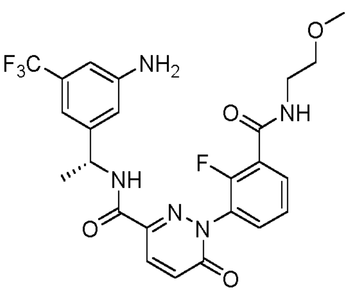 (R)-N-(1-(3-amino-5-(trifluoromethyl)phenyl)ethyl)-1-(2-fluoro-3-((2-methoxyethyl)carbamoyl)phenyl)-6-oxo-1,6-dihydropyridazine-3-carboxamide | $^1H$ NMR (400 MHz, DMSO-$d_6$) δ 8.94 (d, *J* = 8.4 Hz, 1H), 8.54-8.52 (m, 1H), 7.96 (d, *J* = 9.6 Hz, 1H), 7.80-7.72 (m, 2H), 7.46 (t, *J* = 8.0 Hz, 1H), 7.21 (d, *J* = 9.6 Hz, 1H), 6.79 (br d, *J* = 15.6 Hz, 2H), 6.69 (s, 1H), 5.56 (s, 2H), 5.02 (quin, *J* = 7.5 Hz, 1H), 3.47-3.43 (m, 4H), 3.26 (s, 3H), 1.42 (d, *J* = 7.2 Hz, 3H); LC/MS (ESI) m/z = 522.2 $[M+H]^+$ |

(continued)

| No. | Structure/Name | Spectral Data |
|---|---|---|
| 233 | 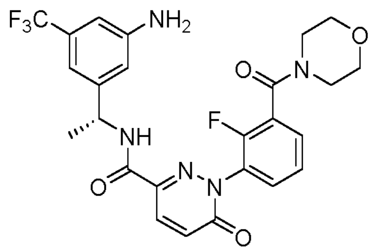 (R)-N-(1-(3-amino-5-(trifluoromethyl)phenyl)ethyl)-1-(2-fluoro-3-(morpholine-4-carbonyl)phenyl)-6-oxo-1,6-dihydropyridazine-3-carboxamide | $^1$H NMR (400 MHz, DMSO-$d_6$) δ 8.97 (d, *J* = 8.4 Hz, 1H), 7.95 (d, *J* = 10.0 Hz, 1H), 7.82-7.77 (m, 1H), 7.63-7.59 (m, 1H), 7.50 (t, *J* = 7.8 Hz, 1H), 7.20 (d, *J* = 9.6 Hz, 1H), 6.79 (br d, *J* = 13.6 Hz, 2H), 6.70 (s, 1H), 5.56 (s, 2H), 5.03 (quin, *J* = 7.2 Hz, 1H), 3.66 (s, 4H), 3.56 (s, 2H), 3.30-3.27 (m, 2H), 1.43 (d, *J* = 7.2 Hz, 3 H).; LC/MS (ESI) m/z = 534.2 [M+H]$^+$ |
| 234 | 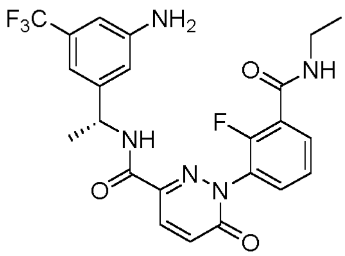 (R)-N-(1-(3-amino-5-(trifluoromethyl)phenyl)ethyl)-1-(3-(ethylcarbamoyl)-2-fluorophenyl)-6-oxo-1,6-dihydropyridazine-3-carboxamide | $^1$H NMR (400 MHz, DMSO-$d_6$) δ 8.92 (d, *J* = 8.0 Hz, 1H), 8.50 (t, *J* = 5.4 Hz, 1H), 7.95 (d, *J* = 10.0 Hz, 1H), 7.79-7.71 (m, 2H), 7.45 (t, *J* = 7.8 Hz, 1H), 7.20 (d, *J* = 9.6 Hz, 1H), 6.78 (br d, *J* = 15.2 Hz, 2H), 6.69 (s, 1H), 5.56 (s, 2H), 5.02 (quin, *J* = 7.4 Hz, 1H), 3.31-3.24 (m, 2H), 1.42 (d, *J* = 6.8 Hz, 3H), 1.11 (t, *J* = 7.2 Hz, 3H); LC/MS (ESI) m/z = 492.2 [M+H]$^+$ |

**Example 235: N-[(1R)-1-[3-amino-5-(trifluoromethyl)phenyl]ethyl]-1-[3-(1,3,4-oxadiazol-2-yl)phenyl]-6-oxo-1,6-dihydropyridazine-3-carboxamide**

Step 1: 2-[3-(4,4,5,5-tetramethyl-1,3,2-dioxaborolan-2-yl)phenyl]-1,3,4-oxadiazole

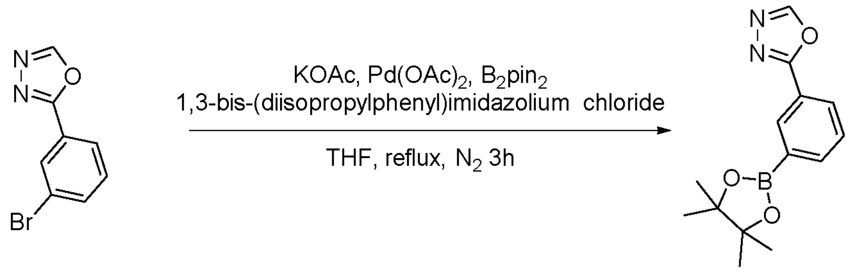


To a solution of 2-(3-bromophenyl)-1,3,4-oxadiazole (570 mg, 2.53 mmol) in THF (3.2 mL), bis(pinacolato)diborane (753 mg, 2.96 mmol), 1,3-bis-(diisopropylphenyl)-imidazolium chloride (65 mg, 0.152 mmol), $Pd(OAc)_2$ (17 mg, 0.076 mmol) and KOAc (621 mg, 6.33 mmol) were added, and the mixture was degassed and purged with $N_2$ for 2 min, and then stirred at 75°C for 3 hours. The reaction mixture was cooled to room temperature, diluted with DCM and filtered through celite. The filtrate was concentrated under reduced pressure to obtain a crude product. The product was purified by silica gel column chromatography to give 2-[3-(4,4,5,5-tetramethyl-1,3,2-dioxaborolan-2-yl)phenyl]-1,3,4-oxadiazole (580 mg, 84%) as a yellow-white solid. $^1$H NMR (400 MHz, $CDCl_3$) δ ppm 8.51 (s, 1H), 8.48 (s, 1H), 8.20 (dt, *J* = 7.6, 4.0 Hz, 1H), 7.99 (dt, *J* = 7.6, 1.0 Hz, 1H), 7.37 (br, 1H), 7.54 (t, *J* = 7.8 Hz, 1H), 1.37 (s, 12 Hz); LC/MS (ESI)m/z = 273.0 [M+H]$^+$.

Step 2: N-[(1R)-1-[3-nitro-5-(trifluoromethyl)phenyl]ethyl]-1-[3-(1,3,4-oxadiazol-2-yl) phenyl]-6-oxo-1,6-dihydropyridazine-3-carboxamide

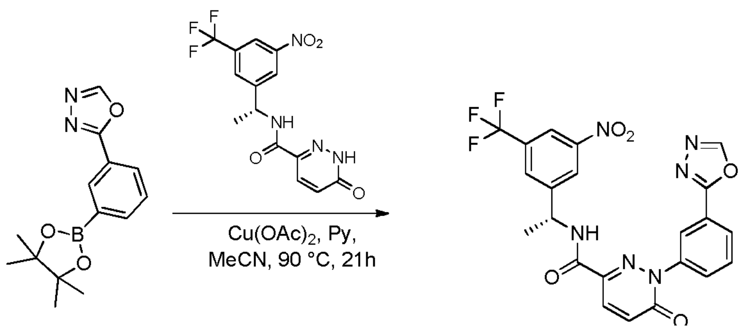


2-[3-(4,4,5,5-tetramethyl-1,3,2-dioxaborolan-2-yl)phenyl]-1,3,4-oxadiazole (100 mg, 0.281 mmol), the compound obtained from Step 4 of Example 221 (191 mg, 0.702 mmol), $Cu(OAc)_2$ (51 mg, 0.281 mmol) and pyridine (0.09 mL, 1.12 mmol) were added to acetonitrile (4 mL), and then the mixture was stirred at 90 °C for 21 hours under air. The reaction mixture was cooled to room termerature and filtered under reduced pressure. The residue was purified by silica gel column chromatography to give N-[(1R)-1-[3-nitro-5-(trifluoromethyl)phenyl]ethyl]-1-[3-(1,3,4-oxadiazol-2-yl)phenyl]-6-oxo-1,6-dihydropyridazine-3-carboxamide (81 mg, 58%) as a yellow-white solid. $^1H$ NMR (400 MHz, $CDCl_3$) $\delta$ ppm 8.53 (s, 1H), 8.41 (s, 1H), 8.32 (t, *J*= 1.8 Hz, 1H), 8.21 (dt, *J* = 8.0, 1.4 Hz, 1H), 8.03 (d, *J* = 10 Hz, 1H), 7.95 (s, 1H), 7.84 (dt, *J* = 2.0, 1.0 Hz, 1H), 7.73 (t, *J* = 7.8 Hz, 1H), 7.40 (d, *J* = 7.2 Hz, 1H), 7.16 (d, *J* = 10 Hz, 1H), 5.35 (quin., *J* = 7.1, 14.3 Hz, 1H), 1.67 (*J* = 7.2 Hz, 3H).

Step 3: N-[(1R)-1-[3-amino-5-(trifluoromethyl)phenyl]ethyl]-1-[3-(1,3,4-oxadiazol-2-yl)-phenyl]-6-oxo-1,6-dihydropyridazine-3-carboxamide

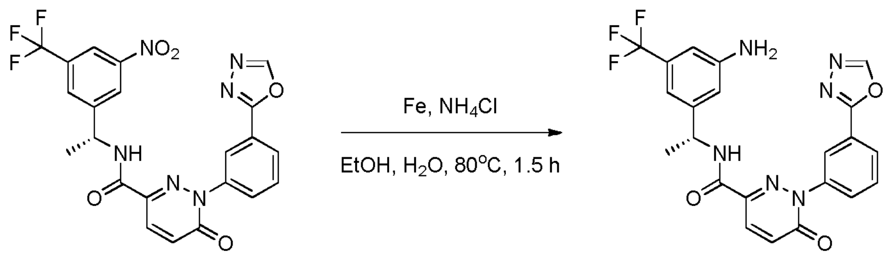


**235**

A mixture of N-[(1R)-1-[3-nitro-5-(trifluoromethyl)phenyl]ethyl]-1-[3-(1,3,4-oxadiazol-2-yl)phenyl]-6-oxo-1,6-dihydropyridazine-3-carboxamide (40 mg, 0.0799 mmol), Fe (45 mg, 0.799 mmol) and $NH_4Cl$ (43 mg, 0.799 mmol) in EtOH (0.8 mL) and $H_2O$ (0.16 mL) was degassed and purged with $N_2$, and then stirred at 80 °C for 1.5 hours under $N_2$ atmosphere. After filteration, the filtrate was poured into water and extracted with DCM. The organic layer was dried over $Na_2SO_4$ and filtered under reduced pressure to obtain a crude product. The product was purified by silica gel column chromatogrphy to give the compound of Example 234 (12.7 mg, 34% yield) as a white solid. $^1H$ NMR (400 MHz, DMSO-$d_6$) $\delta$ ppm 9.42 (s, 1H), 8.94 (d, *J* = 8.0 Hz, 1H), 8.35 (t, *J* = 1.8 Hz, 1H), 8.13 (dt, *J* = 7.6, 1.4 Hz, 1H), 7.99-7.96 (m, 1H), 7.92 (d, *J* = 9.6 Hz, 1H), 7.80 (t, *J* = 9.2 Hz, 1H), 7.19 (d, *J* = 9.6 Hz, 1H), 6.80 (d, *J* = 14.0 Hz, 1H), 6.70 (s, 1H), 5.56 (s, 2H), 5.05 (t, *J* = 7.8 Hz, 1H), 1.44 (d, *J* = 6.8 Hz, 3H). LC/MS: m/z 471.16 (M+H)+(ES). LC/MS (ESI)m/z = 471.2 $[M+H]^+$.

**Example 236 to Example 239**

The compounds shown in the following table were prepared in the same manner as in Example 235.

[Table 14]

| No | Structure / Name | Spectral Data |
|---|---|---|
| 236 | 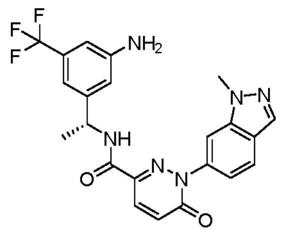 N-[(1R)-1-[3-amino-5-(trifluoromethyl)phenyl]ethyl]-1-(1-methyl-1H-indazol-6-yl)-6-oxo-1,6-dihydropyridazine-3-carboxamide | $^1$H NMR (400 MHz, DMSO-$d_6$) δ ppm 8.88 (d, *J* = 8.4 Hz, 1H), 8.16 (d, *J* = 0.8 Hz, 1H), 7.97 (d, *J* = 9.6 Hz, 1H), 7.93 (s, 1H), 7.89 (d, *J* = 8.4 Hz, 1H), 7.37 (dd, *J* = 8.6, 1.8 Hz, 1H), 7.19 (d, *J* = 9.6 Hz, 1H), 6.80 (d, *J* = 16.0 Hz, 2H), 6.70 (s, 1H), 5.56 (s, 2H), 5.05 (t, *J* = 7.6 Hz, 1H), 4.08 (s, 3H), 1.44 (d, *J* = 7.2 Hz, 3H). LC/MS (ESI) m/z = 457.2 $[M+H]^+$ |
| 237 | 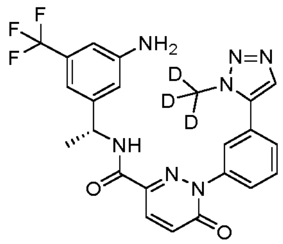 N-[(1R)-1-[3-amino-5-(trifluoromethyl)phenyl]ethyl]-1-[3-(3-(trideuteriomethyl)triazol-4-yl)phenyl]-6-oxo-pyridazine-3-carboxamide | $^1$H NMR (400 MHz, DMSO-$d_6$) δ = 8.91 (d, J = 8.4 Hz, 1H), 7.99 (s, 1H), 7.96 (s, 1H), 7.92 (d, J = 9.6 Hz, 1H), 7.88 - 7.81 (m,1H), 7.76 - 7.69 (m, 2H), 7.19 (d, J = 9.6 Hz, 1H), 6.81 (br d, J = 13.2 Hz, 2H), 6.72 (s, 1H), 5.57 (s, 2H), 5.05 (quin, J = 7.2 Hz, 1H), 1.44 (d, J = 7.2 Hz, 3H). LC/MS (ESI) m/z = 487.2 $[M+H]^+$ |
| 238 | 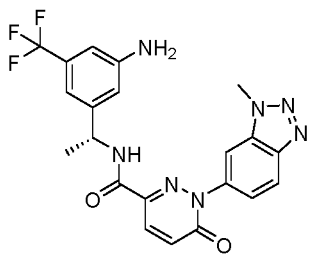 N-[(1R)-1-[3-amino-5-(trifluoromethyl)phenyl]ethyl]-1-(1-methyl-1H-1,2,3-benzotriazol-6-yl)-6-oxo-1,6-dihydropyridazine-3-carboxamide | $^1$H NMR (400 MHz, DMSO-$d_6$) δ ppm 8.90 (d, *J* = 8.0 Hz, 1H), 8.24 (s, 1H), 8.18 (d, *J* = 8.8 Hz, 1H), 7.95 (d, *J* = 10.0 Hz, 1H), 7.68 (dd, *J* = 9.0, 1.8 Hz, 1H), 6.80 (d, *J* = 15.6 Hz, 2H), 6.70 (s, 1H), 5.56 (s, 2H), 5.05 (t, *J* = 7.6 Hz, 1H), 4.35 (s, 3H), 1.44 (d, *J* = 7.2 Hz, 3H). LC/MS (ESI) m/z = 458.1 $[M+H]^+$ |
| 239 | 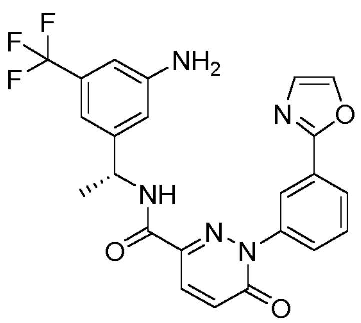 N-[(1R)-1-[3-amino-5-(trifluoromethyl)phenyl]ethyl]-1-[3-(1,3-oxazol-2-yl)phenyl]-6-oxo-1,6-dihydropyridazine-3-carboxamide | $^1$H NMR (400 MHz, DMSO-$d_6$) δ ppm 8.93 (d, *J* = 8.4 Hz, 1H), 8.28-8.27 (m, 2H), 8.07 (d, *J* = 7.6 Hz, 1H), 7.91 (d, *J* = 9.6 Hz, 1H), 7.84 (d, *J* = 9.2 Hz, 1H), 7.20 (t, *J* = 8.0 Hz, 1H), 7.44 (s, 1H), 7.18 (d, *J* = 10.0 Hz, 1H), 6.80 (d, *J* = 14.4 Hz, 2H), 6.69 (s, 1H), 5.56 (s, 2H), 5.04 (quin, *J* = 7.7, 15.2, 1H), 1.44 (d, *J* = 7.2 Hz, 3H). LC/MS (ESI) m/z = 470.1 $[M+H]^+$ |

**Example 240: N-[(1R)-1-[3-amino-5-(trifluoromethyl)phenyl]ethyl]-1-(7-fluoro-1-methyl-1H-indazol-6-yl)-6-oxo-1,6-dihydropyridazine-3-carboxamide**

Step 1: 6-bromo-7-fluoro-1-methyl-1H-indazole

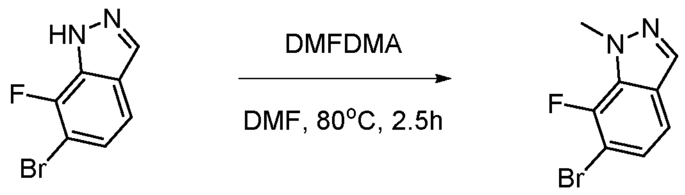


To a solution of 6-bromo-7-fluoro-1H-indazole (700 mg, 3.26 mmol) in DMF (7 mL) was added N,N-dimethylformamide dimethyl acetal (1.74 mL, 13.0 mmol), and the mixture was then stirred at 80°C for 2.5 hours. After being cooled to room temperature, the mixture was poured into water and extracted with EtOAc. The organic layer was washed with brine, dried over $Na_2SO_4$ and filtered under reduced pressure to obtain a crude product. The product was purified by silica column chromatography to give 6-bromo-7-fluoro-1-methyl-1H-indazole (392 mg, 53%) as a light-yellow solid. $^1$H NMR (400 MHz, $CDCl_3$) $\delta$ 7.94 (d, *J* = 2.4 Hz, 1H), 7.34 (d, *J* = 8.8 Hz, 1H), 7.22-7.18 (m, 1H), 4.24 (d, *J* = 1.2 Hz, 3H); LC/MS (ESI) m/z = 228.9 $[M+H]^+$.

Step 2: 7-fluoro-1-methyl-6-(4,4,5,5-tetramethyl-1,3,2-dioxaborolan-2-yl)-1H-indazole

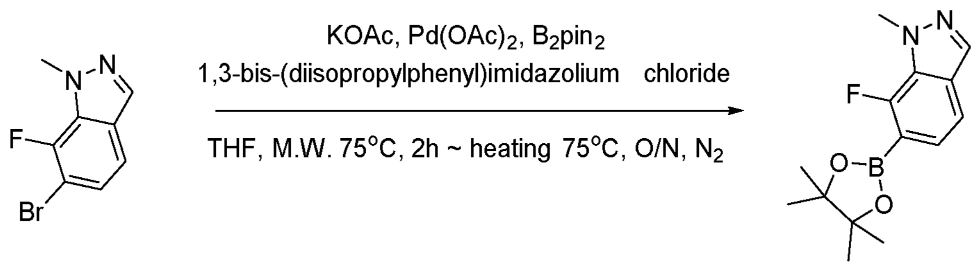


To a solution of 6-bromo-7-fluoro-1-methyl-1H-indazole (300 mg, 1.31 mmol) in THF (2 mL), bis(pinacolato)diborane (389 mg, 1.53 mmol), 1,3-bis-(diisopropylphenyl)-imidazolium chloride (34 mg, 0.0786 mmol), KOAc (321 mg, 3.27 mmol) and $Pd(OAc)_2$ (9 mg, 0.0393 mmol) were added, and the mixture was then stirred at 75°C for 2 hours with microwave irradiation. The reaction mixture was additionally stirred at 75°C for 17 hours. The reaction mixture was cooled to room temperature and filtered through celite. The filtrate was concentrated under reduced pressure to obtain a crude product. The product was purified by silica column chromatography to give 7-fluoro-1-methyl-6-(4,4,5,5-tetramethyl-1,3,2-dioxaborolan-2-yl)-1H-indazole (300 mg, 83%) as a white solid. $^1$H NMR (400 MHz, $CDCl_3$) $\delta$ 7.94 (d, *J* = 2.4 Hz, 1H), 7.46-7.43 (m, 1H), 7.39-7.36 (m, 1H), 4.27 (d, *J* = 1.2 Hz, 3H), 1.40 (s, 12H); LC/MS (ESI) m/z = 277.0 $[M+H]^+$.

Step 3: 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide

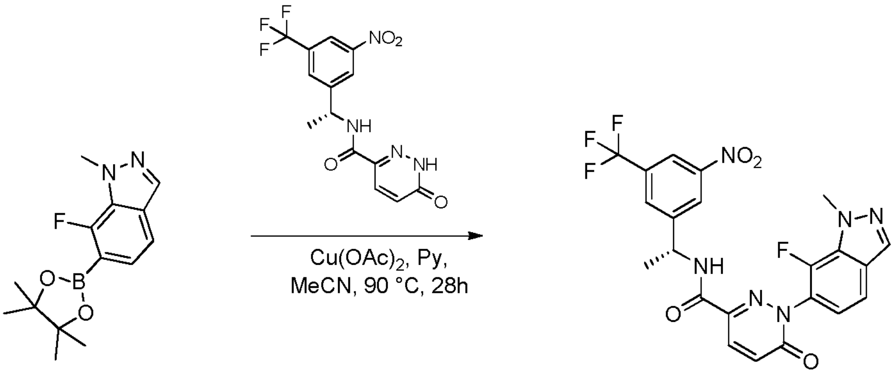


The compound obtained from Step 4 of Example 221 (150 mg, 0.421 mmol), 7-fluoro-1-methyl-6-(4,4,5,5-tetramethyl-1,3,2-dioxaborolan-2-yl)-1H-indazole (291 mg, 1.05 mmol), $Cu(OAc)_2$ (77 mg, 0.421 mmol) and pyridine (0.14 mL, 1.68 mmol) were added to acetonitrile (6 mL), and the mixture was then stirred at 90°C for 28 hours under air. The mixture was cooled to room temperature, poured into water and extracted with EtOAc. The organic layers were washed with aq. $CuSO_4.5H_2O$, dried over $Na_2SO_4$ and concentrated under reduced pressure to obtain a crude product. The product was purified by silica column chromatography to give 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide (97 mg, 46%) as an off-white solid. $^1H$ NMR (400 MHz, $CDCl_3$) δ 8.40 (s, 2H), 8.06-8.02 (m, 2H), 7.93 (s, 1H), 7.63 (d, *J* = 8.8 Hz, 1H), 7.36 (d, *J* = 7.2 Hz, 1H), 7.18-7.12 (m, 2H), 5.33 (quin, *J* = 6.3, 13.2 Hz, 1H), 4.30 (s, 3H), 1.63 (d, *J* = 6.8 Hz, 3H); LC/MS (ESI) m/z = 505.1 $[M+H]^+$.

Step 4: 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide

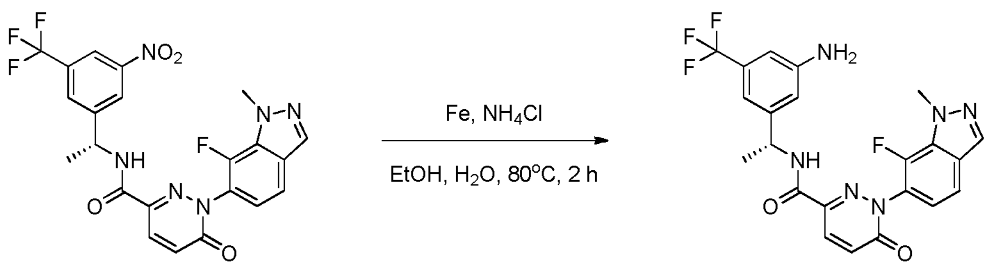


**240**

A mixture of 1-(7-fluoro-1-methyl-1H-indazol-6-yl)-N-[(1R)-1-[3-nitro-5-(trifluoromethyl)phenyl]ethyl]-6-oxo-1,6-dihydropyridazine-3-carboxamide (97 mg, 0.192 mmol), Fe (107 mg, 1.92 mmol) and $NH_4Cl$ (103 mg, 1.92 mmol) in EtOH (2.0 mL) and $H_2O$ (0.8 mL) was degassed, purged with $N_2$ and stirred at 80 °C for 2 hours under $N_2$ atmosphere. After filteration, the filtrate was poured into water and extracted with DCM. The organic layer was dried over $Na_2SO_4$ and filtered under reduced pressure to obtain a crude product. The product was purified by silica gel column chromatography to give the compound of Example 240 (77 mg, 85% yield) as a light-pink solid. $^1H$ NMR (400 MHz, DMSO-$d_6$) δ 8.90 (d, *J* = 8.4 Hz, 1H), 8.24(d, *J* = 2.4 Hz, 1H), 7.97 (d, *J* = 9.6 Hz, 1H), 7.75 (d, *J* = 8.4 Hz, 1H), 7.32(dd, *J* = 8.4, 6.0 Hz, 1H), 7.22 (d, *J* = 10.0 Hz, 1H), 6.78 (d, *J* = 14.0 Hz, 2H), 6.69 (s, 1H), 5.56 (s, 2H), 5.03 (quin, *J* = 7.7, 15.2 Hz, 1H), 4.21 (s, 3H), 1.42 (d, *J* = 7.2 Hz, 1H); LC/MS (ESI) m/z = 475.1 $[M+H]^+$.

**Example 241: (R)-N-(1-(3-amino-5-(trifluoromethyl)phenyl)ethyl)-5-bromo-6-oxo-1-phenyl-1,6-dihydropyridazine-3-carboxamide**

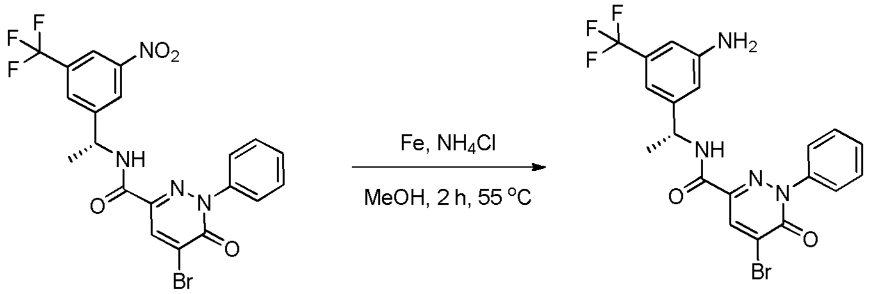


241

The compound of Example 241 (7.2 mg, 12% yield) was obtained as a white solid in the same manner as in Example 56, except that Step 2 of Example 56 was omitted. $^1H$ NMR (400M Hz, DMSO-$d_6$) δ = 8.92 (d, *J* = 8.4 Hz, 1H), 8.32 (s, 1H), 7.71-7.60 (m, 2H), 7.59-7.45 (m, 3H), 6.80 (d, *J* = 16.4 Hz, 2H), 6.70 (s, 1H), 5.54 (s, 2H), 5.07-5.00 (m, 1H), 1.44 (d, *J* = 6.8 Hz, 3H); MS (ESI) m/z = 481.0 $[M+H]^+$).

**Example 242: N-[(1R)-1-[3-(1,1-difluoro-2-methoxy-ethyl)-2-fluoro-phenyl]ethyl]-1-(2-fluorophenyl)-6-oxo-pyridazine-3-carboxamide**

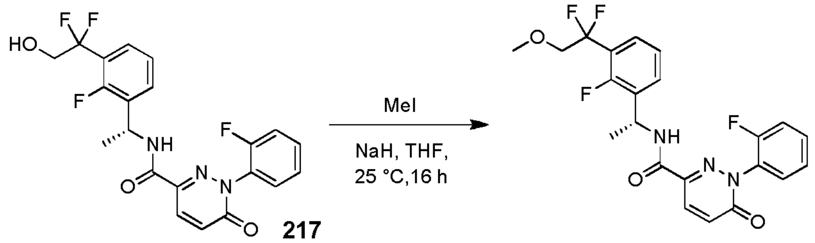


217

242

To a mixture of the compound of Example 217 (30 mg, 68.91 μmol) in THF (2 mL) was added NaH (8.27 mg, 206.72 μmol, 344.53 μL) at 50 °C. The resulting mixture was stirred at 50 °C for 15 minutes. The mixture was added with MeI (19.56 mg, 137.81 μmol, 8.58 μL) and stirred at 25 °C for 16 hours. The reaction mixture was quenched with ice-cold water (10 mL) and extracted with EtOAc (30 mL × 3). The combined organic layer was washed with brine (50 mL), dried over anhydrous $Na_2SO_4$ and concentrated under reduced pressure to obtain a crude product. The product was purified by prep-HPLC (column: Phenomenex C18 75*30mm*3μm; mobile phase: [water($NH_3H_2O$+$NH_4HCO_3$)-ACN];B%: 34%-64%,30 min). Most of $CH_3CN$ was removed under reduced pressure, and the remaining solvent was removed by lyophilization to give the compound of Example 242 (3.5 mg, 11.30% yield) as a white solid. $^1H$ NMR (400 MHz, DMSO-$d_6$) δ = 9.04 (d, *J* = 8.0 Hz, 1H), 7.92 (d, *J* = 10.0 Hz, 1H), 7.69 (dt, *J* = 1.6, 7.6 Hz, 1H), 7.65 - 7.61 (m, 1H), 7.61 - 7.56 (m, 1H), 7.48 - 7.40 (m, 3H), 7.31 - 7.26 (m, 1H), 7.18 (d, *J* = 9.6 Hz, 1H), 5.39 (quin, *J* = 7.2 Hz, 1H), 3.96 (t, *J* = 14.0 Hz, 2H), 3.33 (s, 3H), 1.46 (d, *J* = 7.2 Hz, 3H); MS (ESI) m/z = 450.1 $[M+H]^+$.

**Example 243: N-[(1R)-1-[3-(2-amino-1,1-difluoro-ethyl)-2-fluoro-phenyl]ethyl]-1-(2-fluorophenyl)-6-oxo-pyridazine-3-carboxamide**

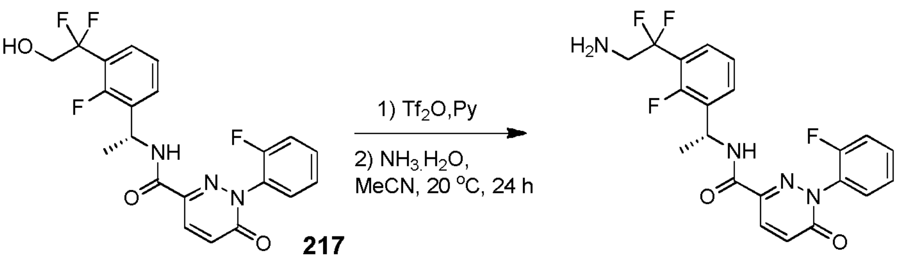


217

243

To a solution of the compound of Example 217 (30 mg, 68.91 μmol) in MeCN (1 mL) was added pyridine (8.72

mg, 110.25 μmol, 8.90 μL). The solution was cooled in an ice-water bath, and $Tf_2O$ (21.39 mg, 75.80 μmol, 12.51 μL) was added dropwise. The mixture was stirred at 25 °C for 5 minutes. Concentrated $NH_3.H_2O$ (0.25 M, 103.86 mL) was added thereto, and the solution was stirred at 20 °C for 24 hours under $N_2$ atmosphere. The reaction mixture was poured into water (30 mL) and extracted with EtOAc (30 mL × 3). The combined organic layer was washed with brine (30 mL), dried over anhydrous $Na_2SO_4$ and concentrated under reduced pressure to obtain a crude product. The product was purified by prep-HPLC (column: Phenomenex C18 75*30mm*3μm; mobile phase: [water($NH_3H_2O$+$NH_4HCO_3$)-ACN];B%: 30%-70%,30 min). Most of $CH_3CN$ was removed under reduced pressure, and the remaining solvent was removed by lyophilization to give the compound of Example 243 (4.6 mg, 15.28% yield) as a white solid. $^1$H NMR (400 MHz, DMSO-$d_6$) δ = 9.03 (d, *J* = 8.0 Hz, 1H), 7.92 (d, *J* = 9.6 Hz, 1H), 7.69 (dt, *J* = 1.6, 7.6 Hz, 1H), 7.63 - 7.56 (m, 2H), 7.49 - 7.39 (m, 3H), 7.28 - 7.23 (m, 1H), 7.18 (d, *J* = 10.0 Hz, 1H), 5.40 (quin, *J* = 7.2 Hz, 1H), 3.20 (br t, *J* = 15.2 Hz, 2H), 1.68 (br s, 2H), 1.46 (d, *J* = 7.2 Hz, 3H); MS (ESI) m/z = 435.0 $[M+H]^+$.

**Example 244: N-[(1R)-1-[5-amino-2-methyl-3-(trifluoromethyl)phenyl]ethyl]-1-(2-fluorophenyl)-6-oxo-pyridazine-3-carboxamide**

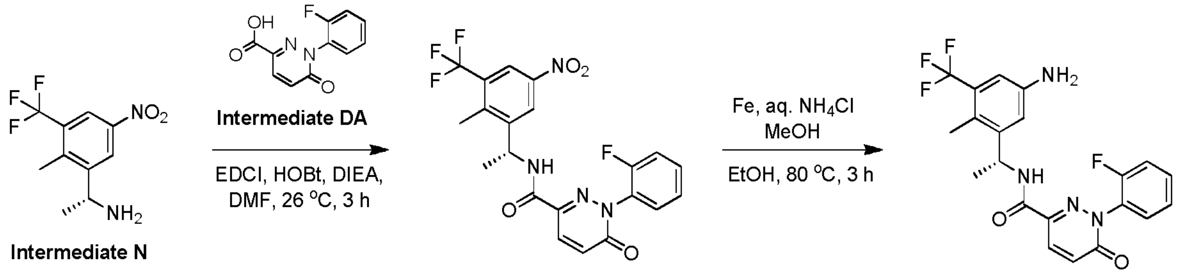


Intermediate N

**244**

The compound of Example 244 (35.2 mg, 37.14% yield) was obtained as a white solid by reacting Intermediate N and Intermediate DA in the same manner as in Steps 3 and 4 of Example 89. $^1$H NMR (400MHz, DMSO-$d_6$) δ = 8.90 (br d, *J* = 7.2 Hz, 1H), 7.92 (d, *J* = 9.6 Hz, 1H), 7.69 (br t, *J* = 7.2 Hz, 1H), 7.63 - 7.53 (m, 1H), 7.49 - 7.38 (m, 2H), 7.18 (d, *J* = 9.6 Hz, 1H), 6.84 (s, 1H), 6.78 (s, 1H), 5.27 (br s, 2H), 5.24 - 5.17 (m, 1H), 2.23 (br s, 3H), 1.38 (br d, *J* = 6.6 Hz, 3H); MS (ESI) m/z = 435.3 $[M+H]^+$.

**Example 245** : **N-[(1R)-1-[5-amino-2-fluoro-3-(trifluoromethyl)phenyl]ethyl]-1-(2-fluorophenyl)-6-oxo-pyridazine-3-carboxamide**

Step 1: Synthesis of 1-bromo-2-fluoro-5-nitro-3-(trifluoromethyl)benzene

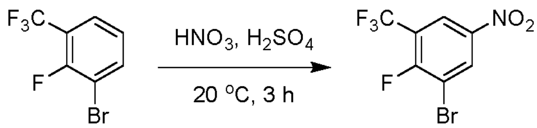


1-bromo-2-fluoro-5-nitro-3-(trifluoromethyl)benzene was obtained in the same manner as in Preparation Example 14 by using 1-bromo-2-fluoro-3-(trifluoromethyl)benzene as a starting material. $^1$H NMR (400MHz, DMSO-$d_6$) δ 8.95 (dd, *J* = 2.4, 5.2 Hz, 1H), 8.55 - 8.51 (m, 1H).

Step 2: Synthesis of 1-[2-fluoro-5-nitro-3-(trifluoromethyl)phenyl]ethanone

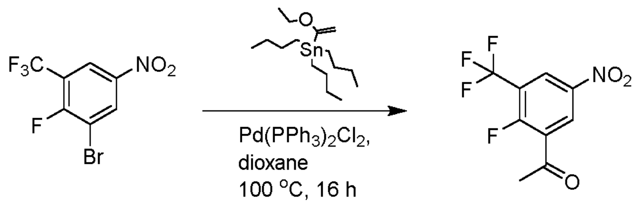


A mixture of 1-bromo-2-fluoro-5-nitro-3-(trifluoromethyl)benzene (5.9 g, 20.49 mmol), tributyl(1-ethoxyvinyl)stannane (8.41 g, 23.29 mmol, 7.86 mL) and $Pd(PPh_3)_2Cl_2$ (1.44 g, 2.05 mmol) in dioxane (50 mL) was stirred at 100°C for 16 hours under $N_2$ atmosphere. The reaction mixture was cooled to 0°C, treated with 4 M HCl (50 mL) and stirred for 1 hour. The reaction mixture was diluted with water (100 mL) and then extracted with EtOAc (100 mL x 3). The extract was dried over $Na_2SO_4$ and filtered, and the filtrate was concentrated. The aqueous layer was adjusted to pH= 9 with aq. NaOH, and then sodium hypochlorite (100 mL) was added slowly while stirring. The product was purified by silica gel column chromatography (5% EtOAc in PE) to give 1-[2-fluoro-5-nitro-3-(trifluoromethyl)phenyl]ethanone (1.6 g, 31.10% yield) as colourless oil. $^1$H NMR (400MHz, DMSO-$d_6$) δ 8.81 (dd, $J$ = 2.8, 5.6 Hz, 1H), 8.71 (dd, $J$ = 2.8, 5.2 Hz, 1H), 2.70 (d, $J$ =4.0 Hz, 3H).

Step 3: Synthesis of (NZ,R)-N-[1-[2-fluoro-5-nitro-3-(trifluoromethyl)phenyl]ethylidene]-2-methyl-propane-2-sulfinamide

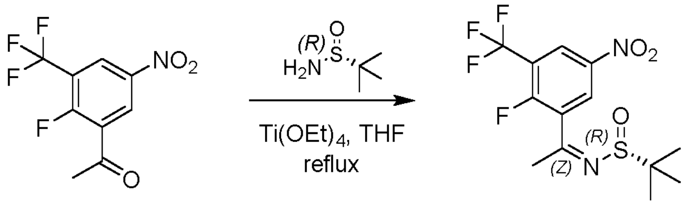


(NZ,R)-N-[1-[2-fluoro-5-nitro-3 -(trifluoromethyl)phenyl]ethylidene]-2-methyl-propane-2-sulfinamide was obtained in the same manner as in Step 2 of Preparation Example 2 by using 1-[2-fluoro-5-nitro-3-(trifluoromethyl)phenyl]ethanone as a starting material. $^1$H NMR (400MHz, DMSO-$d_6$) δ 8.80 (br d, $J$ = 2.4 Hz, 1H), 8.64 - 8.52 (m, 1H), 2.78 - 2.54 (m, 3H), 1.24(s, 6H), 1.21 - 1.11 (m, 3H); LC/MS (ESI) m/z = 354.9 $[M+H]^+$.

Step 4: Synthesis of (R)-N-[(1R)-1-[2-fluoro-5-nitro-3-(trifluoro)phenyl]ethyl]-2-methyl-propane-2-sulfinamide

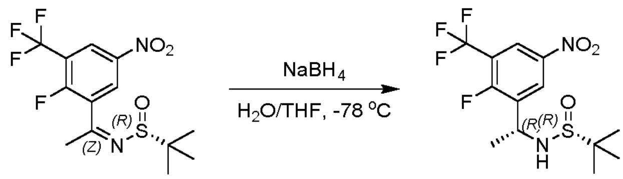


(R)-N-[(1R)-1-[2-fluoro-5-nitro-3-(trifluoro)phenyl]ethyl]-2-methyl-propane-2-sulfinamide was obtained in the same manner as in Step 3 of Preparation Example 2 by using (NZ,R)-N-[1-[2-fluoro-5-nitro-3-(trifluoromethyl)phenyl]ethylidene]-2-methyl-propane-2-sulfinamide as a starting material. $^1$H NMR (400MHz, DMSO-$d_6$) δ 8.83 (dd, $J$ = 2.8, 5.6 Hz, 1H), 8.46 (dd, $J$ = 2.8, 5.6 Hz, 1H), 6.14 (d, $J$ = 8.8 Hz, 1H), 4.83 - 4.73 (m, 1H), 1.48 (d, $J$ = 6.8 Hz, 3H), 1.13 (s, 9H); LC/MS (ESI) m/z = 356.9 $[M+H]^+$.

Step 5: Synthesis of (1R)-1-[2-fluoro-5-nitro-3-(trifluoromethyl)phenyl]ethanamine

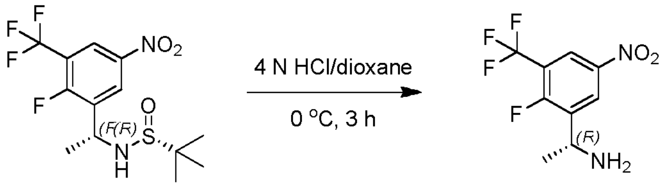


Intermediate AX

Intermediate AX was obtained in the same manner as in Step 4 of Preparation Example 2 by using (R)-N-[(1R)-1-[2-fluoro-5-nitro-3-(trifluoro)phenyl]ethyl]-2-methyl-propane-2-sulfinamide as a starting material. LC/MS (ESI) m/z = 252.9 $[M+H]^+$.

Step 6: Synthesis of N-[(1R)-1-[5-amino-2-fluoro-3-(trifluoromethyl)phenyl]ethyl]-1-(2-fluorophenyl)-6-oxo-pyridazine-3-carboxamide

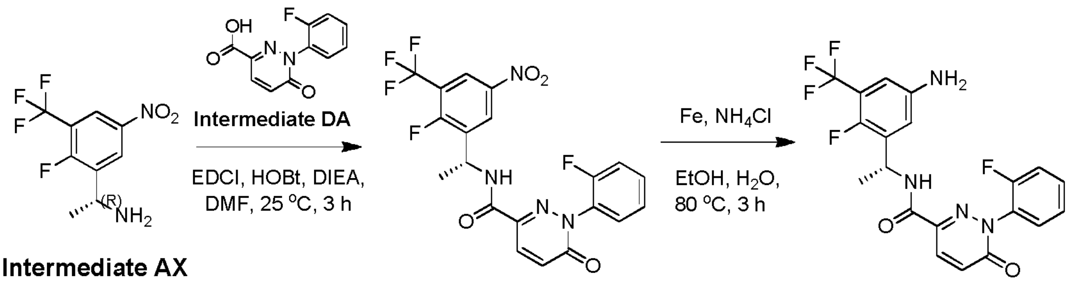


Intermediate AX

245

The compound of Example 245 was obtained by reacting Intermediate AX in the same manner as in Steps 3 and 4 of Example 89. $^1$H NMR (400 MHz, DMSO-$d_6$) δ = 8.97 (d, J = 7.6 Hz, 1H), 7.93 (d, J = 9.6 Hz, 1H), 7.69 (dt, J = 1.6, 7.6 Hz, 1H), 7.62 - 7.56 (m, 1H), 7.48 - 7.40 (m, 2H), 7.19 (d, J = 10.0 Hz, 1H), 6.81 (dd, J = 2.8, 5.6 Hz, 1H), 6.72 (dd, J = 2.8, 5.6 Hz, 1H), 5.38 (s, 2H), 5.23 (quin, J = 7.2 Hz, 1H), 1.42 (d, J = 7.2 Hz, 3H); ; LC/MS (ESI) m/z: 439.3 $[M+H]^+$

**Example 246: N-[(1R)-1-[3-(1,1-difluoro-2-hydroxy-ethyl)-2-methyl-phenyl]ethyl]-1-(2-fluorophenyl)-6-oxo-pyridazine-3-carboxamide**

Step 1: Synthesis of 1-(2-methyl-3-nitrophenyl)ethanone

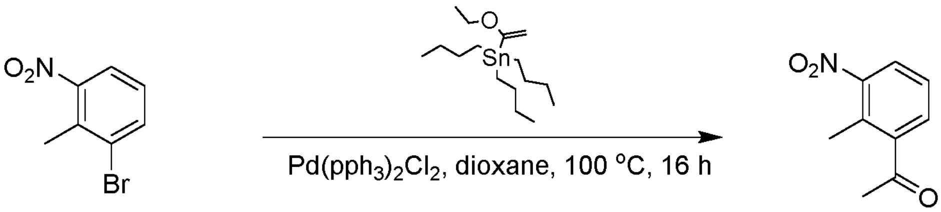


1-(2-methyl-3-nitrophenyl)ethenone was obtained in the same manner as in Preparation Example 12 by using 1-bromo-2-methyl-3-nitrobenzene as a starting material. $^1$H NMR (400 MHz, DMSO-$d_6$) δ ppm 8.05 - 7.89 (m, 2H), 7.56 (t, *J* = 8.0 Hz, 1H), 2.60 (s, 3H), 2.37 (s, 3H).

Step 2: Synthesis of 1-(3-amino-2-methyl-phenyl)ethanone

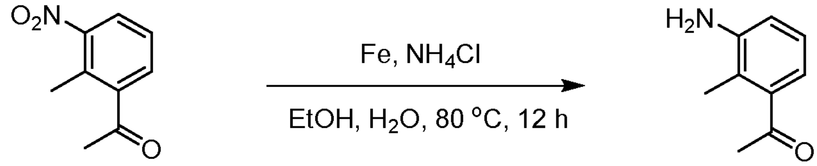


A mixture of 1-(2-methyl-3-nitrophenyl)ethanone (9.2 g, 51.35 mmol), Fe (28.67 g, 513.47 mmol), $NH_4Cl$ (27.47g, 513.47 mmol) in EtOH (100 mL) and $H_2O$ (20 mL) was degassed and purged with $N_2$ for 3 times, and the mixture was then stirred at 80 °C for 12 hours under $N_2$ atmosphere. The reaction mixture was poured into water (100 mL) and extracted with EtOAc (100 mL × 3). The combined organic layer was washed with brine (100 mL), dried over anhydrous $Na_2SO_4$ and concentrated under reduced pressure to give 1-(3-amino-2-methyl-phenyl)ethanone (6.8 g, 88.77% yield) as a crude product of a yellow solid, which was used into the next step without further purification. $^1H$ NMR (400 MHz, DMSO-$d_6$) δ ppm 6.98 (t, J = 7.6 Hz, 1H), 6.84 (dd, J = 1.2, 7.6 Hz, 1H), 6.77 (dd, J =1.2, 8.0 Hz, 1H), 5.05 (s, 2H), 2.46 (s, 3H), 2.06 (s, 3H).

Step 3: Synthesis of 1-(3-iodo-2-methyl-phenyl)ethanone

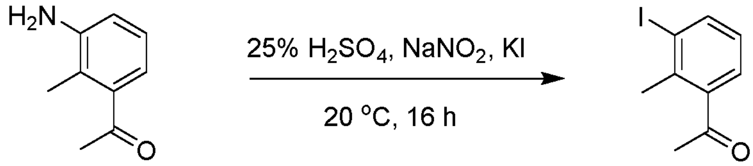


A mixture of 1-(3-amino-2-methyl-phenyl)ethanone (6.5 g, 43.57 mmol) in $H_2SO_4$ (21.6 mL, 975.55 mmol) was cooled to 0 °C, added with a mixed solution of $NaNO_2$ (3.01 g, 43.57 mmol) and $H_2O$ (65 mL), and stirred for 1 hour at 0°C. The reaction mixture was added to a mixed solution of KI (21.70 g, 130.71 mmol) in $H_2O$ (220 mL), followed by stirring at 20 °C for 18 hours. The reaction mixture was poured into water (100 mL) and extracted with EtOAc (100 mL × 3). The combined organic layer was washed with brine (100 mL), dried over anhydrous $Na_2SO_4$ and concentrated under reduced pressure to obtain a crude product. The product was purified by silica gel column chromatography (6% EtOAc in PE) to give 1-(3-iodo-2-methyl-phenyl)ethanone (8.2 g, 72.37% yield) as a yellow solid. $^1H$ NMR (EB4267-390-P1N, 400 MHz, DMSO-$d_6$) δ = 7.99 (dd, J = 1.2, 8.0 Hz, 1H), 7.67 (d, J = 7.6 Hz, 1H), 7.07 (t, J = 7.6 Hz, 1H), 2.53 (s, 3H), 2.41 (s, 3H).

Step 4: Synthesis of ethyl 2-(3-acetyl-2-methyl-phenyl)-2,2-difluoroacetate

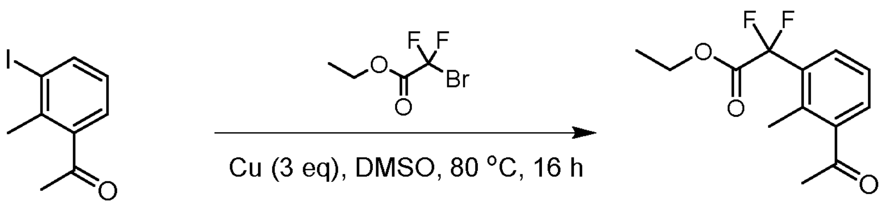


Ethyl 2-(3-acetyl-2-methyl-phenyl)-2,2-difluoro acetate was obtained in the same manner as in Step 1 of Preparation Example 4 by using 1-(3-iodo-2-methyl-phenyl)ethanone as a starting material. $^1H$ NMR (400 MHz, DMSO-$d_6$) δ ppm 7.86 (d, *J* = 7.6 Hz, 1H), 7.72 (d, *J* = 7.6 Hz, 1H), 7.49 (t, *J* = 7.6 Hz, 1H), 4.34 (q, *J* = 7.2 Hz, 2H), 2.57 (s, 3H), 2.29 (s, 3H), 1.22 (t, *J* = 7.2 Hz, 3H); MS (ESI) m/z = 257.0 $[M+H]^+$.

Step 5: Synthesis of ethyl 2-[3-[(Z)-N-[(R)-tert-butylsulfinyl]-C-methyl-carboimidoyl]-2-methyl-phenyl]-2,2-difluoroacetate

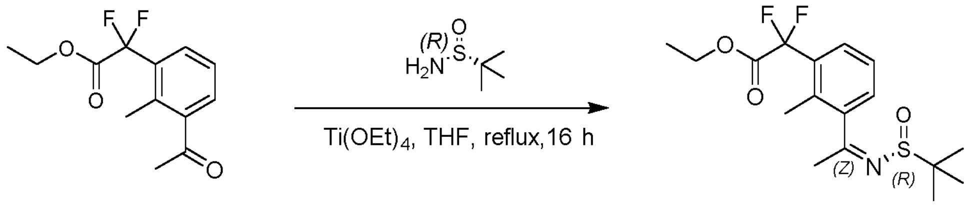


Ethyl 2-(3-acetyl-2-methyl-phenyl)-2,2-difluoroacetate was obtained in the same manner as in Step 2 of Preparation Example 4 by using ethyl 2-(3-acetyl-2-methyl-phenyl)-2,2-difluoro acetate as a starting material. MS (ESI) m/z = 360.0 $[M+H]^+$.

Step 6: Synthesis of (R)-N-[(1R)-1-[3-(1,1-difluoro-2-hydroxy-ethyl)-2-methylphenyl]ethyl]-2-methyl-propane-2-sulfinamide

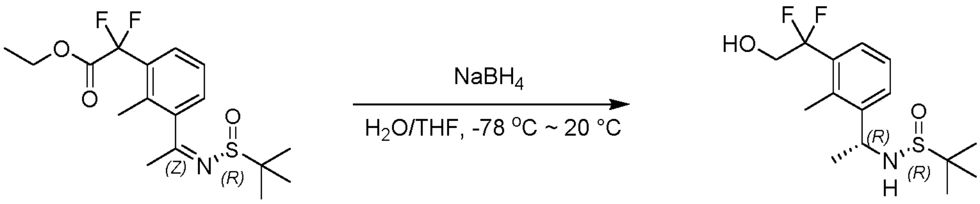


(R)-N-[(1R)-1-[3-(1,1-difluoro-2-hydroxy-ethyl)-2-methyl-phenyl]ethyl]-2-methylpropane-2-sulfinamide was obtained in the same manner as in Step 3 of Preparation Example 4 by using ethyl 2-(3-acetyl-2-methyl-phenyl)-2,2-difluoroacetate as a starting material. $^1$H NMR (400 MHz, DMSO-$d_6$) $\delta$ ppm 7.61 (d, $J$ = 7.6 Hz, 1H), 7.39 - 7.34 (m, 1H), 7.28 (t, $J$ = 7.2 Hz, 1H), 5.73 - 5.60 (m, 2H), 4.69 (quin, $J$ = 6.8 Hz, 1H), 3.88 (dt, $J$ = 6.4, 14.8 Hz, 2H), 2.37 (s, 3H), 1.37 (d, $J$ = 6.8 Hz, 3H), 1.11 - 1.06 (m,9H); MS (ESI) m/z = 320.0 $[M+H]^+$.

Step 7: Synthesis of 2-[3-[(1R)-1-aminoethyl]-2-methyl-phenyl]-2,2-difluoro-ethanol

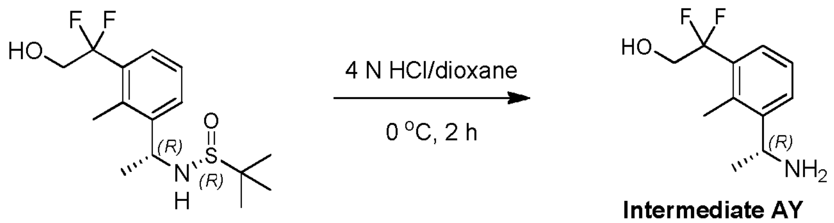


**Intermediate AY**

Intermediate AY was obtained in the same manner as in Step 4 of Preparation Example 4 by using (R)-N-[(1R)-1-[3-(1,1-difluoro-2-hydroxy-ethyl)-2-methyl-phenyl]ethyl]-2-methylpropane-2-sulfinamide as a starting material. MS (ESI) m/z = 216.0 $[M+H]^+$.

Step 8: Synthesis of N-[(1R)-1-[3-(1,1-difluoro-2-hydroxy-ethyl)-2-methyl-phenyl]ethyl]-1-(2-fluorophenyl)-6-oxo-pyridazine-3-carboxamide

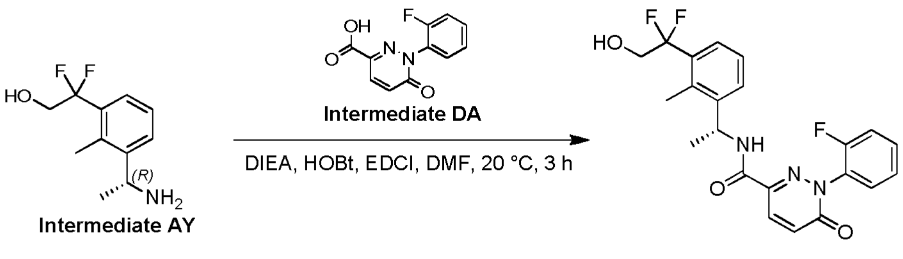


**246**

A mixture of Intermediate AY (30.33 mg, 140.92 μmol,), Intermediate DA (30 mg, 128.10 μmol), EDCI (49.12 mg, 256.21 μmol), HOBt (34.62 mg, 256.21 μmol) and DIEA (49.67 mg, 384.31 μmol, 66.94 μL) in DMF (2 mL) was degassed and purged with $N_2$ for 3 times, and then the mixture was stirred at 20 °C for 3 hours under $N_2$ atmosphere. The reaction mixture was poured into water (30 mL) and extracted with EtOAc (30 mL × 3). The organic layer was washed with brine (50 mL), dried over anhydrous $Na_2SO_4$ and concentrated under reduced pressure to obtain a crude product. The main product was separated by using prep-HPLC (column: Phenomenex C18 75*30mm*3um;mobile phase: [water($NH_3H_2O$+$NH_4HCO_3$)-ACN];B%: 26%-56%,11 min). Then, $CH_3CN$ was removed under reduced pressure, and the remaining solvent was removed by lyophilization to give the compound of Example 246. $^1H$ NMR (400MHz, DMSO-$d_6$) δ ppm 8.99 (d, *J* = 8.0 Hz, 1H), 7.91 (d, *J* = 9.6 Hz, 1H), 7.68 (dt, *J* = 1.6, 7.6 Hz, 1H), 7.62 - 7.55 (m, 2H), 7.48 - 7.39 (m, 2H), 7.35 (d, *J* = 7.2 Hz, 1H), 7.25 (t, *J* = 7.6 Hz, 1H), 7.17 (d, *J* = 10.0 Hz, 1H), 6.14 - 5.46 (m, 1H), 5.37 (quin, *J* = 7.2 Hz, 1H), 3.93 - 3.84 (m, 2H), 2.43 (s, 3H), 1.41 (d, *J* = 7.2 Hz, 3H); MS (ESI) m/z = 432.3 $[M+H]^+$.

**Example 247: N-[(1R)-1-[3-amino-5-(trifluoromethyl)phenyl]ethyl]-1-[2-(difluoromethoxy)phenyl]-6-oxo-pyridazine-3-carboxamide**

Steps 1 and 2: Synthesis of (R)-1-(2-hydroxyphenyl)-N-(1-(3-nitro-5-(trifluoromethyl)phenyl)ethyl)-6-oxo-1,6-dihydropyridazine-3-carboxamide

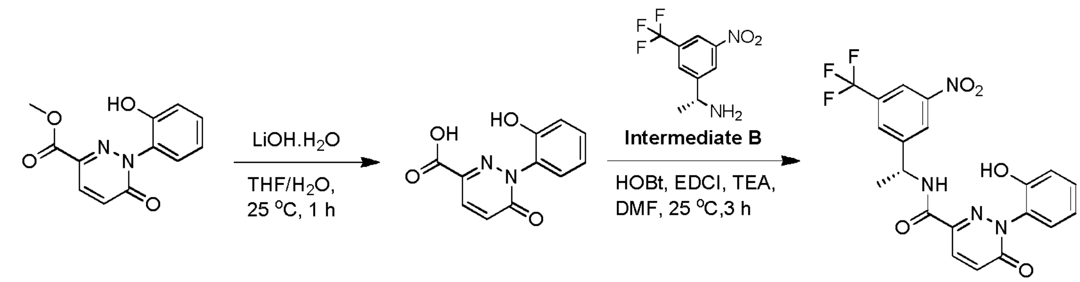


(R)-1-(2-hydroxyphenyl)-N-(1-(3-nitro-5-(trifluoromethyl)phenyl)ethyl)-6-oxo-1,6-dihydropyridazine-3-carboxamide was obtained in the same manner as in Steps 1 and 2 of Example 51 by using as a starting material methyl 1-(2-hydroxyphenyl)-6-oxo-1,6-dihydropyridazine-3-carboxylate prepared by referring to Preparation Example 11.

Step 3: Synthesis of 1-[2-(difluoromethoxy)phenyl]-N-[(1R)-1-[3-nitro-5-(trifluoromethyl)phenyl]ethyl]-6-oxo-pyridazine-3-carboxamide

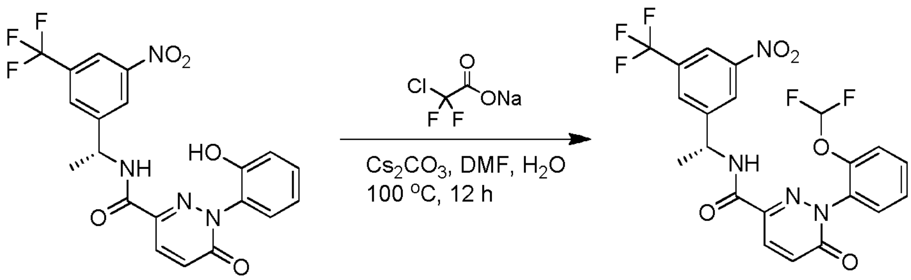

A mixture of 1-(2-hydroxyphenyl)-N-[(1R)-1-[3-nitro-5-(trifluoromethyl)phenyl]ethyl]-6-oxo-pyridazine-3-carboxamide (60 mg, 133.82 μmol), (2-chloro-2,2-difluoro-acetyl)oxysodium (204.03 mg, 1.34 mmol) and $Cs_2CO_3$ (436.02 mg, 1.34 mmol) in DMF (2 mL) and $H_2O$ (0.2 mL) was degassed and purged with $N_2$ for 3 times, and then the mixture was stirred at 100 °C for 16 hours under $N_2$ atmosphere. The reaction mixture was poured into water (30 mL) and extracted with EtOAc (30 mL × 3). The combined organic layer was washed with brine (30 mL), dried over anhydrous $Na_2SO_4$ and concentrated under reduced pressure to obtain a crude product. The residue was purified by flash silica gel chromatography (30% EtOAc in PE) to give 1-[2-(difluoromethoxy)phenyl]-N-[(1R)-1-[3-nitro-5-(trifluoromethyl)phenyl]ethyl]-6-oxo-pyridazine-3-carboxamide (38 mg, 17.21% yield) as yellow oil. MS (ESI) m/z = 499.1 $[M+H]^+$.

Step 4: Synthesis of N-[1R)-1-[3-amino-5-(trifluoromethyl)phenyl]ethyl]-1-[2-(difluoromethoxy)phenyl]-6-oxo-pyridazine-3-carboxamide

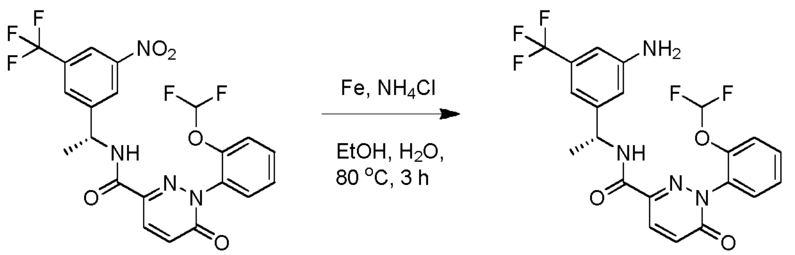


**247**

The compound of Example 247 (3.3 mg, 8.58% yield) was obtained as a white solid in the same manner as in Step 3 of Example 51. $^1H$ NMR (400MHz, DMSO-$d_6$) δ = 8.83 (d, *J* = 8.4 Hz, 1H), 7.94 (d, *J* = 9.6 Hz, 1H), 7.66 (dd, *J* = 1.6, 8.0 Hz, 1H), 7.61 (t, *J* = 7.6 Hz, 1H), 7.48 - 7.43 (m, 1H), 7.41 (d, *J* = 8.0 Hz, 1H), 7.33 (s, 1H), 7.18 (s, 1H), 7.18 - 7.12 (m, 1H), 6.96 (s, 1H), 6.79 (s, 1H), 6.76 (s, 1H), 6.69 (s, 1H), 5.53 (s, 2H), 5.07 - 4.99 (m, 1H), 1.42 (d, *J* = 7.2 Hz, 3H); MS (ESI) m/z = 469.3 $[M+H]^+$.

**Example 248: Tert-butyl 3-[3-[[(1R)-1-[3-amino-5-(trifluoromethyl)phenyl]ethyl]carbamoyl]-6-oxo-pyridazin-1-yl]benzoate**

Step 1: Synthesis of methyl 1-(3-tert-butoxycarbonylphenyl)-6-oxo-pyridazine-3-carboxylate

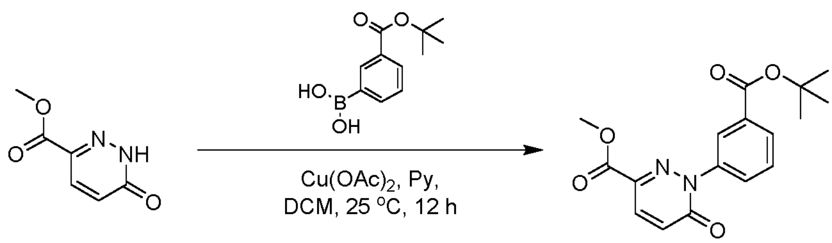


To a mixture of methyl 6-oxo-1H-pyridazine-3-carboxylate (300 mg, 1.95 mmol) and (3-tert-butoxycarbonylphenyl)boronic acid (432.99 mg, 1.95 mmol) in DCM (4 mL), $Cu(OAc)_2$ (177.09 mg, 975.00 μmol), Py (1.00 g, 12.68 mmoL, 1.02 mL) and 4A MS (300 mg) were added, and the mixture was stirred at 25 °C for 16 hours. The reaction mixture was poured into distilled water (20 mL) and extracted with EtOAc (20 mL × 3). The combined organic layer was washed with brine (50 mL × 4), dried over anhydrous $Na_2SO_4$ and concentrated under reduced pressure to give methyl 1-(3-tert-butoxycarbonylphenyl)-6-oxo-pyridazine-3-carboxylate (670 mg, 99.21% yield) as yellow oil. MS (ESI) m/z = 275.0 $[M+H]^+$.

Step 2: Synthesis of 1-(3-tert-butoxycarbonylphenyl)-6-oxo-pyridazine-3-carboxylic acid

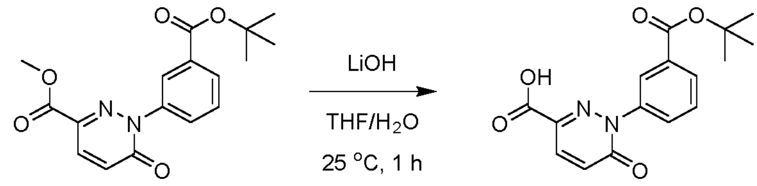


To a solution of methyl 1-(3-tert-butoxycarbonylphenyl)-6-oxo-pyridazine-3-carboxylate (670 mg, 2.03 mmol) in THF (9 mL), $LiOH.H_2O$ (255.34 mg, 6.08 mmol) and $H_2O$ (4.5 mL) were added, and the reaction mixture was stirred at 25 °C for 1.5 hours under air. The mixture was poured into water (30 mL) and extracted with EtOAc (30 mL × 3). The combined organic layer was washed with brine (50 mL), dried over anhydrous $Na_2SO_4$ and concentrated under reduced pressure to give 1-(3-tert-butoxycarbonylphenyl)-6-oxo-pyridazine-3-carboxylic acid (493 mg, 69.41% yield) as a yellow solid. $^1H$ NMR (400 MHz, DMSO-$d_6$) $\delta$ = 8.08 (t, *J* = 1.6 Hz, 1H), 8.00 (d, *J* = 7.6 Hz, 1H), 7.92 (d, *J* =10 Hz, 1H), 7.84 (td, *J* = 1.0, 7.2 Hz, 1H), 7.71 - 7.63 (m, 1H), 7.15 (d, *J* = 10 Hz, 1H), 1.56 (s, 9H); MS (EI) m/z: 261.1 $[M+H]^+$.

Step 3: Synthesis of tert-butyl 3-[3-[[(1R)-1-[3-amino-5-(trifluoromethyl)phenyl]ethyl]carbamoyl]-6-oxo-pyridazin-1-yl]benzoate

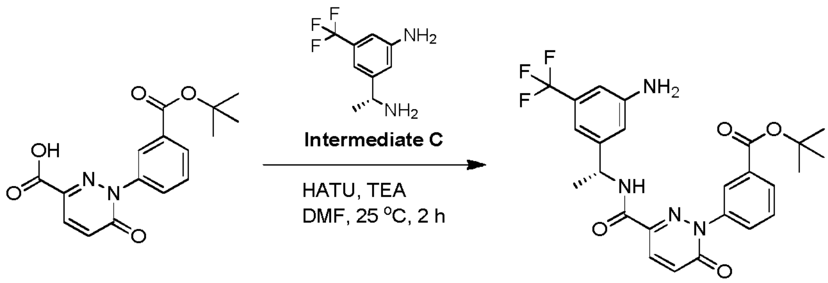


248

To a solution of 1-(3-tert-butoxycarbonylphenyl)-6-oxo-pyridazine-3-carboxylic acid (100 mg, 316.15 µmol) and Intermediate C (64.55 mg, 316.15 µmol) in DMF (5 mL), EDCI (90.91 mg, 474.22 µmol), HOBt (64.08 mg, 474.22 µmol) and TEA (159.95 mg, 1.58 mmol, 220.02 µL) were added, and the reaction mixture was stirred at 25 °C for 12 hours under $N_2$ atmosphere. The reaction mixture was poured into distilled water (10 mL) and extracted with EtOAc (20 mL × 3). The combined organic layer was washed with brine (30 mL), dried over anhydrous $Na_2SO_4$ and concentrated under reduced pressure to obtain a crude product. The product was purified by silica gel column chromatography (58% EtOAc in PE) to give the compound of Example 248 (50 mg, 15.36% yield) as yellow oil. $^1H$ NMR (400 MHz, DMSO-$d_6$) $\delta$ = 8.88 (d, J = 8.4 Hz, 1H), 8.15 (t, J = 1.6 Hz, 1H), 7.99 (td, J = 1.2, 8.0 Hz, 1H), 7.95-7.89 (m, 2H), 7.68 (t, J = 8.0 Hz, 1H), 7.64-7.64 (m, 1H), 7.16 (d, J = 9.6 Hz, 1H), 6.80 (d, J = 12.8 Hz, 2H), 6.70 (s, 1H), 5.54 (s, 2H), 5.04 (quin, J = 7.2 Hz, 1H), 1.56 (s, 9H), 1.44 (d, J = 7.2 Hz, 3H) ; LC/MS (ESI) m/z = 447.3 $[M+H]^+$

**Example 249: 3-(3-{[(1R)-1-[3-amino-5-(trifluoromethyl)phenyl]ethyl]carbamoyl}-6-oxo-1,6-dihydropyridazin-1-yl)benzoic acid**

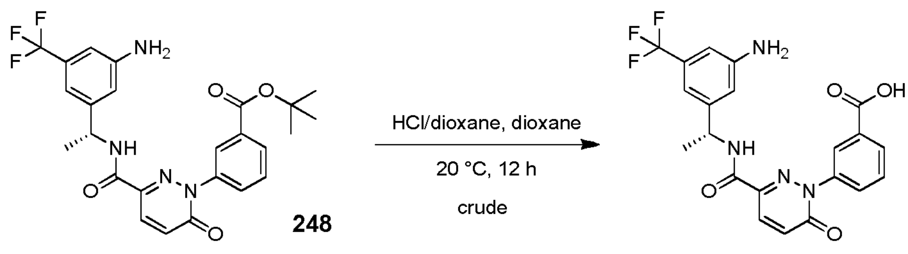


248

249

To a solution of the compound of Example 248 (30 mg, 59.70 µmol) in dioxane (1 mL) was added HCl/dioxane (4 M, 2 mL). The mixture was stirred at 20°C for 12 hours and concentrated under reduced pressure to give the compound

of Example 249 (25 mg, crude, HCl) as a yellow solid, which was used into the next step without further purification.

**Example 250 and Example 251**

Carboxylic acid compounds were prepared in the same manner as in Example 249, and the compounds shown in the following table were prepared through an amide coupling reaction.

[Table 15]

| No | Structure / Name | Spectral Data |
|---|---|---|
| 250 | 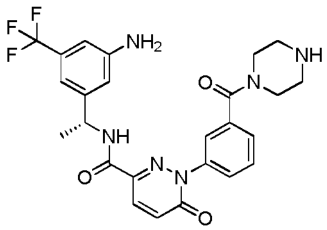 N-[(1R)-1-[3-amino-5-(trifluoromethyl)phenyl] ethyl]-6-oxo-1-[3-(piperazine-1-carbonyl)phenyl] pyridazine-3- carboxamide | $^1$H NMR (400 MHz, DMSO-$d_6$) δ = 8.88 (d, *J* = 8.4 Hz, 1H), 7.89 (d, *J* = 9.6 Hz, 1H), 7.80-7.74 (m, 1H), 7.72 (t, *J* = 1.6 Hz, 1H), 7.61 (t, *J* = 8.0 Hz, 1H), 7.47 (td, *J* = 1.2, 7.6 Hz, 1H), 7.15 (d, *J* = 9.6 Hz, 1H), 6.80 (d, *J* = 11.6 Hz, 2H), 6.70 (s, 1H), 5.54 (s, 2H), 5.03 (quin, *J* = 7.2 Hz, 1H), 3.65-3.38 (m, 4H), 2.84-2.58 (m, 5H), 1.44 (d, *J* = 7.2 Hz, 3H) ; LC/MS (ESI) m/z: 515.4 [M+H]$^+$ |
| 251 | 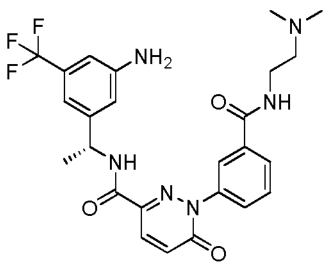 N-[(1R)-1-[3-amino-5-(trifluoromethyl)phenyl] ethyl]-1-[3-[2-(dimethylamino)ethylcarbamoyl] phenyl]-6-oxo-pyridazine-3-carboxamide | $^1$H NMR (400 MHz, DMSO-$d_6$) δ = 8.91 (d, *J* = 8.4 Hz, 1H), 8.51 (t, *J* = 5.6 Hz, 1H), 8.09 (t, *J* = 1.6 Hz, 1H), 7.96-7.88 (m, 2H), 7.84-7.78 (m, 1H), 7.67-7.60 (m, 1H), 7.17 (d, *J* = 9.6 Hz, 1H), 6.79 (d, *J* = 12.4 Hz, 2H), 6.70 (s, 1H), 5.55 (s, 2H), 5.04 (quin, *J* = 7.2 Hz, 1H), 2.82 (s, 2H), 2.39 (t, *J* = 6.8 Hz, 2H), 2.16 (s, 6H), 1.44 (d, *J* = 7.2 Hz, 3H); LC/MS (ESI) m/z: 517.4 [M+H]$^+$ |

**Example 252: N-[(1R)-1-[3-amino-5-(trifluoromethyl)phenyl]ethyl]-1-(3-carbamoylphenyl)-6-oxo-pyridazine-3-carboxamide**

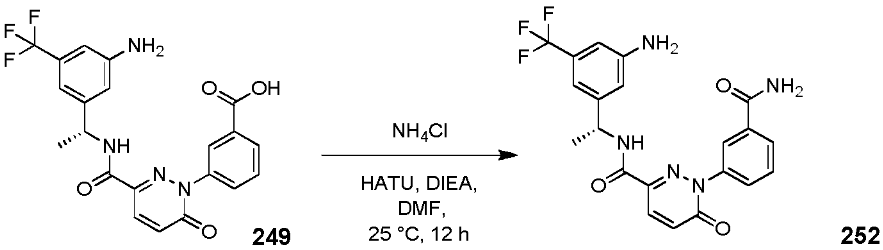


To a mixture of the compound of Example 249 (30 mg, 67.21 μmol) in DMF (2 mL), DIEA (43.43 mg, 336.04 μmol, 58.53 μL) and HATU (38.33 mg, 100.81 μmol) were added, and the reaction mixture was stirred at 25 °C for 2 hours under $N_2$. Then, the reaction mixture was stirred at 60 °C for 1 hour under $N_2$. $NH_4Cl$ (10.79 mg, 201.62 μmol) was added, and the reaction mixture was stirred at 25°C for 10 hours under $N_2$. The mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue, which was purified by prep-HPLC (column: Phenomenex C18 75*30mm*3μm; mobile phase: [water($NH_3H_2O$+$NH_4HCO_3$)-ACN]; B%: 22%-52%, 11min). The remaining solvent was removed by lyophilization to give the compound of Example 252 (5.4 mg, 17.92% yield). $^1$H NMR (400MHz, DMSO-$d_6$) δ = 8.88 (d, *J* = 8.4 Hz, 1H), 8.13 (d, *J* = 2.0 Hz, 1H), 8.07 (br s, 1H), 7.96 (d, *J* = 8.0 Hz,

1H), 7.91 (d, *J* = 10.0Hz, 1H), 7.82 (dd, *J* = 1.2, 8.0 Hz, 1H), 7.63 (t, *J* = 8.0 Hz, 1H), 7.51 ( s, 1H), 7.17 (d, *J* = 10.0 Hz, 1H), 6.80 ( d, *J* = 11.2 Hz, 2H), 6.70 (s, 1H), 5.53 (s, 2H), 5.10 - 4.95 (m, 1H), 1.44 (d, *J* = 7.2 Hz, 3H); MS (ESI) m/z = 446.2 $[M+H]^+$.

**Example 253 to Example 255**

The compounds shown in the following table were prepared by amidation of a carboxylic acid group in a similar manner to Example 252.

[Table 16]

| No. | Structure/Name | Spectral Data |
|---|---|---|
| 253 | 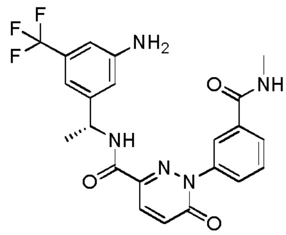 N-[(1R)-1-[3-amino-5-(trifluoromethyl)phenyl] ethyl]-1-[3-(methylcarbamoyl)phenyl]-6-oxo-pyridazine-3-carboxamide | $^1$H NMR (400 MHz, DMSO-$d_6$) $\delta$ = 8.88 (d, *J* = 8.4 Hz, 1H), 8.55 ( d, *J* = 4.6 Hz, 1H), 8.10 - 8.07 (m, 1H), 7.92 (s, 1H), 7.91 - 7.89 (m, 1H), 7.81 (dd, *J* = 0.8, 7.9 Hz, 1H), 7.67 -7.60 (m, 1H), 7.17 (d, *J* = 10.0 Hz, 1H), 6.81 (s, 1H), 6.78 (s, 1H), 6.70 (s, 1H), 5.54 (s, 2H), 5.04 (t, *J* = 7.6 Hz, 1H), 2.80 (d, *J* = 4.4 Hz, 3H), 1.47 - 1.42 (m, 3H); LC/MS (ESI) m/z: 460.3 $[M+H]^+$ |
| 254 | 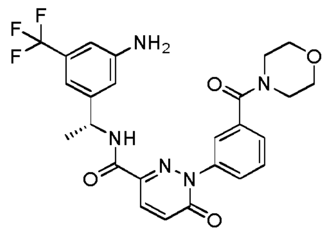 N-[(1R)-1-[3-amino-5-(trifluoromethyl)phenyl] ethyl]-1-[3-(morpholine-4-carbonyl)phenyl]-6-oxo-pyridazine-3-carboxamide | $^1$H NMR (400 MHz, DMSO-$d_6$) $\delta$ = 8.88 (d, *J* = 8.4 Hz, 1H), 7.89 (d, *J* = 10.0 Hz, 1H), 7.81 - 7.77 (m, 1H), 7.77 - 7.75 (m, 1H), 7.62 (t, *J* = 8.0 Hz, 1H), 7.51 (td, *J* = 1.3, 7.6 Hz, 1H), 7.16 (d, *J* = 9.6 Hz, 1H), 6.81 (s, 1H), 6.78 (s, 1H), 6.70 (s, 1H), 5.54 (s, 2H), 5.04 (q, *J* = 7.2 Hz, 1H), 3.72 - 3.41 (m, 8H), 1.45 (d, *J* = 7.2 Hz, 3H); LC/MS (ESI) m/z: 516.4 $[M+H]^+$ |
| 255 | 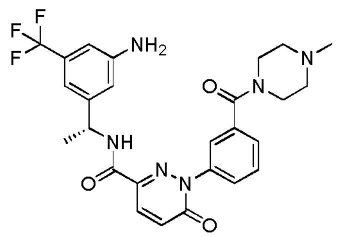 N-[(1R)-1-[3-amino-5-(trifluoromethyl)phenyl] ethyl]-1-[3-(4-methylpiperazine-1-carbonyl) phenyl]-6-oxo-pyridazine-3-carboxamide | $^1$H NMR (400 MHz, DMSO-$d_6$) $\delta$ = 8.88 (d, *J* = 8.4 Hz, 1H), 7.89 (d, *J* = 10.0 Hz, 1H), 7.78 (ddd, *J* = 1.2, 2.0, 8.0 Hz, 1H), 7.72 (t, *J* = 2.0 Hz, 1H), 7.61 (t, *J* = 8.0 Hz, 1H), 7.48 (td, *J* = 1.4, 7.6 Hz, 1H), 7.16 (d, *J* = 10.0 Hz, 1H), 6.81 (s, 1H), 6.78 (s, 1H), 6.70 (s, 1H), 5.54 (s, 2H), 5.10 - 4.96 (m, 1H), 4.79 - 4.76 (m, 1H), 3.62 (br s, 2H), 3.39 (d, *J* = 6.8 Hz, 2H), 2.40 - 2.25 (m, 4H), 2.18 (s, 3H), 1.47 - 1.43 (m, 3H) ; LC/MS (ESI) m/z: 529.4 $[M+H]^+$ |

**Example 256: N-[(1R)-1-[3-[1,1-difluoro-2-(methylamino)ethyl]-2-fluoro-phenyl] ethyl]-1-(2-fluorophenyl)-6-oxo-pyridazine-3-carboxamide**

Step 1: Synthesis of [2,2-difluoro-2-[2-fluoro-3-[(1R)-1-[[l-(2-fluorophenyl)-6-oxo-pyridazine-3-carbonyl]amino]ethyl]phenyl]ethyl]trifluoromethanesulfonate

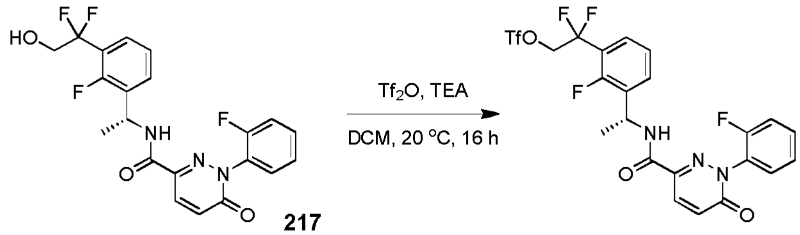


217

To a solution of the compound of Example 217 (100 mg, 229.69 μmol) in DCM (2 mL), $Tf_2O$ (129.61 mg, 459.38 μmol) and TEA (69.73 mg, 689.07 μmol) were added, and then the mixture was stirred at 20 °C for 16 hours. The reaction mixture was poured into water (15 mL) and extracted with DCM (15 mL × 3). The combined organic layer was washed with brine (15 mL), dried over anhydrous $Na_2SO_4$ and concentrated under reduced pressure to give [2,2-difluoro-2-[2-fluoro-3-[(1R)-1-[[1-(2-fluorophenyl)-6-oxo-pyridazine-3-carbonyl]amino]ethyl]phenyl]ethyl]trifluoromethanesulfonate (120 mg, 92.0% yield) as yellow oil. MS (ESI) m/z = 568.1 $[M+H]^+$.

Step 2: Synthesis of N-[(1R)-1-[3-[1,1-difluoro-2-(methylamino)ethyl]-2-fluorophenyl]ethyl]-1-(2-fluorophenyl)-6-oxo-pyridazine-3-carboxamide

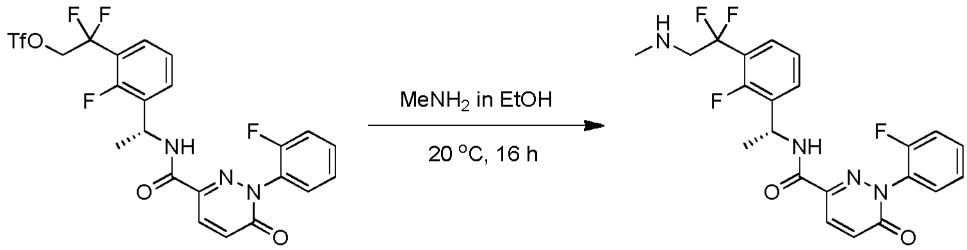


256

A mixture of [2,2-difluoro-2-[2-fluoro-3-[(1R)-1-[[1-(2-fluorophenyl)-6-oxo-pyridazine-3-carbonyl]amino]ethyl]phenyl]ethyl]trifluoromethanesulfonate (60 mg, 105.74 μmol) and methanamine (1 g, 10.63 mmol, 33% purity) was stirred at 20 °C for 16 hours. The reaction mixture was concentrated under vacuum to obtain a crude product. The crude product was purified by prep-TLC (8% MeOH in DCM) to give an impure product. The product was purified by prep-HPLC (column: C18-6 100*30mm*5μm; mobile phase: [water(FA)-ACN]; B%: 10%-40%), and most of MeCN was removed under reduced pressure. The remaining solvent was removed by lyophilization to give the compound of Example 256 (2.0 mg, 3.7% yield) as a white solid. $^1$H NMR (400MHz, DMSO-$d_6$) δ = 9.01 (d, *J* = 8.0 Hz, 1H), 7.97-7.88 (m, 1H), 7.69 (dt, *J* = 1.6, 7.6 Hz, 1H), 7.64-7.55 (m, 2H), 7.49-7.38 (m, 3H), 7.28-7.22 (m, 1H), 7.18 (d, *J* = 10.0 Hz, 1H), 5.39 (quin, *J* = 7.2 Hz, 1H), 3.20 (s, 2H), 3.16 (br s, 1H), 2.26 (s, 3H), 1.45 (d, *J* = 7.2 Hz, 3H); MS (ESI) m/z = 449.1 $[M+H]^+$.

**Example 257: N-[(1R)-1-[3-[2-(dimethylamino)-1,1-difluoro-ethyl]-2-fluorophenyl]ethyl]-1-(2-fluorophenyl)-6-oxo-pyridazine-3-carboxamide**

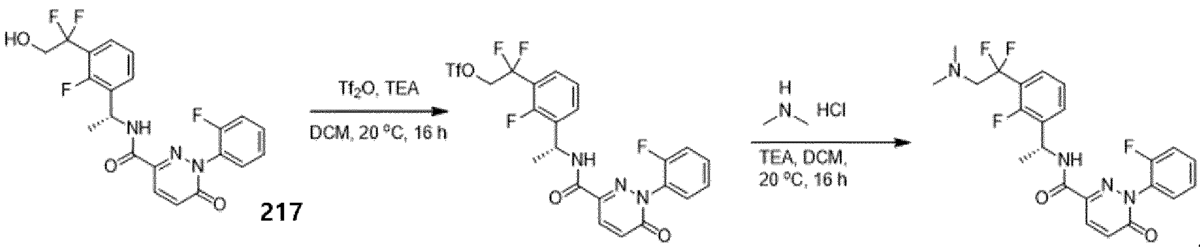


217

257

The compound of Example 257 (2.3 mg, 3.58% yield) was obtained as a white solid in the same manner as in Example 256 by replacing methanamine with dimethanamine in Step 2. $^1$H NMR (400MHz, DMSO-$d_6$) δ = 9.01 (d, *J* =

8.0 Hz, 1H), 7.92 (d, *J* = 10.0 Hz, 1H), 7.68 (dt, *J* = 1.6, 7.6 Hz, 1H), 7.63-7.55 (m, 2H), 7.50-7.38 (m, 3H), 7.30-7.23 (m, 1H), 7.18 (d, *J* = 10.0 Hz, 1H), 5.39 (quin, *J* = 7.2 Hz, 1H), 3.07 (t, *J* = 15.2 Hz, 2H), 2.18 (s, 6H), 1.46 (d, *J* = 7.2 Hz, 3H); MS (ESI) m/z = 463.3 $[M+H]^+$.

**Example 258: N-[(1R)-1-[3-[2-(dimethylamino)-1,1-difluoro-ethyl]phenyl]ethyl]-1-(2-fluorophenyl)-6-oxo-pyridazine-3-carboxamide**

Step 1: Synthesis of (R)-N-(1-(3-(1,1-difluoro-2-hydroxyethyl)phenyl)ethyl)-1-(2-fluorophenyl)-6-oxo-1,6-dihydropyridazine-3-carboxamide

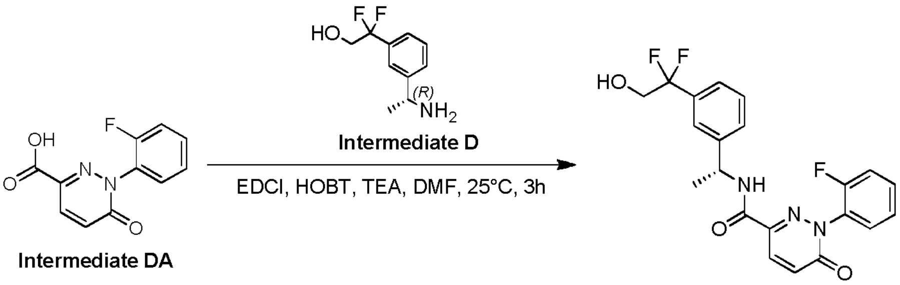


(R)-N-(1-(3-(1,1-difluoro-2-hydroxyethyl)phenyl)ethyl)-1-(2-fluorophenyl)-6-oxo-1,6-dihydropyridazine-3-carboxamide was obtained in a similar manner to Step 2 of Example 51 by using Intermediate D and Intermediate DA.

Steps 2 and 3: Synthesis of N-[(1R)-1-[3-[2-(dimethylamino)-1,1-difluoroethyl]phenyl]ethyl]-1-(2-fluorophenyl)-6-oxo-pyridazine-3-carboxamide

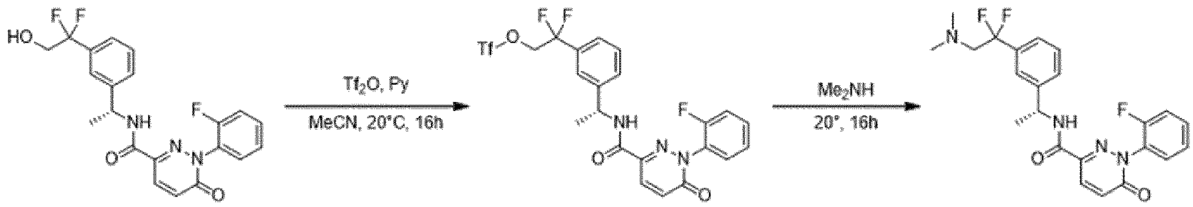


258

The compound of Example 258 (10.3 mg, 12.67% yield) was obtained as a yellow solid in the same manner as in Example 243, except that $Me_2NH$ was used instead of $NH_3 \cdot H_2O$. $^1H$ NMR (400MHz, DMSO-$d_6$) $\delta$ = 8.99 (d, *J* = 8.4 Hz, 1H), 7.93 (d, *J* = 9.6 Hz, 1H), 7.72 - 7.64 (m, 1H), 7.62 - 7.53 (m, 2H), 7.51 - 7.44 (m, 2H), 7.43 - 7.36 (m, 3H), 7.18 (d, *J* = 10.0 Hz, 1H), 5.17 (t, *J* = 8.0 Hz, 1H), 2.98 (t, *J* = 14.8 Hz, 2H), 2.16 (s, 6H), 1.47 (d, *J* = 7.2 Hz, 3H); MS (ESI) m/z = 445.2 $[M+H]^+$.

**Example 259: 1-(2-fluorophenyl)-N-[(1R)-1-[2-fluoro-3-(trifluoromethyl)phenyl]ethyl]-6-oxo-pyridazine-3-carboxamide**

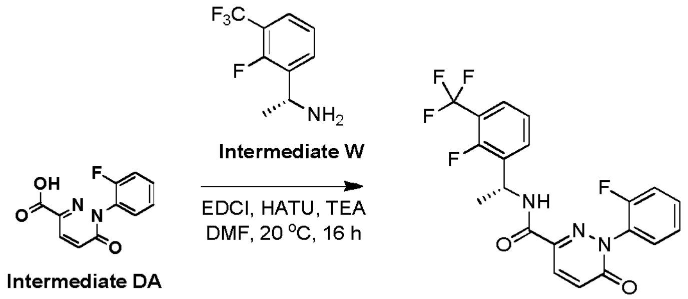


259

The compound of Example 259 (9.3 mg, 10.54% yield) was obtained as a white solid in a similar manner to Step 2 of Example 76 by using Intermediate W and changing the coupling reagents. $^1$H NMR (400MHz, DMSO-$d_6$) $\delta$ = 9.11 (d, $J$ = 7.6 Hz, 1H), 7.92 (d, $J$ = 10.0 Hz, 1H), 7.80 (t, $J$ = 7.2 Hz, 1H), 7.73-7.63 (m, 2H), 7.62-7.55 (m, 1H), 7.49-7.33 (m, 3H), 7.18 (d, $J$ = 10.0 Hz, 1H), 5.40 (quin, $J$ = 7.2 Hz, 1H), 1.48 (d, $J$ = 7.2 Hz, 3H); MS (ESI) m/z = 424.3 [M+H]$^+$.

**Example 260: N-[(1R)-1-(3-cyano-2-fluoro-phenyl)ethyl]-1-(2-fluorophenyl)-6-oxo-pyridazine-3-carboxamide**

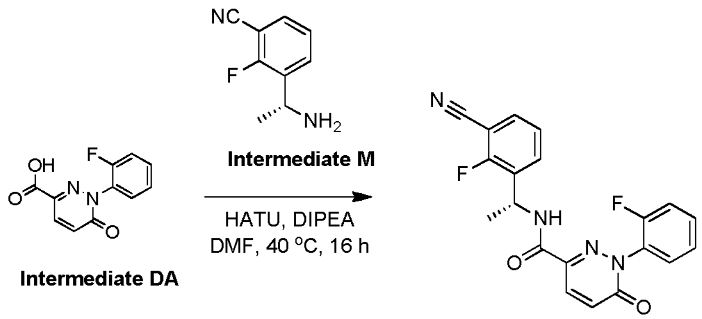


260

The compound of Example 260 (20.1 mg, 20.14% 2-step yield) was obtained as a white solid by reacting Intermediate M and Intermediate DA in a similar manner to Step 2 of Example 64. $^1$H NMR (400MHz, DMSO-$d_6$) $\delta$ = 9.09 (d, $J$ = 7.6 Hz, 1H), 7.91 (d, $J$ = 9.6 Hz, 1H), 7.79-7.86 (m, 2H), 7.69 (td, $J$ = 7.6, 1.6 Hz, 1H), 7.55-7.64 (m, 1H), 7.37-7.49 (m, 3H), 7.18 (d, $J$ = 9.6 Hz, 1H), 5.36 (quin, J = 7.2 Hz, 1H), 1.47 (d, J = 7.2 Hz, 3H); MS (ESI) m/z = 381.1 [M+H]$^+$.

**Example 261 and Example 262: N-[(1R)-1-(2-chloro-3-fluoro-phenyl)ethyl]-1-(2-fluorophenyl)-6-oxo-pyridazine-3-carboxamide and N-[(1S)-1-(2-chloro-3-fluorophenyl)ethyl]-1-(2-fluorophenyl)-6-oxo-pyridazine-3-carboxamide**

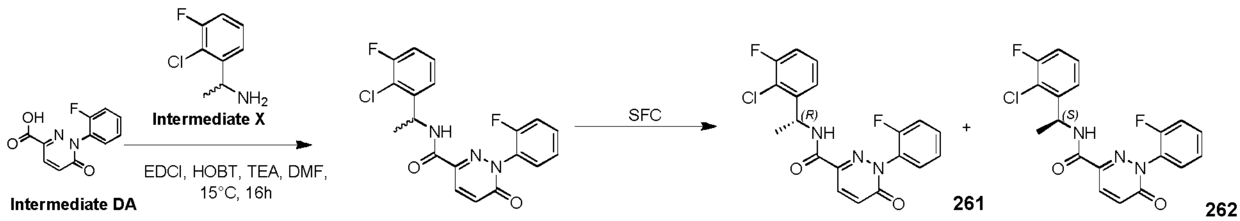


261

262

After performing Step 2 of Example 76 using Intermediate X, the product was separated by SFC to obtain the compound of Example 261 (42.5 mg, 41.97% yield) and the compound of Example 262 (28.1 mg, 68.76 $\mu$mol, 26.80% yield) as a white solid, respectively.

Compound of Example 261: $^1$H NMR (400MHz, DMSO-$d_6$) $\delta$ = 9.12 (d, $J$ = 7.6 Hz, 1H), 7.91 (d, $J$ = 10.0 Hz, 1H), 7.70 (dt, $J$ = 1.6, 7.6 Hz, 1H), 7.63 - 7.55 (m, 1H), 7.50 - 7.40 (m, 2H), 7.39 - 7.33 (m, 2H), 7.32 - 7.25 (m, 1H), 7.18 (d, $J$ = 9.6 Hz, 1H), 5.41 (quin, $J$ = 7.2 Hz, 1H), 1.43 (d, $J$ = 6.8 Hz, 3H); MS (ESI) m/z = 390.3 [M+H]$^+$.

Compound of Example 262: $^1$H NMR (400MHz, DMSO-$d_6$) δ = 9.12 (d, *J* = 7.6 Hz, 1H), 7.91 (d, *J* = 10.0 Hz, 1H), 7.73 - 7.67 (m, 1H), 7.63 - 7.56 (m, 1H), 7.49 - 7.41 (m, 2H), 7.39 - 7.34 (m, 2H), 7.32 - 7.26 (m, 1H), 7.18 (d, *J* = 9.6 Hz, 1H), 5.41 (quin, *J* = 7.2 Hz, 1H), 1.43 (d, *J* = 6.8 Hz, 3H); MS (ESI) m/z = 390.3 $[M+H]^+$.

**Example 263: N-[(1R)-1-[3-amino-5-(difluoromethyl)phenyl]ethyl]-1-(2-fluorophenyl)-6-oxo-pyridine-3-carboxamide**

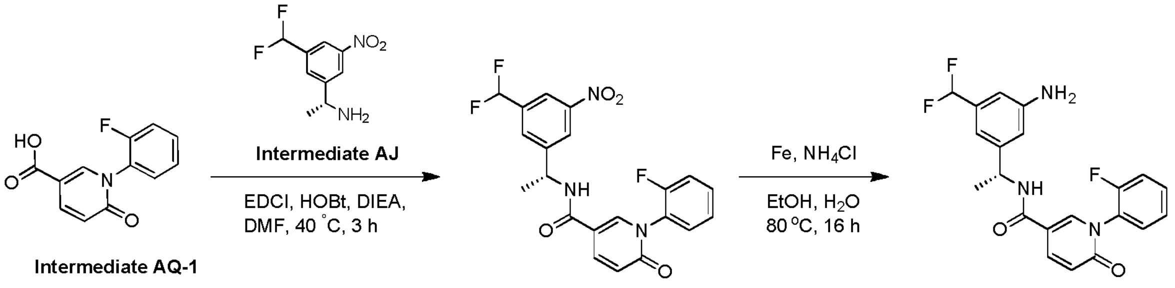


**263**

The compound of Example 263 (13.1 mg, 19.03% 2-step yield) was obtained as a white solid in a similar manner to steps 2 and 3 of Example 51. $^1$H NMR (400MHz, DMSO-$d_6$) δ = 8.54 (d, *J* = 7.6 Hz, 1H), 8.37 (d, *J* = 2.4 Hz, 1H), 7.99 (dd, *J* = 9.6, 2.4 Hz, 1H), 7.55-7.64 (m, 2H), 7.38-7.50 (m, 2H), 6.66-6.96 (m, 1H), 6.65 (s, 2H), 6.54-6.59 (m, 2H), 5.35 (s, 2H), 4.99 (q, *J* = 7.2 Hz, 1H), 1.39 (d, *J* = 6.8 Hz, 3H); MS (ESI) m/z = 402.3 $[M+H]^+$.

**Example 264: N-[(1R)-1-[3-amino-5-(trifluoromethyl)phenyl]ethyl]-6-oxo-1-phenyl-4,5-dihydropyridazine-3-carboxamide**

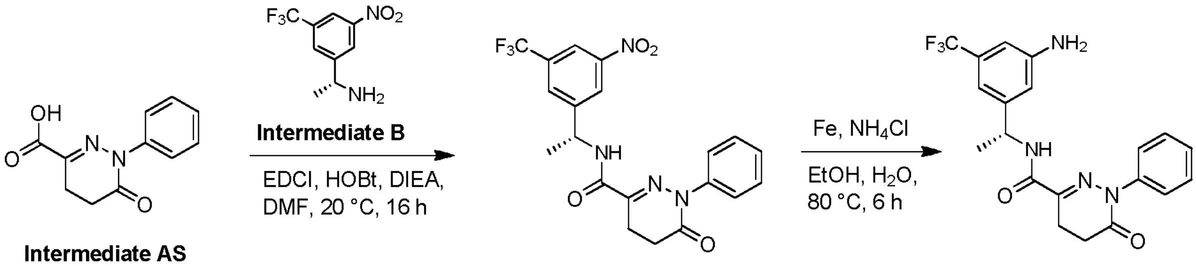


**264**

The compound of Example 264 (19.8 mg, 42.46% yield) was obtained as a white solid in a similar manner to Steps 2 and 3 of Example 51. $^1$H NMR (400 MHz, DMSO-$d_6$) δ = 8.54 (d, *J* = 8.4 Hz, 1H), 7.52 (d, *J* = 7.6 Hz, 2H), 7.43 (t, *J* = 7.2 Hz, 2H), 7.34 - 7.26 (m, 1H), 6.82 (s, 1H), 6.76 (s, 1H), 6.70 (s, 1H), 5.53 (s, 2H), 4.96 (quin, *J* = 7.2 Hz, 1H), 2.89 (t, *J* = 8.4 Hz, 2H), 2.65 (t, *J* = 8.4 Hz, 2H), 1.42 (d, *J* = 7.2 Hz, 3H); MS (ESI) m/z = 405.0 $[M+H]^+$.

**Example 265: N-[(1R)-1-(5-bromo-2-thienyl)ethyl]-1-(2-fluorophenyl)-6-oxo-pyridazine-3-carboxamide**

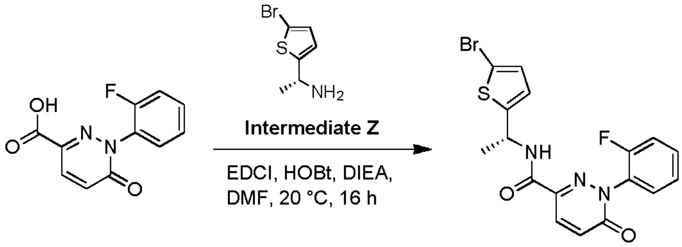


**265**

The compound of Example 265 (10.1 mg, 19.49% yield) was obtained as a white solid by using Intermediate Z in the same manner as in Step 2 of Example 76. $^{1}H$ NMR (400 MHz, DMSO-$d_6$) δ = 9.04 (d, *J*=8.4 Hz, 1H), 7.97 (d, *J*=10.0 Hz, 1H), 7.66 (dt, *J*=1.8, 7.6 Hz, 1H), 7.61-7.54 (m, 1H), 7.48-7.37 (m, 2H), 7.19 (d, *J* = 10.0 Hz, 1H), 7.05 (d, *J* = 3.6 Hz, 1H), 6.83 (dd, *J* = 0.8, 3.6 Hz, 1H), 5.28 (quin, *J* = 7.2 Hz, 1H), 1.53 (d, *J* = 7.2 Hz, 3H); MS (ESI) m/z = 424.4 $[M+H]^+$.

**Example 266: 1-(2-fluorophenyl)-N-[(1R)-1-[5-[4-(methylaminomethyl)-3-bicyclo[4.2.0]octa-1,3,5-trienyl]-2-thienyl]ethyl]-6-oxo-pyridazine-3-carboxamide**

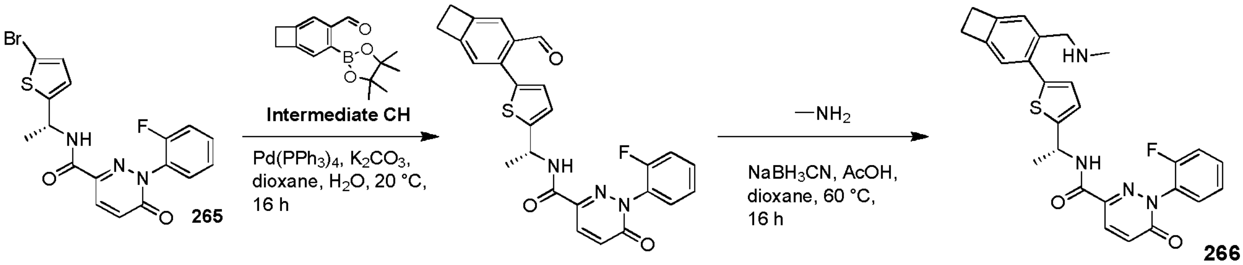


265

266

The compound of Example 266 (8.2 mg, 12.51% yield) was obtained as a white solid by reacting the compound of Example 265 and Intermediate CH in the same manner as in Steps 2 and 3 of Example 137. $^{1}H$ NMR (400 MHz, DMSO-$d_6$) δ = 9.04 (d, *J* = 8.6 Hz, 1H), 7.99 (d, *J* = M 10.0 Hz, 1H), 7.66 (dt, *J* = 1.6, 7.6 Hz, 1H), 7.61 - 7.53 (m, 1H), 7.48 - 7.35 (m, 2H), 7.24 - 7.14 (m, 2H), 7.05 - 6.92 (m, 3H), 5.45 - 5.34 (m, 1H), 3.58 (s, 2H), 3.14 (s, 4H), 2.22 (s, 3H), 1.58 (d, *J*= 7.2 Hz, 3H); MS (ESI) m/z = 489.1 $[M-16+H]^+$.

**Example 267 to Example 293**

The compounds shown in the following table were obtained in the same manner as in Example 266 by using appropriate staring materials and intermediates corresponding to the respective structures of the desired compounds.

[Table 17]

| No. | Structure/Name | Spectral Data |
|---|---|---|
| 267 | 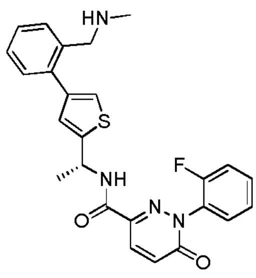 1-(2-fluoropheiryl)-N-[(1R)-1-[4-[2-(methylaminomethyl)phenyl]-2-thienyl] ethyl]-6-oxo-pyridazine-3-carboxamide | $^{1}H$ NMR (400 MHz, DMSO-$d_6$) δ = 9.03 (d, J = 8.4 Hz, 1H), 7.99 (d, J = 9.6 Hz, 1H), 7.66 (dt, J = 1.6, 7.6 Hz, 1H), 7.61 - 7.53 (m, 1H), 7.49 - 7.41 (m, 3H), 7.41 - 7.35 (m, 1H), 7.32 - 7.24 (m, 3H), 7.23 - 7.17 (m, 2H), 5.48 - 5.35 (m, 1H), 3.56 (s, 2H), 2.24 (s, 3H), 1.60 (d, J = 7.2 Hz, 3H); LC/MS (ESI) m/z: 463.3 $[M+H]^+$ |

(continued)

| No. | Structure/Name | Spectral Data |
|---|---|---|
| 268 | 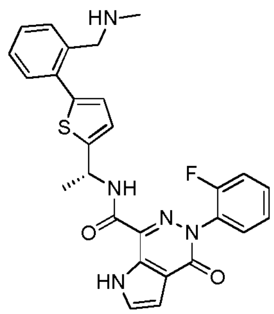 5-(2-fluorophenyl)-N-[(1R)-1-[5-[2-(methylaminomethyl)phenyl]-2-thienyl]ethyl]-4-oxo-1H-pyrrolo[2,3-d]pyridazine-7-carboxamide | $^1$H NMR (400 MHz, DMSO-$d_6$) $\delta$ = 12.24 (br s, 1H), 9.09 (br d, $J$ = 8.4 Hz, 1H), 7.67 - 7.59 (m, 1H), 7.57 - 7.51 (m, 1H), 7.50 - 7.47 (m, 1H), 7.45 (d, $J$ = 2.8 Hz, 1H), 7.44 - 7.36 (m, 2H), 7.36 - 7.30 (m, 2H), 7.30 - 7.26 (m, 1H), 7.13 (d, $J$ = 3.6 Hz, 1H), 7.04 (dd, $J$ = 0.8, 3.6 Hz, 1H), 6.78 (d, $J$ = 2.8 Hz, 1H), 5.49 (t, $J$ = 7.2 Hz, 1H), 3.64 (s, 2H), 2.24 (s, 3H), 1.64 (d, $J$ = 7.2 Hz, 3H); LC/MS (ESI) m/z: 502.4 $[M+H]^+$ |
| 269 | 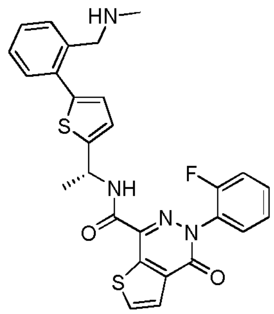 5-(2-fluorophenyl)-N-[(1R)-1-[5-[2-(methylaminomethyl)phenyl]-2-thienyl]ethyl]-4-oxo-thieno[2,3-d]pyridazine-7-carboxamide | $^1$H NMR (400 MHz, DMSO-$d_6$) $\delta$ = 9.27 (d, $J$ = 8.6 Hz, 1H), 8.27 (d, $J$ = 5.2 Hz, 1H), 7.77 - 7.68 (m, 2H), 7.63 - 7.54 (m, 1H), 7.51 - 7.38 (m, 3H), 7.38 - 723 (m, 3H), 7.13 (d, $J$ = 3.6 Hz, 1H), 7.07 - 7.01 (m, 1H), 5.48 (quin, $J$ = 7.2 Hz, 1H), 3.64 (s, 2H), 2.24 (s, 3H), 2.14 - 1.86 (m, 1H), 1.64 (d, $J$ = 7.2 Hz, 3H); LC/MS (ESI) m/z: 519.3 $[M+H]^+$ |
| 270 | 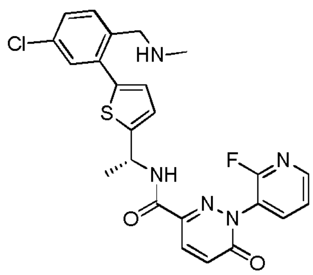 N-[(1R)-1-[5-[5-chloro-2-(methylaminomethyl)phenyl]-2-thienyl]ethyl]-1-(2-fluoro-3-pyridyl)-6-oxo-pyridazine-3-carboxamide | $^1$H NMR (400 MHz, DMSO-$d_6$) $\delta$ = 9.13 (d, $J$ = 8.4 Hz, 1H), 8.40 (br d, $J$ = 4.8 Hz, 1H), 8.35 - 8.28 (m, 1H), 8.16 (s, 1H), 8.00 (d, $J$ = 9.6 Hz, 1H), 7.68 - 7.56 (m, 2H), 7.49 (dd, $J$ = 2.0, 8.4 Hz, 1H), 7.40 (d, $J$ = 2.0 Hz, 1H), 7.23 (d, $J$ = 9.6 Hz, 1H), 7.16 (d, $J$ = 3.6 Hz, 1H), 7.05 (d, $J$ = 3.6 Hz, 1H), 5.54 - 5.33 (m, 1H), 3.90 (s, 2H), 2.37 (s, 3H), 1.60 (d, $J$ = 7.2 Hz, 3H); LC/MS (ESI) m/z: 498.3 $[M+H]^+$ |
| 271 | 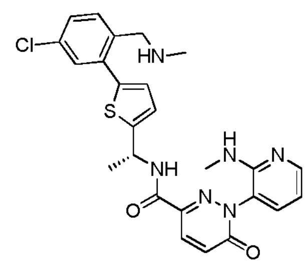 N-[(1R)-1-[5-[5-chloro-2-(methylaminomethyl)phenyl]-2-thienyl]ethyl]-1-[2-(methylamino)-3-pyridyl]-6-oxo-pyridazine-3-carboxamide | $^1$H NMR (400 MHz, DMSO-$d_6$) $\delta$ = 8.97 (d, $J$ = 8.4 Hz, 1H), 8.18 (s, 1H), 8.12 (dd, $J$ = 1.6, 4.8 Hz, 1H), 7.94 (d, $J$ = 10.0 Hz, 1H), 7.57 (d, $J$ = 8.4 Hz, 1H), 7.45 (dt, $J$ = 2.0, 8.0 Hz, 2H), 7.36 (d, $J$ = 2.4 Hz, 1H), 7.16 (d, $J$ = 3.6 Hz, 1H), 7.12 (d, $J$ = 9.8 Hz, 1H), 7.03 (d, $J$ = 2.8 Hz, 1H), 6.62 (dd, $J$ = 4.8, 7.2 Hz, 1H), 6.35 (q, $J$ = 4.4 Hz, 1H), 5.41 (quin, $J$ = 7.2 Hz, 1H), 3.77 (s, 2H), 2.76 (d, $J$ = 4.4 Hz, 3H), 2.31 (s, 3H), 1.59 (d, $J$ = 6.8 Hz, 3H); LC/MS (ESI) m/z: 509.4 $[M+H]^+$ |

(continued)

| No. | Structure/Name | Spectral Data |
|---|---|---|
| 272 | 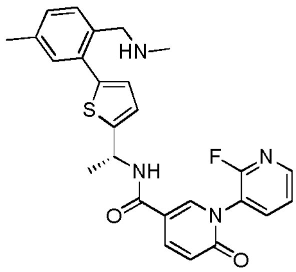 1-(2-fluoro-3-pyridyl)-N-[(1R)-1-[5-[5-methyl-2-(methylaminomethyl)phenyl]-2-thienyl]ethyl]-6-oxo-pyridine-3-carboxamide | $^1H$ NMR (400 MHz, DMSO-$d_6$) δ = 9.08 (d, *J* = 8.0 Hz, 1H), 8.60 (d, *J* = 2.0 Hz, 1H), 8.36 (dd, *J* = 2.4, 8.4 Hz, 1H), 8.14 (br d, *J* = 4.8 Hz, 1H), 8.04 - 7.93 (m, 1H), 7.48 (dd, *J* = 5.2, 7.6 Hz, 1H), 7.37 (d, *J* = 8.0 Hz, 1H), 7.31 (d, *J* = 8.4 Hz, 1H), 7.20 -7.10 (m, 3H), 7.02 (d, *J* = 3.2 Hz, 1H), 5.49 - 5.41 (m, 1H), 3.62 (s, 2H), 2.29 (s, 3H), 2.25 (s, 3H), 1.61 (d, *J* = 6.8 Hz, 3H); LC/MS (ESI) m/z: 477.4 $[M+H]^+$ |
| 273 | 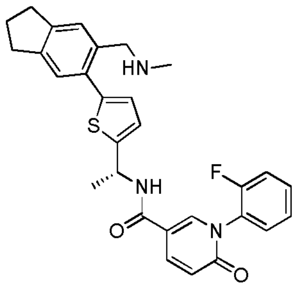 1-(2-fluorophenyl)-N-[(1R)-1-[5-[6-(methylaminomethyl)indan-5-yl]-2-thienyl]ethyl]-6-oxo-pyridine-3-carboxamide | $^1H$ NMR (400 MHz, DMSO-$d_6$) δ = 8.74 (d, *J* = 8.0 Hz, 1H), 8.37 (d, *J* = 2.4 Hz, 1H), 8.01 (dd, *J* = 2.4, 9.6 Hz, 1H), 7.63 - 7.55 (m, 2H), 7.47 (t, *J* = 8.8 Hz, 1H), 7.42 - 7.37 (m, 2H), 7.21 (s, 1H), 7.04 (d, *J* = 4.0 Hz, 1H), 7.00 (d, *J* = 3.2 Hz, 1H), 6.58 (d, *J* = 9.6 Hz, 1H), 5.43 - 5.35 (m, 1H), 3.79 (s, 2H), 2.86 (q, *J* = 7.2 Hz, 4H), 2.34 (s, 3H), 2.05 - 1.99 (m, 2H), 1.56 (d, *J* = 7.2 Hz, 3H) ; LC/MS (ESI) m/z: 502.4 $[M+H]^+$ |
| 274 | 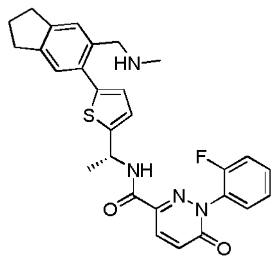 1-(2-fluorophenyl)-N-[(1R)-1-[5-[6-(methylaminomethyl)indan-5-yl]-2-thienyl]ethyl]-6-oxo-pyridazine-3-carboxamide | $^1H$ NMR (400 MHz, DMSO-$d_6$) δ = 9.05 (d, *J* = 8.8 Hz, 1H), 7.99 (d, *J* = 10.0 Hz, 1H), 7.67 (t, *J* = 7.2 Hz, 1H), 7.60 - 7.54 (m, 1H), 7.46 - 7.37 (m, 2H), 7.34 (s, 1H), 7.21 - 7.15 (m, 2H), 7.05 (d, *J* = 3.6 Hz, 1H), 6.97 (d, *J* = 3.6 Hz, 1H), 5.43 - 5.36 (m, 1H), 3.33 (s, 3H), 2.84 (q, *J* = 6.8 Hz, 4H), 2.24 (s, 3H), 2.04 - 1.97 (m, 2H), 1.58 (d, *J* = 7.2 Hz, 3H) ; LC/MS (ESI) m/z: 503.4 $[M+H]^+$ |
| 275 | 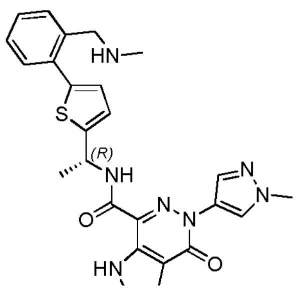 N-[(1R)-1-[5-[2-(methylaminomethyl)phenyl]-2-thienyl]ethyl]-5-(1-methylpyrazol-4-yl)-4-oxo-1H-pyrrolo[2,3-d]pyridazine-7-carboxamide | $^1H$ NMR (400 MHz, DMSO-$d_6$) δ = 9.16 (d, *J* = 8.8 Hz, 1H), 8.48 (s, 1H), 8.31 (s, 1H), 7.49 (d, *J* = 6.4 Hz,1H), 7.42 (d, *J* = 3.2 Hz, 1H), 7.32 (dq, *J* = 1.6, 7.6 Hz, 2H), 7.29 - 7.24 (m, 1H), 7.17 (d, *J* = 3.6 Hz, 1H), 7.09 (dd, *J* = 1.2, 3.6Hz, 1H), 6.78 (d, *J* = 3.2 Hz, 1H), 5.59 - 5.52 (m, 1H), 3.90 (s, 3H), 3.67 (s, 2H), 2.25 (s, 3H), 1.73 (d, *J* = 7.2 Hz, 3H) ; LC/MS (ESI) m/z: 488.4 $[M+H]^+$ |

(continued)

| No. | Structure/Name | Spectral Data |
|---|---|---|
| 276 | 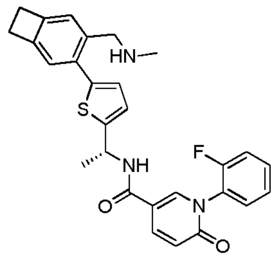 1-(2-fluorophenyl)-N-[(1R)-1-[5-[4-(methylaminomethyl)-3 -bicyclo[4.2.0]octa-1,3,5-trienyl]-2-thienyl]ethyl]-6-oxo-pyridine-3-carboxamide | $^1$H NMR (400 MHz, DMSO-$d_6$) δ = 8.73 (d, *J* = 8.0 Hz, 1H), 8.36 (d, *J* = 2.4 Hz, 1H), 8.01 (dd, *J* = 2.4, 9.6 Hz, 1H), 7.63 - 7.55 (m, 2H), 7.49 - 7.37 (m, 2H), 7.21 (s, 1H), 7.05 - 6.92 (m, 3H), 6.57 (d, *J* = 9.6 Hz, 1H), 5.39 (br t, *J* = 7.2 Hz, 1H), 3.62 (s, 2H), 3.14 (s, 4H), 2.24 (s, 3H), 1.55 (d, *J* = 6.8 Hz, 3H); LC/MS (ESI) m/z: 488.4 $[M+H]^+$ |
| 277 | 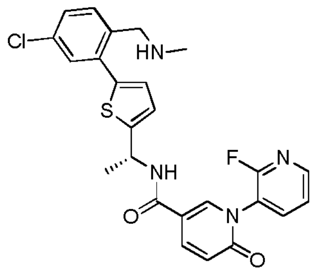 N-[(1R)-1-[5-[5-chloro-2-(methylaminomethyl) phenyl]-2-thienyl]ethyl]-1-(2-fluoro-3-pyridyl)-6-oxo-pyridine-3-carboxamide | $^1$H NMR (400 MHz, DMSO-$d_6$) δ = 9.09 (d, *J* = 8.4 Hz, 1H), 8.60 (d, *J* = 2.4 Hz, 1H), 8.36 (dd, *J* = 2.4, 8.8 Hz, 1H), 8.14 (br d, *J* = 4.8 Hz, 1H), 8.01 - 7.94 (m, 1H), 7.53 (d, *J* = 8.0 Hz, 1H), 7.47 (dd, *J* = 5.2, 7.6 Hz, 1H), 7.41-7.34 (m, 2H), 7.31 (d, *J* = 8.4 Hz, 1H), 7.20 (d, *J* = 3.6 Hz, 1H), 7.05 (d, *J* = 3.6 Hz, 1H), 5.48 - 5.41 (m, 1H), 3.64 (s, 2H), 2.24 (s, 3H), 1.61 (d, *J* = 7.2 Hz, 3H); LC/MS (ESI) m/z: 497.3 $[M+H]^+$ |
| 278 | 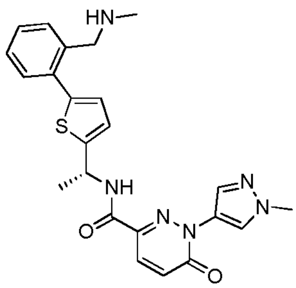 N-[(1R)-1-[5-[2-(methylaminomethyl)phenyl]-2-thienyl]ethyl]-1-(1-methylpyrazol-4-yl)-6-oxo-pyridazine-3-carboxamide | $^1$H NMR (400 MHz, DMSO-$d_6$) δ = 9.09 (d, *J* = 8.8 Hz, 1H), 8.55 (s, 1H), 8.36 (s, 1H), 7.93 (d, *J* = 9.6 Hz, 1H), 7.52 - 7.48 (m, 1H), 7.36 - 7.24 (m, 3H), 7.19 - 7.15 (m, 2H), 7.05 (dd, *J* = 0.8, 3.6 Hz, 1H), 5.48 (quin, *J* = 7.2 Hz, 1H), 3.90 (s, 3H), 3.66 (s, 2H), 2.25 (s, 3H), 1.68 (d, *J* = 6.8 Hz, 3H) ; LC/MS (ESI) m/z: 449.3 $[M+H]^+$ |
| 279 | 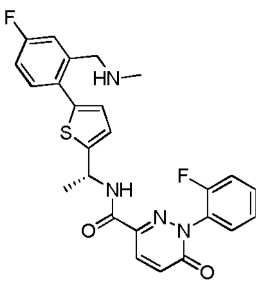 N-[(1R)-1-[5-[4-fluoro-2-(methylaminomethyl) phenyl]-2-thienyl]ethyl]-1-(2-fluorophenyl)-6-oxo-pyridazine-3-carboxamide | $^1$H NMR (400 MHz, DMSO-$d_6$) δ = 9.07 (d, *J* = 8.4 Hz, 1H), 7.99 (d, *J* = 9.6 Hz, 1H), 7.67 (dt, *J* = 1.6, 7.6 Hz, 1H), 7.61 - 7.54 (m, 1H), 7.48 - 7.32 (m, 4H), 7.19 (d, *J* = 9.6 Hz, 1H), 7.10 (dt, *J* = 2.8, 8.4 Hz, 1H), 7.04 (d, *J* = 3.6 Hz, 1H), 7.01 - 6.98 (m, 1H), 5.40 (quin, *J* = 7.2 Hz, 1H), 3.64 (s, 2H), 2.23 (s, 3H), 1.59 (d, *J* = 6.8 Hz, 3H) ; LC/MS (ESI) m/z: 481.4 $[M+H]^+$ |

(continued)

| No. | Structure/Name | Spectral Data |
|---|---|---|
| 280 | 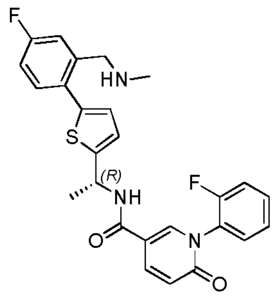 N-[(1R)-1-[5-[4-fluoro-2-(methylaminomethyl) phenyl]-2-thienyl]ethyl]-1-(2-fluorophenyl)-6-oxo-pyridine-3-carboxamide | $^1H$ NMR (400 MHz, DMSO-$d_6$) δ = 8.74 (d, *J* = 8.0 Hz, 1H), 8.36 (d, *J* = 2.4 Hz, 1H), 8.01 (dd, *J* = 2.8, 9.6 Hz, 1H), 7.65 - 7.53 (m, 2H), 7.51 - 7.43 (m, 1H), 7.42 - 7.32 (m, 3H), 7.12 (dt, *J* = 2.8, 8.4 Hz, 1H), 7.05 (d, *J* = 3.6 Hz, 1H), 7.01 (d, *J* = 3.6 Hz, 1H), 6.57 (d, *J* = 9.6 Hz, 1H), 5.46 - 5.34 (m, 1H), 3.69 (s, 2H), 2.26 (s, 3H), 1.56 (d, *J* = 6.8 Hz, 3H) ; LC/MS (ESI) m/z: 480.3 $[M+H]^+$ |
| 281 | 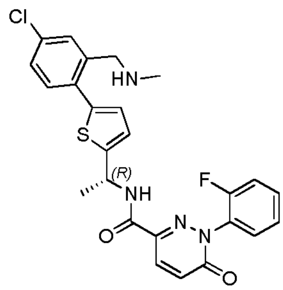 N-[(1R)-1-[5-[4-chloro-2-(methylaminomethyl) phenyl]-2-thienyl]ethyl]-1-(2-fluorophenyl)-6-oxo-pyridazine-3-carboxamide | $^1H$ NMR (400 MHz, DMSO-$d_6$) δ = 9.08 (d, *J* = 8.4 Hz, 1H), 7.99 (d, *J* = 9.6 Hz, 1H), 7.67 (dt, *J* = 1.6, 7.6 Hz, 1H), 7.61 - 7.51 (m, 2H), 7.47 - 7.37 (m, 2H), 7.37 - 7.30 (m, 2H), 7.20 (d, *J* = 10.0 Hz, 1H), 7.11 (d, *J* = 3.6 Hz, 1H), 7.01 (dd, *J* = 0.8, 3.6 Hz, 1H), 5.40 (quin, *J* = 7.2 Hz, 1H), 3.64 (s, 2H), 2.23 (s, 3H), 1.59 (d, *J* = 6.8 Hz, 3H) ; LC/MS (ESI) m/z: 497.3 $[M+H]^+$ |
| 282 | 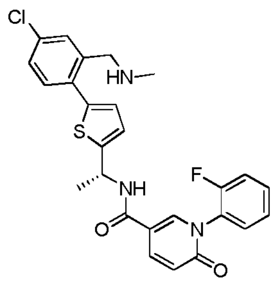 N-[(1R)-1-[5-[4-chloro-2-(methylaminomethyl) phenyl]-2-thienyl]ethyl]-1-(2-fluorophenyl)-6-oxo-pyridine-3-carboxamide | $^1H$ NMR (400 MHz, DMSO-$d_6$) δ = 8.75 (br d, *J* = 7.6 Hz, 1H), 8.36 (s, 1H), 8.00 (dd, *J* = 1.6, 9.6 Hz, 1H), 7.66 - 7.52 (m, 3H), 7.50 - 7.43 (m, 1H), 7.42 - 7.31 (m, 3H), 7.12 (d, *J* = 3.2 Hz, 1H), 7.02 (br d, *J* = 3.6 Hz, 1H), 6.57 (d, *J* = 9.6 Hz, 1H), 5.46 - 5.33 (m, 1H), 3.69 (s, 2H), 2.26 (s, 3H), 1.56 (br d, *J* = 6.8 Hz, 3H) ; LC/MS (ESI) m/z: 4 96.3 $[M+H]^+$ |
| 283 | 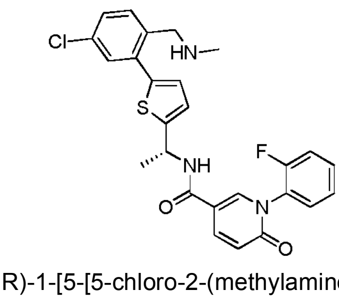 N-[(1R)-1-[5-[5-chloro-2-(methylaminomethyl) phenyl]-2-thienyl]ethyl]-1-(2-fluorophenyl)-6-oxo-pyridine-3-carboxamide | $^1H$ NMR (400 MHz, DMSO-$d_6$) δ = 8.75 (d, J = 8.4 Hz, 1H), 8.37 (d, J = 2.4 Hz, 1H), 8.18 (s, 1H), 8.01 (dd, J = 2.8, 9.6 Hz, 1H), 7.64 - 7.52 (m, 3H), 7.50 - 7.35 (m, 4H), 7.18 (d, J = 3.6 Hz, 1H), 7.04 (dd, J = 0.8, 3.6 Hz, 1H), 6.58 (d, J = 9.6 Hz, 1H), 5.41 (t, J = 7.2 Hz, 1H), 3.70 (s, 2H), 2.27 (s, 3H), 1.57 (d, J = 7.2 Hz, 3H) ; LC/MS (ESI) m/z: 496. 3 $[M+H]^+$ |

(continued)

| No. | Structure/Name | Spectral Data |
|---|---|---|
| 284 | 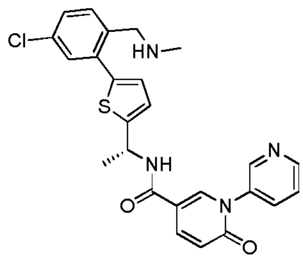 N-[(1R)-1-[5-[5-chloro-2-(methylaminomethyl) phenyl]-2-thienyl]ethyl]-6-oxo-1-(3-pyridyl)pyridine-3-carboxamide | $^1$H NMR (400 MHz, DMSO-$d_6$) δ = 8.77 (d, J = 8.2 Hz, 1H), 8.72 (d, J = 2.0 Hz, 1H), 8.70 - 8.65 (m, 1H), 8.41 (d, J = 2.0 Hz, 1H), 8.00 (dd, J = 2.0, 9.2 Hz, 2H), 7.61 (dd, J = 4.8, 8.4 Hz, 1H), 7.53 (d, J = 8.4 Hz, 1H), 7.42 - 7.34 (m, 2H), 7.21 - 7.17 (m, 1H), 7.06 - 7.02 (m, 1H), 6.58 (d, J = 10.0 Hz, 1H), 5.41 (br t, J = 7.2 Hz, 1H), 3.62 (s, 2H), 2.23 (s, 3H), 1.57 (d, J = 6.8 Hz, 3H) ; LC/MS (ESI) m/z: 496.3 [M+H]$^+$ |
| 285 | 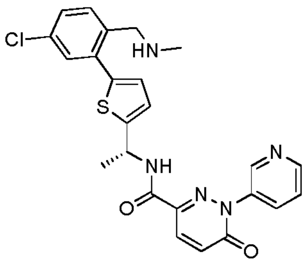 N-[(1R)-1-[5-[5-chloro-2-(methylaminomethyl) phenyl]-2-thienyl]ethyl]-6-oxo-1-(3-pyridyl) pyridazine-3-carboxamide | $^1$H NMR (400 MHz, DMSO-$d_6$) δ = 9.13 (d, J = 8.6 Hz, 1H), 8.95 (d, J = 2.0 Hz, 1H), 8.64 (dd, J = 1.6, 4.8 Hz, 1H), 8.22 - 8.13 (m, 1H), 7.96 (d, J = 10.0 Hz, 1H), 7.63 - 7.55 (m, 1H), 7.53 (d, J = 8.4 Hz, 1H), 7.42 - 7.31 (m, 2H), 7.23 - 7.15 (m, 2H), 7.04 (dd, J = 1.2, 3.6 Hz, 1H), 5.49 - 5.37 (m, 1H), 3.62 (s, 2H), 2.23 (s, 3H), 1.62 (d, J = 7.2 Hz, 3H) ; LC/MS (ESI) m/z: 480.2 [M+H]$^+$ |
| 286 | 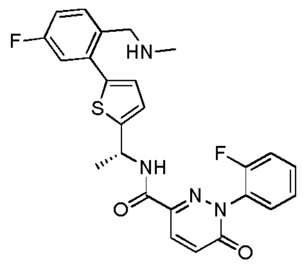 N-[(1R)-1-[5-[5-fluoro-2-(methylaminomethyl) phenyl]-2-thienyl]ethyl]-1-(2-fluorophenyl)-6-oxo-pyridazine-3-carboxamide | $^1$H NMR (400 MHz, Methanol-$d_4$) δ = 8.09 (d, J = 10.0 Hz, 1H), 7.62 - 7.51 (m, 2H), 7.50 - 7.43 (m, 1H), 7.40 - 7.29 (m, 2H), 7.19 (d, J = 10.0 Hz, 1H), 7.14 - 7.06 (m, 2H), 7.04 (dd, J = 0.8, 3.6 Hz, 1H), 6.99 (d, J = 3.6 Hz, 1H), 5.50 (q, J = 6.8 Hz, 1H), 3.78 (s, 2H), 2.30 (s, 3H), 1.67 (d, J = 7.2 Hz, 3H) ; LC/MS (ESI) m/z: 481.3 [M+H]$^+$ |
| 287 | 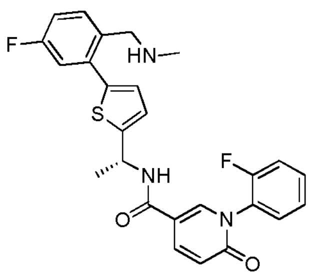 N-[(1R)-1-[5-[5-fluoro-2-(methylaminomethyl) phenyl]-2-thienyl]ethyl]-1-(2-fluorophenyl)-6-oxo-pyridine-3-carboxamide | $^1$H NMR (400 MHz, DMSO-$d_6$) δ = 8.75 (d, J = 8.0 Hz, 1H), 8.37 (d, J = 2.8 Hz, 1H), 8.01 (dd, J = 2.8, 9.6 Hz, 1H), 7.66 - 7.34 (m, 5H), 7.26 - 7.11 (m, 3H), 7.03 (d, J = 3.6 Hz, 1H), 6.57 (d, J = 9.6 Hz, 1H), 5.40 (t, J = 7.2 Hz, 1H), 3.61 (s, 2H), 2.24 (s, 3H), 1.56 (d, J = 7.2 Hz, 3H) ; LC/MS (ESI) m/z: 480.4 [M+H]$^+$ |

(continued)

| No. | Structure/Name | Spectral Data |
|---|---|---|
| 288 | 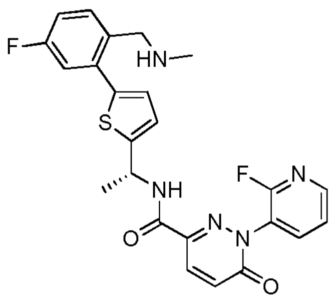 N-[(1R)-1-[5-[5-fluoro-2-(methylaminomethyl) phenyl]-2-thienyl]ethyl]-1-(2-fluoro-3-pyridyl)-6-oxo-pyridazine-3-carboxamide | $^1$H NMR (400 MHz, DMSO-$d_6$) δ = 9.12 (d, J = 8.8 Hz, 1H), 8.40 (d, J = 4.8 Hz, 1H), 8.35 - 8.28 (m, 1H), 8.00 (d, J = 10.0 Hz, 1H), 7.62 (dd, J = 5.6, 7.2 Hz, 1H), 7.52 (br t, J = 7.6 Hz, 1H), 7.26 - 7.07 (m, 4H), 7.02 (d, J = 3.6 Hz, 1H), 5.41 (quin, J = 7.6 Hz, 1H), 3.60 (s, 2H), 2.23 (s, 3H), 1.59 (d, J = 7.2 Hz, 3H) ; LC/MS (ESI) m/z: 482. 4 [M+H]$^+$ |
| 289 | 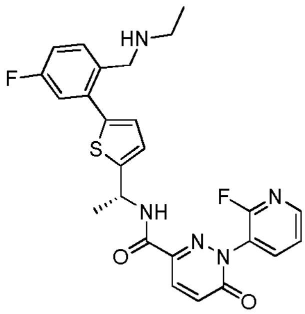 N-[(1R)-1-[5-[2-(ethylaminomethyl)-5-fluorophenyl]-2-thienyl]ethyl]-1-(2-fluoro-3-pyridyl)-6-oxo-pyridazine-3-carboxamide | $^1$H NMR (400 MHz, Methanol-$d_4$) δ = 8.37 (br d, J = 4.8 Hz, 1H), 8.28 - 8.15 (m, 1H), 8.11 (d, J = 10.0 Hz, 1H), 7.58 - 7.44 (m, 2H), 7.22 (d, J = 10.0 Hz, 1H), 7.15 - 7.04 (m, 3H), 7.01 (d, J = 3.6 Hz, 1H), 5.52 (q, J = 7.2 Hz, 1H), 3.83 (s, 2H), 2.56 (q, J = 7.2 Hz, 2H), 1.69 (d, J = 7.2 Hz, 3H), 1.03 (t, J = 7.2 Hz, 3H) ; LC/MS (ESI) m/z: 496.3 [M+H]$^+$ |
| 290 | 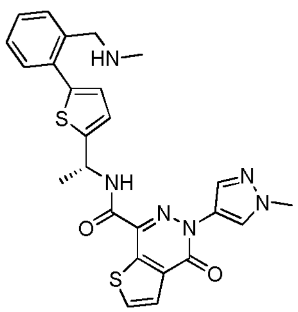 N-[(1R)-1-[5-[2-(methylaminomethyl)phenyl]-2-thienyl]ethyl]-5-(1-methylpyrazol-4-yl)-4-oxo-thieno [2,3-d]pyridazine-7-carboxamide | $^1$H NMR (400 MHz, DMSO-$d_6$) δ = 9.28 (d, *J* = 8.8 Hz, 1H), 8.57 (s, 1H), 8.41 (s, 1H), 8.22 (d, *J* = 5.6 Hz,1H), 7.73 (d, *J* = 5.2 Hz, 1H), 7.50 (d, *J* = 7.2 Hz, 1H), 7.36 - 7.26 (m, 3H), 7.17 (d, *J* = 3.6 Hz, 1H), 7.09 (dd, *J* = 0.8, 3.6 Hz, 1H), 5.59 - 5.52 (m, 1H), 3.92 (s, 3H), 3.70 (s, 2H), 2.27 (s, 3H), 1.73 (d, *J* = 7.2 Hz, 3H) ; LC/MS (ESI) m/z: 505.4 [M+H]$^+$ |
| 291 | 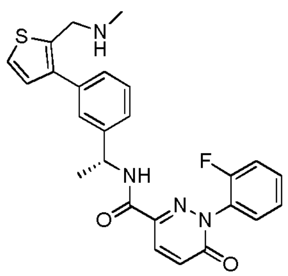 1-(2-fluoropheiryl)-N-[(1R)-1-[3-[2-(methylaminomethyl)-3-thienyl]phenyl] ethyl]-6-oxo-pyridazine-3-carboxamide | $^1$H NMR (400 MHz, DMSO-$d_6$) δ = 8.92 (d, *J* = 8.0 Hz, 1H), 7.94 (d, *J* = 9.6 Hz, 1H), 7.54-7.71 (m, 1H), 7.53-7.70 (m, 2H), 7.07-7.47 (m, 9H), 5.10-5.24 (m, 1H), 3.79 (s, 2H), 2.50 (s, 3H), 2.24 (s, 3H), 1.50 (d, *J* = 6.4 Hz, 3H); LC/MS (ESI) m/z: 463.3 [M+H]$^+$ |

(continued)

| No. | Structure/Name | Spectral Data |
|---|---|---|
| 292 | 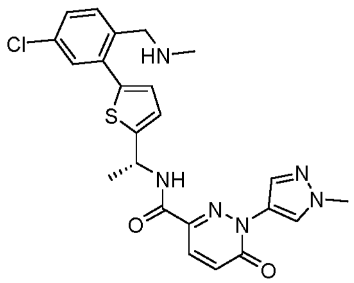 (R)-N-(1-(5-(5-chloro-2-((methylamino)methyl) phenyl)thiophen-2-yl)ethyl)-1-(1-methyl-1H-pyrazol-4-yl)-6-oxo-1,6-dihydropyridazine-3-carboxamide | $^1H$ NMR (400MHz, DMSO-$d_6$) $\delta$ = 9.11 (d, J = 8.4 Hz, 1H), 8.55 (s, 1H), 8.37 (s, 1H), 7.93 (d, J = 9.6 Hz, 1H), 7.53 (br d, J = 8.0 Hz, 1H), 7.44 - 7.31 (m, 2H), 7.26-7.13 (m, 2H), 7.07 (d, J = 3.6 Hz, 1H), 5.48 (br t, J = 7.2 Hz, 1H), 3.90 (s, 3H), 3.79 - 3.43 (m, 2H), 2.34 - 2.09 (m, 3H), 1.68 (d, J = 7.2 Hz, 3H) ; MS (ESI) m/z = 483.4 [M+H]+ |
| 293 | 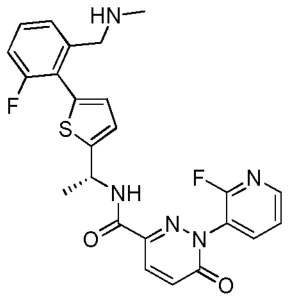 N-[(1R)-1-[5-[2-fluoro-6-(methylaminomethyl) phenyl]-2-thienyl]ethyl]-1-(2-fluoro-3-pyridyl)-6-oxo-pyridazine-3-carboxamide | $^1H$ NMR (400MHz, DMSO-$d_6$) $\delta$ = 9.14 (d, *J* = 8.8 Hz, 1H), 8.40 (d, *J* = 4.8 Hz, 1H), 8.35 - 8.25 (m, 1H), 8.00 (d, *J* = 10.0 Hz, 1H), 7.66 - 7.57 (m, 1H), 7.44 - 7.33 (m, 2H), 7.23 (d, *J* = 10.0 Hz, 1H), 7.18 - 7.12 (m, 1H), 7.06 - 7.02 (m, 1H), 7.00 (d, *J* = 3.6 Hz, 1H), 5.49 - 5.38 (m, 1H), 3.52 (s, 2H), 2.18 (s, 3H), 1.60 (d, *J* = 7.0 Hz, 3H); MS (ESI) m/z = 482.4 [M+H]+ |

**Example 294: 1-(2-fluorophenyl)-N-[(1R)-1-[3-[3-(methylaminomethyl)-2-thienyl]phenyl]ethyl]-6-oxo-pyridazine-3-carboxamide**

Step 1: 1-(2-fluorophenyl)-6-oxo-pyridazine-3-carboxylic acid

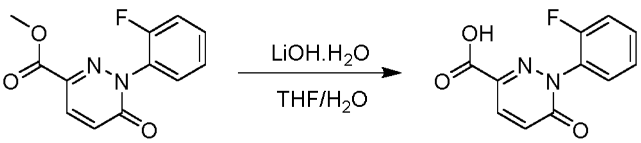


To a solution of 1-(2-fluorophenyl)-6-oxo-pyridazine-3-carboxylate (20 g, 80.58 mmol) in THF (180 mL), $LiOH\cdot H_2O$(10.14 g, 241.73 mmol) and $H_2O$ (20 mL) were added. The mixture was stirred at 20°C for 1 hour. The reaction mixture was poured into water (200 mL) and extracted with EtOAc (100 mL × 3). The organic layer was discarded, and the reaction mixture was then adjusted to pH = 3-4 with aq. 1 N HCl and extracted with EtOAc (200 mL × 3), The combined organic layer was washed with brine (30 mL × 2), dried over anhydrous $Na_2SO_4$ and concentrated under reduced pressure to give 1-(2-fluorophenyl)-6-oxo-pyridazine-3-carboxylic acid (18 g, 92.36% yield) as a white solid. $^1H$ NMR (400 MHz, DMSO-$d_6$) $\delta$ 13.79 (br s, 1H), 7.95 (d, *J* = 9.6 Hz, 1H), 7.65 - 7.53 (m, 2H), 7.46 (t, *J* = 9.2 Hz, 1H), 7.42 - 7.36 (m, 1H), 7.18 (d, *J* = 10.0 Hz, 1H); LC/MS (ESI) m/z = 235.0 [M+H]+.

Step 2: N-[(1R)-1-(3-bromophenyl)ethyl]-1-(2-fluorophenyl)-6-oxo-pyridazine-3-carboxamide

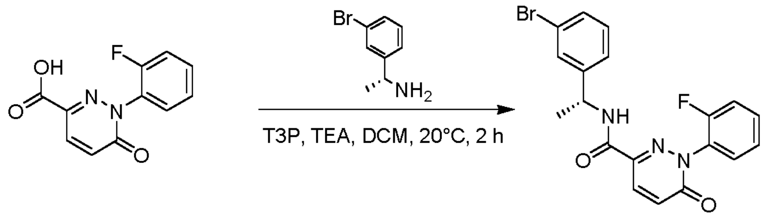


To a solution of (1R)-1-(3-bromophenyl)ethanamine (1 g, 5.00 mmol) and 1-(2-fluorophenyl)-6-oxo-pyridazine-3-carboxylic acid (1.17 g, 5.00 mmol) in DCM (15 mL), $T_3P$ (2.97 mL, 10.00 mmol) and triethylamine (2.09 mL, 14.99 mmol) were added, followed by stirring at 20 °C for 2 hours under $N_2$ atmosphere. The reaction mixture was poured into water (10 mL) and extracted with EtOAc (10 mL×3). The combined organic layer was washed with brine (30 mL), dried over anhydrous $Na_2SO_4$ and filtered under reduced pressure to obtain a crude product. The product was purified by silica gel column chromatography (42% EtOAc in PE) to give N-[(1R)-1-(3-bromophenyl)ethyl]-1-(2-fluorophenyl)-6-oxo-pyridazine-3-carboxamide (1.61 g, 77.56% yield) as a yellow solid. $^1H$ NMR (400 MHz, DMSO-$d_6$) δ ppm 0.93 (m, 1 H) 1.45 (d, *J* = 7.2 Hz, 3 H) 5.11 (quin, *J* = 7.2 Hz, 1 H) 5.76 (s, 1 H) 7.17 (d, *J* = 9.6 Hz, 1 H) 7.28 (m, 1 H) 7.42 (m, 5 H) 7.59 (m, 3 H) 7.68 (t, *J* = 7.6 Hz, 1 H) 7.94 (m, 1 H) 8.96 (d, *J* = 8.0 Hz, 1 H); MS (ESI) m/z = 416.05 $[M+H]^+$.

Step 3: 1-(2-fluorophenyl)-N-[(1R)-1-[3-(3-formyl-2-thienyl)phenyl]ethyl]-6-oxo-pyridazine-3-carboxamide

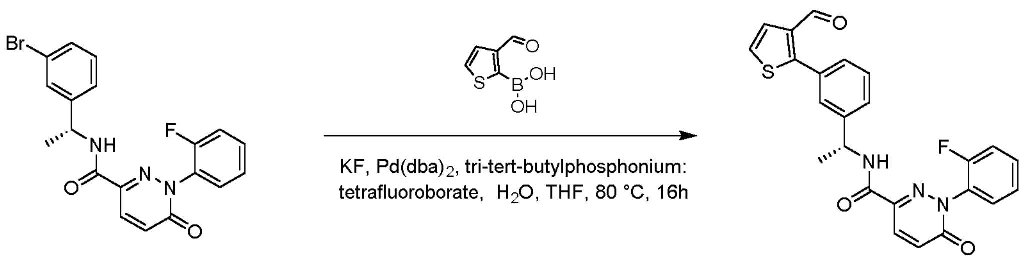


To a solution of N-[(1R)-1-(3-bromophenyl)ethyl]-1-(2-fluorophenyl)-6-oxo-pyridazine-3-carboxamide (556.00 mg, 1.34 mmol) and (3-formyl-2-thienyl)boronic acid (250 mg, 1.60 mmol) in THF (15 mL), KF (143.94 μL, 6.14 mmol), $Pd(dba)_2$ (38.40 mg, 66.79 μmol), $H_2O$ (144.38 mg, 8.01 mmol) and tri-tert-butylphosphonium;tetrafluoroborate (38.75 mg, 133.58 μmol) were added, followed by stirring at 80 °C for 16 hours under $N_2$ atmosphere. The reaction mixture was poured into water (10 mL) and extracted with EtOAc (10 mL×3). The combined organic layer was washed with brine (30 mL), dried over anhydrous $Na_2SO_4$ and concentrated under reduced pressure to obtain a crude product. The product was purified by silica gel column chromatography (37% EtOAc in PE) to give 1-(2-fluorophenyl)-N-[(1R)-1-[3-(3-formyl-2-thienyl)phenyl]ethyl]-6-oxo-pyridazine-3-carboxamide (450 mg, 70.02% yield) as a yellow solid. MS (ESI) m/z = 448.0 $[M+H]^+$.

Step 4: 1-(2-fluorophenyl)-N-[(1R)-1-[3-[3-(methylaminomethyl)-2-thienyl]phenyl]ethyl]-6-oxo-pyridazine-3-carboxamide

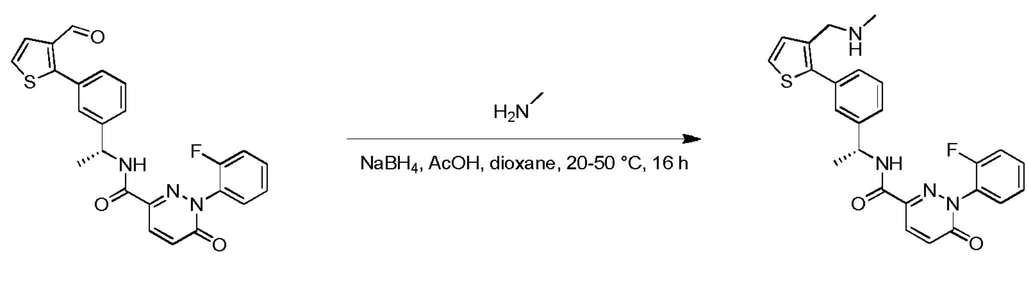


294

To a solution of 1-(2-fluorophenyl)-N-[(1R)-1-[3-(3-formyl-2-thienyl)phenyl]ethyl]-6-oxo-pyridazine-3-carboxamide (150 mg, 335.21 μmol) and methylamine hydrochloride (27.16 mg, 402.25 μmol) in dioxane (4 mL) was added

acetic acid (38.34 μL, 670.42 μmol), followed by stirring at 20 °C for 30 min. The resulting mixture was added with $NaBH_4$ (126.39 mg, 2.01 mmol) and then stirred at 50 °C for 16 hours. The reaction mixture was poured into water (30 mL) and extracted with EtOAc (30 mL×3). The organic layer was washed with brine (50 mL), dried over anhydrous $Na_2SO_4$ and filtered under reduced pressure to obtain a crude product. The product was purified by prep-HPLC (column: C18-6 100*30mm*5μm; mobile phase: [water(FA)-ACN];B%: 18%-48%,15min). $CH_3CN$ was removed under reduced pressure, and the remaining solvent was removed by lyophilization to give the compound of Example 294 (7.4 mg, 4.31% yield) as a white solid. $^1H$ NMR (400 MHz, DMSO-$d_6$) δ ppm 1.50 (d, *J* = 7.2 Hz, 3 H) 2.29 (s, 3 H) 3.71 (s, 2 H) 5.18 (quin, *J* = 7.2 Hz, 1 H) 7.18 (d, *J* = 10.0 Hz, 1 H) 7.22 (d, *J* = 5.2 Hz, 1 H) 7.40 (m, 5 H) 7.52 (m, 2 H) 7.58 (m, 1 H) 7.67 (td, *J* = 7.6, 1.56 Hz, 1 H) 7.94 (d, *J* = 10.0 Hz, 1 H) 8.26 (br s, 1 H) 8.97 (d, *J* = 8.4 Hz, 1 H); MS (ESI) m/z = 463.3 $[M+H]^+$.

**Example 295: N-[(1R)-1-[5-[5-chloro-2-(methylaminomethyl)phenyl]-2-thienyl]ethyl]-1-(2-fluorophenyl)-6-oxo-pyridazine-3-carboxamide**

Step 1: Synthesis of N-[(1R)-1-(5-bromo-2-thienyl)ethyl]-1-(2-fluorophenyl)-6-oxo-pyridazine-3-carboxamide

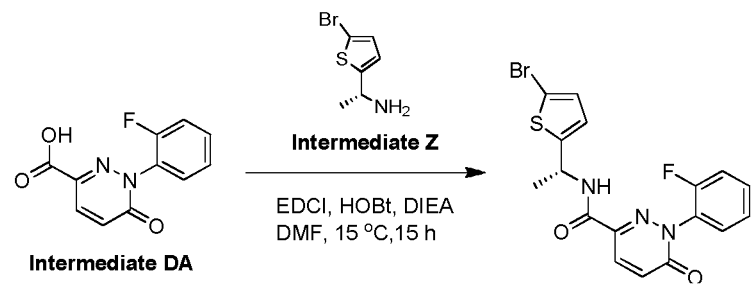


To a solution of Intermediate DA (5 g, 21.35 mmol) and Intermediate Z (5.18 g, 21.35 mmol, HCl) in DMF (70 mL), EDCI (6.14 g, 32.03 mmol), DIEA (8.28 g, 64.05 mmol, 11.16 mL) and HOBt (4.33 g, 32.03 mmol) were added. The mixture was degassed and purged with $N_2$ for 3 times, and then stirred at 15 °C for 15 hours under $N_2$ atmosphere. The reaction mixture was poured into water (200 mL) and extracted with EtOAc (50 mL × 10). The combined organic layer was washed with brine (50 mL × 4), dried over anhydrous $Na_2SO_4$ and concentrated under reduced pressure to obtain a crude product. The product was purified by silica gel column chromatography (25% EtOAc in PE) to give N-[(1R)-1-(5-bromo-2-thienyl)ethyl]-1-(2-fluorophenyl)-6-oxo-pyridazine-3-carboxamide (9.11 g, 92.99% yield) as an off-white solid. $^1H$ NMR (400 MHz, DMSO-$d_6$) δ = 9.04 (br d, *J* = 8.4 Hz, 1H), 7.97 (d, *J* = 9.6 Hz, 1H), 7.66 (br t, *J* = 7.6 Hz, 1H), 7.62 - 7.52 (m, 1H), 7.50 - 7.34 (m, 2H), 7.19 (d, *J* = 10.0 Hz, 1H), 7.05 (d, *J* = 3.6 Hz, 1H), 6.87 - 6.76 (m, 1H), 5.29 (quin, *J* = 7.2 Hz, 1H), 1.53 (d, *J* = 7.2 Hz, 3H). LC/MS (ESI) m/z = 424.0 $[M+H]^+$.

Step 2: Synthesis of N-[(1R)-1-[5-(5-chloro-2-formyl-phenyl)-2-thienyl]ethyl]-1-(2-fluorophenyl)-6-oxo-pyridazine-3-carboxamide

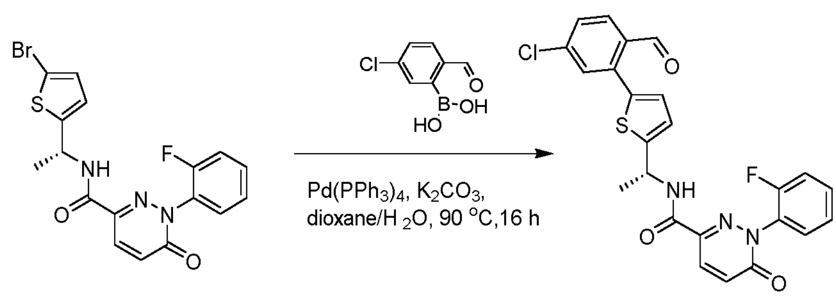


A mixture of N-[(1R)-1-(5-bromo-2-thienyl)ethyl]-1-(2-fluorophenyl)-6-oxo-pyridazine-3-carboxamide (9 g, 21.31 mmol), (5-chloro-2-formyl-phenyl)boronic acid (4.95 g, 26.85 mmol), $K_2CO_3$ (7.36 g, 53.28 mmol), $Pd(PPh_3)_4$ (2.46 g, 2.13 mmol) in dioxane (90 mL) and $H_2O$ (5 mL) was degassed and purged with $N_2$ for 3 times, and then stirred at 90 °C for 16 hours under $N_2$ atmosphere. The reaction mixture was poured into water (150 mL) and extracted with

EtOAc (100 mL × 3). The combined organic layer was washed with $H_2O$ (100 mL), dried over $Na_2SO_4$, filtered and concentrated under reduced pressure to obtain a residue. The residue was purified by silica gel column chromatography (50% EtOAc in PE) to give N-[(1R)-1-[5-(5-chloro-2-formylphenyl)-2-thienyl]ethyl]-1-(2-fluorophenyl)-6-oxo-pyridazine-3-carboxamide (5.3 g, 47.00% yield) as a light yellow solid. $^1H$ NMR (400 MHz, DMSO-$d_6$) $\delta$ = 10.04 (s, 1H), 9.11 (d, $J$ = 8.4 Hz, 1H), 7.99 (d, $J$ = 9.6 Hz, 1H), 7.87 (d, $J$ = 8.8 Hz, 1H), 7.66 (dt, $J$ = 1.6, 7.6 Hz, 1H), 7.61 (dd, $J$ = 2.4, 4.4 Hz, 2H), 7.59 - 7.54 (m, 1H), 7.46 - 7.38 (m, 2H), 7.22 - 7.17 (m, 2H), 7.11 (d, $J$ = 3.2 Hz, 1H), 5.43 (quin, $J$ = 7.2 Hz, 1H), 1.61 (d, $J$ = 7.2 Hz, 3H); MS (EI) m/z: 482.1 $[M+H]^+$.

Step 3: Synthesis of N-[(1R)-1-[5-[5-chloro-2-(methylaminomethyl)phenyl]-2-thienyl]ethyl]-1-(2-fluorophenyl)-6-oxo-pyridazine-3-carboxamide

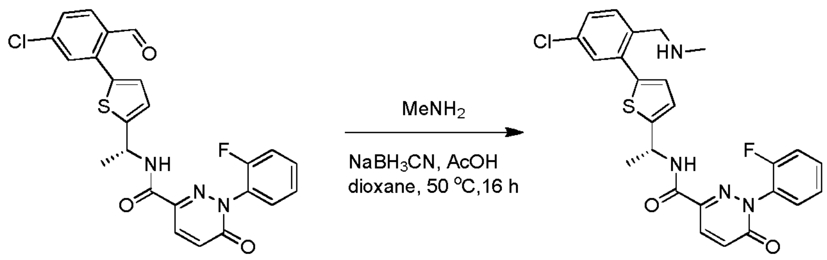


**295**

To a solution of N-[(1R)-1-[5-(5-chloro-2-formyl-phenyl)-2-thienyl]ethyl]-1-(2-fluorophenyl)-6-oxo-pyridazine-3-carboxamide (5.3 g, 11.00 mmol) and AcOH (1.32 g, 22.00 mmol) in dioxane (55 mL) was added $MeNH_2$ (46.12 g, 445.50 mmol, 30% purity), followed by stirring at 15 °C for 1 hour under $N_2$ atmosphere. The reaction mixture was added with $NaBH_3CN$ (2.07 g, 32.99 mmol) and stirred at 50 °C for 16 hours under $N_2$ atmosphere. The reaction mixture was poured into distilled water (100 mL) and extracted with EtOAc (100 mL × 3). The combined organic layer was washed with brine (50 mL × 2), dried over anhydrous $Na_2SO_4$ and concentrated under reduced pressure to obtain a crude product. The product was purified by silica gel column chromatography (2% MeOH in DCM) to give the compound of Example 295 (2.05 g, 36.22% yield) as a yellow solid. $^1H$ NMR (400 MHz, DMSO-$d_6$) $\delta$ = 9.08 (d, $J$ = 8.4 Hz, 1H), 7.99 (d, $J$ = 9.6 Hz, 1H), 7.67 (t, $J$ = 7.6 Hz, 1H), 7.61 - 7.51 (m, 2H), 7.47 - 7.33 (m, 4H), 7.22 - 7.14 (m, 2H), 7.02 (d, $J$ = 3.6 Hz, 1H), 5.45 - 5.36 (m, 1H), 3.64 (s, 2H), 2.24 (s, 3H), 1.59 (d, $J$ = 7.2 Hz, 3H); MS (EI) m/z: 497.1 $[M+H]^+$.

**Example 296: N-[(1R)-1-[5-[5-fluoro-2-(methylaminomethyl)phenyl]-2-thienyl]ethyl]-1-(1-methylpyrazol-4-yl)-6-oxo-pyridazine-3-carboxamide**

Step 1: methyl 1-(1-methylpyrazol-4-yl)-6-oxo-pyridazine-3-carboxylate

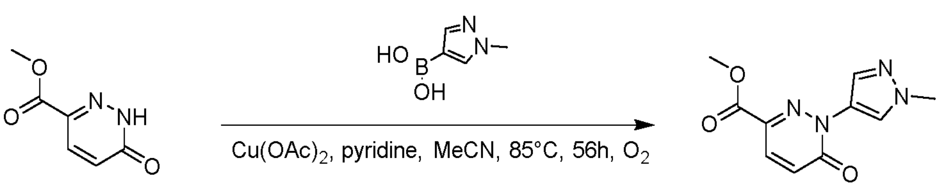


To a solution of methyl 6-oxo-1H-pyridazine-3-carboxylate (2 g, 12.98 mmol) and (1-methylpyrazol-4-yl)boronic acid (1.96 g, 15.57 mmol) in acetonitrile (100 mL), $Cu(OAc)_2$ (707.10 mg, 3.89 mmol) and pyridine (3.08 g, 38.93 mmol) were added, followed by stirring at 85 °C for 56 hours under $O_2$ atmosphere. The mixture was filtrated, the filtrate was concentrated under reduced pressure and purified by silica gel column chromatography (10% EA in DCM) to give methyl 1-(1-methylpyrazol-4-yl)-6-oxo-pyridazine-3-carboxylate (3.08 g, 39.68% yield) as a white solid. MS (ESI) m/z = 235.0 $[M+H]^+$.

Step 2: 1-(1-methylpyrazol-4-yl)-6-oxo-pyridazine-3-carboxylic acid

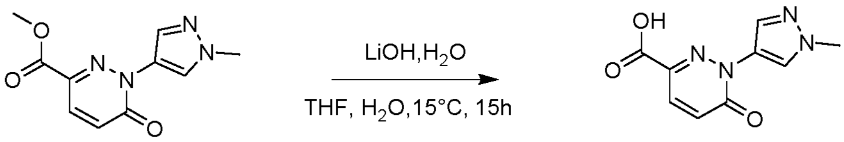


To a solution of methyl 1-(1-methylpyrazol-4-yl)-6-oxo-pyridazine-3-carboxylate (1.5 g, 6.40 mmol) in THF (20 mL) and $H_2O$ (7 mL) was added LiOH.$H_2O$ (806.27 mg, 19.21 mmol), followed by stirring at 15 °C for 15 hours. The reaction mixture was adjusted to pH = 3-4 with aq. 1 N HCl and extracted with EtOAc (50 mL × 3). The organic layer was washed with water (30 mL) and brine (30 mL), dried over anhydrous $Na_2SO_4$, and filtered under reduced pressure to give 1-(1-methylpyrazol-4-yl)-6-oxo-pyridazine-3-carboxylic acid (840 mg, 55.99% yield) as a white solid. MS (ESI) m/z = 220.9 $[M+H]^+$.

Step 3: N-[(1R)-1-(5-bromo-2-thienyl)ethyl]-1-(1-methylpyrazol-4-yl)-6-oxo-pyridazine-3-carboxylic acid

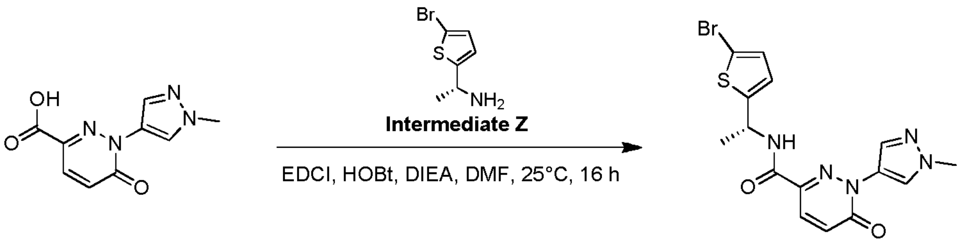


A solution of 1-(1-methylpyrazol-4-yl)-6-oxo-pyridazine-3-carboxylic acid (300 mg, 1.36 mmol), Intermediate E (330.49 mg, 1.36 mmol, HCl), EDCI (522.39 mg, 2.72 mmol), HOBt (368.21 mg, 2.72 mmol) and DIEA (711.97 uL, 4.09 mmol) in DMF (6 mL) was degassed and purged with $N_2$ for 3 times, and then stirred at 25 °C for 3 hours under $N_2$ atmosphere. The reaction mixture was poured into water (30 mL) and extracted with EtOAc (30 mL×3). The combined organic layer was washed with brine (50 mL), dried over anhydrous $Na_2SO_4$ and concentrated under reduced pressure to obtain a crude product. The product was purified by silica gel column chromatography (4 % MeOH in DCM) to give N-[(1R)-1-(5-bromo-2-thienyl)ethyl]-1-(1-methylpyrazol-4-yl)-6-oxo-pyridazine-3-carboxylic acid (210 mg, 30.53% yield) as yellow oil. MS (ESI) m/z = 407.8 $[M+H]^+$.

Step 4: N-[(1R)-1-[5-(5-fluoro-2-formyl-phenyl)-2-thienyl]ethyl]-1-(1-methylpyrazol-4-yl)-6-oxo-pyridazine-3-carboxamide

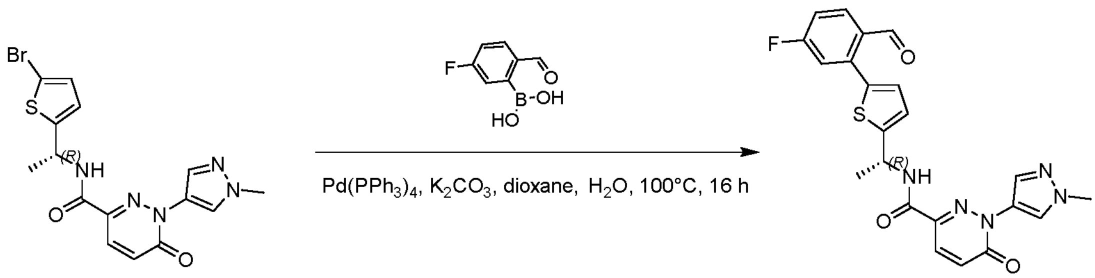


A solution of N-[(1R)-1-(5-bromo-2-thienyl)ethyl]-1-(1-methylpyrazol-4-yl)-6-oxo-pyridazine-3-carboxylic acid (60 mg, 146.96 μmol), (5-fluoro-2-formyl-phenyl)boronic acid (37.02 mg, 220.44 μmol), $K_2CO_3$ (50.78 mg, 367.40 μmol) and $Pd(PPh_3)_4$ (16.98 mg, 14.70 μmol) in dioxane (4 mL) and $H_2O$ (0.4 mL) was degassed and purged with $N_2$ for 3 times, and then stirred at 100 °C for 16 hours under $N_2$ atmosphere. The mixture was filtered under reduced pressure to obtain a crude product. The product was purified by silica gel column chromatography (48% EtOAc in PE) to give N-[(1R)-1-[5-(5-fluoro-2-formyl-phenyl)-2-thienyl]ethyl]-1-(1-methylpyrazol-4-yl)-6-oxo-pyridazine-3-carboxamide (75 mg, 68.95% yield, 61% purity) as yellow oil. MS (ESI) m/z = 452.0 $[M+H]^+$.

Step 5: Synthesis of N-[(1R)-1-[5-[5-fluoro-2-(methylaminomethyl)phenyl]-2-thienyl]ethyl]-1-(1-methylpyrazol-4-yl)-6-oxo-pyridazine-3-carboxamide

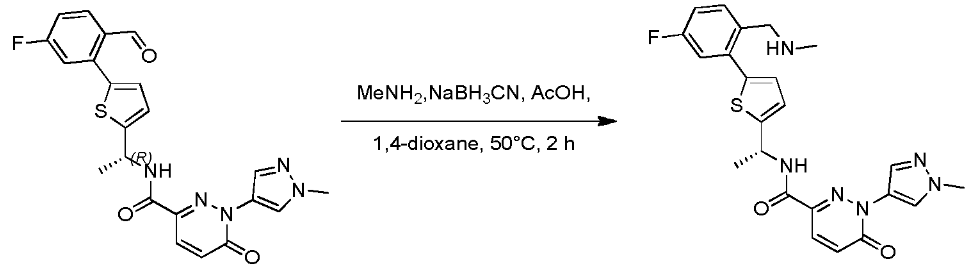


**296**

A solution of N-[(1R)-1-[5-(5-fluoro-2-formyl-phenyl)-2-thienyl]ethyl]-1-(1-methylpyrazol-4-yl)-6-oxo-pyridazine-3-carboxamide (75 mg, 166.12 μmol), methylamine (1.230 g, 11.88 mmol, 30% purity), AcOH (19.95 mg, 332.25 μmol) and $NaBH_3CN$ (31.32 mg, 498.37 μmol) in dioxane (3 mL) was degassed and purged with $N_2$ for 3 times, and then the mixture was stirred at 50 °C for 2 hours under $N_2$ atmosphere. The reaction mixture was poured into water (30 mL) and extracted with EtOAc (30 mL×3). The organic layer was washed with brine (50 mL), dried over anhydrous $Na_2SO_4$ and filtered under reduced pressure to obtain a crude product. The product was purified by prep-HPLC (column: Welch Xtimate C18 150*30mm*5μm; mobile phase: [water($NH_3H_2O$+$NH_4HCO_3$)-ACN];B%: 20%-60%,36min). Most of $CH_3CN$ was removed under reduced pressure, and the remaining solvent was removed by lyophilization to give the compound of Example 296 (5.3 mg, 6.81% yield) as a white solid. $^1H$ NMR (400 MHz, DMSO-$d_6$) δ ppm 9.10 (d, J = 8.8 Hz, 1H), 8.55 (s, 1H), 8.36 (s, 1H), 7.92 (d, J = 9.6 Hz, 1H), 7.52 (dd, J = 6.4, 9.6 Hz, 1H), 7.23 (d, J = 3.6 Hz, 1H), 7.19 - 7.12 (m, 3H), 7.07 (dd, J = 0.8, 3.6 Hz, 1H), 5.54 - 5.40 (m, 1H), 3.90 (s, 3H), 3.62 (s, 2H), 2.24 (s, 3H), 1.68 (d, J = 7.2 Hz, 3H); MS (ESI) m/z = 467.1 $[M+H]^+$.

**Example 297: 1-[3-(dimethylcarbamoyl)-2-fluoro-phenyl]-N-[(1R)-1-[5-[5-fluoro-2-(methylaminomethyl)phenyl] -2-thienyl] ethyl] -6-oxo-pyridazine-3-carboxamide**

Step 1: N-[(1R)-1-(5-bromo-2-thienyl)ethyl]-1-[3-(dimethylcarbamoyl)-2-fluorophenyl]-6-oxo-pyridazine-3-carboxamide

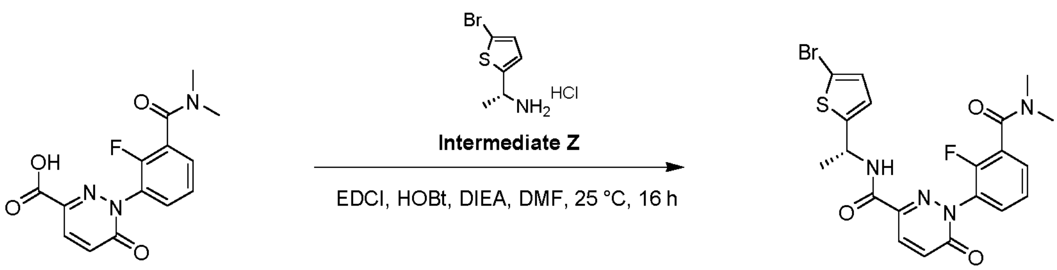


A solution of 1-[3-(dimethylcarbamoyl)-2-fluoro-phenyl]-6-oxo-pyridazine-3-carboxylic acid (30 mg, 98.28 μmol) which was obtained by ester hydrolysis of Intermediate AC, Intermediate Z (23.84 mg, 98.28 μmol), HOBt (26.56 mg, 196.55 μmol), EDCI (37.68 mg, 196.55 μmol) and DIEA (51.35 μL , 294.83 μmol) in DMF (2 mL) was degassed and purged with $N_2$ for 3 times, and stirred at 25 °C for 16 hours under $N_2$ atmosphere. The reaction mixture was poured into water (30 mL) and extracted with EtOAc (30 mL×3). The organic layer was washed with brine (50 mL), dried over anhydrous $Na_2SO_4$ and concentrated under reduced pressure to obtain a crude product. The product was purified by silica gel column chromatography (4% MeOH in DCM) to give N-[(1R)-1-(5-bromo-2-thienyl)ethyl]-1-[3-(dimethylcarbamoyl)-2-fluoro-phenyl]-6-oxo-pyridazine-3-carboxamide (42 mg, 66.70% yield) as yellow oil. MS (ESI) m/z = 515.0 $[M+Na]^+$, 494.8 $[M+H]^+$.

Step 2: 1-[3-(dimethylcarbamoyl)-2-fluoro-phenyl]-N-[1R)-1-[5-(5-fluoro-2-formylphenyl)-2-thienyl]ethyl]-6-oxo-pyridazine-3-carboxamide

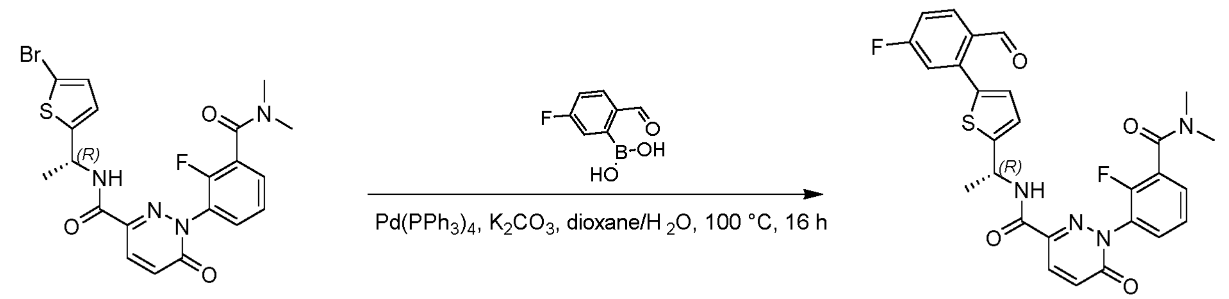


A solution of N-[(1R)-1-(5-bromo-2-thienyl)ethyl]-1-[3-(dimethylcarbamoyl)-2-fluorophenyl]-6-oxo-pyridazine-3-carboxamide (42 mg, 85.13 μmol), (5-fluoro-2-formylphenyl)boronic acid (17.16 mg, 102.16 μmol), $Pd(PPh_3)_4$ (9.84 mg, 8.51 μmol) and $K_2CO_3$ (29.41 mg, 212.83 μmol) in dioxane (3 mL) and $H_2O$ (0.3 mL) was degassed and purged with $N_2$ for 3 times, and then stirred at 100 °C for 16 hours under $N_2$ atmosphere. The reaction mixture was filtered under reduced pressure to obtain a crude product. The product was purified by silica gel column chromatography to give 1-[3-(dimethylcarbamoyl)-2-fluoro-phenyl]-N-[(1R)-1-[5-(5-fluoro-2-formyl-phenyl)-2-thienyl]ethyl]-6-oxo-pyridazine-3-carboxamide (50 mg, 86.18% yield) as yellow oil. MS (ESI) m/z = 537.1 $[M+H]^+$).

Step 3: 1-[3-(dimethylcarbamoyl)-2-fluoro-phenyl]-N-[1R)-1-[5-[5-fluoro-2-(methylaminomethyl)phenyl]-2-thienyl]ethyl]-6-oxo-pyridazine-3-carboxamide

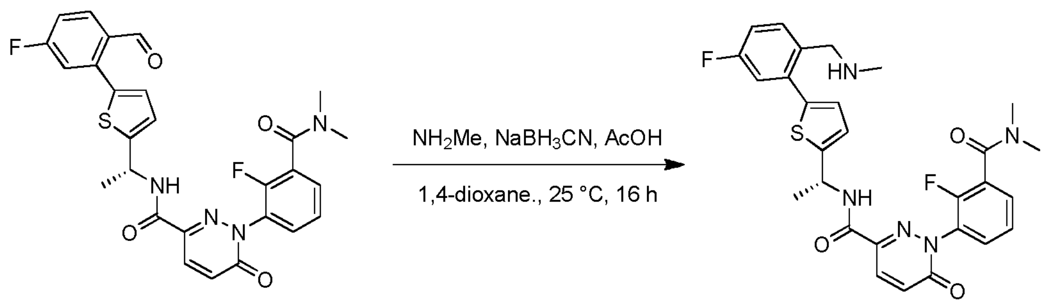


**297**

A solution of 1-[3-(dimethylcarbamoyl)-2-fluoro-phenyl]-N-[(1R)-1-[5-(5-fluoro-2-formyl-phenyl)-2-thienyl]ethyl]-6-oxo-pyridazine-3-carboxamide (50 mg, 93.19 μmol), methylamine (330 mg, 3.19 mmol, 30% purity), AcOH (11.19 mg, 186.38 μmol) and $NaBH_3CN$ (17.57 mg, 279.56 μmol) in dioxane (2 mL) was degassed and purged with $N_2$ for 3 times, and then stirred at 25 °C for 16 hours under $N_2$ atmosphere. The reaction mixture was poured into water (20 mL) and extracted with EtOAc (30 mL×3). The organic layer was dried over anhydrous $Na_2SO_4$ and concentrated under reduced pressure to obtain a crude product. The product was purified by silica gel column chromatography (4% MeOH in DCM) to obtain a yellow oil residue. The residue was purified by prep-HPLC (column: Welch Xtimate C18 150*30mm*5μm; mobile phase: [water($NH_3H_2O$+$NH_4HCO_3$)-ACN];B%: 22%-62%,36min). Most of $CH_3CN$ was removed under reduced pressure, and the remaining solvent was removed by lyophilization to give the compound of Example 297 (1.9 mg, 3.66% yield) as a white solid. $^1H$ NMR (400MHz, DMSO-$d_6$) δ ppm 9.12 (d, J = 8.8 Hz, 1H), 7.99 (d, J = 10.0 Hz, 1H), 7.76 (dt, J = 2.0, 7.6 Hz, 1H), 7.59 - 7.42 (m, 3H), 7.23 - 7.12 (m, 4H), 7.02 (d, J = 3.6 Hz, 1H), 5.41 (t, J = 7.6 Hz, 1H), 3.61 (s, 2H), 3.01 (s, 3H), 2.87 (s, 3H), 2.24 (s, 3H), 1.59 (d, J = 7.2 Hz, 3H); MS (ESI) m/z = 552.4 $[M+H]^+$.

**Example 298: 1-(2-fluorophenyl)-N-[(1R)-1-[5-[2-(methylaminomethyl)phenyl]-2-thienyl]ethyl]-6-oxo-pyridazine-3-carboxamide**

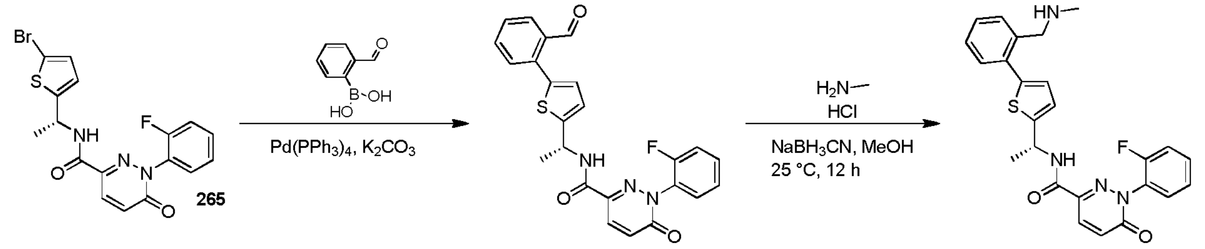


265

298

The compound of Example 298 was obtained in the same manner as in Example 266 by using the compound of Example 265 as a starting material. $^1$H NMR (400 MHz, DMSO-$d_6$) δ = 9.08 (d, J = 8.4 Hz, 1H), 7.99 (d, J = 9.6 Hz, 1H), 7.67 (dt, J = 1.6, 7.6 Hz, 1H), 7.61 - 7.54 (m, 1H), 7.48 (t, J = 7.6 Hz, 1H), 7.46 - 7.35 (m, 2H), 7.35 - 7.24 (m, 3H), 7.20 (d, J = 9.6 Hz, 1H), 7.12 (d, J = 3.6 Hz, 1H), 7.01 - 6.97 (m, 1H), 5.47 - 5.35 (m, 1H), 3.63 (s, 2H), 2.24 (s, 3H), 1.59 (d, J = 7.0 Hz, 3H); LC/MS (ESI) m/z: 463.3 [M+H]$^+$

**Example 299: 1-(2-fluorophenyl)-N-[(1R)-1-[5-[2-(hydroxymethyl)phenyl]-2-thienyl]ethyl]-6-oxo-pyridazine-3-carboxamide**

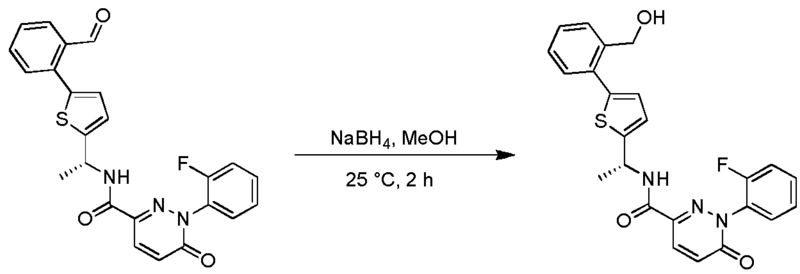


299

The compound of Example 299 was obtained by reducing the ketone group of the compound obtained in Step 2 of Example 298 to an alcohol with $NaBH_4$. $^1$H NMR (400 MHz, DMSO-$d_6$) δ = 9.07 (d, *J* = 8.4 Hz, 1H), 7.99 (d, *J* = 10.0 Hz, 1H), 7.67 (dt, *J* = 1.6, 7.6 Hz, 1H), 7.61 - 7.53 (m, 2H), 7.47 - 7.38 (m, 2H), 7.38 - 7.28 (m, 3H), 7.19 (d, *J* = 10.0 Hz, 1H), 7.09 (d, *J* = 3.6 Hz, 1H), 7.01 (d, *J* = 3.6 Hz, 1H), 5.52 - 5.32 (m, 1H), 5.29 - 5.19 (m, 1H), 4.53 (d, *J* = 5.2 Hz, 2H), 1.64 - 1.56 (m, 3H); LC/MS (ESI) m/z: 450.3 [M+H]$^+$

**Example 300: N-[(1R)-1-[5-[5-fluoro-2-[(2-hydroxyethylamino)methyl]phenyl]-2-thienyl]ethyl]-1-(2-fluorophenyl)-6-oxo-pyridazine-3-carboxamide**

Step 1: Synthesis of N-[(1R)-1-[5-(5-fluoro-2-formyl-phenyl)-2-thienyl]ethyl]-1-(2-fluorophenyl)-6-oxo-pyridazine-3-carboxamide

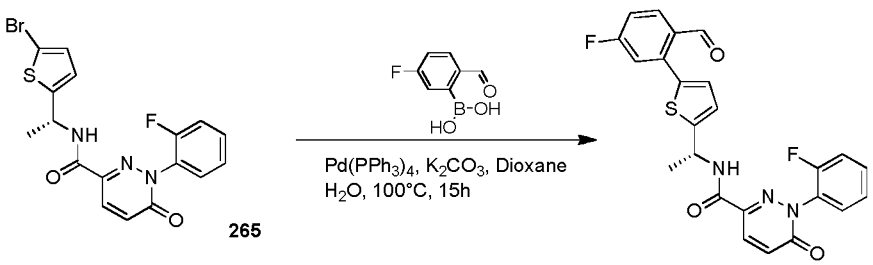


265

N-[(1R)-1-[5-(5-fluoro-2-formyl-phenyl)-2-thienyl]ethyl]-1-(2-fluorophenyl)-6-oxo-pyridazine-3-carboxamide was obtained in the same manner as in Step 1 of Example 266 by changing the boronic acid derivative.

Step 2: Synthesis of N-[(1R)-1-[5-[5-fluoro-2-[(2-hydroxyethylamino)methyl]phenyl]-2-thienyl]ethyl]-1-(2-fluorophenyl)-6-oxo-pyridazine-3-carboxamide

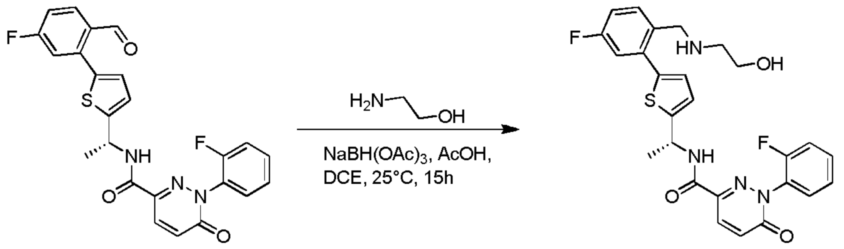


300

To a solution of N-[(1R)-1-[5-(5-fluoro-2-formyl-phenyl)-2-thienyl]ethyl]-1-(2-fluorophenyl)-6-oxo-pyridazine-3-carboxamide (56 mg, 120.31 μmol) and 2-aminoethanol (36.74 mg, 601.54 μmol) in DCE (3 mL) was added AcOH (21.67 mg, 360.92 μmol), and the reaction mixture was stirred at 25 °C for 30 min under $N_2$ atmosphere. After the addition of $NaBH(OAc)_3$ (76.49 mg, 360.92 μmol), the reaction mixture was stirred at 25 °C for 15 hours under $N_2$ atmosphere. The reaction mixture was poured into water (20 mL) and extracted with EtOAc (20 mL × 3). The combined organic layer was washed with brine (20 mL × 2), dried over anhydrous $Na_2SO_4$ and concentrated under reduced pressure to obtain a crude product. The residue was purified by flash silica gel chromatography (7% MeOH in DCM) to give crude product. The crude product was purified by prep-HPLC (column: Welch Xtimate C18 150*30mm*5μm; mobile phase: [water ($NH_3H_2O$+$NH_4HCO_3$)-ACN]; B%: 22%-62%, 36min), and most of MeCN was removed under reduced pressure. The remaining solvent was removed by lyophilization to give the compound of Example 300 (1.6 mg, 2.60% yield) as a white solid. $^1H$ NMR (400MHz, DMSO-$d_6$) δ = 9.07 (d, *J* = 8.4 Hz, 1H), 7.99 (d, *J* = 9.6 Hz, 1H), 7.66 (dt, *J* = 1.6, 7.6 Hz, 1H), 7.61 - 7.51 (m, 2H), 7.47 - 7.37 (m, 2H), 7.21 (s, 1H), 7.20 - 7.12 (m, 3H), 7.02 (d, *J* = 3.2 Hz, 1H), 5.40 (quin, *J* = 7.2 Hz, 1H), 4.48 (br t, *J* = 4.8 Hz, 1H), 3.69 (s, 2H), 3.43 (br s, 2H), 2.54 (t, *J* = 5.6 Hz, 2H), 1.59 (d, *J*= 7.2 Hz, 3H); MS (ESI) m/z = 511.4 $[M+H]^+$.

**Example 301: 1-(1-acetyl-4-piperidyl)-N-[(1R)-1-[5-[5-chloro-2-(methylaminomethyl)phenyl] -2-thienyl] ethyl] -6-oxo-pyridazine-3-carboxamide**

Step 1: Synthesis of (R)-1-(1-acetylpiperidin-4-yl)-N-(1-(5-bromothiophen-2-yl)ethyl)-6-oxo-1,6-dihydropyridazine-3-carboxamide

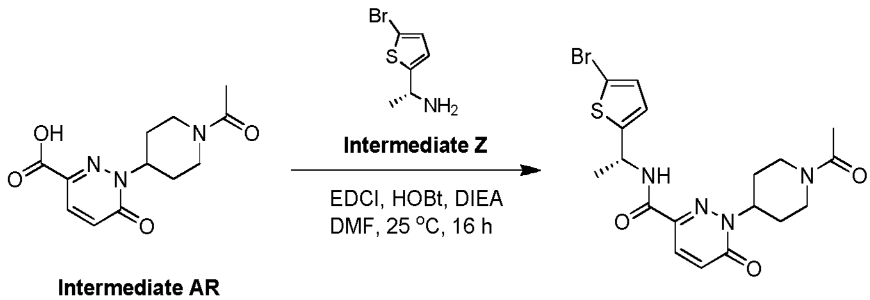


Intermediate AR

(R)-1-(1-acetylpiperidin-4-yl)-N-(1-(5-bromothiophen-2-yl)ethyl)-6-oxo-1,6-dihydropyridazine-3-carboxamide was obtained in the same manner as in Example 265 by using Intermediate AR.

Steps 2 and 3: Synthesis of 1-(1-acetyl-4-piperidyl)-N-[(IR)-1-[5-[5-chloro-2-(methylaminomethyl)phenyl]-2-thienyl]ethyl]-6-oxo-pyridazine-3-carboxamide

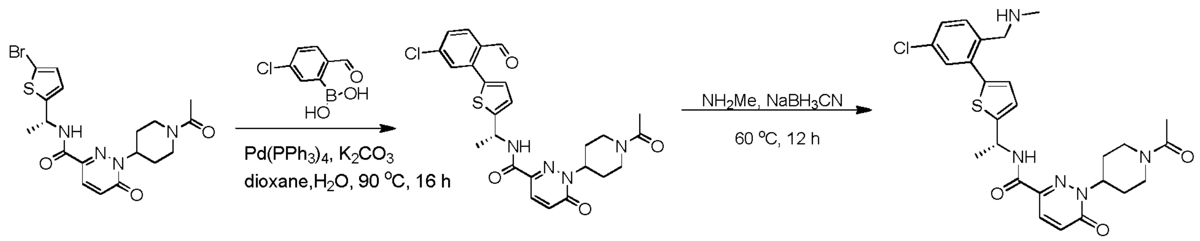


301

The compound of Example 301 was obtained in the same manner as in Example 266. $^1$H NMR (400MHz, DMSO-$d_6$) δ = 7.52 (br d, *J* = 8.8 Hz, 1H), 6.48 - 6.36 (m, 1H), 6.10 (d, *J* = 8.4 Hz, 1H), 6.01 - 5.86 (m, 2H), 5.82 - 5.71 (m, 1H), 5.60 (br d, *J* = 9.6 Hz, 2H), 4.06 - 3.94 (m, 1H), 3.63 - 3.50 (m, 1H), 3.12 (br dd, *J* = 3.6, 12.4 Hz, 1H), 2.59 - 2.46 (m, 1H), 2.20 (s, 2H), 1.79 - 1.71 (m, 1H), 1.25 - 1.17 (m, 1H), 0.81 (s, 3H), 0.71 - 0.43 (m, 6H), 0.42 - 0.29 (m, 2H), 0.22 (br d, *J* = 6.8 Hz, 3H); MS (ESI) m/z = 528.3 $[M+H]^+$.

**Example 302: N-[(1R)-1-[5-[5-fluoro-2-(methylaminomethyl)phenyl]-2-thienyl]ethyl]-1-[2-(methylamino)-3-pyridyl]-6-oxo-pyridazine-3-carboxamide**

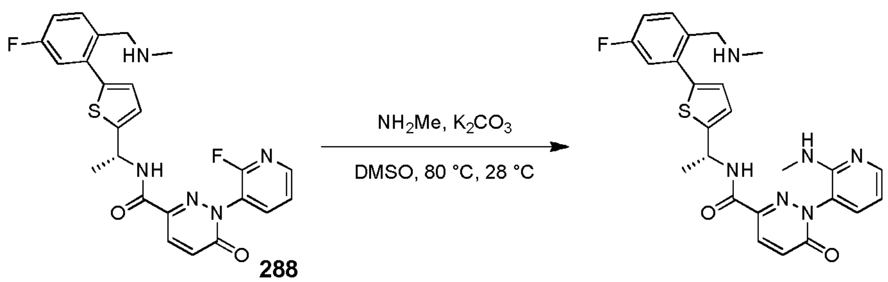


288 302

A mixture of the compound of Example 288 (25 mg, 51.92 μmoL), methanamine (7.01 mg, 103.84 μmoL, HCl) and $K_2CO_3$ (14.35 mg, 103.84 μmoL) in DMSO (2 mL) was degassed and purged with $N_2$ for 3 times, and then the mixture was stirred at 80°C for 28 hours under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by silica gel column chromatography (8% MeOH in DCM) to obtain a residue. The residue was purified by prep-HPLC (Column: Welch Xtimate C18 150*30mm*5μm; mobile phase: [Water ($NH_3H_2O$+$NH_4HCO_3$)-ACN];B%: 20%-60%,36min). Most of $CH_3CN$ was removed under reduced pressure, and the remaining solvent was removed by lyophilization to give the compound of Example 302 (1.8 mg, 3.63 μmoL, 7.00% yield) as a yellow solid. $^1$H NMR (400 MHz, DMSO-$d_6$) δ = 8.97 (d, J = 8.8 Hz, 1H), 8.12 (dd, J = 1.6, 5.2 Hz, 1H), 7.94 (d, J = 4.0 Hz, 1H), 7.52 (dd, J = 6.4, 8.3 Hz, 1H), 7.46 (dd, J = 2.0, 7.6 Hz, 1H), 7.22 - 7.09 (m, 4H), 7.04 - 6.98 (m, 1H), 6.61 (dd, J = 5.2, 7.6 Hz, 1H), 6.35 (q, J = 4.4 Hz, 1H), 5.47 - 5.34 (m, 1H), 3.60 (s, 2H), 2.76 (d, J = 4.4 Hz, 3H), 2.24 (s, 3H), 1.59 (d, J = 7.2 Hz, 3H); MS (ESI) m/z = 493.4 $[M+H]^+$.

**Example 303: 1-(2-fluorophenyl)-6-oxo-N-[(1R)-1-[3-(2-oxo-1-pyridyl)phenyl]ethyl]pyridazine-3-carboxamide**

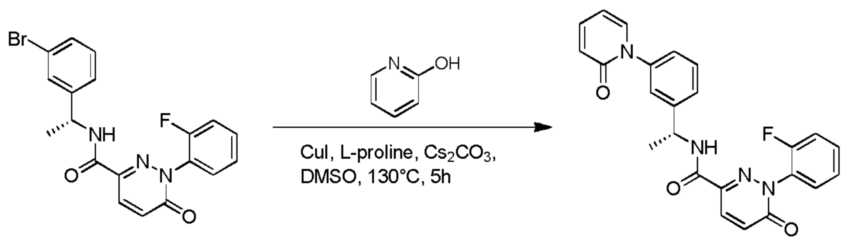


303

A mixture of N-[(1R)-1-(3-bromophenyl)ethyl]-1-(2-fluorophenyl)-6-oxo-pyridazine-3-carboxamide (50 mg, 120.12 μmol), pyridin-2-ol (17.14 mg, 180.18 μmol), CuI (4.58 mg, 24.02 μmol), $Cs_2CO_3$ (117.41 mg, 360.37 μmol) and

(2S)-pyrrolidine-2-carboxylic acid (2.77 mg, 24.02 μmol) in DMSO (2 mL) was degassed and purged with $N_2$ for 3 times, and then the mixture was stirred at 130 °C for 5 hours under $N_2$ atmosphere. The reaction mixture was poured into water (30 mL) and extracted with EtOAc (15 mL × 3). The combined organic layer was washed with brine (20 mL × 2), dried over anhydrous $Na_2SO_4$ and concentrated under reduced pressure to obtain a crude product. The crude product was purified by prep-TLC (DCM: MeOH = 10:1) to obtain a crude product. The product was purified by prep-HPLC (column: Phenomenex C18 75*30mm*3μm; mobile phase: [water($NH_3H_2O$+$NH_4HCO_3$)-ACN];B%: 20%-50%,14min), and most of MeCN was removed under reduced pressure. The remaining solvent was removed by lyophilization to give the compound of Example 303 (2.6 mg, 4.97% yield) as a light-yellow solid. $^1$H NMR (400MHz, DMSO-$d_6$) δ = 8.97 (d, *J* = 8.4 Hz, 1H), 7.94 (d, *J* = 10.0 Hz, 1H), 7.68 (dt, *J* = 1.6, 7.6 Hz, 1H), 7.62 - 7.56 (m, 2H), 7.51 (ddd, *J* = 2.0, 6.8, 9.2 Hz, 1H), 7.48 - 7.43 (m, 3H), 7.42 (s, 2H), 7.27 (dt, *J* = 2.0, 4.4 Hz, 1H), 7.18 (d, *J* = 10.0 Hz, 1H), 6.47 (d, *J* = 9.2 Hz, 1H), 6.31 (dt, *J* = 1.2, 6.8 Hz, 1H), 5.21 (quin, *J* = 7.2 Hz, 1H), 1.49 (d, *J* = 7.2 Hz, 3H); MS (ESI) m/z = 431.3 [M+H]$^+$.

**Example 304: 1-(2-fluorophenyl)-6-oxo-N-[(1R)-1-[3-(4-pyridyloxy)phenyl]ethyl]pyridazine-3-carboxamide**

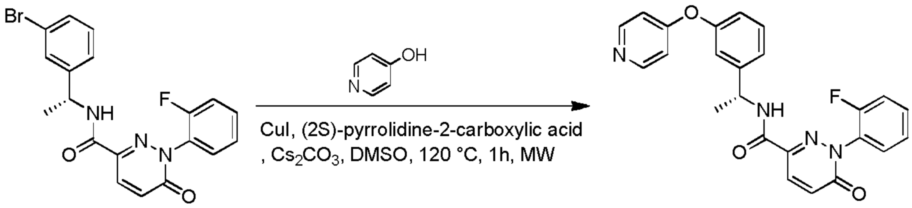


**304**

To a mixture of N-[(1R)-1-(3-bromophenyl)ethyl]-1-(2-fluorophenyl)-6-oxo-pyridazine-3-carboxamide (50 mg, 120.12 μmol) and pyridin-4-ol (17.14 mg, 180.18 μmol) in DMSO (2 mL), CuI (4.58 mg, 24.02 μmol), $Cs_2CO_3$ (117.41 mg, 360.37 μmol) and (2S)-pyrrolidine-2-carboxylic acid (2.77 mg, 24.02 μmol) were added, and the reaction mixture was stirred at 120 °C for 1 hour under $N_2$ atmosphere. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The residue was purified by prep-HPLC (column: Phenomenex C18 75*30mm*3μm; mobile phase: [water ($NH_3H_2O$+$NH_4HCO_3$)-ACN]; B%: 16%-46%, 11min). Most of $CH_3CN$ was removed under reduced pressure, and the remaining solvent was removed by lyophilization to give the compound of Example 304 (3.3 mg, 6.38% yield) as a white solid. $^1$H NMR (400 MHz, DMSO-$d_6$) δ ppm 1.50 (d, *J* =7.2 Hz, 3 H) 5.20 (m, 1 H) 6.23 (m, 2 H) 7.18 (d, *J* =10.0 Hz, 1 H) 7.44 (m, 5 H) 7.58 (m, 2 H) 7.67 (td, *J* =7.6, 1.75 Hz, 1 H) 7.95 (m, 3 H) 8.94 (d, *J* =8.4 Hz, 1 H); MS (ESI) m/z = 431.2 [M+1+H]$^+$.

**Example 305 and Example 306**

The compounds shown in the following table were obtained in the same manner as in Example 304, while appropriately changing the alcohol derivative in Example 304.

[Table 18]

| No. | Structure/Name | Spectral Data |
|---|---|---|
| 305 | 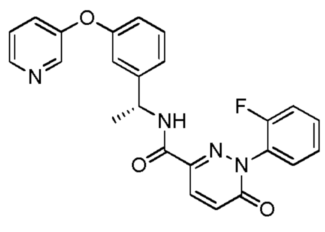 1-(2-fluorophenyl)-6-oxo-N-[(1R)-1-[3-(3-pyridyloxy)phenyl]ethyl]pyridazine-3-carboxamide | $^1$H NMR (400 MHz, DMSO-$d_6$) δ ppm 1.45 (d, *J* =7.2 Hz, 3 H) 5.12 (quin, *J* =7.6 Hz, 1 H) 6.89 (dd, *J* = 8.0, 2.0 Hz, 1 H) 7.12 (s, 1 H) 7.18 (m, 2 H) 7.40 (m, 5 H) 7.58 (m, 1 H) 7.65 (t, *J* =7.6 Hz, 1 H) 7.93 (d, *J* =10.0 Hz, 1 H) 8.36 (s, 2 H) 8.92 (br d, *J* = 8.4 Hz, 1 H); LC/MS (ESI) m/z: 431.3 [M+H]$^+$ |

(continued)

| No. | Structure/Name | Spectral Data |
|---|---|---|
| 306 | 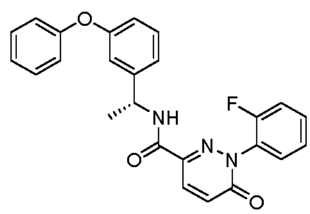 1-(2-fluorophenyl)-6-oxo-N-[(1R)-1-(3-phenoxyphenyl)ethyl]pyridazine-3- carboxamide | $^1$H NMR (400 MHz, DMSO-$d_6$) $\delta$ ppm 1.44 (d, *J*=7.2 Hz, 3 H) 5.11 (quin, *J* =7.2 Hz, 1 H) 6.82 (dd, *J* =8.00, 1.75 Hz, 1 H) 6.98 (br d, *J* = 8.0 Hz, 2 H) 7.07 (s, 1 H) 7.15 (m, 3 H) 7.37 (m, 5 H) 7.58 (m, 1 H) 7.64 (br t, *J* =7.6 Hz, 1 H) 7.93 (d, *J* =10.0 Hz, 1 H) 8.91 (br d, *J* =8.4 Hz, 1 H); LC/MS (ESI) m/z: 430.3 $[M+H]^+$ |

**Example 307: N-[(1R)-1-[3-amino-5-(trifluoromethyl)phenyl]ethyl]-5-(1-methylpyrazol-4-yl)-4-oxo-1H-pyrrolo[2,3-d]pyridazine-7-carboxamide**

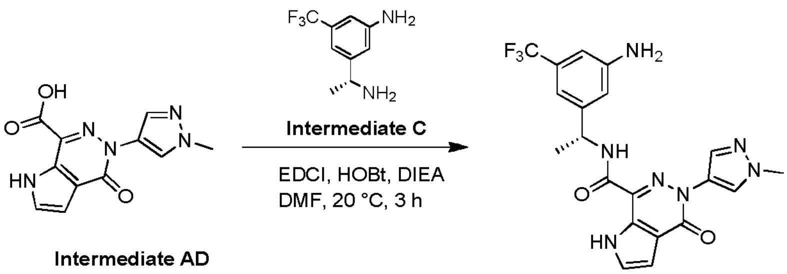


**307**

The compound of Example 307 (1.7 mg, 2.47% yield) was obtained as a white solid in the same manner as in Step 2 of Example 76. $^1$H NMR (400MHz, DMSO-$d_6$) $\delta$= 12.13 (br s, 1H), 8.96 (d, *J* = 8.4 Hz, 1H), 8.47 (s, 1H), 8.30 (s, 1H), 7.39(d, *J* = 3.2 Hz, 1H), 6.89 (s, 1H), 6.85 (s, 1H), 6.77 (d, *J* = 2.8 Hz, 1H), 6.72 (s, 1H), 5.58 (s, 2H), 5.16 (t, *J* = 7.6 Hz, 1H), 3.91(s, 3H), 1.57 (d, *J* = 7.2 Hz, 3H); MS (ESI) m/z = 446.4 $[M+H]^+$.

**Example 308: N-[(1R)-1-[3-amino-5-(trifluoromethyl)phenyl]ethyl]-4-oxo-5-phenyl-1H-pyrrolo[2,3-d]pyridazine-7-carboxamide**

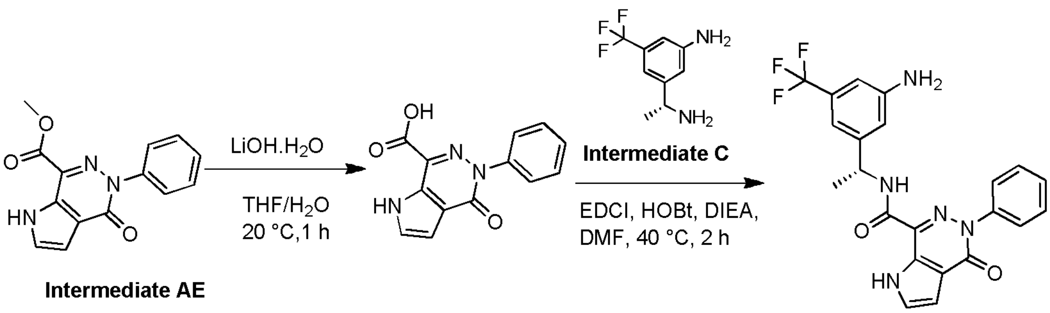


**308**

The compound of Example 308 (10.4 mg, 24.05% yield) was obtained as a white solid in the same manner as in Example 76. $^1$H NMR (400MHz, DMSO-$d_6$) $\delta$ = 12.08 (br s, 1H), 8.88 (br s, 1H), 7.65 (d, *J* = 7.6 Hz, 2H), 7.52 (t, *J* = 7.6 Hz, 2H), 7.45 - 7.38 (m, 2H), 6.86 (s, 1H), 6.82 (s, 1H), 6.75 (d, *J* = 2.8 Hz, 1H), 6.70 (s, 1H), 5.54 (s, 2H), 5.10 (quin, *J* = 7.2 Hz, 1H), 1.49 (d, *J* = 7.2 Hz, 3H); MS (ESI) m/z = 442.3 $[M+H]^+$.

**Example 309: N-[(1R)-1-[3-amino-5-(trifluoromethyl)phenyl]ethyl]-5-(2-fluorophenyl)-4-oxo-thieno[2,3-d]pyridazine-7-carboxamide**

Step 1: Synthesis of 5-(2-fluorophenyl)-4-oxo-thieno[2,3-d]pyridazine-7-carboxylic acid

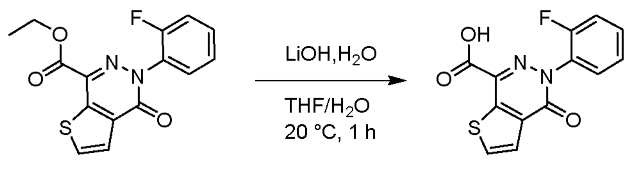


**Intermediate AG-1**

A mixture of Intermediate AG-1 (30 mg, 94.24 μmol) and LiOH (11.28 mg, 471.22 μmol) in THF (2 mL) and $H_2O$ (1 mL) was stirred at 15 °C for 1 hour. The reaction mixture was acidified to pH = 3-4 with 1 N aq. HCl and then extracted with EtOAc (10 mL × 3). The combined organic layer was washed with brine (20 mL), dried over anhydrous $Na_2SO_4$ and concentrated under reduced pressure to afford 5-(2-fluorophenyl)-4-oxo-thieno[2,3-d]pyridazine-7-carboxylic acid (20 mg, 73.11% yield) as a white solid. MS (ESI) m/z = 291.0 $[M+H]^+$.

Step 2: Synthesis of N-[(IR)-1-[3-amino-5-(trifluoromethyl)phenyl]ethyl]-5-(2-fluorophenyl)-4-oxo-thieno[2,3-d]pyridazine-7-carboxamide

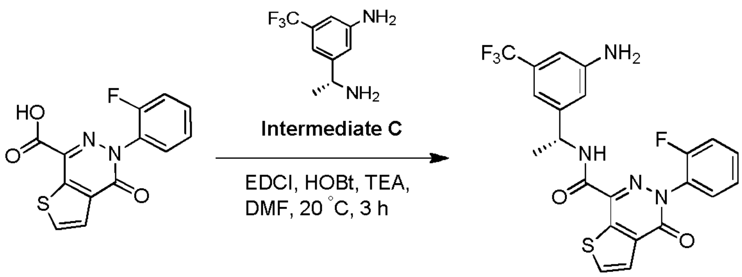


**309**

To a solution of 5-(2-fluorophenyl)-4-oxo-thieno[2,3-d]pyridazine-7-carboxylic acid (20 mg, 68.90 μmol) and Intermediate C (18.24 mg, 75.79 μmol) in DMF (2 mL), TEA (20.92 mg, 206.70 μmol), EDCI (19.81 mg, 103.35 μmol) and HOBt (13.96 mg, 103.35 μmol) were added, and then the reaction mixture was stirred at 50 °C for 16 hours. The mixture was poured into water (10 mL) and extracted with EtOAc (10 mL × 3). The combined organic layer was washed with brine (20 mL), dried over anhydrous $Na_2SO_4$ and concentrated under reduced pressure to obtain a crude product. The crude product was purified by prep-HPLC (column: Phenomenex C18 75*30mm*3μm; mobile phase: [water($NH_3H_2O$+$NH_4HCO_3$)-ACN];B%: 39%-69%,11min), the MeCN solvent was removed under reduced pressure, and the product was lyophilized to afford the compound of Example 309 (2.8 mg, 8.44% yield) as a yellow solid. $^1H$ NMR (400MHz, CHLOROFORM-*d*) δ = 7.75-7.88 (m, 2H), 7.42-7.55 (m, 3H), 7.27-7.38 (m, 2H), 7.03-7.16 (m, 2H), 5.26 (q, *J* = 7.2 Hz, 1H), 1.53-1.62 (m, 3H); MS (ESI) m/z = 477.3 $[M+H]^+$.

**Example 310 and Example 311**

The compounds shown in the following table were prepared in the same manner as in Example 309.

[Table 19]

| No. | Structure/Name | Spectral Data |
|---|---|---|
| 310 | 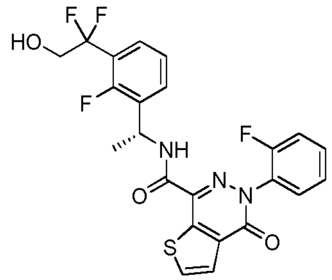 N-[(1R)-1-[3-(1,1-difluoro-2-hydroxy-ethyl)-2-fluoro-phenyl]ethyl]-5-(2-fluorophenyl)-4-oxo-thieno[2,3-d] pyridazine-7-carboxamide | 1H NMR (400 MHz, DMSO-$d_6$) δ = 9.22 (d, J = 8.0 Hz, 1H), 8.25 (d, J = 5.2 Hz, 1H), 7.78 - 7.69 (m, 2H), 7.68 - 7.57 (m, 2H), 7.51 - 7.41 (m, 3H), 7.28 (t, J = 7.2 Hz, 1H), 5.72 (br s, 1H), 5.47 (quin, J = 7.2 Hz, 1H), 3.92 (br t, J = 14.4 Hz, 2H), 1.50 (d, J = 7.2 Hz, 3H); LC/MS (ESI) m/z: 492.3 $[M+H]^+$ |
| 311 | 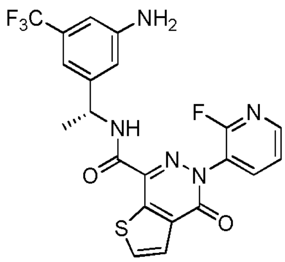 N-[(1R)-1-[3-amino-5-(trifluoromethyl)phenyl]ethyl]-5-(2-fluoro-3 - pyridyl)-4-oxo-thieno[2,3-d]pyridazine-7-carboxamide | $^1$H NMR (400 MHz, DMSO-$d_6$) δ = 9.16 (d, *J* = 8.4 Hz, 1H), 8.42 (br d, *J* = 4.4 Hz, 1H), 8.36 (t, *J* = 8.8 Hz, 1H), 8.28 (d, *J* = 5.2 Hz, 1H), 7.74 (d, *J* = 5.2 Hz, 1H), 7.65 (dd, *J* = 5.2, 7.2 Hz, 1H), 6.84 (s, 1H), 6.80 (s, 1H), 6.70 (s, 1H), 5.57 (s, 2H), 5.10 (br t, *J* = 7.6 Hz, 1H), 1.47 (d, *J* = 7.2 Hz, 3H); LC/MS (ESI) m/z: 478.3 $[M+H]^+$ |

**Example 312: N-[(1R)-1-[3-amino-5-(trifluoromethyl)phenyl]ethyl]-6-(2-fluorophenyl)-7-oxo-thieno[2,3-d]pyridazine-4-carboxamide**

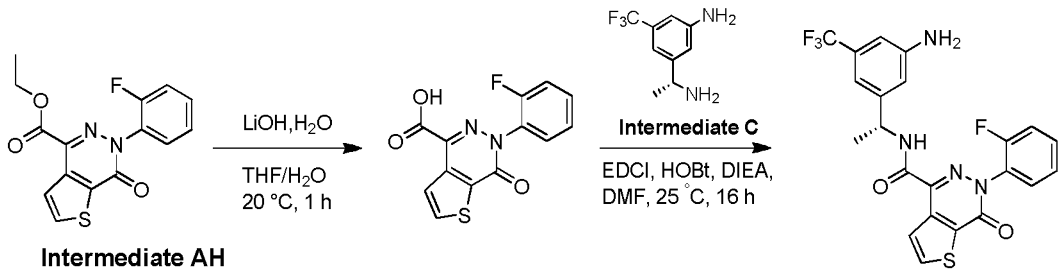


**Intermediate AH**

**312**

The compound of Example 312 (6.1 mg, 8.36% yield) was obtained as a white solid in the same manner as in Example 76. $^1$H NMR (400MHz, DMSO-$d_6$) δ = 8.99 (d, *J* = 8.0 Hz, 1H), 8.37 (d, *J* = 5.2 Hz, 1H), 8.03 (d, *J* = 5.2 Hz, 1H), 7.74 (t, *J* = 7.2 Hz, 1H), 7.55-7.64 (m, 1H), 7.39-7.51 (m, 2H), 6.81 (d, *J* = 11.6 Hz, 2H), 6.70 (s, 1H), 5.56 (s, 2H), 5.09 (q, *J* = 7.2 Hz, 1H), 1.45 (d, *J* = 6.8 Hz, 3H); MS (ESI) m/z = 476.9 $[M+H]^+$.

**Example 313: 4-acetamido-N-[(1R)-1-[3-amino-5-(trifluoromethyl)phenyl]ethyl]-6-oxo-1-phenyl-pyridazine-3-carboxamide**

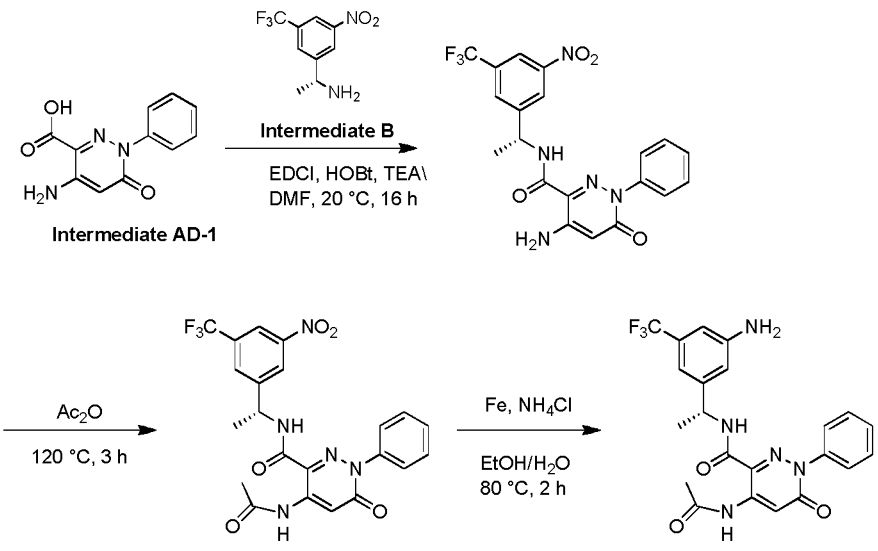


313

The compound of Example 313 (8.4 mg, 16.75% yield) was obtained as a white solid by performing the preparation method described in Example 113 in the order of Step 2, Step 1 and Step 3. $^1$H NMR (400MHz, DMSO-$d_6$) δ = 11.41 (s, 1H), 9.15 (d, *J* = 8.4 Hz, 1H), 7.79 (s, 1H), 7.66 (d, *J* = 8.0 Hz, 2H), 7.57 - 7.49 (m, 2H), 7.48 - 7.42 (m, 1H), 6.84 (s, 1H), 6.78 (s, 1H), 6.71 (s, 1H), 5.57 (s, 2H), 5.06 (quin, *J* = 7.2 Hz, 1H), 2.17 (s, 3H), 1.46 (d, *J* = 7.2 Hz, 3H); MS (ESI) m/z = 460.3 [M+H]$^+$.

**Example 314: N-[(1R)-1-[3-(1,1-difluoro-2-hydroxy-ethyl)-2-fluoro-phenyl]ethyl]-5-(2-fluorophenyl)-4-oxo-1H-pyrrolo[2,3-d]pyridazine-7-carboxamide**

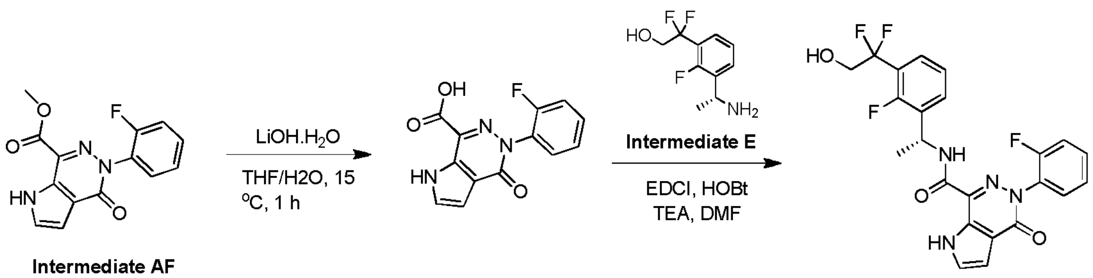


314

The compound of Example 314 (14.4 mg, 27.64% yield) was obtained as a white solid in the same manner as in Steps 2 and 3 of Example 21. $^1$H NMR (400MHz, DMSO-$d_6$) δ = 12.13 (s, 1H), 9.07 (s, 1H), 7.62-7.71 (m, 2H), 7.52-7.60 (m, 1H), 7.35-7.47 (m, 4H), 7.23-7.31 (m, 1H), 6.76 (d, *J* = 2.8 Hz, 1H), 5.72 (t, *J* = 6.4 Hz, 1H), 5.48 (q, *J* = 7.2 Hz, 1H), 3.92 (td, *J* = 14.4, 6.0 Hz, 2H), 1.50 (d, *J* = 7.2 Hz, 3H); MS (ESI) m/z = 475.2 [M+H]$^+$.

**Example 315: N-[(1R)-1-(3-chlorophenyl)ethyl]-4-oxo-5-phenyl-thieno[2,3-d]pyridazine-7-carboxamide**

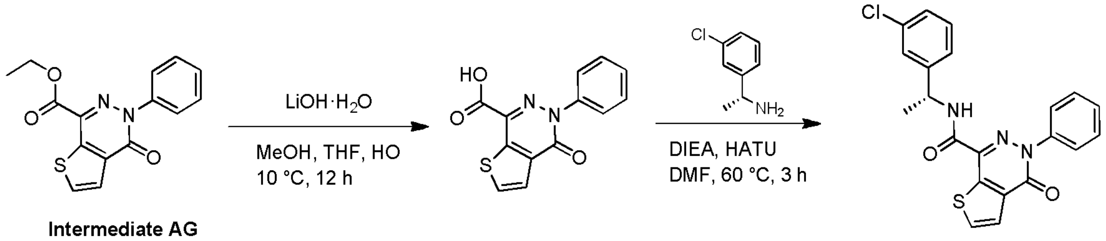


315

The compound of Example 315 (6.3 mg, 7.75% yield) was obtained as a white solid in the same manner as in Steps 2 and 3 of Example 17. $^1H$ NMR (400MHz, DMSO-$d_6$) $\delta$ = 9.07 (d, *J* = 8.4 Hz, 1H), 8.20 (d, *J* = 5.4 Hz, 1H), 7.75-7.65 (m, 3H), 7.57 (t, *J* = 7.6 Hz, 2H), 7.52-7.45 (m, 2H), 7.37 (q, *J* = 7.6 Hz, 2H), 7.32-7.24 (m, 1H), 5.26-5.14 (m, 1H), 1.51 (d, *J* = 7.2 Hz, 3H); MS (ESI) m/z = 410.1 $[M+H]^+$.

**Example 316 and Example 317**

The compounds shown in the following table were prepared in the same manner as in Example 315 by using appropriate starting materials and appropriate intermediates corresponding to the respective structures of the desired compounds based on Preparation Example 33.

[Table 20]

| No. | Structure/Name | Spectral Data |
|---|---|---|
| 316 | 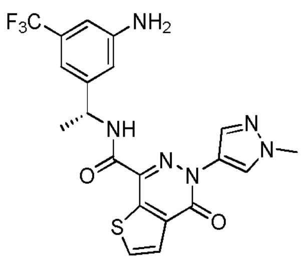 N-[(1R)-1-[3-amino-5-(trifluoromethyl)phenyl]ethyl]-5-(1-methylpyrazol-4-yl)-4-oxo-thieno[2,3- d]pyridazine-7-carboxamide | $^1H$ NMR (400 MHz, DMSO-$d_6$) $\delta$ = 9.07 (d, *J* = 8.4 Hz, 1H), 8.56 (s, 1H), 8.41 (s, 1H), 8.20 (d, *J* = 5.4 Hz, 1H), 7.72 (d, *J* = 5.4 Hz, 1H), 6.89 (s, 1H), 6.85 (s, 1H), 6.72 (s, 1H), 5.59 (s, 2H), 5.20 - 5.14 (m, 1H), 3.93 (s, 3H), 1.57 (d, *J* = 7.2 Hz, 3H); LC/MS (ESI) m/z: 463.3 $[M+H]^+$ |
| 317 | 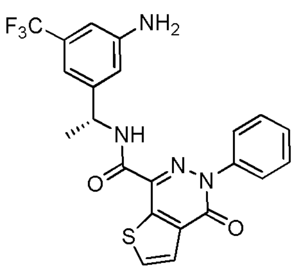 N-[(1R)-1-[3-amino-5-(trifluoromethyl)phenyl]ethyl]-4-oxo-5-phenyl-thieno[2,3-d]pyridazine-7-carboxamide | $^1H$ NMR (400 MHz, DMSO-$d_6$) $\delta$ = 9.02 (d, *J* = 8.4 Hz, 1H), 8.20 (d, *J* = 5.2 Hz, 1H), 7.73 - 7.68 (m, 3H),7.56 (t, *J* = 7.6 Hz, 2H), 7.50 - 7.45 (m, 1H), 6.86 (s, 1H), 6.82 (s, 1H), 6.71 (s, 1H), 5.56 (s, 2H), 5.10 (quin, *J* = 7.2 Hz, 1H), 1.49 (d, *J* = 7.2 Hz, 3H); LC/MS (ESI) m/z: 459.3 $[M+H]^+$ |

**Example 318: N-[(1R)-1-[3-amino-5-(trifluoromethyl)phenyl]ethyl]-1-[3-(2-methyl-1,2,4-triazol-3-yl)phenyl]-6-oxo-pyridine-3-carboxamide**

<u>Step 1: Synthesis of ethyl 6-oxo-1-(3-(4,4,5,5-tetramethyl-1,3,2-dioxaborolan-2-yl)phenyl)-1,6-dihydropyridine-3-carboxylate</u>

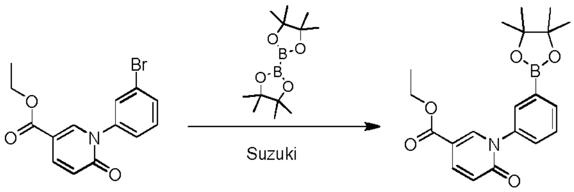


Ethyl 6-oxo-1-(3-(4,4,5,5-tetramethyl-1,3,2-dioxaborolan-2-yl)phenyl)-1,6-dihydropyridine-3-carboxylate was obtained in the same manner as in Step 1 of Example 109.

Step 2: Synthesis of ethyl 1-[3-(2-methyl-1,2,4-triazol-3-yl)phenyl]-6-oxo-pyridine-3-carboxylate

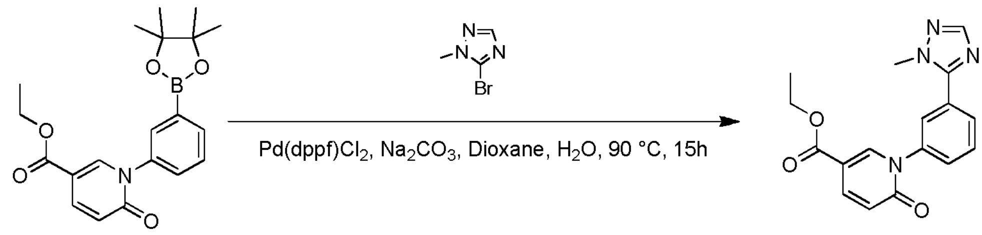


To a solution of ethyl 6-oxo-1-[3-(4,4,5,5-tetramethyl-1,3,2-dioxaborolan-2-yl)phenyl]pyridine-3-carboxylate (100 mg, 270.84 μmol) and 5-bromo-1-methyl-1,2,4-triazole (65.81 mg, 406.26 μmol) in dioxane (3 mL) and $H_2O$ (0.75 mL), $Pd(dppf)Cl_2$ (19.82 mg, 27.08 μmol) and $Na_2CO_3$ (86.12 mg, 812.53 μmol) were added. The mixture was stirred at 90 °C for 15 hours. The reaction mixture was poured into water (50 mL) and extracted with EtOAc (50 mL × 3). The combined organic layer was washed with brine (30 mL × 2), dried over anhydrous $Na_2SO_4$ and concentrated under reduced pressure to obtain a crude product. The crude product was purified by flash silica gel chromatography (32% EtOAc in PE) to give ethyl 1-[3-(2-methyl-1,2,4-triazol-3-yl)phenyl]-6-oxo-pyridine-3-carboxylate (30 mg, 7.14% yield) as yellow oil. MS (ESI) m/z = 325.1 $[M+H]^+$.

Steps 3 and 4: Synthesis ofN-[(IR)-1-[3-amino-5-(trifluoromethyl)phenyl]ethyl]-1-[3-(2-methyl-1,2,4-triazol-3-yl)phenyl]-6-oxo-pyridine-3-carboxamide

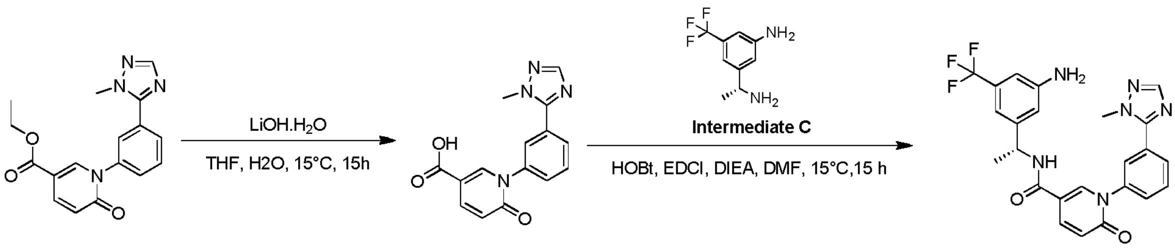


318

The compound of Example 318 (15.2 mg, 53.89% yield) was obtained as a white solid in the same manner as in Example 76. $^1H$ NMR (400MHz, DMSO-$d_6$) δ = 8.58 (d, *J* = 7.6 Hz, 1H), 8.44 (d, *J* = 2.4 Hz, 1H), 8.04 (s, 1H), 7.98 (dd, *J* = 2.8, 9.6 Hz, 1H), 7.95 - 7.84 (m, 2H), 7.79 - 7.72 (m, 1H), 7.72 - 7.63 (m, 1H), 6.76 (s, 2H), 6.70 (s, 1H), 6.57 (d, *J* = 9.6 Hz, 1H), 5.54 (s, 2H), 5.02 (quin, *J* = 7.2 Hz, 1H), 4.00 (s, 3H), 1.40 (d, *J* = 7.2 Hz, 3H); MS (ESI) m/z = 483.4 $[M+H]^+$.

**Example 319 and Example 320**

The compounds shown in the following table were prepared in the same manner as in Example 318.

[Table 21]

| No. | Structure/Name | Spectral Data |
|---|---|---|
| 319 | 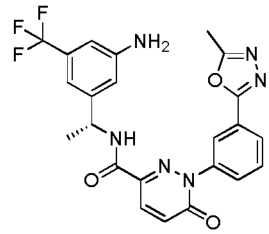 N-[(1R)-1-[3-amino-5-(trifluoromethyl)phenyl] ethyl]-1-[3-(5-methyl-1,3,4-oxadiazol-2-yl) phenyl]-6-oxo-pyridazine-3-carboxamide | $^1$H NMR (400 MHz, METHANOL-$d_4$) δ = 8.37 (s, 1H), 8.16 (d, *J* = 8.4 Hz, 1H), 8.05 (d, *J* = 10.0 Hz, 1H), 7.94 (br d, *J* = 8.0 Hz, 1H), 7.80 - 7.73 (m, 1H), 7.21 (d, *J* = 10.0 Hz, 1H), 6.92 (br d, *J* = 6.0 Hz, 2H), 6.82 (s, 1H), 5.16 (q, *J* = 7.2 Hz, 1H), 2.66 (s, 3H), 1.55 (d, *J* = 7.2 Hz, 3H); LC/MS (ESI) m/z: 485.4 $[M+H]^+$ |
| 320 | 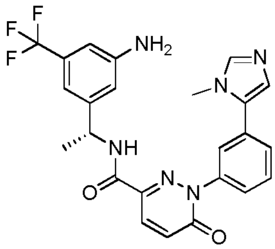 N-[(1R)-1-[3-amino-5-(trifluoromethyl)phenyl] ethyl]-1-[3-(3-methylimidazol-4-yl)phenyl]-6-oxo-pyridazine-3-carboxamide | $^1$H NMR (400 MHz, DMSO-$d_6$) δ = 8.89 (d, *J* = 8.4 Hz, 1H), 7.91 (d, *J* = 10.0 Hz, 1H), 7.81 (s, 1H), 7.74 (s, 1H), 7.70 - 7.65 (m, 1H), 7.64 - 7.57 (m, 2H), 7.19 - 7.11 (m, 2H), 6.80 (br d, *J* = 13.6 Hz, 2H), 6.70 (s, 1H), 5.54 (s, 2H), 5.04 (quin, *J* = 7.2 Hz, 1H), 3.73 (s, 3H), 1.44 (d, *J* = 7.2 Hz, 3H); LC/MS (ESI) m/z: 483.4 $[M+H]^+$ |

**Example 321: N-[(1R)-1-[3-amino-5-(trifluoromethyl)phenyl]ethyl]-1-[3-(2-methylpyrazol-3-yl)phenyl]-6-oxo-pyridine-3-carboxamide**

Step 1: Synthesis of ethyl 1-[3-(2-methylpyrazol-3-yl)phenyl]-6-oxo-pyridine-3-carboxylate

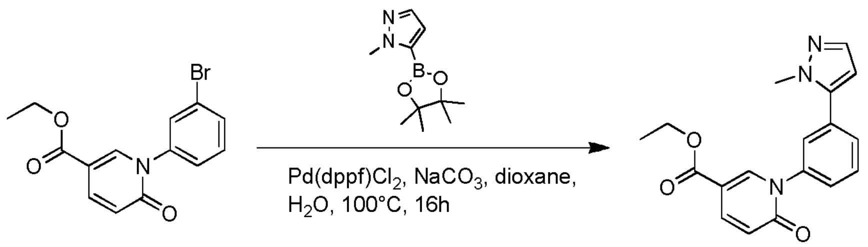


**Intermediate K-1**

A mixture of Intermediate K-1 (800 mg, 2.48 mmol), 1-methyl-5-(4,4,5,5-tetramethyl-1,3,2-dioxaborolan-2-yl)pyrazole (775.03 mg, 3.72 mmol), Pd(dppf)$Cl_2$ (181.70 mg, 248.33 μmol, 0.1*eq*) and $Na_2CO_3$ (789.60 mg, 7.45 mmol) in dioxane (15 mL) and $H_2O$ (1.5 mL) was degassed and purged with $N_2$ for 3 times, and then the mixture was stirred at 100 °C for 16 hours under $N_2$ atmosphere. The reaction mixture was poured into water (60 mL) and extracted with EtOAc (40 mL × 3). The combined organic layer was washed with brine (30 mL × 2), dried over anhydrous $Na_2SO_4$ and concentrated under reduced pressure to obtain a crude product. The product was purified by flash silica gel chromatography (35% EtOAc in PE) to give ethyl 1-[3-(2-methylpyrazol-3-yl)phenyl]-6-oxo-pyridine-3-carboxylate (710 mg, 71.84% yield) as a white solid. MS (ESI) m/z = 324.0 $[M+H]^+$.

Steps 2 and 3: Synthesis ofN-[(lR)-1-[3-amino-5-(trifluoromethyl)phenyl]ethyl]-1-[3-(2-methylpyrazol-3-yl)phenyl]-6-oxo-pyridine-3-carboxamide

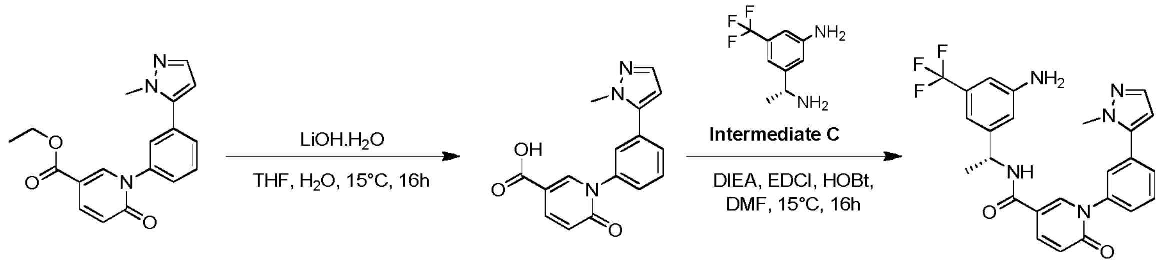


321

The compound of Example 321 (15.2 mg, 31.03% yield) was obtained as a white solid in the same manner as in Example 76. $^1H$ NMR (400MHz, DMSO-$d_6$) $\delta$ = 8.57 (d, *J* = 7.6 Hz, 1H), 8.43 (d, *J* = 2.4 Hz, 1H), 7.97 (dd, *J* = 2.8, 9.6 Hz, 1H), 7.71 - 7.65 (m, 3H), 7.59 - 7.53 (m, 1H), 7.50 (d, *J* = 2.0 Hz, 1H), 6.76 (s, 2H), 6.69 (s, 1H), 6.56 (d, *J* = 9.6 Hz, 1H), 6.50 (d, *J* = 2.0 Hz, 1H), 5.55 (s, 2H), 5.01 (quin, *J* = 7.2 Hz, 1H), 3.90 (s, 3H), 1.39 (d, *J* = 7.2 Hz, 3H); MS (ESI) m/z = 482.4 $[M+H]^+$.

**Example 322: N-[(1R)-1-[3-amino-5-(trifluoromethyl)phenyl]ethyl]-1-(3-isoxazol-4-ylphenyl)-6-oxo-pyridazine-3-carboxamide**

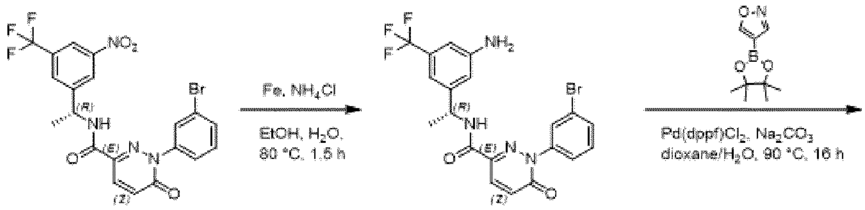


The compound of Example 322 (2.7 mg, 5.33% yield) was obtained as a white solid by subjecting (R)-1-(3-bromophenyl)-N-(1-(3-nitro-5-(trifluoromethyl)phenyl)ethyl)-6-oxo-1,6-dihydropyridazine-3-carboxamide as a starting material to the same reaction as in Step 2 of Example 16 and Step 1 of Example 321. $^1H$ NMR (400MHz, DMSO-$d_6$) $\delta$ = 9.52 (s, 1H), 9.22 (s, 1H), 8.90 (d, *J* = 8.4 Hz, 1H), 7.99 (s, 1H), 7.93 (d, *J* = 9.6 Hz, 1H), 7.84 - 7.80 (m, 1H), 7.64 - 7.59 (m, 2H), 7.18 (d, *J* = 9.6 Hz, 1H), 6.82 (s, 1H), 6.78 (s, 1H), 6.69 (s, 1H), 5.55 (s, 2H), 5.04 (br t, *J* = 7.6 Hz, 1H), 1.44 (d, *J* = 7.2 Hz, 3H); MS (ESI) m/z = 407.3 $[M+H]^+$.

**Example 323 to Example 346**

The compounds of Examples 323 to 341 were prepared in the same manner as in Example 321 by using appropriate starting materials and intermediates corresponding to the respective structures of the desired compounds.

[Table 22]

| No. | Structure/Name | Spectral Data |
|---|---|---|
| 323 | 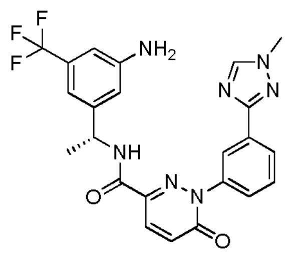 N-[(1R)-1-[3-amino-5-(trifluoromethyl)phenyl] ethyl]-1-[3-(1-methyl-1,2,4-triazol-3-yl) phenyl]-6-oxo-pyridazine-3- carboxamide | $^1H$ NMR (400 MHz, DMSO-$d_6$) $\delta$ = 8.90 (d, J=8.4 Hz, 1H), 8.56 (s, 1H), 8.23 (t, J=1.6 Hz, 1H), 8.07 (td, J=1.2, 7.6 Hz, 1H), 7.91 (d, J=9.6 Hz, 1H), 7.71 - 7.67 (m, 1H), 7.66 - 7.61 (m, 1H), 7.16 (d, J=9.8 Hz, 1H), 6.82 (s, 1H), 6.78 (s, 1H), 6.69 (s, 1H), 5.54 (s, 2H), 5.04 (quin, J=7.2 Hz, 1H), 3.93 (s, 3H), 1.44 (d, J=7.2 Hz, 3H) ; LC/MS (ESI) m/z: 484.4 $[M+H]^+$ |

(continued)

| No. | Structure/Name | Spectral Data |
|---|---|---|
| 324 | 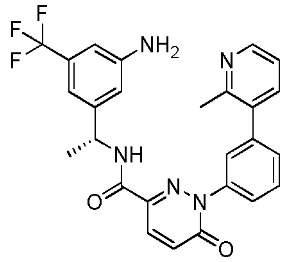 N-[(1R)-1-[3-amino-5-(trifluoromethyl)phenyl] ethyl]-1-[3-(2-methyl-3-pyridyl)phenyl]-6-oxo-pyridazine-3- carboxamide | $^1H$ NMR (400 MHz, DMSO-$d_6$) $\delta$ = 8.86 (d, *J* = 8.4 Hz, 1H), 8.49 (dd, *J* = 1.6, 4.8 Hz, 1H), 7.89 (d, *J* = 9.6 Hz, 1H), 7.81 - 7.70 (m, 2H), 7.70 - 7.59 (m, 2H), 7.51 (d, *J* = 7.6 Hz, 1H), 7.33 (dd, *J* = 4.8, 7.6 Hz, 1H), 7.16 (d, *J* = 9.6 Hz, 1H), 6.82 (s, 1H), 6.78 (s, 1H), 6.70 (s, 1H), 5.54 (br s, 2H), 5.03 (quin, *J* = 7.2 Hz, 1H), 2.49 - 2.48 (m, 3H), 1.45 (d, *J* = 7.2 Hz, 3H) ; LC/MS (ESI) m/z: 494.4 $[M+H]^+$ |
| 325 | 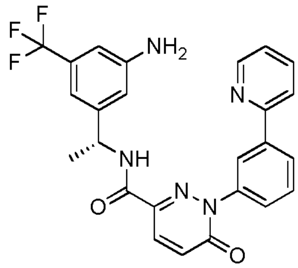 N-[(1R)-1-[3-amino-5-(trifluoromethyl)phenyl] ethyl]-6-oxo-1-[3-(2-pyridyl)phenyl]pyridazine-3-carboxamide | $^1H$ NMR (400 MHz, DMSO-$d_6$) $\delta$ = 8.90 (d, *J* = 8.4 Hz, 1H), 8.69 (d, *J* = 4.0 Hz, 1H), 8.33 (t, *J* = 1.6 Hz, 1H), 8.19 (td, *J* = 1.2, 7.6 Hz, 1H), 8.03 (d, *J* = 8.0 Hz, 1H), 7.95 - 7.89 (m, 2H), 7.75 - 7.70 (m, 1H), 7.69 - 7.64 (m, 1H), 7.44 - 7.37 (m, 1H), 7.17 (d, *J* = 9.6 Hz, 1H), 6.82 (s, 1H), 6.78 (s, 1H), 6.69 (s, 1H), 5.54 (s, 2H), 5.04 (quin, *J* = 7.2 Hz, 1H), 1.44 (d, *J* = 6.8 Hz, 3H) ; LC/MS (ESI) m/z: 480.4 $[M+H]^+$ |
| 326 | 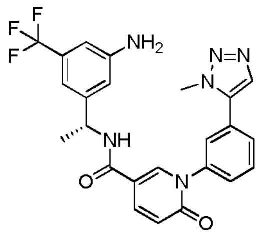 N-[(1R)-1-[3-amino-5-(trifluoromethyl)phenyl] ethyl]-1-[3-(3-methyltriazol-4-yl)phenyl]-6-oxo-pyridine-3- carboxamide | $^1H$ NMR (400 MHz, DMSO-$d_6$) $\delta$ = 8.59 (br d, *J* = 7.6 Hz, 1H), 8.43 (d, *J* = 2.0 Hz, 1H), 8.04 - 7.93 (m, 2H), 7.81 (s, 1H), 7.77 - 7.70 (m, 2H), 7.63 (br d, *J* = 7.2 Hz, 1H), 6.76 (s, 2H), 6.69 (s, 1H), 6.57 (d, *J* = 9.6 Hz, 1H), 5.56 (s, 2H), 5.01 (br t, *J* = 7.2 Hz, 1H), 4.12 (s, 3H), 1.39 (br d, *J* = 7.2 Hz, 3H) ; LC/MS (ESI) m/z: 483.4 $[M+H]^+$ |
| 327 | 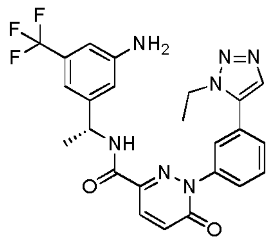 N-[(1R)-1-[3-amino-5-(trifluoromethyl)phenyl] ethyl]-1-[3-(3-ethyltriazol-4-yl)phenyl]-6-oxo-pyridazine-3- carboxamide | $^1H$ NMR (400 MHz, DMSO-$d_6$) $\delta$ = 8.90 (d, *J* = 8.4 Hz, 1H), 7.94 (s, 1H), 7.93 - 7.88 (m, 2H), 7.85 (d, *J* = 8.0 Hz, 1H), 7.74 - 7.68 (m, 1H), 7.67 - 7.62 (m, 1H), 7.18 (d, *J* = 9.6 Hz, 1H), 6.80 (d, *J* = 11.6 Hz, 2H), 6.70 (s, 1H), 5.55 (s, 2H), 5.04 (quin, *J* = 7.2 Hz, 1H), 4.46 (q, *J* = 7.2 Hz, 2H), 1.44 (d, *J* = 6.8 Hz, 3H), 1.37 (t, *J* = 7.2 Hz, 3H); LC/MS (ESI) m/z: 498.4 $[M+H]^+$ |

(continued)

| No. | Structure/Name | Spectral Data |
|---|---|---|
| 328 | 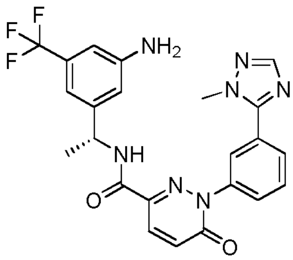 N-[(1R)-1-[3-amino-5-(trifluoromethyl)phenyl] ethyl]-1-[3-(2-methyl-1,2,4-triazol-3-yl) phenyl]-6-oxo-pyridazine-3- carboxamide | $^1$H NMR (400 MHz, DMSO-$d_6$) $\delta$ = 8.91 (br d, $J$ = 8.4 Hz, 1H), 8.10 (s, 1H), 8.04 (s, 1H), 7.94 - 7.85 (m, 3H),7.77 -7.71 (m, 1H), 7.18 (d, $J$ = 9.6 Hz, 1H), 6.81 (s, 1H), 6.78 (s, 1H), 6.70 (s, 1H), 5.55 (s, 2H), 5.04 (quin, $J$ = 7.2 Hz, 1H), 4.01 (s, 3H), 1.44 (d, $J$ = 7.2 Hz, 3H) ; LC/MS (ESI) m/z: 484.4 $[M+H]^+$ |
| 329 | 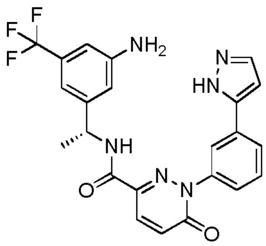 N-[(1R)-1-[3-amino-5-(tnfluoromethyl)phenyl] ethyl]-6-oxo-1-[3-(1H-pyrazol-5-yl)phenyl] pyridazine-3-carboxamide | $^1$H NMR (400 MHz, DMSO-$d_6$) $\delta$ = 12.99 (br s, 1H), 8.89 (br d, $J$ = 8.4 Hz, 1H), 8.03 (s, 1H), 7.95 - 7.88 (m, 2H), 7.77 (br s, 1H), 7.61 - 7.52 (m, 2H), 7.16 (d, $J$ = 9.6 Hz, 1H), 6.82 (s, 1H), 6.78 (br s, 2H), 6.69 (s, 1H), 5.54 (s, 2H), 5.07 - 4.99 (m, 1H), 1.44 (br d, $J$ = 7.2 Hz, 3H) ; LC/MS (ESI) m/z: 469.3 $[M+H]^+$ |
| 330 | 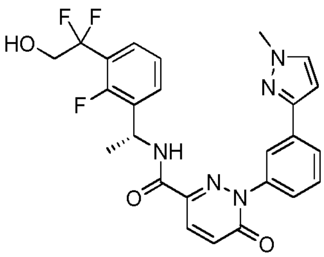 N-[(1R)-1-[3-(1,1-difluoro-2-hydroxy-ethyl)-2-fluoro-phenyl]ethyl]-1-[3-(1-methylpyrazol-3-yl)phenyl]-6-oxo-pyridazine-3-carboxamide | $^1$H NMR (400 MHz, DMSO-$d_6$) $\delta$ = 9.01 (d, $J$ = 8.0 Hz, 1H), 8.02 (s, 1H), 7.91 - 7.85 (m, 2H), 7.77 (d, $J$ = 2.0Hz, 1H), 7.64 (br t, $J$ = 7.2 Hz, 1H), 7.60 - 7.54 (m, 2H), 7.45 - 7.40 (m, 1H), 7.26 (t, $J$ = 7.6 Hz, 1H), 7.15 (d, $J$ = 9.6 Hz, 1H), 6.76 (d, $J$ = 2.4 Hz, 1H), 5.72 (br s, 1H), 5.41 (quin, $J$ = 7.2 Hz, 1H), 3.95 - 3.91 (m, 1H), 3.90 (s, 3H), 3.88 (br s, 1H), 1.47 (d, $J$ = 7.2 Hz, 3H) ; LC/MS (ESI) m/z: 498.3 $[M+H]^+$ |
| 331 | 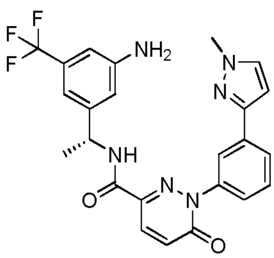 N-[(1R)-1-[3-amino-5-(trifluoromethyl)phenyl] ethyl]-1-[3-(1-methylpyrazol-3-yl)phenyl]-6-oxo-pyridazine-3-carboxamide | $^1$H NMR (400 MHz, DMSO-$d_6$) $\delta$ = 8.89 (d, $J$ = 8.4 Hz, 1H), 8.01 (s, 1H), 7.93 - 7.84 (m, 2H), 7.76 (d, $J$ = 2.0 Hz, 1H), 7.58 - 7.51 (m, 2H), 7.16 (d, $J$ = 9.6 Hz, 1H), 6.82 (s, 1H), 6.78 (s, 1H), 6.75 (d, $J$ = 2.0 Hz, 1H), 6.69 (s, 1H), 5.54 (s, 2H), 5.07 - 4.99 (m, 1H), 3.89 (s, 3H), 1.44 (d, $J$ = 7.2 Hz, 3H) ; LC/MS (ESI) m/z: 483.4 $[M+H]^+$ |

(continued)

| No. | Structure/Name | Spectral Data |
|---|---|---|
| 332 | 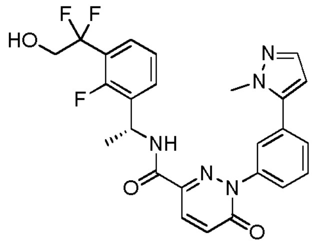<br>N-[(1R)-1-[3-(1,1-difluoro-2-hydroxy-ethyl)-2-fluoro-phenyl]ethyl]-1-[3-(2-methylpyrazol-3-yl)phenyl]-6-oxo-pyridazine-3-carboxamide | $^1$H NMR (400 MHz, DMSO-$d_6$) δ = 9.02 (d, *J* = 8.0 Hz, 1H), 7.89 (d, *J* = 9.6 Hz, 2H), 7.78 (td, *J* = 1.6, 7.6 Hz, 1H), 7.70 - 7.62 (m, 3H), 7.51 (d, *J* = 2.0 Hz, 1H), 7.43 (br t, *J* = 7.2 Hz, 1H), 7.27 (t, *J* = 7.6 Hz, 1H), 7.17 (d, *J* = 9.6 Hz, 1H), 6.50 (d, *J* = 2.0 Hz, 1H), 5.91 - 5.61 (m, 1H), 5.41 (quin, *J* = 7.2 Hz, 1H), 3.95 (s, 1H), 3.92 (s, 3H), 3.88 (s, 1H), 1.48 (d, *J* = 7.2 Hz, 3H) ; LC/MS (ESI) m/z: 498.3 $[M+H]^+$ |
| 333 | 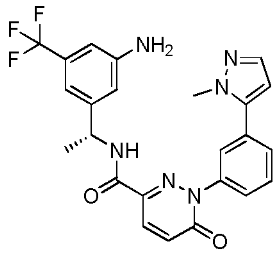<br>N-[(1R)-1-[3-amino-5-(trifluoromethyl)phenyl] ethyl]-1-[3-(2-methylpyrazol-3-yl)phenyl]-6-oxo-pyridazine-3-carboxamide | $^1$H NMR (400 MHz, DMSO-$d_6$) δ = 8.90 (d, *J* = 8.4 Hz, 1H), 7.91 (d, *J* = 9.6 Hz, 1H), 7.86 (s, 1H), 7.76 (td, *J* = 2.0, 7.2 Hz, 1H), 7.68 - 7.62 (m, 2H), 7.50 (d, *J* = 2.0 Hz, 1H), 7.17 (d, *J* = 9.6 Hz, 1H), 6.82 (s, 1H), 6.78 (s, 1H), 6.70 (s, 1H), 6.49 (d, *J* = 2.0 Hz, 1H), 5.55 (s, 2H), 5.04 (quin, *J* = 7.2 Hz, 1H), 3.90 (s, 3H), 1.44 (d, *J* = 7.2 Hz, 3H) ; LC/MS (ESI) m/z: 483.4 $[M+H]^+$ |
| 334 | 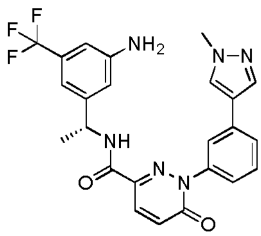<br>N-[(1R)-1-[3-amino-5-(trifluoromethyl)phenyl] ethyl]-1-[3-(1-methylpyrazol-4-yl)phenyl]-6-oxo-pyridazine-3-carboxamide | $^1$H NMR (400 MHz, DMSO-$d_6$) δ = 8.89 (d, *J* = 8.4 Hz, 1H), 8.20 (s, 1H), 7.97 - 7.85 (m, 2H), 7.80 (t, *J* = 1.6 Hz, 1H), 7.66 (d, *J* = 7.6 Hz, 1H), 7.51 (t, *J* = 8.0 Hz, 1H), 7.46 - 7.40 (m, 1H), 7.16 (d, *J* = 9.6 Hz, 1H), 6.82 (s, 1H), 6.78 (s,1H), 6.69 (s, 1H), 5.55 (s, 2H), 5.04 (quin, *J* = 7.2 Hz, 1H), 3.87 (s, 3H), 1.44 (d, *J* = 7.2 Hz, 3H) ; LC/MS (ESI) m/z: 483.4 $[M+H]^+$ |
| 335 | 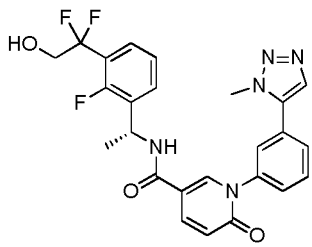<br>N-[(1R)-1-[3-(1,1-difluoro-2-hydroxy-ethyl)-2-fluoro-phenyl]ethyl]-1-[3-(3-methyltriazol-4-yl) phenyl]-6-oxo-pyridine-3-carboxamide | $^1$H NMR (400 MHz, DMSO-$d_6$) δ = 8.68 (d, *J* = 7.6 Hz, 1H), 8.47 (d, *J* = 2.4 Hz, 1H), 8.00 (s, 1H), 7.97 (dd, *J* = 2.4, 9.6 Hz, 1H), 7.82 (s, 1H), 7.74 (q, *J* = 7.6 Hz, 2H), 7.66 - 7.62 (m, 1H), 7.57 (br t, *J* = 7.2 Hz, 1H), 7.42 (br t, *J* = 7.2 Hz, 1H), 7.27 (t, *J* = 7.6 Hz, 1H), 6.57 (d, *J* = 9.6 Hz, 1H), 5.71 (br s, 1H), 5.36 (t, *J* = 7.2 Hz, 1H), 4.13 (s, 3H), 3.91 (br t, *J* = 14.4 Hz, 2H), 1.43 (d, *J* = 7.2 Hz, 3H) ; LC/MS (ESI) m/z: 498.4 $[M+H]^+$ |

(continued)

| No. | Structure/Name | Spectral Data |
|---|---|---|
| 336 | 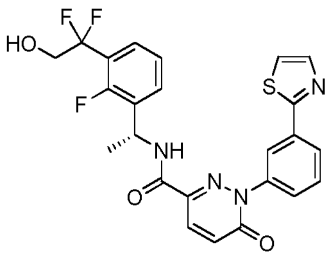 N-[(1R)-1-[3-(1,1-difluoro-2-hydroxy-ethyl)-2-fluoro-phenyl]ethyl]-6-oxo-1-(3-thiazol-2-ylphenyl)pyridazine-3-carboxamide | $^1H$ NMR (400 MHz, DMSO-$d_6$) $\delta$ = 9.05 (d, $J$ = 8.0 Hz, 1H), 8.25 (t, $J$ = 2.0Hz, 1H), 8.06 (d, $J$ = 7.6 Hz, 1H),7.99 (d, $J$ = 3.2 Hz, 1H), 7.93 - 7.85 (m, 2H), 7.84 - 7.77 (m, 1H), 7.72 - 7.62 (m, 2H), 7.46 - 7.40 (m, 1H), 7.30 - 7.24 (m, 1H), 7.17 (d, $J$ = 9.6 Hz, 1H), 5.72 (t, $J$ = 6.4 Hz, 1H), 5.42 (quin, $J$ = 7.2 Hz, 1H), 3.91 (dt, $J$ = 6.4, 14.4 Hz, 2H), 1.47 (d, $J$ = 7.2 Hz, 3H) ; LC/MS (ESI) m/z: 501.3 $[M+H]^+$ |
| 337 | 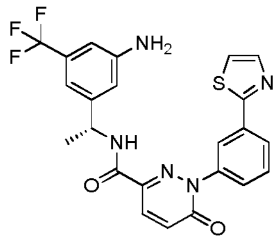 N-[(1R)-1-[3-amino-5-(trifluoromethyl)phenyl] ethyl]-6-oxo-1-(3-thiazol-2-ylphenyl) pyridazine-3-carboxamide | $^1H$ NMR (400 MHz, DMSO-$d_6$) $\delta$ = 8.92 (d, $J$ = 8.4 Hz, 1H), 8.24 (t, $J$ = 1.6 Hz, 1H), 8.05 (d, $J$ = 8.0 Hz, 1H),7.97 (d, $J$ = 3.2 Hz, 1H), 7.92 (d, $J$ = 9.6 Hz, 1H), 7.86 (d, $J$ = 3.2 Hz, 1H), 7.81 - 7.76 (m, 1H), 7.71 - 7.66 (m, 1H), 7.17 (d, $J$ = 9.6 Hz, 1H), 6.82 (s, 1H), 6.79 (s, 1H), 6.70 (s, 1H), 5.54 (s, 2H), 5.04 (quin, $J$ = 7.2 Hz, 1H), 1.44 (d, $J$ = 7.2 Hz, 3H) ; LC/MS (ESI) m/z: 486.3 $[M+H]^+$ |
| 338 | 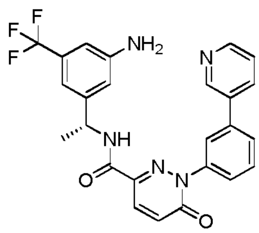 N-[(1R)-1-[3-amino-5-(tnfluoromethyl)phenyl] ethyl]-6-oxo-1-[3-(3-pyridyl)phenyl]pyridazine-3-carboxamide | $^1H$ NMR (400 MHz, DMSO-$d_6$) $\delta$ = 8.95 (d, $J$ = 2.0 Hz, 1H), 8.91 (d, $J$ = 8.0 Hz, 1H), 8.61 (d, $J$ = 4.8 Hz, 1H), 8.13 (brd, $J$ = 8.8 Hz, 1H), 8.02 (s, 1H), 7.92 (d, $J$ = 9.6 Hz, 1H), 7.85 (d, $J$ = 7.2 Hz, 1H), 7.75 - 7.71 (m, 1H), 7.68 (d, $J$ = 7.6 Hz, 1H), 7.52 (dd, $J$ = 4.8, 7.6 Hz, 1H), 7.18 (d, $J$ = 9.6 Hz, 1H), 6.82 (s, 1H), 6.79 (s, 1H), 6.69 (s, 1H), 5.53 (s, 2H), 5.07 - 5.01 (m, 1H), 1.44 (d, $J$ = 7.2 Hz, 3H) ; LC/MS (ESI) m/z: 480.4 $[M+H]^+$ |
| 339 | 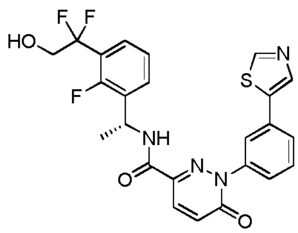 N-[(1R)-1-[3-(1,1-difluoro-2-hydroxy-ethyl)-2-fluorophenyl]ethyl]-6-oxo-1-(3-thiazol-5-ylphenyl)pyridazine-3-carboxamide | $^1H$ NMR (400 MHz, CHLOROFORM-d) $\delta$ = 8.90 (s, 1H), 8.33 (s, 1H), 8.02 (d, $J$ = 9.6 Hz, 1H), 7.92 - 7.86 (m, 2H), 7.81 (d, $J$ = 9.2 Hz, 1H), 7.62 - 7.51 (m, 3H), 7.47 (t, $J$ = 7.2 Hz, 1H), 7.24 - 7.20 (m, 1H), 7.12 (d, $J$ = 9.6 Hz, 1H), 5.47 - 5.37 (m, 1H),4.30 - 4.19 (m, 1H), 4.05 - 3.93 (m, 1H), 1.60 (d, $J$ = 7.2 Hz, 3H) ; LC/MS (ESI) m/z: 501.3 $[M+H]^+$ |

(continued)

| No. | Structure/Name | Spectral Data |
|---|---|---|
| 340 | 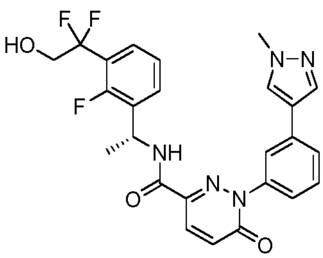 N-[(1R)-1-[3-(1,1-difluoro-2-hydroxy-ethyl)-2-fluoro-phenyl]ethyl]-1-[3-(1-methylpyrazol-4-yl)phenyl]-6-oxo-pyridazine-3-carboxamide | $^1$H NMR (400 MHz, DMSO-$d_6$) $\delta$ = 9.01 (d, *J* = 8.0 Hz, 1H), 8.21 (s, 1H), 7.92 (s, 1H), 7.89 (d, *J* = 10.0 Hz, 1H), 7.81 (s, 1H), 7.69 - 7.61 (m, 2H), 7.53 (t, *J* = 8.0 Hz, 1H), 7.48 - 7.39 (m, 2H), 7.29 - 723 (m, 1H), 7.15 (d, *J* = 10.0 Hz, 1H), 5.73 (t, *J* = 6.4 Hz, 1H), 5.40 (m, *J* = 7.2 Hz, 1H), 3.96 - 3.90 (m, 2H), 3.87 (s, 3H), 1.47 (d, *J* = 7.2 Hz, 3H) ; LC/MS (ESI) m/z: 498.4 $[M+H]^+$ |
| 341 | 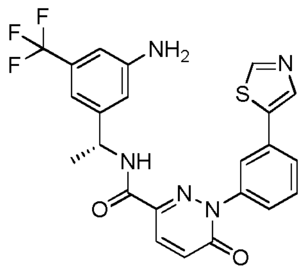 N-[(1R)-1-[3-amino-5-(trifluoromethyl)phenyl] ethyl]-6-oxo-1-(3-thiazol-5-ylphenyl) pyridazine-3-carboxamide | $^1$H NMR (400 MHz, CHLOROFORM-d) $\delta$ = 8.85 (s, 1H), 8.18 (s, 1H), 8.04 (d, *J* = 9.6 Hz, 1H), 7.82 - 7.78 (m, 1H), 7.69 - 7.63 (m, 1H), 7.57 (dd, *J* = 2.0, 3.8 Hz, 2H), 7.29 (d, *J* = 8.0 Hz, 1H), 7.12 (d, *J* = 10.0 Hz, 1H), 7.01 (s, 1H), 6.92 ( d, *J* = 17.6 Hz, 2H), 5.20 (q, *J* = 7.2 Hz, 1H), 1.55 (d, *J* = 7.2 Hz, 3H) ; LC/MS (ESI) m/z: 486.3 $[M+H]^+$ |
| 342 | 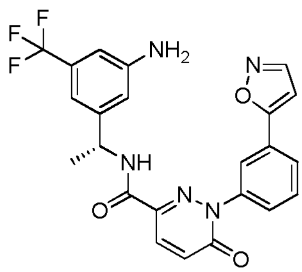 N-[(1R)-1-[3-amino-5-(trifluoromethyl)phenyl] ethyl]-1-(3-isoxazol-5-ylphenyl)-6-oxo-pyridazine-3-carboxamide | $^1$H NMR (400 MHz, DMSO-$d_6$) $\delta$ = 8.93 (d, *J* = 8.4 Hz, 1H), 8.71 (d, *J* = 2.0 Hz, 1H), 8.19 (s, 1H), 7.99 (d, J = 8.0 Hz, 1H), 7.92 (d, *J* = 9.6 Hz, 1H), 7.84 - 7.78 (m, 1H), 7.75 - 7.69 (m, 1H), 7.19 (d, *J* = 10.0 Hz, 1H), 7.12 (d, *J* = 2.0 Hz, 1H), 6.80 (br d, *J* = 14.4 Hz, 2H), 6.69 (s, 1H), 5.55 (s, 2H), 5.04 (s, 1H), 1.44 (d, *J* = 7.2 Hz, 3H) ; LC/MS (ESI) m/z: 470.3 $[M+H]^+$ |
| 343 | 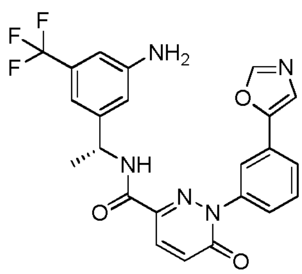 N-[(1R)-1-[3-amino-5-(trifluoromethyl)phenyl] ethyl]-1-(3-oxazol-5-ylphenyl)-6-oxo-pyridazine-3-carboxamide | $^1$H NMR (400 MHz, DMSO-$d_6$) $\delta$ = 8.91 (d, *J* = 8.4 Hz, 1H), 8.50 (s, 1H), 8.02 (s, 1H), 7.92 (d, *J* = 10.0 Hz, 1H), 7.85 - 7.81 (m, 1H), 7.78 (s, 1H), 7.68 - 7.65 (m, 2H), 7.17 (d, *J* = 9.6 Hz, 1H), 6.80 (d, *J* = 14.8 Hz, 2H), 6.70 (s, 1H), 5.55 (s, 2H), 5.04 (t, *J* = 7.6 Hz, 1H), 1.44 (d, *J* = 7.2 Hz, 3H); LC/MS (ESI) m/z: 470.3 $[M+H]^+$ |

(continued)

| No. | Structure/Name | Spectral Data |
|---|---|---|
| 344 | 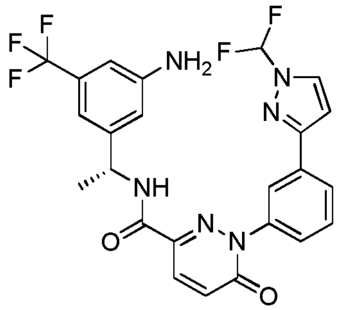 N-[(1R)-1-[3-amino-5-(trifluoromethyl)phenyl] ethyl]-1-[3-[1-(difluoromethyl)pyrazol-3-yl] phenyl]-6-oxo-pyridazine-3-carboxamide | $^1$H NMR (400 MHz, DMSO-$d_6$) δ ppm 1.44 (d, *J* =7.2 Hz, 3 H) 5.04 (quin, *J* =7.2 Hz, 1 H) 5.55 (s, 2 H) 6.69 (s, 1 H) 6.80 (br d, *J* =15.2 Hz, 2 H) 7.10 (d, *J* =2.8 Hz, 1 H) 7.17 (d, *J* =10.0 Hz, 1 H) 7.66 (m, 2 H) 7.72 (s, 1 H) 7.87 (s, 1 H) 7.92 (d, *J* =10.0 Hz, 1 H) 7.99 (m, 1 H) 8.02 (s, 1 H) 8.14 (s, 1 H) 8.34 (d, *J* =2.8 Hz, 1 H) 8.91 (d, *J* =8.4 Hz, 1 H) ; LC/MS (ESI) m/z: 519.3 $[M+H]^+$ |
| 345 | 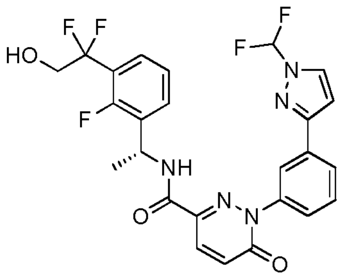 N-[(1R)-1-[3-(1,1-difluoro-2-hydroxy-ethyl)-2-fluoro-phenyl]ethyl]-1-[3-[1-(difluoromethyl) pyrazol-3-yl]phenyl]-6-oxo-pyridazine-3-carboxamide | $^1$H NMR (400 MHz, DMSO-$d_6$) δ ppm 1.47 (d, *J* =7.2 Hz, 3 H) 3.91 (br t, *J* =14.4 Hz, 2 H) 5.41 (quin, *J* =7.6 Hz, 1 H) 5.71 (m, 1 H) 7.12 (d, *J* =2.8 Hz, 1 H) 7.17 (d, *J* =10.0 Hz, 1 H) 7.27 (m, 1 H) 7.43 (m, 1 H) 7.67 (m, 3 H) 7.73 (s, 1 H) 7.88 (d, *J* =4.0 Hz, 1 H) 7.91 (s, 1 H) 8.00 (d, *J* =7.6 Hz, 1 H) 8.03 (s, 1 H) 8.16 (d, *J* =1.6 Hz, 1 H) 8.35 (d, *J* =2.8 Hz, 1 H) 9.03 (d, *J* =8.00 Hz, 1 H) ; LC/MS (ESI) m/z: 534.3 $[M+H]^+$ |
| 346 | 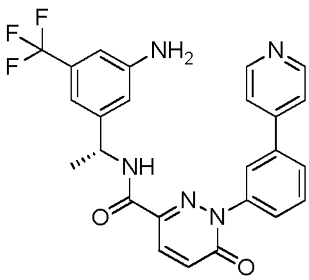 N-[(1R)-1-[3-amino-5-(trifluoromethyl)phenyl] ethyl]-6-oxo-1-[3-(4-pyridyl)phenyl]pyridazine-3-carboxamide | $^1$H NMR (400 MHz, DMSO-$d_6$) δ ppm 1.44 (d, *J*=7.2 Hz, 3 H) 5.05 (q, *J* =7.2 Hz, 1 H) 5.54 (s, 2 H) 6.70 (s, 1 H) 6.81 (br d, *J* =14.0 Hz, 2 H) 7.18 (d, *J* =10.0 Hz, 1 H) 7.70 (m, 1 H) 7.76 (m, 3 H) 7.92 (m, 2 H) 8.09 (t, *J* =2.0 Hz, 1 H) 8.67 (m, 2 H) 8.91 (d, *J* =8.4 Hz, 1 H) ; LC/MS (ESI) m/z: 535.4 $[M+H]^+$ |

**Example 347: N-[(1R)-1-[3-amino-5-(trifluoromethyl)phenyl]ethyl]-1-[3-(1-methyltetrazol-5-yl)phenyl]-6-oxo-pyridazine-3-carboxamide**

Step 1: Synthesis of 1-[3-(1-methyltetrazol-5-yl)phenyl]-6-oxo-pyridazine-3-carboxylic acid

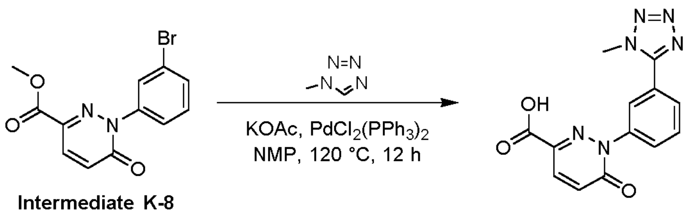


A mixture of Intermediate K-8 (100 mg, 323.59 μmol), 1-methyltetrazole (68.00 mg, 808.76 μmol), KOAc (47.62 mg, 485.26 μmol) and $Pd(PPh_3)_2Cl_2$ (22.71 mg, 32.35 μmol) in NMP (3 mL) was degassed and purged with $N_2$ for 3 times, and then the mixture was stirred at 120 °C for 12 hours under $N_2$ atmosphere. The reaction mixture was poured into water (20 mL), adjusted to pH= 3-4 with 1 N aq. HCl and extracted with EtOAc (30 mL×3). The combined organic layer was washed with brine (30 mL×2), dried over anhydrous $Na_2SO_4$ and concentrated under reduced pressure to obtain a crude product. The product was purified by prep-HPLC (column: C18-6 100*30mm*5μm; mobile phase: [water(FA)-ACN];B%: 0%-30%,15min), most of MeCN was removed under reduced pressure, and the remaining solvent was completely removed by lyophilization to give (1-[3-(1-methyltetrazol-5-yl)phenyl]-6-oxo-pyridazine-3-carboxylic acid (13 mg, 12.26% yield) as a white solid. MS (ESI) m/z = 299.1 $[M+H]^+$.

Step 2: Synthesis of N-[(lR)-1-[3-amino-5-(trifluoromethyl)phenyl]ethyl]-1-[3-(1-methyltetrazol-5-yl)phenyl]-6-oxo-pyridazine-3-carboxamide

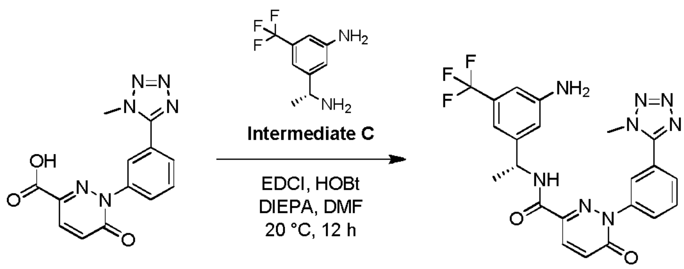


347

To a solution of 1-[3-(1-methyltetrazol-5-yl)phenyl]-6-oxo-pyridazine-3-carboxylic acid (13 mg, 43.59 μmol) and Intermediate C (10.49 mg, 43.59 μmol) in DMF (3 mL), DIEA (16.90 mg, 130.76 μmol, 22.78 uL), EDCI (10.03 mg, 52.30 μmol) and HOBt (7.07 mg, 52.30 μmol) were added, and the mixture was stirred at 20 °C for 12 hours. The reaction mixture was poured into water (20 mL) and extracted with EtOAc (30 mL × 3). The combined organic layer was washed with brine (20 mL×2), dried over anhydrous $Na_2SO_4$ and concentrated under reduced pressure to obtain a crude product. The product was purified by silica gel column chromatography (3% MeOH in DCM) and prep-HPLC (column: Phenomenex C18 75*30mm*3μm; mobile phase: [water($NH_3H_2O$+$NH_4HCO_3$)-ACN];B%: 18%-48%,10min ), the MeCN solvent was removed by concentration under reduced pressure, and the product was lyophilized to give the compound of Example 347 (3.6 mg, 17.05% yield) as a white solid. $^1H$ NMR (400MHz, DMSO-$d_6$) δ = 8.91 (d, *J* = 8.4 Hz, 1H), 8.20 (s, 1H), 8.05 -7.89 (m, 3H), 7.85 - 7.77 (m, 1H), 7.20 (d, *J* = 9.6 Hz, 1H), 6.80 (br d, *J* = 11.2 Hz, 2H), 6.70 (s, 1H), 5.55 (s, 2H), 5.05 (quin, *J* = 7.2 Hz, 1H), 4.21 (s, 3H), 1.44 (d, *J* = 7.2 Hz, 3H); MS (ESI) m/z = 485.3 $[M+H]^+$.

**Example 348 to Example 350**

The compounds shown in the following table were prepared in the same manner as in Example 347.

[Table 23]

| No. | Structure/Name | Spectral Data |
|---|---|---|
| 348 | 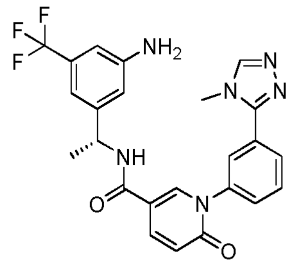 N-[(1R)-1-[3-amino-5-(trifluoromethyl)phenyl]ethyl]-1-[3-(4-methyl-1,2,4-triazol-3-yl)phenyl]-6-oxo-pyridine-3- carboxamide | $^1$H NMR (400 MHz, DMSO-$d_6$) δ = 8.62 (s, 1H), 8.59 (d, *J* = 8.0 Hz, 1H), 8.44 (d, *J* = 2.4 Hz, 1H), 7.98 (dd, *J* = 2.4, 9.6 Hz, 1H), 7.90 - 7.86 (m, 2H), 7.76 - 7.71 (m, 1H), 7.69 - 7.65 (m, 1H), 6.76 (s, 2H), 6.69 (s, 1H), 6.57 (d, *J* = 9.6 Hz, 1H), 5.55 (s, 2H), 5.01 (t, *J* = 7.2 Hz, 1H), 3.78 (s, 3H), 1.40 (d, *J* = 7.2 Hz, 3H) ; LC/MS (ESI) m/z: 483.4 [M+H]$^+$ |
| 349 | 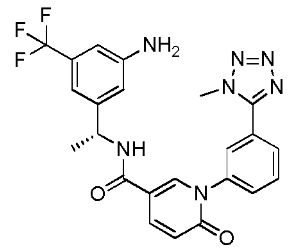 N-[(1R)-1-[3-amino-5-(trifluoromethyl)phenyl]ethyl]-1-[3-(1-methyltetrazol-5-yl)phenyl]-6-oxo-pyridine-3-carboxamide | $^1$H NMR (400 MHz, DMSO-$d_6$) δ = 8.59 (d, *J* = 8.0 Hz, 1H), 8.44 (d, *J* = 2.4 Hz, 1H), 8.04 - 7.95 (m, 3H), 7.85 - 7.77 (m, 2H), 6.76 (s, 2H), 6.69 (s, 1H), 6.58 (d, *J* = 9.6 Hz, 1H), 5.55 (s, 2H), 5.02 (quin, *J* = 7.2 Hz, 1H), 4.20 (s, 3H), 1.40 (d, *J* = 6.8 Hz, 3H) ; LC/MS (ESI) m/z: 484.4 [M+H]$^+$ |
| 350 | 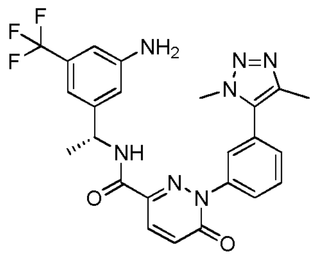 N-[(1R)-1-[3-amino-5-(trifluoromethyl)phenyl]ethyl]-1-[3-(3,5-dimethyltriazol-4-yl)phenyl]-6-oxo-pyridazine-3-carboxamide | $^1$H NMR (400 MHz, DMSO-$d_6$) δ = 8.88 (d, *J* = 8.4 Hz, 1H), 7.90 (d, *J* = 9.6 Hz, 1H), 7.88 - 7.81 (m, 2H), 7.72 (t, *J* = 7.6 Hz, 1H), 7.63 - 7.58 (m, 1H), 7.18 (d, *J* = 10.0 Hz, 1H), 6.80 (d, *J* = 11.2 Hz, 2H), 6.70 (s, 1H), 5.55 (s, 2H), 5.04 (quin, *J* = 7.2 Hz, 1H), 3.98 (s, 3H), 2.27 (s, 3H), 1.45 (d, *J* = 7.2 Hz, 3H) ; LC/MS (ESI) m/z: 498.4 [M+H]$^+$ |

**Example 351: N-[(1R)-1-[3-amino-5-(trifluoromethyl)phenyl]ethyl]-1-[3-(1-methyl-6-oxo-2-pyridyl)phenyl]-6-oxo-pyridazine-3-carboxamide**

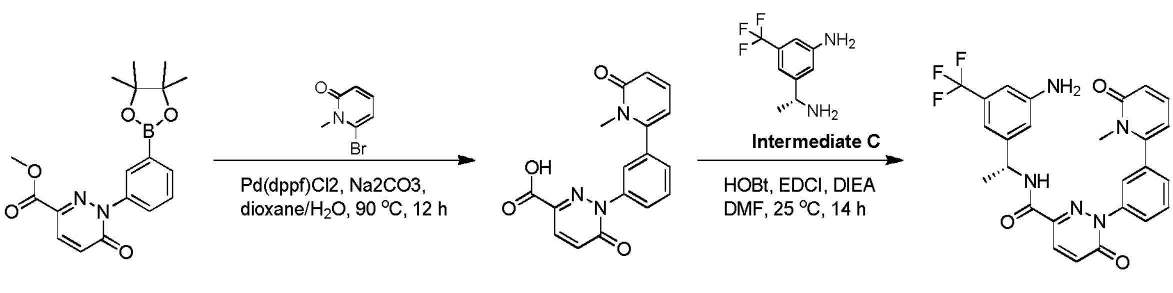


351

The compound of Example 351 was prepared in a similar manner to Steps 2 and 4 of Example 318. $^1$H NMR (400 MHz, DMSO-$d_6$) δ = 8.89 (d, *J* = 8.4 Hz, 1H), 7.81-7.95 (m, 3H), 7.69 (t, *J* = 7.6 Hz, 1H), 7.59 (d, *J* = 7.6 Hz, 1H), 7.48 (dd, *J* = 9.2, 7.2 Hz, 1H), 7.18 (d, *J* = 9.6 Hz, 1H), 6.81 (d, *J* = 11.2 Hz, 2H), 6.71 (s, 1H), 6.48 (d, *J* = 8.8 Hz, 1H),

6.22 (d, $J$ = 6.8 Hz, 1H), 5.55 (s, 2H), 5.05 (q, $J$ = 7.2 Hz, 1H), 3.31-3.32 (m, 3H), 1.46 (d, $J$ = 7.2 Hz, 3H) ; LC/MS (ESI) m/z: 510.2 $[M+H]^+$

**Example 352: N-[(1R)-1-[3-(1,1-difluoro-2-hydroxy-ethyl)-2-fluoro-phenyl]ethyl]-1-(2,4-difluorophenyl)-6-oxo-pyridazine-3-carboxamide**

Step 1: Synthesis of dimethyl (2E)-2-[(2,4-difluorophenyl)hydrazono]-3-oxo-glutarate

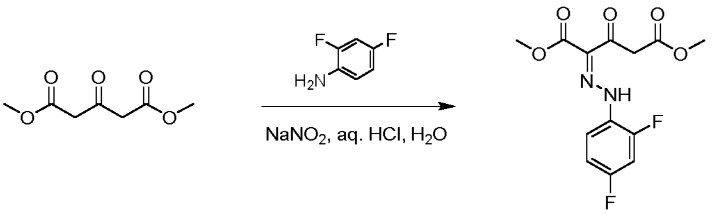


A mixed solutionof 10 M HCl (10 mL) and water (20 mL), and 2,4-difluoroaniline (2.58 g, 19.98 mmol) were added to a solution of $NaNO_2$ (1.38 g, 19.98 mmol) in water (15 mL) at 5 °C. The reaction mixture was poured into a mixed solution of dimethyl 3-oxopentanedioate (3.48 g, 19.98 mmol, 2.88 mL) in EtOH (12 mL) and NaOAc (12.00 g, 146.25 mmol) in water (40 mL). The mixture was stirred at 25 °C for 0.5 hour. The reaction mixture was poured into water (100 mL) and extracted with EtOAc (80 mL×3). The organic layer was washed with brine (50 mL × 2), dried over anhydrous $Na_2SO_4$ and filtered under reduced pressure. The crude product of dimethyl (2E)-2-[(2,4-difluorophenyl)hydrazono]-3-oxo-glutarate (6.2 g, 83.61% yield) as yellow oil was used in the next step without further purification. MS (ESI) m/z = 315.0 $[M+H]^+$).

Step 2: Synthesis of methyl 1-(2,4-difluorophenyl)-4-hydroxy-6-oxo-pyridazine-3-carboxylate

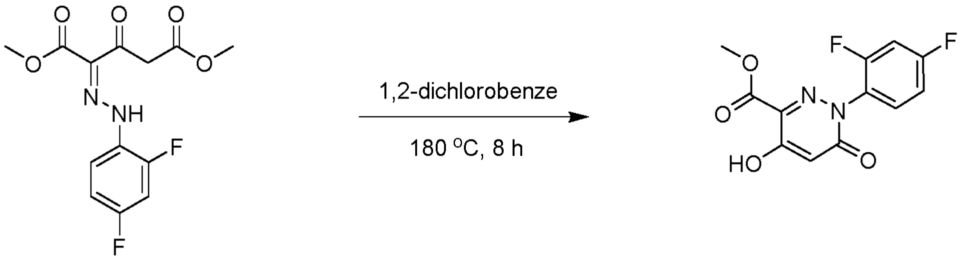


A solution of dimethyl (2E)-2-[(2,4-difluorophenyl)hydrazono]-3-oxo-glutarate (6.2 g, 19.73 mmol) in 1,2-dichlorobenzene (62 mL) was stirred at 175 °C for 6 hours under air. The product was purified by silica gel column chromatography (25% EtOAc in PE) to give methyl 1-(2,4-difluorophenyl)-4-hydroxy-6-oxo-pyridazine-3-carboxylic acid (3.3 g, 59.01% yield) as yellow oil. $^1H$ NMR (400 MHz, DMSO-$d_6$) $\delta$ ppm 12.21 (br s, 1H), 7.63 (dt, J = 6.0, 8.8 Hz, 1H), 7.53 - 7.48 (m, 1H), 7.28 -7.23 (m, 1H), 6.22 (s, 1H), 3.83 (s, 3H); MS (ESI) m/z = 282.9 $[M+H]^+$.

Step 3: Synthesis of methyl 1-(2,4-difluorophenyl)-6-oxo-4-(trifluoromethylsulfonyloxy)pyridazine-3-carboxylate

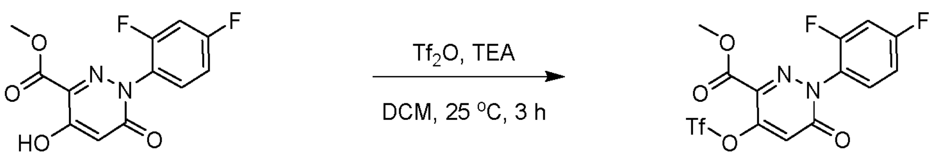


To a solution of methyl 1-(2,4-difluorophenyl)-4-hydroxy-6-oxo-pyridazine-3-carboxylic acid (500 mg, 1.77

mmol) in DCM (30 mL) was added a mixed solution of $Tf_2O$ (1.25 g, 4.43 mmol, 730.84 uL) and DCM (20 mL) at -70 °C dropwise, followed by stirring at 25°C for 3 hours under $N_2$ atmosphere. The reaction mixture was poured into water (30 mL) and extracted with EtOAc (30 mL×3). The organic layer was washed with brine (30 mL), dried over anhydrous $Na_2SO_4$ and filtered under reduced pressure to obtain a crude product. The crude product was purified by silica gel column chromatography (20% EtOAc in PE) to give methyl 1-(2,4-difluorophenyl)-6-oxo-4-(trifluoromethylsulfonyloxy)pyridazine-3-carboxylate (600 mg, 76.04% yield) as colorless oil. MS (ESI) m/z = 414.9 $[M+H]^+$.

Step 4: Synthesis of methyl 1-(2,4-difluorophenyl)-6-oxopyridazine-3-carboxylate

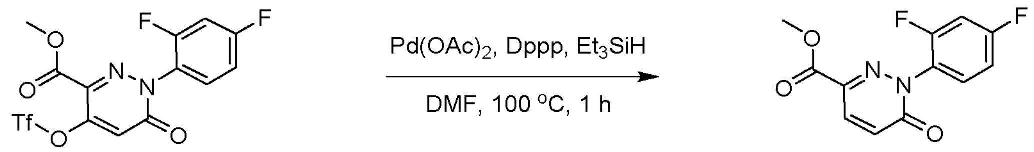


A solution of methyl 1-(2,4-difluorophenyl)-6-oxo-4-(trifluoromethylsulfonyloxy)pyridazine-3-carboxylate (250 mg, 603.48 μmol), $Pd(OAc)_2$ (20.32 mg, 90.52 μmol), DPPP (74.67 mg, 181.05 μmol) and $Et_3SiH$ (91.22 mg, 784.53 μmol, 125.31 μL) in DMF (3 mL) was degassed and purged with $N_2$ for 3 times, and then the mixture was stirred at 100 °C for 1 hour under $N_2$ atmosphere. The reaction mixture was poured into water (30 mL) and extracted with EtOAc (30 mL×3). The organic layer was washed with brine (30 mL), dried over anhydrous $Na_2SO_4$ and filtered under reduced pressure to obtain a crude product. The crude product was purified by silica gel column chromatography (20% EtOAc in PE) to give methyl 1-(2,4-difluorophenyl)-6-oxopyridazine-3-carboxylate (70 mg, 39.83% yield) as colorless oil. MS (ESI) m/z = 247.0 $[M+H]^+$).

Step 5: 1-(2,4-difluorophenyl)-6-oxo-pyridazine-3-carboxylic acid

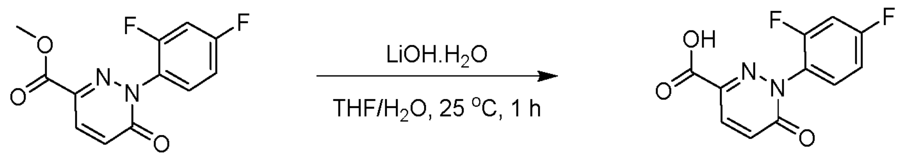


A solution of methyl 1-(2,4-difluorophenyl)-6-oxo-pyridazine-3-carboxylate (70 mg, 262.96 μmol) and $LiOH{\cdot}H_2O$ (33.10 mg, 788.88 μmol) in THF (2 mL) and $H_2O$ (1 mL) was stirred at 25 °C for 1 hour under air. The reaction mixture was adjusted to pH = 3-4 with 1 N HCl and then extracted with EtOAc (30 mL×3). The organic layer was washed with water (30 mL) and brine (30 mL), dried over anhydrous $Na_2SO_4$ and filtered under reduced pressure to give 1-(2,4-difluorophenyl)-6-oxo-pyridazine-3-carboxylic acid (60 mg, 77.10% yield) as a crude product of a white solid, which was used in the next step without further purification. MS (ESI) m/z = 253 $[M+H]^+$.

Step 6: N-[(1R)-1-[3-(1,1-difluoro-2-hydroxy-ethyl)-2-fluoro-phenyl]ethyl]-1-(2,4-difluorophenyl)-6-oxo-pyridazine-3-carboxamide

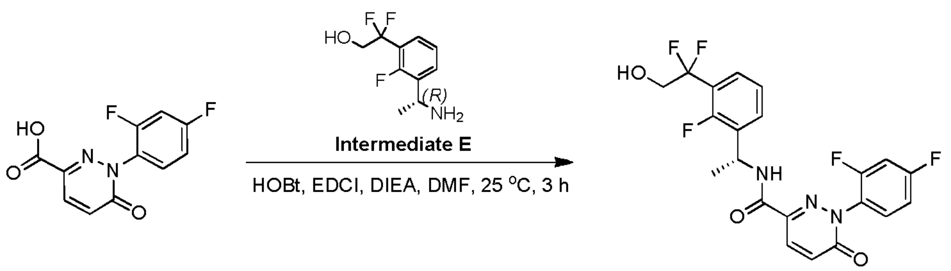


352

A solution of 1-(2,4-difluorophenyl)-6-oxo-pyridazine-3-carboxylic acid (30 mg, 118.97 μmol), Intermediate E (31.29 mg, 122.40 μmol, HCl), EDCI (45.61 mg, 237.93 μmol), HOBt (32.15 mg, 237.93 μmol) and DIEA (46.13 mg, 356.90 μmol, 62.17 μL) in DMF (2 mL) was degassed and purged with $N_2$ for 3 times, and then the mixture was stirred at 25 °C for 3 hours under $N_2$ atmosphere. The reaction mixture was poured into water (30 mL) and extracted with EtOAc (30 mL×3). The organic layer was washed with brine (30 mL), dried over anhydrous $Na_2SO_4$ and filtered under reduced pressure to obtain a crude product. The product was purified by silica gel column chromatography (20% EtOAc in PE) to obtain a residue, which was purified by prep-HPLC (column: Phenomenex C18 75*30mm*3μm; mobile phase: [water($NH_3H_2O$+$NH_4HCO_3$)-ACN];B%: 28%-58%,11min). Most of $CH_3CN$ was removed under reduced pressure, and the remaining solvent was removed by lyophilization to give the compound of Example 352 (3.4 mg, 6.21% yield) as a white solid. $^1$H NMR (400 MHz, DMSO-$d_6$) δ ppm 9.03 (d, J = 8.0 Hz, 1H), 7.92 (d, J = 10.0 Hz, 1H), 7.79 (dt, J = 6.0, 8.8 Hz, 1H), 7.65 - 7.51 (m, 2H), 7.43 (t, J = 6.8 Hz, 1H), 7.34 (t, J = 8.8 Hz, 1H), 7.30 - 7.25 (m, 1H), 7.18 (d, J = 9.6 Hz, 1H), 5.71 (t, J = 6.4 Hz, 1H), 5.40 (quin, J = 7.2 Hz, 1H), 3.91 (dt, J = 6.4, 14.4 Hz, 2H), 1.46 (d, J = 7.2 Hz, 3H); MS (ESI) m/z = 454.1 $[M+H]^+$.

**Example 353 to Example 362**

The compounds shown in the following table were obtained in the same manner as in Example 352 by using starting materials and intermediates corresponding to the respective structures of the desired compounds, which were prepared based on Steps 1 to 3 of Preparation Example 8 and Step 2 of Preparation Example 29.

[Table 24]

| No. | Structure/Name | Spectral Data |
|---|---|---|
| 353 | 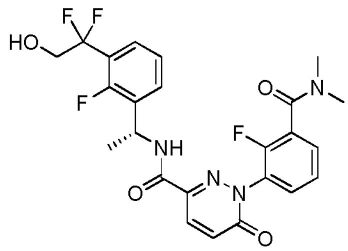 N-[(1R)-1-[3-(1,1-difluoro-2-hydroxy-ethyl)-2-fluoro-phenyl]ethyl]-1-[3-(dimethylcarbamoyl)-2-fluoro-phenyl]-6-oxo-pyridazine-3- carboxamide | $^1$H NMR (400MHz, DMSO-$d_6$) δ = 9.06 (d, *J* = 8.0 Hz, 1H), 7.93 (d, *J* = 10.0 Hz, 1H), 7.82 - 7.74 (m, 1H), 7.62 (br t, *J* = 7.2 Hz, 1H), 7.59 - 7.55 (m, 1H), 7.52 - 7.46 (m, 1H), 7.43 (br t, *J* = 6.8 Hz, 1H), 7.31 - 7.24 (m, 1H), 7.19 (d, *J* = 9.6 Hz, 1H), 5.70 (t, *J* = 6.4 Hz, 1H), 5.40 (quin, *J* = 7.2 Hz, 1H), 3.91 (dt, *J* = 6.4, 14.4 Hz, 2H), 3.03 (s, 3H), 2.90 (s, 3H), 1.46 (d, *J* = 7.2 Hz, 3H); MS (ESI) m/z = 507.4 $[M+H]^+$. |
| 354 | 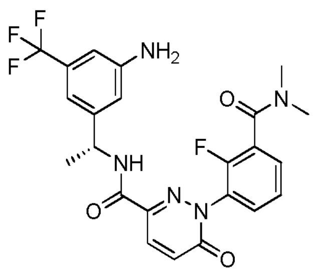 N-[(1R)-1-[3-amino-5-(trifluoromethyl)phenyl]ethyl]-1-[3-(dimethylcarbamoyl)-2-fluoro-phenyl]-6-oxo-pyridazine-3-carboxamide | $^1$H NMR (400 MHz, DMSO-$d_6$) δ = 8.94 (d, *J* = 8.4 Hz, 1H), 7.94 (d, *J* = 10.0 Hz, 1H), 7.84 - 7.69 (m, 1H), 7.61-7.52 (m, 1H), 7.51-7.42 (m, 1H), 7.19 (d, *J* = 9.6 Hz, 1H), 6.79 (br d, *J* = 12.8 Hz, 2H), 6.70 (s, 1H), 5.53 (s, 2H), 5.02 (quin, *J* = 7.2 Hz, 1H), 3.02 (s, 3H), 2.89 (s, 3H), 1.43 (d, *J* = 7.2 Hz, 3H); MS (ESI) m/z = 492.3 $[M+H]^+$. |
| 355 | 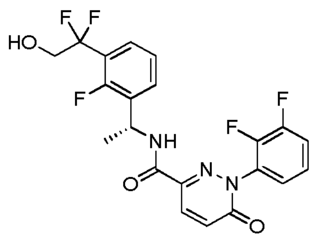 N-[(1R)-1-[3-(1,1-difluoro-2-hydroxy-ethyl)-2-fluoro-phenyl]ethyl]-1-(2,3-difluorophenyl)-6-oxo-pyridazine-3-carboxamide | $^1$H NMR (400 MHz, DMSO-$d_6$) δ = 9.05 *(d, J* = 8.0 Hz, 1H), 7.93 (d, *J* = 10.0 Hz, 1H), 7.71 -7.54 (m, 3H), 7.49 - 7.40 (m, 2H), 7.30 - 7.24 (m, 1H), 7.21 (d, *J* = 9.6 Hz, 1H), 5.71 (br s, 1H), 5.40 (quin, *J* = 7.2 Hz, 1H), 3.97 - 3.86 (m, 2H), 1.46 (d, *J* = 6.8 Hz, 3H) LC/MS (ESI) m/z: 454.3 $[M+H]^+$ |

(continued)

| No. | Structure/Name | Spectral Data |
|---|---|---|
| 356 | 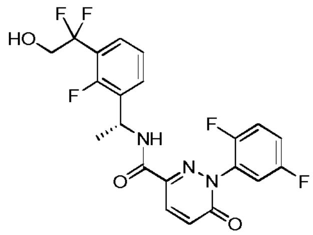 N-[(1R)-1-[3-(1,1-difluoro-2-hydroxy-ethyl)-2-fluoro-phenyl]ethyl]-1-(2,5-difluorophenyl)-6-oxo-pyridazine-3-carboxamide | $^1$H NMR (400 MHz, DMSO-$d_6$) δ = 9.04 (d, *J* = 8.0 Hz, 1H), 7.92 (d, *J* = 10.0 Hz, 1H), 7.72 (ddd, *J* = 3.2, 5.6, 8.4 Hz, 1H), 7.62 (br t, *J* = 6.8 Hz, 1H), 7.58 - 7.52 (m, 1H), 7.51 - 7.46 (m, 1H), 7.46 - 7.41 (m, 1H), 7.30 - 7.25 (m, 1H), 7.19 (d, *J* = 9.6 Hz, 1H), 5.71 (br t, *J* = 6.0 Hz, 1H), 5.40 (quin, *J* = 7.2 Hz, 1H), 3.91 (dt, *J* = 5.6, 14.4 Hz, 2H), 1.47 (d, *J* = 7.2 Hz, 3H); LC/MS (ESI) m/z: 454.3 [M+H]$^+$ |
| 357 | 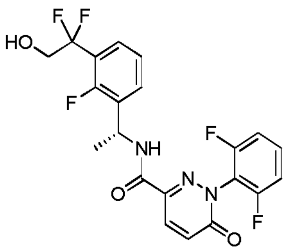 N-[(1R)-1-[3-(1,1-difluoro-2-hydroxy-ethyl)-2-fluoro-phenyl]ethyl]-1-(2,6-difluorophenyl)-6-oxo-pyridazine-3-carboxamide | $^1$H NMR (400 MHz, DMSO-$d_6$) δ = 9.11 (br d, *J* = 8.0 Hz, 1H), 7.97 (d, *J* =10.0 Hz, 1H), 7.74 - 7.65 (m, 1H), 7.60 (br t, *J* = 7.2 Hz, 1H), 7.42 (br t, *J* = 8.4 Hz, 3H), 7.31 - 7.22 (m, 2H), 5.70 (t, *J* = 6.4 Hz, 1H), 5.39 (quin, *J* = 7.2 Hz, 1H), 3.91 (dt, *J* = 6.4, 14.4 Hz, 2H), 1.45 (d, *J* = 7.2 Hz, 3H) LC/MS (ESI) m/z: 454.3 [M+H]$^+$ |
| 358 | 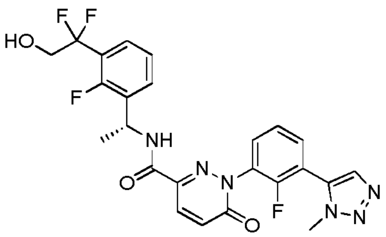 N-[(1R)-1-[3-(1,1-difluoro-2-hydroxy-ethyl)-2-fluoro-phenyl]ethyl]-1-[2-fluoro-3-(3-methyltriazol-4-yl) phenyl]-6-oxo-pyridazine-3- carboxamide | $^1$H NMR (400 MHz, DMSO-$d_6$) ) δ = 9.06 (d, *J* = 8.0 Hz, 1H), 8.00 (s, 1H), 7.97 - 7.85 (m, 2H), 7.83 - 7.75 (m, 1H), 7.66 - 7.57 (m, 2H), 7.46 - 7.40 (m, 1H), 7.30 - 7.24 (m, 1H), 7.21 (d, *J* = 9.6 Hz, 1H), 5.72 (br s, 1H), 5.41 (quin, *J* = 7.2 Hz, 1H), 4.04 (s, 3H), 3.91 (t, *J* = 14.4 Hz, 2H), 1.47 (d, *J* = 7.2 Hz, 3H); LC/MS (ESI) m/z: 517.4 [M+H]$^+$ |
| 359 | 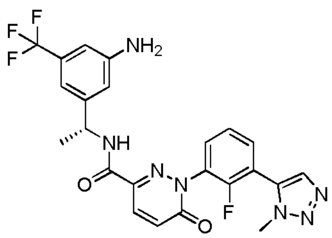 N-[(1R)-1-[3-amino-5-(trifluoromethyl)phenyl]ethyl]-1-[2-fluoro-3-(3-methyltriazol-4-yl)phenyl]-6-oxo-pyridazine-3- carboxamide | $^1$H NMR (400 MHz, DMSO-$d_6$) δ = 8.95 (d, *J* = 8.4 Hz, 1H), 7.99 (s, 1H), 7.96 (d, *J* = 10.0 Hz, 1H), 7.93 - 7.84 (m, 1H), 7.78 (t, *J* = 6.4 Hz, 1H), 7.64 - 7.56 (m, 1H), 7.21 (d, *J* = 9.6 Hz, 1H), 6.79 (br d, *J* = 13.2 Hz, 2H), 6.70 (s, 1H), 5.55 (s, 2H), 5.03 (quin, *J* = 7.2 Hz, 1H), 4.03 (s, 3H), 1.43 (d, *J* = 7.2 Hz, 3H); LC/MS (ESI) m/z: 502.4 [M+H]$^+$ |

(continued)

| No. | Structure/Name | Spectral Data |
|---|---|---|
| 360 | 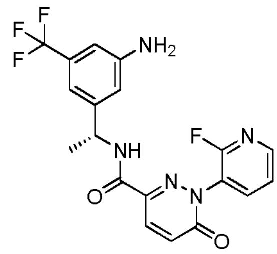 N-[(1R)-1-[3-amino-5-(trifluoromethyl)phenyl]ethyl]-1-(2-fluoro-3-pyridyl)-6-oxo-pyridazine-3-carboxamide | $^1$H NMR (400 MHz, DMSO-$d_6$) $\delta$ = 8.95 (d, *J* = 8.4 Hz, 1H), 8.41 (d, *J* = 4.8 Hz, 1H), 8.32 (ddd, *J* = 1.6, 7.6, 9.6 Hz, 1H), 7.95 (d, *J* = 10.0 Hz, 1H), 7.63 (t, *J* = 6.4 Hz, 1H), 7.21 (d, *J* = 9.6 Hz, 1H), 6.80 (s, 1H), 6.77 (s, 1H), 6.70 (s, 1H), 5.54 (s, 2H), 5.03 (quin, *J* = 7.2 Hz, 1H), 1.42 (d, *J* = 7.2 Hz, 3H); LC/MS (ESI) m/z: 422.3 [M+H]$^+$ |
| 361 | 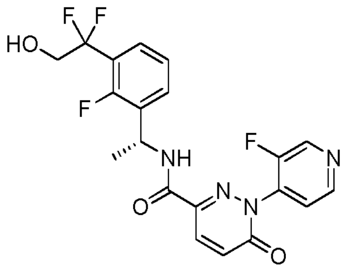 N-[(1R)-1-[3-(1,1-difluoro-2-hydroxy-ethyl)-2-fluoro-phenyl]ethyl]-1-(3-fluoro-4-pyridyl)-6-oxo-pyridazine-3-carboxamide | 1H NMR (400 MHz, DMSO-$d_6$) $\delta$ = 9.08 (d, J = 8.0 Hz, 1H), 8.86 (d, J = 2.0 Hz, 1H), 8.70 (d, J = 5.2 Hz, 1H), 7.94 (d, J = 10.0 Hz, 1H), 7.88 (t, J = 5.6 Hz, 1H), 7.62 (t, J = 6.8 Hz, 1H), 7.43 (t, J = 6.8 Hz, 1H), 7.28 (t, J = 7.6 Hz, 1H), 7.23 (d, J = 9.6 Hz, 1H), 5.72 (t, J = 6.4 Hz, 1H), 5.44 - 5.37 (m, 1H), 3.91 (dt, J = 6.4, 14.4 Hz, 2H), 1.46 (d, J = 7.2 Hz, 3H) ; LC/MS (ESI) m/z: 437.3 [M+H]$^+$ |
| 362 | 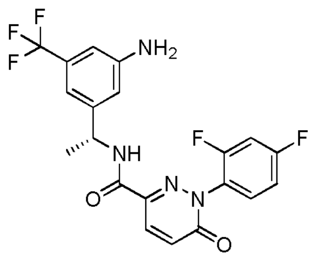 N-[(1R)-1-[3-amino-5-(trifluoromethyl)phenyl]ethyl]-1-(2,4-difluorophenyl)-6-oxo-pyridazine-3-carboxamide | 1H NMR (400 MHz, DMSO-$d_6$) $\delta$ = 8.92 (d, J = 8.4 Hz, 1H), 7.94 (d, J = 10.0 Hz, 1H), 7.77 (dt, J = 6.0, 8.8 Hz, 1H), 7.54 (t, J = 9.6 Hz, 1H), 7.35 - 7.30 (m, 1H), 7.18 (d, J = 10.0 Hz, 1H), 6.80 (s, 1H), 6.77 (s, 1H), 6.70 (s, 1H), 5.54 (br s, 2H), 5.02 (quin, J = 7.2 Hz, 1H), 1.43 (d, J = 7.2 Hz, 3H) ; LC/MS (ESI) m/z: 439.3 [M+H]$^+$ |

**Example 363: N-[(1R)-1-[3-amino-5-(trifluoromethyl)phenyl]ethyl]-1-(2-fluorophenyl)-4-hydroxy-6-oxo-pyridazine-3-carboxamide**

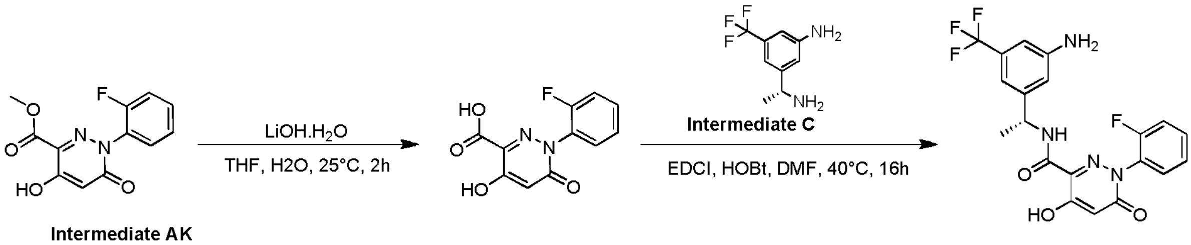


Intermediate AK

363

The compound of Example 363 (1.5 mg, 2.81% yield) was obtained in the same manner as in Example 76 as a white solid. $^1$H NMR (400 MHz, DMSO-$d_6$) $\delta$ = 13.46 - 11.56 (m, 1H), 10.28 - 9.58 (m, 1H), 7.55 - 7.49 (m, 2H), 7.42 - 7.32 (m, 2H), 6.78 (br d, *J* = 10.4 Hz, 2H), 6.71 (s, 1H), 6.00 (br s, 1H), 5.77 - 5.37 (m, 2H), 5.01 (quin, *J* = 7.2 Hz, 1H), 1.41 (d, *J* = 6.8 Hz, 3H); MS (ESI) m/z = 437.1 [M+H]$^+$.

**Example 364: 4-benzyloxy-N-[(1R)-1-[3-(1,1-difluoro-2-hydroxy-ethyl)-2-fluorophenyl]ethyl]-1-(2-fluorophenyl)-6-oxo-pyridazine-3-carboxamide**

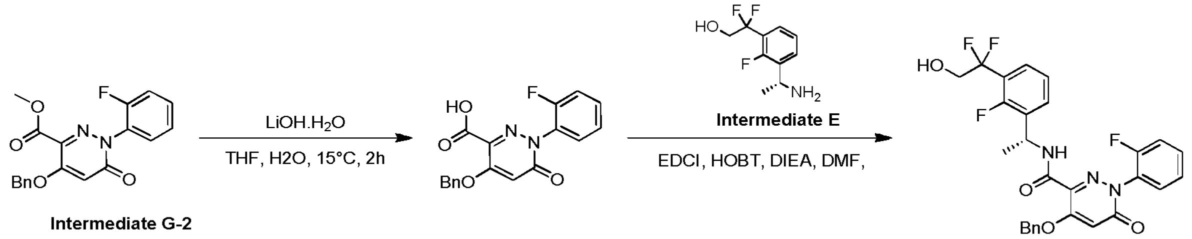


Intermediate G-2

**364**

The compound of Example 364 (2.4 mg, 3.02% yield) was obtained as a white solid in the same manner as in Example 76. $^1H$ NMR (400MHz, DMSO-$d_6$) $\delta$ = 9.25 (d, *J* = 7.6 Hz, 1H), 7.59 - 7.50 (m, 2H), 7.46 - 7.34 (m, 9H), 7.03 (t, *J* = 7.6 Hz, 1H),6.68 (s, 1H), 5.71 (t, *J* = 6.4 Hz, 1H), 5.29 - 5.20 (m, 3H), 3.89 (dt, *J* = 6.4, 14.4 Hz, 2H), 1.36 (d, *J* = 7.2 Hz, 3H); MS (ESI) m/z = 542.4 $[M+H]^+$.

**Example 365: N-[(1R)-1-[3-(1,1-difluoro-2-hydroxy-ethyl)-2-fluoro-phenyl]ethyl]-1-(2-fluorophenyl)-4-hydroxy-6-oxo-pyridazine-3-carboxamide**

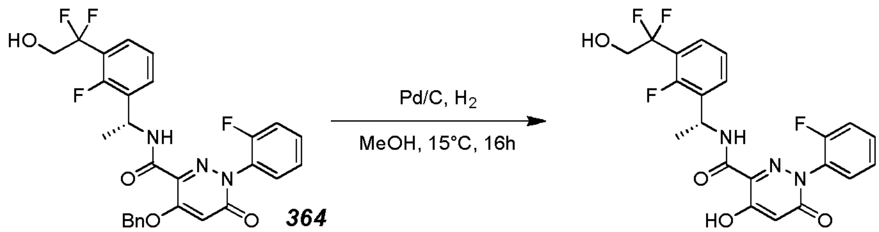


***364***

**365**

The compound of Example 365 (6.1 mg, 24.27% yield) was obtained as a white solid by reacting the compound of Example 364 in the same manner as in Preparation Example 3. $^1H$ NMR (400MHz, DMSO-$d_6$) $\delta$ = 12.66 - 12.16 (m, 1H), 9.50 (br s, 1H), 7.62 (t, *J* = 6.8 Hz, 1H), 7.59 - 7.52 (m, 2H), 7.48 - 7.35 (m, 3H), 7.32 - 7.26 (m, 1H), 6.21 (s, 1H), 5.71 (br t, *J* = 6.0 Hz, 1H), 5.37 (quin, *J* = 7.2 Hz, 1H), 3.91 (dt, *J* = 5.2, 14.2 Hz, 2H), 1.45 (d, *J* = 7.2 Hz, 3H); MS (ESI) m/z = 452.3 $[M+H]^+$.

**Example 366: 4-cyclopropyl-N-[(1R)-1-[3-(1,1-difluoro-2-hydroxy-ethyl)-2-fluoro-phenyl]ethyl]-1-(2-fluoro-4-methoxy-phenyl)-6-oxopyridazine-3-carboxamide**

Step 1: Synthesis of 4-cyclopropyl-1-(2-fluoro-4-methoxy-phenyl)-6-oxo-pyridazine-3-carboxylic acid

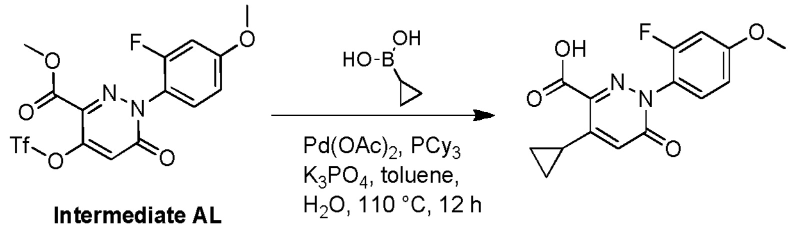


**Intermediate AL**

A mixture of Intermediate AL (300 mg, 703.74 $\mu$mol), cyclopropylboronic acid (120.90 mg, 1.41 mmol), Pd(OAc)$_2$ (23.70 mg, 105.56 $\mu$mol), $K_3PO_4$ (746.90 mg, 3.52 mmol) and P(Cy)$_3$ (59.20 mg, 211.12 $\mu$mol, 68.44 $\mu$L) in toluene (4 mL) and $H_2O$ (0.5 mL) was degassed and purged with $N_2$ for 3 times, and then the mixture was stirred at 100 °C for 12

hours under $N_2$ atmosphere. The reaction mixture was poured into water (20 mL) and extracted with EtOAc (20 mL × 3). The combined organic layer was discarded, and the aqueous layer was adjusted to pH = 3-4 with 1 N aq. HCl and extracted with EtOAc (30 mL × 3). The organic layer was washed with water (30 mL) and brine (30 mL), dried over $Na_2SO_4$, filtered and concentrated under reduced pressure to obtain a crude product. 4-cyclopropyl-1-(2-fluoro-4-methoxy-phenyl)-6-oxo-pyridazine-3-carboxylic acid (cryde, 200 mg, 79.31% yield) as a yellow solid was used in the next step without further purification. MS (ESI) m/z = 304.9 $[M+H]^+$.

Step 2: Synthesis of 4-cyclopropyl-N-[(1R)-1-[3-(1,1-difluoro-2-hydroxy-ethyl)-2-fluoro-phenyl]ethyl]-1-(2-fluoro-4-methoxy-phenyl)-6-oxopyridazine-3-carboxamide

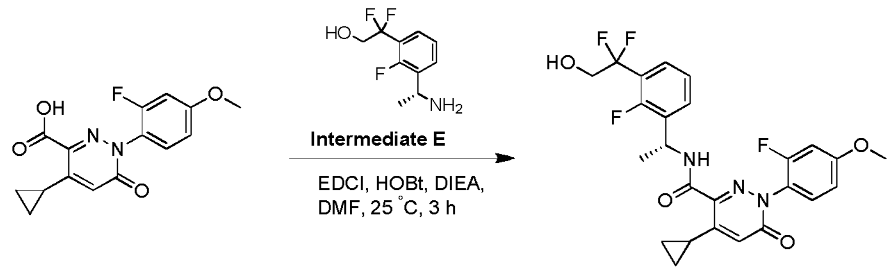


366

The compound of Example 366 (6 mg, 11.83% yield) was obtained in the same manner as in Step 2 of Example 76 as a white solid. $^1$H NMR (400MHz, DMSO-$d_6$) δ = 9.26 (d, *J* = 7.6 Hz, 1H), 7.58 (t, *J* = 6.8 Hz, 1H), 7.51 - 7.40 (m, 2H),7.30 (t, *J* = 7.6 Hz, 1H), 7.04 (dd, *J* = 2.4, 12.0 Hz, 1H), 6.93 (dd, *J* = 2.4, 8.8 Hz, 1H), 6.62 (s, 1H), 5.72 (br s, 1H), 5.33 (quin, *J* = 7.2 Hz, 1H), 3.91 (br t, *J* = 14.4 Hz, 2H), 3.83 (s, 3H), 1.98 - 1.90 (m, 1H), 1.44 (d, *J* = 6.8 Hz, 3H), 1.04 - 0.94 (m, 2H), 0.93 - 0.88 (m, 1H), 0.87 - 0.80 (m, 1H); MS (ESI) m/z = 506.4 $[M+H]^+$.

**Example 367 and Example 368**

The compounds shown in the following table were obtained in the same manner as in Example 366 by using appropriate starting materials and intermediates corresponding to the respective structures of the desired compounds based on Preparation Example 38.

[Table 25]

| No. | Structure/Name | Spectral Data |
|---|---|---|
| 367 | 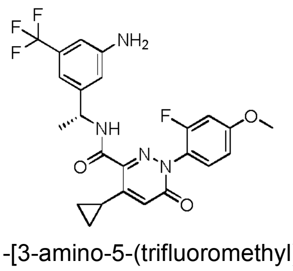 N-[(1R)-1-[3-amino-5-(trifluoromethyl)phenyl] ethyl]-4-cyclopropyl-1-(2-fluoro-4-methoxy-phenyl)-6-oxo-pyridazine-3-carboxamide | $^1$H NMR (400 MHz, DMSO-$d_6$) δ = 9.14 (d, *J* = 8.0 Hz, 1H), 7.45 (t, *J* = 8.8 Hz, 1H), 7.03 (dd, *J* = 2.4, 12.0Hz, 1H), 6.91 (dd, *J* = 2.4, 8.8 Hz, 1H), 6.77 (d, *J* = 8.4 Hz, 2H), 6.71 (s, 1H), 6.61 (s, 1H), 5.58 (s, 2H), 4.97 (quin, *J* = 7.2 Hz,1H), 3.82 (s, 3H), 1.97 - 1.90 (m, 1H), 1.39 (d, *J* = 7.2 Hz, 3H), 1.02 - 0.82 (m, 4H); LC/MS (ESI) m/z: 491.3 $[M+H]^+$ |

(continued)

| No. | Structure/Name | Spectral Data |
|---|---|---|
| 368 | 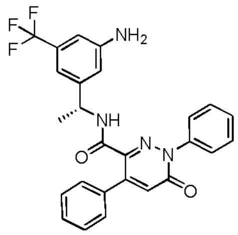<br>N-[(1R)-1-[3-amino-5-(trifluoromethyl)phenyl] ethyl]-6-oxo-1,4-diphenyl-pyridazine-3-carboxamide | $^1$H NMR (400 MHz, DMSO-$d_6$) δ = 9.28 (d, *J* = 8.0 Hz, 1H), 7.67 (d, *J* = 7.6 Hz, 2H), 7.55 (t, *J* = 7.2 Hz, 2H), 7.49 - 7.38 (m, 4H), 7.34 - 7.28 (m, 2H), 7.12 (s, 1H), 6.81 (s, 1H), 6.74 (s, 2H), 5.59 (s, 2H), 4.83 (quin, *J* = 7.2 Hz, 1H), 1.34(d, *J* = 7.2 Hz, 3H); LC/MS (ESI) m/z: 479.3 $[M+H]^+$ |

**Example 369: N-[(1R)-1-[3-amino-5-(trifluoromethyl)phenyl]ethyl]-1-[3-(3-methyltriazol-4-yl)phenyl]-6-oxo-pyridazine-3-carboxamide**

Step 1: Synthesis of tributyl-(3-methyltriazol-4-yl)stannane

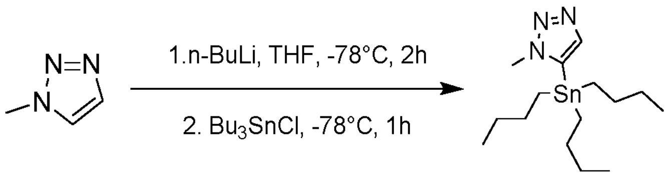


A solution of 1-methyltriazole (3 g, 36.10 mmol) in THF (30 mL) was cooled to -78 °C, followed by the dropwise addition of n-BuLi (2.5 M, 899.66 μL), and the mixture was stirred at - 78 °C for 2 hours, added dropwise with tributyl(chloro)stannane (16.94 g, 52.04 mmol, 14 mL), and then the resulting mixture was stirred at -78°C for 1 hour under $N_2$ atmosphere. The reaction mixture was added with $H_2O$ dropwise slowly at 0°C and extracted with EtOAc (70 mL×3). The combined organic layer was washed with brine (50×2 mL), dried over $Na_2SO_4$ and concentrated under reduced pressure to obtain a crude product of tributyl-(3-methyltriazol-4-yl)stannane (17.35 g, 34.97 mmol, 96.85% yield) as light yellow oil. $^1$H NMR (400 MHz, DMSO-$d_6$) δ = 7.58 (br s, 1H), 4.06 - 4.00 (m, 3H), 1.50 - 1.44 (m, 4H), 1.28 (br d, *J* = 7.2 Hz, 8H), 1.19 - 1.12 (m, 6H), 0.87 - 0.82 (m, 9H).

Step 2: Synthesis of methyl 1-(3-bromophenyl)-6-oxo-pyridazine-3-carboxylate

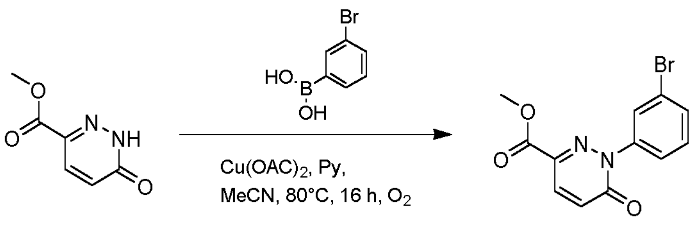


**Intermediate K-8**

A mixture of (3-bromophenyl)boronic acid (15.64 g, 77.86 mmol), methyl 6-oxo-1H-pyridazine-3-carboxylate (10 g, 64.88 mmol), $Cu(OAc)_2$ (3.54 g, 19.46 mmol) and Py (33.36 g, 421.74 mmol, 34.04 mL) in acetonitrile (200 mL) was degassed and purged with $O_2$ for 3 times, and then stirred at 80 °C for 16 hours under $O_2$ atmosphere. The reaction mixture was poured into distilled water (100 mL) and extracted with EtOAc (100 mL×3). The combined organic layer was washed with brine (100 mL), dried over $Na_2SO_4$ and concentrated under reduced pressure to obtain a crude product,

which was purified by silica gel column chromatography (100% DCM) to give methyl 1-(3-bromophenyl)-6-oxo-pyridazine-3-carboxylate (17.5 g, 51.96 mmol, 80.09% yield) as a white solid. 1H NMR (400 MHz, DMSO-$d_6$) δ = 7.93 (d, J = 9.6 Hz, 1H), 7.83 (t, J = 2.0 Hz, 1H), 7.71 (d, J = 8.0 Hz, 1H),7.60 (d, J = 8.8 Hz, 1H), 7.51 (t, J = 8.0 Hz, 1H), 7.17 (d, J = 10.0 Hz, 1H), 3.87 (s, 3H). LC/MS (ESI) m/z = 310.8 $[M+H]^+$

Step 3: Synthesis of 1-[3-(3-methyltriazol-4-yl)phenyl]-6-oxopyridazine-3-carboxylic acid

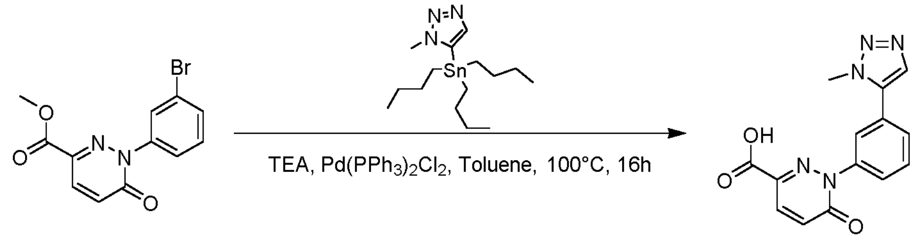


**Intermediate K-8**

To Intermediate K-8 (8.05 g, 26.04 mmol) in toluene (100 mL), tributyl-(3-methyltriazol-4-yl)stannane (14.54 g, 39.06 mmol), TEA (2.64 g, 26.04 mmol, 3.62 mL) and $Pd(PPh_3)_2Cl_2$ (1.83 g, 2.60 mmol) were added. The mixture was degassed and purged with $N_2$ for 3 times, and then stirred at 100 °C for 16 hours under $N_2$ atmosphere. The reaction mixture was poured into water (200 mL) and extracted with EtOAc (100 mL×4). The combined organic layer was washed with brine (50 mL×3), dried over anhydrous $Na_2SO_4$ and concentrated under reduced pressure to obtain a crude product. The product was purified by silica gel column chromatography (8% MeOH in DCM) to give 1-[3-(3-methyltriazol-4-yl)phenyl]-6-oxo-pyridazine-3-carboxylic acid (2.96 g, 9.75 mmol, 37.45% yield) as yellow oil. LC/MS (ESI) m/z = 298.0 $[M+H]^+$

Step 4: Synthesis of N-[(IR)-1-[3-amino-5-(trifluoromethyl)phenyl]ethyl]-1-[3-(3-methyltriazol-4-yl)phenyl]-6-oxo-pyridazine-3-carboxamide

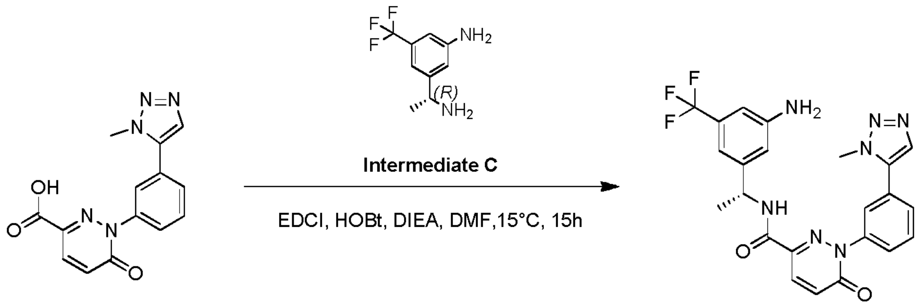


**369**

To a solution of 1-[3-(3-methyltriazol-4-yl)phenyl]-6-oxo-pyridazine-3-carboxylic acid (2.96 g, 9.96 mmol) and Intermediate C (2.40 g, 9.96 mmol) in DMF (40 mL), EDCI (2.86 g, 14.94 mmol), DIEA (3.86 g, 29.87 mmol, 5.20 mL) and HOBt (2.02 g, 14.94 mmol) were added. The mixture was degassed and purged with $N_2$ for 3 times, and then stirred at 15 °C for 15 hours under $N_2$ atmosphere. The reaction mixture was poured into distilled water (100 mL) and extracted with EtOAc (50 mL×6). The combined organic layer was washed with brine (30 mL×5), dried over anhydrous $Na_2SO_4$ and concentrated under reduced pressure to obtain a crude product as yellow oil. The product was purified by silica gel column chromatography (75% EtOAc in PE) to give the compound of Example 369 (1.02 g, 21.19% yield) as a light yellow solid. $^1H$ NMR (400 MHz, DMSO-$d_6$) δ = 8.91 (d, J = 8.4 Hz, 1H), 7.99 (s, 1H), 7.96 (s, 1H), 7.92 (d, J = 9.6 Hz, 1H), 7.87 - 7.80 (m,1H), 7.75 - 7.68 (m, 2H), 7.18 (d, J = 9.6 Hz, 1H), 6.80 (br d, J = 13.2 Hz, 2H), 6.70 (s, 1H), 5.56 (s, 2H), 5.04 (quin, J = 7.2 Hz, 1H), 4.12 (s, 3H), 1.44 (d, J = 7.2 Hz, 3H). LC/MS (ESI) m/z = 484.4 $[M+H]^+$.

**Example 370: N-[(1R)-1-[3-(1,1-difluoro-2-hydroxy-ethyl)-2-fluoro-phenyl]ethyl]-1-[3-(3-methyltriazol-4-yl)phenyl]-6-oxo-pyridazine-3-carboxamide**

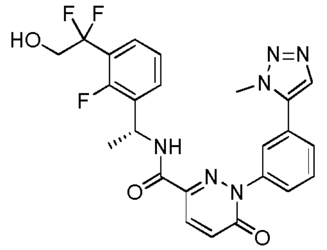

**370**

The compound of Example 370 was prepared in the same manner as in Example 369 by using Intermediate E. $^1H$ NMR (400 MHz, DMSO-$d_6$) $\delta$ = 9.02 (d, J = 8.0 Hz, 1H), 8.01 (s, 1H), 7.97 (s, 1H), 7.90 (d, J = 9.6 Hz, 1H), 7.87 - 7.81 (m,1H), 7.76 - 7.71 (m, 2H), 7.64 (br t, J = 7.2 Hz, 1H), 7.44 (br t, J = 6.8 Hz, 1H), 7.31 - 7.24 (m, 1H), 7.18 (d, J = 9.6 Hz, 1H),5.72 (t, J = 6.0 Hz, 1H), 5.41 (br t, J = 7.6 Hz, 1H), 4.14 (s, 3H), 3.91 (dt, J = 5.6, 14.0 Hz, 2H), 1.48 (d, J = 7.2 Hz, 3H) ; LC/MS (ESI) m/z: 499.4 $[M+H]^+$.

**Example 371: N-[(1R)-1-[3-amino-5-(trifluoromethyl)phenyl]ethyl]-1-[2-fluoro-5-(3-methyltriazol-4-yl)phenyl]-6-oxo-pyridazine-3-carboxamide**

Step 1: Synthesis of methyl 1-(2-fluoro-5-(1-methyl-1H-1,2,3-triazol-5-yl)phenyl)-6-oxo-1,6-dihydropyridazine-3-carboxylate

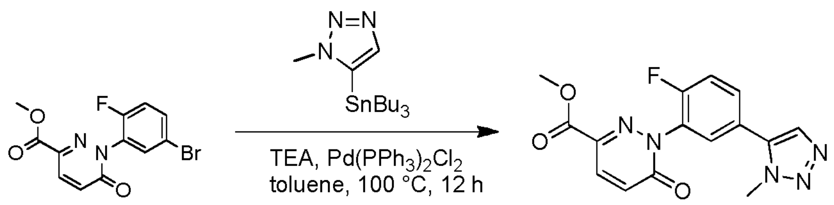


**Intermediate K-21**

Methyl 1-(2-fluoro-5-(1-methyl-1H-1,2,3-triazol-5-yl)phenyl)-6-oxo-1,6-dihydropyridazine-3-carboxylate was obtained by using a tributylstannyl derivative as an intermediate and changing the reagents and solvents accordingly in Step 2 of Example 318.

Steps 2 and 3: Synthesis of N-[(IR)-1-[3-amino-5-(trifluoromethyl)phenyl]ethyl]-1-[2-fluoro-5-(3-methyltriazol-4-yl)phenyl]-6-oxo-pyridazine-3-carboxamide

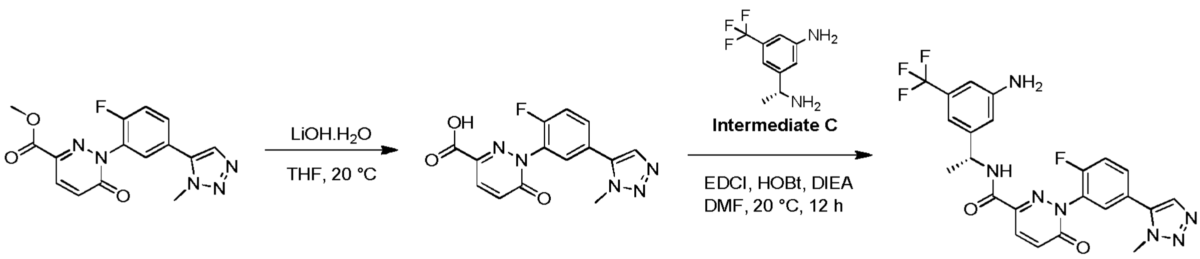


**371**

The compound of Example 371 (4.9 mg, 9.50 μmol, 9.98% yield) was obtained in the same manner as in Example 76 as a white solid. $^1H$ NMR (400MHz, DMSO-$d_6$) $\delta$ = 8.93 (d, *J* =8.4 Hz, 1H), 8.03 - 7.92 (m, 3H), 7.85 (td, *J*

=2.0, 8.8 Hz, 1H), 7.66 (t, *J* =9.2 Hz, 1H), 7.22 (d, *J* =10.0 Hz, 1H), 6.78 (br d, *J* =12.4 Hz, 2H), 6.69 (s, 1H), 5.54 (s, 2H), 5.03 (br t, *J* =7.2 Hz, 1H), 4.11 (s, 3H), 1.42 (d, *J* =7.2 Hz, 3H); MS (ESI) m/z = 502.4 $[M+H]^+$.

**Example 372: N-[(1R)-1-[3-(1,1-difluoro-2-hydroxy-ethyl)-2-fluoro-phenyl]ethyl]-1-[2-fluoro-5-(3-methyltriazol-4-yl)phenyl]-6-oxo-pyridazine-3-carboxamide**

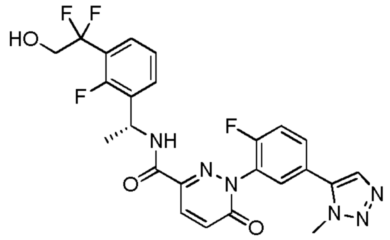

**372**

The compound of Example 372 (12.2 mg, 20.26% yield) was obtained as a white solid in the same manner as in Example 371 by replacing Intermediate C with Intermediate E. $^1H$ NMR (400MHz, DMSO-$d_6$) $\delta$ = 9.05 (br d, *J* =8.0 Hz, 1H), 8.07 - 7.98 (m, 2H), 7.95 (d, *J* =10.0 Hz, 1H), 7.89 - 7.79 (m, 1H), 7.71 - 7.57 (m, 2H), 7.43 (br t, *J* =6.8 Hz, 1H), 7.32 - 7.19 (m, 2H), 5.71 (t, *J* =6.4 Hz, 1H), 5.40 (quin, *J* =7.2 Hz, 1H), 4.13 (s, 3H), 3.91 (dt, *J* =6.4, 14.3 Hz, 2H), 1.46 (br d, *J* =7.2 Hz, 3H); MS (ESI) m/z = 517.4 $[M+H]^+$).

**Example 373: N-[(1R)-1-[3-amino-5-(trifluoromethyl)phenyl]ethyl]-1-[3-(5-methyltetrazol-1-yl)phenyl]-6-oxo-pyridazine-3-carboxamide**

Step 1: Synthesis of methyl 1-[3-(5-methyltetrazol-1-yl)phenyl]-6-oxo-pyridazine-3-carboxylate

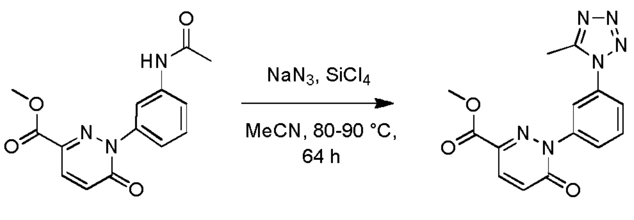


**Intermediate K-4**

A mixture of Intermediate K-4 (200 mg, 696.21 μmol), $NaN_3$ (400 mg, 6.15 mmol) and $SiCl_4$ (354.85 mg, 2.09 mmol, 239.77 uL) in dry acetonitrile (4 mL) was stirred at 20 °C for 16 hours and then at 90 °C for additional 30 hours. The reaction mixture was poured into water (30 mL) and extracted with EtOAc (30 mL × 3). The combined organic layer was washed with brine (50 mL), dried over anhydrous $Na_2SO_4$ and concentrated under reduced pressure to obtain a residue. The aqueous layer was adjusted to pH = 11 with aq. NaOH and then added with NaClO (10 mL). The product was purified by silica gel column chromatography (57% EtOAc in PE) to give methyl 1-[3-(5-methyltetrazol-1-yl)phenyl]-6-oxo-pyridazine-3-carboxylate (120 mg, 30.62% yield) as yellow oil. MS (ESI) m/z = 312.9 $[M+H]^+$.

Steps 2 and 3: Synthesis of N-[(lR)-1-[3-amino-5-(trifluoromethyl)phenyl]ethyl]-1-[2-fluoro-5-(3-methyltriazol-4-yl)phenyl]-6-oxo-pyridazine-3-carboxamide

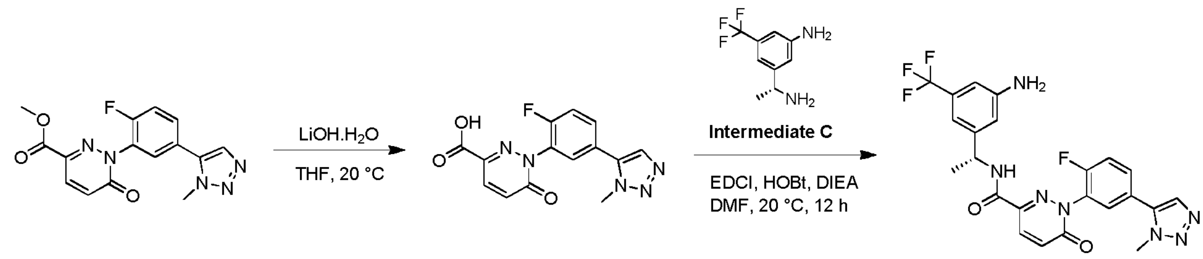

373

The compound of Example 373 (9.6 mg, 12.27% yield) was obtained as a white solid in the same manner as in Example 76. $^1$H NMR (400MHz, DMSO-$d_6$) δ = 8.92 (d, *J* = 8.4 Hz, 1H), 8.15 - 8.08 (m, 1H), 8.04 - 7.97 (m, 1H), 7.92 (d, *J* = 10.0 Hz, 1H), 7.86 - 7.78 (m, 2H), 7.20 (d, *J* = 10.0 Hz, 1H), 6.80 (br d, *J* = 12.0 Hz, 2H), 6.70 (s, 1H), 5.55 (s, 2H), 5.04 (br t, *J* = 7.6 Hz, 1H), 2.61 (s, 3H), 1.45 (d, *J* = 7.2 Hz, 3H); MS (ESI) m/z = 485.1 $[M+H]^+$.

**Example 374: N-[(1R)-1-[3-amino-5-(trifluoromethyl)phenyl]ethyl]-1-[3-(5-methyltetrazol-1-yl)phenyl]-6-oxo-pyridine-3-carboxamide**

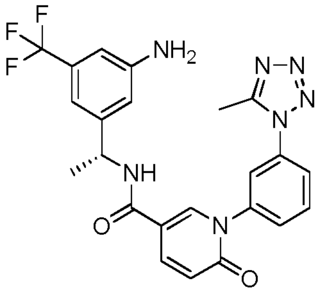
374

The compound of Example 374 (23.7 mg, 53.11% yield) was obtained as a white solid in the same manner as in Example 373 by using a starting material corresponding to the structure of the desired compound based on Preparation Example 7. $^1$H NMR (400 MHz, DMSO-$d_6$) δ = 8.60 (d, *J* = 8.0 Hz, 1H), 8.44 (d, *J* = 2.4 Hz, 1H), 8.01 - 7.93 (m, 2H), 7.86 - 7.77 (m, 3H), 6.76 (s, 2H), 6.69 (s, 1H), 6.58 (d, *J* = 9.6 Hz, 1H), 5.55 (s, 2H), 5.02 (br t, *J* = 7.2 Hz, 1H), 2.61 (s, 3H), 1.40 (d, *J* = 7.2 Hz, 3H); MS (ESI) m/z = 484.4 $[M+H]^+$.

**Example 375: N-[(1R)-1-[3-amino-5-(difluoromethyl)phenyl]ethyl]-1-(2-fluorophenyl)-6-oxo-pyridazine-3-carboxamide**

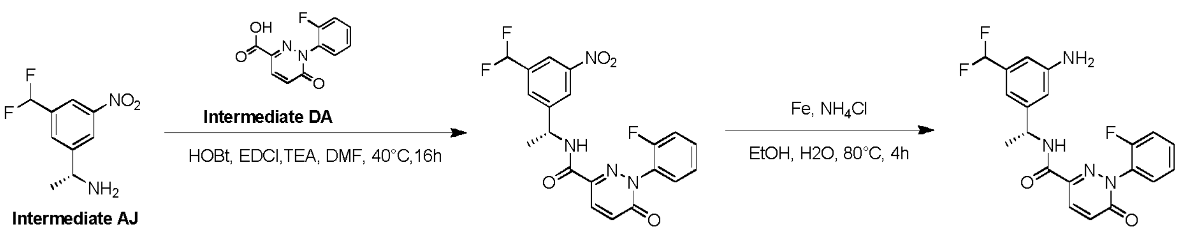

375

The compound of Example 375 (11.9 mg, 30.97% yield) was obtained as a white solid by reacting Intermediate AJ with Intermediate DA in the same manner as in Steps 3 and 4 of Example 89. H NMR (400 MHz, DMSO-$d_6$) δ = 8.88 (br d, *J* = 8.4 Hz, 1H), 7.94 (d, *J* = 10.0 Hz, 1H), 7.68 (br t, *J* = 7.2 Hz, 1H), 7.62 - 7.54 (m, 1H), 7.48 - 7.38 (m, 2H), 7.18 (d, *J* = 10.0 Hz, 1H), 6.95 (s, 1H), 6.81 (s, 1H), 6.68 - 6.65 (m, 2H), 6.57 (s, 1H), 5.36 (s, 2H), 5.00 (quin, *J* = 7.2 Hz, 1H), 1.41 (br d, *J* = 6.8 Hz, 3H); MS (ESI) m/z = 403.1 $[M+H]^+$.

Example 376: N-[(1R)-1-[3-amino-5-(trifluoromethyl)phenyl]ethyl]-1-(3-isoxazol-3-ylphenyl)-6-oxo-pyridazine-3-carboxamide

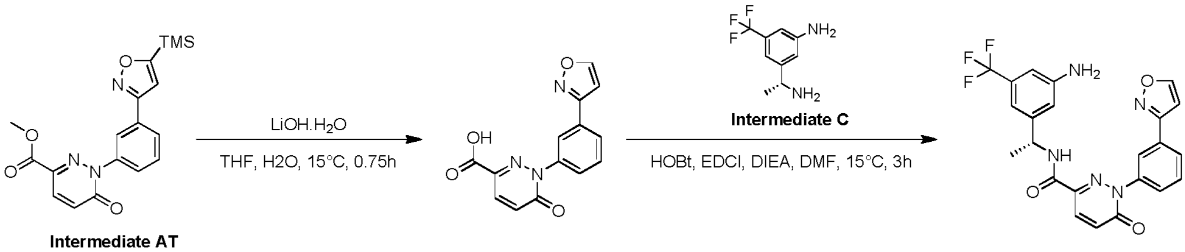


Intermediate AT

376

The compound of Example 376 (7.9 mg, 12.40% yield) was obtained as a white solid in the same manner as in Example 76. $^1$H NMR (400MHz, DMSO-$d_6$) δ = 9.06 (d, *J* = 1.6 Hz, 1H), 8.92 (d, *J* = 8.4 Hz, 1H), 8.18 (t, *J* = 1.6 Hz, 1H), 8.01 (td, *J* = 1.2, 8.0 Hz, 1H), 7.92 (d, *J* = 9.6 Hz, 1H), 7.84 - 7.77 (m, 1H), 7.74 - 7.66 (m, 1H), 7.21 (d, *J* = 1.6 Hz, 1H), 7.18 (d, *J* = 9.6 Hz, 1H), 6.82 (s, 1H), 6.78 (s, 1H), 6.70 (s, 1H), 5.54 (s, 2H), 5.04 (quin, *J* = 7.2 Hz, 1H), 1.44 (d, *J* = 7.2 Hz, 3H); MS (ESI) m/z = 470.3 $[M+H]^+$.

**Example 377: N-[(1R)-1-[3-amino-5-(trifluoromethyl)phenyl]ethyl]-1-[3-(5-methyltriazol-1-yl)phenyl]-6-oxo-pyridine-3-carboxamide**

Step 1: Synthesis of ethyl 1-(3-(5-methyl-1H-1,2,3-triazol-1-yl)phenyl)-6-oxo-1,6-dihydropyridine-3-carboxylate

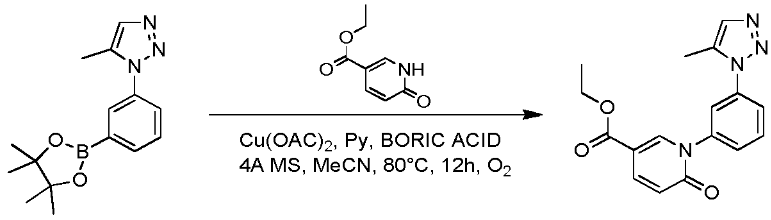


Intermediate CI

The compound of ethyl 1-(3-(5-methyl-1H-1,2,3-triazol-1-yl)phenyl)-6-oxo-1,6-dihydropyridine-3-carboxylate was obtained in the same manner as in Step 1 of Example 21.

Steps 2 and 3: Synthesis of N-[(1R)-1-[3-amino-5-(trifhroromethyl)phenyl]ethyl]-1-[3-(5 -methyltriazol-1-yl)phenyl]-6-oxo-pyridine-3-carboxamide

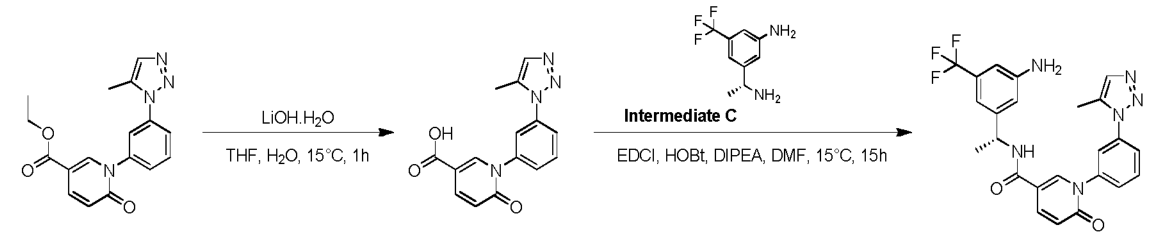


377

The compound of Example 377 (7.7 mg, 11.82% yield) was obtained as a white solid in a similar manner to Example 76. $^1$H NMR (400MHz, DMSO-$d_6$) δ = 8.59 (d, *J* = 7.6 Hz, 1H), 8.44 (d, *J* = 2.4 Hz, 1H), 7.98 (dd, *J* = 2.8, 9.6 Hz, 1H), 7.84 (t, *J* = 1.6 Hz, 1H), 7.79 - 7.76 (m, 1H), 7.75 - 7.70 (m, 2H), 6.76 (s, 2H), 6.69 (s, 1H), 6.57 (d, *J* = 9.6 Hz,

1H), 5.54 (s, 2H), 5.02 (quin, *J* = 7.2 Hz, 1H), 2.38 (s, 3H), 1.40 (d, *J* = 6.8 Hz, 3H); MS (ESI) m/z = 483.4 $[M+H]^+$.

**Example 378: N-[(1R)-1-[3-amino-5-(trifluoromethyl)phenyl]ethyl]-1-[3-(5-methyltriazol-1-yl)phenyl]-6-oxo-pyridazine-3-carboxamide**

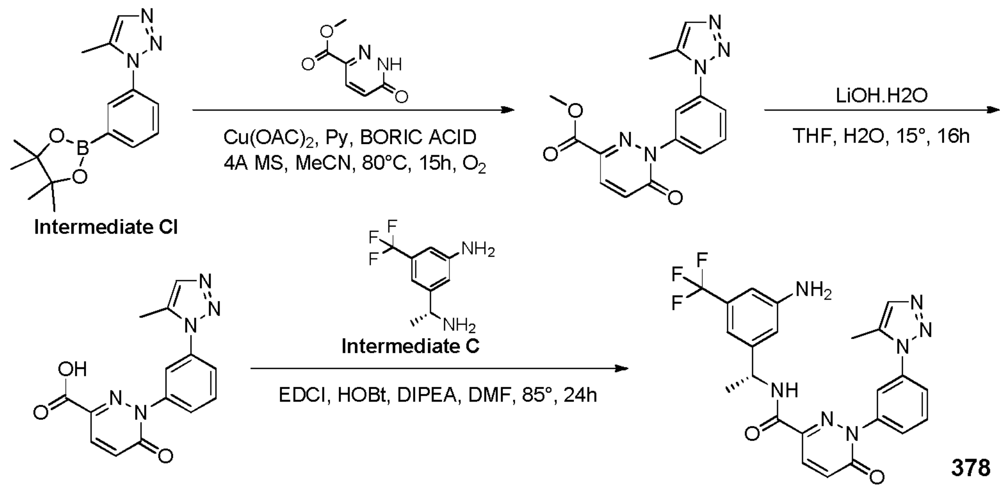


378

The compound of Example 378 (11.1 mg, 22.75% yield) was obtained as a white solid in the same manner as in Example 377 by using methyl 6-oxo-1,6-dihydropyridazine-3-carboxylate instead of ethyl 6-oxo-1,6-dihydropyridine-3-carboxylate in Step 1. $^1H$ NMR (400 MHz, DMSO-$d_6$) $\delta$ = 8.92 (d, *J* = 8.4 Hz, 1H), 8.02 (t, *J* = 2.0 Hz, 1H), 7.97 - 7.86 (m, 2H), 7.82 - 7.76 (m, 1H), 7.76 - 7.68 (m, 2H), 7.19 (d, *J* = 9.6 Hz, 1H), 6.80 (br d, *J* = 11.2 Hz, 2H), 6.70 (s, 1H), 5.54 (s, 2H), 5.04 (quin, *J* = 7.2 Hz, 1H), 2.38 (s, 3H), 1.44 (d, *J* = 7.2 Hz, 3H); MS (ESI) m/z = 484.4 $[M+H]^+$.

**Example 379: 4-amino-N-[(1R)-1-[3-(1,1-difluoro-2-hydroxy-ethyl)-2-fluoro-phenyl]ethyl]-1-(2-fluorophenyl)-6-oxo-pyridazine-3-carboxamide**

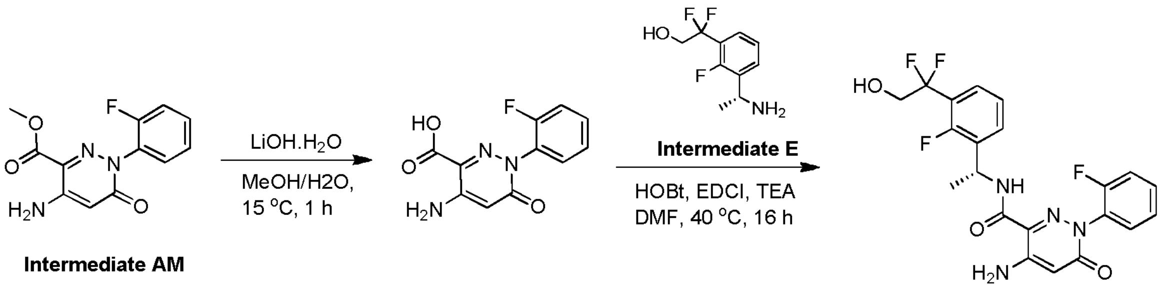


379

The compound of Example 379 (7.8 mg, 7.12% 5-Step yield) was obtained as a white solid in a similar manner to Example 76. $^1H$ NMR (400MHz, DMSO-$d_6$) $\delta$ = 8.94 (d, *J* = 8.0 Hz, 1H), 7.49-7.65 (m, 3H), 7.33-7.45 (m, 3H), 7.19-7.30 (m, 3H), 5.78 (s, 2H), 5.36 (q, *J* = 7.2 Hz, 1H), 3.91 (t, *J* = 14.4 Hz, 2H), 1.44 (d, *J* = 7.2 Hz, 3H); MS (ESI) m/z = 451.1 $[M+H]^+$.

**Example 380: [2-[3-[(1R)-1-[[4-acetamido-1-(2-fluorophenyl)-6-oxo-pyridazine-3-carbonyl]amino]ethyl]-2-fluoro-phenyl]-2,2-difluoro-ethyl]acetate**

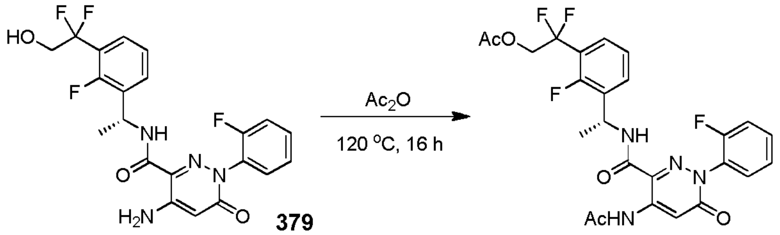


379 380

A mixture of the compound of Example 379 (76 mg, 168.74 μmol) in $Ac_2O$ (5 mL) was stirred at 120 °C for 16 hours. The mixture was neutralized with sat. aq. $NaHCO_3$ and extracted with EtOAc (10 mL × 3). The combined organic layer was washed with brine (10 mL), dried over anhydrous $Na_2SO_4$ and concentrated under reduced pressure to obtain a crude product. The product was purified by prep-HPLC (column: Phenomenex C18 75*30mm*3μm; mobile phase: [water($NH_3H_2O$+$NH_4HCO_3$)-ACN];B%: 37%-67%,10min). The desired fraction was concentrated under reduced pressure, and the remaining solvent was removed by lyophilization to afford the compound of Example 380 (5.7 mg, 6.19% yield) as a white solid. $^1$H NMR (400MHz, DMSO-$d_6$) δ = 11.37 (s, 1H), 9.31 (d, *J* = 8.0 Hz, 1H), 7.79 (s, 1H), 7.67 (td, *J* = 7.6, 1.6 Hz, 2H), 7.55-7.62 (m, 1H), 7.39-7.52 (m, 3H), 7.30-7.36 (m, 1H), 5.42 (q, *J* = 7.2 Hz, 1H), 4.61-4.76 (m, 2H), 2.17 (s, 3H), 2.04 (s, 3H), 1.48 (d, *J* = 7.2 Hz, 3H); MS (ESI) m/z = 535.3 [M+H]$^+$.

**Example 381: 4-amino-N-[(1R)-1-[3-amino-5-(trifluoromethyl)phenyl]ethyl]-1-(2-fluorophenyl)-6-oxo-pyridazine-3-carboxamide**

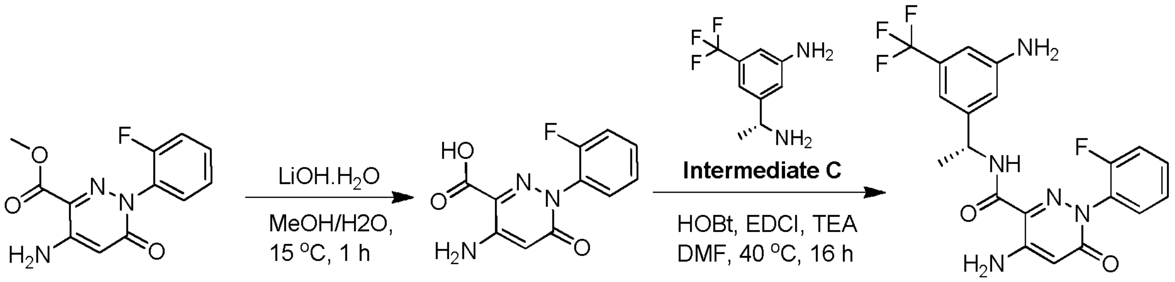


381

Intermediate AM

The compound of Example 381 (12.8 mg, 18.13% yield) was obtained as a white solid in the same manner as in Example 379 by replacing Intermediate E with Intermediate C. $^1$H NMR (400MHz, DMSO-$d_6$) δ = 8.84 (d, J = 8.4 Hz, 1H), 7.46-7.62 (m, 2H), 7.32-7.41 (m, 2H), 7.25 (s, 2H), 6.77 (d, *J* = 11.2 Hz, 2H), 6.70 (s, 1H), 5.79 (s, 1H), 5.56 (s, 2H), 4.94-5.04 (m, 1H), 1.41 (d, *J* = 7.2 Hz, 3H), 1.33-1.49 (m, 1H); MS (ESI) m/z = 436.3 [M+H]$^+$.

**Example 382: 4-acetamido-N-[(1R)-1-[3-amino-5-(trifluoromethyl)phenyl]ethyl]-1-(2-fluorophenyl)-6-oxo-pyridazine-3-carboxamide**

Steps 1 and 2: Synthesis of 4-amino-1-(2-fluorophenyl)-N-[(1R)-1-[3-nitro-5-(trifluoromethyl)phenyl]ethyl]-6-oxo-pyridazine-3-carboxamide

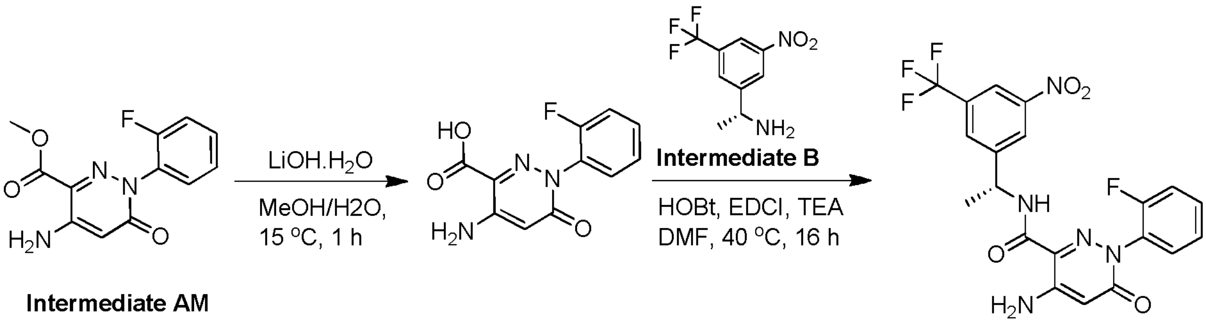


**Intermediate AM**

4-amino-1-(2-fluorophenyl)-N-[(1R)-1-[3-nitro-5-(trifluoromethyl)phenyl]ethyl]-6-oxo-pyridazine-3-carboxamide (36 mg, 13.46% yield) was obtained as a white solid in the same manner as in Example 379 by replacing Intermediate E with Intermediate B. MS (ESI) m/z = 466.2 $[M+H]^+$.

Step 3: Synthesis of 4-acetamido-1-(2-fluorophenyl)-N-[(1R)-1-[3-nitro-5-(trifluoromethyl)phenyl]ethyl]-6-oxo-pyridazine-3-carboxamide

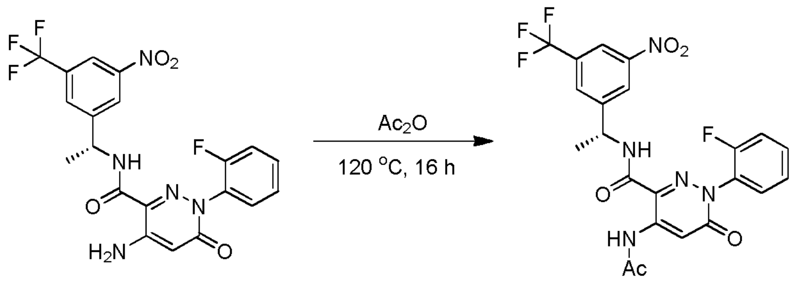


A mixture of 4-amino-1-(2-fluorophenyl)-N-[(1R)-1-[3-nitro-5-(trifluoromethyl)phenyl]ethyl]-6-oxo-pyridazine-3-carboxamide (36 mg, 77.36 μmol) in $Ac_2O$ (3 mL) was stirred at 120 °C for 16 hours. The mixture was concentrated under reduced pressure to give 4-acetamido-1-(2-fluorophenyl)-N-[(1R)-1-[3-nitro-5-(trifluoromethyl)phenyl]ethyl]-6-oxo-pyridazine-3-carboxamide (crude, 45 mg) as a yellow solid. MS (ESI) m/z = 343.2 $[M+H]^+$.

Step 4: Synthesis of 4-acetamido-N-[(1R)-1-[3-amino-5-(trifluoromethyl)phenyl]ethyl]-1-(2-fluorophenyl)-6-oxo-pyridazine-3-carboxamide

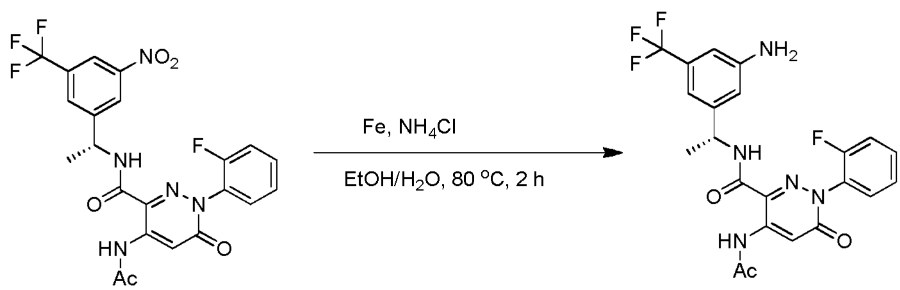


**382**

A mixture of 4-acetamido-1-(2-fluorophenyl)-N-[(1R)-1-[3-nitro-5-(trifluoromethyl)phenyl]ethyl]-6-oxo-pyridazine-3-carboxamide (45 mg, 88.69 μmol), Fe (49.53 mg, 886.89 μmol) and $NH_4Cl$ (47.44 mg, 886.89 μmol) in EtOH (2.5 mL) and $H_2O$ (0.5 mL) was stirred at 80 °C for 2 hours. The crude product was filtered to obtain a filtrate, and the filtrate was concentrated under reduced pressure to obtain a crude product. The residue was purified by prep-HPLC (column: Phenomenex C18 75*30mm*3μm;mobile phase: [water($NH_3H_2O$+$NH_4HCO_3$)-ACN];B%: 30%-60%, 14min). The desired fraction was concentrated under reduced pressure, and the remaining solvent was removed by lyophilization

to afford the compound of Example 382 (6.2 mg, 20.73% 2-steps yield) as a white solid. $^1$H NMR (400MHz, DMSO-$d_6$) δ = 11.47 (s, 1H), 9.22 (d, *J* = 7.6 Hz, 1H), 7.79 (s, 1H), 7.51-7.69 (m, 2H), 7.35-7.49 (m, 2H), 6.79 (d, *J* = 15.6 Hz, 2H), 6.71 (s, 1H), 5.57 (s, 2H), 5.05 (t, *J* = 7.2 Hz, 1H), 2.18 (s, 3H), 1.44 (d, *J* = 7.2 Hz, 3H); MS (ESI) m/z = 478.2 $[M+H]^+$.

**Example 383: N-[(1R)-1-[3-amino-5-(trifluoromethyl)phenyl]ethyl]-1-(2-fluorophenyl)-4-(methylamino)-6-oxo-pyridazine-3-carboxamide**

Steps 1 and 2: Synthesis of tert-butyl N-[3-[[(1R)-1-[3-amino-5-(trifluoromethyl)phenyl]ethyl]carbamoyl]-1-(2-fluorophenyl)-6-oxo-pyridazin-4-yl]-N-methylcarbamate

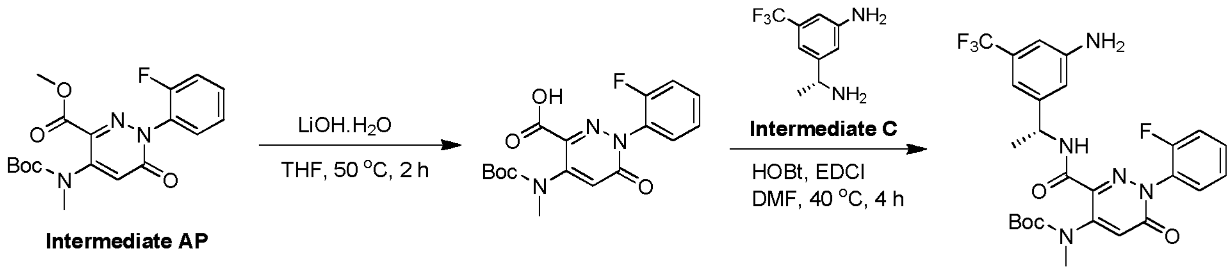


Tert-butyl N-[3-[[(1R)-1-[3-amino-5-(trifluoromethyl)phenyl]ethyl]carbamoyl]-1-(2-fluorophenyl)-6-oxo-pyridazin-4-yl]-N-methyl-carbamate (139 mg, 65.62% yield) was obtained as a yellow solid in the same manner as in Example 76. MS (ESI) m/z = 364.1 $[M+H]^+$.

Step 3: Synthesis of N-[(1R)-1-[3-amino-5-(trifluoromethyl)phenyl]ethyl]-1-(2-fluorophenyl)-4-(methylamino)-6-oxo-pyridazine-3-carboxamide

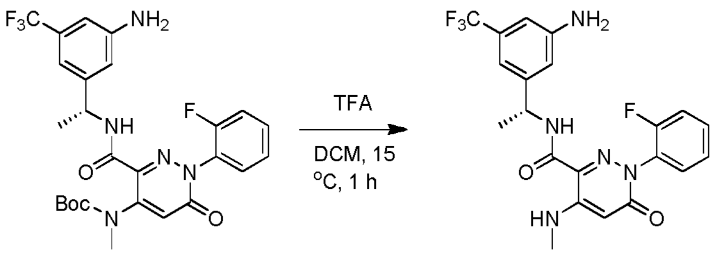


383

The compound of Example 383 (21.3 mg, 18.00% 2-Step yield) was obtained as a white solid in the same manner as in Step 5 of Example 79. $^1$H NMR (400MHz, DMSO-$d_6$) δ = 8.89 (d, *J* = 8.0 Hz, 1H), 7.98-8.10 (m, 1H), 7.59 (td, *J* = 7.6, 1.6 Hz, 1H), 7.48-7.55 (m, 1H), 7.32-7.41 (m, 2H), 6.77 (d, *J* = 11.6 Hz, 2H), 6.69 (s, 1H), 5.64 (s, 1H), 5.55 (s, 2H), 4.98 (q, *J* = 7.2 Hz, 1H), 2.77 (d, *J* = 4.8 Hz, 3H), 1.41 (d, *J* = 7.2 Hz, 3H); MS (ESI) m/z = 450.2 $[M+H]^+$).

**Example 384: N-[(1R)-1-[3-amino-5-(trifluoromethyl)phenyl]ethyl]-1-(2-fluorophenyl)-4-(methanesulfonamido)-6-oxo-pyridazine-3-carboxamide**

Step 1: Synthesis of 1-(2-fluorophenyl)-4-(methanesulfonamido)-N-[(1R)-1-[3-nitro-5-(trifluoromethyl)phenyl]ethyl]-6-oxo-pyridazine-3-carboxamide

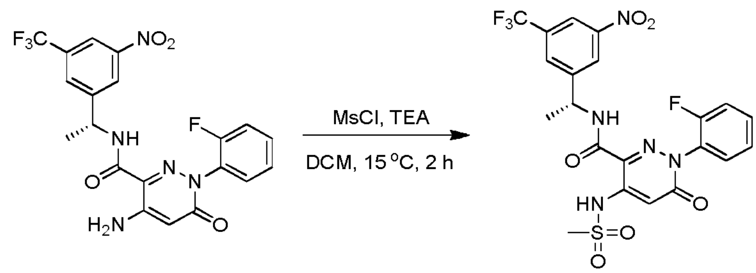


To a mixture of 4-amino-1-(2-fluorophenyl)-N-[(1R)-1-[3-nitro-5-(trifluoromethyl)phenyl]ethyl]-6-oxo-pyridazine-3-carboxamide (35 mg, 75.21 μmol), which was obtained from Step 2 of Example 382, and TEA (76.11 mg, 752.11 μmol) in DCM (3 mL) was added MsCl (86.16 mg, 752.11 μmol) at 0°C, and the mixture was stirred at 15 °C for 2 hours. After the addition of methanesulfonyl chloride (129.23 mg, 1.13 mmol) and DMAP (2.76 mg, 22.56 μmol) at 0°C, the mixture was stirred at 15 °C for 2 hours. The mixture was diluted with water (10 mL) and extracted with EtOAc (10 mL × 3). The combined organic layer was washed with brine (10 mL), dried over anhydrous $Na_2SO_4$ and concentrated under reduced pressure to give 1-(2-fluorophenyl)-4-(methanesulfonamido)-N-[(1R)-1-[3-nitro-5-trifluoromethyl)phenyl]ethyl]-6-oxo-pyridazine-3-carboxamide (crude, 44 mg) as a yellow solid. MS (ESI) m/z = 544.2 $[M+H]^+$.

Step 2: Synthesis of N-[(1R)-1-[3-amino-5-(trifluoromethyl)phenyl]ethyl]-1-(2-fluorophenyl)-4-(methanesulfonamido)-6-oxo-pyridazine-3-carboxamide

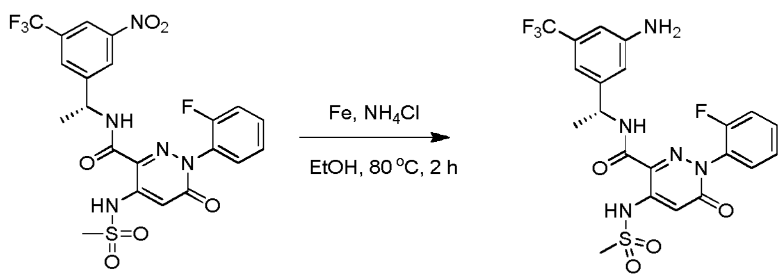


384

The compound of Example 384 (4.3 mg, 10.02% 2-Step yield) was obtained as a white solid in the same manner as in Step 4 of Example 382. $^1H$ NMR (400MHz, DMSO-$d_6$) δ = 8.21 (d, *J* = 7.6 Hz, 1H), 8.15-8.24 (m, 1H), 7.52 (s, 2H), 7.32-7.44 (m, 2H), 6.81-7.30 (m, 3H), 6.78 (s, 1H), 6.70 (s, 1H), 5.56 (s, 2H), 4.98 (s, 1H), 3.18 (s, 3H), 1.41 (d, *J* = 6.8 Hz, 3H); MS (ESI) m/z = 514.3 $[M+H]^+$.

**Example 385: N-[(1R)-1-[3-amino-5-(trifluoromethyl)phenyl]ethyl]-1-(2-methylthiazol-5-yl)-6-oxo-pyridazine-3-carboxamide**

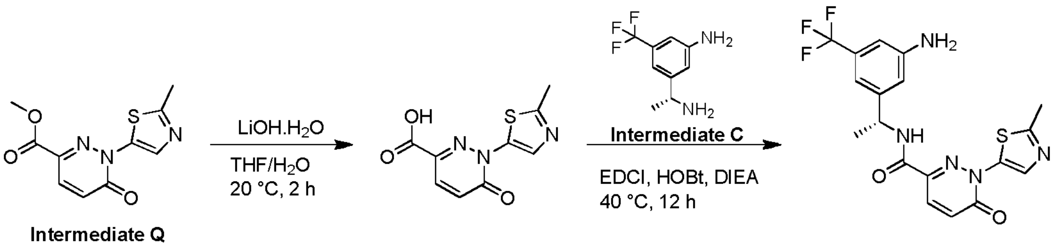


Intermediate Q

385

The compound of Example 385 (8.4 mg, 15.03 % yield) was prepared in the same manner as in Example 76. $^1H$ NMR (400MHz, DMSO-$d_6$) δ = 9.05 (d, J = 8.4 Hz, 1H), 8.66 (s, 1H), 7.94 (d, J = 9.6 Hz, 1H), 7.27 (d, J = 9.6 Hz, 1H), 6.87 (s, 1H), 6.83 (s, 1H), 6.72 (s, 1H), 5.57 (s, 2H), 5.17 - 5.06 (m, 1H), 2.66 (s, 3H), 1.52 (d, J = 7.2 Hz, 3H); MS (ESI) m/z = 424.3 $[M+H]^+$

**Example 386 to Example 388**

The compounds in the following table were prepared in the same manner as in Example 385 by using starting materials corresponding to the respective structures of the desired compounds based on Preparation Example 7.

[Table 26]

| No. | Structure/Name | Spectral Data |
| --- | --- | --- |
| 386 | 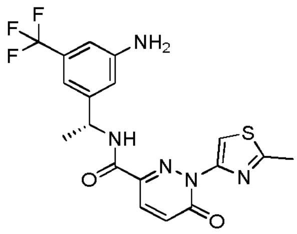 N-[(1R)-1-[3-amino-5-(trifluoromethyl)phenyl]ethyl]-1-(2-methylthiazol-4-yl)-6-oxo-pyridazine-3-carboxamide | $^1H$ NMR (400MHz, DMSO-$d_6$) δ = 8.87 (d, J = 8.4 Hz, 1H), 7.90 (d, J = 9.6 Hz, 1H), 7.85 (s, 1H), 7.15 (d, J = 9.6 Hz, 1H), 6.82 (s, 1H), 6.77 (s, 1H), 6.70 (s, 1H), 5.55 (s, 2H), 5.02 (quin, J = 7.2 Hz, 1H), 2.70 (s, 3H), 1.44 (d, J = 7.2 Hz, 3H); MS (ESI) m/z = 424.3 $[M+H]^+$ |
| 387 | 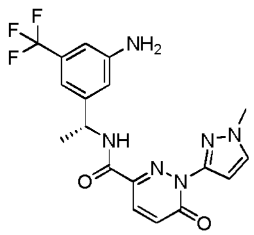 N-[(1R)-1-[3-amino-5-(trifluoromethyl)phenyl]ethyl]-1-(1-methylpyrazol-3-yl)-6-oxo-pyridazine-3-carboxamide | $^1H$ NMR (400MHz, DMSO-$d_6$) δ = 8.82 (d, J = 8.0 Hz, 1H), 7.88 (d, J = 9.6 Hz, 1H), 7.82 (d, J = 2.4 Hz, 1H), 7.12 (d, J = 9.6 Hz, 1H), 6.83 (s, 1H), 6.78 (s, 1H), 6.70 (s, 1H), 6.50 (d, J = 2.4 Hz, 1H), 5.55 (s, 2H), 5.02 (quin, J = 7.2 Hz, 1H), 3.88 (s, 3H), 1.44 (d, J = 7.2 Hz, 3H); MS (ESI) m/z = 407.3 $[M+H]^+$ |
| 388 | 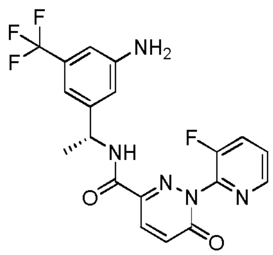 N-[(1R)-1-[3-amino-5-(trifluoromethyl)phenyl]ethyl]-1-(2-methylthiazol-4-yl)-6-oxo-pyridazine-3-carboxamide | $^1H$ NMR (400MHz, DMSO-$d_6$) δ = 8.87 (d, J = 8.4 Hz, 1H), 7.90 (d, J = 9.6 Hz, 1H), 7.85 (s, 1H), 7.15 (d, J = 9.6 Hz, 1H), 6.82 (s, 1H), 6.77 (s, 1H), 6.70 (s, 1H), 5.55 (s, 2H), 5.02 (quin, J = 7.2 Hz, 1H), 2.70 (s, 3H), 1.44 (d, J = 7.2 Hz, 3H); MS (ESI) m/z = 422.2 $[M+H]^+$ |

**Example 389: N-[(1R)-1-[3-amino-5-(trifluoromethyl)phenyl]ethyl]-1-[4-fluoro-3-(3-methyltriazol-4-yl)phenyl]-6-oxo-pyridazine-3-carboxamide**

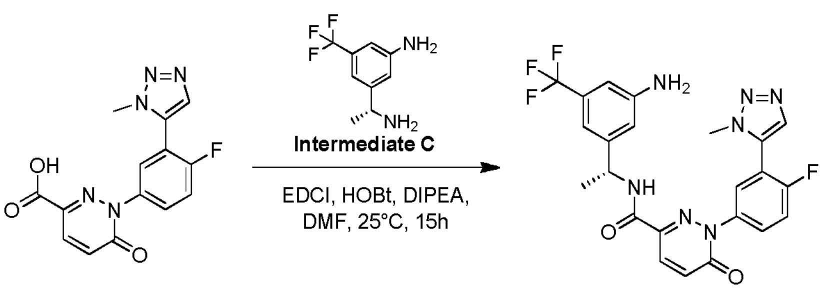


Intermediate U-1

389

The compound of Example 389 was prepared in a similar manner to Step 2 of Example 76. $^1$H NMR (400MHz, DMSO-$d_6$) $\delta$ = 8.89 (d, $J$ = 8.4 Hz, 1H), 8.03 - 7.96 (m, 2H), 7.96 - 7.87 (m, 2H), 7.63 (t, $J$ = 9.2 Hz, 1H), 7.18 (d, $J$ = 9.6 Hz, 1H), 6.80 (br d, $J$ = 11.2 Hz, 2H), 6.70 (s, 1H), 5.55 (s, 2H), 5.11 - 4.95 (m, 1H), 4.04 (s, 3H), 1.45 (d, $J$ = 7.2 Hz, 3H); MS (ESI) m/z = 502.3 [M+H]$^+$.

**Example 390: N-[(1R)-1-[3-(1,1-difluoro-2-hydroxy-ethyl)-2-fluoro-phenyl]ethyl]-1-[4-fluoro-3-(3-methyltriazole-4)-yl)phenyl]-6-oxo-pyridazine-3-carboxamide**

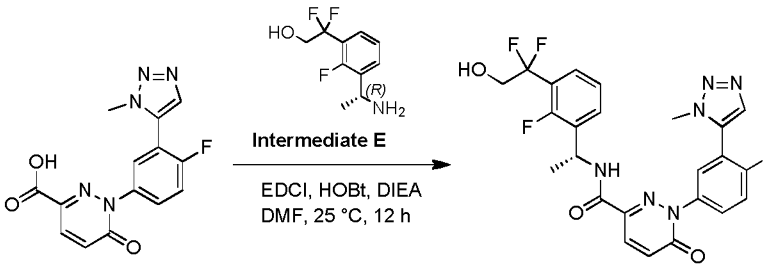


Intermediate U-1

390

The compound of Example 390 was prepared in the same manner as in Example 389 by using Intermediate E. $^1$H NMR (400MHz, DMSO-$d_6$) $\delta$ = 8.99 (d, J = 8.0 Hz, 1H), 8.07 - 7.98 (m, 2H), 7.96 (ddd, J = 2.8, 4.8, 8.8 Hz, 1H), 7.89 (d, J = 9.6 Hz, 1H), 7.68 - 7.61 (m, 2H), 7.44 (t, J = 7.2 Hz, 1H), 7.27 (t, J = 7.6 Hz, 1H), 7.18 (d, J = 9.6 Hz, 1H), 5.71 (br t, J = 6.0 Hz, 1H), 5.41 (quin, J = 7.2 Hz, 1H), 4.06 (s, 3H), 3.91 (dt, J = 5.2, 14.4 Hz, 2H), 1.48 (d, J = 7.2 Hz, 3H); MS (ESI) m/z = 517.4 [M+H]$^+$.

**Example 391: N-[(1R)-1-[3-amino-5-(trifluoromethyl)phenyl]ethyl]-1-[3-fluoro-5-(3-methyltriazol-4-yl)phenyl]-6-oxo-pyridazine-3-carboxamide**

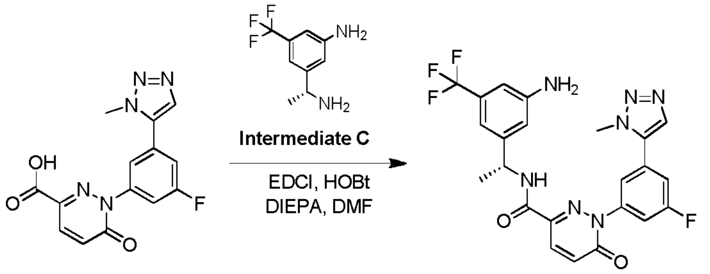


Intermediate U-2

391

The compound of Example 391 was prepared in the same manner as in Example 389 by using Intermediated U-2. $^1$H NMR (400 MHz, DMSO-$d_6$): $\delta$ = 8.92 (d, $J$ = 8.4 Hz, 1H), 8.04 (s, 1H), 7.92 (d, $J$ = 9.6 Hz, 1H), 7.88 (t, $J$ = 1.6 Hz, 1H), 7.84 (td, $J$ = 2.0, 9.6 Hz, 1H), 7.68 (td, $J$ = 2.0, 9.6 Hz, 1H), 7.20 (d, $J$ = 9.6 Hz, 1H), 6.80 (br d, $J$ = 12.0 Hz, 2H), 6.71 (s, 1H), 5.54 (s, 2H), 5.09 - 5.02 (m, 1H), 4.14 (s, 3H), 1.46 (d, $J$ = 7.2 Hz, 3H); MS (ESI) m/z = 502.4 [M+H]$^+$.

**Example 392: N-[(1R)-1-[3-amino-5-(trifluoromethyl)phenyl]ethyl]-1-[5-(3-methyltriazol-4-yl)-3-pyridyl]-6-oxo-pyridazine-3-carboxamide**

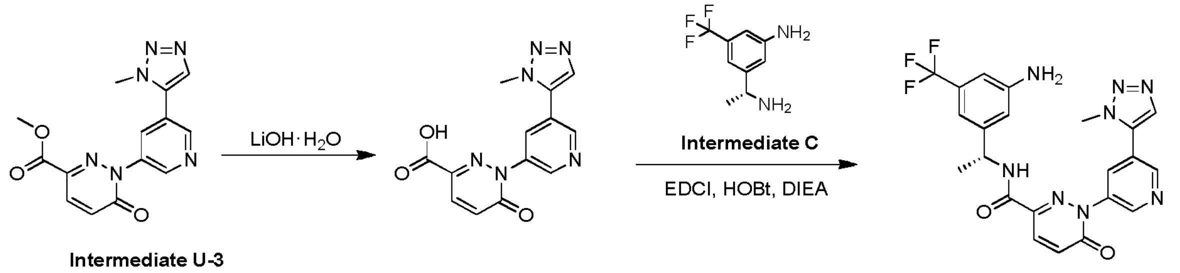


392

The compound of Example 392 was prepared in the same manner as in Example 76 by using Intermediate U-3 and Intermediate C. $^1$H NMR (400 MHz, METHANOL-d$_4$): $\delta$ = 9.09 (d, *J* = 2.4 Hz, 1H), 8.85 (d, *J* = 2.0 Hz, 1H), 8.48 (t, *J* = 2.4 Hz, 1H), 8.05 - 8.02 (m, 2H), 7.21 (d, *J* = 9.6 Hz, 1H), 6.90 (br d, *J* = 6.0 Hz, 2H), 6.81 (s, 1H), 5.15 (q, *J* = 7.2 Hz, 1H), 4.19 (s, 3H), 1.54 (d, *J* = 7.2 Hz, 3H); MS (ESI) m/z = 485.4 [M+H]$^+$

**Example 393: N-[(1R)-1-[3-(1,1-difluoro-2-hydroxy-ethyl)-2-fluoro-phenyl]ethyl]-6-oxo-1-[3-[3-(trideuteriomethyl)triazol-4-yl]phenyl]pyridazine-3-carboxamide**

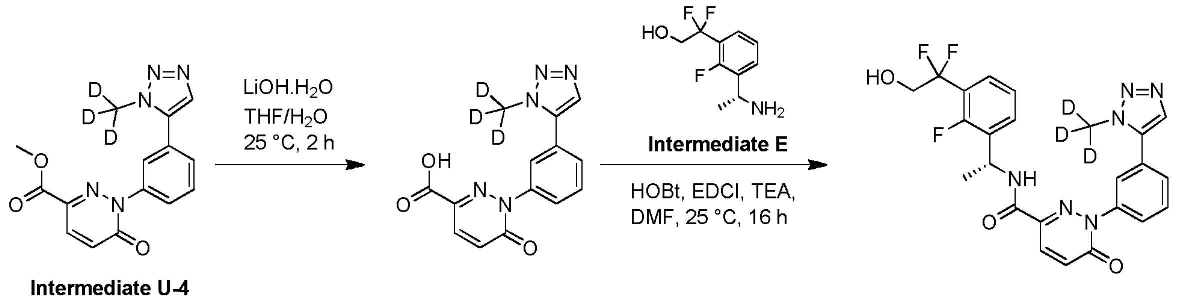


393

The compound of Example 373 was prepared in the same manner as in Steps 2 and 3 of Example 21 by using Intermediate U-4 and Intermediate E. $^1$H NMR (400MHz, DMSO-d$_6$) $\delta$ = 9.02 (d, J = 8.0 Hz, 1H), 8.01 (s, 1H), 7.97 (s, 1H), 7.90 (d, J = 10.0 Hz, 1H), 7.85 (dt, J = 2.4, 4.8 Hz, 1H), 7.76 - 7.71 (m, 2H), 7.68 - 7.60 (m, 1H), 7.48 - 7.40 (m, 1H), 7.31 - 7.24 (m, 1H), 7.18 (d, J = 10.0 Hz, 1H), 5.72 (t, J = 6.4 Hz, 1H), 5.41 (t, J = 7.6 Hz, 1H), 4.04 - 3.79 (m, 2H), 1.48 (d, J = 7.2 Hz, 3H); MS (ESI) m/z = 502.4 [M+H]$^+$.

Example 394: N-[(1S)-1-[4-amino-6-(trifluoromethyl)-2-pyridyl]ethyl]-1-(2-**fluorophenyl)-6-oxo-pyridazine-3-carboxamide**

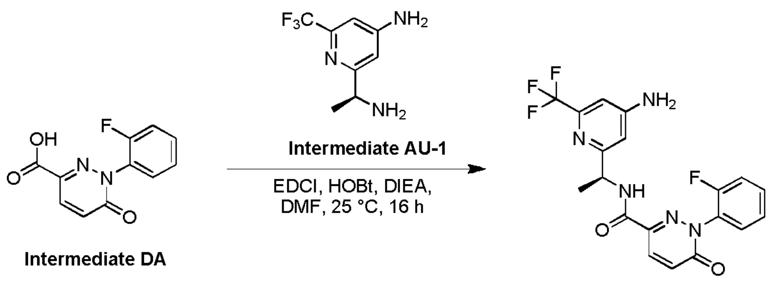


394

The compound of Example 394 (11.5 mg, 8.2% yield) was obtained as a yellow solid in the same manner as in Step 2 of Example 76 by using Intermediate AU-1. $^1$H NMR (400 MHz, $CDCl_3$) $\delta$ = 8.48 (d, J = 7.2 Hz, 1H), 8.05 (d, J = 9.6 Hz, 1H), 7.56 - 7.45 (m, 2H), 7.37 - 7.27 (m, 2H), 7.25 (s, 1H), 7.11 (d, J = 9.6 Hz, 1H), 6.78 (d, J = 2.0 Hz, 1H), 6.60 (d, J = 1.6 Hz, 1H), 5.17 - 5.06 (m, 1H), 4.48 (s, 2H), 1.54 (d, J = 6.8 Hz, 3H); MS (EI) m/z: 422.2 [M+H]$^+$.

**Example 395: N-[(1R)-1-[4-amino-6-(trifluoromethyl)-2-pyridyl]ethyl]-1-(2-fluorophenyl)-6-oxo-pyridazine-3-carboxamide**

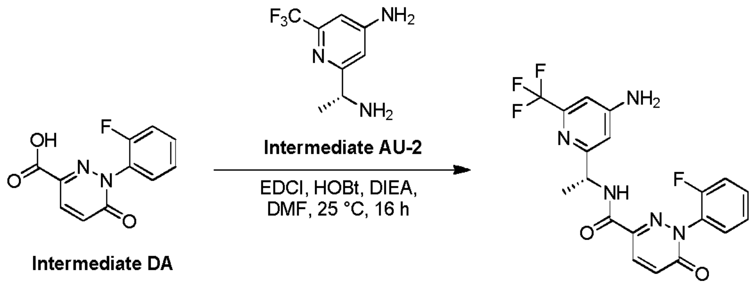


**395**

The compound of Example 395 (6.27 mg, 17.9% yield) was obtained as a colorless solid in the same manner as in Step 2 of Example 76 by using Intermediate AU-2. $^1H$ NMR (400 MHz, $CDCl_3$) $\delta$ = 8.48 (d, J = 7.2 Hz, 1H), 8.05 (d, J = 9.6 Hz, 1H), 7.56 - 7.45 (m, 2H), 7.37 - 7.28 (m, 2H), 7.12 (d, J = 9.6 Hz, 1H), 6.79 (d, J = 2.0 Hz, 1H), 6.60 (d, J = 2.0 Hz, 1H), 5.18 - 5.08 (m, 1H), 4.44 (s, 2H), 1.54 (d, J = 6.8 Hz, 3H); MS (EI) m/z: 422.2 $[M+H]^+$.

**Example 396: N-[(1R)-1-[2-[5-fluoro-2-(methylaminomethyl)phenyl]thiazol-5-yl]ethyl]-1-(2-fluorophenyl)-6-oxo-pyridazine-3-carboxamide**

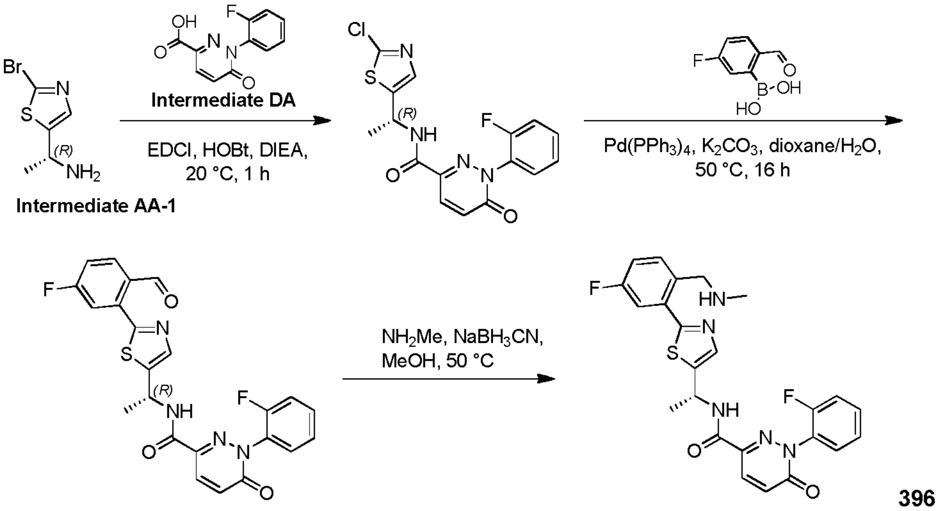


**396**

The compound of Example 396 (7.49 mg, 58% yield) was obtained as a yellow solid in the same manner as in Example 132 by using 1-(2-fluorophenyl)-6-oxo-1,6-dihydropyridazine-3-carboxylic acid, which was obtained from Step 1 of Example 76, and Intermediate AA-1. $^1H$ NMR (400 MHz, $CDCl_3$) $\delta$ = 8.54 (s, 1H), 8.08 (d, J = 9.6 Hz, 1H), 7.82 (s, 1H), 7.63 - 7.56 (m, 1H), 7.55 - 7.45 (m, 2H), 7.43 - 7.38 (m, 1H), 7.36 - 7.31 (m, 2H), 7.21 - 7.12 (m, 2H), 5.65 - 5.52 (m, 1H), 4.14 (s, 2H), 2.63 (s, 3H), 1.76 (d, J = 7.2 Hz, 3H); MS (EI) m/z: 482.1 $[M+H]^+$.

**Example 397: N-[(1R)-1-[3-(1,1-difluoro-2-hydroxy-ethyl)-2-fluoro-phenyl]ethyl]-1-[3-(3-methyltriazol-4-yl)phenyl]-6-oxo-pyridazine-3-carboxamide**

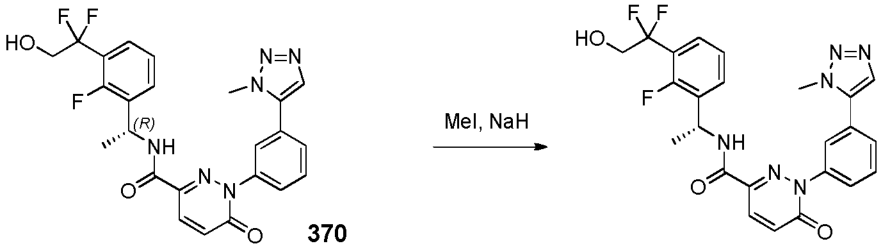


370

397

The compound of Example 397 was prepared in the same manner as in Example 242 by using the compound of Example 370. $^1$H NMR (400 MHz, DMSO-$d_6$) δ = 8.98 (br d, *J* = 8.0 Hz, 1H), 8.03 - 7.91 (m, 2H), 7.91 - 7.86 (m, 1H), 7.83 (dt, *J* = 2.0, 4.4 Hz, 1H), 7.71 (d, *J* = 4.8 Hz, 2H), 7.63 (br t, *J* = 7.2 Hz, 1H), 7.44 (br t, *J* = 7.2 Hz, 1H), 7.27 (t, *J* = 7.6 Hz, 1H), 7.17 (d, *J* = 9.6 Hz, 1H), 5.43 - 5.33 (m, 1H), 4.12 (s, 3H), 3.94 (s, 2H), 3.31 (s, 3H), 1.47 (d, *J*= 7.2 Hz, 3H); LC/MS (ESI) m/z: 513.4 $[M+H]^+$.

**Example 398: (R)-1-(2,4-difluorophenyl)-N-(1-(5-(2-((methylamino)methyl)phenyl)thiophenyl-2-yl)ethyl)-6-oxo-1,6-dihydropyridazine-3-carboxamide**

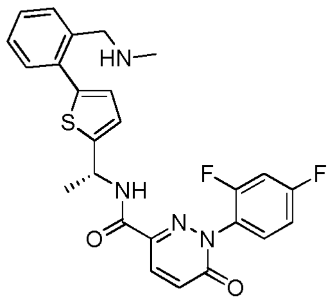

398

The compound of Example 398 was prepared in the same manner as in Examples 265 and 266 by using the product obtained from Step 5 of Example 352 and (2-formylphenyl)boronic acid. $^1$H NMR (400 MHz, DMSO-$d_6$) δ = 9.06 (d, *J* = 8.4 Hz, 1H), 7.98 (d, *J* = 10.0 Hz, 1H), 7.76 (dt, *J* = 6.4, 8.4 Hz, 1H), 7.59 - 7.45 (m, 2H), 7.36 - 7.24 (m, 4H), 7.20 (d, *J* = 10.0 Hz, 1H), 7.12 (d, *J* = 3.6 Hz, 1H), 7.01 (d, *J* = 3.6 Hz, 1H), 5.41 (quin, *J* = 7.6 Hz, 1H), 3.67 (s, 2H), 2.26 (s, 3H), 1.59 (d, *J*= 7.2 Hz, 3H); LC/MS (ESI) m/z: 481.4 $[M+H]^+$

**Example 399 to Example 415**

The compounds in the following table were prepared in a similar manner to Example 398.

[Table 27]

| No. | Structure/Name | Spectral Data |
|---|---|---|
| 399 | 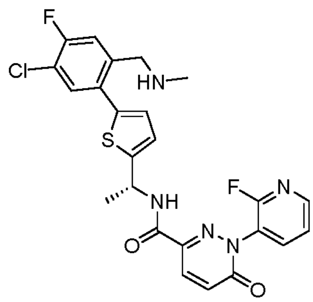 N-[(1R)-1-(5-{5-chloro-4-fluoro-2-[(methylamino)methyl] phenyl}thiophen-2-yl)ethyl]-1-(2-fluoropyridin-3-yl)-6-oxo-1,6-dihydropyridazine-3-carboxamide | $^1$H NMR (400 MHz, DMSO-$d_6$) δ = 9.13 (d, *J* = 8.4 Hz, 1H), 8.40 (d, *J* = 5.2 Hz, 1H), 8.31 (ddd, *J* = 2.0, 7.6,9.2 Hz, 1H), 8.00 (d, *J* = 10.0 Hz, 1H), 7.62 (ddd, *J* = 1.2, 5.6, 6.8 Hz, 1H), 7.58 - 7.44 (m, 2H), 7.23 (d, *J* = 10.0 Hz, 1H), 7.11 (d, *J* = 3.6 Hz, 1H), 7.04 - 7.00 (m, 1H), 5.44 - 5.36 (m, 1H), 3.65 - 3.63 (m, 2H), 2.23 (s, 3H), 1.59 (d, *J* = 6.8 Hz, 3H); LC/MS (ESI) m/z: 516.3 $[M+H]^+$ |

(continued)

| No. | Structure/Name | Spectral Data |
|---|---|---|
| 400 | 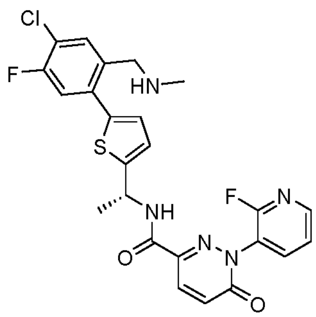 N-[(1R)-1-(5-{4-chloro-5-fluoro-2-[(methylamino)methyl] phenyl}thiophen-2-yl)ethyl]-1-(2-fluoropyridin-3-yl)-6-oxo-1,6-dihydropyridazine-3-carboxamide | $^1$H NMR (400 MHz, DMSO-$d_6$) $\delta$ = 8.35 (br d, *J* = 4.8 Hz, 1H), 8.18 (t, *J* = 8.0 Hz, 1H), 8.09 (d, *J* = 9.6 Hz, 1H), 7.60 (d, *J* = 7.6 Hz, 1H), 7.53 (dd, *J* = 5.2, 6.8 Hz, 1H), 7.22 (dd, *J* = 10.0, 11.2 Hz, 2H), 7.04 (d, *J* = 4.0 Hz, 1H),7.01 (d, *J* = 3.6 Hz, 1H), 5.50 (q, *J* = 6.8 Hz, 1H), 3.73 (s, 2H), 2.29 (s, 3H), 1.67 (d, *J* = 7.2 Hz, 3H); LC/MS (ESI) m/z: 516.3 $[M+H]^+$ |
| 401 | 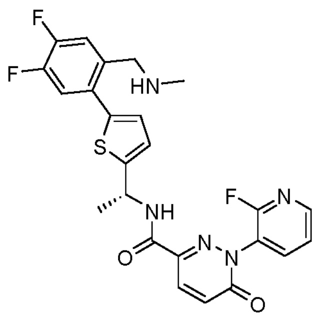 N-[(1R)-1-(5-{4,5-difluoro-2-[(methylamino)methyl] phenyl}thiophen-2-yl)ethyl]-1-(2-fluoropyridin-3-yl)-6-oxo-1,6-dihydropyridazine-3-carboxamide | $^1$H NMR (400 MHz, DMSO-$d_6$) $\delta$ = 9.12 (d, *J* = 8.8 Hz, 1H), 8.40 (d, *J* = 4.8 Hz, 1H), 8.31 (ddd, *J* = 2.0, 8.0, 9.6 Hz, 1H), 8.00 (d, *J* = 10.0 Hz, 1H), 7.62 (ddd, *J* = 1.2, 5.2, 7.6 Hz, 1H), 7.55 (dd, *J* = 8.8, 12.4 Hz, 1H), 7.39 (dd, *J* = 8.4, 11.6 Hz, 1H), 7.23 (d, *J* = 10.0 Hz, 1H), 7.12 (d, *J* = 3.6 Hz, 1H), 7.02 (dd, *J* = 0.8, 3.6 Hz, 1H), 5.40 (br t, *J* = 7.6 Hz, 1H), 3.60 (s, 2H), 2.22 (s, 3H), 1.59 *(d, J* = 7.2 Hz, 3H); LC/MS (ESI) m/z: 500.3 $[M+H]^+$ |
| 402 | 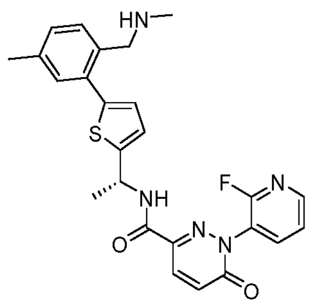 1-(2-fluoropyridin-3-yl)-N-[(1R)-1-(5-{5-methyl-2-[(methylamino)methyl]phenyl}thiophen-2-yl)ethyl]-6-oxo-1,6-dihydropyridazine-3- carboxamide | $^1$H NMR (400 MHz, DMSO-$d_6$) $\delta$ = 8.42 (br s, 1H), 8.36 (br d, J = 4.8 Hz, 1H), 8.23 - 8.16 (m, 1H), 8.09 (d, J = 10.0 Hz, 1H), 7.53 (dd, J = 5.2, 7.2 Hz, 1H), 7.45 (d, J = 8.0 Hz, 1H), 7.34 - 7.27 (m, 2H), 7.21 (d, J = 10.0 Hz, 1H), 7.07 (d, J = 3.2 Hz, 1H), 6.95 (d, J = 3.6 Hz, 1H), 5.50 (q, J = 6.8 Hz, 1H), 4.27 (s, 2H), 2.60 (s, 3H), 2.38 (s, 3H), 1.68 (d, J = 6.8 Hz, 3H); LC/MS (ESI) m/z: 478.4 $[M+H]^+$ |
| 403 | 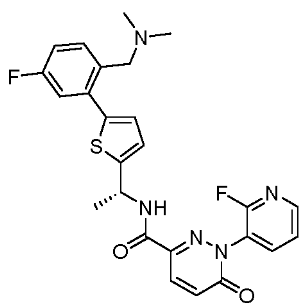 N-[(1R)-1-(5-{2-[(dimethylamino)methyl]-5-fluorophenyl} thiophen-2-yl)ethyl]-1-(2-fluoropyridin-3-yl)-6-oxo-1,6-dihydropyridazine-3-carboxamide | $^1$H NMR (400 MHz, DMSO-$d_6$) $\delta$ = 8.34 (br d, *J* = 3.6 Hz, 1H), 8.17 (br t, *J* = 8.0 Hz, 1H), 8.08 (br d, *J* = 10.0 Hz, 1H), 7.60 - 7.45 (m, 2H), 7.17 (br dd, *J* = 9.6, 18.0 Hz, 3H), 7.03 (br s, 2H), 5.58 - 5.41 (m, 1H), 3.85 (br s, 2H), 2.35 (s, 6H), 1.66 (br d, *J* = 6.8 Hz, 3H); LC/MS (ESI) m/z: 496.3 $[M+H]^+$ |

(continued)

| No. | Structure/Name | Spectral Data |
|---|---|---|
| 404 | 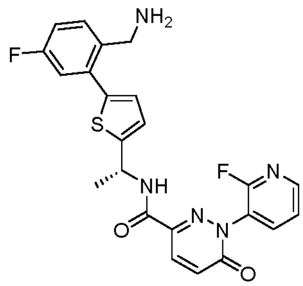 N-[(1R)-1-(5-{2-[(aminomethyl)-5-fluorophenyl]thiophen-2-yl)ethyl]-1-(2-fluoropyridin-3-yl)-6-oxo-1,6-dihydropyridazine-3-carboxamide | $^1H$ NMR (400 MHz, DMSO-$d_6$) δ = 9.12 (d, *J* = 8.8 Hz, 1H), 8.40 (d, *J* = 4.8 Hz, 1H), 8.32 (ddd, *J* = 2.0, 7.6, 9.6 Hz, 1H), 8.00 (d, *J* = 10.0 Hz, 1H), 7.66 - 7.56 (m, 2H), 7.27 - 7.06 (m, 4H), 7.03 (d, *J* = 3.6 Hz, 1H), 5.41 (quin, *J* = 7.6 Hz, 1H), 3.74 (s, 2H), 2.26 -1.71 (m, 2H), 1.59 (d, *J* = 7.2 Hz, 3H); LC/MS (ESI) m/z: 468.1 $[M+H]^+$ |
| 405 | 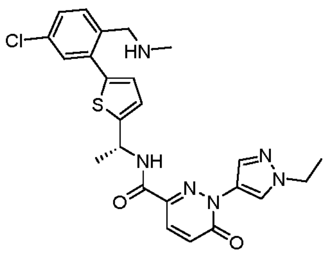 N-[(1R)-1-(5-{5-chloro-2-[(methylamino)[methyl]phenyl} thiophen-2-yl)ethyl]-1-(1-ethyl-1H-pyrazol-4-yl)-6-oxo-1,6-dihydropyridazine-3-carboxamide | $^1H$ NMR (400 MHz, DMSO-$d_6$) δ = 8.52 (s, 1H), 8.28 (s, 1H), 7.99 (d, *J* = 9.6 Hz, 1H), 7.44 (d, *J* = 7.6 Hz, 1H), 7.36 - 7.32 (m, 2H), 7.15 (d, *J* = 9.6 Hz, 1H), 7.09 (d, *J* = 4.0 Hz, 1H), 7.01 (d, *J* = 3.6 Hz, 1H), 5.56 (q, *J* = 6.8 Hz,1H), 4.23 (q, *J* = 7.2 Hz, 2H), 3.76 (s, 2H), 2.29 (s, 3H), 1.75 (d, *J* = 6.8 Hz, 3H), 1.49 (t, *J* = 7.2 Hz, 3H); LC/MS (ESI) m/z: 497.4 $[M+H]^+$ |
| 406 | 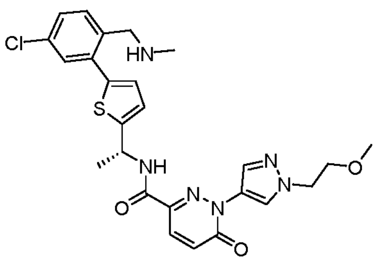 N-[(1R)-1-(5-{5-chloro-2-[(methylamino)[methyl]phenyl} thiophen-2-yl)ethyl]-1-[1-(2-methoxyethyl)-1H-pyrazol-4-yl)-6-oxo-1,6-dihydropyridazine-3-carboxamide | $^1H$ NMR (400 MHz, DMSO-$d_6$) δ = 9.10 (d, J = 8.8 Hz, 1H), 8.55 (s, 1H), 8.38 (s, 1H), 7.92 (d, J = 10.0 Hz, 1H), 7.53 (d, J = 8.0 Hz, 1H), 7.42 - 7.31 (m, 2H), 7.21 (d, J = 3.6 Hz, 1H), 7.17 (d, J = 9.6 Hz, 1H), 7.08 (d, J = 2.8 Hz, 1H), 5.56 - 5.39 (m, 1H), 4.32 (t, J = 5.2 Hz, 2H), 3.70 (t, J = 5.2 Hz, 2H), 3.64 (s, 2H), 3.22 (s, 3H), 2.24 (s, 3H), 2.05 (br d, J = 18.0 Hz, 1H), 1.68 (d, J = 7.2 Hz, 3H); LC/MS (ESI) m/z: 527.4 $[M+H]^+$ |
| 407 | 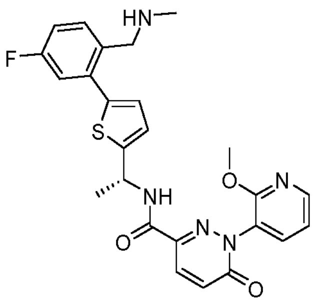 N-[(1R)-1-(5-{5-fluoro-2-[(methylamino)[methyl]phenyl} thiophen-2-yl)ethyl]-1-(2-methoxypyridin-3-yl)-6-oxo-1,6-dihydropyridazine-3-carboxamide | $^1H$ NMR (400 MHz, DMSO-$d_6$) δ = 9.04 (d, *J* = 8.8 Hz, 1H), 8.31 (dd, *J* = 2.0, 5.2 Hz, 1H), 8.01 - 7.91 (m, 2H), 7.55 - 7.48 (m, 1H), 7.22 - 7.16 (m, 3H), 7.16 - 7.12 (m, 2H), 7.01 (dd, *J* = 0.8, 3.6 Hz, 1H), 5.40 (quin, *J* = 7.2 Hz, 1H), 3.86 (s, 3H), 3.60 (s, 2H), 2.23 (s, 3H), 1.58 (d, *J* = 7.2 Hz, 3H); LC/MS (ESI) m/z: 494.4 $[M+H]^+$ |

(continued)

| No. | Structure/Name | Spectral Data |
|---|---|---|
| 408 | 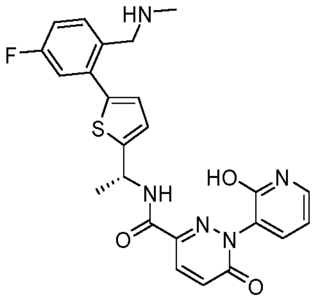 N-[(1R)-1-(5-{5-fluoro-2-[(methylamino)[methyl]phenyl} thiophen-2-yl)ethyl]-1-(2-hydroxypyridin-3-yl)-6-oxo-1,6-dihydropyridazine-3-carboxamide | $^1$H NMR (400 MHz, DMSO-$d_6$) δ = 8.42 (td, *J* = 1.2, 3.2 Hz, 1H), 8.25 (ddd, *J* = 1.8, 7.6, 9.6 Hz, 1H), 7.99 (d, *J* = 10.0 Hz, 1H), 7.62 (ddd, *J* = 1.2, 4.8, 7.6 Hz, 1H), 7.24 (d, *J* = 10.0 Hz, 1H), 0.99 (t, *J* = 7.2 Hz, 3H); LC/MS (ESI) m/z: 250.1 [M+H]$^+$ |
| 409 | 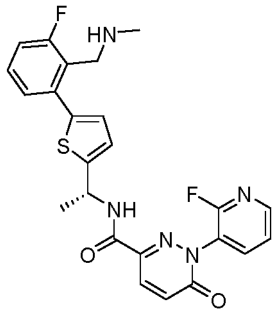 N-[(1R)-1-(5-{3-fluoro-2-[(methylamino)[methyl]phenyl} thiophen-2-yl)ethyl]-(2-fluoropyridin-3-yl)-6-oxo-1,6-dihydropyridazine-3-carboxamide | $^1$H NMR (400 MHz, DMSO-$d_6$) δ = 9.13 (d, *J* = 8.6 Hz, 1H), 8.40 (br d, *J* = 4.8 Hz, 1H), 8.35 - 8.26 (m, 1H), 8.00 (d, *J* = 10.0 Hz, 1H), 7.62 (dd, *J* = 5.6, 7.1 Hz, 1H), 7.38 - 7.28 (m, 2H), 7.26 - 7.14 (m, 3H), 7.03 (d, *J* = 3.6 Hz, 1H), 5.41 (quin, *J* = 7.6 Hz, 1H), 3.59 (br s, 2H), 2.26 (s, 3H), 1.81 (br s, 1H), 1.59 (br d, *J* = 7.2 Hz, 3H); LC/MS (ESI) m/z: 482.4 [M+H]$^+$ |
| 410 | 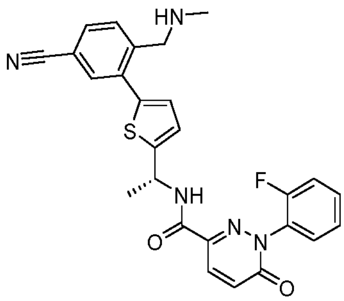 N-[(1R)-1-(5-{5-cyano-2-[(methylamino)[methyl]phenyl} thiophen-2-yl)ethyl]-1-(2-fluorophenyl)-6-oxo-1,6-dihydropyridazine-3-carboxamide | $^1$H NMR (400 MHz, DMSO-$d_6$) δ = 9.10 (d, *J* = 8.4 Hz, 1H), 7.99 (d, *J* = 9.6 Hz, 1H), 7.83 - 7.75 (m, 2H),7.73 (d, *J* = 7.2 Hz, 1H), 7.67 (dt, *J* = 1.6, 7.6 Hz, 1H), 7.61 - 7.55 (m, 1H), 7.47 - 7.38 (m, 2H), 7.23 - 7.17 (m, 2H), 7.05 (dd, *J* = 0.8, 3.6 Hz, 1H), 5.46 - 5.38 (m, 1H), 3.71 (s, 2H), 2.23 (s, 3H), 1.60 (d, *J* = 7.2 Hz, 3H); LC/MS (ESI) m/z: 488.4 [M+H]$^+$ |
| 411 | 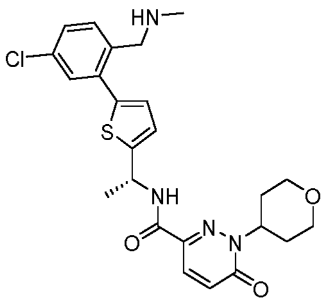 N-[(1R)-1-(5-{5-chloro-2-[(methylamino)[methyl]phenyl} thiophen-2-yl)ethyl]-1-(oxan-4-yl)-6-oxo-1,6-dihydropyridazine-3-carboxamide | $^1$H NMR (400 MHz, DMSO-$d_6$) δ = 9.01 (d, *J* = 8.8 Hz, 1H), 7.86 (d, *J* = 9.6 Hz, 1H), 7.54 (d, *J* = 8.4 Hz, 1H), 7.42 - 7.34 (m, 2H), 7.21 (d, *J* = 3.6 Hz, 1H), 7.07 - 7.04 (m, 1H), 7.02 (d, *J* = 9.6 Hz, 1H), 5.46 (quin, *J* = 7.2 Hz, 1H), 5.04 (tt, *J* = 4.0, 11.6 Hz, 1H), 4.04 - 3.94 (m, 2H), 3.64 (s, 2H), 3.60 (br t, *J* = 11.2 Hz, 2H), 2.30 - 2.16 (m, 5H), 2.16 -1.94 (m, 1H), 1.71 -1.63 (m, 5H); LC/MS (ESI) m/z: 487.4 [M+H]$^+$ |

(continued)

| No. | Structure/Name | Spectral Data |
|---|---|---|
| 412 | 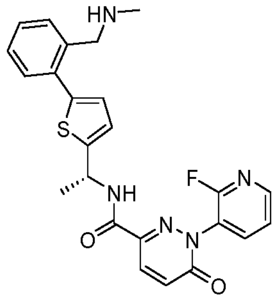 1-(2-fluoropyridin-3-yl)-N-[(1R)-1-(5-{2-[(methylamino) methyl]phenyl}thiophen-2-yl)ethyl] -6-oxo-1,6-dihydropyridazine-3- carboxamide | $^1$H NMR (400 MHz, DMSO-$d_6$) δ = 9.09 (d, *J* = 8.8 Hz, 1H), 8.43 - 8.35 (m, 1H), 8.35 - 8.26 (m, 1H), 8.04 - 7.97 (m, 1H), 7.62 (ddd, *J* = 1.2, 4.8, 7.6 Hz, 1H), 7.49 (d, *J* = 7.6 Hz, 1H), 7.35 - 7.26 (m, 3H), 7.25 - 7.20 (m, 1H), 7.14 - 7.09 (m, 1H), 7.00 (dd, *J* = 0.8, 3.6 Hz, 1H), 5.41 (quin, *J* = 7.2 Hz, 1H), 3.64 (s, 2H), 2.24 (s, 3H), 1.59 (d, *J* = 7.2 Hz, 3H); LC/MS (ESI) m/z: 464.4 $[M+H]^+$ |
| 413 | 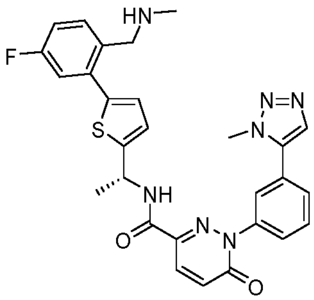 N-[(1R)-1-(5-{5-fluoro-2-[(methylamino)[methyl]phenyl} thiophen-2-yl)ethyl]-1-[3-(1-methyl-1H-1,2,3-triazol-5-yl) phenyl]-6-oxo-1,6-dihydropyridazine-3-carboxamide | $^1$H NMR (400 MHz, DMSO-$d_6$) δ = 9.08 (d, *J* = 8.4 Hz, 1H), 8.01 - 7.93 (m, 3H), 7.86 - 7.82 (m, 1H), 7.73 - 7.70 (m, 2H), 7.69 - 7.65 (m, 1H), 7.35 (dt, *J* = 2.8, 8.8 Hz, 1H), 7.24 (dd, *J* = 2.8, 10.0 Hz, 1H), 7.21 (d, *J* = 10.0 Hz, 1H), 7.14 (d, *J* = 3.6 Hz, 1H), 7.09 (d, *J* = 3.6 Hz, 1H), 5.49 - 5.39 (m, 1H), 4.12 (s, 3H), 4.10 (br s, 2H), 2.49 (br s, 3H), 1.63 (d, *J* = 7.2 Hz, 3H); LC/MS (ESI) m/z: 544.4 $[M+H]^+$ |
| 414 | 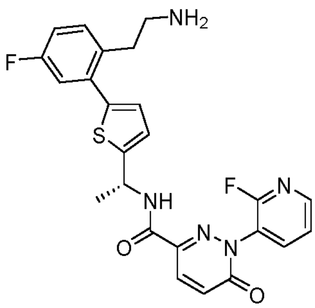 N-[(1R)-1-[5-[2-(2-aminoethyl)-5-fluorophenyl]-2-thienyl] ethyl]-1-(2-fluoro-3-pyridyl)-6-oxopyridazine-3-carboxamide | $^1$H NMR (400 MHz, DMSO-$d_6$) δ = 9.11 (d, *J* = 8.4 Hz, 1H), 8.40 (d, *J* = 4.8 Hz, 1H), 8.31 (t, *J* = 8.4 Hz, 1H),8.00 (d, *J* = 10.0Hz, 1H), 7.62 (dd, *J* = 5.6, 7.2 Hz, 1H), 7.37 - 7.32 (m, 1H), 7.23 (d, *J* = 9.6 Hz, 1H), 7.19 - 7.12 (m, 1H), 7.11 -7.08 (m, 1H), 7.02 (s, 2H), 5.44 - 5.37 (m, 1H), 2.77 - 2.61 (m, 4H), 1.59 (d, *J* = 6.8 Hz, 3H); MS (ESI) m/z = 482.4 $[M+H]^+$ |
| 415 | 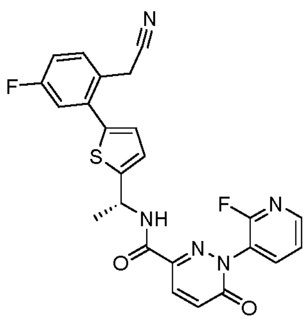 N-[(1R)-1-[5-[2-(cyanomethyl)-5-fluorophenyl]-2-thienyl] ethyl]-1-(2-fluoro-3-pyridyl)-6-oxopyridazine-3-carboxamide | $^1$H NMR (400 MHz, DMSO-$d_6$) δ = 9.13 (d, *J* = 8.4 Hz, 1H), 8.40 (d, *J* = 4.8 Hz, 1H), 8.36 - 8.25 (m, 1H), 8.00 (d, *J* = 9.6 Hz, 1H), 7.68 - 7.53 (m, 2H), 7.33 - 7.20 (m, 3H), 7.13 (d, *J* = 3.6 Hz, 1H), 7.08 (d, *J* = 3.6 Hz, 1H), 5.42 (quin, *J* = 7.2 Hz, 1H), 4.04 (s, 2H), 1.60 (d, J=7.2 Hz, 3H); MS (ESI) m/z = 478.3 $[M+H]^+$ |

**[Experimental Example]**

**Experimental Example 1: SOS-catalyzed nucleotide exchange assay**

SOS1 activity was measured by SOS 1-mediated GTP loading quantification of $KRAS^{G12D}$. GTP loaded $KRAS^{G12D}$ was detected by HTRF (Homogeneous Time Resolved Fluorescence) using anti-GST terbium (Cisbio, France) as a donor and a fluorescence GTP analogue (EDA-GTP-DY-647P1, [DY-647P1 labeled 2'/3'-O-(2-aminoethyl-carbamoyl)guanosine-5'-triphosphate (Jena bioscience, Germany)]) as an acceptor.

Assay buffer was composed of 10 mM HEPES pH 7.4, 150 mM NaCl, 5 mM $MgCl_2$, 1 mM DTT, 0.05% BSA Fraction V pH 7.0 (Sigma) and 0.0025% (v/v) Igepal (Sigma). $KRAS^{G12D}$ working solution was prepared by adding equal amounts of GST-$KRAS^{G12D}$ (100 nM) and anti-GST-terbium (2 nM; Cisbio, France) to the assay buffer and culturing on ice for 10 minutes. $SOS1_{cat}$ working solution was prepared by adding equal amounts of HIS-$SOS1_{cat}$ (160 nM; Cytoskeleton, US) and EDA-GTP-DY-647P1 (200 nM; Jena bioscience, Germany) to the assay buffer. Control solution was prepared by adding equal volumes of EDA-GTP-DY-647P1 and the assay buffer for normalization control to exclude GEF-independent GTP loading and background signals. In order to induce a reaction, 5 uL of the $SOS1_{cat}$ working solution or the control solution was injected into each well. 5 uL of a compound having various concentrations from 128 pM to 10 $\mu$M was added to wells containing the $SOS1_{cat}$ working solution and incubated at room temperature for 10 minutes to bind to SOS1.

10 uL of the $KRAS^{G12D}$ solution was added to each well and incubated for 30 minutes at room temperature while shaking. HTRF was measured using a Thermo Varioskan (excitation 334 nm, emission 665 nm). The $IC_{50}$ of each inhibitor was analyzed using Graphad Prism.

The SOS1 inhibitory activity of the Example compounds measured by the above method was evaluated based on the following criteria, and the results are shown in Tables 28 and 29.

| $IC_{50}$ range | ≤1,000 nM | 1,000 nM < $IC_{50}$ ≤ 10,000 nM | >10,000 nM |
|---|---|---|---|
| Class | A | B | C |

As shown in Tables 28 and 29 below, the Example compounds herein exhibited excellent SOS1 inhibitory activity.

**Experimental Example 2: KRAS $^{G12C}$::SOS1 protein-protein interaction (PPI) inhibition assay**

Protein-protein interaction between SOS1 and $KRAS^{G12C}$ was detected using KRAS G12C/SOS1 binding kit from CISBIO (64KRASG12PEH, CISBIO, FRANCE) according to the manufacturer's assay method. Briefly, the maximum concentration of an inhibitor sample was set to be 3 $\mu$M, and the inhibitor sample was diluted 7 times by 1/3, and samples having a total of 8 concentrations were prepared. In HTRF (Homogeneous Time-Resolved Fluorescence) exclusive 96-well low-volume white plate (66PL96005, CISBIO, FRANCE), 2 $\mu$L of an inhibitor sample prepared for each concentration, 5 $\mu$L of a mixed solution of labeled $KRAS^{G12C}$ protein and GTP diluted with the dilution solution provided by the manufacturer, and 5 $\mu$L of Tag2 labeled SOS1 protein were mixed, and added with 8 $\mu$L of a diluted solution where anti-Tag1 $KRAS^{G12C}$ XI,665 (HTRF acceptor) labeled antibody and anti-Tag2 SOS1 Terbium cryptate (HTRF donor) labeled antibody were mixed in an equal ratio and cultured at room temperature for 2 hours. Thereafter, the HTRF signal generated by the antibody was measured using a Thermo Varioskan multi-purpose plate signal detector, and the result value was calculated as the ratio of the signals emitted at 665 nm and 620 nm as shown below.

$$\text{Result value} = 10^4 \text{ X } 665 \text{ nm signal}/620 \text{ nm signal}$$

The $IC_{50}$ value of each inhibitor was analyzed with the result values of three replicates by using the Graphad Prism 9.0 software.

The SOS1 inhibitory activity of the Example compounds measured by the above method was evaluated based on the following criteria, and the results are shown in Tables 28 and 29.

| $IC_{50}$ range | ≤1,000 nM | 1,000 nM < $IC_{50}$ ≤ 10,000 nM | >10,000 nM |
|---|---|---|---|
| Class | A | B | C |
| NA = Not Applied | | | |

As shown in Table 28 below, the Example compounds herein exhibited excellent SOS1 inhibitory activity.

[Table 28]

| Example No. | **SOS-catalyzed nucleotide exchange assay** | Example No. | **SOS-catalyzed nucleotide exchange assay** |
|---|---|---|---|
| 1 | B | 81 | NA |
| 2 | B | 82 | NA |
| 3 | C | 83 | NA |
| 4 | B | 84 | NA |
| 5 | C | 85 | A |
| 6 | C | 86 | NA |
| 7 | C | 87 | NA |
| 8 | B | 88 | NA |
| 9 | B | 89 | A |
| 10 | B | 90 | NA |
| 11 | B | 91 | NA |
| 12 | C | 92 | NA |
| 13 | A | 93 | NA |
| 14 | B | 94 | A |
| 15 | A | 95 | A |
| 16 | A | 96 | A |
| 17 | B | 97 | B |
| 18 | B | 98 | NA |
| 19 | B | 99 | NA |
| 20 | A | 100 | NA |
| 21 | A | 101 | NA |
| 22 | A | 102 | NA |
| 23 | A | 103 | NA |
| 24 | B | 104 | NA |
| 25 | A | 105 | NA |
| 26 | A | 106 | NA |
| 27 | A | 107 | NA |
| 28 | C | 108 | NA |
| 29 | A | 109 | NA |
| 30 | A | 110 | NA |
| 31 | A | 111 | NA |
| 32 | A | 112 | NA |
| 33 | A | 113 | A |
| 34 | B | 114 | NA |
| 35 | A | 115 | B |
| 36 | A | 116 | B |

(continued)

| Example No. | **SOS-catalyzed nucleotide exchange assay** | Example No. | **SOS-catalyzed nucleotide exchange assay** |
|---|---|---|---|
| 37 | A | 117 | B |
| 38 | A | 118 | B |
| 39 | A | 119 | C |
| 40 | A | 120 | B |
| 41 | A | 121 | NA |
| 42 | A | 122 | NA |
| 43 | A | 123 | NA |
| 44 | A | 124 | B |
| 45 | A | 125 | B |
| 46 | A | 126 | A |
| 47 | A | 127 | B |
| 48 | B | 128 | A |
| 49 | B | 129 | A |
| 50 | B | 130 | A |
| 51 | A | 131 | NA |
| 52 | B | 132 | NA |
| 53 | B | 133 | NA |
| 54 | A | 134 | NA |
| 55 | A | 135 | NA |
| 56 | A | 136 | NA |
| 57 | A | 137 | A |
| 58 | A | 138 | A |
| 59 | A | 139 | NA |
| 60 | C | 140 | A |
| 61 | B | 141 | A |
| 62 | A | 142 | A |
| 63 | B | 143 | A |
| 64 | B | 144 | B |
| 65 | A | 145 | NA |
| 66 | B | 146 | NA |
| 67 | A | 147 | NA |
| 68 | A | 148 | C |
| 69 | B | 149 | A |
| 70 | B | 150 | A |
| 71 | A | 151 | A |
| 72 | A | 152 | A |
| 73 | A | 153 | A |

(continued)

| Example No. | **SOS-catalyzed nucleotide exchange assay** | Example No. | **SOS-catalyzed nucleotide exchange assay** |
|---|---|---|---|
| 74 | A | 154 | B |
| 75 | A | 155 | B |
| 76 | NA | 156 | B |
| 77 | NA | 157 | B |
| 78 | A | 158 | A |
| 79 | NA | 159 | A |
| 80 | NA | 160 | B |

**Experimental Example 2: KRAS $^{G12C}$::SOS1 protein-protein interaction (PPI) inhibition assay**

Protein-protein interaction between SOS1 and KRAS$^{G12C}$ was detected using KRAS G12C/SOS1 binding kit from CISBIO (64KRASG12PEH, CISBIO, FRANCE) according to the manufacturer's assay method. Briefly, the maximum concentration of an inhibitor sample was set to be 3 μM, and the inhibitor sample was diluted 7 times by 1/3, and samples having a total of 8 concentrations were prepared. In HTRF (Homogeneous Time-Resolved Fluorescence) exclusive 96-well low-volume white plate (66PL96005, CISBIO, FRANCE), 2 μL of an inhibitor sample prepared for each concentration, 5 μL of a mixed solution of Tag1 labeled KRAS$^{G12C}$ protein and GTP diluted with the dilution solution provided by the manufacturer, and 5 μL of Tag2 labeled SOS1 protein were mixed, and added with 8 μL of a diluted solution where anti-Tag1 KRAS$^{G12C}$ XI,665 (HTRF acceptor) labeled antibody and anti-Tag2 SOS1 Terbium cryptate (HTRF donor) labeled antibody were mixed in an equal ratio and cultured at room temperature for 2 hours. Thereafter, the HTRF signal generated by the antibody was measured using a Thermo Varioskan multi-purpose plate signal detector, and the result value was calculated as the ratio of the signals emitted at 665 nm and 620 nm as shown below.

$$\text{Result value} = 10^4 \text{ X } 665 \text{ nm signal}/620 \text{ nm signal}$$

The $IC_{50}$ value of each inhibitor was analyzed with the result values of three replicates by using the Graphad Prism 9.0 software.

The degree of inhibition of the Example compounds on SOS1 and KRAS$^{G12C}$ interaction measured by the above method was evaluated based on the following criteria, and the results are shown in Table 29.

| $IC_{50}$ range | ≤1,000 nM | 1,000 nM < $IC_{50}$ ≤ 10,000 nM | >10,000 nM |
|---|---|---|---|
| Class | A | B | C |
| NA = Not Applied | | | |

[Table 29]

| Example No. | **KRAS $^{G12C}$::SOS1 PPI assay** | Example No. | **KRAS $^{G12C}$::SOS1 PPI assay** |
|---|---|---|---|
| 140 | A | 278 | A |
| 141 | A | 279 | A |
| 142 | A | 280 | A |
| 143 | A | 281 | A |
| 144 | A | 282 | A |
| 145 | A | 283 | A |
| 146 | A | 284 | A |

(continued)

| Example No. | **KRAS $^{G12C}$::SOS1 PPI assay** | Example No. | **KRAS $^{G12C}$::SOS1 PPI assay** |
|---|---|---|---|
| 147 | A | 285 | A |
| 148 | C | 286 | A |
| 149 | A | 287 | A |
| 150 | A | 288 | A |
| 151 | A | 289 | A |
| 152 | A | 290 | A |
| 153 | A | 291 | A |
| 154 | A | 292 | A |
| 155 | A | 293 | A |
| 156 | B | 294 | A |
| 157 | B | 295 | A |
| 158 | A | 296 | A |
| 159 | A | 297 | A |
| 160 | A | 298 | A |
| 161 | A | 299 | B |
| 162 | A | 300 | A |
| 163 | A | 301 | A |
| 164 | A | 302 | A |
| 165 | A | 303 | C |
| 166 | A | 304 | C |
| 167 | A | 305 | C |
| 168 | A | 306 | C |
| 169 | A | 307 | A |
| 170 | A | 308 | A |
| 171 | A | 309 | A |
| 172 | A | 310 | A |
| 173 | A | 311 | A |
| 174 | A | 312 | A |
| 175 | B | 313 | A |
| 176 | C | 314 | A |
| 177 | A | 315 | B |
| 178 | A | 316 | A |
| 179 | A | 317 | A |
| 180 | A | 318 | A |
| 181 | A | 319 | A |
| 182 | A | 320 | A |
| 183 | A | 321 | A |
| 184 | A | 322 | A |

(continued)

| Example No. | **KRAS $^{G12C}$::SOS1 PPI assay** | Example No. | **KRAS $^{G12C}$::SOS1 PPI assay** |
|---|---|---|---|
| 185 | A | 323 | A |
| 186 | A | 324 | A |
| 187 | A | 325 | A |
| 188 | A | 326 | A |
| 189 | A | 327 | A |
| 190 | A | 328 | A |
| 191 | A | 329 | A |
| 192 | A | 330 | A |
| 193 | A | 331 | A |
| 194 | A | 332 | A |
| 195 | A | 333 | A |
| 196 | A | 334 | A |
| 197 | A | 335 | A |
| 198 | A | 336 | A |
| 199 | A | 337 | A |
| 200 | A | 338 | A |
| 201 | A | 339 | A |
| 202 | A | 340 | A |
| 203 | A | 341 | A |
| 204 | A | 342 | A |
| 205 | A | 343 | A |
| 206 | A | 344 | A |
| 207 | A | 345 | A |
| 208 | A | 346 | A |
| 209 | A | 347 | A |
| 210 | A | 348 | A |
| 211 | A | 349 | A |
| 212 | B | 350 | A |
| 213 | A | 351 | A |
| 214 | A | 352 | A |
| 215 | A | 353 | A |
| 216 | B | 354 | A |
| 217 | A | 355 | A |
| 218 | A | 356 | A |
| 219 | A | 357 | B |
| 220 | A | 358 | A |
| 221 | A | 359 | A |
| 222 | A | 360 | A |

(continued)

| Example No. | **KRAS $^{G12C}$::SOS1 PPI assay** | Example No. | **KRAS $^{G12C}$::SOS1 PPI assay** |
|---|---|---|---|
| 223 | A | 361 | A |
| 224 | A | 362 | A |
| 225 | A | 363 | C |
| 226 | A | 364 | B |
| 227 | A | 365 | C |
| 228 | A | 366 | B |
| 229 | B | 367 | B |
| 230 | A | 368 | A |
| 231 | A | 369 | A |
| 232 | A | 370 | A |
| 233 | A | 371 | A |
| 234 | A | 372 | A |
| 235 | A | 373 | A |
| 236 | A | 374 | A |
| 237 | A | 375 | A |
| 238 | A | 376 | A |
| 239 | B | 377 | A |
| 240 | A | 378 | A |
| 241 | A | 379 | A |
| 242 | A | 380 | A |
| 243 | A | 381 | A |
| 244 | A | 382 | A |
| 245 | A | 383 | A |
| 246 | A | 384 | A |
| 247 | A | 385 | A |
| 248 | A | 386 | A |
| 249 | A | 387 | A |
| 250 | A | 388 | C |
| 251 | A | 389 | A |
| 252 | A | 390 | A |
| 253 | A | 391 | A |
| 254 | A | 392 | A |
| 255 | A | 393 | A |
| 256 | A | 394 | C |
| 257 | A | 395 | B |
| 258 | A | 396 | A |
| 259 | A | 397 | A |
| 260 | A | 398 | A |

(continued)

| Example No. | **KRAS $^{G12C}$::SOS1 PPI assay** | Example No. | **KRAS $^{G12C}$::SOS1 PPI assay** |
|---|---|---|---|
| 261 | C | 399 | A |
| 262 | B | 400 | A |
| 263 | A | 401 | A |
| 264 | A | 402 | A |
| 265 | A | 403 | A |
| 266 | A | 404 | A |
| 267 | A | 405 | A |
| 268 | A | 406 | A |
| 269 | A | 407 | A |
| 270 | A | 408 | A |
| 271 | A | 409 | A |
| 272 | A | 410 | A |
| 273 | A | 411 | A |
| 274 | A | 412 | A |
| 275 | A | 413 | A |
| 276 | A | 414 | A |
| 277 | A | 415 | A |

## Claims

**1.** A compound of Formula I below, or a solvate, stereoisomer or pharmaceutically acceptable salt thereof:

[Formula I]

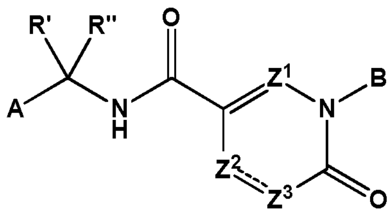

wherein

- - - - - - is a single bond or a double bond;
$Z^1$ is Nor CH;
when $Z^1$ is N, then $Z^2$ and $Z^3$ are both $CHR^1$ and - - - - - - is a single bond, or $Z^2$ and $Z^3$ are both $CR^1$ and - - - - - - is a double bond;
when $Z^1$ is CH, then $Z^2$ is N or $CR^1$, $Z^3$ is $CR^1$, and - - - - - - is a double bond; or
when $Z^1$ is N, $Z^2$ and $Z^3$ are both $CR^1$, and - - - - - - is a double bond, then two $R^1$ may be optionally linked to each other together with the carbon atom to which they are attached to form a thiophene or pyrrole ring;
each $R^1$ is independently selected from the group consisting of H, halogen, CN, OH, $NR^bR^c$, $C_1$-$C_6$ alkoxy, $C_1$-$C_6$ acylamino, $C_1$-$C_6$ alkylsulfonylamino, $C_1$-$C_6$ alkyl, $C_2$-$C_6$ alkenyl, $C_2$-$C_6$ alkynyl, $C_3$-$C_6$ cycloalkyl, $C_{6-}C_{10}$ aryl, $C_{6-}C_{10}$ aryloxy, ($C_{6-}C_{10}$ aryl)-($C_1$-$C_6$ alkyl)oxy and $C_{6-}C_{10}$ arylamino;
R’ and R" are each independently H or $C_1$-$C_3$ alkyl, or R’ and R" may be taken together with the carbon atom to which they are attached to form $C_3$-$C_4$ cycloalkyl, and said $C_1$-$C_3$ alkyl and $C_3$-$C_4$ cycloalkyl may be optionally

substituted with at least one halogen, OH, CN, $C_1$-$C_3$ alkoxy or $NR^bR^c$;
A is $Cy_1$ or $Cy_1$-Y-$Cy_2$;
Y is O, S, or a direct bond;
$Cy_1$ is $C_6$-$C_{10}$ aryl or 5- or 6-membered heteroaryl containing 1 or 2 heteroatoms selected from N, O and S;
$Cy_1$ may be optionally substituted with 1 to 3 $R^{2a}$;
$R^{2a}$ is selected from the group consisting of H, halogen, OH, CN, oxo, $SF_5$, $NR^bR^c$, -Si($C_{1-3}$ alkyl)$_3$, -$SO_2R^b$, -C(O)$R^b$, $C_{1-}C_6$ alkyl, $C_{1-}C_6$ haloalkyl, $C_{1-}C_6$ alkoxy, $C_{1-}C_6$ haloalkoxy, $C_{3-}C_6$ cycloalkyl and

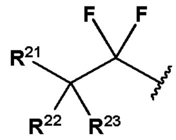

,

$R^{21}$ is H, halogen, OH, $NR^bR^c$, $C_{1-}C_6$ alkoxy or $C_{1-}C_6$ acyloxy, and $R^{22}$ and $R^{23}$ are each independently H, halogen or $C_{1-}C_2$ alkyl;
$Cy_2$ is $C_6$-$C_{10}$ aryl, phenyl fused with $C_3$-$C_6$ cycloalkyl, or 5- or 6-membered heteroaryl containing 1 or 2 heteroatoms selected from N, O and S;
$Cy_2$ may be optionally substituted with 1 to 3 $R^{2b}$;
$R^{2b}$ is selected from the group consisting of H, halogen, OH, CN, oxo, $NR^bR^c$; $C_{1-}C_6$ alkyl; $C_{1-}C_6$ alkyl substituted with halogen, CN, OH, $NR^bR^c$ or $C_{1-}C_6$ alkoxy; $C_1$-$C_6$ alkyl optionally interrupted by 1 to 3 oxygen atoms and/or nitrogen atoms; and $C_{1-}C_6$ alkyl substituted with hydroxy-($C_1$-$C_6$ alkyl)amino-;
B is H, $C_{1-}C_6$ alkyl, $C_{1-}C_6$ haloalkyl, $C_{1-}C_6$ hydroxyalkyl, $C_{1-}C_6$ alkoxy-$C_{1-}C_6$ alkyl, $C_{1-}C_6$ alkyl substituted with $NR^bR^c$, -$(CH_2)_o$-$Cy_3$ or -$(CH_2)_o$-$Cy_3$-W-$Cy_4$;
W is NH, C(O) or a direct bond;
o is an integer of 0 or 1;
$Cy_3$ is selected from the group consisting of $C_{3-}C_8$ cycloalkyl, $C_{3-}C_8$ cycloalkenyl, 5- or 6-membered saturated or partially unsaturated heterocycloalkyl containing 1 or 2 heteroatoms selected from N, O and S, bridged bicyclic $C_{5-10}$ cycloalkyl, $C_6$-$C_{10}$ aryl, phenyl fused with a 5- or 6-membered cyclic group containing 1 heteroatom selected from N, O and S, and 5- to 10-membered heteroaryl containing 1 to 3 heteroatoms selected from N, O and S;
$Cy_3$ may be optionally substituted with 1 to 3 $R^{3a}$,
$R^{3a}$ is selected from the group consisting of H, halogen, OH, CN, oxo, $C_{1-}C_6$ alkyl; $C_{1-}C_6$ alkyl substituted with halogen, OH, CN or $C_{1-}C_6$ alkoxy; $C_3$-$C_6$ cycloalkyl, $C_{1-}C_6$ alkoxy, $C_{1-}C_6$ haloalkoxy, $C_{1-}C_6$ haloalkylamino, $C_{1-}C_6$ hydroxyalkylamino, ($C_3$-$C_6$ cycloalkyl)carbonylamino, - $NR^bR^c$, -$NR^bCOR^c$, -$NR^bC(O)OR^c$, -$SO_2R^b$, -C(O)$R^b$, -C(O)O$R^b$, -$NR^bSO_2R^c$ and -CON$R^{b1}R^{c1}$;
$Cy_4$ is selected from the group consisting of saturated or partially unsaturated 4- to 10-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S;
$Cy_4$ may be optionally substituted with 1 to 3 $R^{3b}$,
$R^{3b}$ is H, deuterium, halogen, OH, CN, oxo, $NR^bR^c$, $C_{1-}C_6$ alkyl, $C_{1-}C_6$ alkyl substituted with deuterium, $C_{1-}C_6$ haloalkyl, $C_{1-}C_6$ hydroxyalkyl, $C_{1-}C_6$ alkoxy-$C_{1-}C_6$ alkyl, $C_{1-}C_6$ alkoxy or $C_{1-}C_6$ haloalkoxy;
$R^b$ and $R^c$ are each independently H or $C_1$-$C_6$ alkyl; and
one of $R^{b1}$ and $R^{c1}$ is H or $C_{1-}C_6$ alkyl, and the other of $R^{b1}$ and $R^{c1}$ is H, $C_{1-}C_6$ alkyl, $C_{1-}C_6$ alkyl substituted with $NR^bR^c$, or $C_{1-}C_6$ alkyl substituted with $C_{1-}C_6$ alkoxy.

2. The compound, or solvate, stereoisomer or pharmaceutically acceptable salt thereof according to claim 1, **characterized in that** in Formula I above,

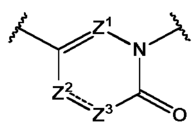

is selected from the group consisting of

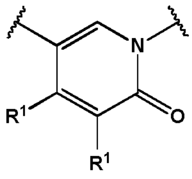 , 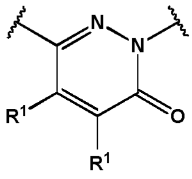 ,

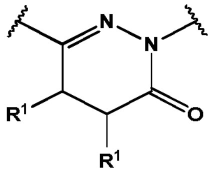 , 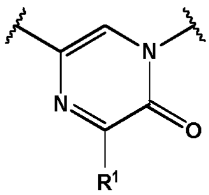 , 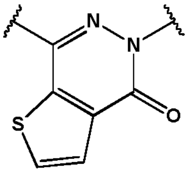 , 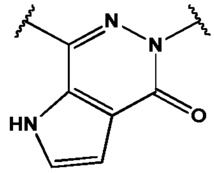 , and 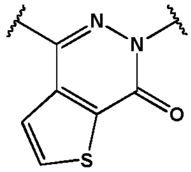 ,

wherein said $R^1$ is the same or different from each other.

3. The compound, or solvate, stereoisomer or pharmaceutically acceptable salt thereof according to claim 2, **characterized in that** in Formula I above,

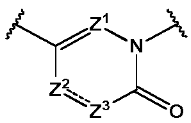 is 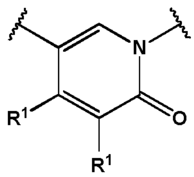 or 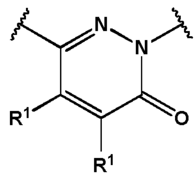 ,

wherein said $R^1$ is the same or different from each other.

4. The compound, or solvate, stereoisomer or pharmaceutically acceptable salt thereof according to claim 3, **characterized in that** each $R^1$ is independently selected from the group consisting of H, F, Br, Cl, I, CN, OH, $OCH_3$, amino, methylamino, dimethylamino, ethylamino, acetylamino, methylsulfonylamino, ethylsulfonylamino, methyl, ethyl, ethenyl, ethynyl, cyclopropyl, cyclobutyl, cyclopentyl, phenyl, phenoxy, benzyloxy and phenylamino.

5. The compound, or solvate, stereoisomer or pharmaceutically acceptable salt thereof according to claim 4, **characterized in that** one of two $R^1$ substituted on the same ring is H and the other is not H.

6. The compound, or solvate, stereoisomer or pharmaceutically acceptable salt thereof according to claim 4, **characterized in that** two $R^1$ substituted on the same ring are both H.

7. The compound, or solvate, stereoisomer or pharmaceutically acceptable salt thereof according to claim 2, **characterized in that** in Formula I above,

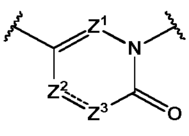 is 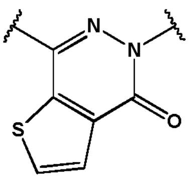 , 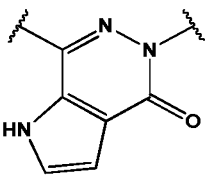 , or 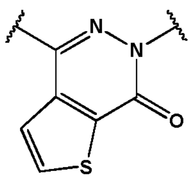 .

8. The compound, or solvate, stereoisomer or pharmaceutically acceptable salt thereof according to claim 1, **characterized in that** R' and R" are each H or $C_1$-$C_3$ alkyl, and R' and R" may be optionally taken together with the carbon atom to which they are attached to form $C_3$-$C_4$ cycloalkyl.

9. The compound, or solvate, stereoisomer or pharmaceutically acceptable salt thereof according to claim 8, **characterized in that** in Formula I,

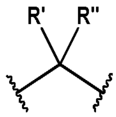 is 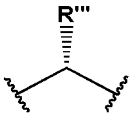, 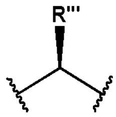, or 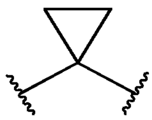,

and R‴ is methyl or ethyl.

**10.** The compound, or solvate, stereoisomer or pharmaceutically acceptable salt thereof according to claim 9, **characterized in that** the compound is a compound represented by Formula IA below:

[Formula IA]

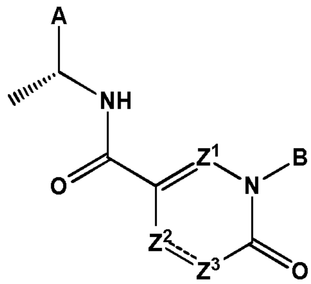

wherein A, $Z^1$, $Z^2$, $Z^3$ and B are as defined in claim 1.

**11.** The compound, or solvate, stereoisomer or pharmaceutically acceptable salt thereof according to claim 1, **characterized in that** A is $Cy_1$.

**12.** The compound, or solvate, stereoisomer or pharmaceutically acceptable salt thereof according to claim 11, **characterized in that** $Cy_1$ is $C_6$-$C_{10}$ aryl, or 5- or 6-membered heteroaryl containing 1 or 2 heteroatoms selected from N and S.

**13.** The compound, or solvate, stereoisomer or pharmaceutically acceptable salt thereof according to claim 12, **characterized in that** A is $Cy_1$, and $Cy_1$ is phenyl, naphthalenyl, thiophenyl or pyridinyl.

**14.** The compound, or solvate, stereoisomer or pharmaceutically acceptable salt thereof according to claim 11, **characterized in that** $Cy_1$ has any one of the following ring structures optionally substituted with 1 to 3 $R^{2a}$:

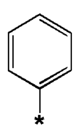 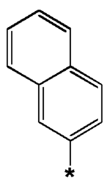 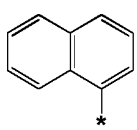 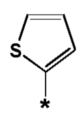 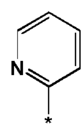.

**15.** The compound, or solvate, stereoisomer or pharmaceutically acceptable salt thereof according to claim 11, **characterized in that** $Cy_1$ may be optionally substituted with 1 to 3 $R^{2a}$, and each $R^{2a}$ is independently selected from the group consisting of F, Cl, Br, I, OH, CN, SFs, - $Si(CH_3)_3$, $CH_3SO_2$-, methyl, ethyl, propyl, isopropyl, $CF_3$, $CHF_2$, $CH_2F$, $NH_2$, $CH_3NH_2$-, $(CH_3)_2N$-, methoxy, ethoxy, $OCF_3$, $OCHF_2$, $OCH_2F$, cyclopropyl, cyclobutyl, cyclopentyl,

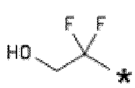,

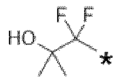, 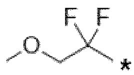, 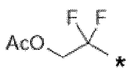, 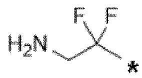, 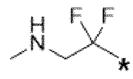 and 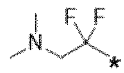 .

**16.** The compound, or solvate, stereoisomer or pharmaceutically acceptable salt thereof according to claim 1, **characterized in that** A is $Cy_1$-Y-$Cy_2$.

**17.** The compound, or solvate, stereoisomer or pharmaceutically acceptable salt thereof according to claim 16, **characterized in that**

$Cy_1$ is $C_6$-$C_{10}$ aryl, or 5- or 6-membered heteroaryl containing 1 or 2 heteroatoms selected from N and S;
Y is O or a direct bond; and
$Cy_2$ is $C_6$-$C_{10}$ aryl, phenyl fused with $C_3$-$C_5$ cycloalkyl, or 5- or 6-membered heteroaryl containing 1 or 2 heteroatoms selected from N and S.

**18.** The compound, or solvate, stereoisomer or pharmaceutically acceptable salt thereof according to claim 17, **characterized in that**

$Cy_1$ is phenyl, and $Cy_2$ is phenyl, pyrrolyl, pyrazolyl, thiophenyl, pyridinyl, or 2-oxo-1,2-dihydropyridinyl, or $Cy_1$ is thiazolyl, thiophenyl or pyrazolyl, and $Cy_2$ is phenyl, 2,3-dihydroindenyl or bicyclo[4.2.0]octa-1,3,5-trienyl.

**19.** The compound, or solvate, stereoisomer or pharmaceutically acceptable salt thereof according to claim 17, **characterized in that** Y is a direct bond.

**20.** The compound, or solvate, stereoisomer or pharmaceutically acceptable salt thereof according to any one of claims 16 to 19, **characterized in that** $Cy_1$-Y-$Cy_2$ has any one of the following ring structures optionally substituted with $R^{2a}$ and $R^{2b}$:

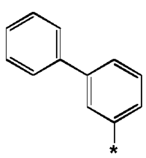 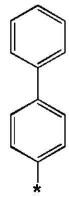 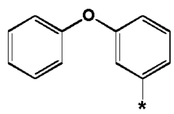 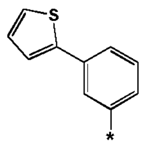 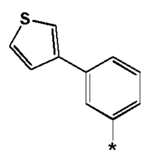 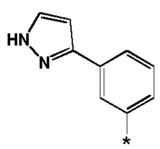 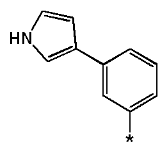

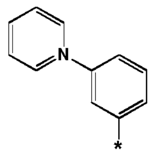 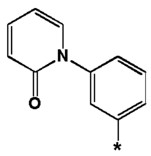 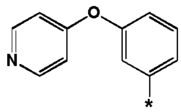 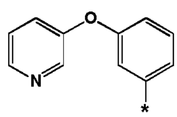 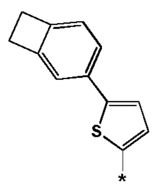 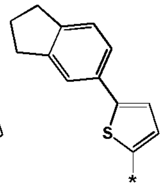

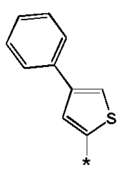 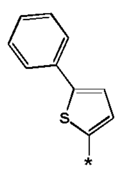 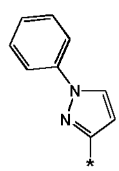 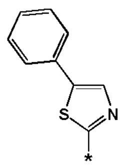 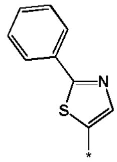 .

**21.** The compound, or solvate, stereoisomer or pharmaceutically acceptable salt thereof according to claim 16, **characterized in that**

$Cy_1$ is optionally substituted with one $R^{2a}$, wherein $R^{2a}$ is selected from the group consisting of H, halogen, OH, CN, amino, $C_1$-$C_6$ alkyl, $C_1$-$C_6$ haloalkyl, $C_1$-$C_6$ alkoxy and $C_1$-$C_6$ haloalkoxy; and
$Cy_2$ is optionally substituted with 1 to 3 $R^{2b}$, wherein $R^{2b}$ is selected from the group consisting of H, halogen,

OH, CN, oxo, $NR^bR^c$; $C_{1-}C_6$ alkyl; $C_{1-}C_6$ alkyl substituted with halogen, CN, OH, $NR^bR^c$ or $C_{1-}C_6$ alkoxy; $C_1$-$C_6$ alkyl optionally interrupted by 1 to 3 oxygen atoms and/or nitrogen atoms; and $C_1$-$C_6$ alkyl substituted with hydroxy-($C_1$-$C_6$ alkyl)amino-.

**22.** The compound, or solvate, stereoisomer or pharmaceutically acceptable salt thereof according to claim 21, **characterized in that** $R^{2a}$ is H.

**23.** The compound, or solvate, stereoisomer or pharmaceutically acceptable salt thereof according to claim 21, **characterized in that**

$R^{2a}$ is H; and
each $R^{2b}$ is independently selected from the group consisting of H, F, Cl, Br, I, OH, CN, oxo, amino, $CH_3NH$-, $(CH_3)_2N$-, $(CH_3)_2NCH_2$- methyl, ethyl, cyanomethyl, hydroxymethyl, aminomethyl, $CH_3NHCH_2$-, $C_2H_5NHCH_2$- and $HOC_2H_4NHCH_2$-.

**24.** The compound, or solvate, stereoisomer or pharmaceutically acceptable salt thereof according to claim 1, **characterized in that** A in Formula I is selected from the following structures:

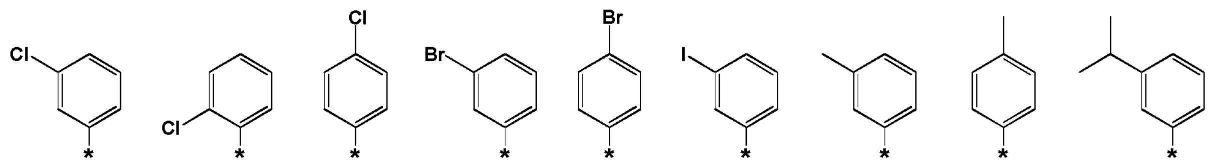

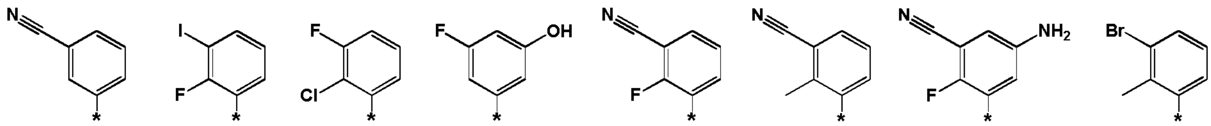

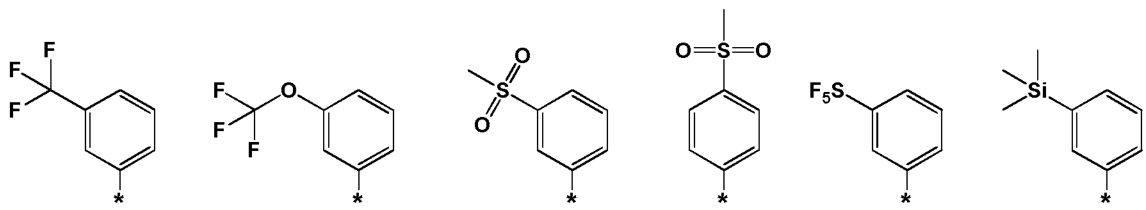

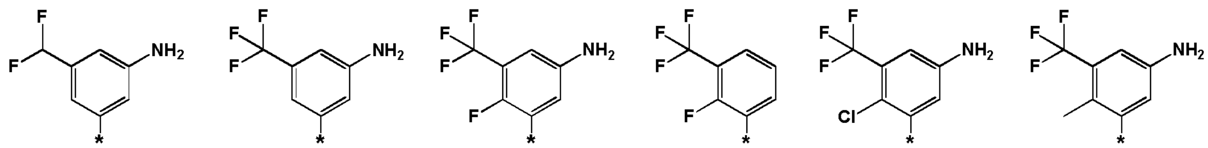

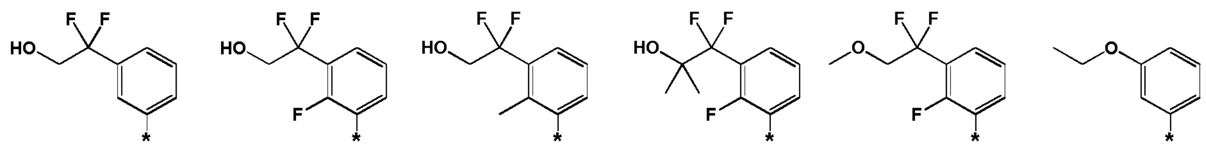

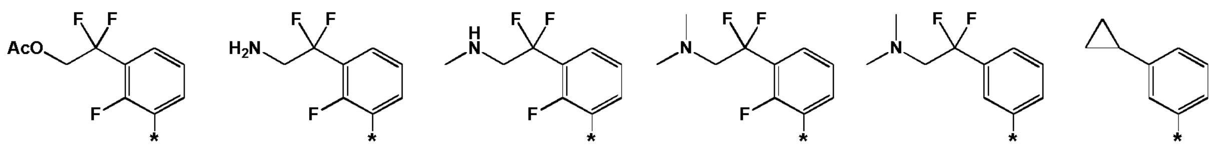

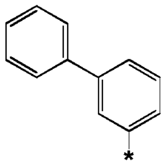
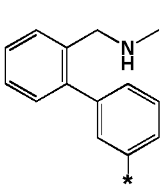
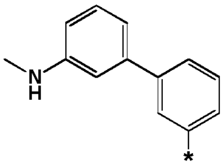
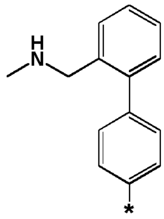
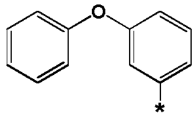
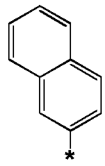

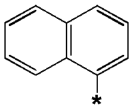
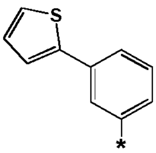
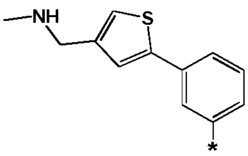
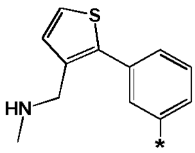
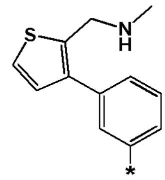
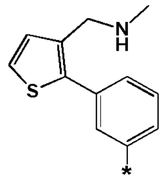

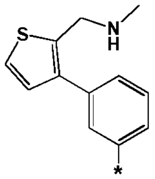
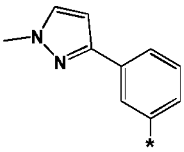
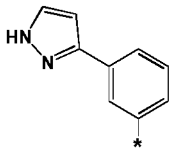
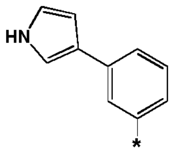
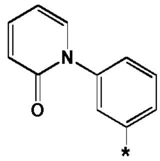
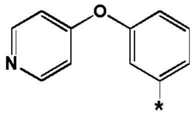

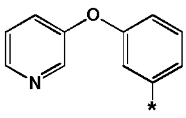
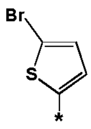
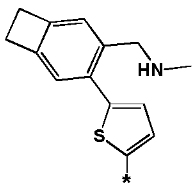
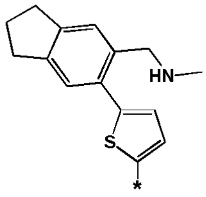
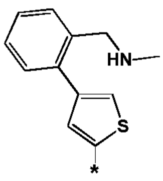
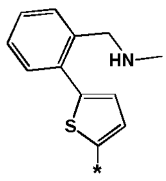

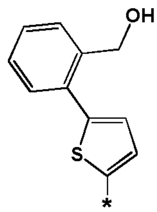
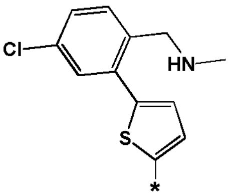
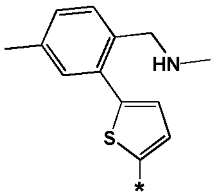
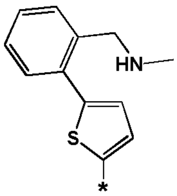
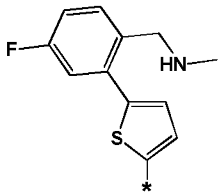

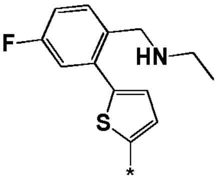
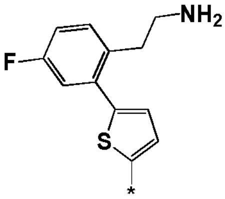
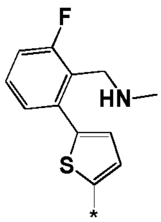
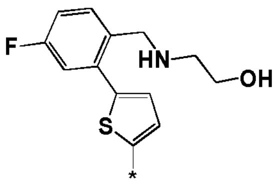
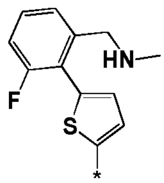

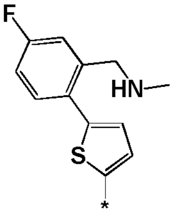
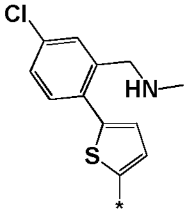
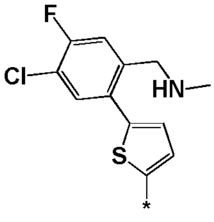
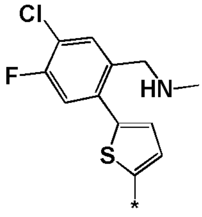
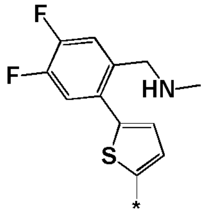

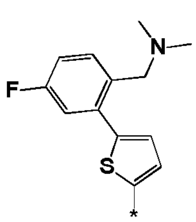 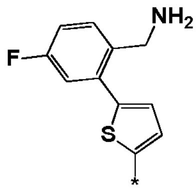 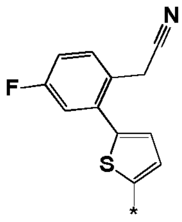 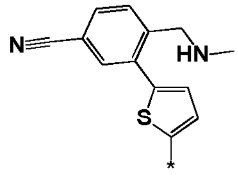 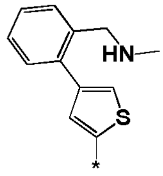

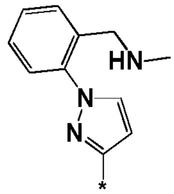 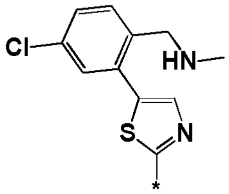 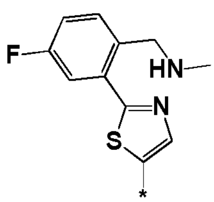 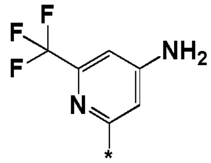

**25.** The compound, or solvate, stereoisomer or pharmaceutically acceptable salt thereof according to claim 1, **characterized in that** B is H, $C_{1-}C_6$ alkyl, $C_{1-}C_6$ haloalkyl, $C_{1-}C_6$ hydroxyalkyl, $C_{1-}C_6$ alkoxy-$C_{1-}C_6$ alkyl, or $C_{1-}C_6$ alkyl substituted with $NR^bR^c$.

**26.** The compound, or solvate, stereoisomer or pharmaceutically acceptable salt thereof according to claim 1, **characterized in that** B is $-(CH_2)_o-Cy_3$, and o is 0 or 1.

**27.** The compound, or solvate, stereoisomer or pharmaceutically acceptable salt thereof according to claim 26, **characterized in that** $Cy_3$ is selected from the group consisting of $C_{3-}C_8$ cycloalkyl, $C_{3-}C_8$ cycloalkenyl, 6-membered saturated or partially unsaturated heterocycloalkyl containing one N, O or S, bridged bicyclic $C_{5-8}$ cycloalkyl, $C_6$-$C_{10}$ aryl, phenyl fused with 5-membered heterocycloalkyl containing one N, O or S, 5- or 6-membered heteroaryl containing 1 or 2 heteroatoms selected from N or S, and 9- or 10-membered bicyclic heteroaryl containing 1 to 3 N.

**28.** The compound, or solvate, stereoisomer or pharmaceutically acceptable salt thereof according to claim 27, **characterized in that** $Cy_3$ is $C_{3-}C_6$ cycloalkyl, $C_{3-}C_6$ cycloalkenyl, tetrahydropyranyl, dihydropyranyl, thianyl, 1,1-dioxothianyl, piperidinyl, dihydropyridinyl, tetrahydropyridinyl, bicyclo[1.1.1]pentanyl, bicyclo[2.2.1]heptanyl, $C_{6-10}$ aryl, thiophenyl, thiazolyl, pyrazolyl, pyridinyl, pyrimidinyl, dihydroisobenzofuranyl, indolyl, indazolyl or benzotriazolyl.

**29.** The compound, or solvate, stereoisomer or pharmaceutically acceptable salt thereof according to claim 26, **characterized in that** $Cy_3$ has any one of the following ring structures optionally substituted with 1 to 3 $R^{3a}$:

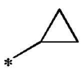 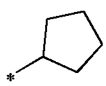 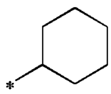 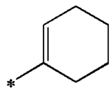 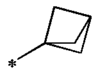 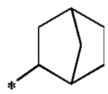 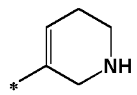 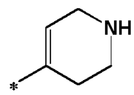

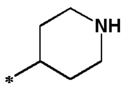 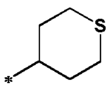 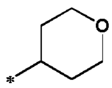 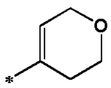 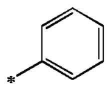 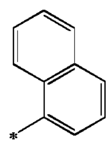 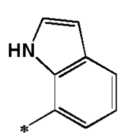 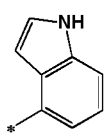

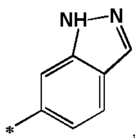 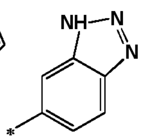 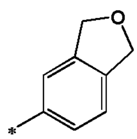 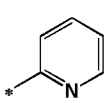 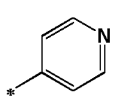 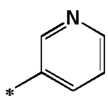 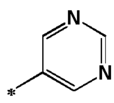 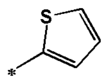

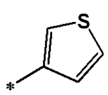 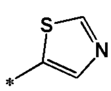 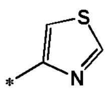 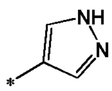 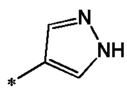

**30.** The compound, or solvate, stereoisomer or pharmaceutically acceptable salt thereof according to claim 26, **characterized in that** $R^{3a}$ is selected from the group consisting of H, F, Cl, Br, I, OH, CN, oxo, methyl, ethyl, amino, $CH_3NH$-, $(CH_3)_2NH$-, 1,1,1-trifluoropropan-2-yl amino, $CH_3CONH$-, $(CH_3CO)(CH_3)N$-, $CH_3OCONH$-, cyclopropylcarbonylamino, hydroxymethyl, 1-hydroxyethyl, 2-hydroxypropan-2-yl, methoxy, ethoxy, isopropoxy, methoxymethyl, 2-methoxyethyl, $OCHF_2$, $OCF_3$, $CH_3SO_2$-, $CH_3CO$-, $CH_3SO_2NH$-, -COOH, $-COOC(CH_3)_3$, - $CONH_2$, $-CONHCH_3$, $-CONHC_2H_5$, $-CON(CH_3)_2$, $-CONHC_2H_4OCH_3$ and $-CONHC_2H_4N(CH_3)_2$.

**31.** The compound, or solvate, stereoisomer or pharmaceutically acceptable salt thereof according to claim 1, **characterized in that** B is $-(CH_2)_o-Cy_3-W-Cy_4$, and o is 0.

**32.** The compound, or solvate, stereoisomer or pharmaceutically acceptable salt thereof according to claim 31, **characterized in that**

$Cy_3$ is $C_6$-$C_{10}$ aryl, or 5- or 6-membered heteroaryl containing 1 or 2 heteroatoms selected from N or S;
W is NH, C(O) or a direct bond; and
$Cy_4$ is selected from the group consisting of saturated or partially unsaturated 4- to 7-membered heterocycloalkyl containing 1 or 2 heteroatoms selected from N, O or S, $C_6$-$C_{10}$ aryl, and 5- or 6-membered heteroaryl containing 1 to 4 heteroatoms selected from N, O and S.

**33.** The compound, or solvate, stereoisomer or pharmaceutically acceptable salt thereof according to claim 32, **characterized in that** $Cy_3$ is $C_6$-$C_{10}$ aryl, $Cy_4$ is saturated or partially unsaturated 4- to 7-membered heterocycloalkyl containing 1 or 2 heteroatoms selected from N, O or S, and W is NH or C(O).

**34.** The compound, or solvate, stereoisomer or pharmaceutically acceptable salt thereof according to claim 32, **characterized in that** W is a direct bond.

**35.** The compound, or solvate, stereoisomer or pharmaceutically acceptable salt thereof according to claim 32, **characterized in that**

$Cy_3$ is phenyl or pyridinyl; and
$Cy_4$ is oxetanyl, tetrahydrofuranyl, pyrrolidinyl, 2-oxo-pyrrolidinyl, piperidinyl, morpholinyl, imidazolidinyl, 2-oxo-imidazolidinyl, piperazinyl, 2-oxo-piperazinyl, hexahydropyrimidinyl, 2-oxo-hexahydropyrimidinyl, phenyl, oxazolyl, isoxazolyl, thiazolyl, pyrazolyl, imidazolyl, oxadiazolyl, triazolyl, tetrazolyl, pyridinyl or 2-oxo-pyridinyl.

**36.** The compound, or solvate, stereoisomer or pharmaceutically acceptable salt thereof according to claim 31, **characterized in that** $Cy_3$-W-$Cy_4$ has any one of the following ring structures optionally substituted with $R^{3a}$ and $R^{3b}$:

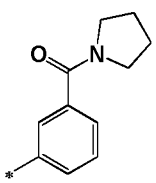 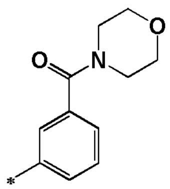 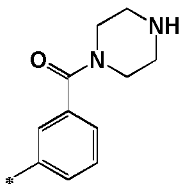 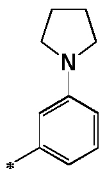 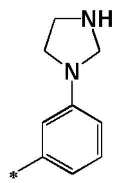 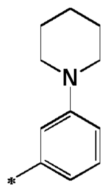 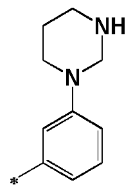 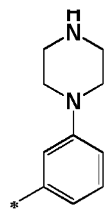

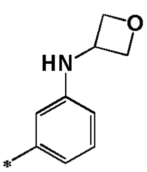 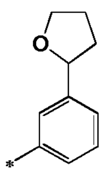 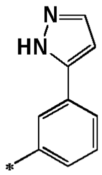 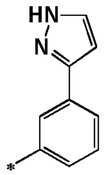 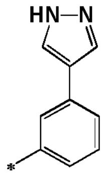 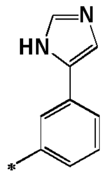 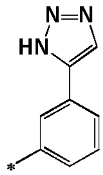 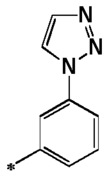 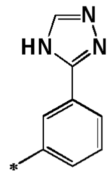

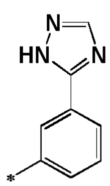 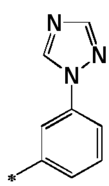 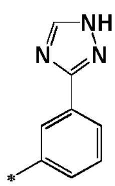 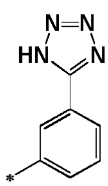 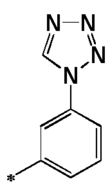 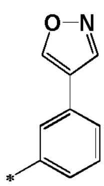 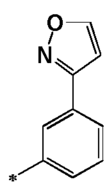 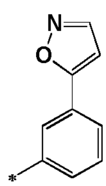 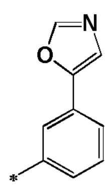

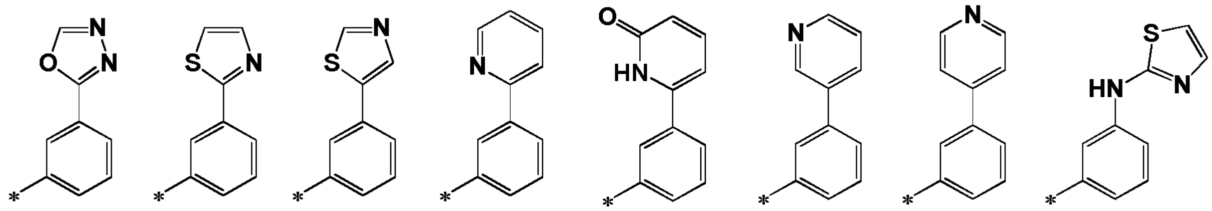

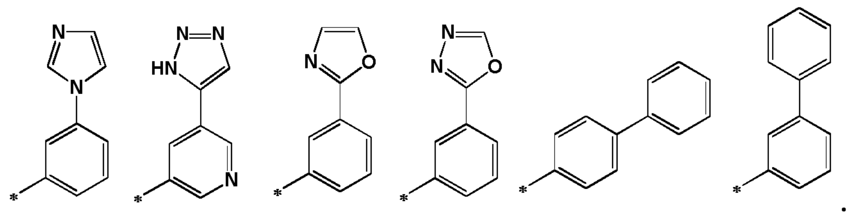

**37.** The compound, or solvate, stereoisomer or pharmaceutically acceptable salt thereof according to claim 31, **characterized in that**

$Cy_3$ may be optionally substituted with one or two $R^{3a}$, wherein $R^{3a}$ is H, halogen, OH or CN; and
$Cy_4$ may be optionally substituted with 1 to 3 $R^{3b}$, wherein $R^{3b}$ is H, deuterium, halogen, OH, CN, oxo, $C_{1-}C_6$ alkyl, $C_{1-}C_6$ alkyl substituted with deuterium or $C_{1-}C_6$ haloalkyl.

**38.** The compound, or solvate, stereoisomer or pharmaceutically acceptable salt thereof according to claim 37, **characterized in that**

$R^{3a}$ is H or F; and
$R^{3b}$ is selected from the group consisting of H, F, oxo, methyl, ethyl, $CHF_2$ and $CD_3$.

**39.** The compound, or solvate, stereoisomer or pharmaceutically acceptable salt thereof according to claim 31, **characterized in that** B is any one of the following structures: H, $CH_3$,

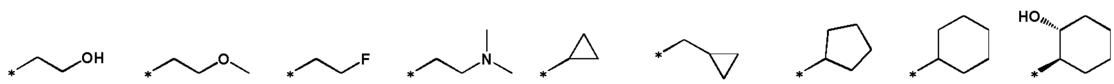

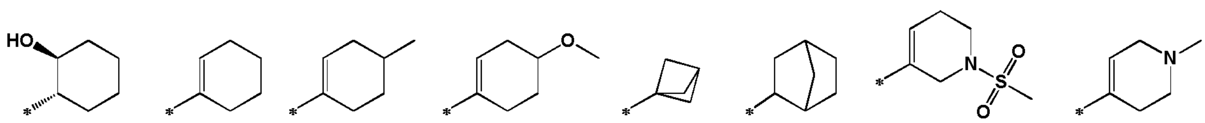

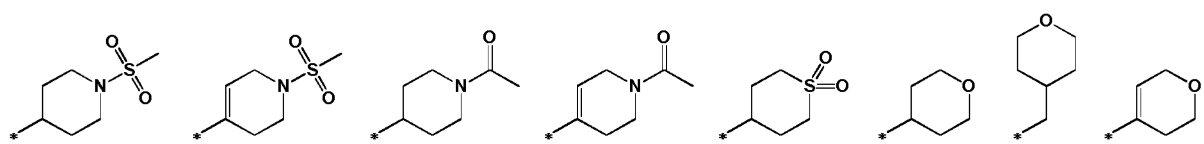

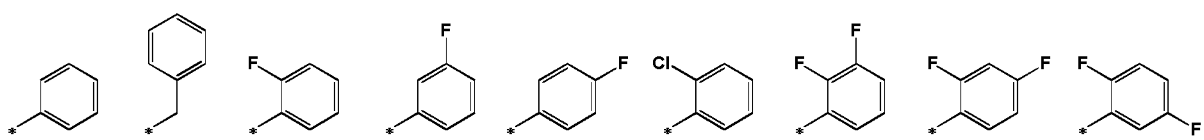

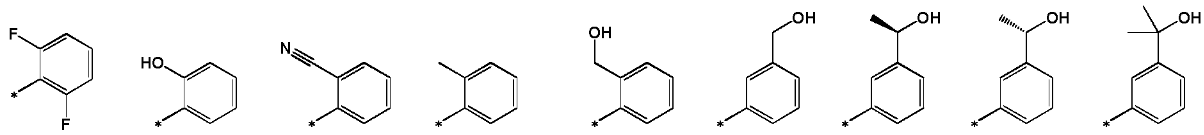

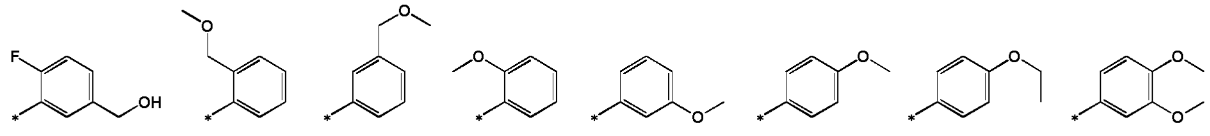

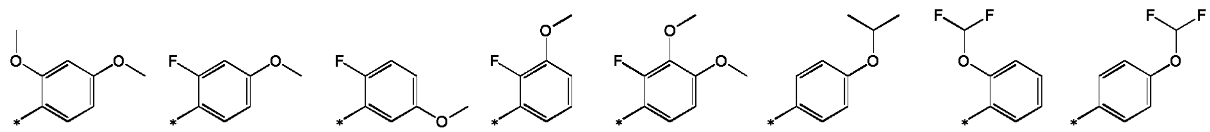

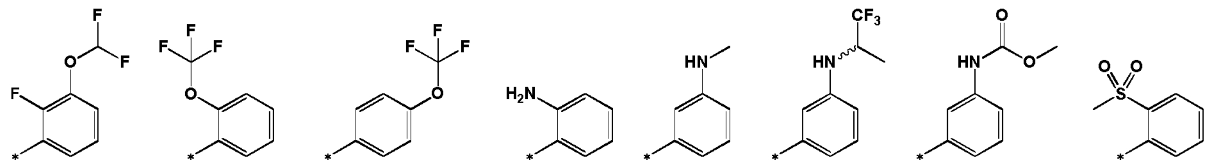

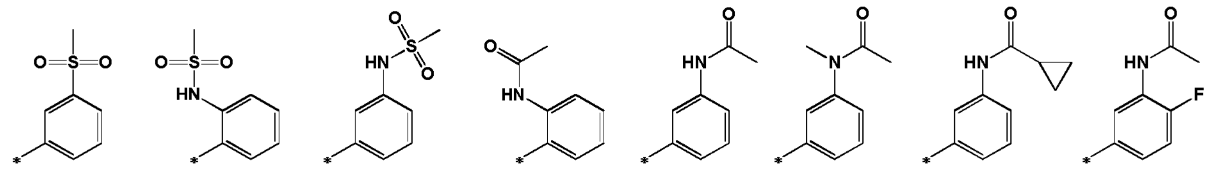

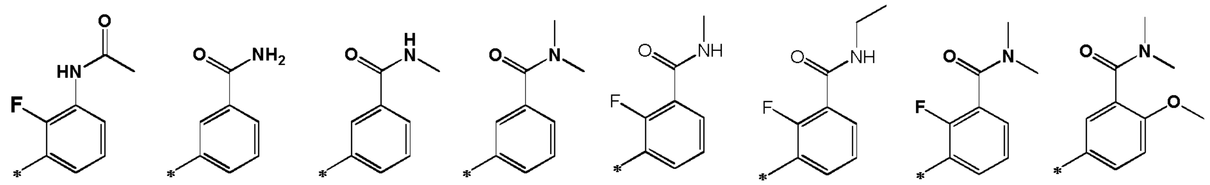

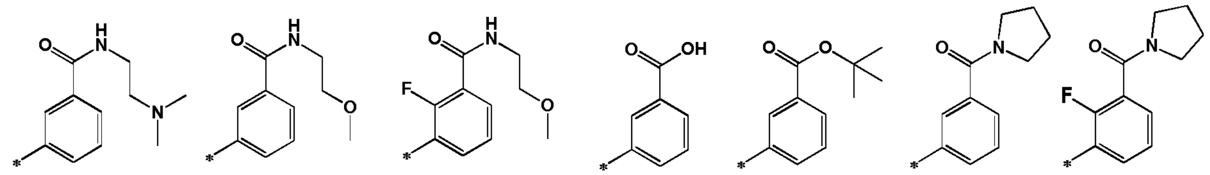

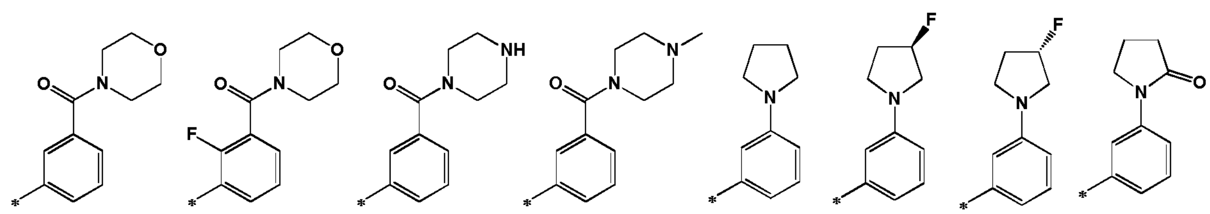

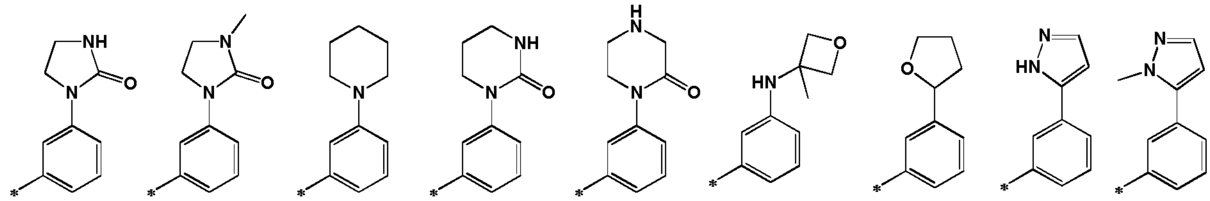

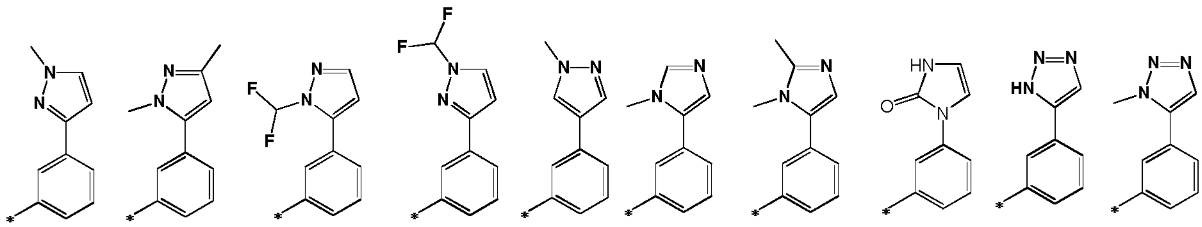

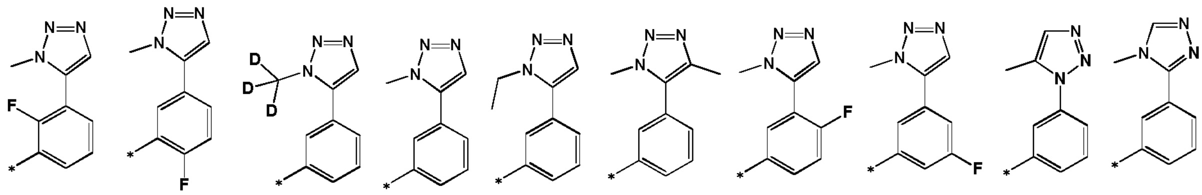

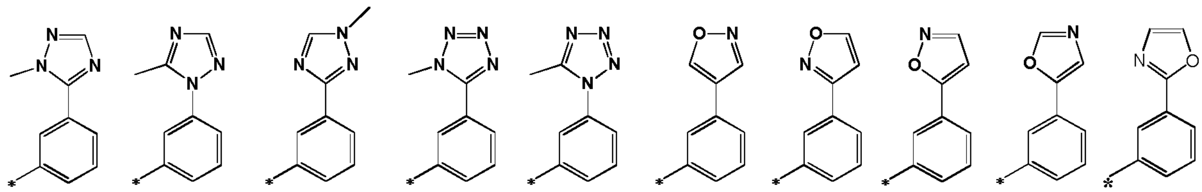

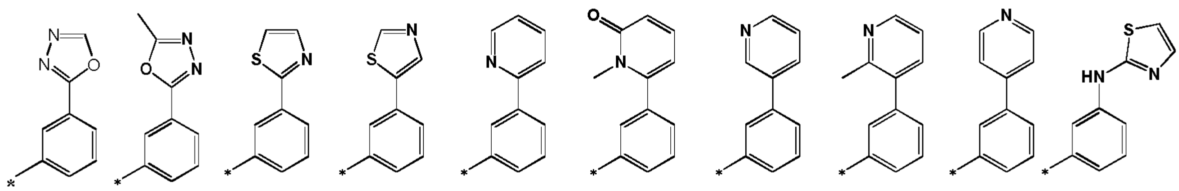

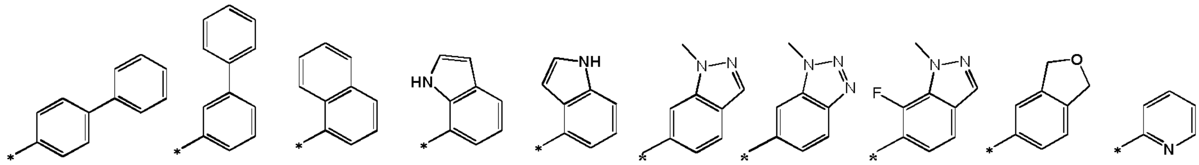

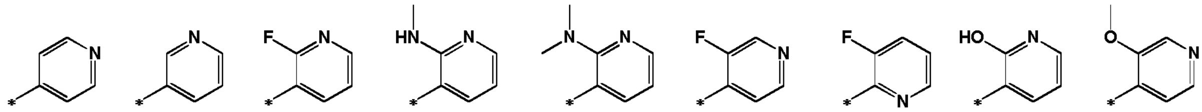

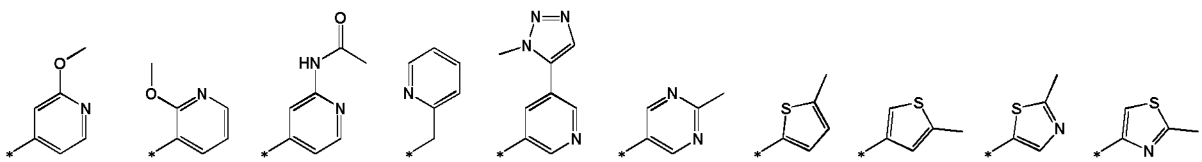

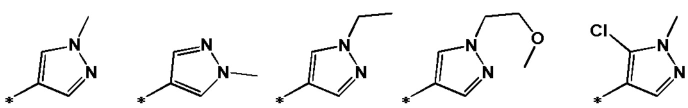.

40. The compound, or solvate, stereoisomer or pharmaceutically acceptable salt thereof according to claim 1, **characterized in that** the compound is selected from:

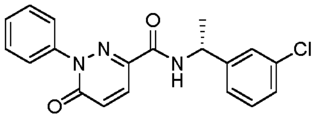 , 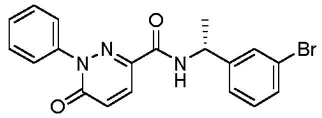 , 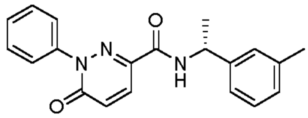 ,

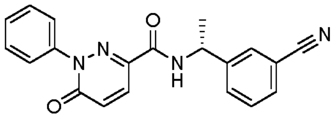 , 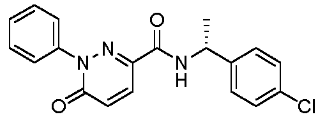 , 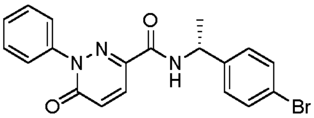 ,

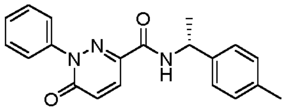 , 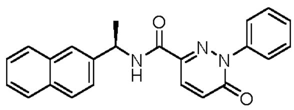 , 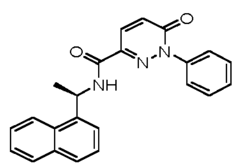 , 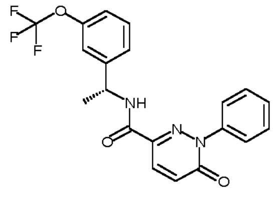 ,

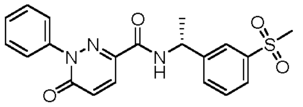 , 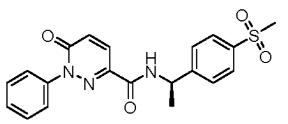 , 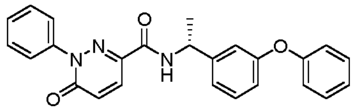 ,

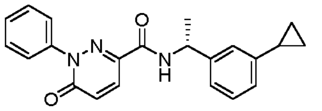 , 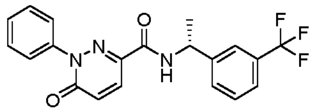 , 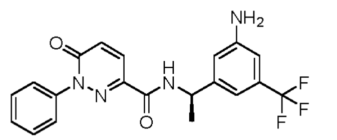 ,

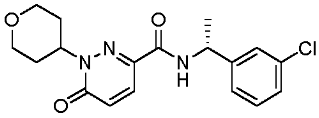 , 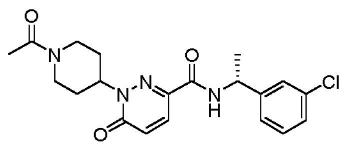 , 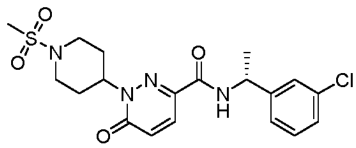 ,

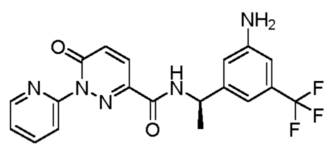 , 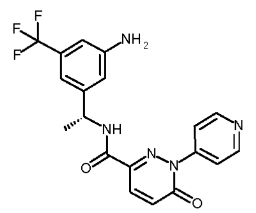 , 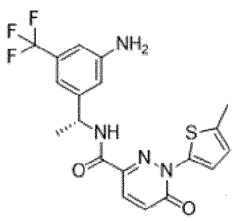 , 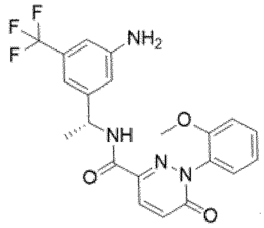 ,

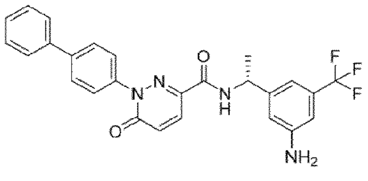 , 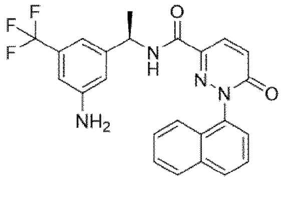 , 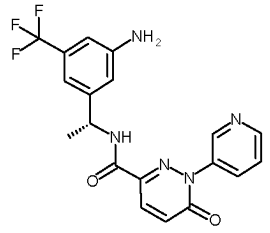 ,

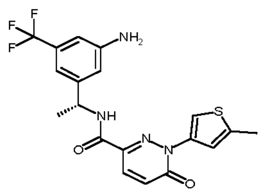 , 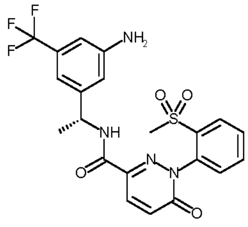 , 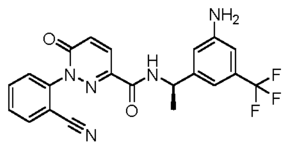 , 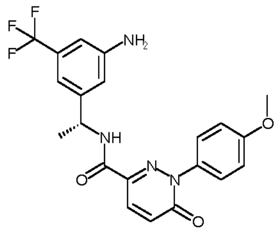 ,

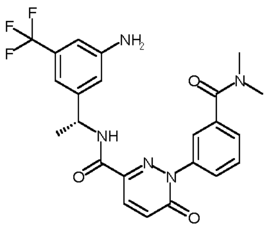 , 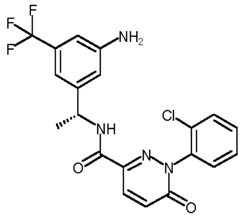 , 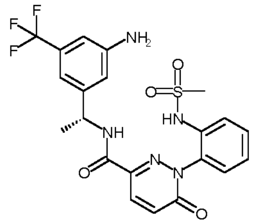 , 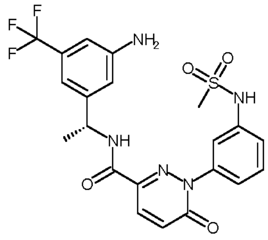 ,

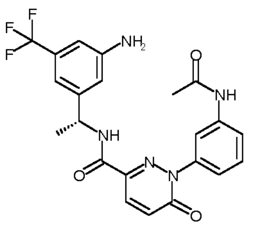 , 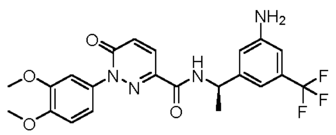 , 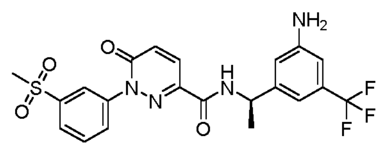 ,

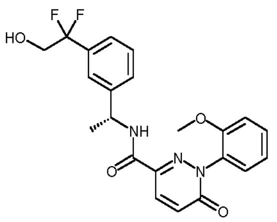 , 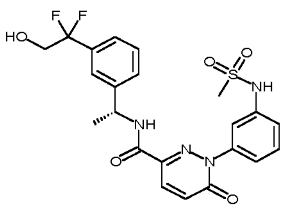 , 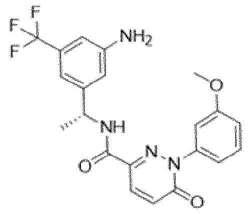 , 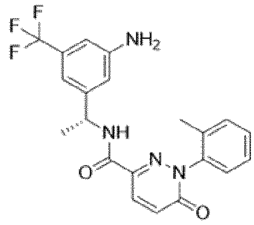 ,

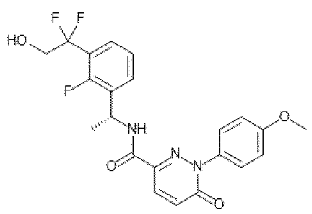 , 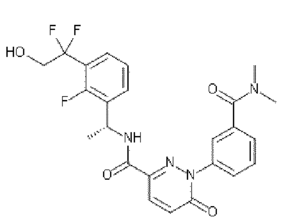 , 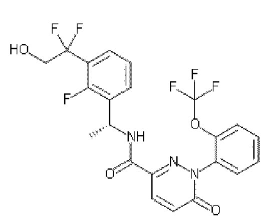 , 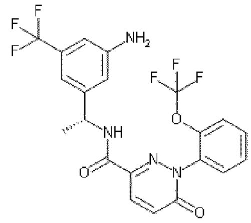 ,

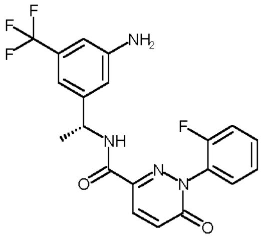 , 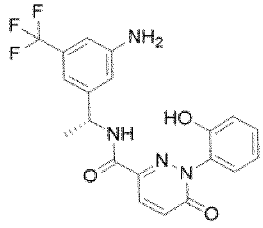 , 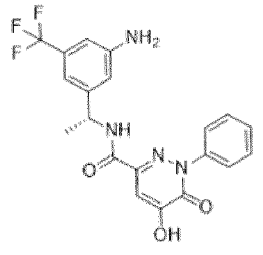 , 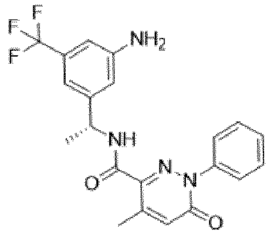 ,

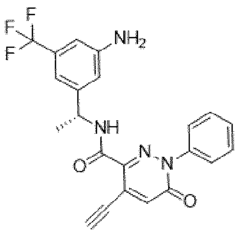,
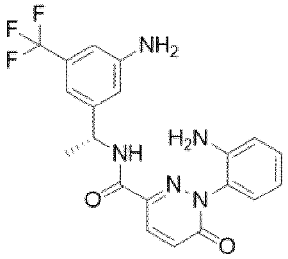,
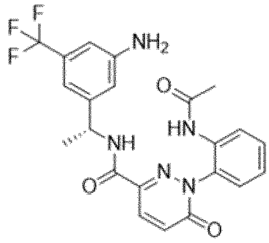,
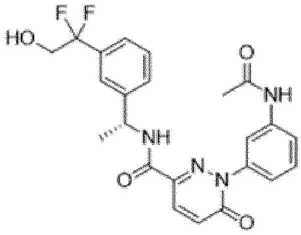,

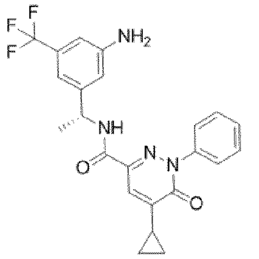,
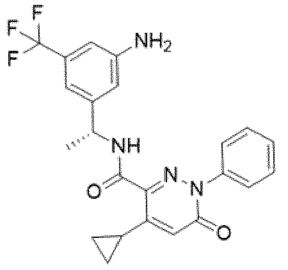,
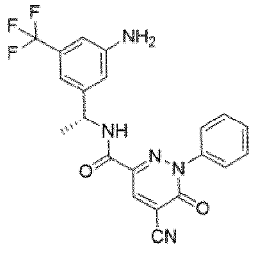,
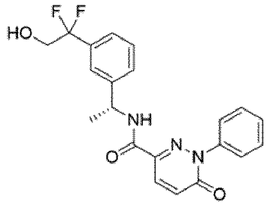,

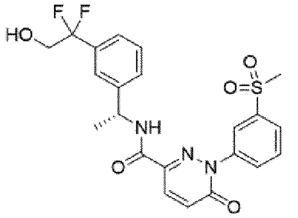,
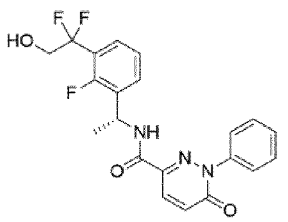,
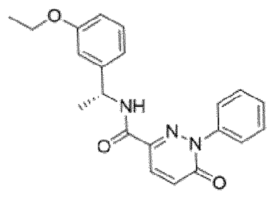,
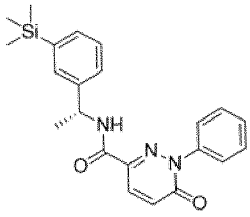,

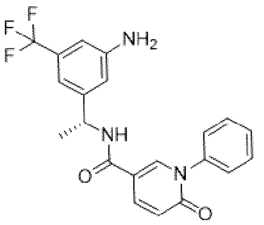,
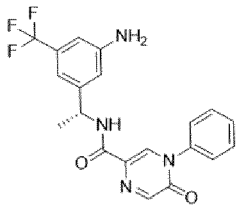,
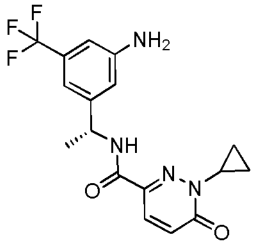,
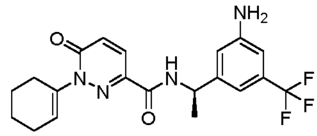,

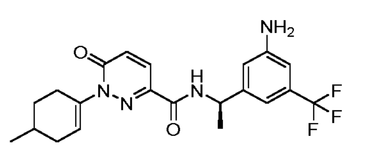,
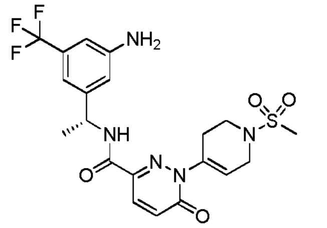,
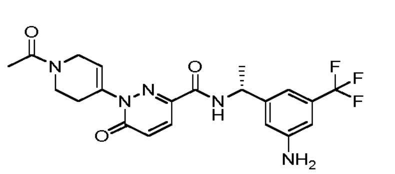,

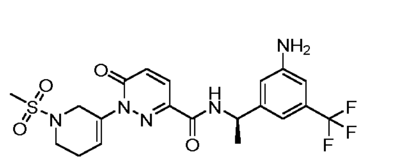,
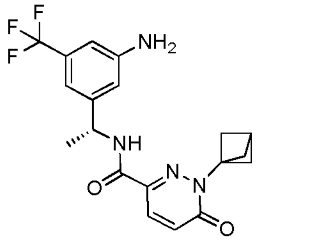,
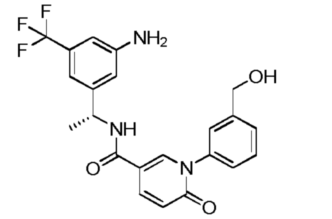,

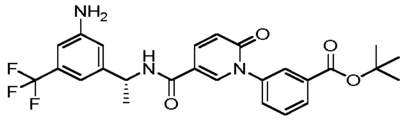 , 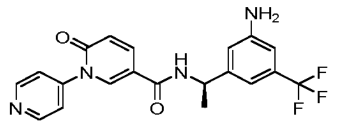 , 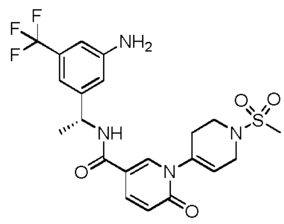 ,

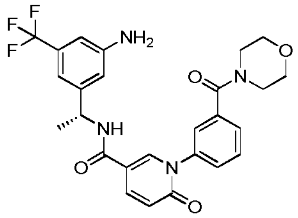 , 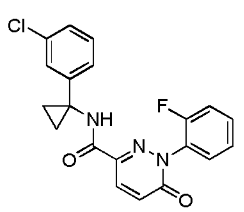 , 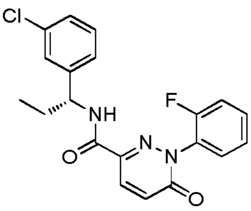 , 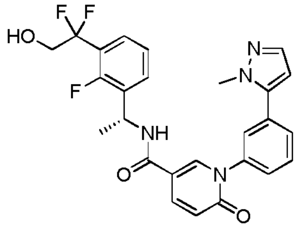 ,

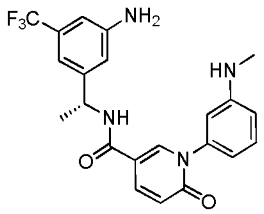 , 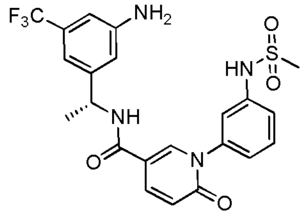 , 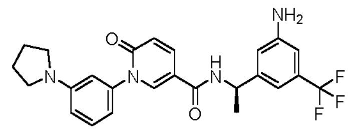 ,

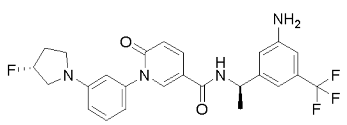 , 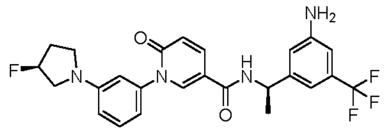 , 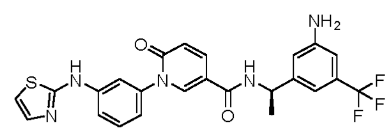 ,

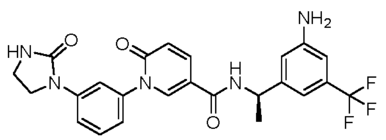 , 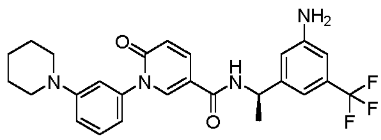 , 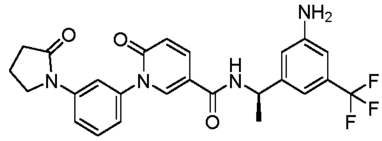 ,

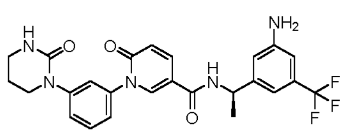 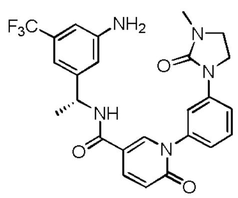 , 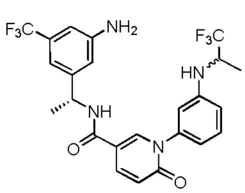 , 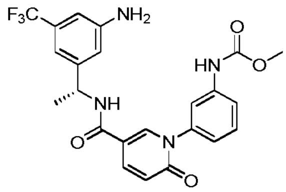 ,

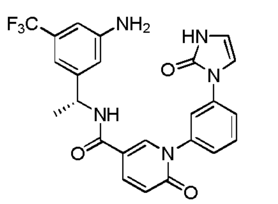 , 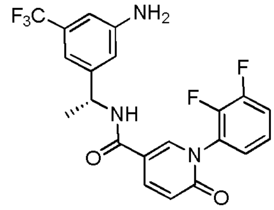 , 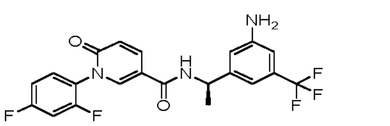 ,

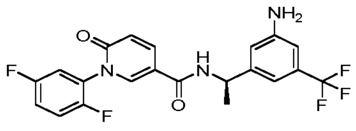 , 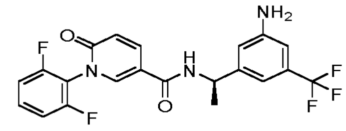 , 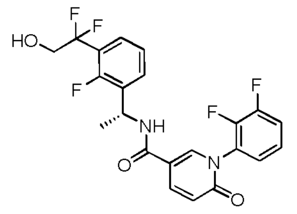 ,

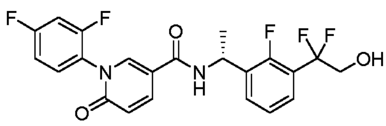 , 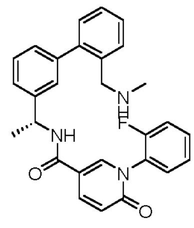 , 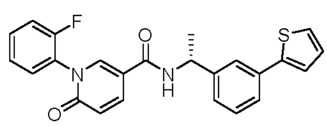 ,

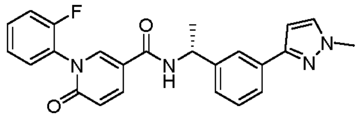 , 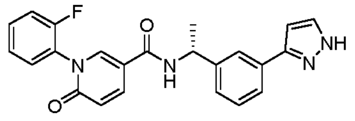 , 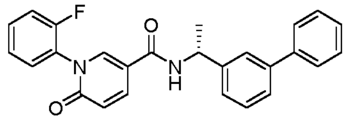 ,

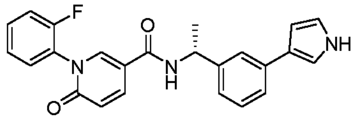 , 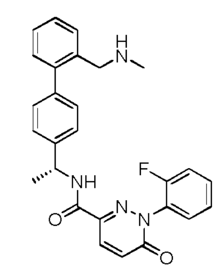 , 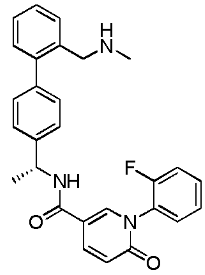 , 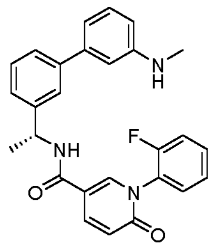 ,

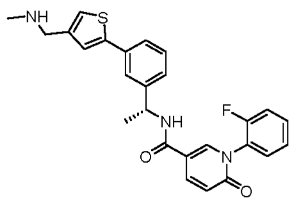 , 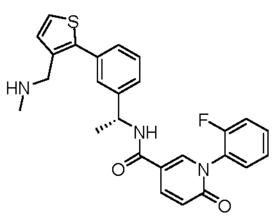 , 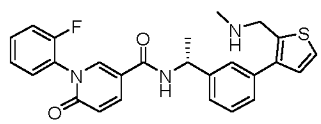 , 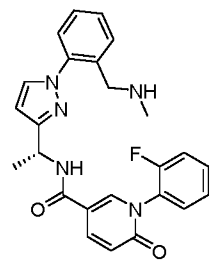 ,

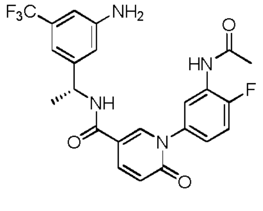 , 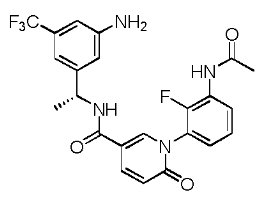 , 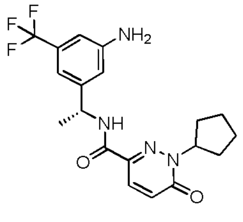 , 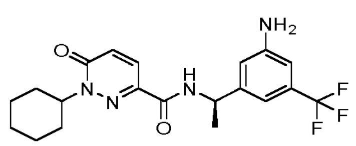 ,

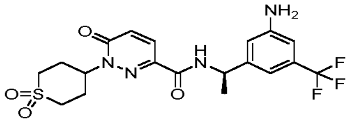 , 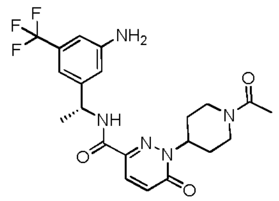 , 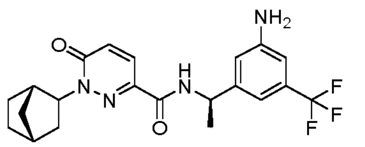 ,

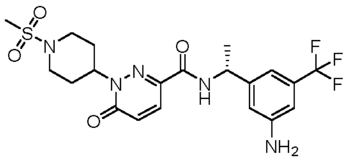 , 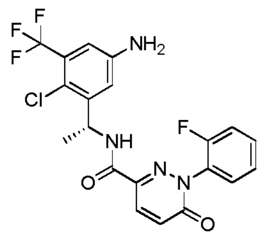 , 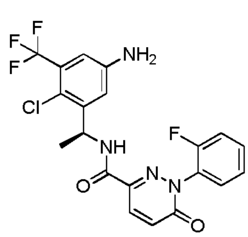 , 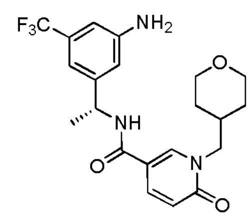 ,

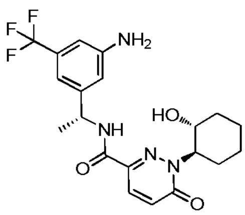 , 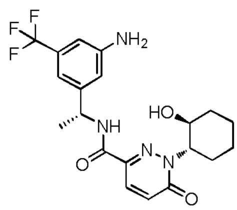 , 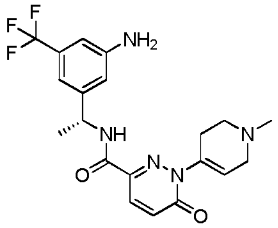 , 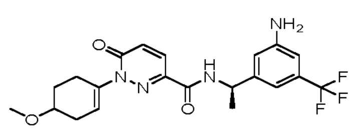 ,

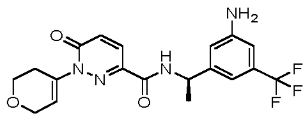 , 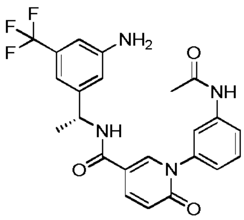 , 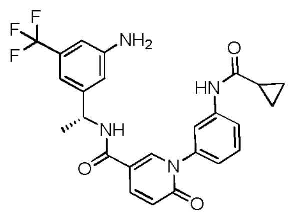 , 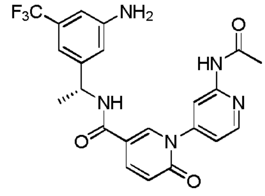 ,

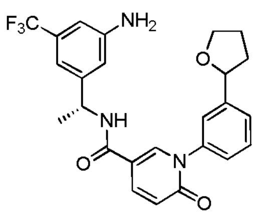 , 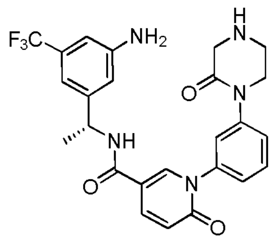 , 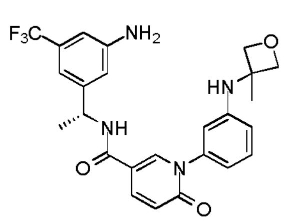 , 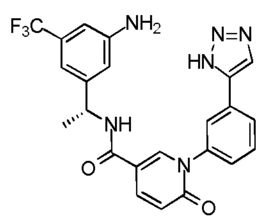 ,

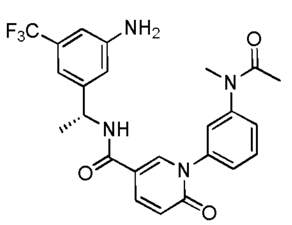 , 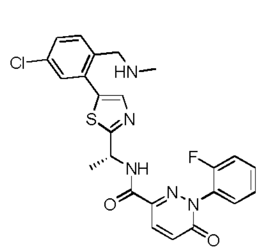 , 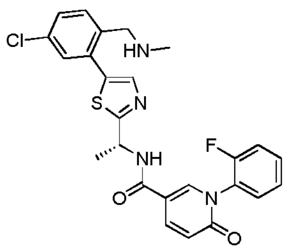 , 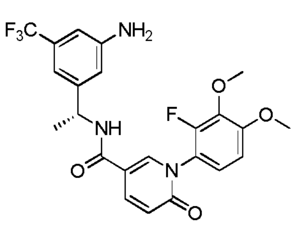 ,

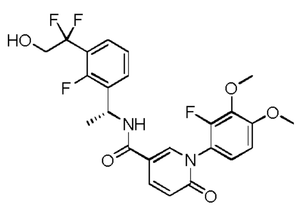 , 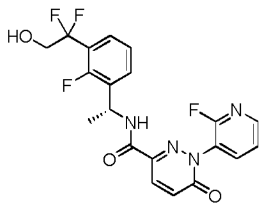 , 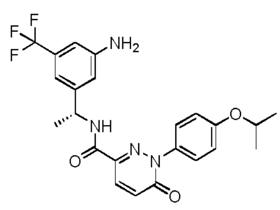 , 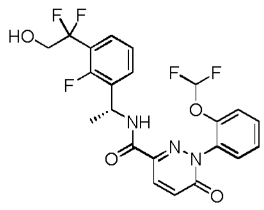 ,

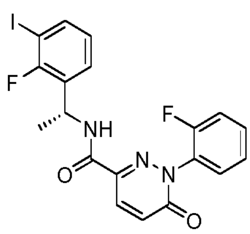 , 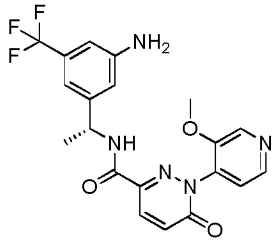 , 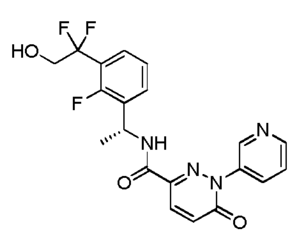 , 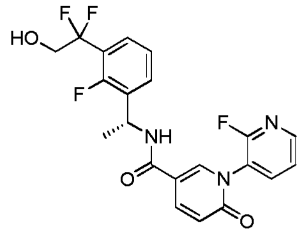 ,

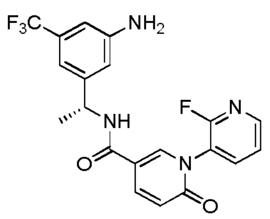 , 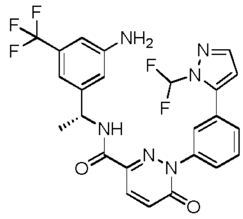 , 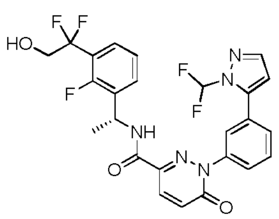 , 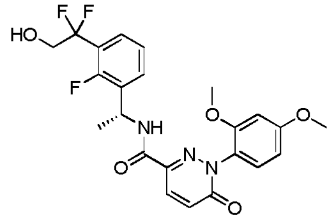 ,

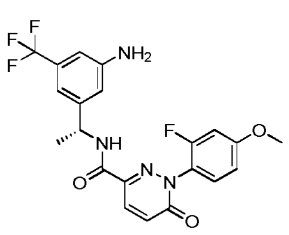 , 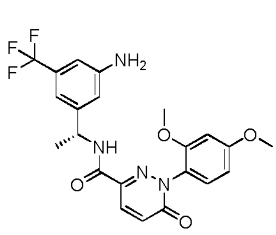 , 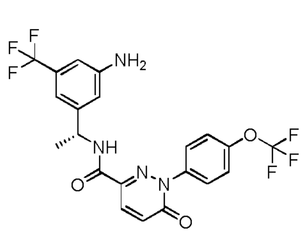 , 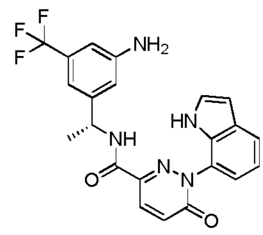 ,

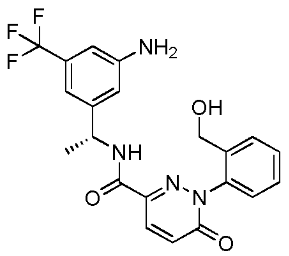 , 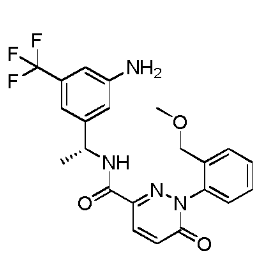 , 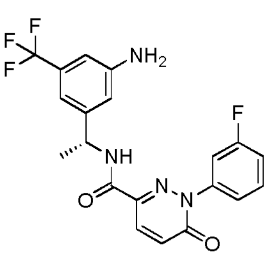 , 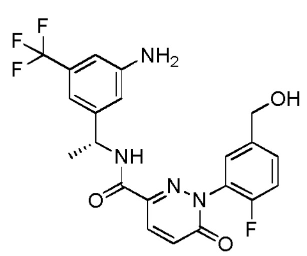 ,

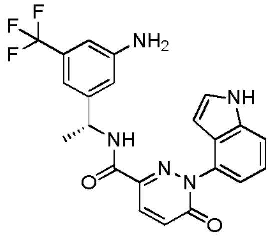 , 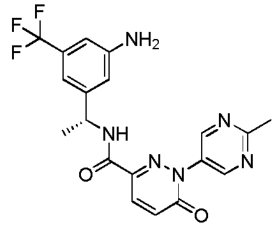 , 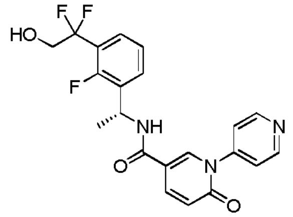 , 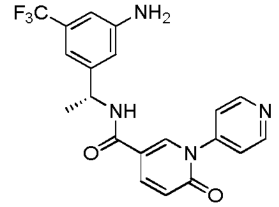 ,

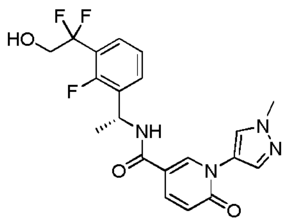 , 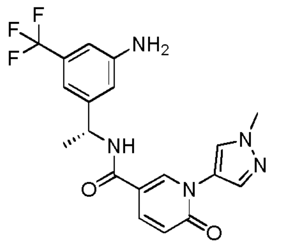 , 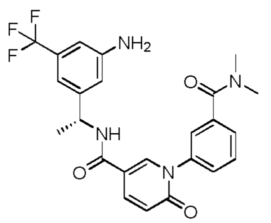 , 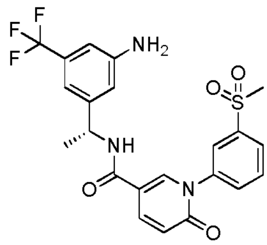 ,

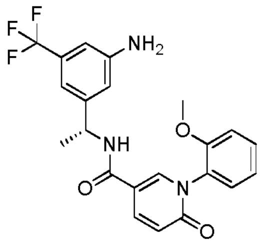 , 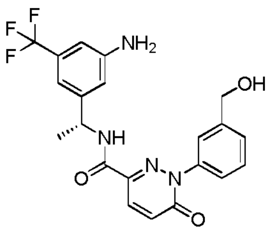 , 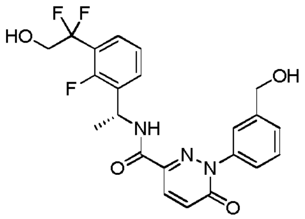 , 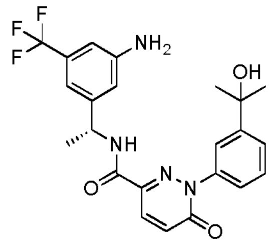 ,

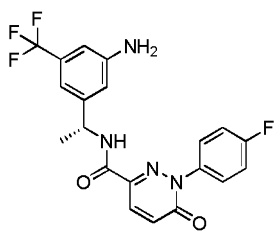 , 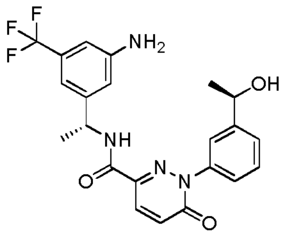 , 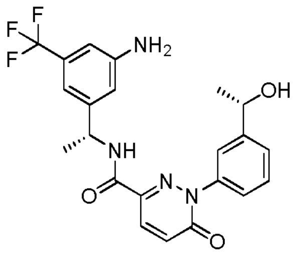 , 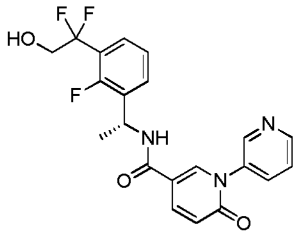 ,

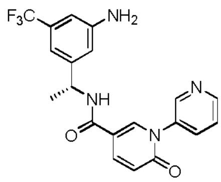 , 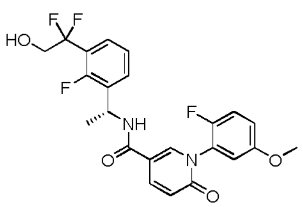 , 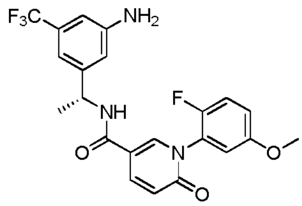 , 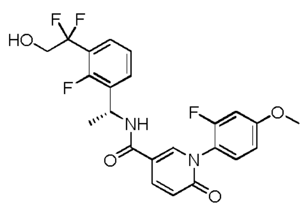 ,

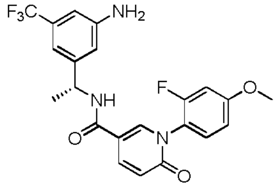 , 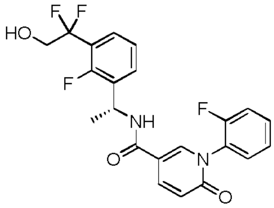 , 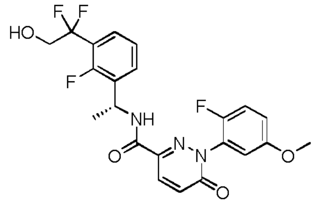 , 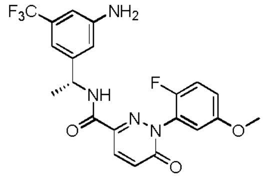 ,

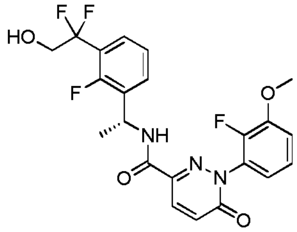 , 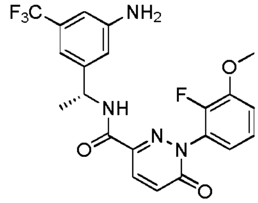 , 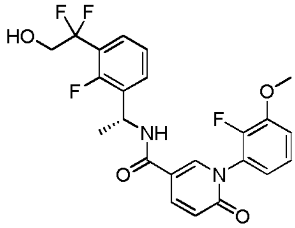 , 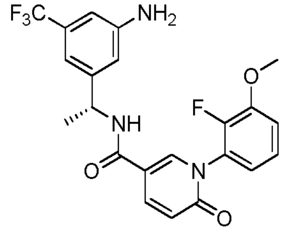 ,

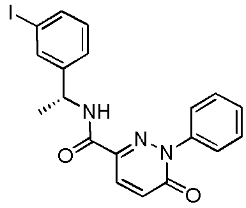, 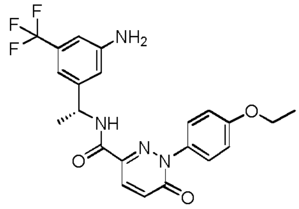, 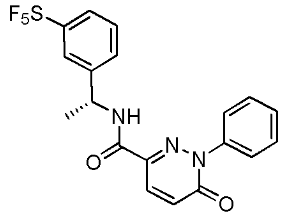, 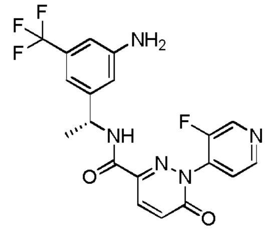,

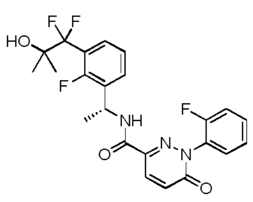, 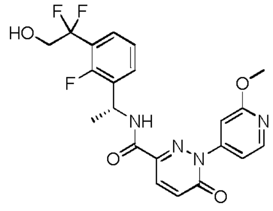, 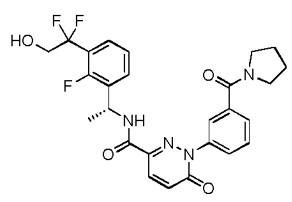, 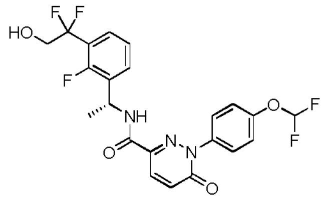,

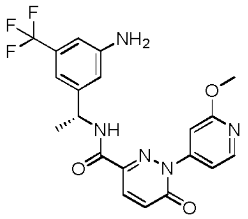, 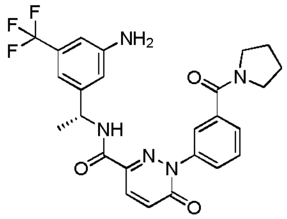, 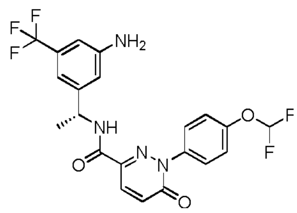, 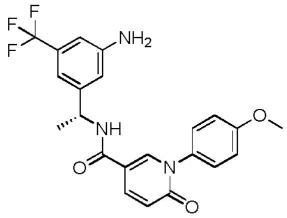,

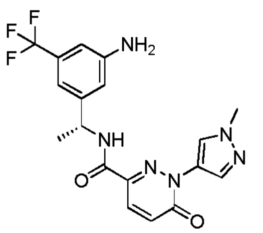, 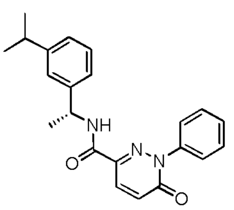, 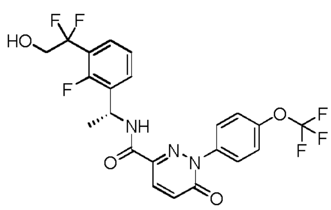, 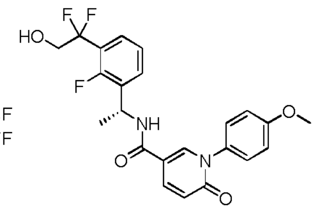,

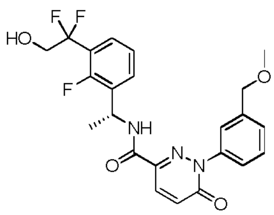, 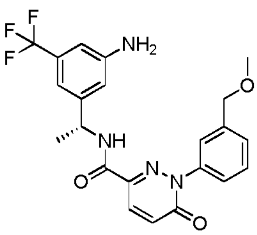, 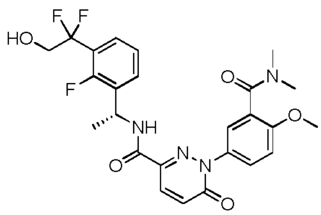, 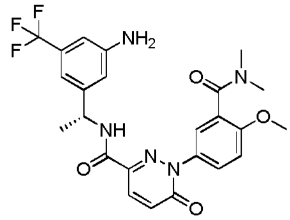,

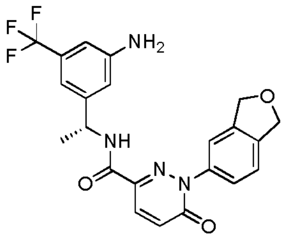, 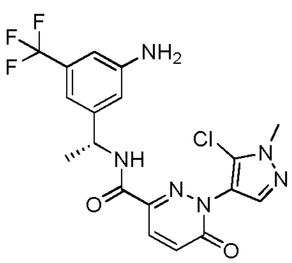, 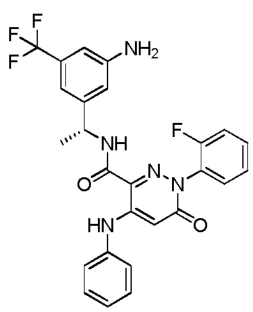, 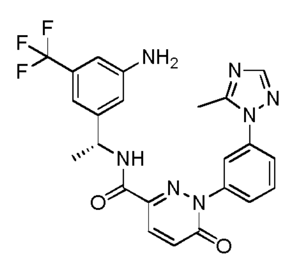,

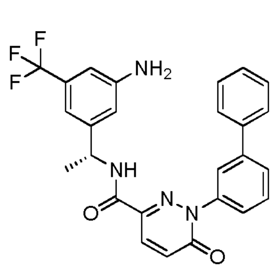, 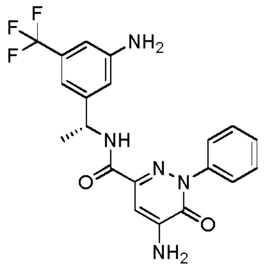, 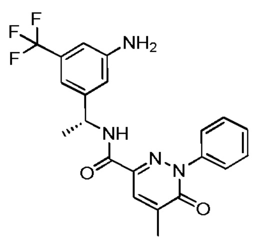, 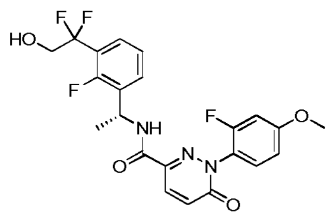,

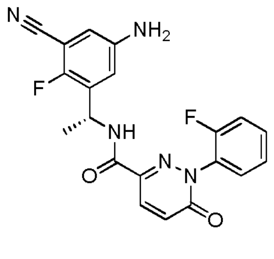, 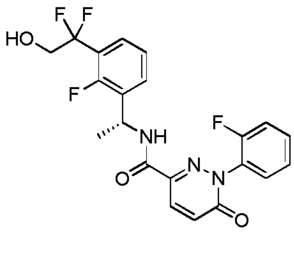, 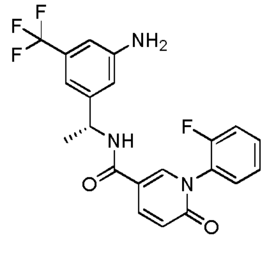, 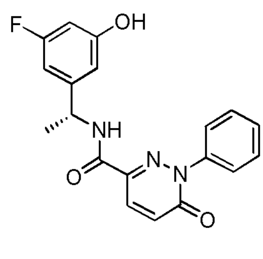,

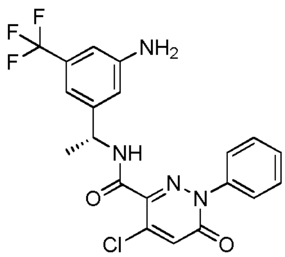, 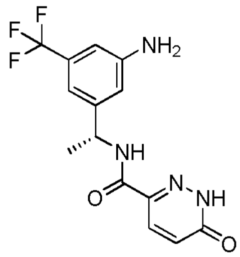, 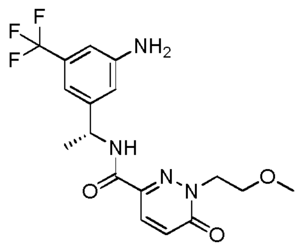, 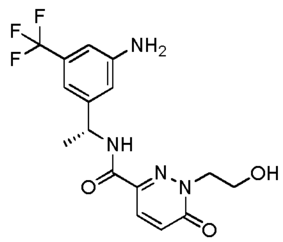,

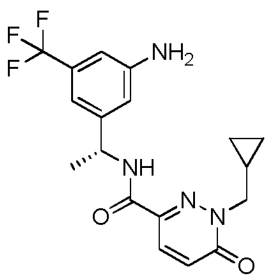, 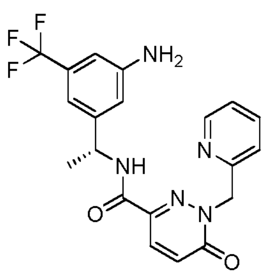, 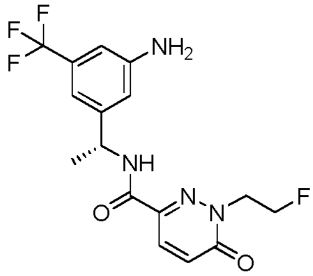, 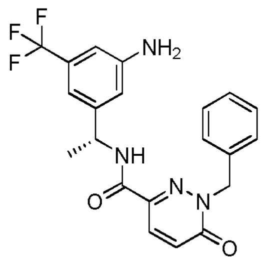,

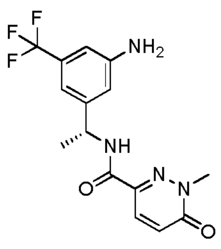, 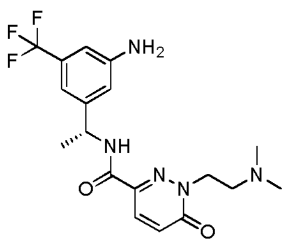, 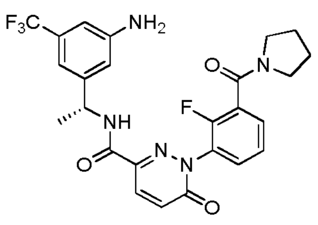, 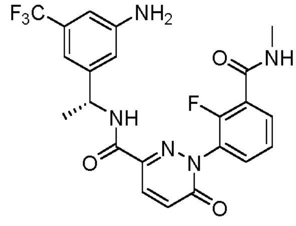,

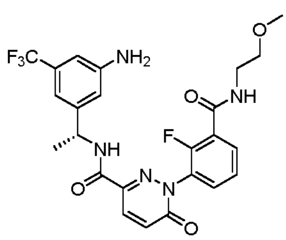, 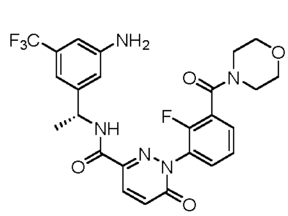, 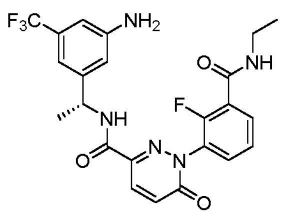, 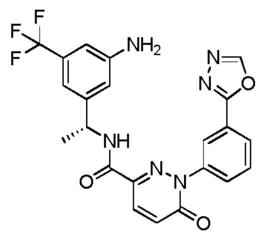,

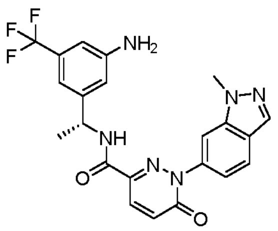,
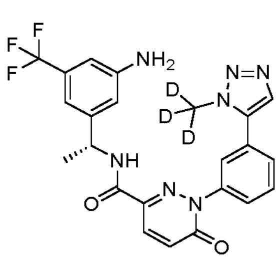,
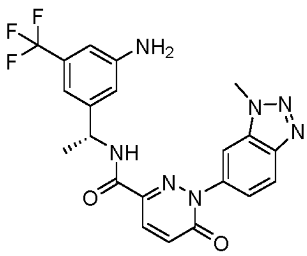,
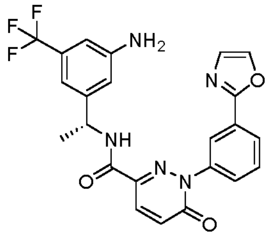,
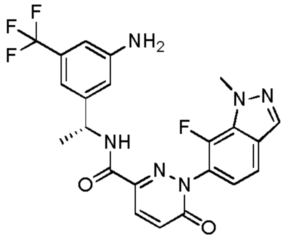,
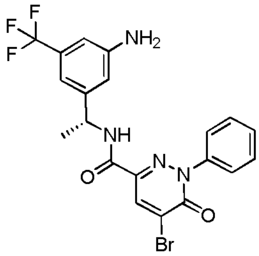,
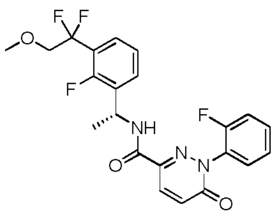,
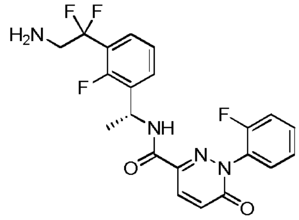,
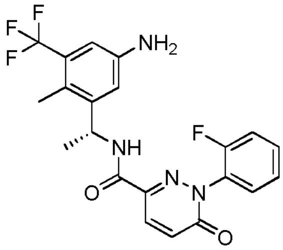,
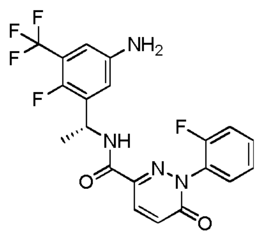,
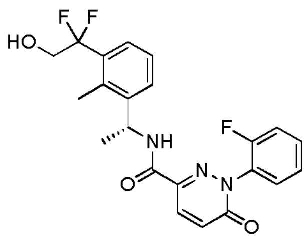,
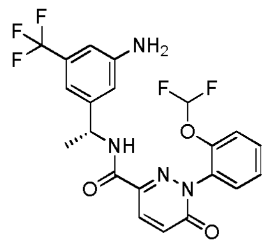,
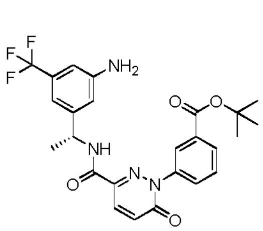,
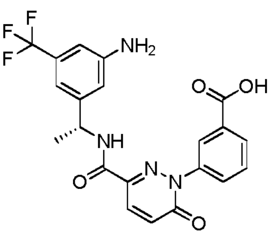,
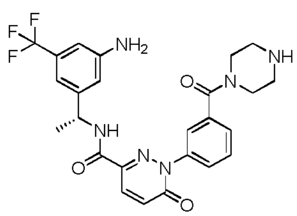,
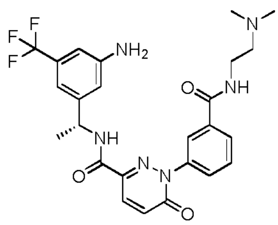,
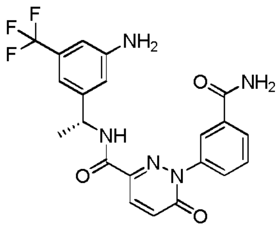,
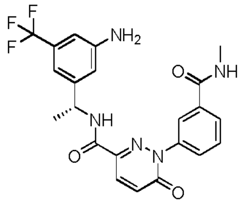,
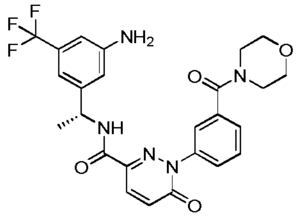,
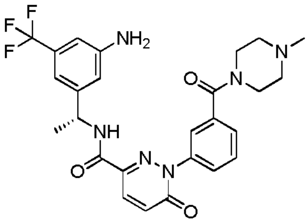,
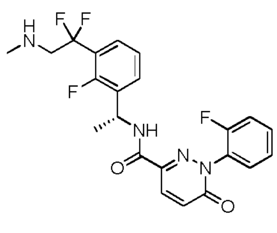,
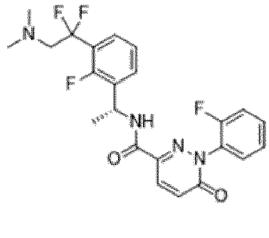,
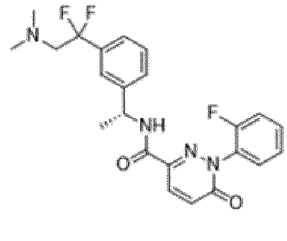,
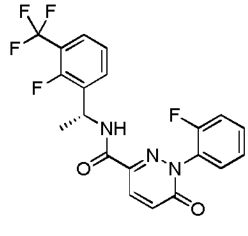,

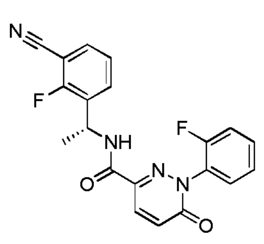 , 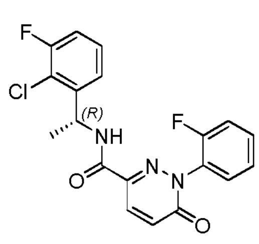 , 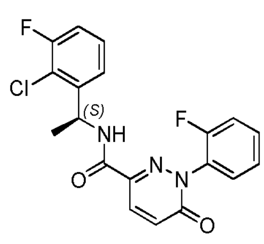 , 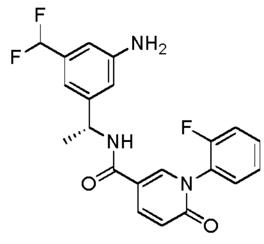 ,

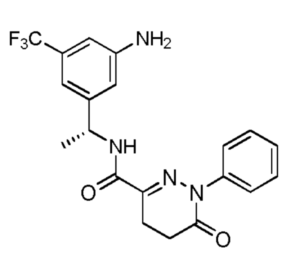 , 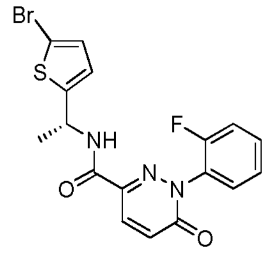 , 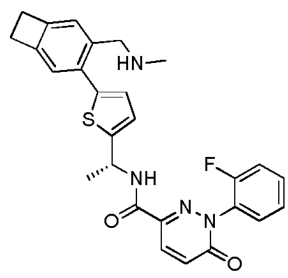 , 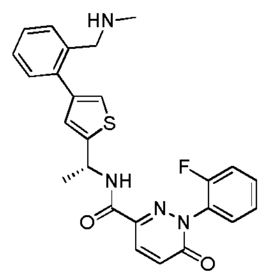 ,

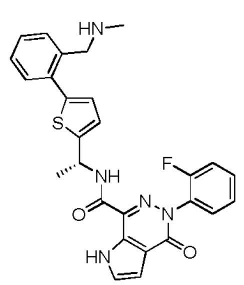 , 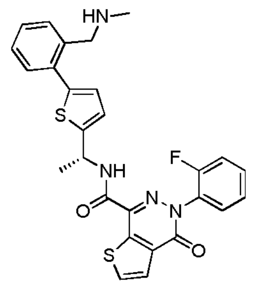 , 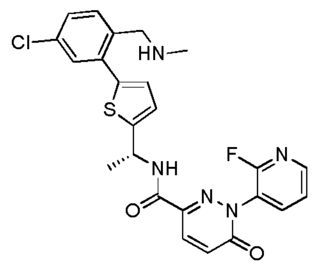 , 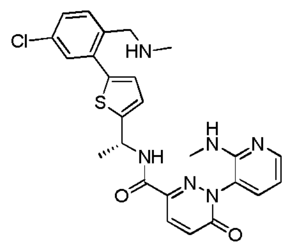 ,

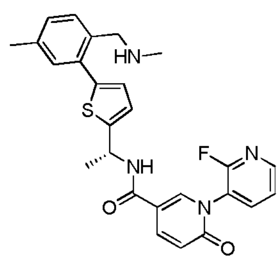 , 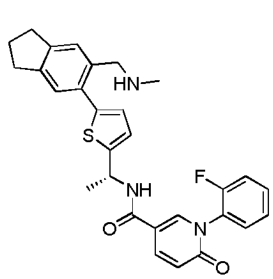 , 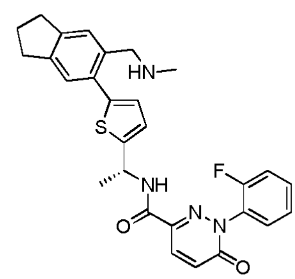 , 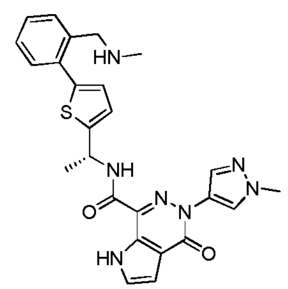 ,

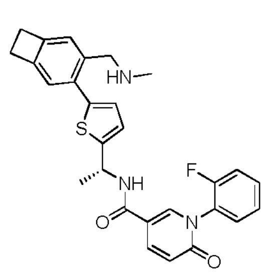 , 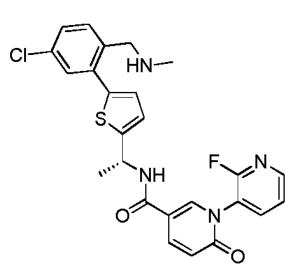 , 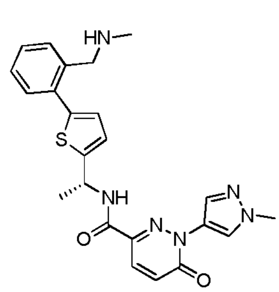 , 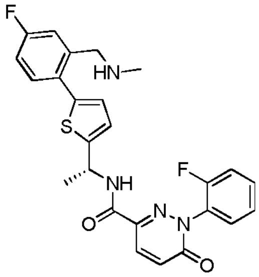 ,

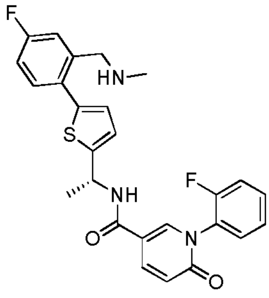 , 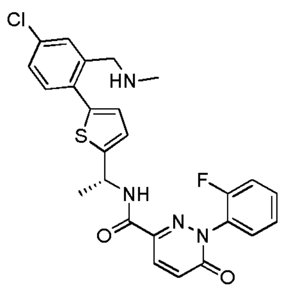 , 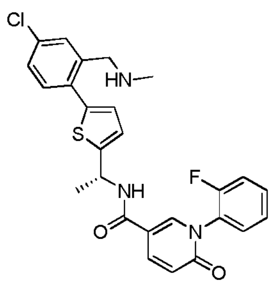 , 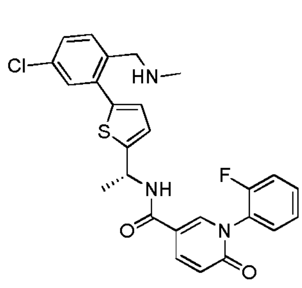 ,

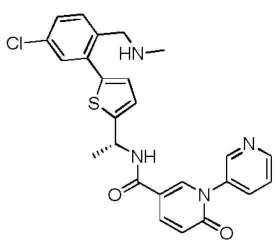 , 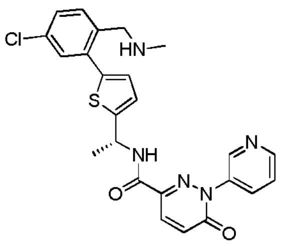 , 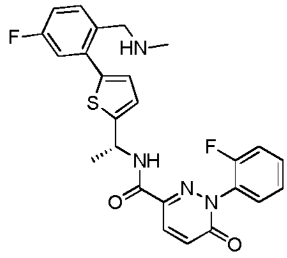 , 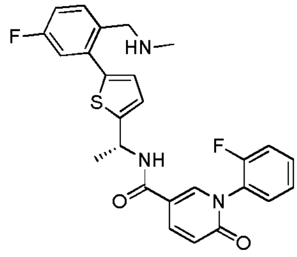 ,

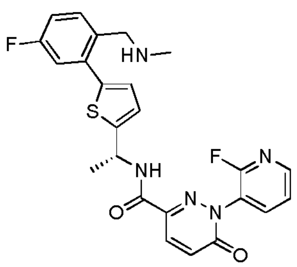 , 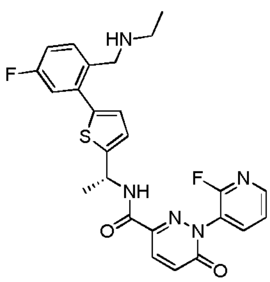 , 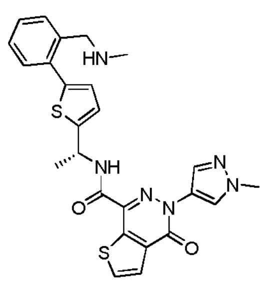 , 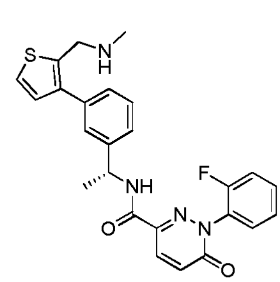 ,

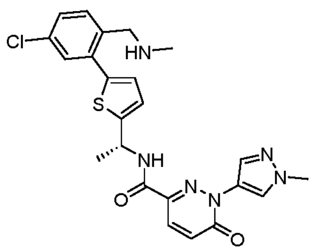 , 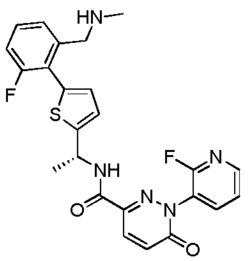 , 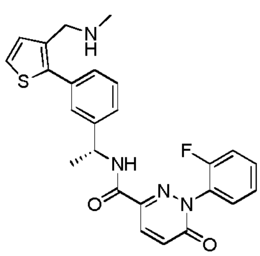 , 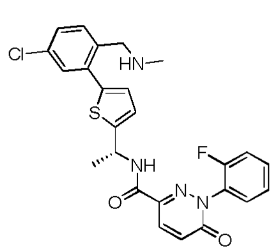 ,

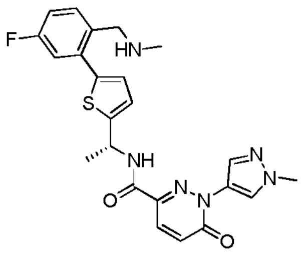 , 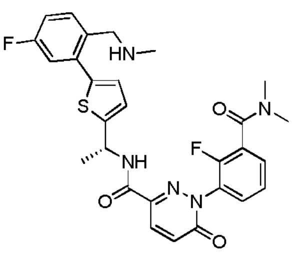 , 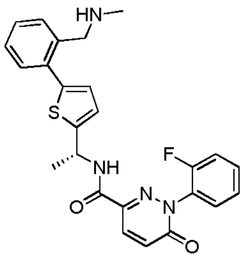 , 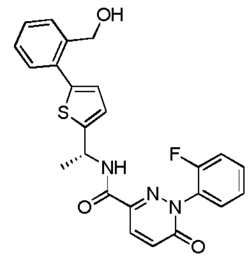 ,

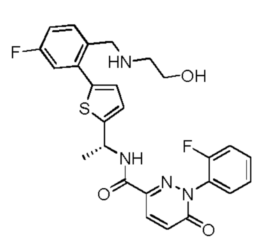, 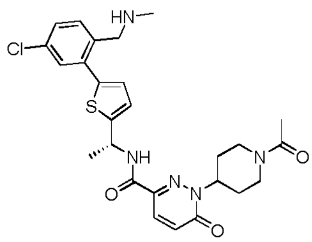, 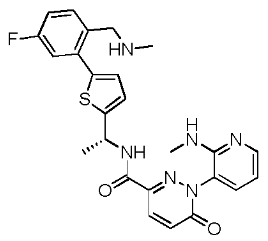, 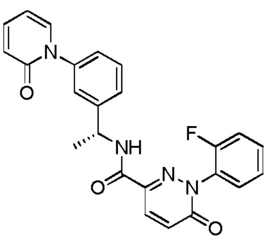,

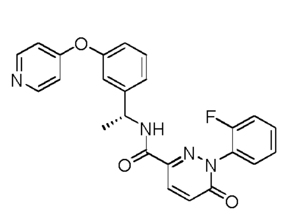, 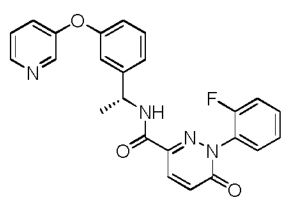, 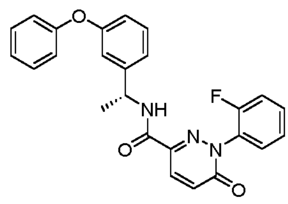, 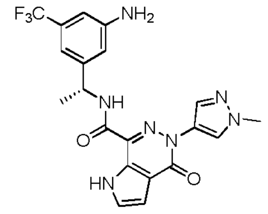,

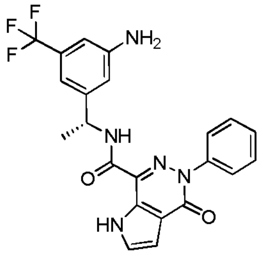, 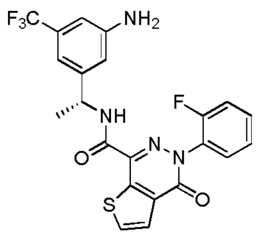, 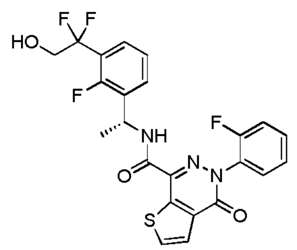, 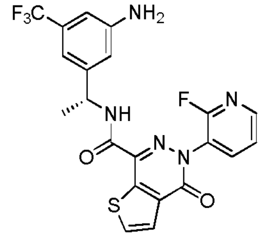,

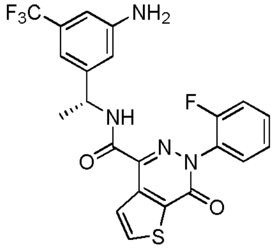, 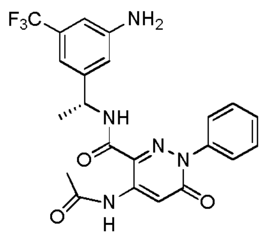, 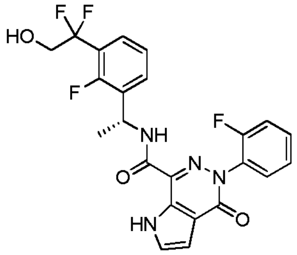, 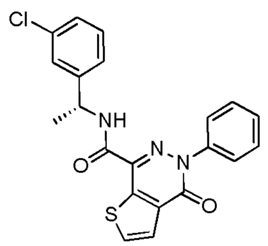,

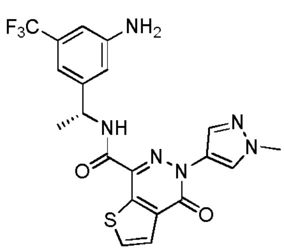, 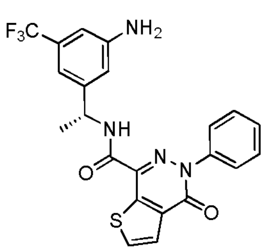, 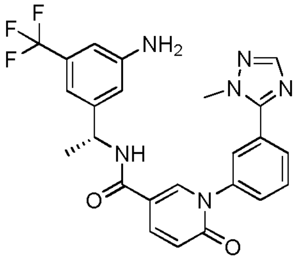, 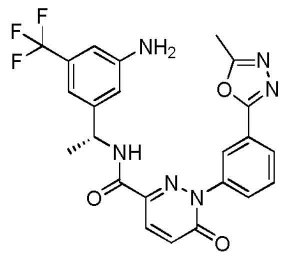,

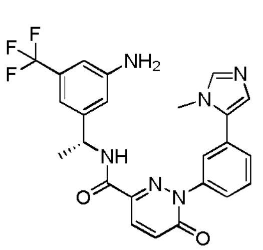, 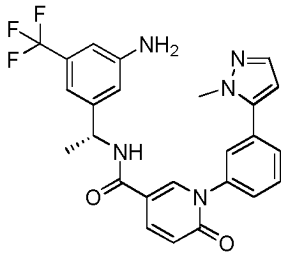, 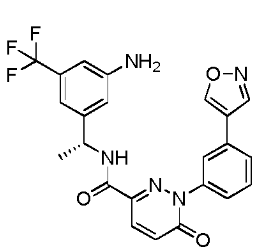, 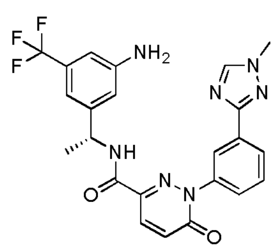,

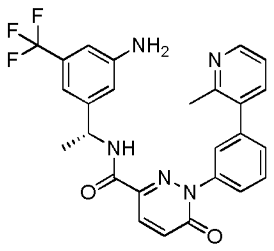 , 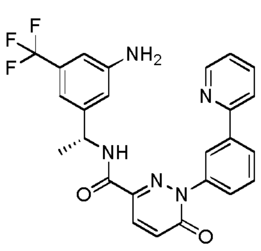 , 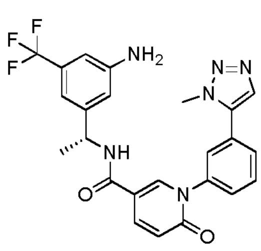 , 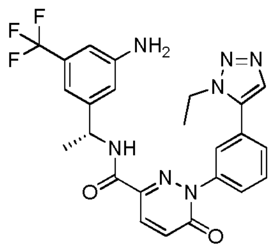 ,

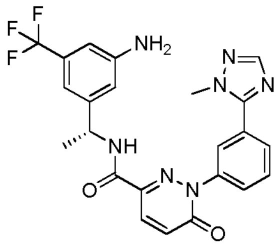 , 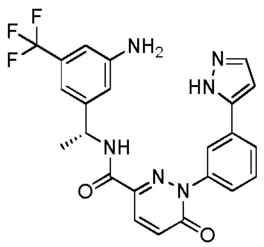 , 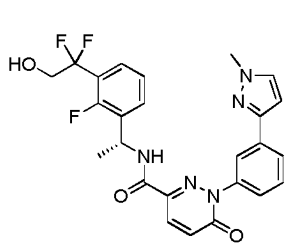 , 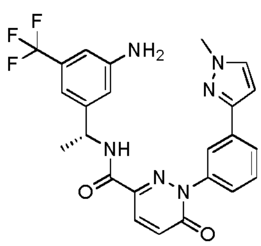 ,

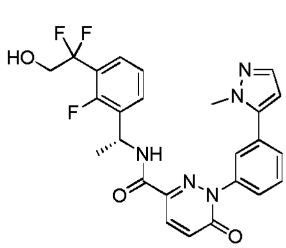 , 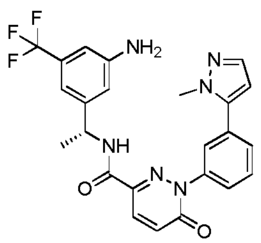 , 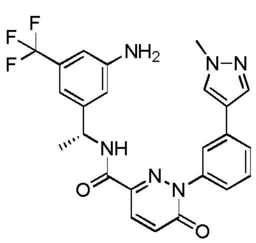 , 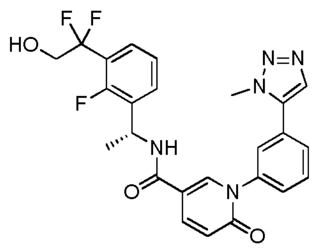 ,

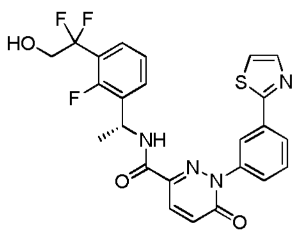 , 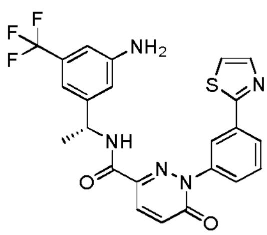 , 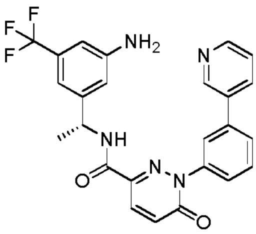 , 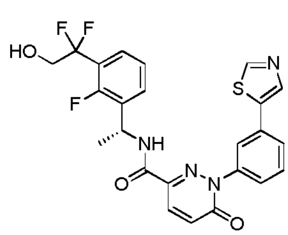 ,

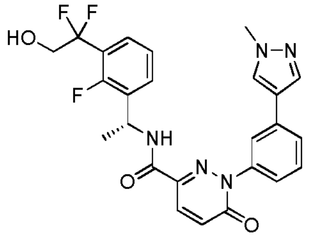 , 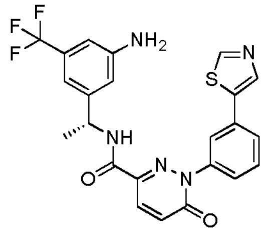 , 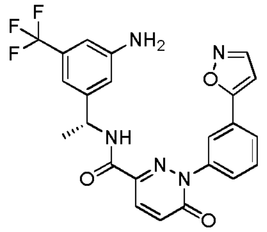 , 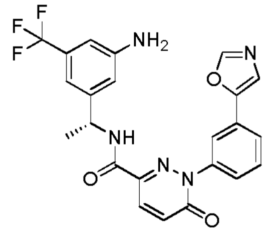 ,

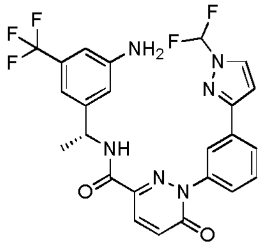 , 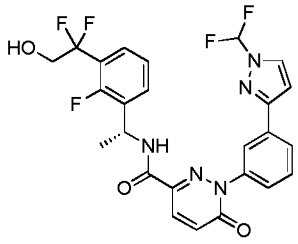 , 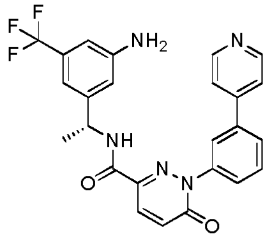 , 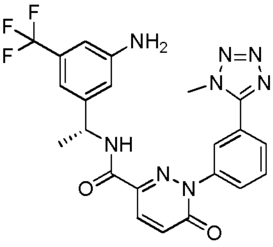 ,

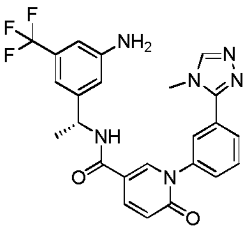 , 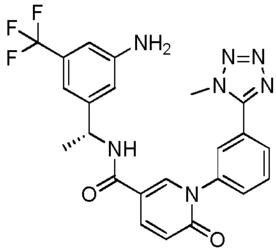 , 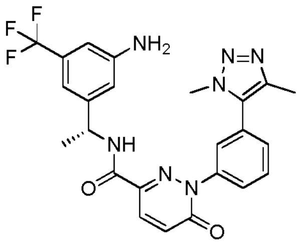 , 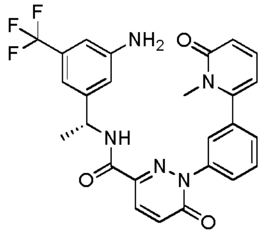 ,

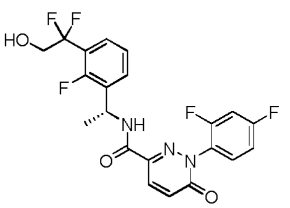 , 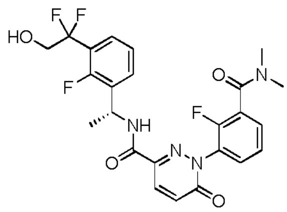 , 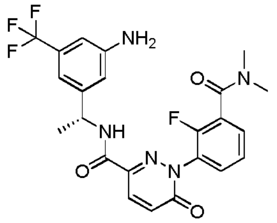 , 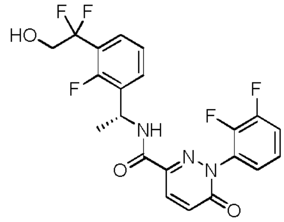 ,

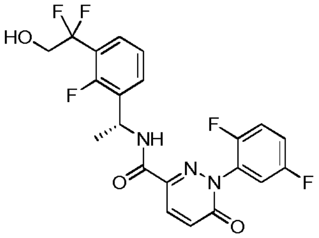 , 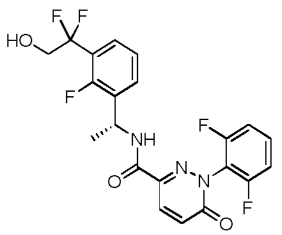 , 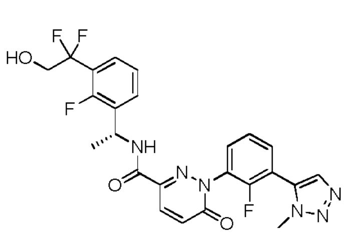 ,

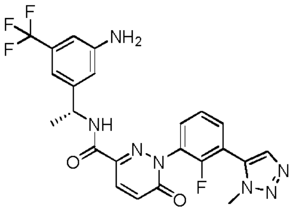 , 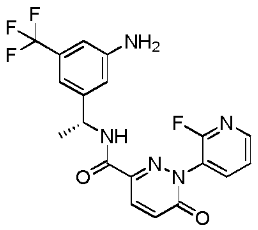 , 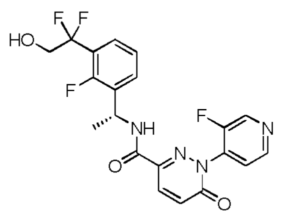 , 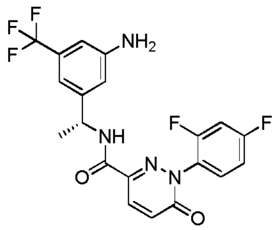 ,

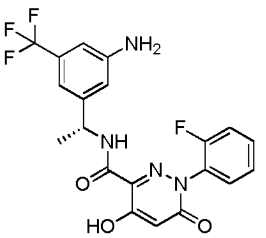 , 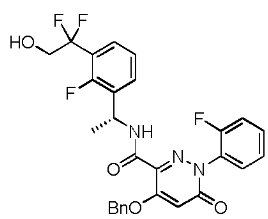 , 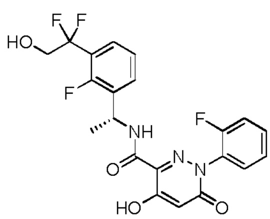 , 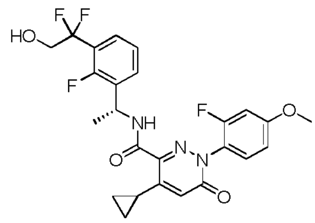 ,

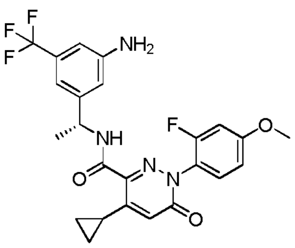 , 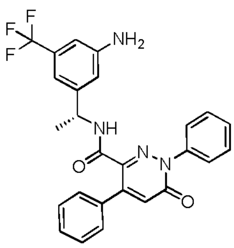 , 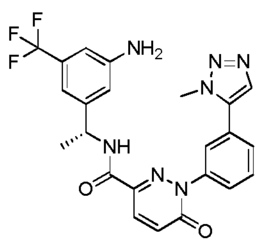 , 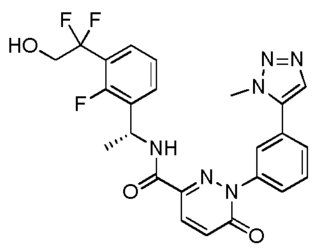 ,

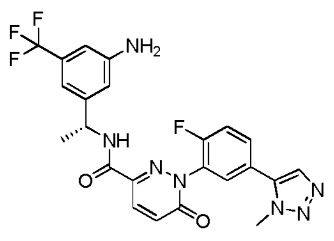
,
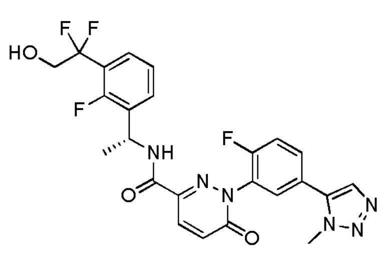
,
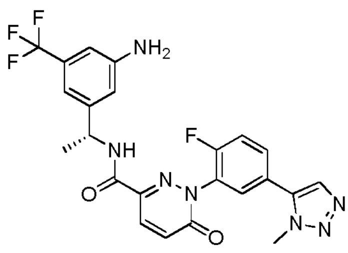
,

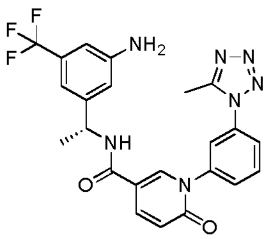
,
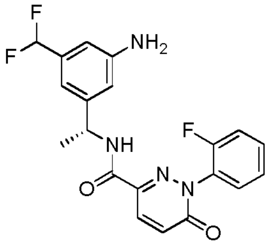
,
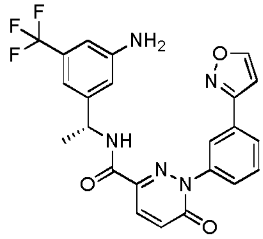
,
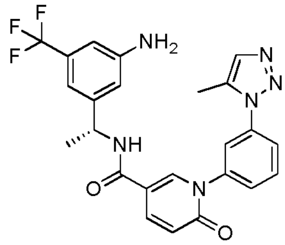
,

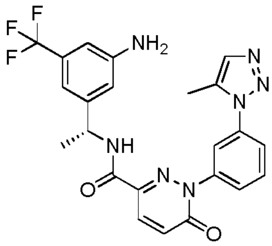
,
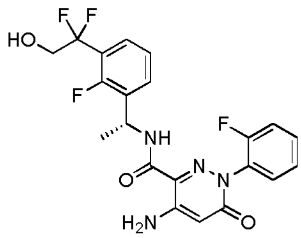
,
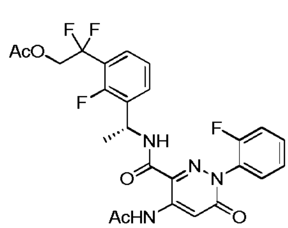
,
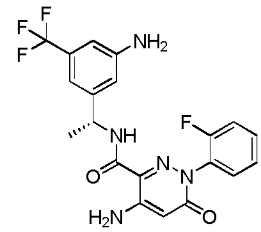
,

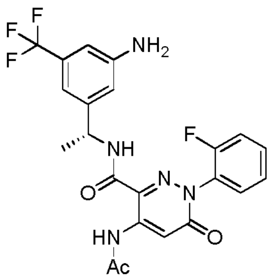
,
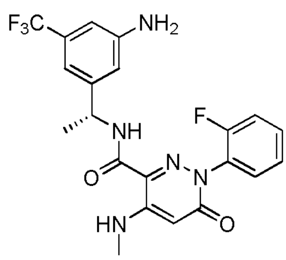
,
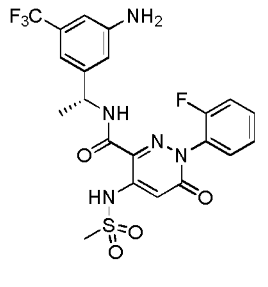
,
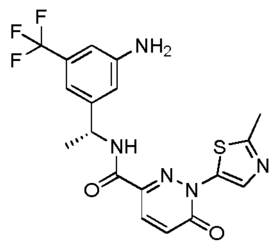
,

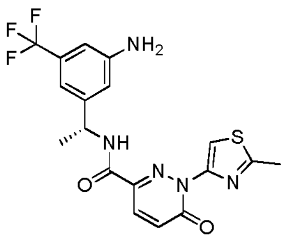
,
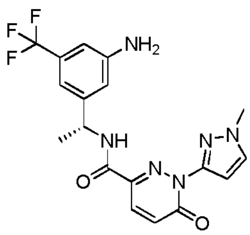
,
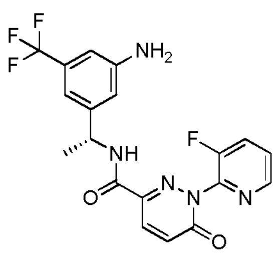
,
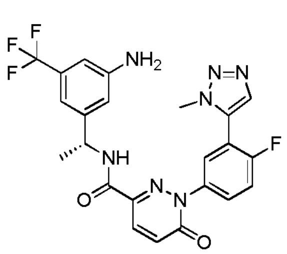
,

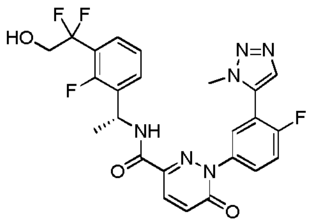 , 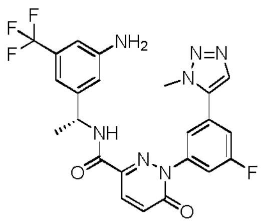 , 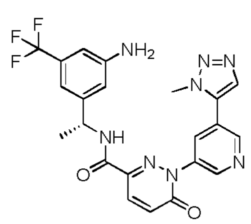 , 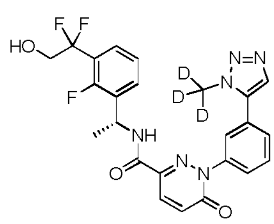 ,

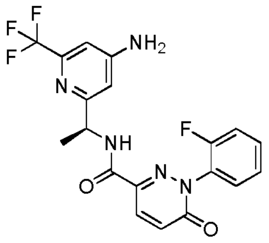 , 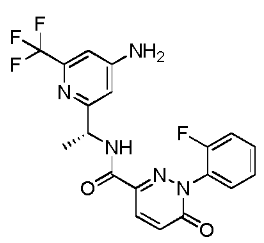 , 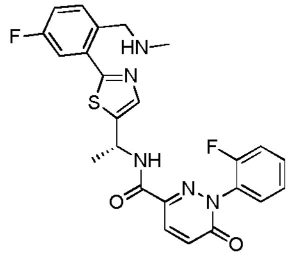 , 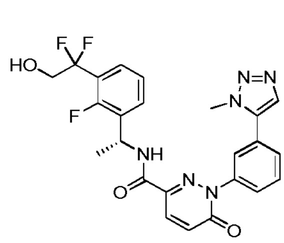 ,

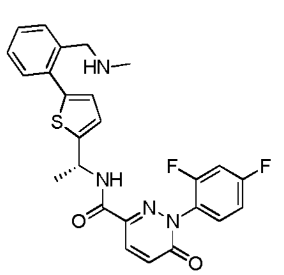 , 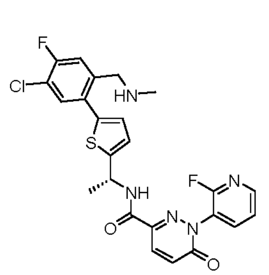 , 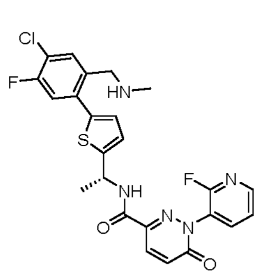 , 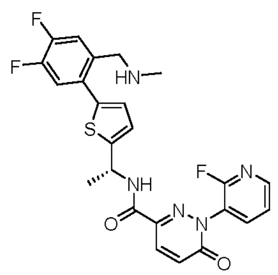 ,

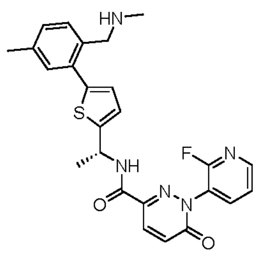 , 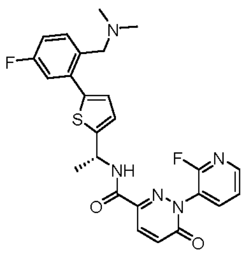 , 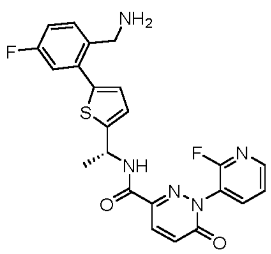 , 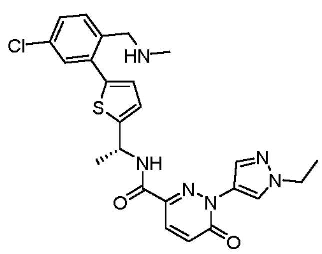 ,

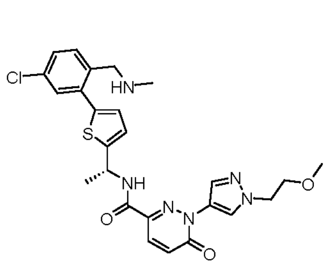 , 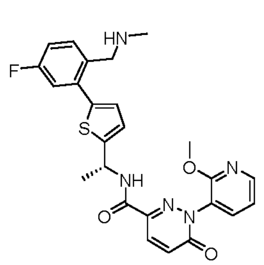 , 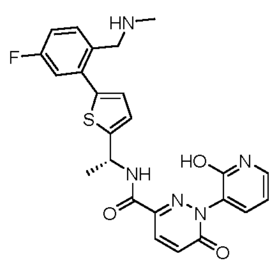 , 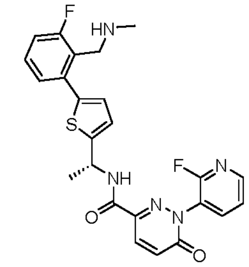 ,

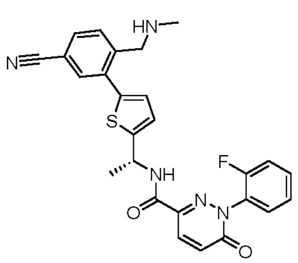 , 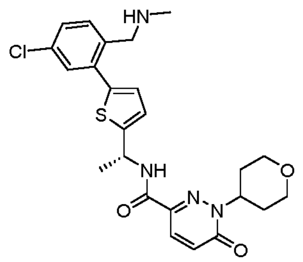 , 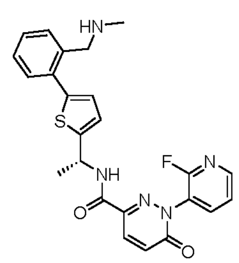 , 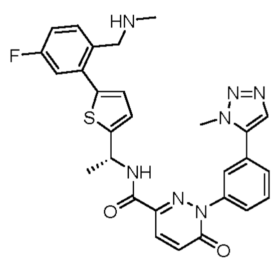 ,

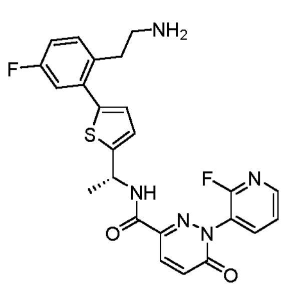 , and 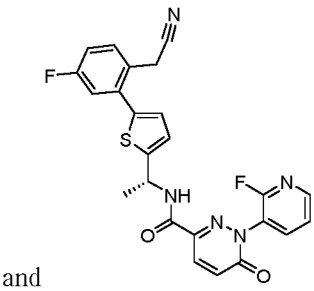 .

**41.** A pharmaceutical composition comprising the compound, or solvate, stereoisomer or pharmaceutically acceptable salt thereof according to any one of claims 1 to 40 as an active ingredient.

**42.** The pharmaceutical composition according to claim 41, **characterized in that** the pharmaceutical composition is for the prevention or treatment of a SOS1 mediated disease.

**43.** The pharmaceutical composition according to claim 42, **characterized in that** the SOS1 mediated disease is cancer or RASopathy.

**44.** The pharmaceutical composition according to claim 43, **characterized in that** the cancer is selected from the group consisting of pancreatic cancer, lung cancer, colorectal cancer, biliary tract cancer, multiple myeloma, melanoma, uterine cancer, cervical cancer, endometrial cancer, thyroid cancer, chronic lymphocytic leukemia, acute myeloid leukemia, bladder cancer, urothelial cancer, gastric cancer, squamous cell carcinoma of the head and neck, diffuse large B cell lymphoma, esophageal cancer, hepatocellular cancer, breast cancer, ovarian cancer, prostate cancer, glioblastoma, kidney cancer and sarcoma.

**45.** The pharmaceutical composition according to claim 43, **characterized in that** the RASopathy is selected from the group consisting of neurofibromatosis type 1, Noonan syndrome, Leopard syndrome, capillary malformation-arteriovenous malformation syndrome, Costello syndrome, CFC syndrome (Cardio-Facio-Cutaneous syndrome), Legius syndrome, and hereditary gingival fibromatosis.

**46.** A method for preventing or treating a SOS1 mediated disease, comprising administering to a subject the compound, or solvate, stereoisomer or pharmaceutically acceptable salt thereof according to any one of claims 1 to 40.

**47.** Use of the compound, or solvate, stereoisomer or pharmaceutically acceptable salt thereof according to any one of claims 1 to 40 for use in the prevention or treatment of a SOS1 mediated disease.

**48.** Use of the compound, or solvate, stereoisomer or pharmaceutically acceptable salt thereof according to any one of claims 1 to 40 for the manufacture of a medicament for the prevention or treatment of a SOS1 mediated disease.

## INTERNATIONAL SEARCH REPORT

International application No.
**PCT/KR2022/012254**

**A. CLASSIFICATION OF SUBJECT MATTER**

**C07D 237/14**(2006.01)i; **A61K 31/50**(2006.01)i; **A61P 35/00**(2006.01)i; **C07D 405/04**(2006.01)i; **C07D 401/04**(2006.01)i; **C07D 409/04**(2006.01)i

According to International Patent Classification (IPC) or to both national classification and IPC

**B. FIELDS SEARCHED**

Minimum documentation searched (classification system followed by classification symbols)

C07D 237/14(2006.01); A61K 31/131(2006.01); A61K 31/496(2006.01); A61K 31/50(2006.01); C07C 317/32(2006.01); C07C 317/34(2006.01); C07D 413/14(2006.01); C07D 453/02(2006.01); C07D 471/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: SOS1(Son of Sevenless1), RAS 패밀리 단백질(RAS-family protein), 암(cancer)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2014-031928 A2 (JONES, P. et al.) 27 February 2014 (2014-02-27)<br>See abstract; and example 93. | 1-4,6,11-15,25<br>5,7-10,16-24,26-40 |
| X | KR 10-2015-0013563 A (CHUGAI SEIYAKU KABUSHIKIKAISHA) 05 February 2015 (2015-02-05)<br>See claims 1 and 11; and example 689. | 1-6,8-15,24,25 |
| A | WO 2019-122129 A1 (BOEHRINGER INGELHEIM INTERNATIONAL GMBH) 27 June 2019 (2019-06-27)<br>See entire document. | 1-40 |
| A | WO 2021-127429 A1 (MIRATI THERAPEUTICS, INC.) 24 June 2021 (2021-06-24)<br>See entire document. | 1-40 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2022** | **28 November 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
PCT/KR2022/012254

C. DOCUMENTS CONSIDERED TO BE RELEVANT

| Category* | Citation of document, with indication, where appropriate, of the relevant passages | Relevant to claim No. |
|---|---|---|
| A | WO 2008-011557 A2 (BORCHARDT, A. J. et al.) 24 January 2008 (2008-01-24)<br>See entire document. | 1-40 |

Form PCT/ISA/210 (second sheet) (July 2022)

**INTERNATIONAL SEARCH REPORT**

International application No.
**PCT/KR2022/012254**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **46**
because they relate to subject matter not required to be searched by this Authority, namely:

Claim 46 pertains to a method for treatment of the human body by surgery or therapy, and thus pertains to a subject matter on which the International Searching Authority is not required to carry out an international search under the provisions of PCT Article 17(2)(a)(i) and PCT Rule 39.1(iv).

2. ☑ Claims Nos.: **42-45**
because they relate to parts of the international application that do not comply with the prescribed requirements to such an extent that no meaningful international search can be carried out, specifically:

Claims 42-45 refer to multiple dependent claims, which do not meet the requirement of PCT Rule 6.4(a).

3. ☑ Claims Nos.: **41, 46-48**
because they are dependent claims and are not drafted in accordance with the second and third sentences of Rule 6.4(a).

**INTERNATIONAL SEARCH REPORT**
**Information on patent family members**

International application No.
**PCT/KR2022/012254**

| Patent document cited in search report | Publication date (day/month/year) | Patent family member(s) | Publication date (day/month/year) |
|---|---|---|---|
| WO 2014-031928 A2 | 27 February 2014 | EP 2888256 A2 | 01 July 2015 |
| | | EP 2888256 A4 | 17 February 2016 |
| | | US 2014-0073634 A1 | 13 March 2014 |
| | | WO 2014-031928 A3 | 22 May 2014 |
| KR 10-2015-0013563 A | 05 February 2015 | AR 090836 A1 | 10 December 2014 |
| | | AU 2013-253539 A1 | 13 November 2014 |
| | | BR 112014026266 A2 | 27 June 2017 |
| | | CA 2871237 A1 | 31 October 2013 |
| | | CN 104379560 A | 25 February 2015 |
| | | EP 2842939 A1 | 04 March 2015 |
| | | EP 2842939 A4 | 11 November 2015 |
| | | EP 2842939 B1 | 30 May 2018 |
| | | HK 1203926 A1 | 06 November 2015 |
| | | JP 6177768 B2 | 09 August 2017 |
| | | MX 2014012991 A | 14 August 2015 |
| | | RU 2014147017 A | 10 June 2016 |
| | | SG 11201406871 A | 27 November 2014 |
| | | TW 201348187 A | 01 December 2013 |
| | | US 2015-0152047 A1 | 04 June 2015 |
| | | US 9695118 B2 | 04 July 2017 |
| | | WO 2013-161851 A1 | 31 October 2013 |
| WO 2019-122129 A1 | 27 June 2019 | AR 114164 A1 | 29 July 2020 |
| | | AU 2018-390927 A1 | 28 May 2020 |
| | | BR 112020010123 A2 | 10 November 2020 |
| | | CA 3085835 A1 | 27 June 2019 |
| | | CL 2020001501 A1 | 13 November 2020 |
| | | CL 2021000907 A1 | 29 October 2021 |
| | | CN 111372932 A | 03 July 2020 |
| | | CO 2020007218 A2 | 19 June 2020 |
| | | CR 20200312 A | 11 September 2020 |
| | | CR 20210307 A | 27 July 2021 |
| | | EA 202091491 A1 | 13 November 2020 |
| | | EC SP20040257 A | 31 August 2020 |
| | | EP 3728254 A1 | 28 October 2020 |
| | | JO P20200154 A1 | 21 June 2019 |
| | | JP 2021-506864 A | 22 February 2021 |
| | | KR 10-2020-0111163 A | 28 September 2020 |
| | | MA 51290 A | 31 March 2021 |
| | | PE 20210163 A1 | 26 January 2021 |
| | | SG 11202005881 A | 29 July 2020 |
| | | TW 201938557 A | 01 October 2019 |
| | | US 10829487 B2 | 10 November 2020 |
| | | US 2019-0194192 A1 | 27 June 2019 |
| | | US 2021-0009588 A1 | 14 January 2021 |
| WO 2021-127429 A1 | 24 June 2021 | AR 120851 A1 | 23 March 2022 |
| | | AU 2022-405170 A1 | 30 June 2022 |
| | | BR 112022012106 A2 | 20 September 2022 |
| | | CA 3161278 A1 | 24 June 2021 |
| | | CN 115135315 A | 30 September 2022 |
| | | CO 2022010241 A2 | 21 October 2022 |

**INTERNATIONAL SEARCH REPORT**
**Information on patent family members**

International application No.
**PCT/KR2022/012254**

| Patent document cited in search report | Publication date (day/month/year) | Patent family member(s) | Publication date (day/month/year) |
|---|---|---|---|
| | | EP 4076418 A1 | 26 October 2022 |
| | | IL 294048 A | 01 August 2022 |
| | | KR 10-2022-0130126 A | 26 September 2022 |
| | | TW 202136266 A | 01 October 2021 |
| | | US 2021-0188857 A1 | 24 June 2021 |
| WO 2008-011557 A2 | 24 January 2008 | AU 2007-275221 A1 | 24 January 2008 |
| | | BR PI0713187 A2 | 16 October 2012 |
| | | CA 2658764 A1 | 24 January 2008 |
| | | CN 101790527 A | 28 July 2010 |
| | | EP 2044061 A2 | 08 April 2009 |
| | | JP 2009-544625 A | 17 December 2009 |
| | | US 2008-0021026 A1 | 24 January 2008 |
| | | US 2008-0021217 A1 | 24 January 2008 |
| | | WO 2008-011557 A3 | 31 July 2008 |
| | | WO 2008-011560 A2 | 24 January 2008 |
| | | WO 2008-011560 A3 | 27 March 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Med. Chem.,* 2021, vol. 64 (10), 6569-6580
- *Current Opinion in Chemical Biology,* 2021, vol. 62, 109-118